# EUROPEAN PATENT SPECIFICATION

(11) **EP 2 941 484 B1**
(45) Date of publication and mention of the grant of the patent: **04.03.2020**
(21) Application number: 14700050.9
(22) Date of filing: 02.01.2014
(51) Int. Cl.: C12N 5/0784

(54) **METHOD FOR OBTAINING IMMUNO-SUPPRESSIVE DENDRITIC CELLS**
VERFAHREN ZUR GEWINNUNG IMMUNSUPPRESSIVER DENDRITISCHER ZELLEN
PROCÉDÉ D'OBTENTION DE CELLULES DENDRITIQUES IMMUNOSUPPRESSIVES

(30) Priority: 03.01.2013 GB 201300052; 03.01.2013 US 201361748550 P
(43) Date of publication of application: 11.11.2015
(73) Proprietor: Transimmune AG, 40212 Düsseldorf (DE); Yale University, New Haven, CT 06511 (US)
(72) Inventor: HENCO, Karsten, 40629 Düsseldorf (DE); BAUER, Günter, 24640 Schmalfeld (DE); DUCKWORTH, Justin, Weybridge Surrey KT13 8XB (GB); HAYDAY, Adrian, Orpington Kent BR6 9DR (GB); EDELSON, Richard, Westport Connecticut 06880 (US); TIGELAAR, Robert, New Haven Connecticut 06512 (US); GIRARDI, Michael, Madison Connecticut 06443 (US)
(74) Representative: Maiwald Patent- und Rechtsanwaltsgesellschaft mbH
(86) International application number: PCT/EP2014/050012
(87) International publication number: WO 2014/106631

(56) References cited:
- WO-A1-2011/137365
- SPISEK RADEK ET AL: "Maturation state of dendritic cells during the extracorporeal photopheresis and its relevance for the treatment of chronic graft-versus-host disease.", TRANSFUSION JAN 2006, vol. 46, no. 1, January 2006 (2006-01), pages 55-65, XP55112614, ISSN: 0041-1132
- BAIRD A H ET AL: "Generation of immature dendritic cells by modified Extracorporeal photophoresis", JOURNAL OF INVESTIGATIVE DERMATOLOGY, vol. 130, no. Suppl. 1, April 2010 (2010-04), page S136, XP55112893, & ANNUAL MEETING OF THE SOCIETY-FOR-INVESTIGATIVE-DERMATOLOGY; ATLANTA, GA, USA; MAY 05 -08, 2010 ISSN: 0022-202X
- BERGER CAROLE ET AL: "Rapid generation of maturationally synchronized human dendritic cells: contribution to the clinical efficacy of extracorporeal photochemotherapy.", BLOOD 2 DEC 2010, vol. 116, no. 23, 2 December 2010 (2010-12-02), pages 4838-4847, XP55112768, ISSN: 1528-0020
- KHALIL D ET AL: "Conversion of monocytes to dendritic cells by platelet signaling", JOURNAL OF INVESTIGATIVE DERMATOLOGY, vol. 132, no. Suppl. 1, May 2012 (2012-05) , page S101, XP55113116, & 75TH ANNUAL MEETING OF THE SOCIETY-FOR-INVESTIGATIVE-DERMATOLOGY; RALEIGH, NC, USA; MAY 09 -12, 2012
- DURAZZO T S ET AL: "Platelet induction of monocyle-to-dendritic cell maturation", JOURNAL OF INVESTIGATIVE DERMATOLOGY, vol. 130, no. Suppl. 1, April 2010 (2010-04), page S121, XP55113123, & ANNUAL MEETING OF THE SOCIETY-FOR-INVESTIGATIVE-DERMATOLOGY; ATLANTA, GA, USA; MAY 05 -08, 2010 ISSN: 0022-202X
- HU JIM ET AL: "Tolerogenic dendritic cells and their potential applications.", IMMUNOLOGY MAR 2011, vol. 132, no. 3, March 2011 (2011-03), pages 307-314, XP55112839, ISSN: 1365-2567 cited in the application
- A. Zimmer ET AL: "Identification of a New Phenotype of Tolerogenic Human Dendritic Cells Induced by Fungal Proteases from Aspergillus oryzae", THE JOURNAL OF IMMUNOLOGY, vol. 186, no. 7, 2 March 2011 (2011-03-02) , pages 3966-3976, XP055243904, US ISSN: 0022-1767, DOI: 10.4049/jimmunol.1003184
- PHILLIPS BRETT EUGENE ET AL: "Clinical Tolerogenic Dendritic Cells: Exploring Therapeutic Impact on Human Autoimmune Disease.", FRONTIERS IN IMMUNOLOGY 2017, vol. 8, 1279, 1 October 2017 (2017-10-01), pages 1-9, ISSN: 1664-3224, DOI: 10.3389/fimmu.2017.01279
- HARRY RACHEL A ET AL: "Generation and characterisation of therapeutic tolerogenic dendritic cells for rheumatoid arthritis", ANNALS OF THE RHEUMATIC DISE, B M J GROUP, GB, vol. 69, no. 11, 1 November 2010 (2010-11-01), pages 2042-2050, XP009153777, ISSN: 1468-2060, DOI: 10.1136/ARD.2009.126383
- VENTURA ALESSANDRA ET AL: "Extracorporeal Photochemotherapy Drives Monocyte-to-Dendritic Cell Maturation to Induce Anticancer Immunity.", CANCER RESEARCH 15 JUL 2018, vol. 78, no. 14, 15 July 2018 (2018-07-15) , pages 4045-4058, ISSN: 1538-7445
- WILCOX RYAN A: "Cutaneous T-cell lymphoma: 2016 update on diagnosis, risk-stratification, and management.", AMERICAN JOURNAL OF HEMATOLOGY JAN 2016, vol. 91, no. 1, January 2016 (2016-01), pages 151-165, ISSN: 1096-8652

### SEQUENCE LISTING

<110> TRANSIMMUNE AG, YALE UNIVERSITY
<120> Method for obtaining immuno-suppressive dendritic cells
<130> T 7757-WO
<150> GB1300052.6
   <151> 2013-01-03
<150> USP 61/748,550
   <151> 2013-01-03
<160> 105
<170> PatentIn version 3.5
<210> 1
   <211> 10515
   <212> DNA
   <213> Homo sapiens
<400> 1
   ggaggaggga gagcacaggc tttgaccgat agtaacctct gcgctcggtg cagccgaatc      60
   tataaaagga actagtcccg gcaaaaaccc cgtaattgcg agcgagagtg agtggggccg     120
   ggacccgcag agccgagccg acccttctct cccgggctgc ggcagggcag ggcggggagc     180
   tccgcgcacc aacagagccg gttctcaggg cgctttgctc cttgtttttt ccccggttct     240
   gttttctccc cttctccgga aggcttgtca aggggtagga gaaagagacg caaacacaaa     300
   agtggaaaac agttaatgac cagccacggc gtccctgctg tgagctctgg ccgctgcctt     360
   ccagggctcc cgagccacac gctgggggtg ctggctgagg gaacatggct tgttggcctc    420
   agctgaggtt gctgctgtgg aagaacctca ctttcagaag aagacaaaca tgtcagctgc     480
   tgctggaagt ggcctggcct ctatttatct tcctgatcct gatctctgtt cggctgagct     540
   acccacccta tgaacaacat gaatgccatt ttccaaataa agccatgccc tctgcaggaa     600
   cacttccttg ggttcagggg attatctgta atgccaacaa cccctgtttc cgttacccga     660
   ctcctgggga ggctcccgga gttgttggaa actttaacaa atccattgtg gctcgcctgt     720
   tctcagatgc tcggaggctt cttttataca gccagaaaga caccagcatg aaggacatgc     780
   gcaaagttct gagaacatta cagcagatca agaaatccag ctcaaacttg aagcttcaag     840
   atttcctggt ggacaatgaa accttctctg ggttcctgta tcacaacctc tctctcccaa     900
   agtctactgt ggacaagatg ctgagggctg atgtcattct ccacaaggta tttttgcaag     960
   gctaccagtt acatttgaca agtctgtgca atggatcaaa atcagaagag atgattcaac    1020
   ttggtgacca agaagtttct gagctttgtg gcctaccaag ggagaaactg gctgcagcag    1080
   agcgagtact tcgttccaac atggacatcc tgaagccaat cctgagaaca ctaaactcta    1140
   catctccctt cccgagcaag gagctggctg aagccacaaa aacattgctg catagtcttg    1200
   ggactctggc ccaggagctg ttcagcatga gaagctggag tgacatgcga caggaggtga 1260
   tgtttctgac caatgtgaac agctccagct cctccaccca aatctaccag gctgtgtctc 1320
   gtattgtctg cgggcatccc gagggagggg ggctgaagat caagtctctc aactggtatg 1380
   aggacaacaa ctacaaagcc ctctttggag gcaatggcac tgaggaagat gctgaaacct 1440
   tctatgacaa ctctacaact ccttactgca atgatttgat gaagaatttg gagtctagtc 1500
   ctctttcccg cattatctgg aaagctctga agccgctgct cgttgggaag atcctgtata 1560
   cacctgacac tccagccaca aggcaggtca tggctgaggt gaacaagacc ttccaggaac 1620
   tggctgtgtt ccatgatctg gaaggcatgt gggaggaact cagccccaag atctggacct 1680
   tcatggagaa cagccaagaa atggaccttg tccggatgct gttggacagc agggacaatg 1740
   accacttttg ggaacagcag ttggatggct tagattggac agcccaagac atcgtggcgt 1800
   ttttggccaa gcacccagag gatgtccagt ccagtaatgg ttctgtgtac acctggagag 1860
   aagctttcaa cgagactaac caggcaatcc ggaccatatc tcgcttcatg gagtgtgtca 1920
   acctgaacaa gctagaaccc atagcaacag aagtctggct catcaacaag tccatggagc 1980
   tgctggatga gaggaagttc tgggctggta ttgtgttcac tggaattact ccaggcagca 2040
   ttgagctgcc ccatcatgtc aagtacaaga tccgaatgga cattgacaat gtggagagga 2100
   caaataaaat caaggatggg tactgggacc ctggtcctcg agctgacccc tttgaggaca 2160
   tgcggtacgt ctgggggggc ttcgcctact tgcaggatgt ggtggagcag gcaatcatca 2220
   gggtgctgac gggcaccgag aagaaaactg gtgtctatat gcaacagatg ccctatccct 2280
   gttacgttga tgacatcttt ctgcgggtga tgagccggtc aatgcccctc ttcatgacgc 2340
   tggcctggat ttactcagtg gctgtgatca tcaagggcat cgtgtatgag aaggaggcac 2400
   ggctgaaaga gaccatgcgg atcatgggcc tggacaacag catcctctgg tttagctggt 2460
   tcattagtag cctcattcct cttcttgtga gcgctggcct gctagtggtc atcctgaagt 2520
   taggaaacct gctgccctac agtgatccca gcgtggtgtt tgtcttcctg tccgtgtttg 2580
   ctgtggtgac aatcctgcag tgcttcctga ttagcacact cttctccaga gccaacctgg 2640
   cagcagcctg tgggggcatc atctacttca cgctgtacct gccctacgtc ctgtgtgtgg 2700
   catggcagga ctacgtgggc ttcacactca agatcttcgc tagcctgctg tctcctgtgg 2760
   cttttgggtt tggctgtgag tactttgccc tttttgagga gcagggcatt ggagtgcagt 2820
   gggacaacct gtttgagagt cctgtggagg aagatggctt caatctcacc acttcggtct 2880
   ccatgatgct gtttgacacc ttcctctatg gggtgatgac ctggtacatt gaggctgtct 2940
   ttccaggcca gtacggaatt cccaggccct ggtattttcc ttgcaccaag tcctactggt 3000
   ttggcgagga aagtgatgag aagagccacc ctggttccaa ccagaagaga atatcagaaa 3060
   tctgcatgga ggaggaaccc acccacttga agctgggcgt gtccattcag aacctggtaa 3120
   aagtctaccg agatgggatg aaggtggctg tcgatggcct ggcactgaat ttttatgagg 3180
   gccagatcac ctccttcctg ggccacaatg gagcggggaa gacgaccacc atgtcaatcc 3240
   tgaccgggtt gttccccccg acctcgggca ccgcctacat cctgggaaaa gacattcgct 3300
   ctgagatgag caccatccgg cagaacctgg gggtctgtcc ccagcataac gtgctgtttg 3360
   acatgctgac tgtcgaagaa cacatctggt tctatgcccg cttgaaaggg ctctctgaga 3420
   agcacgtgaa ggcggagatg gagcagatgg ccctggatgt tggtttgcca tcaagcaagc 3480
   tgaaaagcaa aacaagccag ctgtcaggtg gaatgcagag aaagctatct gtggccttgg 3540
   cctttgtcgg gggatctaag gttgtcattc tggatgaacc cacagctggt gtggaccctt 3600
   actcccgcag gggaatatgg gagctgctgc tgaaataccg acaaggccgc accattattc 3660
   tctctacaca ccacatggat gaagcggacg tcctggggga caggattgcc atcatctccc 3720
   atgggaagct gtgctgtgtg ggctcctccc tgtttctgaa gaaccagctg ggaacaggct 3780
   actacctgac cttggtcaag aaagatgtgg aatcctccct cagttcctgc agaaacagta 3840
   gtagcactgt gtcatacctg aaaaaggagg acagtgtttc tcagagcagt tctgatgctg 3900
   gcctgggcag cgaccatgag agtgacacgc tgaccatcga tgtctctgct atctccaacc 3960
   tcatcaggaa gcatgtgtct gaagcccggc tggtggaaga catagggcat gagctgacct 4020
   atgtgctgcc atatgaagct gctaaggagg gagcctttgt ggaactcttt catgagattg 4080
   atgaccggct ctcagacctg ggcatttcta gttatggcat ctcagagacg accctggaag 4140
   aaatattcct caaggtggcc gaagagagtg gggtggatgc tgagacctca gatggtacct 4200
   tgccagcaag acgaaacagg cgggccttcg gggacaagca gagctgtctt cgcccgttca 4260
   ctgaagatga tgctgctgat ccaaatgatt ctgacataga cccagaatcc agagagacag 4320
   acttgctcag tgggatggat ggcaaagggt cctaccaggt gaaaggctgg aaacttacac 4380
   agcaacagtt tgtggccctt ttgtggaaga gactgctaat tgccagacgg agtcggaaag 4440
   gattttttgc tcagattgtc ttgccagctg tgtttgtctg cattgccctt gtgttcagcc 4500
   tgatcgtgcc accctttggc aagtacccca gcctggaact tcagccctgg atgtacaacg 4560
   aacagtacac atttgtcagc aatgatgctc ctgaggacac gggaaccctg gaactcttaa 4620
   acgccctcac caaagaccct ggcttcggga cccgctgtat ggaaggaaac ccaatcccag 4680
   acacgccctg ccaggcaggg gaggaagagt ggaccactgc cccagttccc cagaccatca 4740
   tggacctctt ccagaatggg aactggacaa tgcagaaccc ttcacctgca tgccagtgta 4800
   gcagcgacaa aatcaagaag atgctgcctg tgtgtccccc aggggcaggg gggctgcctc 4860
   ctccacaaag aaaacaaaac actgcagata tccttcagga cctgacagga agaaacattt 4920
   cggattatct ggtgaagacg tatgtgcaga tcatagccaa aagcttaaag aacaagatct 4980
   gggtgaatga gtttaggtat ggcggctttt ccctgggtgt cagtaatact caagcacttc 5040
   ctccgagtca agaagttaat gatgccatca aacaaatgaa gaaacaccta aagctggcca 5100
   aggacagttc tgcagatcga tttctcaaca gcttgggaag atttatgaca ggactggaca 5160
   ccaaaaataa tgtcaaggtg tggttcaata acaagggctg gcatgcaatc agctctttcc 5220
   tgaatgtcat caacaatgcc attctccggg ccaacctgca aaagggagag aaccctagcc 5280
   attatggaat tactgctttc aatcatcccc tgaatctcac caagcagcag ctctcagagg 5340
   tggctctgat gaccacatca gtggatgtcc ttgtgtccat ctgtgtcatc tttgcaatgt 5400
   ccttcgtccc agccagcttt gtcgtattcc tgatccagga gcgggtcagc aaagcaaaac 5460
   acctgcagtt catcagtgga gtgaagcctg tcatctactg gctctctaat tttgtctggg 5520
   atatgtgcaa ttacgttgtc cctgccacac tggtcattat catcttcatc tgcttccagc 5580
   agaagtccta tgtgtcctcc accaatctgc ctgtgctagc ccttctactt ttgctgtatg 5640
   ggtggtcaat cacacctctc atgtacccag cctcctttgt gttcaagatc cccagcacag 5700
   cctatgtggt gctcaccagc gtgaacctct tcattggcat taatggcagc gtggccacct 5760
   ttgtgctgga gctgttcacc gacaataagc tgaataatat caatgatatc ctgaagtccg 5820
   tgttcttgat cttcccacat ttttgcctgg gacgagggct catcgacatg gtgaaaaacc 5880
   aggcaatggc tgatgccctg gaaaggtttg gggagaatcg ctttgtgtca ccattatctt 5940
   gggacttggt gggacgaaac ctcttcgcca tggccgtgga aggggtggtg ttcttcctca 6000
   ttactgttct gatccagtac agattcttca tcaggcccag acctgtaaat gcaaagctat 6060
   ctcctctgaa tgatgaagat gaagatgtga ggcgggaaag acagagaatt cttgatggtg 6120
   gaggccagaa tgacatctta gaaatcaagg agttgacgaa gatatataga aggaagcgga 6180
   agcctgctgt tgacaggatt tgcgtgggca ttcctcctgg tgagtgcttt gggctcctgg 6240
   gagttaatgg ggctggaaaa tcatcaactt tcaagatgtt aacaggagat accactgtta 6300
   ccagaggaga tgctttcctt aacaaaaata gtatcttatc aaacatccat gaagtacatc 6360
   agaacatggg ctactgccct cagtttgatg ccatcacaga gctgttgact gggagagaac 6420
   acgtggagtt ctttgccctt ttgagaggag tcccagagaa agaagttggc aaggttggtg 6480
   agtgggcgat tcggaaactg ggcctcgtga agtatggaga aaaatatgct ggtaactata 6540
   gtggaggcaa caaacgcaag ctctctacag ccatggcttt gatcggcggg cctcctgtgg 6600
   tgtttctgga tgaacccacc acaggcatgg atcccaaagc ccggcggttc ttgtggaatt 6660
   gtgccctaag tgttgtcaag gaggggagat cagtagtgct tacatctcat agtatggaag 6720
   aatgtgaagc tctttgcact aggatggcaa tcatggtcaa tggaaggttc aggtgccttg 6780
   gcagtgtcca gcatctaaaa aataggtttg gagatggtta tacaatagtt gtacgaatag 6840
   cagggtccaa cccggacctg aagcctgtcc aggatttctt tggacttgca tttcctggaa 6900
   gtgttctaaa agagaaacac cggaacatgc tacaatacca gcttccatct tcattatctt 6960
   ctctggccag gatattcagc atcctctccc agagcaaaaa gcgactccac atagaagact 7020
   actctgtttc tcagacaaca cttgaccaag tatttgtgaa ctttgccaag gaccaaagtg 7080
   atgatgacca cttaaaagac ctctcattac acaaaaacca gacagtagtg gacgttgcag 7140
   ttctcacatc ttttctacag gatgagaaag tgaaagaaag ctatgtatga agaatcctgt 7200
   tcatacgggg tggctgaaag taaagaggaa ctagactttc ctttgcacca tgtgaagtgt 7260
   tgtggagaaa agagccagaa gttgatgtgg gaagaagtaa actggatact gtactgatac 7320
   tattcaatgc aatgcaattc aatgcaatga aaacaaaatt ccattacagg ggcagtgcct 7380
   ttgtagccta tgtcttgtat ggctctcaag tgaaagactt gaatttagtt ttttacctat 7440
   acctatgtga aactctatta tggaacccaa tggacatatg ggtttgaact cacacttttt 7500
   tttttttttt tgttcctgtg tattctcatt ggggttgcaa caataattca tcaagtaatc 7560
   atggccagcg attattgatc aaaatcaaaa ggtaatgcac atcctcattc actaagccat 7620
   gccatgccca ggagactggt ttcccggtga cacatccatt gctggcaatg agtgtgccag 7680
   agttattagt gccaagtttt tcagaaagtt tgaagcacca tggtgtgtca tgctcacttt 7740
   tgtgaaagct gctctgctca gagtctatca acattgaata tcagttgaca gaatggtgcc 7800
   atgcgtggct aacatcctgc tttgattccc tctgataagc tgttctggtg gcagtaacat 7860
   gcaacaaaaa tgtgggtgtc tccaggcacg ggaaacttgg ttccattgtt atattgtcct 7920
   atgcttcgag ccatgggtct acagggtcat ccttatgaga ctcttaaata tacttagatc 7980
   ctggtaagag gcaaagaatc aacagccaaa ctgctggggc tgcaagctgc tgaagccagg 8040
   gcatgggatt aaagagattg tgcgttcaaa cctagggaag cctgtgccca tttgtcctga 8100
   ctgtctgcta acatggtaca ctgcatctca agatgtttat ctgacacaag tgtattattt 8160
   ctggcttttt gaattaatct agaaaatgaa aagatggagt tgtattttga caaaaatgtt 8220
   tgtacttttt aatgttattt ggaattttaa gttctatcag tgacttctga atccttagaa 8280
   tggcctcttt gtagaaccct gtggtataga ggagtatggc cactgcccca ctatttttat 8340
   tttcttatgt aagtttgcat atcagtcatg actagtgcct agaaagcaat gtgatggtca 8400
   ggatctcatg acattatatt tgagtttctt tcagatcatt taggatactc ttaatctcac 8460
   ttcatcaatc aaatattttt tgagtgtatg ctgtagctga aagagtatgt acgtacgtat 8520
   aagactagag agatattaag tctcagtaca cttcctgtgc catgttattc agctcactgg 8580
   tttacaaata taggttgtct tgtggttgta ggagcccact gtaacaatac tgggcagcct 8640
   tttttttttt ttttttaatt gcaacaatgc aaaagccaag aaagtataag ggtcacaagt 8700
   ctaaacaatg aattcttcaa cagggaaaac agctagcttg aaaacttgct gaaaaacaca 8760
   acttgtgttt atggcattta gtaccttcaa ataattggct ttgcagatat tggatacccc 8820
   attaaatctg acagtctcaa atttttcatc tcttcaatca ctagtcaaga aaaatataaa 8880
   aacaacaaat acttccatat ggagcatttt tcagagtttt ctaacccagt cttatttttc 8940
   tagtcagtaa acatttgtaa aaatactgtt tcactaatac ttactgttaa ctgtcttgag 9000
   agaaaagaaa aatatgagag aactattgtt tggggaagtt caagtgatct ttcaatatca 9060
   ttactaactt cttccacttt ttccagaatt tgaatattaa cgctaaaggt gtaagacttc 9120
   agatttcaaa ttaatctttc tatatttttt aaatttacag aatattatat aacccactgc 9180
   tgaaaaagaa aaaaatgatt gttttagaag ttaaagtcaa tattgatttt aaatataagt 9240
   aatgaaggca tatttccaat aactagtgat atggcatcgt tgcattttac agtatcttca 9300
   aaaatacaga atttatagaa taatttctcc tcatttaata tttttcaaaa tcaaagttat 9360
   ggtttcctca ttttactaaa atcgtattct aattcttcat tatagtaaat ctatgagcaa 9420
   ctccttactt cggttcctct gatttcaagg ccatatttta aaaaatcaaa aggcactgtg 9480
   aactattttg aagaaaacac aacattttaa tacagattga aaggacctct tctgaagcta 9540
   gaaacaatct atagttatac atcttcatta atactgtgtt accttttaaa atagtaattt 9600
   tttacatttt cctgtgtaaa cctaattgtg gtagaaattt ttaccaactc tatactcaat 9660
   caagcaaaat ttctgtatat tccctgtgga atgtacctat gtgagtttca gaaattctca 9720
   aaatacgtgt tcaaaaattt ctgcttttgc atctttggga cacctcagaa aacttattaa 9780
   caactgtgaa tatgagaaat acagaagaaa ataataagcc ctctatacat aaatgcccag 9840
   cacaattcat tgttaaaaaa caaccaaacc tcacactact gtatttcatt atctgtactg 9900
   aaagcaaatg ctttgtgact attaaatgtt gcacatcatt cattcactgt atagtaatca 9960
   ttgactaaag ccatttgtct gtgttttctt cttgtggttg tatatatcag gtaaaatatt 10020
   ttccaaagag ccatgtgtca tgtaatactg aaccactttg atattgagac attaatttgt 10080
   acccttgtta ttatctacta gtaataatgt aatactgtag aaatattgct ctaattcttt 10140
   tcaaaattgt tgcatccccc ttagaatgtt tctatttcca taaggattta ggtatgctat 10200
   tatcccttct tataccctaa gatgaagctg tttttgtgct ctttgttcat cattggccct 10260
   cattccaagc actttacgct gtctgtaatg ggatctattt ttgcactgga atatctgaga 10320
   attgcaaaac tagacaaaag tttcacaaca gatttctaag ttaaatcatt ttcattaaaa 10380
   ggaaaaaaga aaaaaaattt tgtatgtcaa taactttata tgaagtatta aaatgcatat 10440
   ttctatgttg taatataatg agtcacaaaa taaagctgtg acagttctgt tggtctacag 10500
   aaaaaaaaaa aaaaa 10515
<210> 2
   <211> 4564
   <212> DNA
   <213> Homo sapiens
<400> 2
   ggggaggcgc cgggggcgcg cgcgcgcgcg ctgggcgctg ctgggctgcg gcggcggcgg 60
   cggcggcggt ggttactatg gcggagtcgg ccggagcctc ctccttcttc ccccttgttg 120
   tcctcctgct cgccggcagc ggcgggtccg ggccccgggg ggtccaggct ctgctgtgtg 180
   cgtgcaccag ctgcctccag gccaactaca cgtgtgagac agatggggcc tgcatggttt 240
   ccattttcaa tctggatggg atggagcacc atgtgcgcac ctgcatcccc aaagtggagc 300
   tggtccctgc cgggaagccc ttctactgcc tgagctcgga ggacctgcgc aacacccact 360
   gctgctacac tgactactgc aacaggatcg acttgagggt gcccagtggt cacctcaagg 420
   agcctgagca cccgtccatg tggggcccgg tggagctggt aggcatcatc gccggcccgg 480
   tgttcctcct gttcctcatc atcatcattg ttttccttgt cattaactat catcagcgtg 540
   tctatcacaa ccgccagaga ctggacatgg aagatccctc atgtgagatg tgtctctcca 600
   aagacaagac gctccaggat cttgtctacg atctctccac ctcagggtct ggctcagggt 660
   tacccctctt tgtccagcgc acagtggccc gaaccatcgt tttacaagag attattggca 720
   agggtcggtt tggggaagta tggcggggcc gctggagggg tggtgatgtg gctgtgaaaa 780
   tattctcttc tcgtgaagaa cggtcttggt tcagggaagc agagatatac cagacggtca 840
   tgctgcgcca tgaaaacatc cttggattta ttgctgctga caataaagat aatggcacct 900
   ggacacagct gtggcttgtt tctgactatc atgagcacgg gtccctgttt gattatctga 960
   accggtacac agtgacaatt gaggggatga ttaagctggc cttgtctgct gctagtgggc 1020
   tggcacacct gcacatggag atcgtgggca cccaagggaa gcctggaatt gctcatcgag 1080
   acttaaagtc aaagaacatt ctggtgaaga aaaatggcat gtgtgccata gcagacctgg 1140
   gcctggctgt ccgtcatgat gcagtcactg acaccattga cattgccccg aatcagaggg 1200
   tggggaccaa acgatacatg gcccctgaag tacttgatga aaccattaat atgaaacact 1260
   ttgactcctt taaatgtgct gatatttatg ccctcgggct tgtatattgg gagattgctc 1320
   gaagatgcaa ttctggagga gtccatgaag aatatcagct gccatattac gacttagtgc 1380
   cctctgaccc ttccattgag gaaatgcgaa aggttgtatg tgatcagaag ctgcgtccca 1440
   acatccccaa ctggtggcag agttatgagg cactgcgggt gatggggaag atgatgcgag 1500
   agtgttggta tgccaacggc gcagcccgcc tgacggccct gcgcatcaag aagaccctct 1560
   cccagctcag cgtgcaggaa gacgtgaaga tctaactgct ccctctctcc acacggagct 1620
   cctggcagcg agaactacgc acagctgccg cgttgagcgt acgatggagg cctacctctc 1680
   gtttctgccc agccctctgt ggccaggagc cctggcccgc aagagggaca gagcccggga 1740
   gagactcgct cactcccatg ttgggtttga gacagacacc ttttctattt acctcctaat 1800
   ggcatggaga ctctgagagc gaattgtgtg gagaactcag tgccacacct cgaactggtt 1860
   gtagtgggaa gtcccgcgaa acccggtgca tctggcacgt ggccaggagc catgacaggg 1920
   gcgcttggga ggggccggag gaaccgaggt gttgccagtg ctaagctgcc ctgagggttt 1980
   ccttcgggga ccagcccaca gcacaccaag gtggcccgga agaaccagaa gtgcagcccc 2040
   tctcacaggc agctctgagc cgcgctttcc cctcctccct gggatggacg ctgccgggag 2100
   actgccagtg gagacggaat ctgccgcttt gtctgtccag ccgtgtgtgc atgtgccgag 2160
   gtgcgtcccc cgttgtgcct ggttcgtgcc atgcccttac acgtgcgtgt gagtgtgtgt 2220
   gtgtgtctgt aggtgcgcac ttacctgctt gagctttctg tgcatgtgca ggtcgggggt 2280
   gtggtcgtca tgctgtccgt gcttgctggt gcctcttttc agtagtgagc agcatctagt 2340
   ttccctggtg cccttccctg gaggtctctc cctcccccag agcccctcat gccacagtgg 2400
   tactctgtgt ctggcaggct actctgccca ccccagcatc agcacagctc tcctcctcca 2460
   tctcagactg tggaaccaaa gctggcccag ttgtccatga caaaagaggc ttttgggcca 2520
   aaatgtgagg gtggtgggtg ggatgggcag ggaaggaatc ctggtggaag tcttgggtgt 2580
   tagtgtcagc catgggaaat gagccagccc aagggcatca tcctcagcag catcgaggaa 2640
   gggccgagga atgtgaagcc agatctcggg actcagattg gaatgttaca tctgtctttc 2700
   atctcccaga tcctggaaac agcagtgtat atttttggtg gtggtgggtt tggggtgggg 2760
   aagggaaggg cgggcaagga gtggggaggg agtctggggt gggagggagg catctgcatg 2820
   ggtcttcttt tactggactg tctgatcagg gtggagggaa ggtgagaggt ttgcatccac 2880
   ttcaggagcc ctactgaagg gaacagcctg agccgaacat gttatttaac ctgagtatag 2940
   tatttaacga agcctagaag cacggctgtg ggtggtgatt tggtcagcat atcttaggta 3000
   tataataact ttgaagccat aacttttaac tggagtggtt tgatttcttt ttttaatttt 3060
   attgggaggg tttggatttt aacttttttt aatgttgtta aatattaagt ttttgtaaaa 3120
   ggaaaaccat ctctgtgatt acctctcaat ctatttgttt ttaaagaaat ccctaaaaaa 3180
   aaaaattatc caattgaacg cacatagctc aatcacactg gaaatgtttg tccttgcacc 3240
   tgagcctgtt cccactcagc agtgagagtt cctctttgcc ctgaggctca gtctctctcg 3300
   tattttgtcc ccacccccaa ttccttgagt ggtttttgct ctagggccct ttcttgcact 3360
   gtccagctgg ttgtaccctc tccaggcatt tattcaacaa atgtgggtga agtgcctgct 3420
   gggtgccagg tgctgggaat acatctgtgg acaagacatg cttgggtcct actcctggag 3480
   cactgtaaaa agagctgatt caagtaagta gatgcctgtt ttgagaccag aaggtttcat 3540
   aattggttct acgacccttt tgagcctaga attattgttc ttatataaga tcactgaaga 3600
   aagaggaacc cccacaaccc cctccacaaa gagaccaggg gcgggtgatg agacctgggg 3660
   tttagaaccc caggtgagac ctcaaatcac tgcattcatt ctgagccccc ttcctgtccc 3720
   caggggaggt gtattgtgta tgtagcctta gagcatctct gcctccaacc cagcagttct 3780
   ctgccaaagc ttgtggagga gggagagccc tgtccctgcc ctcaggctcc ccagtgctcc 3840
   tggcccttct atttatttga ctgattattg cttctttcct tgcattaaag gagatcttcc 3900
   cctaaccttt gggccaattt actggccact aatttcgttt aaataccatt gtgtcattgg 3960
   ggggaccgtc tttacccctg ctgacctccc acctatccgc cctgcagcag aaccttggcg 4020
   gtttataggt aatgatggaa cttagactcc tcttcccaga gtcacaagta gcctctggga 4080
   tctgccaaca cacgtccact cccaagccac tagcccactc cccagttggc ccttctgccc 4140
   ttaccccaca cacagtccaa ctcttccacc tctggggaag atggagcagg tctttgggaa 4200
   gctcccacac ccacctctgc cactcttaac actaagtgag agttggggag aaactgaagc 4260
   cgtgtttttg gccccccgag gctaaccctg atccatagtg ctacctgcac ctctggattc 4320
   tggattcaca gaccaagtcc aagcccgttc ttacgtcgcc ataaaggccc ccgaacggca 4380
   ttctcggtac ttctgtttgt ttttgtacat tttattagaa aggactgtaa aatagccact 4440
   tagacacttt acctcttcag tatgcaaatg taaataaatt gtaatatagg aaatcttttg 4500
   ttttaatata agaatgagcc tgtccaattt ctgctgtaca ttattaaaag ttttattcac 4560
   agag 4564
<210> 3
   <211> 3740
   <212> DNA
   <213> Homo sapiens
<400> 3
   gggacggcgg cggcgcagct cggaacccgc cagggtccag ggtccaggtt ccagcgcccg        60
   gcggcccagg caccccccga gcccagctcc acacaccgtt cctggatctc ctctccccag       120
   gcggagcgtg cccctgccca gtccagtgac cttcgcctgt tggagccctg gttaattttt       180
   gcccagtctg cctgttgtgg ggctcctccc ctttggggat ataagcccgg cctggggctg       240
   ctccgttctc tgcctggcct gaggctccct gagccgcctc cccaccatca ccatggccaa       300
   gggcttctat atttccaagt ccctgggcat cctggggatc ctcctgggcg tggcagccgt       360
   gtgcacaatc atcgcactgt cagtggtgta ctcccaggag aagaacaaga acgccaacag       420
   ctcccccgtg gcctccacca ccccgtccgc ctcagccacc accaaccccg cctcggccac       480
   caccttggac caaagtaaag cgtggaatcg ttaccgcctc cccaacacgc tgaaacccga       540
   ttcctaccgg gtgacgctga gaccgtacct cacccccaat gacaggggcc tgtacgtttt       600
   taagggctcc agcaccgtcc gtttcacctg caaggaggcc actgacgtca tcatcatcca       660
   cagcaagaag ctcaactaca ccctcagcca ggggcacagg gtggtcctgc gtggtgtggg       720
   aggctcccag ccccccgaca ttgacaagac tgagctggtg gagcccaccg agtacctggt 780
   ggtgcacctc aagggctccc tggtgaagga cagccagtat gagatggaca gcgagttcga 840
   gggggagttg gcagatgacc tggcgggctt ctaccgcagc gagtacatgg agggcaatgt 900
   cagaaaggtg gtggccacta cacagatgca ggctgcagat gcccggaagt ccttcccatg 960
   cttcgatgag ccggccatga aggccgagtt caacatcacg cttatccacc ccaaggacct 1020
   gacagccctg tccaacatgc ttcccaaagg tcccagcacc ccacttccag aagaccccaa 1080
   ctggaatgtc actgagttcc acaccacgcc caagatgtcc acgtacttgc tggccttcat 1140
   tgtcagtgag ttcgactacg tggagaagca ggcatccaat ggtgtcttga tccggatctg 1200
   ggcccggccc agtgccattg cggcgggcca cggcgattat gccctgaacg tgacgggccc 1260
   catccttaac ttctttgctg gtcattatga cacaccctac ccactcccaa aatcagacca 1320
   gattggcctg ccagacttca acgccggcgc catggagaac tggggactgg tgacctaccg 1380
   ggagaactcc ctgctgttcg accccctgtc ctcctccagc agcaacaagg agcgggtggt 1440
   cactgtgatt gctcatgagc tggcccacca gtggttcggg aacctggtga ccatagagtg 1500
   gtggaatgac ctgtggctga acgagggctt cgcctcctac gtggagtacc tgggtgctga 1560
   ctatgcggag cccacctgga acttgaaaga cctcatggtg ctgaatgatg tgtaccgcgt 1620
   gatggcagtg gatgcactgg cctcctccca cccgctgtcc acacccgcct cggagatcaa 1680
   cacgccggcc cagatcagtg agctgtttga cgccatctcc tacagcaagg gcgcctcagt 1740
   cctcaggatg ctctccagct tcctgtccga ggacgtattc aagcagggcc tggcgtccta 1800
   cctccacacc tttgcctacc agaacaccat ctacctgaac ctgtgggacc acctgcagga 1860
   ggctgtgaac aaccggtcca tccaactccc caccaccgtg cgggacatca tgaaccgctg 1920
   gaccctgcag atgggcttcc cggtcatcac ggtggatacc agcacgggga ccctttccca 1980
   ggagcacttc ctccttgacc ccgattccaa tgttacccgc ccctcagaat tcaactacgt 2040
   gtggattgtg cccatcacat ccatcagaga tggcagacag cagcaggact actggctgat 2100
   agatgtaaga gcccagaacg atctcttcag cacatcaggc aatgagtggg tcctgctgaa 2160
   cctcaatgtg acgggctatt accgggtgaa ctacgacgaa gagaactgga ggaagattca 2220
   gactcagctg cagagagacc actcggccat ccctgtcatc aatcgggcac agatcattaa 2280
   tgacgccttc aacctggcca gtgcccataa ggtccctgtc actctggcgc tgaacaacac 2340
   cctcttcctg attgaagaga gacagtacat gccctgggag gccgccctga gcagcctgag 2400
   ctacttcaag ctcatgtttg accgctccga ggtctatggc cccatgaaga actacctgaa 2460
   gaagcaggtc acacccctct tcattcactt cagaaataat accaacaact ggagggagat 2520
   cccagaaaac ctgatggacc agtacagcga ggttaatgcc atcagcaccg cctgctccaa 2580
   cggagttcca gagtgtgagg agatggtctc tggccttttc aagcagtgga tggagaaccc 2640
   caataataac ccgatccacc ccaacctgcg gtccaccgtc tactgcaacg ctatcgccca 2700
   gggcggggag gaggagtggg acttcgcctg ggagcagttc cgaaatgcca cactggtcaa 2760
   tgaggctgac aagctccggg cagccctggc ctgcagcaaa gagttgtgga tcctgaacag 2820
   gtacctgagc tacaccctga acccggactt aatccggaag caggacgcca cctctaccat 2880
   catcagcatt accaacaacg tcattgggca aggtctggtc tgggactttg tccagagcaa 2940
   ctggaagaag ctttttaacg attatggtgg tggctcgttc tccttctcca acctcatcca 3000
   ggcagtgaca cgacgattct ccaccgagta tgagctgcag cagctggagc agttcaagaa 3060
   ggacaacgag gaaacaggct tcggctcagg cacccgggcc ctggagcaag ccctggagaa 3120
   gacgaaagcc aacatcaagt gggtgaagga gaacaaggag gtggtgctcc agtggttcac 3180
   agaaaacagc aaatagtccc cagcccttga agtcacccgg cccccatgca aggtgcccac 3240
   atgtgtccat cccagcggct ggtgcagggc ctccattcct ggagcccgag gcaccagtgt 3300
   cctcccctca aggacaaagt ctccagccca cgttctctct gcctgtgagc cagtctagtt 3360
   cctgatgacc caggctgcct gagcacctcc cagcccctgc ccctcatgcc aaccccgccc 3420
   taggcctggc atggcacctg tcgcccagtg ccctggggct gatctcaggg aagcccagct 3480
   ccagggccag atgagcagaa gctctcgatg gacaatgaac ggccttgctg ggggccgccc 3540
   tgtaccctct ttcacctttc cctaaagacc ctaaatctga ggaatcaaca gggcagcaga 3600
   tctgtatatt tttttctaag agaaaatgta aataaaggat ttctagatga aaaaaaaaaa 3660
   aaaaaaaaaa aaaaaaaaaa aaaaaaaaaa aaaaaaaaaa aaaaaaaaaa aaaaaaaaaa 3720
   aaaaaaaaaa aaaaaaaaaa 3740
<210> 4
   <211> 3033
   <212> DNA
   <213> Homo sapiens
<400> 4
   aacacaactg gcacatctct tttctcatct cttgaaaaaa accaacagag aaaaaagtac      60
   cttgagaata aaggtaatga ttaatctgtc aggcacaaaa gggattgttt tggggatttc     120
   gggttctaag tcgcagattc aaacaaatag cagcgaacag ggaatgacag ttccaccaga     180
   agacgattaa gccacagcct ctaattggaa cggcatttgt acagtcagag actcttacca     240
   gacatctcca ggaatctgtg agccattgtc aaaacgtcca ttttcatctg gctgtgaaag     300
   tgaggaccac aacaggtagg tattggtaga aacaggagtc ctcagagaag ccccaagatg     360
   cagcctgagg gagcagaaaa gggaaaaagc ttcaagcaga gactggtctt gaagagcagc     420
   ttagcgaaag aaaccctctc tgagttcttg ggcacgttca tcttgattgt ccttggatgt     480
   ggctgtgttg cccaagctat tctcagtcga ggacgttttg gaggggtcat cactatcaat 540
   gttggatttt caatggcagt tgcaatggcc atttatgtgg ctggcggtgt ctctggtggt 600
   cacatcaacc cagctgtgtc tttagcaatg tgtctctttg gacggatgaa atggttcaaa 660
   ttgccatttt atgtgggagc ccagttcttg ggagcctttg tgggggctgc aaccgtcttt 720
   ggcatttact atgatggact tatgtccttt gctggtggaa aactgctgat cgtgggagaa 780
   aatgcaacag cacacatttt tgcaacatac ccagctccgt atctatctct ggcgaacgca 840
   tttgcagatc aagtggtggc caccatgata ctcctcataa tcgtctttgc catctttgac 900
   tccagaaact tgggagcccc cagaggccta gagcccattg ccatcggcct cctgattatt 960
   gtcattgctt cctccctggg actgaacagt ggctgtgcca tgaacccagc tcgagacctg 1020
   agtcccagac ttttcactgc cttggcaggc tgggggtttg aagtcttcag agctggaaac 1080
   aacttctggt ggattcctgt agtgggccct ttggttggtg ctgtcattgg aggcctcatc 1140
   tatgttcttg tcattgaaat ccaccatcca gagcctgact cagtctttaa gacagaacaa 1200
   tctgaggaca aaccagagaa atatgaactc agtgtcatca tgtagtggca tgctcagctc 1260
   tggatttgca gtcagtttgg gattctcttc agaaagatgg catctaagtg tctgtgttct 1320
   tgtaagcctg aggtggaatc cacccagttt tgtctgctag ccatatggga catctaattg 1380
   gaaaagcatc tgcataaaag tttggaaaca atgaccactt ctctaccatt gtcccccacc 1440
   cccacccccc agaataacgc tgactgtccc ctgaaacagc cttctctcct gccctgttta 1500
   tttcatcctc gatgggaatt cttgctaggt aagcactaat aactcggcat cttgacgata 1560
   gtcccatttg ggtggtttca gctgcactat ctgtatgaaa tggtgtcacc aaaacccttt 1620
   tcttcagtat cgacaaagat tacattctga gtaccaacca aaccctaaat tgaaagacaa 1680
   aactatggtt tcagtcaaca tattcatgaa ttagggagct aatgggttaa gcttccagtt 1740
   cccgctatgc tactggattt gtataaatac tgatattctc caaacctagt ggtgtaggga 1800
   gcaagagaat gcagctggaa ggcacaaggg gaggacattg tggcattcag aaactgcagg 1860
   agacaagatg aatttgagaa gccaaatgga atttttaatg gaaaccattt atcagattaa 1920
   tctcttgctc tcctgcattt tagaggacac caattaattt cctggtcttt agtatataat 1980
   aacctaaaat accattgtaa cctcagtcat gaaaaataca tcactctgtc tttttagctc 2040
   aaatgtattt tcctaattgc ccacttgaga acagacattt gacaagttat atcaacgact 2100
   gtgcttgtcc attattttac acatgcccta gaagccaaaa ctgaaagcca ctggatcctg 2160
   gtctagctga atcttcagag tgggaggtct ccaaaaagat attaccttat tgggcttaac 2220
   aattcacaag gcactttcac acccattatc taatttaatc ctcataatga ctatgtgagg 2280
   caaatgccac attgcccatt tttcagataa agaaacaaaa tcttagggaa gataagttga 2340
   gttgtccaag agcacactga aagttgaatg ttatctaatg cattcctcta cctttcagaa 2400
   gatcagtagc tggctgagaa tctttgccaa atcttccttg ctagccagaa gtggaattgg     2460
   cagcttctag aatatgtaca cctctggaca aaatgttcct caatcttaag atacaaagac     2520
   cctcattgtc tgggtctatt cccacactta ctgagtacag atgaaggaaa gtggtagcaa     2580
   tttaatcata actttcattt gctgaaaaac attatgagaa ggcctccctt cctaagccac     2640
   ctctggtctt gctaagtctt gatcttgctt cctgccagca ccaaacatta cattcagggg     2700
   atttcctctg gctcagtctt ttccccttga agttctctaa tagatgttac ttttgacaaa     2760
   agatcgccta tgagttacaa gcaccagggg atgctctaca tcaagggatg caccttcagt     2820
   caaactgtca aaaagcccag aattcccaaa ggcattaggt ttcccaactg ctttgtgctg     2880
   atatcagaac agcagaaatt aaatgtgaaa tgtttctgat gacttatgtt ctacaatcta     2940
   tggacatacg ggattttttt ttcttgcttt gaagctacct ggatatttcc tatttgaaat     3000
   aaaattgttc ggtcattgtt gaaaaaaaaa aaa                                  3033
<210> 5
   <211> 979
   <212> DNA
   <213> Homo sapiens
<400> 5
   gagttaggtg acgctgcggg gcgggcggac agactgcggg acggacggtg gacgctggga      60
   cgcgtttgta gctccggccc cgccgttccg acccccgccg ccgtcgccgc catgacgggg     120
   ctagcactgc tctactccgg ggtcttcgtg gccttctggg cctgcgcgct ggccgtgggt     180
   tcctgacgga gacttcgccc ttcatgtggt ccaacctggg cattggccta gctatctccc     240
   tgtctgtggt tggggcagcc tggggcatct atattaccgg ctcctccatc attggtggag     300
   gagtgaaggc ccccaggatc aagaccaaga acctggtcag catcatcttc tgtgaggctg     360
   tggccatcta cggcatcatc atggcaattg tcattagcaa catggctgag cctttcagtg     420
   ccacagaccc caaggccatc ggccatcgga actaccatgc aggctactcc atgtttgggg     480
   ctggcctcac cgtaggcctg tctaacctct tctgtggagt ctgcgtgggc atcgtgggca     540
   gtggggctgc cctggccgat gctcagaacc ccagcctctt tgtaaagatt ctcatcgtgg     600
   agatctttgg cagcgccatt ggcctctttg gggtcatcgt cgcaattctt cagacctcca     660
   gagtgaagat gggtgactag atgatatgtg tgggtggggc cgtgcctcac ttttatttat     720
   tgctggtttt cctgggacag ctggagctgt gtcccttagc ctttcagagg cttggtgttc     780
   agggccctcc ctgcactccc ctcttgctgc gtgttgattt ggaggcactg cagtccaggc     840
   cgagtcctca gtgcggggag caggctgctg ctgctgactc tgtgcagctg cgcacctgtg     900
   tcccccacct ccaccctcaa cccatcttcc tagtgtttgt gaaataaact tggtatttgt     960
   ctgggtcagt gcaaaaaaa                                                  979
<210> 6
   <211> 1727
   <212> DNA
   <213> Homo sapiens
<400> 6
   gcgcaactcg tttgcagcgg cgcagcccag acgcgcctgc agctggggct caccccaacc 60
   tcgctgccag ccgagaactc caagatggga ggcaagctca gcaagaagaa gaagggctac 120
   aatgtgaacg acgagaaagc caaggagaaa gacaagaagg ccgagggcgc ggcgacggaa 180
   gaggagggga ccccgaagga gagtgagccc caggcggccg cagagcccgc cgaggccaag 240
   gagggcaagg agaagcccga ccaggacgcc gagggcaagg ccgaggagaa ggagggcgag 300
   aaggacgcgg cggctgccaa ggaggaggcc ccgaaggcgg agcccgagaa gacggagggc 360
   gcggcagagg ccaaggctga gcccccgaag gcgcccgagc aggagcaggc ggcccccggc 420
   cccgctgcgg gcggcgaggc ccccaaagct gctgaggccg ccgcggcccc ggccgagagc 480
   gcggcccctg ccgccgggga ggagcccagc aaggaggaag gggaacccaa aaagactgag 540
   gcgcccgcag ctcctgccgc ccaggagacc aaaagtgacg gggccccagc ttcagactca 600
   aaacccggca gctcggaggc tgccccctct tccaaggaga cccccgcagc cacggaagcg 660
   cctagttcca cacccaaggc ccagggcccc gcagcctctg cagaagagcc caagccggtg 720
   gaggccccgg cagctaattc cgaccaaacc gtaaccgtga aagagtgaca aggacagcct 780
   ataggaaaaa caataccact taaaacaatc tcctctctct ctctctctct ctctctctat 840
   ctctctctct atctcctctc tctctctcct ctcctatctc tcctctctct ctctcctata 900
   ctaacttgtt tcaaattgga agtaatgata tgtattgccc aaggaaaaat acaggatgtt 960
   gtcccatcaa gggagggagg gggtgggaga atccaaatag tatttttgtg gggaaatatc 1020
   taatatacct tcagtcaact ttaccaagaa gtcctggatt tccaagatcc gcgtctgaaa 1080
   gtgcagtaca tcgtttgtac ctgaaactgc cgccacatgc actcctccac cgctgagagt 1140
   tgaatagctt ttcttctgca atgggagttg ggagtgatgc gtttgattct gcccacaggg 1200
   cctgtgccaa ggcaatcaga tctttatgag agcagtattt tctgtgtttt ctttttaatt 1260
   tacagccttt cttattttga tattttttta atgttgtgga tgaatgccag ctttcagaca 1320
   gagcccactt agcttgtcca catggatctc aatgccaatc ctccattctt cctctccaga 1380
   tatttttggg agtgacaaac attctctcat cctacttagc ctacctagat ttctcatgac 1440
   gagttaatgc atgtccgtgg ttgggtgcac ctgtagttct gtttattggt cagtggaaat 1500
   gaaaaaaaaa aaaaaaaaaa gtctgcgttc attgcagttc cagtttctct tccattctgt 1560
   gtcacagaca ccaacacacc actcattgga aaatggaaaa aaaaaacaaa aaaaaaacaa 1620
   aaaaatgtac aatggatgca ttgaaattat atgtaattgt ataaatggtg caacagtaat 1680
   aaagttaaac aattaaaaag aagtaataaa gacaaaaaaa aaaaaaa 1727
<210> 7
   <211> 2852
   <212> DNA
   <213> Homo sapiens
<400> 7
   caaggctggg accagaaacc aggactgttg actgcagccc ggtattcatt ctttccatag      60
   cccacagggc tgtcaaagac cccagggcct agtcagaggc tcctccttcc tggagagttc     120
   ctggcacaga agttgaagct cagcacagcc ccctaacccc caactctctc tgcaaggcct     180
   caggggtcag aacactggtg gagcagatcc tttagcctct ggattttagg gccatggtag     240
   agggggtgtt gccctaaatt ccagccctgg tctcagccca acaccctcca agaagaaatt     300
   agaggggcca tggccaggct gtgctagccg ttgcttctga gcagattaca agaagggact     360
   aagacaagga ctcctttgtg gaggtcctgg cttagggagt caagtgacgg cggctcagca     420
   ctcacgtggg cagtgccagc ctctaagagt gggcaggggc actggccaca gagtcccagg     480
   gagtcccacc agcctagtcg ccagaccttc tgtgggatca tcggacccac ctggaacccc     540
   acctgctggc cctcacggaa gaacaacagc tgatgtttga gaaactgact ctgtattgcg     600
   acagctacat ccagctcatc cccatttcct tcgtgctggg cttctacgtg acgctggtcg     660
   tgacccgctg gtggaaccag tacgagaacc tgccgtggcc cgaccgcctc atgagcctgg     720
   tgtcgggctt cgtcgaaggc aaggacgagc aaggccggct gctgcggcgc acgctcatcc     780
   gctacgccaa cctgggcaac gtgctcatcc tgcgcagcgt cagcaccgca gtctacaagc     840
   gcttccccag cgcccagcac ctggtgcaag caggctttat gactccggca gaacacaagc     900
   agttggagaa actgagccta ccacacaaca tgttctgggt gccctgggtg tggtttgcca     960
   acctgtcaat gaaggcgtgg cttggaggtc gaatccggga ccctatcctg ctccagagcc    1020
   tgctgaacga gatgaacacc ttgcgtactc agtgtggaca cctgtatgcc tacgactgga    1080
   ttagtatccc actggtgtat acacaggtgg tgactgtggc ggtgtacagc ttcttcctga    1140
   cttgtctagt tgggcggcag tttctgaacc cagccaaggc ctaccctggc catgagctgg    1200
   acctcgttgt gcccgtcttc acgttcctgc agttcttctt ctatgttggc tggctgaagg    1260
   tggcagagca gctcatcaac ccctttggag aggatgatga tgattttgag accaactgga    1320
   ttgtcgacag gaatttgcag gtgtccctgt tggctgtgga tgagatgcac caggacctgc    1380
   ctcggatgga gccggacatg tactggaata agcccgagcc acagcccccc tacacagctg    1440
   cttccgccca gttccgtcga gcctccttta tgggctccac cttcaacatc agcctgaaca    1500
   aagaggagat ggagttccag cccaatcagg aggacgagga ggatgctcac gctggcatca    1560
   ttggccgctt cctaggcctg cagtcccatg atcaccatcc tcccagggca aactcaagga    1620
   ccaaactact gtggcccaag agggaatccc ttctccacga gggcctgccc aaaaaccaca 1680
   aggcagccaa acagaacgtt aggggccagg aagacaacaa ggcctggaag cttaaggctg 1740
   tggacgcctt caagtctgcc ccactgtatc agaggccagg ctactacagt gccccacaga 1800
   cgcccctcag ccccactccc atgttcttcc ccctagaacc atcagcgccg tcaaagcttc 1860
   acagtgtcac aggcatagac accaaagaca aaagcttaaa gactgtgagt tctggggcca 1920
   agaaaagttt tgaattgctc tcagagagcg atggggcctt gatggagcac ccagaagtat 1980
   ctcaagtgag gaggaaaact gtggagttta acctgacgga tatgccagag atccccgaaa 2040
   atcacctcaa agaacctttg gaacaatcac caaccaacat acacactaca ctcaaagatc 2100
   acatggatcc ttattgggcc ttggaaaaca ggtctgtcct ccacctgaac caggggcact 2160
   gcattgccct gtgccccacc ccagcttccc ttgctctgag cctacccttc ctccacaatt 2220
   tcctagggtt ccatcactgc cagagcacac tggacctacg cccagcactg gcttggggta 2280
   tatacttggc caccttcaca gggatcctag ggaagtgttc gggacctttt ctcacttcac 2340
   cctggtatca cccggaagac ttcttgggac caggtgaagg aagatgaggt tgtgctgacc 2400
   agaatgctgc tggagaactg ccccagggct gacaggccag gcttagctga gcagatgtta 2460
   tcactggccc caacttactt tgagcaaggg tggctgaccc aaaaccatga ggtggcagtc 2520
   agctggatga cagatgaaca cttcccccat aactatttag ggtagtaccc aagcactaca 2580
   ggaaagggtg gcaggaactg cctcactcct aggaactggt agatggtgag gttgagggtg 2640
   tccagcgccc ttaggtcatt ttctcactgc ctgggaacct caccaaaata cttcttgctt 2700
   ccttggggtc agcccaaagc tgtcacaaaa tcagatattt ccctttattc cagatttcct 2760
   ggacactttc acccaattat aaacacccca cttcagcccc aatcacgtgg gaggaagtgt 2820
   aacttccctt ttaaaaaaaa aaaaaaaaaa aa 2852
<210> 8
   <211> 1238
   <212> DNA
   <213> Homo sapiens
<400> 8
   agactaggat ccctggaaaa tggagaagct gtgctaatag agggggccca gaaatcccca      60
   ctctagaatg ctgtagaatg ttgggagaca cccaggatgt gagccaggga ctttctggaa     120
   gtgtttgttc tggccccacc cgaccccagg cagtccccag ctgtctgcac agtcggatgg     180
   ggagggggct tgcacagagt tggagccaga ggagagagct ggctcatccc ctacggtagg     240
   atggggaaac ctcacagacc acattgtcac ccggcctcag ctctccgccc cggcgctcag     300
   agggtaactc tcacccacct cgtccgcttc tctgaaccag agtgacccag gctgcgctcc     360
   gccccgctct cctaccccga gttggcacgg aggcccggca gccatggcgg tggaaggagg     420
   aatgaaatgt gtgaagttct tgctctacgt cctcctgctg gccttttgcg cctgtgcagt     480
   gggactgatt gccgtgggtg tcggggcaca gcttgtcctg agtcagacca taatccaggg     540
   ggctacccct ggctctctgt tgccagtggt catcatcgca gtgggtgtct tcctcttcct     600
   ggtggctttt gtgggctgct gcggggcctg caaggagaac tattgtctta tgatcacgtt     660
   tgccatcttt ctgtctctta tcatgttggt ggaggtggcc gcagccattg ctggctatgt     720
   gtttagagat aaggtgatgt cagagtttaa taacaacttc cggcagcaga tggagaatta     780
   cccgaaaaac aaccacactg cttcgatcct ggacaggatg caggcagatt ttaagtgctg     840
   tggggctgct aactacacag attgggagaa aatcccttcc atgtcgaaga accgagtccc     900
   cgactcctgc tgcattaatg ttactgtggg ctgtgggatt aatttcaacg agaaggcgat     960
   ccataaggag ggctgtgtgg agaagattgg gggctggctg aggaaaaatg tgctggtggt    1020
   agctgcagca gcccttggaa ttgcttttgt cgaggttttg ggaattgtct ttgcctgctg    1080
   cctcgtgaag agtatcagaa gtggctacga ggtgatgtag gggtctggtc tcctcagcct     1140
   cctcatctgg gggagtggaa tagtatcctc caggtttttc aattaaacgg attatttttt    1200
   cagaccgaaa agagatggtc tgagtttgtc ttagagtg                            1238
<210> 9
   <211> 1790
   <212> DNA
   <213> Homo sapiens
<400> 9
   taattacaaa aactaatgac taagagagag gtggctagag ctgaggcccc tgagtcaggc 60
   tgtgggtggg atcatctcca gtacaggaag tgagactttc atttcctcct ttccaagaga 120
   gggctgaggg agcagggttg agcaactggt gcagacagcc tagctggact ttgggtgagg 180
   cggttcagcc atgaggctgg ctgtgctttt ctcgggggcc ctgctggggc tactggcaga 240
   gagcactgga acaaccagcc acaggactac caagagccac aaaaccacca ctcacaggac 300
   aaccaccaca ggcaccacca gccacggacc cacgactgcc actcacaacc ccaccaccac 360
   cagccatgga aacgtcacag ttcatccaac aagcaatagc actgccacca gccagggacc 420
   ctcaactgcc actcacagtc ctgccaccac tagtcatgga aatgccacgg ttcatccaac 480
   aagcaacagc actgccacca gcccaggatt caccagttct gcccacccag aaccacctcc 540
   accctctccg agtcctagcc caacctccaa ggagaccatt ggagactaca cgtggaccaa 600
   tggttcccag ccctgtgtcc acctccaagc ccagattcag attcgagtca tgtacacaac 660
   ccagggtgga ggagaggcct ggggcatctc tgtactgaac cccaacaaaa ccaaggtcca 720
   gggaagctgt gagggtgccc atccccacct gcttctctca ttcccctatg gacacctcag 780
   ctttggattc atgcaggacc tccagcagaa ggttgtctac ctgagctaca tggcggtgga 840
   gtacaatgtg tccttccccc acgcagcaca gtggacattc tcggctcaga atgcatccct 900
   tcgagatctc caagcacccc tggggcagag cttcagttgc agcaactcga gcatcattct 960
   ttcaccagct gtccacctcg acctgctctc cctgaggctc caggctgctc agctgcccca 1020
   cacaggggtc tttgggcaaa gtttctcctg ccccagtgac cggtccatct tgctgcctct 1080
   catcatcggc ctgatccttc ttggcctcct cgccctggtg cttattgctt tctgcatcat 1140
   ccggagacgc ccatccgcct accaggccct ctgagcattt gcttcaaacc ccagggcact 1200
   gagggggttg gggtgtggtg ggggggtacc cttatttcct cgacacgcaa ctggctcaaa 1260
   gacaatgtta ttttccttcc ctttcttgaa gaacaaaaag aaagccgggc atgacggctc 1320
   atgcctgtaa tcccagcact ttgggaggct gaggcaggtg gatcactgga ggtcaggagt 1380
   ttgagaccag cctggccaac atggtgaaac cctgtctcta ctaaaaatac aattagccag 1440
   gtgtggcggc gtaatcccag ctggcctgta atcccagcta cttgggaggc tgaggcagaa 1500
   ctgcttgaac ccaggaggtg gaggttgcag tgagccgtca tcgcgccact aagccaagat 1560
   cgcgccactg cactccagcc tgggcgacag agccagactg tctcaaataa ataaatatga 1620
   gataatgcag tcgggagaag ggagggagag aattttatta aatgtgacga actgcccccc 1680
   cccccccccc agcaggagag cagcaaaatt tatgcaaatc tttgacgggg ttttccttgt 1740
   cctgccagga ttaaaagcca tgagtttctt gtcaaaaaaa aaaaaaaaaa 1790
<210> 10
   <211> 6017
   <212> DNA
   <213> Homo sapiens
<400> 10
   gggcggggct cgggccggtc cgcccgcgcg caggtgagtg agccagggcg gagcgcagct      60
   gcgccgggct tgggcgcctg gggccgccgc tccccaccgt cgttttcccc accgaggccg     120
   aggcgtcccg gagtcatggc cggcctgaac tgcggggtct ctatcgcact gctaggggtt     180
   ctgctgctgg gtgcggcgcg cctgccgcgc ggggcagaag cttttgagat tgctctgcca     240
   cgagaaagca acattacagt tctcataaag ctggggaccc cgactctgct ggcaaaaccc     300
   tgttacatcg tcatttctaa aagacatata accatgttgt ccatcaagtc tggagaaaga     360
   atagtcttta cctttagctg ccagagtcct gagaatcact ttgtcataga gatccagaaa     420
   aatattgact gtatgtcagg cccatgtcct tttggggagg ttcagcttca gccctcgaca     480
   tcgttgttgc ctaccctcaa cagaactttc atctgggatg tcaaagctca taagagcatc     540
   ggtttagagc tgcagttttc catccctcgc ctgaggcaga tcggtccggg tgagagctgc     600
   ccagacggag tcactcactc catcagcggc cgaatcgatg ccaccgtggt caggatcgga     660
   accttctgca gcaatggcac tgtgtcccgg atcaagatgc aagaaggagt gaaaatggcc     720
   ttacacctcc catggttcca ccccagaaat gtctccggct tcagcattgc aaaccgctca 780
   tctataaaac gtctgtgcat catcgagtct gtgtttgagg gtgaaggctc agcaaccctg 840
   atgtctgcca actacccaga aggcttccct gaggatgagc tcatgacgtg gcagtttgtc 900
   gttcctgcac acctgcgggc cagcgtctcc ttcctcaact tcaacctctc caactgtgag 960
   aggaaggagg agcgggttga atactacatc ccgggctcca ccaccaaccc cgaggtgttc 1020
   aagctggagg acaagcagcc tgggaacatg gcggggaact tcaacctctc tctgcaaggc 1080
   tgtgaccaag atgcccaaag tccagggatc ctccggctgc agttccaagt tttggtccaa 1140
   catccacaaa atgaaagcaa taaaatctac gtggttgact tgagtaatga gcgagccatg 1200
   tcactcacca tcgagccacg gcccgtcaaa cagagccgca agtttgtccc tggctgtttc 1260
   gtgtgtctag aatctcggac ctgcagtagc aacctcaccc tgacatctgg ctccaaacac 1320
   aaaatctcct tcctttgtga tgatctgaca cgtctgtgga tgaatgtgga aaaaaccata 1380
   agctgcacag accaccggta ctgccaaagg aaatcctact cactccaggt gcccagtgac 1440
   atcctccacc tgcctgtgga gctgcatgac ttctcctgga agctgctggt gcccaaggac 1500
   aggctcagcc tggtgctggt gccagcccag aagctgcagc agcatacaca cgagaagccc 1560
   tgcaacacca gcttcagcta cctcgtggcc agtgccatac ccagccagga cctgtacttc 1620
   ggctccttct gcccgggagg ctctatcaag cagatccagg tgaagcagaa catctcggtg 1680
   acccttcgca cctttgcccc cagcttccaa caagaggcct ccaggcaggg tctgacggtg 1740
   tcctttatac cttatttcaa agaggaaggc gttttcacgg tgacccctga cacaaaaagc 1800
   aaggtctacc tgaggacccc caactgggac cggggcctgc catccctcac ctctgtgtcc 1860
   tggaacatca gcgtgcccag agaccaggtg gcctgcctga ctttctttaa ggagcggagc 1920
   ggcgtggtct gccagacagg gcgcgcattc atgatcatcc aggagcagcg gacccgggct 1980
   gaggagatct tcagcctgga cgaggatgtg ctccccaagc caagcttcca ccatcacagc 2040
   ttctgggtca acatctctaa ctgcagcccc acgagcggca agcagctaga cctgctcttc 2100
   tcggtgacac ttaccccaag gactgtggac ttgactgtca tcctcatcgc agcggtggga 2160
   ggtggagtct tactgctgtc tgccctcggg ctcatcattt gctgtgtgaa aaagaagaaa 2220
   aagaagacaa acaagggccc cgctgtgggt atctacaatg acaacatcaa tactgagatg 2280
   ccgaggcagc caaaaaagtt tcagaaaggg cgaaaggaca atgactccca tgtgtatgca 2340
   gtcatcgagg acaccatggt atatgggcat ctgctacagg attccagcgg ctccttcctg 2400
   cagccagagg tggacaccta ccggccgttc cagggcacca tgggggtctg tcctccctcc 2460
   ccacccacca tatgctccag ggccccaact gcaaagttgg ccactgagga gccacctcct 2520
   cgctcccctc ctgagtctga gagtgaaccg tacaccttct cccatcccaa caatggggat 2580
   gtaagcagca aggacacaga cattccctta ctgaacactc aggagcccat ggagccagca 2640
   gaataacttg atccattcca gacgctttgc tgagtttcat aaagcagggc actgagacac 2700
   ccgtccgtgt tcctaaccag aaatcctaaa gaagaggaat tatacagaag gaacagcagg 2760
   aggttttcct ggacaccgcc aacttcacat tgctcagtgg actcattcta agggcaagac 2820
   attgaaaatg atgaattcca atctggatac agtcatgaca gctcatgtgc tcctcaactt 2880
   aggctgtgcg gttagccagc ctgtaatgag aggagagagg cctgagtcac ctagcatagg 2940
   gttgcagcaa gccctggatt cagagtgtta aacagaggct tgccctcttc aggacaacag 3000
   ttccaattcc aaggagccta cctgaggtcc ctactctcac tggggtcccc aggatgaaaa 3060
   cgacaatgtg cctttttatt attatttatt tggtggtcct gtgttattta agagatcaaa 3120
   tgtataacca cctagctctt ttcacctgac ttagtaataa ctcatactaa ctggtttgga 3180
   tgcctgggtt gtgacttcta ctgaccgcta gataaacgtg tgcctgtccc ccaggtggtg 3240
   ggaataattt acaatctgtc caaccagaaa agaatgtgtg tgtttgagca gcattgacac 3300
   atatctgctt tgataagaga cttcctgatt ctctaggtcg gttcgtggtt atcccattgt 3360
   ggaaattcat cttgaatccc attgtcctat agtcctagca ataagagaaa tttcctcaag 3420
   tttccatgtg cggttctcct agctgcagca atactttgac atttaaagag aaatttagag 3480
   aatattctca tcctctaaaa atgtttaaat atataccaaa cagtggcccc ctgcattagt 3540
   tttctgttgc cactgcaacc tattacttgg tagcttaaaa acaacacatt agcttatagt 3600
   cctggggatc agaattccaa aatggatgtc cctgaatgaa aatcaaggtg tcagcagagc 3660
   tgtgctcctt ctgaaggctc tagggagaag ccggttcctt gccatttcaa gcttctagag 3720
   gctggctgca ttcccaggct ccagtggctg gtcaagcttt tctcacatgg catcactgtg 3780
   acactggccc tcccacttcc ctctttgact tacaaagccc accaggaaga tccaggataa 3840
   tctctccatc taaagttcct tcatcatcct ggaagagcct tttgccatgc aagacaacat 3900
   agccacaggt ggggattagg accagaacat ctttggggtg ctgttattct gcctaccaca 3960
   ccttcctgcc actgactccc acaggagagg ctacaaaatg atctggcgca cagggatgtt 4020
   ttgtttagct tgcggactct aacacttaaa aaaaaaccca gatcagaaga tctggccatg 4080
   ctggggctca cattctcacc tagcaacaac tggctggagc tgggcaccag ctctgccttt 4140
   agaaggggtg tccacttcac caggtcacca cagcccacac tacgccctat cacttcccac 4200
   aatgaggctg agtgtttgtt tctactgatc aatgcccctg caggttgcat ttattgtaat 4260
   gaaaaagaaa gactgggatt aatctctaat caggtgagta gaccatgaga ccaatgtgtg 4320
   ctcacattac cctttttctt ttttttcttt ttctttttct tttttttttt aatgtgagac 4380
   aggatctcat tctgttgcct aggctggagt gcagtggcgc aatctcggct cactgcaacc 4440
   tctgcctcct gggctcaagc aattctccca cctcagcctc ccaaatagct gggatcactg 4500
   gcacaaacca ccatgcccag ctaattttgt attttttgta gagacagggt ttcaccatgt 4560
   tgcccaggct ggtctcaacc tcctgggctc aagcaatcct cctgcctcgg cctcccaaag 4620
   tgctgggatt acagatgtga gccaccgcat ccagccccac accctcattt ataccaatta 4680
   cctgcccagt aactgtggac ttttgcttcc tcacccctgc tctgatctgg aaggagaggg 4740
   attatgttat agcttgtcag cacagtccca agttcaatat ttctgcggca aaaacttcct 4800
   tcaaaaaata aatgtacttc attgtattca atgaattcac cttggaaatg caccgcctca 4860
   acttgttcac atggcataaa tgaaaggaat tttatagtct cctaaatggc gtgtactgca 4920
   agacctcttg aacactttcc agaggatagg atatttaagt catgcccttg gcgttgccta 4980
   tggcaccttt cccttctgaa agtctggttc ctgcccagtg acccttggcc ttgtgagccg 5040
   agatgctgac cctgcataaa gggccaaagg agggctgcgg cttccttccc tcactgaaga 5100
   gcccttattt gaattcactg tgtggagccc tagccctcca ttctcgacat tccccaacct 5160
   cccagcccct tccaagcagg actaggtgcc ctgcattcca cccaaggtgg gattggcctt 5220
   ccttaggctg gctacttgtc accatcaccg acatcactgt tgcctgcaag gacaccacgt 5280
   ggccattttc cttcaactga gggctcaaaa ctcctggaca agttgctggc tcctgagacc 5340
   agtatttcct ggagctgtgc ctcagtgaag gggcccagcc tgaggaaccc tggctctttt 5400
   ctttaaagcc caggccccac ttacgtaaaa catttcaggg tcactggaaa cagtgaagtg 5460
   ccatttgttg aagcctactg catgccagcc cactgctcat ccacgtggtc tgccatgcct 5520
   acgaggaagg ccagcgcatg caggactggt ctctaatgct gtggtcattg cacagaaggg 5580
   aaaggtctca aggaagagtc aactggaaca agcacaagcc caccggacat ggccttggta 5640
   aaggttagca gactggtgtg tgtggatctg cagtgcttca ctggaaataa tttattcatt 5700
   gcagatactt tttaggtggc attttattca tttcctgtgc tttaaataaa caaatgtacc 5760
   aaaaaacaag tatcaagctg tttaagtgct tcggctactt gtcccctggt tcagtagagg 5820
   ccccggtttc ccagttgttg actgtgacag gctcagcatg ggctcagcag atgctgtctt 5880
   aatttgtgga tgatacagaa agccaggctt tgggatacaa gttctttcct cttcatttga 5940
   tgccgtgcac tgtgtgaagc agatgttttt gtccggaaat aaaaataata gtcttggagt 6000
   ctcgccaaaa aaaaaaa 6017
<210> 11
   <211> 6651
   <212> DNA
   <213> Homo sapiens
<400> 11
   ataacacccg gccccgccgg gcggccgcgg gtgggtagag aacatggact tcccgtgcct        60
   ctggctaggg ctgttgctgc ctttggtagc tgcgctggat ttcaactacc accgccagga       120
   agggatggaa gcgtttttga agactgttgc ccaaaactac agttctgtca ctcacttaca 180
   cagtattggg aaatctgtga aaggtagaaa cctgtgggtt cttgttgtgg ggcggtttcc 240
   aaaggaacac agaattggga ttccagagtt caaatacgtg gcaaatatgc atggagatga 300
   gactgttggg cgggagctgc tgctccatct gattgactat ctcgtaacca gtgatggcaa 360
   agaccctgaa atcacaaatc tgatcaatag tacccggata cacatcatgc cttccatgaa 420
   cccagatgga tttgaagccg tcaaaaagcc tgactgttat tacagcatcg gaagggaaaa 480
   ttataaccag tatgacttga atcgaaattt ccccgatgct tttgaatata ataatgtctc 540
   aaggcagcct gaaactgtgg cagtcatgaa gtggctgaaa acagagacgt ttgtcctctc 600
   tgcaaacctc catggtggtg ccctcgtggc cagttaccca tttgataatg gtgttcaagc 660
   aactggggca ttatactccc gaagcttaac gcctgatgat gatgtttttc aatatcttgc 720
   acatacctat gcttcaagaa atcccaacat gaagaaagga gacgagtgta aaaacaaaat 780
   gaactttcct aatggtgtta caaatggata ctcttggtat ccactccaag gtggaatgca 840
   agattacaac tacatctggg cccagtgttt tgaaattacg ttggagctgt catgctgtaa 900
   atatcctcgt gaggagaagc ttccatcctt ttggaataat aacaaagcct cattaattga 960
   atatataaag caggtgcacc taggtgtaaa gggtcaagtt tttgatcaga atggaaatcc 1020
   attacccaat gtaattgtgg aagtccaaga cagaaaacat atctgcccct atagaaccaa 1080
   caaatatgga gagtattatc tccttctctt gcctgggtct tatataataa atgttacagt 1140
   ccctggacat gatccacaca tcacaaaggt gattattccg gagaaatccc agaacttcag 1200
   tgctcttaaa aaggatattc tacttccatt ccaagggcaa ttggattcta tcccagtatc 1260
   aaatccttca tgcccaatga ttcctctata cagaaatttg ccagaccact cagctgcaac 1320
   aaagcctagt ttgttcttat ttttagtgag tcttttgcac atattcttca aataaagtaa 1380
   aatgtgaaac tcaacccaca tcaccacctg gaatcaggga ttgctcactc caggttactg 1440
   caaccctaac tcactctagt gggaccttga ctggagaaac tccacgatct tcctgaagaa 1500
   gagaaatgga tgtttccaaa ttccacaata agcaatatgt ggtgataatg aaaagaatga 1560
   ttcagtcttg acggtgaatg gaagacactt acctaacaag tactgctcat ttacactcaa 1620
   attaatcttg aagtagtctt aaaatgtgta agaagttaaa acttgagaag caaaaaaatg 1680
   cctgcaaaaa gaagatcatt ttgtatacag agaaccggat gaatataagc aatgaagatg 1740
   aacatttatt gatcttctac atacaagact tcaccataag gccaggagca gtggctcaca 1800
   ccttgtaatc ccagcacttt gggaggccaa ggtgggcgga tcaccctgag gtcaggagtt 1860
   caaaaccagc ctgaccaaca tggtgaaacc ctgtctctac taaatattag cggggtgtgg 1920
   tggcgggcac ctgtaatcgc agcctttcag gaggctgaga caggagaatc gcttgaaccc 1980
   tagaggcgga gtttgcagtg agccgagata gtgccattgt actccagctt gggcaacaga 2040
   gtaagactct gtctcaaaaa aaaaaaaaca aaaacaaaca aacaaaaaaa acacctcacc 2100
   atgagtgcta catgtgaata gatattaagt gccatatata attagttctc agaagaaggg 2160
   agaaatgatc ataggactgg gaattgtttt gcaaacgttc taggagatgt gagagaaaat 2220
   atgtaaccac atcttagtgg cccaagaaaa tacaggcctg aagggataag attgtgtctc 2280
   tatagagctt caaagcatac aggtcaatta agaaagcccc tctctctcca gagccgtttc 2340
   cctagctttt ggcacctgga tgccacagtc ctccattagg ctgatgactc caaagatgta 2400
   actctagcct cttgcctgag cttcagactc gcgtcccact gcccacagga cacatccacc 2460
   tggatgtgac tcacaggtac ctccaaccca tcatgtggag atactcatcc tgttccccct 2520
   agagctgctc ttcctgctgc attctctctc tcaattactg ggaccaccaa gctaggaacc 2580
   tgggagtcat ccttgatact ttctcttcct ccttaatcct gtgtattcag caagtaacta 2640
   aaggttggtg ttggccaggc atggtggctc atgcctgtaa tcccagcatt ttgggaggcc 2700
   aaggcgggcg gatcacttga ggtcaggagc tcaagaccag cctggccaac atggtgaaac 2760
   cccatctcta ctaaaaaaaa aaaaaaaatt agtcgggcgt ggtggtgcat gcctgtaatc 2820
   ccagctactg gggaggctga ggcaggagaa tcgcttgaac ctgggaggca gaggttgcag 2880
   tgagccggga ttgcgccatt gtactccagc ctgggtgaag aagtgagact ctgtcttaaa 2940
   aaaaaaaatt ggtgctgata aatattgatg aattctgctc tctgctctct atggttgtca 3000
   acactgcaga gttgaggcct catatctcac ctgcactgct gcaacagctt actggtccct 3060
   tgctcccagc cttctcctct tcagtccatc gtccacacag cactggggaa ggggagccac 3120
   ttgaaacaaa agtcaacaac tggttgtagt tcataaacac agagctgttt gtgtcccctg 3180
   tatctggaat gccattatga cccactacat tttttctttc ctacccctct taaaactcag 3240
   ttcaggtagc agctccacta ggaagccttg gctgaccata atcccattca attccatttc 3300
   acctcttcgc aggcagtctg ggttagggga ccctttctct ttgctcccca aaataaactg 3360
   gttatctcta ctattggatt tacaacattg tattataatc ttctccatgt gtgccttctc 3420
   tagtagaatg tgagctcttt gaggccaagg tctatttaat ttgtttgaaa aattcattgt 3480
   tatatcctca aagcctagca catagtaggt actgaatgaa tgaatgaaca aggggtgcca 3540
   ggagactgct actcccagtc cttcccagaa actgcctagg gctttgagtc attttatgaa 3600
   gctaggtctt aatgcgtagg caacctccca gctcactatg aacgctgaca gaagagtgtt 3660
   ttcatgtcta taatcaagaa ttccagatac attcctttta ctgaaccttg aattgatcct 3720
   aagattggta gtaaaggtat tatgttacct cctaacagca ctacaaagta ccttttttta 3780
   tcagaaaaaa attttaccat taggactcaa tttgaagtac taatgcttct caagttctcc 3840
   actatgagag ttaccctgta ttagaccgtt acctataaga attaaggggt aaagcactaa 3900
   acagaaaaga aaaaaaaaat agcaactctg gtgagcagat ttctttcctt tcttccttcc 3960
   ttctcctctt cctaccttcc tccctccttt ccctctcctc cccttctctc cctttccctc 4020
   cccttccctt ccttttcttc tttcctccgc tcccctcccc ttccctcccc ttcccatcct 4080
   tctttctctt ttttttactt aatccccagt gtgacagtaa tataggctga tttctagaag 4140
   tgtggtgtat tactcatgga aagtgagttg ccttggttat tactttcaat tgaaagttct 4200
   atgggatcta gaaatgagac atactggcat ggagagtgag aacgacaaag gaatgaagag 4260
   ctacaggagc atttaggcca tttctatgcc aagcttattc tacatgcaca aaatcataca 4320
   tgttaataaa tataaacaaa ttggaggctt atttaaacca attatgaaat ctggtaattt 4380
   gtgcagcagc aatagatgat aaccaaaaaa aactcataat aatctgaata tcttgatcat 4440
   ttgtatttaa agaagcagta attatatact tgaaagtaca taatatagta ttgcaaaaat 4500
   gactttggta tattacaaat taaaagtata taagatgaaa cttgatttgc tatcaagccc 4560
   caagcaattt ttcaactggg cattgaattc taacttttct aagatagcaa tttttgaaga 4620
   gacacgaaca aaaatctgaa ttagttcatg agccttaatg taaatctctt gctgaaatag 4680
   tttttaaaat cagaatttag ttatctatca gactcaaaat catttaaaga ctaacaaaac 4740
   acaatcatga tattctaact gtggtcaaac caggtaccca agccacctcc ctgcccaacg 4800
   cctttccggc ttttcccctc cctcttgggc tggtggttat gctcctccag ctctagttca 4860
   gctataattc cttttataga gaaaccaacc tgatacacac tttcatgatg ggagaaaaat 4920
   gtgggagtga aatggtattt agaaagcagc agtcaggcac ggtggctcat gcctgtaatc 4980
   ccagcacttt gggaggctga ggcaggcgga tcacttgagg tcaggagctc gagaccagcc 5040
   tggccaacac ggtgaaaccc catctctact aaaaaaaaat acaaaaatta gccgggcgtg 5100
   gtggcaggca cctgtaatcc cagctacttg ggaggctgag gcaggagaaa tcgcctgaac 5160
   ccagaaggca gaggttgcag tgagccaaga tcacatcact gcactgcact ccagccgggg 5220
   tgacagagcg aacctctgtc tcaaaaaaaa aaaaagaaaa aagaaagaaa gaaaaaaggc 5280
   agaagccctg gattcaaatc cgccacacat tcagtttctt tatctgtaaa atggagacca 5340
   ccccccgcca cgctgaacgg tgattctgtg actggtaaga gatgctacat ttttggtgct 5400
   tgttcaggtg gaggaaagat gatagttaac actcaggtaa taagtatttt gaaggcagta 5460
   taatatacct tcttaaagag tatacctact caaatgttgg taaatgttga catgattgaa 5520
   tctaaatggc aaagagtatt ttagaaaaac attaagtccc tgcagataaa tgacagtgtt 5580
   gatttggatg cttaattaca ttcagacatg aactgttgga tgtatctgaa atgttaaaag 5640
   ctttttctca acatttccaa aagtctttcc aagaaatcaa tgttatgttt tgttccagaa 5700
   gcaaatttgc atttgtgatc tgtttctaaa aatggtacaa gttagctctg tttagaaagt 5760
   aaaaatatct gatgttagat tggaagtatc tcttcctggg gaatccagaa agataagcat 5820
   agcatattgt cttactgcaa tagataagtt gcttattgag aagtctggtt gttattctat 5880
   atggtaacaa tacagttgat gtatatttta tgatagatcc tttatatttt cctcatgact 5940
   ttagaagggg gaagggggag aaaattatga tgaccagact agttaaagag cattgaaagt 6000
   ccacagtact gtagctaaag tagaagtttg ggtttgttat agactttaca ttatatcaac 6060
   taataagcag atactgtaca gtattgctca ccattttatc atacttttgc atatgaacta 6120
   ctccattgcc ttttatagat gttttatagc tgatcttacc agttttcctg gtaacttttt 6180
   ttatttcttt tttttttttt tgagacggag tctcgcccta acacccaggt tggagtgcag 6240
   tgccgtgatc tcggctcact gcaacctctg cctcccgggt tcaagcaatt ctcctgtctc 6300
   agcctcccga gtacctggga ctaccggtgc ctgtctccac gcccggctaa ttttttgtat 6360
   ttgtagtaga gacggggttt caccgtgtta gccaggatgg tctcgatctc ctgacctcat 6420
   gatctgcctg cctctgcctg gacctcccaa agtgctggga ttacaggcgt gagcccccgc 6480
   gcccagccac tttctttaat actataacta agaatttatt aaaatgcaca aattgtctaa 6540
   gactgtaaag tttattgggg agaggccatg actacctctg aatttagtaa atttaaaata 6600
   tttctgattc tcaataaaga actaatatcc atataaaaaa aaaaaaaaaa a 6651
<210> 12
   <211> 3055
   <212> DNA
   <213> Homo sapiens
<400> 12
   atttccggag ggggaggccc gcggctgccg ccgccatttc gggcgctgct gtgaagctga      60
   aaccggagcc ggtccgctgg gcggcgggcg ccgggggccg gaggggcgcg cgcggcggcg     120
   gcaccccagc gtttaggcgc ggaggcagcc atggcgggca acttcgactc ggaggagcgg    180
   agtagctggt actgggggag gttgagtcgg caggaggcgg tggcgctgct gcagggccag     240
   cggcacgggg tgttcctggt gcgggactcg agcaccagcc ccggggacta tgtgctcagc     300
   gtctcagaga actcgcgcgt ctcccactac atcatcaaca gcagcggccc gcgcccgccg     360
   gtgccaccgt cgcccgccca gcctccgccc ggggtgagcc cctccagact ccgaatagga     420
   gatcaagagt ttgattcatt gcctgcttta ctggaattct acaaaataca ctatttggac     480
   actacaacgt tgatagaacc agtttccaga tccaggcagg gtagtggagt gattctcagg     540
   caggaggagg cggagtatgt gcgagccctc tttgacttta atgggaatga tgaggaagat     600
   cttcccttta agaaaggaga catcttgaga atccgggaca agcctgaaga gcagtggtgg    660
   aatgcggagg acagcgaagg caagagaggg atgattccag tcccttacgt cgagaagtat     720
   agacctgcct ccgcctcagt atcggctctg attggaggtc ggtgagctgg taaaggttac     780
   gaagattaat gtgagtggtc agtgggaagg ggagtgtaat ggcaaacgag gtcacttccc     840
   attcacacat gtccgtctgc tggatcaaca gaatcccgat gaggacttca gctgagtata     900
   gttcaacagt tttgctgaca gatgggaaca atcttttttt tttttttcca actgccatct     960
   atacaatttt cttacagatg tcaaaagcag tctagtttat ataagcattc tgttacctgt    1020
   gatatttttt agactgaact gctccattcc tagtcttaat taccatattc agggtacgaa    1080
   ctggagggct tgtgtgttag cttctgaatt ggcaattgga ggcggtagtg gtcgtgcctg    1140
   tgtgtatcag aagggatagg tatcttgcct cctttctctc aggcagtgca aatcaccctg    1200
   tggaaaaccg atggacagga aggagtgtta cacactgctt accctgattt attcagtggt    1260
   tttgttttca ttctggaacc atactatcaa atggcgacag actgttccgt tccacccccg    1320
   tgaagtaatc atgcaccgtg tgaatagtat caagcaggat tgctttcatt gtatggagca    1380
   tgaccagcgt gtgactcatt ctgacatttc agatcctaag aattctaaga acactactag    1440
   aagcatttgt tccctcctag tcaatgcttc atactttttc ttgggattct tttagccctt    1500
   gacattcttg tcccccaaac ctgtaagtag gtgaattcct aagataagtg tgtattttca    1560
   ttccaggtga aaagcaggat gtaccgagca ctttattcag tgcatagctt taagccagtg    1620
   ttggattcac taagtggaca gccagtctcc cagctctctg ccttccccaa aagggtcgta    1680
   gtaggtcacc cttctacagc agctaactag agtcctaact aatgggatcc agcagggcca    1740
   tttctccaga gggccagtat cctattagga gactcttgga attcttaggt tctactcaag    1800
   agtggaagga ccaatcacct ctgatattct gtggaaggtt ttggggtcaa attctgccct    1860
   ctgcattctg tgcaacttgt ataaaagtca agttagtatt acatgaattt ggggtagggt    1920
   tagtgctttg aaaaaatgtt gaaccggctg ggcgcggtgg ctcacgtctg taatcccagc    1980
   actttgggag gccgaggcgg gtggatcatg aggtcaggag ttcgagacca gcctggccaa    2040
   catagtgaaa ccccatctct gctaaagata taaaaaatta gcccggcgtg gtggtgcacg    2100
   cctgtaatcc cagctactcg ggaggctgag gcaggagaat tgcttcaacc tgggaggtgg    2160
   aggctgcagt gagccgagat cgcaccactg cgttccagcc tgagcgacag ggcaagactc    2220
   agtctcaaaa aaaaaaaaaa ggaaaaaaaa aagaaaaaaa aatgttgaac caattgtgaa    2280
   ttacttatgt attattcatt tctcatgggg agagtaatgc tgttgaagaa cattacattg    2340
   taaactgcct tcatttttgg ctctttgttt atgttcaggt ttagtttaca aacccattta    2400
   agtatggaat gatttatatg gggtcaggtg ctccacaaaa tagacctatg agaccaaaaa    2460
   tgacctaggc tatttagacg acagcatgaa acttccacgt tagttctcag tctataaagg    2520
   cacttaccgg tctctggtgt ggtatgacca atagaaacac cttatagttt gctttggacc    2580
   tcattttgga aaaataatct gcctttctaa ttgttctgca taggttaaaa tgataaattt    2640
   acattctttg aacctatacc agattgtggt gtccgagtga ccggcacact gtctgacaca    2700
   cagtcagtgt gcacgtattt gtctgagtga atgaggagac ctgagaaacc ggtgacgtgg     2760
   cacagggaag ccagctggcc caggattccg tacatggccg caagcagact aacgcgttga     2820
   cgctaattta atgtatttta cctcacacta aggtcatgct tgataaagac gttaaactca     2880
   acttgtaaaa tggtagccca gtgctatgca cagagtgggt gctcattagt gttgaatgaa     2940
   cacatttgta atactacatg taattccatc tgactgcttt gttaaatttt cagttagaac     3000
   gtagatactg taaagtccac acacacatta aatcttgttt tcctgaaagt atggc          3055
<210> 13
   <211> 1983
   <212> DNA
   <213> Homo sapiens
<400> 13
   ctctgaaggg agctactcag aagcgggagt ctccgagaga agaaaagcag gtggaaggag     60
   aggaagcgga tgccgtgggg tttacagcag gaaaatccgt ggagacagca gatccgagaa    120
   gcggcgatgt ttgcgtagaa ccctgtcagc tgagccatga cccatgaacc atggaagctt    180
   gactctagat tgaccatctt gagatgccaa agatgtccac gtcctaatcc catgtgggag    240
   acagaataat ggccctgcag accttcccag ctggccatga cccctcattt gaccagctct    300
   tcccttctct ctgaccagca ccatgcttct cctggtgaca agccttctgc tctgtgagtt    360
   accacaccca gcattcctcc tgatcccaga gaaatcggat ctgcgaacag tggcaccagc    420
   ctctagtctc aatgtgaggt ttgactccag gacgatgaat ttaagctggg actgccaaga    480
   aaacacaacc ttcagcaagt gtttcttaac tgacaagaag aacagagtcg tggaacccag    540
   gctcagtaac aacgaatgtt cgtgcacatt tcgtgaaatt tgtctgcatg aaggagtcac    600
   atttgaggtt cacgtgaata ctagtcaaag aggatttcaa cagaaactgc tttatccaaa    660
   ttcaggaagg gagggtaccg ctgctcagaa tttctcctgt ttcatctaca atgcggattt    720
   aatgaactgt acctgggcga ggggtccgac ggccccccgt gacgtccagt attttttgta     780
   catacgaaac tcaaagagaa ggagggagat ccggtgtcct tattacatac aagactcagg    840
   aacccatgtg ggatgtcacc tggataacct gtcaggatta acgtctcgca attactttct    900
   ggttaacgga accagccgag aaattggcat ccaattcttt gattcacttt tggacacaaa    960
   gaaaatagaa cgattcaacc ctcccagcaa tgtcaccgta cgttgcaaca cgacgcactg   1020
   cctcgtacgg tggaaacagc ccaggaccta tcagaagctg tcgtacctgg actttcagta   1080
   ccagctggac gtccacagaa agaataccca gcctggcacg gaaaacctac tgattaatgt   1140
   ttctggtgat ttggaaaata gatacaactt tccaagctct gagcccagag caaaacacag   1200
   tgtgaagatc agagctgcag acgtccgcat cttgaattgg agctcctgga gtgaagccat   1260
   tgaatttggt tctgacgacg ggaacctcgg ctctgtgtac atttatgtgc tcctaatcgt   1320
   gggaaccctt gtctgtggca tcgtcctcgg cttcctcttt aaaaggttcc ttaggataca     1380
   gcggctgttc ccgccagttc cacagatcaa agacaaactg aatgataacc atgaggtgga     1440
   agacgagatc atctgggagg aattcacccc agaggaaggg aaaggctacc gcgaagaggt     1500
   cttgaccgtg aaggaaatta cctgagaccc agagggtgta ggaatggcat ggacatctcc     1560
   gcctccgcga cacgggggaa ctgttttctt gatgatgctg tgaaccttta tatcattttc     1620
   tatgttttta tttaaaaaca tgacatttgg ggccaggcgc ggtggctcac gcctgtaatc     1680
   ccagcacttt gggaggccaa ggcaggcgga tcacctgagg tcaggagttc aagaccagcc     1740
   tgcccaacat ggtgaaaccc catctggact aaaaatgcag aaatttaccc aggcacggcg     1800
   gcggacgccc atcatcccag ctacttggga ggctgaggca ggagaattgc ttgaacccgt     1860
   gaggcggagg ttgtagtgag ccaagatcgc accattgcac accaacctgc gtgacagagc     1920
   aagattgcat ctcaaaacaa acaataataa taaataataa aaacctgata tttggctggg     1980
   caa                                                                   1983
<210> 14
   <211> 6363
   <212> DNA
   <213> Homo sapiens
<400> 14
   ggctgacatc acttaggaaa gcgaaggggg tagggctgcc agatcagttt gtcaccaccc      60
   aggctccctt gcctttggct gggtgcaact tccattttag gtgttggatc tgagggggaa     120
   aaaaaagaga gagggagaga gagagaaaga agagcaggaa agatcccgaa aggaggaaga     180
   ggtggcgaaa aatcaactgc cctgctggat ttgtctttct cagcaccttg gcgaagcctt     240
   gggtttcttt cttaaaggac tgatttttag aactccacat ttgaggtgtg tggcttttga     300
   agaaaatgta tgtactgacg ggaaaaggag gataagcaag tcgaattttt gtcttacgct     360
   ctctccttcc tgcttcctcc ttgctgtggt ggctgggatg cttcttccat gattttttga      420
   atctagactg ggctgttctc tgtgttaaac caatcagttg cgaccttctc ttaacagtgt     480
   gaagtgaggg ggtctctctc cctccttctc cttcctctgt gattcacctt cctttttacc      540
   ctgccctgcg gcggctccgc cccttacctt catggacgac tcagaggtgg agtcgaccgc     600
   cagcatcttg gcctctgtga aggaacaaga ggcccagttt gagaagctga cccgggcgct     660
   ggaggaggaa cggcgccacg tctcggcgca gctggaacgc gtccgggtct caccacaaga     720
   tgccaaccca ctcatggcca acggcacact cacccgccgg catcagaacg gccggtttgt     780
   gggcgatgct gaccttgaaa gacagaaatt ttcagatttg aaactcaacg gaccccagga     840
   tcacagtcac cttctatata gcaccatccc caggatgcag gagccggggc agattgtgga     900
   gacctacacg gaggaggatc ctgagggagc catgtctgta gtctctgtgg agacctcaga     960
   tgatgggacc actcggcgca cagagaccac ggtcaagaaa gtagtgaaga ctgtgacaac 1020
   acggacagta cagccagtcg ctatgggacc agacgggttg cctgtggatg cttcatcagt 1080
   ttctaacaac tatatccaga ctttgggtcg tgatttccgc aagaatggca atgggggacc 1140
   tggtccctat gtggggcaag ctggcactgc tacccttcct aggaacttcc actaccctcc 1200
   tgatggttat agtcgccact atgaagatgg ttatccaggt ggcagtgata actatggcag 1260
   tctgtcccgg gtgacccgca ttgaggagcg gtataggccc agcatggaag gctaccgggc 1320
   acctagtaga caggatgtgt atgggcccca accccaggtt cgggtaggtg ggagcagcgt 1380
   ggatctgcat cgctttcatc cagagcctta tgggctagag gatgaccagc gtagtatggg 1440
   ctatgatgac ctggattatg gtatgatgtc tgattatggc actgcccgtc ggactgggac 1500
   accctctgac cctcgtcggc gcctcaggag ctatgaagac atgattggtg aggaggtgcc 1560
   atcggatcaa tactactggg ctcctttggc ccagcatgag cgaggaagtt tagcaagctt 1620
   ggatagcctg cgcaaaggag ggcctccacc tcctaattgg agacagccag agctgccaga 1680
   ggtgatcgcc atgcttggat tccgcttgga tgctgtcaag tccaatgcag ctgcatacct 1740
   gcaacactta tgctaccgca atgacaaggt gaagactgac gtgcggaagc tcaagggcat 1800
   cccagtactg gtgggattgt tagaccatcc caaaaaggaa gtgcaccttg gagcctgtgg 1860
   agctctcaag aatatctctt ttggacgtga ccaggataac aagattgcca taaaaaactg 1920
   tgatggtgtg cctgcccttg tgcgattgct tcgaaaggct cgtgatatgg accttactga 1980
   agttattacc ggaaccctgt ggaatctttc atcccatgac tcaatcaaaa tggagattgt 2040
   ggaccatgca ctgcatgcct tgacagatga agtgatcatt cctcattctg gttgggagcg 2100
   ggaacctaat gaagactgta agccacgcca tattgagtgg gaatcggtgc tcaccaacac 2160
   agctggctgc cttaggaatg taagctcaga gaggagtgaa gctcgccgga aacttcggga 2220
   atgtgatggt ttagttgatg ccctcatttt cattgttcag gctgagattg ggcagaagga 2280
   ttcagacagc aagcttgtag agaactgtgt ttgccttctt cggaacttat catatcaagt 2340
   tcaccgggag atcccacagg cagagcgtta ccaagaggca gctcccaatg ttgccaacaa 2400
   tactgggcca catgctgcca gttgctttgg ggccaagaag ggcaaagatg agtggttctc 2460
   cagagggaaa aaacctatag aggatccagc aaacgataca gtggatttcc ctaaaagaac 2520
   gagtccagct cgaggctatg agctcttatt tcagccagag gtggttcgga tatacatctc 2580
   acttcttaag gagagcaaga ctcctgccat cctagaagcc tcagctggag ctatccagaa 2640
   cttgtgtgct gggcgctgga cgtatggtcg atacatccgc tctgctctgc gtcaagagaa 2700
   ggctctttct gccatagctg acctcctgac taatgaacat gaacgggtgg tgaaagctgc 2760
   atctggagca ctgagaaacc tggctgtgga tgctcgcaac aaagaattaa ttggtaaaca 2820
   tgctattcct aacttggtaa agaatctgcc aggaggacag cagaactcct cttggaattt 2880
   ctctgaggac actgtcatct ctattttgaa cactatcaac gaggttatcg ctgagaactt 2940
   ggaggctgcc aaaaagcttc gagagacaca gggtattgag aagctggtgt tgatcaacaa 3000
   atcagggaac cgctcagaaa aagaagttcg agcagcagca cttgtattac agacaatctg 3060
   gggatataag gaactgcgga agccactgga aaaagaagga tggaagaaat cagactttca 3120
   ggtgaatcta aacaatgctt cccgaagcca gagcagtcat tcatatgatg atagtactct 3180
   ccctctcatt gaccggaacc aaaaatcaga taagaaacct gatcgggaag aaattcagat 3240
   gagcaatatg ggatcaaaca caaaatcact agataacaac tattccacac caaatgagag 3300
   aggagaccac aatagaacac tggatcgatc gggggatcta ggcgacatgg agccattgaa 3360
   gggaacaaca cccttgatgc aggacgaggg gcaggaatct ctggaggaag agttggatgt 3420
   gttggttttg gatgatgagg ggggccaagt gtcttacccc tccatgcaga agatttagca 3480
   ccactatctc cgttccatct gggcttatat gtacttttat tttttggtgg tgaaattgac 3540
   tgatgatttt cctttttctt cgctggacta ttgtgccaac tgccaggctg cctcctgccc 3600
   ttacagccct aagtggctgc cttctttcca tcaactccca acttcttcct gtgaagttta 3660
   attgtctcaa cgcctccccc tcccccattc cctccatttt tctcccaaga aacctgactc 3720
   aattatttgc atattttgag aaactgctgc agattagttc tttttgccag ttttccctgg 3780
   aactcctggc cttttgtgga ggggagggat ggagagaata ggaatcttca ctagaagccg 3840
   tgggaagaat tggaagttac atgctgtata tgcaatgtcc agcagtctga taaactgacg 3900
   attcttaatc aagatttttt tcctgatggg gaagggactt ttattttctt ttagagaggg 3960
   gaaagtgtga gctcttccct tattcctaat ggctattttt gaagcaaaga aggccagcaa 4020
   cattggcaca tgccacctgg caaaggaccc ttgagtaagt gaaggtctcc taaaactggg 4080
   attaagaaac cttgctctcc tcatctccaa ggcagggacc atcaagaacc tacagactcc 4140
   atctcttctg caagcctcat gccaaccctg ggctattgct gctgcccctt aaacacaggc 4200
   tgtccttaac ccacctctcc tgccctgtga tatgtctgct gagttggcct ggccatttcc 4260
   aagaggctgt agaaagggga gaatgtcaag gaagactttt ggtagagaag gagcagaaag 4320
   atgtgttttt gggaagaaga agacctctag gaggagctag taggaatgta catgaagcaa 4380
   ttagtctgaa actggcttcc ccactccccc gtttctcctt ttcctatcct tataggcctg 4440
   tcccttgcct ctgccctgga ttggttggca aactaaagga cttgatgtac ataactcctg 4500
   tcccttttcc cttacaaggt ggggattgcc cctggctttg cctcttcttt gtgcctttgg 4560
   cctggggtgc atctcctccc gcccttccat gtgcctttct ttgcctctgc agtctcattt 4620
   ctcataattt tgcaaattat attttgttgc tttcttacct actattggcc ctaaatagca 4680
   gaaagaagag aagtgaccga gagaacctca gattcttcat tgaggattgg tatagccatg 4740
   atttcagtca tagcaagctt ttgctcaaca gcatatgggt gggattttgc aaaaatccta      4800
   ttctgatgaa tctcaaagta aggctggtaa gagaagtgag tggtgtgact cttactcctt      4860
   aggtgcccag aatttaccat catctctgaa ggagttacag ggaagtggtc tccccaattc      4920
   tcccctccct ccagtattgc cccctctcac tttagcatat attaattagc aggttgggct      4980
   agagaaatca gctgctatgc gggttgatta ttattattat ttctaatcct tttccttatt      5040
   tgccttctac tccccttaat ctaatctaaa agctctgttc catgcaactg gagttcctta     5100
   tccctctctt ccccttccct tatatattga ggctatgggg taggagaaaa gtgcacaacc      5160
   caccaccccc tttactcgtg cattaaaatt tcttatttac ccttttcccc cttcccattt      5220
   cttcccactt tcatctacct tttctggcaa aaaggagcct tttgctctct gtgaccctaa      5280
   gagcacactg cacagggaaa attgccccat ccagacctgg ctccactctt gatctctctt      5340
   gtcctcttct gctcttttcc tggtgctctt ttttctcggt ggggtgtggg taatagaaca      5400
   gccgtgggct tttggggacc tttaactttt ttttctctct tttgtttata aaaaacacta      5460
   aacattcaat tccagagaac caaaaatccc accttcccac cgaacactac taaggggctt      5520
   gtgttctgct ccataccttt tctcttttct ttctgtcttg ttaatgcttt taaaaacaaa      5580
   tgagtttttt atataaataa agtttttaaa gtgtgtatgt ggggggtctg tgtcatttct      5640
   tcacttcaag ctgttatttc ttccctgctt tgcatctttg ttacttcctt atgtatcagt      5700
   gtcctttcca gagcaaccag aaggaggtta taccaggatt tattttgagc tcagccccaa      5760
   ctctttatca agcaacattc ttgttaacta tatgtgaaac attttttctt ctgaagattc     5820
   ttaaaaattg aatgtggctg aagttgaaca tgggagctta ttgctaattt agagatagga      5880
   aactgaagca taaagaatta atgacttact ttaattactg gaattcttct gcaacatttg      5940
   acaaaactaa ccttgaataa ggcccactgt aatacgtagc tctcttaaat ataacactta      6000
   ggactagaag attagaaact accaatccca actacgtaat aggaaaatgt aggatcaaaa      6060
   ggcccatgta tataagtact gaccactggg ccataatgtt gcttctcagg ctatatgcag      6120
   tcctttagtc agaagtcaat aggcctattt attaatattt tacagaccat attacctgga      6180
   ttaccaggga ctatctttgc tgcagagatc aagggttaag atctatggga agatacttat      6240
   ttttctgagg tccttatgtc ctgtcatata attaaagact caagagaatt tatgtgaaat      6300
   gctttctgta tgcccaaatc tttagattaa aattatatag ctgctcctga aaaaaaaaaa      6360
   aaa                                                                    6363
<210> 15
   <211> 3783
   <212> DNA
   <213> Homo sapiens
<400> 15
   ggggcggggc cgggagggta cttagggccg gggctggccc aggctacggc ggctgcaggg      60
   ctccggcaac cgctccggca acgccaaccg ctccgctgcg cgcaggctgg gctgcaggct     120
   ctcggctgca gcgctgggtg gatctaggat ccggcttcca acatgtggca gctctgggcc     180
   tccctctgct gcctgctggt gttggccaat gcccggagca ggccctcttt ccatcccctg     240
   tcggatgagc tggtcaacta tgtcaacaaa cggaatacca cgtggcaggc cgggcacaac     300
   ttctacaacg tggacatgag ctacttgaag aggctatgtg gtaccttcct gggtgggccc     360
   aagccacccc agagagttat gtttaccgag gacctgaagc tgcctgcaag cttcgatgca     420
   cgggaacaat ggccacagtg tcccaccatc aaagagatca gagaccaggg ctcctgtggc     480
   tcctgctggg ccttcggggc tgtggaagcc atctctgacc ggatctgcat ccacaccaat     540
   gcgcacgtca gcgtggaggt gtcggcggag gacctgctca catgctgtgg cagcatgtgt     600
   ggggacggct gtaatggtgg ctatcctgct gaagcttgga acttctggac aagaaaaggc     660
   ctggtttctg gtggcctcta tgaatcccat gtagggtgca gaccgtactc catccctccc     720
   tgtgagcacc acgtcaacgg ctcccggccc ccatgcacgg gggagggaga tacccccaag     780
   tgtagcaaga tctgtgagcc tggctacagc ccgacctaca aacaggacaa gcactacgga     840
   tacaattcct acagcgtctc caatagcgag aaggacatca tggccgagat ctacaaaaac     900
   ggccccgtgg agggagcttt ctctgtgtat tcggacttcc tgctctacaa gtcaggagtg     960
   taccaacacg tcaccggaga gatgatgggt ggccatgcca tccgcatcct gggctgggga    1020
   gtggagaatg gcacacccta ctggctggtt gccaactcct ggaacactga ctggggtgac    1080
   aatggcttct ttaaaatact cagaggacag gatcactgtg gaatcgaatc agaagtggtg    1140
   gctggaattc cacgcaccga tcagtactgg gaaaagatct aatctgccgt gggcctgtcg    1200
   tgccagtcct gggggcgaga tcggggtaga aatgcatttt attctttaag ttcacgtaag    1260
   atacaagttt cagacagggt ctgaaggact ggattggcca aacatcagac ctgtcttcca    1320
   aggagaccaa gtcctggcta catcccagcc tgtggttaca gtgcagacag gccatgtgag    1380
   ccaccgctgc cagcacagag cgtccttccc cctgtagact agtgccgtag ggagtacctg    1440
   ctgccccagc tgactgtggc cccctccgtg atccatccat ctccagggag caagacagag    1500
   acgcaggaat ggaaagcgga gttcctaaca ggatgaaagt tcccccatca gttcccccag    1560
   tacctccaag caagtagctt tccacatttg tcacagaaat cagaggagag acggtgttgg    1620
   gagccctttg gagaacgcca gtctcccagg ccccctgcat ctatcgagtt tgcaatgtca    1680
   caacctctct gatcttgtgc tcagcatgat tctttaatag aagttttatt ttttcgtgca    1740
   ctctgctaat catgtgggtg agccagtgga acagcgggag acctgtgcta gttttacaga    1800
   ttgcctcctt atgacgcggc tcaaaaggaa accaagtggt caggagttgt ttctgaccca    1860
   ctgatctcta ctaccacaag gaaaatagtt taggagaaac cagcttttac tgtttttgaa 1920
   aaattacagc ttcaccctgt caagttaaca aggaatgcct gtgccaataa aagttttctc 1980
   caacttgaag tctactctga tgggatctca gatcctttgt cactgcctat agacttgtag 2040
   ctgctgtctc tctttgtccc tgcagagaat cacgtcctgg aactgcatgt tcttgcgact 2100
   cttgggactt catcttaact tctcgctgcc ccagccatgt tttcaaccat ggcatccctc 2160
   ccccaattag ttccctgtca tcctcgtcaa ccttctctgt aagtgcctgg taagcttgcc 2220
   cttgcttaag aactcaaaac atagctgtgc tctatttttt tgttgttgtt gtgactgaca 2280
   gagtgagatt ccgtctccca ggctggagtg cagtggcgcc ttctcagctc actgcaacct 2340
   gcagcctcct agattcaagc gattctcctg cttcagcctt ccgagtagct gggatgacag 2400
   gcactcacca atatgcctgg gtaatttttg tatttttaag tacatacagg atttcaccat 2460
   gttggccagg ctagtttcaa actcccggcc tcaggtggtc tgcctgcctc agcctcccaa 2520
   agtgttggga ttacaggcgt gagccactgg gccctgcctg tattttttat cagccacaaa 2580
   tccagcaaca agctgaggat tcagctcata aaacaggctt ggtgtcttgg tgatctcaca 2640
   taaccaagat gctaccccgt ggggaaccac atccccctgg atgccctcca gccttggttt 2700
   gggctggagt cagggcctgt atacagtatt ttgaatttgt atgccactgg tttgcattgc 2760
   tggtcaggaa ctctagtgct ttgcatagcc ctggtttaga aacatgttat agcagttctt 2820
   ggtatagagc aaactagaag aaccagcaat cattccactg tcctgccaag gtacacctca 2880
   gtactcccct tcccaactga agtggtatga ggctagctct ttccaaaagc attcaagttt 2940
   ggcttctgat gtgactcaga atttaggaac cagatgctag atcaaataag ctctgaaaat 3000
   ctgaggaaca ttgtaggaaa ggtttgttaa gcatctctta agtgccatga tgagcataac 3060
   agccggccgt cgtggctcac gcctgtaatc ccagcacttt gggaggccaa ggtgggagga 3120
   tgacaaggtc aggagttcaa gaccagcctg gccaacatgc tgaaacctca cctctactaa 3180
   aaatacaaaa attagctggg catggtggca catgcctgta atcccagcta cttgggaggc 3240
   tgaggcagga gaatcgcttg aacccgggag gcggaggttg cagtgagcca agacagtgcc 3300
   agtgcactcc agcctcggtg acagcgcaag gctccgtctc aataattaaa aaaaaaaaaa 3360
   aaaaaaaaaa ggccgggcgc agtggctcaa gcctgtaatc ccagcacttt gggaggctga 3420
   ggcgggcaga tcacctgagg tcaggagttt tgagatcagc cttggcaaca cggtgaaacc 3480
   ccatctctac taaaaataca aaattagcca agcatgctgg cacatgcctg taatcccagc 3540
   tactcgggag gctgaggtac gagaatcgct tgaacctggg aggcagagga tgcagtgagc 3600
   cgagatcacg ccattgcact ccagcctggg ggacaagagt gaatctgtgt ctcaccaaaa 3660
   aaaaaaagaa aaagaaagat gcttaacaaa ggttaccata agccacaaat tcataaccac 3720
   ttatccttcc agtttcaagt agaatatatt cataacctca ataaagttct ccctgctccc 3780
   aaa 3783
<210> 16
   <211> 1466
   <212> DNA
   <213> Homo sapiens
<400> 16
   ggtgcgtccg cgggtggctg ccccgcaggt gcgcgcggcc ggggctggcg gcgactctct 60
   ccaccgggcc gcccgggagg ctcatgcagc gcggctgggt cccgcggcgc ccggatcggg 120
   gaagtgaaag tgcctcggag gaggagggcc ggtccggcag tgcagccgcc tcacaggtcg 180
   gcggacgggc caggcgggcg gcctcctgaa ccgaaccgaa tcggctcctc gggccgtcgt 240
   cctcccgccc ctcctcgccc gccgccggag ttttctttcg gtttcttcca agattcctgg 300
   ccttccctcg acggagccgg gcccagtgcg ggggcgcagg gcgcgggagc tccacctcct 360
   cggctttccc tgcgtccaga ggctggcatg gcgcgggccg agtactgagc gcacggtcgg 420
   ggcacagcag ggccgggggg tgcagctggc tcgcgcctcc tctccggccg ccgtctcctc 480
   cggtccccgg cgaaagccat tgagacacca gctggacgtc acgcgccgga gcatgtctgg 540
   gagtcagagc gaggtggctc catccccgca gagtccgcgg agccccgaga tgggacggga 600
   cttgcggccc gggtcccgcg tgctcctgct cctgcttctg ctcctgctgg tgtacctgac 660
   tcagccaggc aatggcaacg agggcagcgt cactggaagt tgttattgtg gtaaaagaat 720
   ttcttccgac tccccgccat cggttcagtt catgaatcgt ctccggaaac acctgagagc 780
   ttaccatcgg tgtctatact acacgaggtt ccagctcctt tcctggagcg tgtgtggggg 840
   caacaaggac ccatgggttc aggaattgat gagctgtctt gatctcaaag aatgtggaca 900
   tgcttactcg gggattgtgg cccaccagaa gcatttactt cctaccagcc ccccaatttc 960
   tcaggcctca gagggggcat cttcagatat ccacacccct gcccagatgc tcctgtccac 1020
   cttgcagtcc actcagcgcc ccaccctccc agtaggatca ctgtcctcgg acaaagagct 1080
   cactcgtccc aatgaaacca ccattcacac tgcgggccac agtctggcag ctgggcctga 1140
   ggctggggag aaccagaagc agccggaaaa aaatgctggt cccacagcca ggacatcagc 1200
   cacagtgcca gtcctgtgcc tcctggccat catcttcatc ctcaccgcag ccctttccta 1260
   tgtgctgtgc aagaggagga gggggcagtc accgcagtcc tctccagatc tgccggttca 1320
   ttatatacct gtggcacctg actctaatac ctgagccaag aatggaagct tgtgagggta 1380
   aactgtggct tattcttaca aaaagtgtaa taaaggagac tgacccctga caacatggta 1440
   ggcactgtat aaaaaaaaaa aaaaaa 1466
<210> 17
   <211> 2804
   <212> DNA
   <213> Homo sapiens
<400> 17
   aaagccagac tgattcatag aaactccttt aaaacacggt gaaaagaaac cgcccattac      60
   acaccccagt acaccagcag aggaaactta taacctcggg aggcaggtcc ttcccctcag     120
   tgcggtcaca tacttccaga agagcggacc agggctgctg ccagcacctg ccactcagag     180
   cgcctctgtc gctgggaccc ttcagaactc tctttgctca caagttacca aaaaaaaaag     240
   agccaacatg ttggtattgc tggctggtat ctttgtggtc cacatcgcta ctgttattat     300
   gctatttgtt agcaccattg ccaatgtctg gttggtttcc aatacggtag atgcatcagt     360
   aggtctttgg aaaaactgta ccaacattag ctgcagtgac agcctgtcat atgccagtga     420
   agatgccctc aagacagtgc aggccttcat gattctctct atcatcttct gtgtcattgc     480
   cctcctggtc ttcgtgttcc agctcttcac catggagaag ggaaaccggt tcttcctctc     540
   aggggccacc acactggtgt gctggctgtg cattcttgtg ggggtgtcca tctacactag      600
   tcattatgcg aatcgtgatg gaacgcagta tcaccacggc tattcctaca tcctgggctg     660
   gatctgcttc tgcttcagct tcatcatcgg cgttctctat ctggtcctga gaaagaaata     720
   aggccggacg agttcatggg gatctggggg gtggggagga ggaagccgtt gaatctggga     780
   gggaagtgga ggttgctgta caggaaaaac cgagataggg gaggggggag ggggaagcaa      840
   aggggggagg tcaaatccca aaccattact gaggggattc tctactgcca agcccctgcc    900
   ctggggagaa agtagttggc tagtactttg atgctccctt gatggggtcc agagagcctc     960
   cctgcagcca ccagacttgg cctccagctg ttcttagtga cacacactgt ctggggcccc    1020
   atcagctgcc acaacaccag ccccacttct gggtcatgca ctgaggtcca cagacctact    1080
   gcactgagtt aaaatagcgg tacaagttct ggcaagagca gatactgtct ttgtgctgaa    1140
   tacgctaagc ctggaagcca tcctgccctt ctgacccaaa gcaaaacatc acattccagt    1200
   ctgaagtgcc tactgggggg ctttggcctg tgagccattg tccctctttg gaacagatat    1260
   ttagctctgt ggaattcagt gacaaaatgg gaggaggaaa gagagtttgt aaggtcatgc    1320
   tggtgggtta gctaaaccaa gaaggagacc ttttcacaat ggaaaacctg ggggatggtc     1380
   agagcccagt cgagacctca cacacggctg tccctcatgg agacctcatg ccatggtctt    1440
   tgctaggcct cttgctgaaa gccaaggcag ctcttctgga gtttctctaa agtcactagt    1500
   gaacaattcg gtggtaaaag taccacacaa actatgggat ccaaggggca gtcttgcaac    1560
   agtgccatgt tagggttatg tttttaggat tcccctcaat gcagtcagtg tttcttttaa    1620
   gtatacaaca ggagagagat ggacatggct cattgtagca caatcctatt actcttcctc    1680
   taacattttt gaggaagttt tgtctaatta tcaatattga ggatcagggc tcctaggctc    1740
   agtggtagct ctggcttaga caccacctgg agtgatcacc tcttggggac cctgcctatc    1800
   ccacttcaca ggtgaggcat ggcaattctg gaagctgatt aaaacacaca taaaccaaaa 1860
   ccaaacaaca ggcccttggg tgaaaggtgc tatataattg tgaagtatta agcctaccgt 1920
   atttcagcca tgataagaac agagtgcctg cattcccagg aaaatacgaa aatcccatga 1980
   gataaataaa aatataggtg atgggcagat cttttcttta aaataaaaaa gcaaaaactc 2040
   ttgtggtacc tagtcagatg gtagacgagc tgtctgctgc cgcaggagca cctctataca 2100
   ggacttagaa gtagtatgtt attcctggtt aagcaggcat tgctttgccc tggagcagct 2160
   attttaagcc atctcagatt ctgtctaaag gggttttttg ggaagacgtt ttctttatcg 2220
   ccctgagaag atctacccca gggagaatct gagacatctt gcctactttt ctttattagc 2280
   tttctcctca tccatttctt ttataccttt cctttttggg gagttgttat gccatgattt 2340
   ttggtattta tgtaaaagga ttattactaa ttctatttct ctatgtttat tctagttaag 2400
   gaaatgttga gggcaagcca ccaaattacc taggctgagg ttagagagat tggccagcaa 2460
   aaactgtggg aagatgaact ttgtcattat gatttcatta tcacatgatt atagaaggct 2520
   gtcttagtgc aaaaaacata cttacatttc agacatatcc aaagggaata ctcacatttt 2580
   gttaagaagt tgaactatga ctggagtaaa ccatgtattc ccttatcttt tacttttttt 2640
   ctgtgacatt tatgtctcat gtaatttgca ttactctggt ggattgttct agtactgtat 2700
   tgggcttctt cgttaataga ttatttcata tactataatt gtaaatattt tgatacaaat 2760
   gtttataact ctagggatat aaaaacagat tctgattccc ttca 2804
<210> 18
   <211> 3196
   <212> DNA
   <213> Homo sapiens
<400> 18
   ctctacccgg ttggcaggcg gcctggccca gccccttctc taaggaagcg catttcctgc    60
   ctccctgggc cggccgggct ggatgagccg ggagctccct gctgccggtc ataccacagc   120
   cttcatctgc gccctggggc caggactgct gctgtcactg ccatccattg gagcccagca   180
   ccccctcccc gcccatcctt cggacagcaa ctccagccca gccccgcgtc cctgtgtcca   240
   cttctcctga cccctcggcc gccaccccag aaggctggag cagggacgcc gtcgctccgg   300
   ccgcctgctc ccctcgggtc cccgtgcgag cccacgccgg ccccggtgcc cgcccgcagc   360
   cctgccactg gacacaggat aaggcccagc gcacaggccc ccacgtggac agcatggacc   420
   gcggcacgct ccctctggct gttgccctgc tgctggccag ctgcagcctc agccccacaa   480
   gtcttgcaga aacagtccat tgtgaccttc agcctgtggg ccccgagagg ggcgaggtga   540
   catataccac tagccaggtc tcgaagggct gcgtggctca ggcccccaat gccatccttg   600
   aagtccatgt cctcttcctg gagttcccaa cgggcccgtc acagctggag ctgactctcc   660
   aggcatccaa gcaaaatggc acctggcccc gagaggtgct tctggtcctc agtgtaaaca 720
   gcagtgtctt cctgcatctc caggccctgg gaatcccact gcacttggcc tacaattcca 780
   gcctggtcac cttccaagag cccccggggg tcaacaccac agagctgcca tccttcccca 840
   agacccagat ccttgagtgg gcagctgaga ggggccccat cacctctgct gctgagctga 900
   atgaccccca gagcatcctc ctccgactgg gccaagccca ggggtcactg tccttctgca 960
   tgctggaagc cagccaggac atgggccgca cgctcgagtg gcggccgcgt actccagcct 1020
   tggtccgggg ctgccacttg gaaggcgtgg ccggccacaa ggaggcgcac atcctgaggg 1080
   tcctgccggg ccactcggcc gggccccgga cggtgacggt gaaggtggaa ctgagctgcg 1140
   cacccgggga tctcgatgcc gtcctcatcc tgcagggtcc cccctacgtg tcctggctca 1200
   tcgacgccaa ccacaacatg cagatctgga ccactggaga atactccttc aagatctttc 1260
   cagagaaaaa cattcgtggc ttcaagctcc cagacacacc tcaaggcctc ctgggggagg 1320
   cccggatgct caatgccagc attgtggcat ccttcgtgga gctaccgctg gccagcattg 1380
   tctcacttca tgcctccagc tgcggtggta ggctgcagac ctcacccgca ccgatccaga 1440
   ccactcctcc caaggacact tgtagcccgg agctgctcat gtccttgatc cagacaaagt 1500
   gtgccgacga cgccatgacc ctggtactaa agaaagagct tgttgcgcat ttgaagtgca 1560
   ccatcacggg cctgaccttc tgggacccca gctgtgaggc agaggacagg ggtgacaagt 1620
   ttgtcttgcg cagtgcttac tccagctgtg gcatgcaggt gtcagcaagt atgatcagca 1680
   atgaggcggt ggtcaatatc ctgtcgagct catcaccaca gcggaaaaag gtgcactgcc 1740
   tcaacatgga cagcctctct ttccagctgg gcctctacct cagcccacac ttcctccagg 1800
   cctccaacac catcgagccg gggcagcaga gctttgtgca ggtcagagtg tccccatccg 1860
   tctccgagtt cctgctccag ttagacagct gccacctgga cttggggcct gagggaggca 1920
   ccgtggaact catccagggc cgggcggcca agggcaactg tgtgagcctg ctgtccccaa 1980
   gccccgaggg tgacccgcgc ttcagcttcc tcctccactt ctacacagta cccataccca 2040
   aaaccggcac cctcagctgc acggtagccc tgcgtcccaa gaccgggtct caagaccagg 2100
   aagtccatag gactgtcttc atgcgcttga acatcatcag ccctgacctg tctggttgca 2160
   caagcaaagg cctcgtcctg cccgccgtgc tgggcatcac ctttggtgcc ttcctcatcg 2220
   gggccctgct cactgctgca ctctggtaca tctactcgca cacgcgtgag taccccaggc 2280
   ccccacagtg agcatgccgg gcccctccat ccacccgggg gagcccagtg aagcctctga 2340
   gggattgagg ggccctggcc aggaccctga cctccgcccc tgcccccgct cccgctccca 2400
   ggttccccca gcaagcggga gcccgtggtg gcggtggctg ccccggcctc ctcggagagc 2460
   agcagcacca accacagcat cgggagcacc cagagcaccc cctgctccac cagcagcatg 2520
   gcatagcccc ggccccccgc gctcgcccag caggagagac tgagcagccg ccagctggga 2580
   gcactggtgt gaactcaccc tgggagccag tcctccactc gacccagaat ggagcctgct 2640
   ctccgcgcct acccttcccg cctccctctc agaggcctgc tgccagtgca gccactggct 2700
   tggaacacct tggggtccct ccaccccaca gaaccttcaa cccagtgggt ctgggatatg 2760
   gctgcccagg agacagacca cttgccacgc tgttgtaaaa acccaagtcc ctgtcatttg 2820
   aacctggatc cagcactggt gaactgagct gggcaggaag ggagaacttg aaacagattc 2880
   aggccagccc agccaggcca acagcacctc cccgctggga agagaagagg gcccagccca 2940
   gagccacctg gatctatccc tgcggcctcc acacctgaac ttgcctaact aactggcagg 3000
   ggagacagga gcctagcgga gcccagcctg ggagcccaga gggtggcaag aacagtgggc 3060
   gttgggagcc tagctcctgc cacatggagc cccctctgcc ggtcgggcag ccagcagagg 3120
   gggagtagcc aagctgcttg tcctgggcct gcccctgtgt attcaccacc aataaatcag 3180
   accatgaaac cagtga 3196
<210> 19
   <211> 4446
   <212> DNA
   <213> Homo sapiens
<400> 19
   cgcaccgccg ccgaggacgc gcgcccgagc ctagtcccca cgccgcggcg cgcccgggct      60
   ccctgctgat cccagaacaa tcaaccatga cgaccgaatc tggatcagac tcggaatcca     120
   agccggacca ggaggccgag ccccaggagg cggcgggggc gcaggggcgc gcgggggcgc     180
   ccgtgccgga gccgcccaag gaggagcagc agcaggccct ggagcagttc gccgccgctg     240
   cagcgcacag caccccggtg cggagggagg tcactgacaa ggaacaggag tttgctgcca     300
   gggctgcaaa acagctcgaa tatcagcaat tagaagacga taaactttct cagaaatcat     360
   ctagcagtaa actctctcgg tctccattaa agattgtcaa aaagcctaaa agcatgcagt     420
   gcaaagtgat acttctcgat ggatcagaat atacctgtga tgtagagaaa cgctccagag     480
   gacaagtgct gtttgataaa gtgtgtgaac acttgaactt gctagagaaa gactactttg     540
   ggcttacgta tcgagatgct gaaaaccaga agaattggtt ggaccctgct aaggaaataa     600
   aaaaacaggt tcgaagtggt gcttggcact tttcatttaa tgtgaaattt tatccaccag     660
   accctgccca actatctgaa gatatcacca ggtactacct ctgcttgcag ttgcgagatg     720
   acatcgtgtc cggaaggctg ccctgctcct ttgttaccct ggccttgctg ggctcctaca     780
   ctgtccagtc agagctcgga gactatgacc cagatgaatg tgggagcgat tacattagtg     840
   agttccgctt tgcaccaaac cacactaaag aactggaaga caaagtgatc gagctgcaca     900
   agagccacag aggaatgacg ccagcagaag cagagatgca tttcttggaa aatgccaaaa     960
   aattatcaat gtatggggta gatttacatc atgctaagga ctcagaaggg gtagaaatta 1020
   tgttaggagt ttgtgcaagt ggtctgttga tatatcgcga ccggctgcga ataaacagat 1080
   ttgcctggcc caaggttcta aagatttcat acaaacggaa caacttttac attaagatcc 1140
   ggccgggaga gtttgaacaa tttgaaagca ccattgggtt taagctgcca aaccatcgag 1200
   ctgccaagcg tttatggaaa gtatgtgttg agcatcatac atttttcaga ctactgttac 1260
   cagaagcacc tcccaagaaa ttcctaacct tgggttccaa gtttcgttat agtggcagga 1320
   cacaagcgca aacgagaaga gccagtgcgt tgatagatcg cccagcacct tactttgaac 1380
   gctcatccag caaacgttat accatgtctc gcagcttgga tggagaggtt ggtactggcc 1440
   agtacgccac aacaaaaggc atctctcaga ccaacttgat caccactgtg actccggaga 1500
   agaaggctga ggaggagcgg gacgaggaag aggacaaacg gaggaagggg gaagaagtca 1560
   cgcccatctc ggccatccgg cacgagggaa agtcacctgg gcttggcact gactcatgtc 1620
   ccttgtcacc cccatccacc cattgtgccc ccacatctcc cacagagctc cgtaggaggt 1680
   gtaaggagaa tgactgcaaa ctgccaggtt atgagccgtc cagagctgag cacctgcctg 1740
   gagagcccgc cttggactct gatggcccag ggaggcctta cctaggggat caagatgtgg 1800
   cttttagcta cagacagcaa actggcaagg ggaccaccct gttctccttc tccttgcagc 1860
   tccctgagtc attcccctcc ctcctagatg atgatggata cctctctttc cccaaccttt 1920
   ctgaaaccaa cctcctgccc cagagcttgc agcattacct cccgatccgc tcaccgtccc 1980
   ttgtgccctg tttcctcttc atctttttct ttctgctgtc tgcctccttc tcagtgccat 2040
   acgctctcac tctctccttc cctctggctc tgtgcctctg ctacctggag cccaaggcgg 2100
   cctccttgag cgcctcccta gacaatgacc cgagtgacag ttcagaggaa gagactgaca 2160
   gtgagcgcac ggacaccgca gccgacgggg agaccaccgc cactgagtcg gaccaggagg 2220
   aagatgcaga gctcaaggca caggagctag aaaaaactca agatgacctg atgaaacatc 2280
   aaaccaacat tagcgagctg aaaagaacct tcttagaaac ctcaacagac actgccgtaa 2340
   cgaatgaatg ggagaagagg ctttccacct cccccgtgcg actggccgcc aggcaggagg 2400
   atgcccccat gatcgaacca cttgtccctg aagagactaa gcagtcttct ggggaaaagc 2460
   tcatggatgg ctctgaaatc ttcagtttat tagagtctgc gcgaaaacca acagaattca 2520
   taggaggggt tacttctact tctcaaagct gggttcagaa aatggaaacc aagacggagt 2580
   ccagtggaat agagacggaa cccaccgtgc accacctgcc gcttagcact gagaaggtgg 2640
   tgcaggagac cgtgttggtg gaggagcggc gtgtggtgca cgcgagtggg gatgcttctt 2700
   actcggcggg agacagcggg gatgctgcag cacagcccgc attcacaggc attaaaggga 2760
   aagagggctc tgccttgacg gagggggcta aagaggaagg aggggaggag gtcgctaaag 2820
   ctgtcctgga acaggaagag acagccgctg cttcccgtga gcgacaagag gagcagagtg 2880
   cagccatcca catttcagaa actttggaac aaaaacctca ttttgagtcc tcaacggtga 2940
   agacggaaac catcagtttt ggcagtgttt caccgggagg agtaaagcta gaaatttcca 3000
   cgaaggaagt gccagtagtt cacaccgaaa ccaaaaccat cacatatgaa tcatcacagg 3060
   tcgatccagg cacagatctg gagccaggcg tgctgatgag tgcacagacg atcacatctg 3120
   aaaccaccag taccaccacc actacgcaca tcaccaaaac tgtgaaaggg ggcatttcag 3180
   agacaagaat tgagaagcga atagtcatca cgggggatgc agacattgac catgaccagg 3240
   cgctggctca ggcaattaaa gaggccaaag agcagcaccc tgacatgtca gtgaccaaag 3300
   tagtggtcca taaagagaca gagatcacac cagaagatgg agaggattga ccagaggaat 3360
   aacttagctt gcacatgaat gcagtcatgc aaaccgttag gaaaaccaga gcctatatgg 3420
   agttccctct tctaacccaa ctgacttgta tctgtccgtg gaaaatttca gtccagaaga 3480
   attgaccttg accattaata aagacactgg cagagagatc ttcccataat aaagcaatct 3540
   gattcagcat cactaaaccg ataatgcatg aagcaacgat aaaattacaa aagagcagca 3600
   tttttaattt tcacaaaatg tctcagtttt cagctatacc tgcacgttca taaccaacaa 3660
   tataaaccgt ggtctcatgt aacacataaa caattcatgc ctttcatagt ttattattat 3720
   taaagtctaa acaaaattgc aatttcttag gtaaccttat atttacaata aatgaagatt 3780
   accctcaaat gctagaagct gtctaggtcc gtccggtgtg tcagattttc ctcagattag 3840
   atgtgccaat aaccaagttt attcagtaaa caacttgtac ttgtttcatc tggttttatt 3900
   actctcaccc ataaacagta atgactctct gaccctctgg aaatatgtaa tgcttccaat 3960
   cttgctttgt gtatctcatt taatttgtta taaggtagta ctgattttag catattaatg 4020
   cgatttcttc cttgttgttt gctttggtct gtgttcaatc cagagagctt aaattgtcat 4080
   tattttggga agaaaacctg tatttttgtt agtttacaat attatgaaat ttcacttcag 4140
   gagaaactgc tgggcttcct gtggctttgt tttcttagtt actttttccg tgccgtgtat 4200
   tttttaattg atttttcttc ttttacttga aaagaaagtg ttttattttc aaatctggtc 4260
   catatttaca ttctagttca gagccaagcc ttaaactgta cagaatttcc actgtaatta 4320
   aaactattta gtgttagtta taaatagcct tcaaaaagag agattctcca ttacacgatc 4380
   acctgcatca cagcccatgg tgaatgtatg tttctgcata gcgaaataaa aatggcaaat 4440
   gcactg 4446
<210> 20
   <211> 1839
   <212> DNA
   <213> Homo sapiens
<400> 20
   agcaacgggg tgcggcaggg tggggaacgc gggagcgggg ccagctccca ggaaagctgg 60
   tctgcgagcg gcccctgccc ggctcccagg tccctgcgcg accccgccct tcccgagacc 120
   ccagccgggc tgccgcccgc gtcccggaag ctccagcctg aaccatgttt ttcacttgtg 180
   gcccaaatga ggccatggtg gtctccgggt tctgccgaag ccccccagtc atggtggctg 240
   gagggcgtgt ctttgtcctg ccctgcatcc aacagatcca gaggatctct ctcaacacac 300
   tgaccctcaa tgtcaagagt gaaaaggttt acactcgcca tggggtcccc atctcagtca 360
   ctggcattgc ccaggtaaaa atccaggggc agaacaagga gatgttggcg gccgcctgtc 420
   agatgttcct ggggaagacg gaggctgaga ttgcccacat tgccctggag acgttagagg 480
   gccaccagag ggccatcatg gcccacatga ctgtggagga gatctataag gacaggcaga 540
   aattctcaga acaggttttc aaagtggcct cctcagacct ggtcaacatg ggcatcagtg 600
   tggttagcta cactctgaag gacattcacg atgaccagga ctatttgcac tctttgggga 660
   aggctcgaac agctcaagtc caaaaagatg cacggattgg agaagcagag gccaagagag 720
   atgctgggat ccgggaagct aaagccaagc aggaaaaggt gtctgctcag tacctgagtg 780
   agatcgagat ggccaaggca cagagagatt acgaactgaa gaaggccgcc tatgacatcg 840
   aggtcaacac ccgccgagca caggctgacc tggcctatca gcttcaggtg gccaagacta 900
   agcagcagat tgaggagcag cgggtgcagg tgcaggtggt ggagcgggcc cagcaggtgg 960
   cagtgcagga gcaggagatc gcccggcggg agaaggagct ggaggcccgg gtgcggaagc 1020
   cagcggaagc ggagcgctac aagctggagc gcctagccga ggcagagaag tcccaactaa 1080
   ttatgcaggc ggaggcagaa gccgcgtctg tgcggatgcg tggggaagct gaggcctttg 1140
   ccataggggc ccgagcccga gccgaggctg agcagatggc caagaaggca gaagccttcc 1200
   agctgtacca agaggctgct cagctggaca tgctgctaga gaagctgccc caggtggcag 1260
   aggagatcag tggtcccttg acttcagcca ataagatcac actggtgtcc agcggcagtg 1320
   ggaccatggg ggcagccaaa gtgactgggg aagtactgga cattctaact cgcctgccag 1380
   agagtgtgga aagactcaca ggcgtgagca tctcccaggt gaatcacaag cctttgagaa 1440
   cagcctgagc cttcagccct cacagatgcc cagcctcata gctgaagttg cctgaatgat 1500
   cctcctgttg catgtaaccc actggcctcc ctgagcatgt ccattgacag tgaggtccca 1560
   cccctcatct ctccttgcca aatagtttgt gccttgtctt gaagggggtt gctccccttg 1620
   ccaacctcac actgctatga ttgccaactc cagcggtccc atgtcagcct tctgatgatc 1680
   ccactccacc ccacctcaac ttatttaact tcctaattaa atcagactgt ttgagcctgt 1740
   tgtctagaat attttcctga ccaagactga gggatgggct ggaggttttc aactttgcta 1800
   cccaaataaa ttgctgtaag taagtactaa aaaaaaaaa 1839
<210> 21
   <211> 2132
   <212> DNA
   <213> Homo sapiens
<400> 21
   ggggagccct ggcctcccca cctcctcccg tccccaccct gttcccagca ctcaagcctt      60
   gccaccgccg agccgggctt cctgggtgtt tcaggcaagg aagtctaggt ccctgggggg     120
   tgacccccaa ggaaaaggca gcctccctgc gcacccggtt gcccggagcc ctctccaggg     180
   ccggctgggc tgggggttgc cctggccagc aggggcccgg gggcgatgcc acccggtgcc     240
   gactgaggcc accgcaccat ggcccgctcg ctgacctggc gctgctgccc ctggtgcctg     300
   acggaggatg agaaggccgc cgcccgggtg gaccaggaga tcaacaggat cctcttggag     360
   cagaagaagc aggaccgcgg ggagctgaag ctgctgcttt tgggcccagg cgagagcggg     420
   aagagcacct tcatcaagca gatgcggatc atccacggcg ccggctactc ggaggaggag     480
   cgcaagggct tccggcccct ggtctaccag aacatcttcg tgtccatgcg ggccatgatc     540
   gaggccatgg agcggctgca gattccattc agcaggcccg agagcaagca ccacgctagc     600
   ctggtcatga gccaggaccc ctataaagtg accacgtttg agaagcgcta cgctgcggcc     660
   atgcagtggc tgtggaggga tgccggcatc cgggcctact atgagcgtcg gcgggaattc     720
   cacctgctcg attcagccgt gtactacctg tcccacctgg agcgcatcac cgaggagggc     780
   tacgtcccca cagctcagga cgtgctccgc agccgcatgc ccaccactgg catcaacgag     840
   tactgcttct ccgtgcagaa aaccaacctg cggatcgtgg acgtcggggg ccagaagtca     900
   gagcgtaaga aatggatcca ttgtttcgag aacgtgatcg ccctcatcta cctggcctca     960
   ctgagtgaat acgaccagtg cctggaggag aacaaccagg agaaccgcat gaaggagagc    1020
   ctcgcattgt ttgggactat cctggaacta ccctggttca aaagcacatc cgtcatcctc    1080
   tttctcaaca aaaccgacat cctggaggag aaaatcccca cctcccacct ggctacctat    1140
   ttccccagtt tccagggccc taagcaggat gctgaggcag ccaagaggtt catcctggac    1200
   atgtacacga ggatgtacac cgggtgcgtg gacggccccg agggcagcaa gaagggcgca    1260
   cgatcccgac gcctcttcag ccactacaca tgtgccacag acacacagaa catccgcaag    1320
   gtcttcaagg acgtgcggga ctcggtgctc gcccgctacc tggacgagat caacctgctg    1380
   tgacccaggc cccacctggg gcaggcggca ccggcgggcg ggtgggaggt gggagtggct    1440
   gcagggaccc ctagtgtccc tggtctatct ctccagcctc ggcccacacg caagggagtc    1500
   gggggacgga cggcccgctg ctggccgctc tcttctctgc ctctcaccag gacagccgcc    1560
   ccccagggta ctcctgccct tgcttgactc agtttccctc ctttgaaagg gaaggagcaa    1620
   aacggccatt tgggatgcca gggtggatga aaaggtgaag aaatcagggg attgaggact    1680
   tgggtgggtg ggcatctctc aggagcccca tctccgggcg tgtcacctcc tgggcagggt    1740
   tctgggaccc tctgtgggtg acgcacaccc tgggatgggg ctagtagagc cttcaggcgc      1800
   cttcgggcgt ggactctggc gcactctagt ggacaggaga aggaacgcct tccaggaacc      1860
   tgtggactag gggtgcaggg acttcccttt gcaaggggta acagaccgct ggaaaacact      1920
   gtcactttca gagctcggtg gctcacagcg tgtcctgccc cggtttgcgg acgagagaaa      1980
   tcgcggccca caagcatccc cccatccctt gcaggctggg ggctgggcat gctgcatctt      2040
   aaccttttgt atttattccc tcaccttctg cagggctccg tgcgggctga aattaaagat       2100
   ttcttagagg ctgcgtcgcc agcgtcctgt tt                                    2132
<210> 22
   <211> 3798
   <212> DNA
   <213> Homo sapiens
<400> 22
   ggggcaagtg actcatgagc acgagttgtt aagcgatgga atttgggaga tcaagccaca        60
   ctgcttaaaa catcacatga tctccctctg gccccgtatt tcataaaaca gagcggatcg       120
   caggaggccg acactgtgac tcctggtgga tgggactggg gagtcagagt caagccctga       180
   ctggttgcag gcgctcggag tcagcatgga aagtctctgc ggggtcctgg gatttctgct       240
   gctggctgca ggactgcctc tccaggctgc caagcgattt cgtgatgtgc tgggccatga       300
   acagtatccc gatcacatga gagagcacaa ccaattacgt ggctggtctt cggatgaaaa     360
   tgaatgggat gaacacctgt atccagtgtg gaggagggga gacggcaggt ggaaggactc     420
   ctgggaagga ggccgtgtgc aggcagtcct gaccagtgac tcaccggctc tggtgggttc       480
   caatatcact tttgtggtga acctggtgtt ccccagatgc cagaaggaag atgctaatgg       540
   caatatcgtc tatgagaaga actgcaggaa tgatttggga ctgacatctg acctgcatgt       600
   ctacaactgg actgcagggg cagatgatgg tgactgggaa gatggcacca gccgaagcca       660
   gcatctcagg ttcccggaca ggaggccctt ccctcgcccc catggatgga agaaatggag       720
   ctttgtctac gtctttcaca cacttggcca gtatttccaa aaactgggtc ggtgttcagc       780
   acgggtttct ataaacacag tcaacttgac agctggccct caggtcatgg aagtgactgt       840
   ctttcgaaga tacggccggg catacattcc catctcgaag gtgaaagatg tgtatgtgat       900
   aacagatcag atccctgtat tcgtgaccat gtcccagaag aatgacagga acttgtctga       960
   tgagatcttc ctcagagacc tccccatcgt cttcgatgtc ctcattcatg atcccagcca      1020
   cttcctcaac gactctgcca tttcctacaa gtggaacttt ggggacaaca ctggcctgtt      1080
   tgtctccaac aatcacactt tgaatcacac ttatgtgctc aatggaacct tcaaccttaa      1140
   cctcaccgtg caaactgcag tgcccgggcc atgccctccc ccttcgcctt cgactccgcc      1200
   tccaccttca actccgccct cacctccgcc ctcacctctg cccacattat caacacctag      1260
   cccctcttta atgcctactg gttacaaatc catggagctg agtgacattt ccaatgaaaa 1320
   ctgccgaata aacagatatg gctacttcag agccaccatc acaattgtag aggggatcct 1380
   ggaagtcagc atcatgcaga tagcagatgt ccccatgccc acaccgcagc ctgccaactc 1440
   cctgatggac ttcactgtga cctgcaaagg ggccaccccc atggaagcct gtacgatcat 1500
   ctccgacccc acctgccaga tcgcccagaa ccgggtctgc agccctgtgg ctgtggatgg 1560
   gctgtgcctg ctgtctgtga gaagagcctt caatgggtct ggcacctact gtgtgaattt 1620
   cactctggga gatgatgcaa gcctggccct caccagcacc ctgatctcta tccctggcaa 1680
   agacccagac tcccctctga gagcagtgaa tggtgtcctg atctccatcg gctgcctggc 1740
   tgtgcttgtc accatggtta ccatcttgct gtacaaaaaa cacaaggcgt acaagccaat 1800
   aggaaactgc cccaggaaca cggtcaaggg caagggcctg agtgttctcc tcagtcacgc 1860
   gaaagccccg ttcttccgag gagaccagga gaaggatcca ttgctccagg acaagccaag 1920
   gacactctaa gtctttggcc ttccctctga ccaggaaccc actcttctgt gcatgtatgt 1980
   gagctgtgca gaagtatgtg gctgggaact gttgttctct aaggattatt gtaaaatgta 2040
   tatcgtggct tagggagtgt ggttaaatag cattttagag aagacatggg aagacttagt 2100
   gtttcttccc atctgtattg tggtttttac actgttcgtg gggtggacac gctgtgtctg 2160
   aaggggaggt ggggtcactg ctacttaagg tcctaggtta actgggggag ataccacaga 2220
   tgcctcagct ttccacataa catgggcatg aacccagcta atcaccacct gaaggccatg 2280
   cttcatctgc cttccaactc actgagcatg cctgagctcc tgacaaaatt ataatgggcc 2340
   cgggctttgt gtatggtgcg tgtgtgtaca tattctactc attaaaaagg cagtctaata 2400
   agctgtgtga ttattatatt ggggagaaaa ttttccctgt gtagttcagt gaactaccca 2460
   tccattcatt tatccatgga cacttataaa gcatgtggtg ggctgcactt gacctatgag 2520
   accttgtcta ttctatacca gcagaccctt aatagcaacc aagtgagatc tgagagtcca 2580
   gactgtatct accttgatga aggtagacaa ctggataaat atgatggtat tatagaatga 2640
   ctgctcattt ttgaggcatt gtggaggacc aacattcagt ctggtacttt gacacccccc 2700
   ccccccacta ggtggcctag aagcaaaaag aagctttttg tttggaaatt gtctccaaag 2760
   aatgtaaaaa ttgctgacca cttaggacag ggcctctcag ccaggagtgt ttggcaatgt 2820
   ttggagagat ttgtggttgt caaaactagg gtggggtgct actggcatct agtggggaga 2880
   gcccagggct gctgaacacc ctgtgcctgc gggacagccc cacagccaag gagcccagag 2940
   caactgaagg agtcctgggc acaggcatcc caacagcagc cttgtatctg aaccgagccc 3000
   tgacatctaa agctttgtga cctagtgaca tggcatgtta ggagtctttt ctgcctctgt 3060
   ctcagctgta gaaggggtat gctgtccctc tctgactcac ttcacatggc cgctactgct 3120
   cctgatggaa gaatgagtgc aggtacagcc ttgtgttaca gtctcagcct ggggacagaa 3180
   acacgtgaag aagcttgatt gattgattga ttgattgatt gattgattga ttgattgatt 3240
   gattgatttt tacatactaa ggagcccgtt cactagtacc caaaacagga cattgatttc 3300
   aggttgctgc agaatccaca gaccgcttag actccacccc atggttccaa acactgtctt 3360
   gtggaggtgg gaccccataa tttggatttc tgagaaacca acagagaatc cttattctgc 3420
   tggttccagg acaagaatga gcaaggctgg gcttgggatc acttccttgc tcaggtcaac 3480
   agggggcagc agagaacagc agatacagaa cagccaccaa agcggcaggt ccatgacttt 3540
   gtttatctag cttcatctaa acaaatttta atctagtagt aggaaagaag tctgaaatag 3600
   taaattgtgt cgatttatat tttccaagtg accctggtaa gggaactgtc tgcagaatgg 3660
   aagaaatagc ctaagagaca gggatggctt gattgatgtt ggctgagaat taaaacttct 3720
   cacgaaaagt acatgtgtat gtaacaaatt attccaggaa ttgcttattt ctagggcttt 3780
   tataaatgtc tgcatcct 3798
<210> 23
   <211> 2996
   <212> DNA
   <213> Homo sapiens
<400> 23
   atgtggtgcg gcgggaggaa gtgcggcttg ttttcccggc taggctctgg agcggcgggc 60
   gcggcgcgat gcgcgggtac ccgggagcga acggctgcga gccctgatga agctcgagca 120
   gccccagcct gatggaggcg cctccgtggg agccggtgcg caatgactcc ctgcctccca 180
   cgctgagccc cgcggtgccg ccctacgtga agctcggcct caccgcggtc tacaccgtct 240
   tctacgcgct cctcttcgtg ttcatctatg cgcagctctg gctggtgctg cgctaccgtc 300
   acaagcggct cagctaccag agcgtcttcc tcttcctttg cctcttctgg gcctcgctgc 360
   gtaccgtgct cttctccttt tacttccgag acttcgtggc agccaactcg ttcagcccct 420
   tcgtcttctg gctgctctac tgcttccccg tgtgtctaca gttcttcacc ctcacgctca 480
   tgaacttgta cttcacgcag gtgattttca aggccaagtc aaaatattct ccagagctac 540
   tcaaataccg gttacccctc tacctggcct cacttttcat cagcctcgtt ttcctgttgg 600
   tgaatctgac ctgtgctgtg ctggtgaaga cgggagactg ggacaggaag gttatcgtct 660
   ctgtgagagt ggccatcaat gacacactct ttgtgctgtg tgctatctct ctctccatct 720
   gcctctacaa aatctccaag atgtccctgg ccaacatcta cttggagtca aagggctcat 780
   cagtgtgtca ggtaactgcc attggtgtca ccgtcatctt gctctacacc tctcgggcct 840
   gctacaacct gttcatcttg tcattttctc agatcaagaa cgtccattcc tttgattatg 900
   actggtacaa tgtatccgac caggcagatc tgaagagcca gctgggtgac gccggctacg 960
   tagtgtttgg cgtggtgctt ttcgtgtggg agctcctacc caccaccttg gtggtttatt 1020
   tcttccgagt cagaaatccc acgaaggatc ttaccaatcc tgggatggtc cccagccatg 1080
   gattcagtcc cagatcttac ttctttgaca acccccgaag atatgacagt gatgatgacc 1140
   ttgcctggaa cattgcccct cagggacttc agggaagttt tgctccagac tactatgatt 1200
   ggggacaaca aaataacagc ttcctggcac aagcaggaac tttgcatcaa gactccactt 1260
   tggatccaga caaagcaagc caagggtagc agcagctgac acagccctat ggaagagttc 1320
   tctgttgaaa gccttcagcc agacagaccg gatgacagct gagttgctaa ggcagttttc 1380
   cttaggaaac agaactctag tttttgctat agctttctca tggctccaca gggctaagca 1440
   ataatttaga gcaataaact ctttagtact agcagagaat ctggctattt cagtgggtat 1500
   aatttaaact tataaaagag gttctgtact tttataaaga tgtattttat ataacttaaa 1560
   tactaatgct aaagtatact aggtttttcc ttgattgtta attgcaacgt atgttgtagt 1620
   ttgcacagac tttcatgcat aattcacttt aaaacgtata gaatacgtgg tctaatagtt 1680
   taaagctttg gggaaagttt ccacaaatct tacctctgaa ggtccctctt gtgagtgcca 1740
   cgtggtgggc tcctttcacc gccactcaag cattccaagt tcaggagaag cagagtacca 1800
   tggtctgtac gtaacaggct caacagcagc agcagcagca gcagcagcag cagcaatttc 1860
   tcgtaaactc tgtcctaagc ctggtccttc ttcatctgaa agcactacta caagcactcc 1920
   agtaacaagt ggatactgtt aagatgtagt tgctgacact attaaacctc ctctgctgtg 1980
   tgtgtagcca ttttgtagag ttttcttcag cccggtgtaa ctgaatactc actacctcag 2040
   tacctcagta ggatgcaagg actgtgcctt ctttgactca gccagtgctt gctatagtca 2100
   ggctacaagc caagaggtcc ccacagagta ttaacaataa atacttctgg ccttcaagct 2160
   ctaaaggatt gcagactcct gacagctttt ctgtaagaca tgcctgtcat ttgtatgagg 2220
   ctgacacggg gctcactgcc tgttattttt agatagtgtt ctattaaaag ctatgtgtat 2280
   gagaaagtag gctctgccta cgtggcggca gcatccccat atcagccaga gagtgttcca 2340
   gacagtggtc ctcttgtgcc atctccgtgc tgtcctggga aaacgctgga acaacgtggc 2400
   cctcctggag gccggatgct ctggcttctt ctggtcctat aggtcacaga gccacgccca 2460
   ctttctccca ttttgttata tttaagattg ggagcccagt ttccagtggg ttgtaatggg 2520
   gtcttcgttg tacagaggac gacgtggagg aacttgcgca gccccgcccc acaccctgcc 2580
   tttgggtttg agtaaacatc tgggttgcaa gccatttaaa attgcttctt tgccagggtg 2640
   aattctggca aagcctacac aaatcgcctg tacgatagca ctgtataaaa gtttatctgt 2700
   caccatacct gcaatgattt ggctgttgca gccagtgccc acagctcctg tgtccttgtc 2760
   tctagcaggg cggtacctgt cagcagccaa gggcaaggct tgctggaaag cacagcctca 2820
   ttaaggctgt tctgtttgca cccattttgt agcacatgca cactttacag ttggtgaatg 2880
   ctgggcgtct gttttcttac agtaacaagc aagctatcat ccatttttac aataaagttg 2940
   tcagcattca tgtcagcaat aaaaagacta cagctcttaa aaaaaaaaaa aaaaaa 2996
<210> 24
   <211> 4762
   <212> DNA
   <213> Homo sapiens
<400> 24
   aagggaggga cctggaggac tcagggtagg attgtagagc tcagggacag ggagtggagg 60
   gcgccgcggt gagaccgctg ttgtgtagtt gttagactgg gcggggctgg gacgcaggcg 120
   ggacccgtgg ggacgcgagc ccgggcgcca aatgcccaga cagaggcgac cgcgggctcg 180
   gcttggccac gacctctcgg cacccttagg cctgtgcacc ctggctgtct gcccttggtc 240
   cccaacccac catgccctca cccgcgcctc ccaccgagct gctgccgtgg gagcgcgcgg 300
   tggtgctgct gtcgtgtgcg ctgtcagctc tgggctcggg cctcctggtg gccacgcacg 360
   ccctgtggcc tgacctgcgt agccgggcgc ggcgcctgct actcttcctg tcgctagcgg 420
   acctgctctc ggctgcctcg tacttctacg gagtgctgca ggactttgcg ggcacttcgt 480
   gggattgcgt ccttcagggc gctctctcta ctttcgccaa caccagctcc ttcttctgga 540
   cggtggccat cgccctctac ctatacctca gcatcgtccg aactacgcgc gggccctcca 600
   cggaccacct aatctgggct tttcacctca tcagctgggg tgtcccgttg gccatcacag 660
   tggcagccgt ctctctaaag aagataggct atgatgcctc ggatgtgtct gtgggctggt 720
   gctggatcaa cctggaggct gaggaccgtg tcctgtggat gctactgacc gggaagctgt 780
   gggagatgct ggcttatatc ttgctacctc tgctgtacct tttggtcaga aagcacatca 840
   acagggcgca ccaggcgctc tcggagtacc ggcccatctg cgaggggcgc cagctgcagc 900
   gaggctcctc cacttccacg gcagataaga agttggtcct cattccgctc atattcatct 960
   gcctccgcgt ctggagcacc gtgcgctttg tcctgacgct ctgtggttcc ccggctgtac 1020
   agacacccgt gctggttgtt ctgcatggca ttggaaacac cttccaggga ggggccaact 1080
   gcatcatgtt cgtcctctgt acccgggcag tccgcacaag gctcttttct ctttgctgct 1140
   gctgtcctcg gccctccacc cagagccctc cgggggctcc tacgcccccc aagataggag 1200
   aatctcagga atccagacgg accccagaag tgcccagcac ttgagcggtt ggctttcctc 1260
   cctgtccgta ctggcgctgc cttcctggtt cctgcttcag gattaggaga ccaagcatgc 1320
   tgtcagcctg gcctaaagtg aagatcggag cccagtggag ggacagccat cagtatggac 1380
   tcttctacct cccagacttg caggcaggca gtgtgttcct tgcacactca tgtcctggtt 1440
   cagtggggtt tgtttgtgta agcacaaaga ccgtgagact cagctatgcg ggataccgcc 1500
   agggcattgg ttctcagtgt ccccggccac caacgttgtt tacagttgga gggatccgta 1560
   cctgtggacc catcccaggc ttgactgaat aataatgaac accatcttgg atcccagaca 1620
   tcgcagtatg gagtccgggc ttgccagggc gttgagaatc cctagagacc ctgggggtaa 1680
   ttagcagtgc tggtcagctg agggtaatat ccttaaacta ggtcccaagt ccacggaagt 1740
   cacaggagac tgccatcatt gatgaaaacc acccgggcac acactcatgg gcatctcacg 1800
   gaatctctgc gtgggtcatc gcccctcagt gtttattaca tctgaatttc agcacaggcc 1860
   agagtccaga gtcctgtcca ttagtcaatc acttaacatc ctcgttccct ccaggtacca      1920
   ggacagaagg cagctcagct ccagccccta tggacaatta ccagttcacc tactgttctc      1980
   acagagccct ctcctcacgg tgactcatac agggggaggg ggtctggctc tgcactttgc 2040
   ttaggaaagg tgaccagtga atggatctga ttccaccctg aaggtgaatg tttgaagagt 2100
   cagcctcggg ctggcctgta gctccgtgta gtttccaggt tggtcctagc accccaaata 2160
   cttaaaaggc ctttgtgttt tgtagttatc tgggggatat agaaatagga ttctgagatg 2220
   attacatttt attttgtttt tttgagacag ggtctcatgt ggacatgctg gcctggaact 2280
   tgttatgtag atcaggctgg cctccaactc acagagatcc acctgcctct gcctcctgat 2340
   agttacattt ttaaaaaaat gttgtgttgt gttgtgcctg ttaggcaggt gctccgccac 2400
   tgagttcaat cccatgcccc tccccactcc cttttttttg agacataatc ttactaagtt 2460
   gcctagactg gccttcaact ctcagtcgac caggctggtc ttgaacttaa gattttccta 2520
   cctcaacttc ccaggaagct agaattgtcg cctttggcta gcaagcctgg ccagagcggg 2580
   gcttaactga gatctgctca gtggctcttc cacccacaca tccctcattt cagaaagcag 2640
   tgtgtgccga cgtgcaggct ctcggtgtgg gaagtgtgcc cgccaccttg cagtcaaggt 2700
   gaggtagcgc ccttggattc tgtcaatacc ctcacaaaac cgagaggatg ctcagagcct 2760
   ggctcaagat ggaagcagag tcacacgggg catcagcgcc catctctatc aggaaaggaa 2820
   tagtcgcgca gagggctgtg tctgtatgct cctcttctgg cttcctaaag catttcttta 2880
   ggaaactcta caagaggtgg gttccctttt gggagtctaa gaaagctcct tggcttggta 2940
   ctctgtctgc ttacaaaacc agtctgtgta gggtgtcctg agatcctgtg aagatattac 3000
   agccaatgtg tgcactgtca gcgtgtagcg cagcggtaat ggatctcctg gcttctaaaa 3060
   tggaacagaa attctaggaa tttgtctcct ggaggggaga aaataaacca ccaaatacta 3120
   cggaggaaat gatgttggat aaacccagct ggacagagca gtgtgaaacc ctggctgttt 3180
   ggggctttgt tcaaggaaag gcaggcaggg ggcatccgaa agccctgctc ctggagcctg      3240
   gggcttaccc aagttttctc tgatgccact ggccaggatt gctgtccgta aaacccccta      3300
   caaagtctac acttggctct ttccatctgt ttcccatgct gccctgtcac cgaaggtggc 3360
   tggtggtgac ccgaggcccg gtgcctgacc tctagcccta cgtgctcaca gacatcaacc 3420
   ttagctcagc cggtggccaa gtgggtcttc taggcagcca gggttcagag ctgaagagga 3480
   atagaagaat ccttggggcc aggaggggcc ttagcaccgg tgaggaccag ctcccctttg 3540
   ctttgctcag cctcgtgggt tcctatctct actaggaagt gtcattaata tctgtctgtc 3600
   cgtgttcctc tgtcacccaa atgtgacctc agccttaatg ggtgctacag cccccacacc 3660
   cttcttaagc actttatgaa tttttttttt ccgagacagg gtttctctgt atagccctgg 3720
   ctgtcctgga actcactctg tagaccaggc tggcctcgaa ctcagaaatc cgcctgcctc 3780
   tgcctcctga gtgctgggat taaagacgtg tgtcaccacc gcctggtaca ctttgtgaat 3840
   cttgttctgc aaatccgttg ccagctgctt cctgcctttg actttctgag gcagagagag 3900
   agagagggag agagggagag agggagagag agagggagag aatgtgtgtg tgcgtgagat 3960
   attatgctct agcatttatc tctcttcatt tttttttttt gttttgtttt ttaagtttcg 4020
   aaacagggtt ctcaggctgg agagatggct cagcggctaa gagcactgac tgctcttccg 4080
   aaggtcctga gttcaaattc cagcaaccac atggtggctc acaaccattt gtaatgagat 4140
   ctgatgccct cttctggtgt gtctgaggac agatacagtg tacttatata taataaataa 4200
   ataaatcgtt aatttttttt ttaaaaaaga atcagggttc tcactacttg tccaggcagg 4260
   ccttaagcag ttttactgtt tctgggatta caggcctatg ccaccccgag ccggcttttt 4320
   agtttgttct ctctgtctct ctgtctctct gtctctctct ctctctctct ctctctctct 4380
   ctctctctct ctctctctct ctttctctct ctatgtgtgt ctctttctct ctctgcctct 4440
   ctgtctgtct gtctctctct ctgcttctct ctctgaaaat aaaaataaaa actattatgt 4500
   gtccaaagta caaatgagac catatgtgtt tgtttgtatg tgtacgggta ttttgtgtgt 4560
   acgcagttcc cttagaggcc agagggactc tgatcccctg aatctggagt catagctgtg 4620
   aactctgggc aaagggaact aagcctgggt cctttgcagg aacagcaacg ctcttatctg 4680
   ttgagctagc tctccagccc tctctgcaaa tgtttgtgtg aactcacagt tgtggtataa 4740
   ttaaacctgg catgagattt tt 4762
<210> 25
   <211> 1919
   <212> DNA
   <213> Homo sapiens
<400> 25
   gcagtcatct gactcggtga ctcacccgcg gccgcgcttc ctctgatccg ggccgggcgg      60
   gaagtcgggt cccgaggctc cggctcggca gaccgggcgg aaagcagccg agcggccatg     120
   gagctgtgcg ggctggggct gccccggccg cccatgctgc tggcgctgct gttggcgaca     180
   ctgctggcgg cgatgttggc gctgctgact caggtggcgc tggtggtgca ggtggcggag     240
   gcggctcggg ccccgagcgt ctcggccaag ccggggccgg cgctgtggcc cctgccgctc     300
   tcggtgaaga tgaccccgaa cctgctgcat ctcgccccgg agaacttcta catcagccac     360
   agccccaatt ccacggcggg cccctcctgc accctgctgg aggaagcgtt tcgacgatat      420
   catggctata tttttggttt ctacaagtgg catcatgaac ctgctgaatt ccaggctaaa      480
   acccaggttc agcaacttct tgtctcaatc acccttcagt cagagtgtga tgctttcccc      540
   aacatatctt cagatgagtc ttatacttta cttgtgaaag aaccagtggc tgtccttaag      600
   gccaacagag tttggggagc attacgaggt ttagagacct ttagccagtt agtttatcaa      660
   gattcttatg gaactttcac catcaatgaa tccaccatta ttgattctcc aaggttttct      720
   cacagaggaa ttttgattga tacatccaga cattatctgc cagttaagat tattcttaaa      780
   actctggatg ccatggcttt taataagttt aatgttcttc actggcacat agttgatgac      840
   cagtctttcc catatcagag catcactttt cctgagttaa gcaataaagg aagctattct      900
   ttgtctcatg tttatacacc aaatgatgtc cgtatggtga ttgaatatgc cagattacga      960
   ggaattcgag tcctgccaga atttgatacc cctgggcata cactatcttg gggaaaaggt     1020
   cagaaagacc tcctgactcc atgttacagt agacaaaaca agttggactc ttttggacct     1080
   ataaacccta ctctgaatac aacatacagc ttccttacta catttttcaa agaaattagt     1140
   gaggtgtttc cagatcaatt cattcatttg ggaggagatg aagtggaatt taaatgttgg     1200
   gaatcaaatc caaaaattca agatttcatg aggcaaaaag gctttggcac agattttaag     1260
   aaactagaat ctttctacat tcaaaaggtt ttggatatta ttgcaaccat aaacaaggga     1320
   tccattgtct ggcaggaggt ttttgatgat aaagcaaagc ttgcgccggg cacaatagtt      1380
   gaagtatgga aagacagcgc atatcctgag gaactcagta gagtcacagc atctggcttc     1440
   cctgtaatcc tttctgctcc ttggtactta gatttgatta gctatggaca agattggagg     1500
   aaatactata aagtggaacc tcttgatttt ggcggtactc agaaacagaa acaacttttc     1560
   attggtggag aagcttgtct atggggagaa tatgtggatg caactaacct cactccaaga     1620
   ttatggcctc gggcaagtgc tgttggtgag agactctgga gttccaaaga tgtcagagat     1680
   atggatgacg cctatgacag actgacaagg caccgctgca ggatggtcga acgtggaata     1740
   gctgcacaac ctctttatgc tggatattgt aaccatgaga acatgtaaaa aatggagggg     1800
   aaaaaggcca cagcaatctg tactacaatc aactttattt tgaaatcatg taaaataaga     1860
   tattagactg ttttttgaat aaaatatttt tattgattga aaaaaaaaaa aaaaaaaaa      1919
<210> 26
   <211> 1428
   <212> DNA
   <213> Homo sapiens
<400> 26
   ggccggcggc atccgaggcg catgactagt tggggccaaa ccagtgctcc tgccacctct 60
   ctggctgccc cctagagcct gcccatccca gcctgaccaa tgtccacagc cagggagcag 120
   ccaatcttca gcacacgggc gcacgtgttc caaattgacc cagccaccaa gcgaaactgg 180
   atcccagcgg gcaagcacgc actcactgtc tcctatttct acgatgccac ccgcaatgtg 240
   taccgcatca tcagcatcgg aggcgccaag gccatcatca acagcactgt cactcccaac 300
   atgaccttca ccaaaacttc ccagaagttc gggcagtggg ccgacagtcg cgccaacaca 360
   gtctacggcc tgggctttgc ctctgaacag catctgacac agtttgccga gaagttccag 420
   gaagtgaagg aagcagccag gctggccagg gagaaatctc aggatggcgg ggagctcacc 480
   agtccagccc tggggctcgc ctcccaccag gtgcccccga gccctctcgt cagtgccaac 540
   ggccccggcg aggaaaaact gttccgcagc cagagcgctg atgcccccgg ccccacagag 600
   cgcgagcggc taaagaagat gttgtctgag ggctccgtgg gcgaggtaca gtgggaggcc 660
   gagtttttcg cactgcagga cagcaacaac aagctggcag gcgccctgcg agaggccaac 720
   gccgccgcag cccagtggag gcagcagctg gaggctcagc gtgcagaggc cgagcggctg 780
   cggcagcggg tggctgagct ggaggctcag gcagcttcag aggtgacccc caccggtgag 840
   aaggaggggc tgggccaggg ccagtcgctg gaacagctgg aagctctggt gcaaaccaag 900
   gaccaggaga ttcagaccct gaagagtcag actggggggc cccgcgaggc cctggaggct 960
   gccgagcgtg aggagactca gcagaaggtg cagacccgca atgcggagtt ggagcaccag 1020
   ctgcgggcga tggagcgcag cctggaggag gcacgggcag agcgggagcg ggcgcgggct 1080
   gaggtgggcc gggcagcgca gctgctggac gtcagcctgt ttgagctgag tgagctgcgt 1140
   gagggcctgg cccgcctggc tgaggctgcg ccctgagccg gggctggttt tctatgaacg 1200
   attccggcct gggatgcggg ccaggctgca ggcggcatag ttgggcccat tcgtcctgga 1260
   aagggactgg ggggtcccaa cttagccctg ggtgggccgg gccgggctgg gctggggtgg 1320
   gccccagtcg gctctggttg ttggcagctt tggggctgtt tttgagcttc tcattgtgta 1380
   gaatttctag atcccccgat tacatttcta agcgtggcaa aaaaaaaa 1428
<210> 27
   <211> 3249
   <212> DNA
   <213> Homo sapiens
<400> 27
   caagcttagc ctggccggga aacgggaggc gtggaggccg ggagcagccc ccggggtcat      60
   cgccctgcca ccgccgcccg attgctttag cttggaaatt ccggagctga agcggccagc     120
   gagggaggat gaccctctcg gcccgggcac cctgtcagtc cggaaataac tgcagcattt     180
   gttccggagg ggaaggcgcg aggtttccgg gaaagcagca ccgccccttg gcccccaggt    240
   ggctagcgct ataaaggatc acgcgcccca gtcgacgctg agctcctctg ctactcagag     300
   ttgcaacctc agcctcgcta tggctcccag cagcccccgg cccgcgctgc ccgcactcct     360
   ggtcctgctc ggggctctgt tcccaggacc tggcaatgcc cagacatctg tgtccccctc 420
   aaaagtcatc ctgccccggg gaggctccgt gctggtgaca tgcagcacct cctgtgacca 480
   gcccaagttg ttgggcatag agaccccgtt gcctaaaaag gagttgctcc tgcctgggaa 540
   caaccggaag gtgtatgaac tgagcaatgt gcaagaagat agccaaccaa tgtgctattc 600
   aaactgccct gatgggcagt caacagctaa aaccttcctc accgtgtact ggactccaga 660
   acgggtggaa ctggcacccc tcccctcttg gcagccagtg ggcaagaacc ttaccctacg 720
   ctgccaggtg gagggtgggg caccccgggc caacctcacc gtggtgctgc tccgtgggga 780
   gaaggagctg aaacgggagc cagctgtggg ggagcccgct gaggtcacga ccacggtgct 840
   ggtgaggaga gatcaccatg gagccaattt ctcgtgccgc actgaactgg acctgcggcc 900
   ccaagggctg gagctgtttg agaacacctc ggccccctac cagctccaga cctttgtcct 960
   gccagcgact cccccacaac ttgtcagccc ccgggtccta gaggtggaca cgcaggggac 1020
   cgtggtctgt tccctggacg ggctgttccc agtctcggag gcccaggtcc acctggcact 1080
   gggggaccag aggttgaacc ccacagtcac ctatggcaac gactccttct cggccaaggc 1140
   ctcagtcagt gtgaccgcag aggacgaggg cacccagcgg ctgacgtgtg cagtaatact 1200
   ggggaaccag agccaggaga cactgcagac agtgaccatc tacagctttc cggcgcccaa 1260
   cgtgattctg acgaagccag aggtctcaga agggaccgag gtgacagtga agtgtgaggc 1320
   ccaccctaga gccaaggtga cgctgaatgg ggttccagcc cagccactgg gcccgagggc 1380
   ccagctcctg ctgaaggcca ccccagagga caacgggcgc agcttctcct gctctgcaac 1440
   cctggaggtg gccggccagc ttatacacaa gaaccagacc cgggagcttc gtgtcctgta 1500
   tggcccccga ctggacgaga gggattgtcc gggaaactgg acgtggccag aaaattccca 1560
   gcagactcca atgtgccagg cttgggggaa cccattgccc gagctcaagt gtctaaagga 1620
   tggcactttc ccactgccca tcggggaatc agtgactgtc actcgagatc ttgagggcac 1680
   ctacctctgt cgggccagga gcactcaagg ggaggtcacc cgcaaggtga ccgtgaatgt 1740
   gctctccccc cggtatgaga ttgtcatcat cactgtggta gcagccgcag tcataatggg 1800
   cactgcaggc ctcagcacgt acctctataa ccgccagcgg aagatcaaga aatacagact 1860
   acaacaggcc caaaaaggga cccccatgaa accgaacaca caagccacgc ctccctgaac 1920
   ctatcccggg acagggcctc ttcctcggcc ttcccatatt ggtggcagtg gtgccacact 1980
   gaacagagtg gaagacatat gccatgcagc tacacctacc ggccctggga cgccggagga 2040
   cagggcattg tcctcagtca gatacaacag catttggggc catggtacct gcacacctaa 2100
   aacactaggc cacgcatctg atctgtagtc acatgactaa gccaagagga aggagcaaga 2160
   ctcaagacat gattgatgga tgttaaagtc tagcctgatg agaggggaag tggtggggga 2220
   gacatagccc caccatgagg acatacaact gggaaatact gaaacttgct gcctattggg 2280
   tatgctgagg ccccacagac ttacagaaga agtggccctc catagacatg tgtagcatca 2340
   aaacacaaag gcccacactt cctgacggat gccagcttgg gcactgctgt ctactgaccc 2400
   caacccttga tgatatgtat ttattcattt gttattttac cagctattta ttgagtgtct 2460
   tttatgtagg ctaaatgaac ataggtctct ggcctcacgg agctcccagt cctaatcaca 2520
   ttcaaggtca ccaggtacag ttgtacaggt tgtacactgc aggagagtgc ctggcaaaaa 2580
   gatcaaatgg ggctgggact tctcattggc caacctgcct ttccccagaa ggagtgattt 2640
   ttctatcggc acaaaagcac tatatggact ggtaatggtt acaggttcag agattaccca 2700
   gtgaggcctt attcctccct tccccccaaa actgacacct ttgttagcca cctccccacc 2760
   cacatacatt tctgccagtg ttcacaatga cactcagcgg tcatgtctgg acatgagtgc 2820
   ccagggaata tgcccaagct atgccttgtc ctcttgtcct gtttgcattt cactgggagc 2880
   ttgcactatg cagctccagt ttcctgcagt gatcagggtc ctgcaagcag tggggaaggg 2940
   ggccaaggta ttggaggact ccctcccagc tttggaagcc tcatccgcgt gtgtgtgtgt 3000
   gtgtatgtgt agacaagctc tcgctctgtc acccaggctg gagtgcagtg gtgcaatcat 3060
   ggttcactgc agtcttgacc ttttgggctc aagtgatcct cccacctcag cctcctgagt 3120
   agctgggacc ataggctcac aacaccacac ctggcaaatt tgattttttt tttttttcca 3180
   gagacggggt ctcgcaacat tgcccagact tcctttgtgt tagttaataa agctttctca 3240
   actgccaaa 3249
<210> 28
   <211> 4909
   <212> DNA
   <213> Homo sapiens
<400> 28
   tagacgcacc ctctgaagat ggtgactccc tcctgagaag ctggacccct tggtaaaaga        60
   caaggccttc tccaagaaga atatgaaagt gttactcaga cttatttgtt tcatagctct       120
   actgatttct tctctggagg ctgataaatg caaggaacgt gaagaaaaaa taattttagt       180
   gtcatctgca aatgaaattg atgttcgtcc ctgtcctctt aacccaaatg aacacaaagg       240
   cactataact tggtataaag atgacagcaa gacacctgta tctacagaac aagcctccag       300
   gattcatcaa cacaaagaga aactttggtt tgttcctgct aaggtggagg attcaggaca       360
   ttactattgc gtggtaagaa attcatctta ctgcctcaga attaaaataa gtgcaaaatt       420
   tgtggagaat gagcctaact tatgttataa tgcacaagcc atatttaagc agaaactacc       480
   cgttgcagga gacggaggac ttgtgtgccc ttatatggag ttttttaaaa atgaaaataa       540
   tgagttacct aaattacagt ggtataagga ttgcaaacct ctacttcttg acaatataca       600
   ctttagtgga gtcaaagata ggctcatcgt gatgaatgtg gctgaaaagc atagagggaa 660
   ctatacttgt catgcatcct acacatactt gggcaagcaa tatcctatta cccgggtaat 720
   agaatttatt actctagagg aaaacaaacc cacaaggcct gtgattgtga gcccagctaa 780
   tgagacaatg gaagtagact tgggatccca gatacaattg atctgtaatg tcaccggcca 840
   gttgagtgac attgcttact ggaagtggaa tgggtcagta attgatgaag atgacccagt 900
   gctaggggaa gactattaca gtgtggaaaa tcctgcaaac aaaagaagga gtaccctcat 960
   cacagtgctt aatatatcgg aaattgaaag tagattttat aaacatccat ttacctgttt 1020
   tgccaagaat acacatggta tagatgcagc atatatccag ttaatatatc cagtcactaa 1080
   tttccagaag cacatgattg gtatatgtgt cacgttgaca gtcataattg tgtgttctgt 1140
   tttcatctat aaaatcttca agattgacat tgtgctttgg tacagggatt cctgctatga 1200
   ttttctccca ataaaagctt cagatggaaa gacctatgac gcatatatac tgtatccaaa 1260
   gactgttggg gaagggtcta cctctgactg tgatattttt gtgtttaaag tcttgcctga 1320
   ggtcttggaa aaacagtgtg gatataagct gttcatttat ggaagggatg actacgttgg 1380
   ggaagacatt gttgaggtca ttaatgaaaa cgtaaagaaa agcagaagac tgattatcat 1440
   tttagtcaga gaaacatcag gcttcagctg gctgggtggt tcatctgaag agcaaatagc 1500
   catgtataat gctcttgttc aggatggaat taaagttgtc ctgcttgagc tggagaaaat 1560
   ccaagactat gagaaaatgc cagaatcgat taaattcatt aagcagaaac atggggctat 1620
   ccgctggtca ggggacttta cacagggacc acagtctgca aagacaaggt tctggaagaa 1680
   tgtcaggtac cacatgccag tccagcgacg gtcaccttca tctaaacacc agttactgtc 1740
   accagccact aaggagaaac tgcaaagaga ggctcacgtg cctctcgggt agcatggaga 1800
   agttgccaag agttctttag gtgcctcctg tcttatggcg ttgcaggcca ggttatgcct 1860
   catgctgact tgcagagttc atggaatgta actatatcat cctttatccc tgaggtcacc 1920
   tggaatcaga ttattaaggg aataagccat gacgtcaata gcagcccagg gcacttcaga 1980
   gtagagggct tgggaagatc ttttaaaaag gcagtaggcc cggtgtggtg gctcacgcct 2040
   ataatcccag cactttggga ggctgaagtg ggtggatcac cagaggtcag gagttcgaga 2100
   ccagcccagc caacatggca aaaccccatc tctactaaaa atacaaaaat gagctaggca 2160
   tggtggcaca cgcctgtaat cccagctaca cctgaggctg aggcaggaga attgcttgaa 2220
   ccggggagac ggaggttgca gtgagccgag tttgggccac tgcactctag cctggcaaca 2280
   gagcaagact ccgtctcaaa aaaagggcaa taaatgccct ctctgaatgt ttgaactgcc 2340
   aagaaaaggc atggagacag cgaactagaa gaaagggcaa gaaggaaata gccaccgtct 2400
   acagatggct tagttaagtc atccacagcc caagggcggg gctatgcctt gtctggggac 2460
   cctgtagagt cactgaccct ggagcggctc tcctgagagg tgctgcaggc aaagtgagac 2520
   tgacacctca ctgaggaagg gagacatatt cttggagaac tttccatctg cttgtatttt 2580
   ccatacacat ccccagccag aagttagtgt ccgaagaccg aattttattt tacagagctt 2640
   gaaaactcac ttcaatgaac aaagggattc tccaggattc caaagttttg aagtcatctt 2700
   agctttccac aggagggaga gaacttaaaa aagcaacagt agcagggaat tgatccactt 2760
   cttaatgctt tcctccctgg catgaccatc ctgtcctttg ttattatcct gcattttacg 2820
   tctttggagg aacagctccc tagtggcttc ctccgtctgc aatgtccctt gcacagccca 2880
   cacatgaacc atccttccca tgatgccgct cttctgtcat cccgctcctg ctgaaacacc 2940
   tcccaggggc tccacctgtt caggagctga agcccatgct ttcccaccag catgtcactc 3000
   ccagaccacc tccctgccct gtcctccagc ttcccctcgc tgtcctgctg tgtgaattcc 3060
   caggttggcc tggtggccat gtcgcctgcc cccagcactc ctctgtctct gctcttgcct 3120
   cgacccttcc tcctcctttg cctaggaggc cttctcgcat tttctctagc tgatcagaat 3180
   tttaccaaaa ttcagaacat cctccaattc cacagtctct gggagacttt ccctaagagg 3240
   cgacttcctc tccagccttc tctctctggt caggcccact gcagagatgg tggtgagcac 3300
   atctgggagg ctggtctccc tccagctgga attgctgctc tctgagggag aggctgtggt 3360
   ggctgtctct gtccctcact gccttccagg agcaatttgc acatgtaaca tagatttatg 3420
   taatgcttta tgtttaaaaa cattccccaa ttatcttatt taatttttgc aattattcta 3480
   attttatata tagagaaagt gacctatttt ttaaaaaaat cacactctaa gttctattga 3540
   acctaggact tgagcctcca tttctggctt ctagtctggt gttctgagta cttgatttca 3600
   ggtcaataac ggtcccccct cactccacac tggcacgttt gtgagaagaa atgacatttt 3660
   gctaggaagt gaccgagtct aggaatgctt ttattcaaga caccaaattc caaacttcta 3720
   aatgttggaa ttttcaaaaa ttgtgtttag attttatgaa aaactcttct actttcatct 3780
   attctttccc tagaggcaaa catttcttaa aatgtttcat tttcattaaa aatgaaagcc 3840
   aaatttatat gccaccgatt gcaggacaca agcacagttt taagagttgt atgaacatgg 3900
   agaggacttt tggtttttat atttctcgta tttaatatgg gtgaacacca acttttattt 3960
   ggaataataa ttttcctcct aaacaaaaac acattgagtt taagtctctg actcttgcct 4020
   ttccacctgc tttctcctgg gcccgctttg cctgcttgaa ggaacagtgc tgttctggag 4080
   ctgctgttcc aacagacagg gcctagcttt catttgacac acagactaca gccagaagcc 4140
   catggagcag ggatgtcacg tcttgaaaag cctattagat gttttacaaa tttaattttg 4200
   cagattattt tagtctgtca tccagaaaat gtgtcagcat gcatagtgct aagaaagcaa 4260
   gccaatttgg aaacttaggt tagtgacaaa attggccaga gagtgggggt gatgatgacc 4320
   aagaattaca agtagaatgg cagctggaat ttaaggaggg acaagaatca atggataagc 4380
   gtgggtggag gaagatccaa acagaaaagt gcaaagttat tccccatctt ccaagggttg 4440
   aattctggag gaagaagaca cattcctagt tccccgtgaa cttcctttga cttattgtcc 4500
   ccactaaaac aaaacaaaaa acttttaatg ccttccacat taattagatt ttcttgcagt 4560
   ttttttatgg cattttttta aagatgccct aagtgttgaa gaagagtttg caaatgcaac 4620
   aaaatattta attaccggtt gttaaaactg gtttagcaca atttatattt tccctctctt 4680
   gcctttctta tttgcaataa aaggtattga gccatttttt aaatgacatt tttgataaat 4740
   tatgtttgta ctagttgatg aaggagtttt ttttaacctg tttatataat tttgcagcag 4800
   aagccaaatt ttttgtatat taaagcacca aattcatgta cagcatgcat cacggatcaa 4860
   tagactgtac ttattttcca ataaaatttt caaactttgt actgttaaa 4909
<210> 29
   <211> 3499
   <212> DNA
   <213> Homo sapiens
<400> 29
   cgcggacccg gccggcccag gcccgcgccc gccgcggccc tgagaggccc cggcaggtcc        60
   cggcccggcg gcggcagcca tggccggggg gccgggcccg ggggagcccg cagcccccgg       120
   cgcccagcac ttcttgtacg aggtgccgcc ctgggtcatg tgccgcttct acaaagtgat       180
   ggacgccctg gagcccgccg actggtgcca gttcgccgcc ctgatcgtgc gcgaccagac       240
   cgagctgcgg ctgtgcgagc gctccgggca gcgcacggcc agcgtcctgt ggccctggat       300
   caaccgcaac gcccgtgtgg ccgacctcgt gcacatcctc acgcacctgc agctgctccg       360
   tgcgcgggac atcatcacag cctggcaccc tcccgccccg cttccgtccc caggcaccac       420
   tgccccgagg cccagcagca tccctgcacc cgccgaggcc gaggcctgga gcccccggaa       480
   gttgccatcc tcagcctcca ccttcctctc cccagctttt ccaggctccc agacccattc       540
   agggcctgag ctcggcctgg tcccaagccc tgcttccctg tggcctccac cgccatctcc       600
   agccccttct tctaccaagc caggcccaga gagctcagtg tccctcctgc agggagcccg       660
   cccctttccg ttttgctggc ccctctgtga gatttcccgg ggcacccaca acttctcgga        720
   ggagctcaag atcggggagg gtggctttgg gtgcgtgtac cgggcggtga tgaggaacac       780
   ggtgtatgct gtgaagaggc tgaaggagaa cgctgacctg gagtggactg cagtgaagca       840
   gagcttcctg accgaggtgg agcagctgtc caggtttcgt cacccaaaca ttgtggactt       900
   tgctggctac tgtgctcaga acggcttcta ctgcctggtg tacggcttcc tgcccaacgg       960
   ctccctggag gaccgtctcc actgccagac ccaggcctgc ccacctctct cctggcctca      1020
   gcgactggac atccttctgg gtacagcccg ggcaattcag tttctacatc aggacagccc      1080
   cagcctcatc catggagaca tcaagagttc caacgtcctt ctggatgaga ggctgacacc      1140
   caagctggga gactttggcc tggcccggtt cagccgcttt gccgggtcca gccccagcca     1200
   gagcagcatg gtggcccgga cacagacagt gcggggcacc ctggcctacc tgcccgagga     1260
   gtacatcaag acgggaaggc tggctgtgga cacggacacc ttcagctttg gggtggtagt     1320
   gctagagacc ttggctggtc agagggctgt gaagacgcac ggtgccagga ccaagtatct     1380
   gaaagacctg gtggaagagg aggctgagga ggctggagtg gctttgagaa gcacccagag     1440
   cacactgcaa gcaggtctgg ctgcagatgc ctgggctgct cccatcgcca tgcagatcta     1500
   caagaagcac ctggacccca ggcccgggcc ctgcccacct gagctgggcc tgggcctggg     1560
   ccagctggcc tgctgctgcc tgcaccgccg ggccaaaagg aggcctccta tgacccagga     1620
   gaactcctac gtgtccagca ctggcagagc ccacagtggg gctgctccat ggcagcccct     1680
   ggcagcgcca tcaggagcca gtgcccaggc agcagagcag ctgcagagag gccccaacca     1740
   gcccgtggag agtgacgaga gcctaggcgg cctctctgct gccctgcgct cctggcactt     1800
   gactccaagc tgccctctgg acccagcacc cctcagggag gccggctgtc ctcaggggga     1860
   cacggcagga gaatcgagct gggggagtgg cccaggatcc cggcccacag ccgtggaagg     1920
   actggccctt ggcagctctg catcatcgtc gtcagagcca ccgcagatta tcatcaaccc     1980
   tgcccgacag aagatggtcc agaagctggc cctgtacgag gatggggccc tggacagcct     2040
   gcagctgctg tcgtccagct ccctcccagg cttgggcctg gaacaggaca ggcaggggcc     2100
   cgaagaaagt gatgaatttc agagctgatg tgttcacctg ggcagatccc ccaaatccgg     2160
   aagtcaaagt tctcatggtc agaagttctc atggtgcacg agtcctcagc actctgccgg     2220
   cagtgggggt gggggcccat gcccgcgggg gagagaagga ggtggccctg ctgttctagg     2280
   ctctgtgggc ataggcaggc agagtggaac cctgcctcca tgccagcatc tgggggcaag     2340
   gaaggctggc atcatccagt gaggaggctg gcgcatgttg ggaggctgct ggctgcacag     2400
   acccgtgagg ggaggagagg ggctgctgtg caggggtgtg gagtagggag ctggctcccc     2460
   tgagagccat gcagggcgtc tgcagcccag gcctctggca gcagctcttt gcccatctct     2520
   ttggacagtg gccaccctgc acaatggggc cgacgaggcc tagggccctc ctacctgctt     2580
   acaatttgga aaagtgtggc cgggtgcggt ggctcacgcc tgtaatccca gcactttggg     2640
   aggccaaggc aggaggatcg ctggagccca gtaggtcaag accagccagg gcaacatgat     2700
   gagaccctgt ctctgccaaa aaatttttta aactattagc ctggcgtggt agcgcacgcc     2760
   tgtggtccca gctgctgggg aggctgaagt aggaggatca tttatgcttg ggaggtcgag     2820
   gctgcagtga gtcatgattg tatgactgca ctccagcctg ggtgacagag caagaccctg     2880
   tttcaaaaag aaaaaccctg ggaaaagtga agtatggctg taagtctcat ggttcagtcc     2940
   tagcaagaag cgagaattct gagatcctcc agaaagtcga gcagcaccca cctccaacct     3000
   cgggccagtg tcttcaggct ttactgggga cctgcgagct ggcctaatgt ggtggcctgc     3060
   aagccaggcc atccctgggc gccacagacg agctccgagc caggtcaggc ttcggaggcc     3120
   acaagctcag cctcaggccc aggcactgat tgtggcagag gggccactac ccaaggtcta     3180
   gctaggccca agacctagtt acccagacag tgagaagccc ctggaaggca gaaaagttgg     3240
   gagcatggca gacagggaag ggaaacattt tcagggaaaa gacatgtatc acatgtcttc     3300
   agaagcaagt caggtttcat gtaaccgagt gtcctcttgc gtgtccaaaa gtagcccagg     3360
   gctgtagcac aggcttcaca gtgattttgt gttcagccgt gagtcacact acatgccccc     3420
   gtgaagctgg gcattggtga cgtccaggtt gtccttgagt aataaaaacg tatgttgcaa     3480
   taaaaaaaaa aaaaaaaaa                                                  3499
<210> 30
   <211> 4267
   <212> DNA
   <213> Homo sapiens
<400> 30
   attcgcctct gggaggttta ggaagcggct ccgggtcggt ggccccagga cagggaagag      60
   cgggcgctat ggggagccgg acgccagagt cccctctcca cgccgtgcag ctgcgctggg     120
   gcccccggcg ccgacccccg ctgctgccgc tgctgttgct gctgctgccg ccgccaccca     180
   gggtcggggg cttcaactta gacgcggagg ccccagcagt actctcgggg cccccgggct     240
   ccttcttcgg attctcagtg gagttttacc ggccgggaac agacggggtc agtgtgctgg     300
   tgggagcacc caaggctaat accagccagc caggagtgct gcagggtggt gctgtctacc     360
   tctgtccttg gggtgccagc cccacacagt gcacccccat tgaatttgac agcaaaggct     420
   ctcggctcct ggagtcctca ctgtccagct cagagggaga ggagcctgtg gagtacaagt     480
   ccttgcagtg gttcggggca acagttcgag cccatggctc ctccatcttg gcatgcgctc     540
   cactgtacag ctggcgcaca gagaaggagc cactgagcga ccccgtgggc acctgctacc     600
   tctccacaga taacttcacc cgaattctgg agtatgcacc ctgccgctca gatttcagct     660
   gggcagcagg acagggttac tgccaaggag gcttcagtgc cgagttcacc aagactggcc     720
   gtgtggtttt aggtggacca ggaagctatt tctggcaagg ccagatcctg tctgccactc     780
   aggagcagat tgcagaatct tattaccccg agtacctgat caacctggtt caggggcagc     840
   tgcagactcg ccaggccagt tccatctatg atgacagcta cctaggatac tctgtggctg     900
   ttggtgaatt cagtggtgat gacacagaag actttgttgc tggtgtgccc aaagggaacc     960
   tcacttacgg ctatgtcacc atccttaatg gctcagacat tcgatccctc tacaacttct    1020
   caggggaaca gatggcctcc tactttggct atgcagtggc cgccacagac gtcaatgggg    1080
   acgggctgga tgacttgctg gtgggggcac ccctgctcat ggatcggacc cctgacgggc    1140
   ggcctcagga ggtgggcagg gtctacgtct acctgcagca cccagccggc atagagccca    1200
   cgcccaccct taccctcact ggccatgatg agtttggccg atttggcagc tccttgaccc 1260
   ccctggggga cctggaccag gatggctaca atgatgtggc catcggggct ccctttggtg 1320
   gggagaccca gcagggagta gtgtttgtat ttcctggggg cccaggaggg ctgggctcta 1380
   agccttccca ggttctgcag cccctgtggg cagccagcca caccccagac ttctttggct 1440
   ctgcccttcg aggaggccga gacctggatg gcaatggata tcctgatctg attgtggggt 1500
   cctttggtgt ggacaaggct gtggtataca ggggccgccc catcgtgtcc gctagtgcct 1560
   ccctcaccat cttccccgcc atgttcaacc cagaggagcg gagctgcagc ttagagggga 1620
   accctgtggc ctgcatcaac cttagcttct gcctcaatgc ttctggaaaa cacgttgctg 1680
   actccattgg tttcacagtg gaacttcagc tggactggca gaagcagaag ggaggggtac 1740
   ggcgggcact gttcctggcc tccaggcagg caaccctgac ccagaccctg ctcatccaga 1800
   atggggctcg agaggattgc agagagatga agatctacct caggaacgag tcagaatttc 1860
   gagacaaact ctcgccgatt cacatcgctc tcaacttctc cttggacccc caagccccag 1920
   tggacagcca cggcctcagg ccagccctac attatcagag caagagccgg atagaggaca 1980
   aggctcagat cttgctggac tgtggagaag acaacatctg tgtgcctgac ctgcagctgg 2040
   aagtgtttgg ggagcagaac catgtgtacc tgggtgacaa gaatgccctg aacctcactt 2100
   tccatgccca gaatgtgggt gagggtggcg cctatgaggc tgagcttcgg gtcaccgccc 2160
   ctccagaggc tgagtactca ggactcgtca gacacccagg gaacttctcc agcctgagct 2220
   gtgactactt tgccgtgaac cagagccgcc tgctggtgtg tgacctgggc aaccccatga 2280
   aggcaggagc cagtctgtgg ggtggccttc ggtttacagt ccctcatctc cgggacacta 2340
   agaaaaccat ccagtttgac ttccagatcc tcagcaagaa tctcaacaac tcgcaaagcg 2400
   acgtggtttc ctttcggctc tccgtggagg ctcaggccca ggtcaccctg aacggtgtct 2460
   ccaagcctga ggcagtgcta ttcccagtaa gcgactggca tccccgagac cagcctcaga 2520
   aggaggagga cctgggacct gctgtccacc atgtctatga gctcatcaac caaggcccca 2580
   gctccattag ccagggtgtg ctggaactca gctgtcccca ggctctggaa ggtcagcagc 2640
   tcctatatgt gaccagagtt acgggactca actgcaccac caatcacccc attaacccaa 2700
   agggcctgga gttggatccc gagggttccc tgcaccacca gcaaaaacgg gaagctccaa 2760
   gccgcagctc tgcttcctcg ggacctcaga tcctgaaatg cccggaggct gagtgtttca 2820
   ggctgcgctg tgagctcggg cccctgcacc aacaagagag ccaaagtctg cagttgcatt 2880
   tccgagtctg ggccaagact ttcttgcagc gggagcacca gccatttagc ctgcagtgtg 2940
   aggctgtgta caaagccctg aagatgccct accgaatcct gcctcggcag ctgccccaaa 3000
   aagagcgtca ggtggccaca gctgtgcaat ggaccaaggc agaaggcagc tatggcgtcc 3060
   cactgtggat catcatccta gccatcctgt ttggcctcct gctcctaggt ctactcatct     3120
   acatcctcta caagcttgga ttcttcaaac gctccctccc atatggcacc gccatggaaa     3180
   aagctcagct caagcctcca gccacctctg atgcctgagt cctcccaatt tcagactccc     3240
   attcctgaag aaccagtccc cccaccctca ttctactgaa aaggaggggt ctgggtactt     3300
   cttgaaggtg ctgacggcca gggagaagct cctctcccca gcccagagac atacttgaag     3360
   ggccagagcc aggggggtga ggagctgggg atccctcccc cccatgcact gtgaaggacc     3420
   cttgtttaca cataccctct tcatggatgg gggaactcag atccagggac agaggcccca     3480
   gcctccctga agcctttgca ttttggagag tttcctgaaa caacttggaa agataactag     3540
   gaaatccatt cacagttctt tgggccagac atgccacaag gacttcctgt ccagctccaa     3600
   cctgcaaaga tctgtcctca gccttgccag agatccaaaa gaagccccca gctaagaacc     3660
   tggaacttgg ggagttaaga cctggcagct ctggacagcc ccaccctggt gggccaacaa     3720
   agaacactaa ctatgcatgg tgccccagga ccagctcagg acagatgcca cacaaggata     3780
   gatgctggcc cagggcccag agcccagctc caaggggaat cagaactcaa atggggccag     3840
   atccagcctg gggtctggag ttgatctgga acccagactc agacattggc acctaatcca     3900
   ggcagatcca ggactatatt tgggcctgct ccagacctga tcctggaggc ccagttcacc     3960
   ctgatttagg agaagccagg aatttcccag gaccctgaag gggccatgat ggcaacagat     4020
   ctggaacctc agcctggcca gacacaggcc ctccctgttc cccagagaaa ggggagccca     4080
   ctgtcctggg cctgcagaat ttgggttctg cctgccagct gcactgatgc tgcccctcat     4140
   ctctctgccc aacccttccc tcaccttggc accagacacc caggacttat ttaaactctg     4200
   ttgcaagtgc aataaatctg acccagtgcc cccactgacc agaactagaa aaaaaaaaaa     4260
   aaaaaaa                                                               4267
<210> 31
   <211> 8787
   <212> DNA
   <213> Homo sapiens
<400> 31
   ggcgggtgct tctagggcgc tcccagagcc gcctccccct gttgctggca tcccgagctt      60
   cctcccttgc cagccaggac gctgccgact tgtctttgcc cgctgctccg cagacggggc     120
   tgcaaagctg caactaatgg tgttggcctc cctgcccacc tgtggaagca actgcgctga     180
   ttgatgcgcc acagactttt ttcccctcga cctcgccggc gtcccctccc acagatccag     240
   catcacccag tgaatgtaca ttagggtggt ttccccccca gcttcgggct ttgtttgggt     300
   ttgattgtgt ttggctcttc gctaagctga tttatgcagc agaagcccca ccggctggag     360
   agaaacaaaa gctcttttct ttgtcccgga gcaggctgcg gagcccttgc agagccctct     420
   ctccagtcgc cgccggggcc cttggccgtc gaaggaggtg cttctcgcgg agaccgcggg 480
   acccgccgtg ccgagccggg agggccgcag gggccctgag atgccgagcg gtgcccgggc 540
   ccgcttacct gcaccgcttg ctccgagccg cggggtccgc ctgctaggcc tgcggaaaac 600
   gtcctagcga cactcggccc gcgggccccg aggtgcgccc gggaggcgcg agcccgcgtc 660
   cggaaggcag tcaggcggcg ggcgcggggc gggctgtttt gcattatgtg cggctcggcc 720
   ctggcttttt ttaccgctgc atttgtctgc ctgcaaaacg accggcgagg tcccgcctcg 780
   ttcctctggg cagcctgggt gttttcactt gttcttggac tgggccaagg tgaagacaat 840
   agatgtgcat cttcaaatgc agcatcctgt gccaggtgcc ttgcgctggg tccagaatgt 900
   ggatggtgtg ttcaagagga tttcatttca ggtggatcaa gaagtgaacg ttgtgatatt 960
   gtttccaatt taataagcaa aggctgctca gttgattcaa tagaataccc atctgtgcat 1020
   gttataatac ccactgaaaa tgaaattaat acccaggtga caccaggaga agtgtctatc 1080
   cagctgcgtc caggagccga agctaatttt atgctgaaag ttcatcctct gaagaaatat 1140
   cctgtggatc tttattatct tgttgatgtc tcagcatcaa tgcacaataa tatagaaaaa 1200
   ttaaattccg ttggaaacga tttatctaga aaaatggcat ttttctcccg tgactttcgt 1260
   cttggatttg gctcatacgt tgataaaaca gtttcaccat acattagcat ccaccccgaa 1320
   aggattcata atcaatgcag tgactacaat ttagactgca tgcctcccca tggatacatc 1380
   catgtgctgt ctttgacaga gaacatcact gagtttgaga aagcagttca tagacagaag 1440
   atctctggaa acatagatac accagaagga ggttttgacg ccatgcttca ggcagctgtc 1500
   tgtgaaagtc atatcggatg gcgaaaagag gctaaaagat tgctgctggt gatgacagat 1560
   cagacgtctc atctcgctct tgatagcaaa ttggcaggca tagtggtgcc caatgacgga 1620
   aactgtcatc tgaaaaacaa cgtctatgtc aaatcgacaa ccatggaaca cccctcacta 1680
   ggccaacttt cagagaaatt aatagacaac aacattaatg tcatctttgc agttcaagga 1740
   aaacaatttc attggtataa ggatcttcta cccctcttgc caggcaccat tgctggtgaa 1800
   atagaatcaa aggctgcaaa cctcaataat ttggtagtgg aagcctatca gaagctcatt 1860
   tcagaagtga aagttcaggt ggaaaaccag gtacaaggca tctattttaa cattaccgcc 1920
   atctgtccag atgggtccag aaagccaggc atggaaggat gcagaaacgt gacgagcaat 1980
   gatgaagttc ttttcaatgt aacagttaca atgaaaaaat gtgatgtcac aggaggaaaa 2040
   aactatgcaa taatcaaacc tattggtttt aatgaaaccg ctaaaattca tatacacaga 2100
   aactgcagct gtcagtgtga ggacaacaga ggacctaaag gaaagtgtgt agatgaaact 2160
   tttctagatt ccaagtgttt ccagtgtgat gagaataaat gtcattttga tgaagatcag 2220
   ttttcttctg agagttgcaa gtcacacaag gatcagcctg tttgcagtgg tcgaggagtt 2280
   tgtgtttgtg ggaaatgttc atgtcacaaa attaagcttg gaaaagtgta tggaaaatac 2340
   tgtgaaaagg atgacttttc ttgtccatat caccatggaa atctgtgtgc tgggcatgga 2400
   gagtgtgaag caggcagatg ccaatgcttc agtggctggg aaggtgatcg atgccagtgc 2460
   ccttcagcag cagcccagca ctgtgtcaat tcaaagggcc aagtgtgcag tggaagaggc 2520
   acgtgtgtgt gtggaaggtg tgagtgcacc gatcccagga gcatcggccg cttctgtgaa 2580
   cactgcccca cctgttatac agcctgcaag gaaaactgga attgtatgca atgccttcac 2640
   cctcacaatt tgtctcaggc tatacttgat cagtgcaaaa cctcatgtgc tctcatggaa 2700
   caacagcatt atgtcgacca aacttcagaa tgtttctcca gcccaagcta cttgagaata 2760
   tttttcatca ttttcatagt tacattcttg attgggttgc ttaaagtcct gatcattaga 2820
   caggtgatac tacaatggaa tagtaataaa attaagtcct catcagatta cagagtgtca 2880
   gcctcaaaaa aggataagtt gattctgcaa agtgtttgca caagagcagt cacctaccga 2940
   cgtgagaagc ctgaagaaat aaaaatggat atcagcaaat taaatgctca tgaaactttc 3000
   aggtgcaact tctaaaaaaa gatttttaaa cacttaatgg gaaactggaa ttgttaataa 3060
   ttgctcctaa agattataat tttaaaagtc acaggaggag acaaattgct cacggtcatg 3120
   ccagttgctg gttgtacact cgaacgaaga ctgacaagta tcctcatcat gatgtgactc 3180
   acatagctgc tgactttttc agagaaaaat gtgtcttact actgtttgag actagtgtcg 3240
   ttgtagcact ttactgtaat atataactta tttagatcag catagaatgt agatcctctg 3300
   aagagcactg attacacttt acaggtacct gttatcccta cgcttcccag agagaacaat 3360
   gctgtgagag agtttagcat tgtgtcacta caagggtaca gtaatccctg cactggacat 3420
   gtgaggaaaa aaataatctg gcaagtatat tctaaggttg ccaaacactt caacagttgg 3480
   tggttgaata gacaagaaca gctagatgaa taaatgattc gtgtttcact ctttcaagag 3540
   gtgaacagat acaaccttaa tcttaaaaga ttattgcttt ttaaagtgtg tagttttatg 3600
   catgtgtgtt tatggtttgc ttatttttgc aagatggata ctaattccag cattctctcc 3660
   tctttgcctt tatgttttgt tttctttttt acaggataag tttatgtatg tcacagatga 3720
   ctggattaat taagtgctaa gttactactg ccataaaaaa ctaataatac aatgtcactt 3780
   tatcagaata ctagttttaa aagctgaatg ttaatagggg acactgtaaa gtatcatcaa 3840
   aacctgaata gcttcattgt gcacaagtgt ggagttttgt atcctcttac ctggtaaact 3900
   gaagggattg tttggccatt tcatttatct tatcattaat tcacaagata gttagaaatt 3960
   ctgcctcaag caaagtacca cattttgaat gttttcttag attttgattg caagtagata 4020
   tcagcatttt ttaaatgaaa agctatatta tcttctccct tcaaggcagc ctaaggatgt 4080
   tctttcccag aatcactcca acccttcttg ccagaattca taaaagtaca aaattggaga 4140
   atagatgata tcttagaaat aagctttttt tttttttttt tttttttttg agacggagtt 4200
   tcactcttgt cacccaggct gaagtgcaat ggcgcaatta gggttcactg caacctctgc 4260
   ctcccgggtt caagcagttc tcctgcctca gcctcctgag tagctgggat tacaggcatc 4320
   caccaccgtg cccagctaat ttttgtattt ttagtagaga cggggttttg ccatgttgga 4380
   caggttgatc tcaaactcct gacctcaggt gatctaccct cctcggcctc ccagagtgtt 4440
   gggattacag gcatgagcca ccatgccagg ctgctaattc tcctttttag tgagttaggg 4500
   aactgagcct cagaaaactt aaacgatttc tcagaaaaca ctcaagtgat aaagtggcca 4560
   cattggaaag gagtttttat cttctcattg tcaggccagt gttcattgca caatatcatg 4620
   ctacctcttg aatctttaaa atattcaatt ggcaaatgtt tttcaatgtg atttactcat 4680
   gtcttaagtg tatgaggaaa gttcaaagca aaatagaaag gaataattca aactgaattg 4740
   tccataatca gcttccagtc tttcatgcta atcagcttct taagagactg aagtatggca 4800
   tacctacagg ggaattcctt cgcaccatag cctgtatgaa cagtgttccc tggagttctc 4860
   cagtgctcag cttgagacct tgatacacgg gccatgagcc ctgtcttccc caatggaaat 4920
   ttatttacac ttaccttatc cctatggact tagtctgatt ttattggcta ggagtctaac 4980
   agtcctgtgt ggatatacag ttttgcccat gacaacaaag gaatctatcc gaaatatctt 5040
   tttttttata ataaacttcc aagatttgct gtcttccagc acttgagtta aagtactaga 5100
   tactgcattt tgatgaagac taaccccatc tcatattcta ccctaaagag aactgaaaaa 5160
   cctataataa gttgttctgg agccaataaa cacagcagct ctgttagatg tcctctacag 5220
   ccaagcactt tcaatgctaa cttgaactgc atttccttcc tcaaatgaga gattgacata 5280
   attcagtact gtgagtcact tgtataagaa acctttgatc actaaaaata atgtaaaaat 5340
   tgggtttagt agcctaatac acataacgtt cttcttaaaa aggaaaatgg atggatgcct 5400
   gacaaccctc caaaagaaaa aagtgtaaga tagccattaa gatgatgaca atttttgaaa 5460
   tgaacattat gatatttatg aacaataaac aaatttccgt atggaatgaa ttatccaaaa 5520
   agagtataac aaaatgaaat ccttaaaaat ccagagttta tatttttttt ataccctcac 5580
   ttgtttgcac taactttata gtggaccaag gctgttacca taggaaggga caaacttcct 5640
   tgtaggcaac tcagtgttag acgatgattg tggttatgct tgcaaagtct tgtgcttatc 5700
   ttttttgttt ttacttaaaa agctaatttt taaagattgt agggcttgta ttttacttga 5760
   ataattgata tcttcctgtg taatgatttg tgagatgaga attaatattt gactagttag 5820
   aattaattaa atggtaaggg aacacagggt actcttaggt taaataatgt atgcaaatag 5880
   agtctatttt caactaatat ggccacagga gccttttgag attcattgat attaaacaca 5940
   attaatgaaa ttttaaattg ttaacagaat tgagaacttg aacaacactt ttagtactgc 6000
   agcatttttg tgccctaaag tatgtaatga tttataaatg tgccatacat acactacaac 6060
   ataacatttg ctttgttatg cattttattt ctctggggac accattgcac tgcagtgcac 6120
   acgtatttat aaacatttgt tatatttttg gaaacttgct aatatttatt aagtcataga 6180
   cttttctgga ggacttaaaa attcactaaa aatctgatta tgtcttaaat gttcagttta 6240
   tctttggttt attaaaataa aaaaaaaatc taagattaaa cacagtagat atctctggag 6300
   gcaattttcc aaaactcaac attaaaattt gtggatgcat gagatgcaat ccttcaaaga 6360
   atgaatctga aatatatttt taatatttac ttaatatcca ctgaagatat ctttatgcaa 6420
   gacaagagtc agccatcaga cactgaaata tattatgata gattatgaag aattttctct 6480
   gtagaattat attcttcctg gaacctggta gagtagatta gactcaaagg ctttttcttc 6540
   cttttcttac tcctgttttt tccactcact cttcccaaga gatttcctaa agcttcaagc 6600
   ttaataagcc taatagtgaa aaataactga atttaatggt ataatgaagt tcttcatttc 6660
   cagacatctt taattgatct taaagctcat ttgagtcttt gcccctgaac aaagacagac 6720
   ccattaaaat ctaagaattc taaattttca caactgtttg agcttctttt cattttgaag 6780
   gatttggaat atatatgttt tcataaaagt atcaagtgaa atatagttac atgggagctc 6840
   aatcatgtgc agattgcatt ctgttatgtt gactcaatat ttaatttaca actatcctta 6900
   tttatattga cctcaagaac tccattttat gcaatgcaga ccactgagat atagctaaca 6960
   ttctttcaaa taattttcct tttcttttat aattcctcta tagcaaattt ttatgtataa 7020
   ctgattatac atatccatat ttatatttca ttgattccaa gacatcactt tttcaattta 7080
   acatctctga aattgtgaca tttcttgcaa ctgttggcac ttcagatgca gtgtttaaaa 7140
   ttatgcttga ataaatatta cactaatcca actttaccta aatgtttatg catctaggca 7200
   aattttgttt tcttataaag atttgagagc ccatttatga caaaatatga aggcgaaatt 7260
   taaggacaac tgagtcacgc acaactcaac atggagccta actgattatc agctcagatc 7320
   ccgcatatct tgagtttaca aaagctcttt caggtcccca tttatacttt acgtgagtgc 7380
   gaatgatttc agcaaaccct aacttaacta acaagaatgg gtaggtatgt ctacgtttca 7440
   ttaacaaatt tttattattt ttattctatt atatgagatc cttttatatt atcatctcac 7500
   ttttaaacaa aattaactgg aaaaatatta catggaactg tcatagttag gttttgcagc 7560
   atcttacatg tcttgtatca atggcaggag aaaaatatga taaaaacaat cagtgctgtg 7620
   aaaaacaact ttcttctaga gtcctcttac tttttattct tctttatcat ttgtgggttt 7680
   ttcccccttg gctctgatca ctttaacttc aagcttatgt aacgactgtt ataaaactgc 7740
   atatttaaat tatttgaatt atatgaaata attgttcagc tatctgggca gctgttaatg 7800
   taaacctgag agtaataaca ctactctttt atctacctgg aatacttttc tgcataaaat 7860
   ttatctttgt aagctaactc tattaatcag gtttcttcta gcctctgcaa cctacttcag 7920
   ttagaattgt ctaatactgc tctattaatc aggtttctag cctctacaac ctacttcagt 7980
   taaaattgtc taatacagca atatttaaaa aaaaaacact gcaattgtca aggatggaaa 8040
   atgtgtgatt tgtgtaaaca atttttacca actttacatt ttcctacaga taaatgtgaa 8100
   attttgataa gaagtctacg caatgacaag tatggtacat aaattttatt aagaatattg 8160
   agtataaagt actttaattc taaattataa gaaaatatac atttgcacat attaatatag 8220
   aaattcattt tgtgtatatt taacatagct tttaaactat tttacattag ctacttcatt 8280
   atggtttctt gaacttctga aaaaaattag aaatgtatta aacttatcag taacataaaa 8340
   acttattttg tttcacctaa cgaatactgc gtttgtaaaa ataaatttaa tatagaatat 8400
   atttttaaat taaatatttg aatataaaat agctctaaga aagaagcaaa ttatcactga 8460
   acatatttct tattatttct ggctttgaat tatacgtaac ttaaattgtc ttaaatgata 8520
   cagaatattg gagaatatga tactttcaca taatatacta tgaacctgtt catataactc 8580
   tgattgacta ctaacttctg ttttatgtat ttattaaaga gctgacactg tagtttgtgg 8640
   tgagatgttt atttttctaa cagagcttat aacagttagg acaaggcatt taattaatgc 8700
   atcattctgt ttagtagtag gtgttaatca atatgaaatt ctctgtttta aaataaaaat 8760
   gtaaaaatct aagaataaaa aaaaaaa 8787
<210> 32
   <211> 3081
   <212> DNA
   <213> Homo sapiens
<400> 32
   attagaggct ccagccccgc cgacttgcag acgtgagatc gggcacacct gagcggcggc      60
   ggggcggtcg tggccacatc cggggcgacg tgcctgagtc accccgtccc gccagcgtct     120
   gccagtccag ccagtccgcc cagtctctcg cgtccgagac tcgcctccag cctcccacct     180
   ccgcccgggc cgcgcgagcc tcgcgggggc gggggcgggg cgccaagggg cggggctgtc     240
   tcttaaaggg ccccgggccg ctgcccttag gccacttcct gggggcggag aggacctcag     300
   cggctgcggc gacacccagg gaaggcggcg cggccgggtc ccgaaactcc tggctgtttc     360
   catcagagcc ctcggacact cccagcccgg gctgagcacg catcgtcgct ccccggcgga     420
   tacaaggggg ctccgccatc cgctcccgtc agttcggcct ccatctcctg ggacccgcgc     480
   cggcagccag gccaggcctc tgagtggccc cagagccctg gctggactcg tccacggcgg     540
   cagcgatctg cccggggtct cggaggccat cccttcagag tcggccctgt gctcgccacc     600
   gtcacctgct ggttggattc cggaaaccca ctgtctgaag accacagagg ggtgtcgctg     660
   accaccccaa atcggatacg tccagacctc aagctccctt cccctctctg gctgccctct     720
   gctcttttca tctcttctct caaccttttg gggatttctg tgtcctgaca ccacctcccc      780
   atccaccacc aaagtagccg gggtgagccc caaaccttac tgggtgtgct ccacctgtgc     840
   ctccaaccca gcgaatctga cagcttcgac ccaattctgc acacacccag gaagttctgc 900
   cttttctttt ctttcggtgt ctcctgtact tcccaaaatt tctcctcctc ctgtgccctc 960
   ttcgcccccc tcctttgggg gccccgtgac cctgaatgtg gggggcacac tatattccac 1020
   cactttggag accctgaccc gcttcccaga ctctatgctg ggggccatgt ttagggccgg 1080
   cacccccatg ccccccaacc tcaattccca aggaggcggc cactacttca tcgaccggga 1140
   tggcaaggcc ttccggcaca tcctcaattt cctgaggctg ggccgcctgg acctgccccg 1200
   tgggtacgga gagacagcgc tgctcagggc agaggctgac ttctaccaga tccggcccct 1260
   cctggacgcg ctgcgggaac tggaggcctc tcaggggacc cctgcaccca cagctgccct 1320
   gctccacgca gatgtagatg tcagcccccg cctggtgcac ttctctgctc gccggggacc 1380
   ccatcactat gagctgagct ccgtccaggt ggacaccttc cgagccaacc ttttctgcac 1440
   cgactctgag tgtctaggtg ctttgcgggc ccgatttggt gtggccagtg gggatagggc 1500
   agaggggagc ccacattttc atctggagtg ggccccccgc cccgtggaac tccccgaggt 1560
   ggagtatggg agactggggc tgcagccgct gtggactggg gggccaggag agcggcggga 1620
   ggtggtgggc accccaagct tcctggagga ggtgctgcgg gtggctctcg agcacggctt 1680
   ccgactagac tctgtcttcc ccgaccccga agacctgctc aactccaggt ctctgcgctt 1740
   tgtccggcac tgaggatgct gttctcagtt tgactgtggg gaggagagag aatggggtac 1800
   tagcacccct gaagcctctt tccagctctg cttcaggagc tatgagagtc gggactctcc 1860
   tgcacctgac tggagctcag atgtgggcag gaattcccaa acctgagccc accaaggact 1920
   cacaagtggt ccagaaggtc tcaacctgtg ctgaccctgg gaggggtagg gaaggttctc 1980
   tcagcttgtt cttgcctaag gctgagcacc tccagtctct ccttgatttg gagctcagtg 2040
   tttaagggct tggaaaaggg gggaacatct ctttacccag actagaccta gcaaaaccct 2100
   ggaaggatat tgaggtctgg ggaaaaggga ggactttgca ttttcccaat gcggtctctt 2160
   ggaccatggc ttctactcct gaagctgggt ggcctggcct ggcctgacca atgagaggcc 2220
   agaacactct ggaacatcgg aagaggagtt ctttgctatg ttccaagcca tctactgagg 2280
   gaggcagaaa ggccacaacc caccctaggt tgatgtatgg gagctaggac agtccccatg 2340
   gcaatggggc tggagcatcc ctcatctgga agaatcccat actgatggca gggctggcca 2400
   gggggaagag ggtagtatct gtgggtcctg gcctttcttc atgtgtgcgt gcatatcagc 2460
   ccgtgtggct gactgatgta taggtccctg gcatcctggt tcatatctgt gttgctgact 2520
   acagtgtctg tgatgtccgc atgtccaggc ctgtttgggg ttgcctagcg actcttctgg 2580
   cacagggtgt gtctgtggta tacctgtgag gtggttgaca attagtagtt taatcacagg 2640
   gtgtgtgtgt gtgtgtgtgt gtgtgtgttt atgtgcacgc atgtatatgc atcaccacgt 2700
   agccaggagg ggcctgttgg ggtttgagtc actgggatct tcctggtgag aggtaagaga 2760
   agtcactggg cttagctggg cctctgaggc ctgtatggaa ctcttggttg ctgaggcaac 2820
   catggacctg ttgctaggag atagctgggg aaggcccaag gccgcccagg gcagagagag 2880
   gagacgaaga gtttgggaca gtgggggagg agatgggaag ggatgggatt tctgggtccc 2940
   agagcgggtg ggatactcac gcacagcttc ttcactggtg gggggtgggg cacacattat 3000
   ttctcactgg tcatgattta caagaagaaa aataaaactg cttttggaac cacaaaaaaa 3060
   aaaaaaaaaa aaaaaaaaaa a 3081
<210> 33
   <211> 3767
   <212> DNA
   <213> Homo sapiens
<400> 33
   aagaaagagc cccgccccta gtcttatgac tcgcactgaa gcgccgattc ctggcttttg      60
   caaggctgtg gtcggtggtc atcagtgctc ttgacccagg tccagcgagc cttttccctg     120
   gtgttgcagc tgttgttgta ccgccgccgt cgccgccgtc gccgcctgct ctgcggggtc     180
   atggtgtgct tccgcctctt cccggttccg ggctcagggc tcgttctggt ctgcctagtc     240
   ctgggagctg tgcggtctta tgcattggaa cttaatttga cagattcaga aaatgccact     300
   tgcctttatg caaaatggca gatgaatttc acagtacgct atgaaactac aaataaaact     360
   tataaaactg taaccatttc agaccatggc actgtgacat ataatggaag catttgtggg     420
   gatgatcaga atggtcccaa aatagcagtg cagttcggac ctggcttttc ctggattgcg     480
   aattttacca aggcagcatc tacttattca attgacagcg tctcattttc ctacaacact     540
   ggtgataaca caacatttcc tgatgctgaa gataaaggaa ttcttactgt tgatgaactt     600
   ttggccatca gaattccatt gaatgacctt tttagatgca atagtttatc aactttggaa     660
   aagaatgatg ttgtccaaca ctactgggat gttcttgtac aagcttttgt ccaaaatggc     720
   acagtgagca caaatgagtt cctgtgtgat aaagacaaaa cttcaacagt ggcacccacc     780
   atacacacca ctgtgccatc tcctactaca acacctactc caaaggaaaa accagaagct     840
   ggaacctatt cagttaataa tggcaatgat acttgtctgc tggctaccat ggggctgcag     900
   ctgaacatca ctcaggataa ggttgcttca gttattaaca tcaaccccaa tacaactcac     960
   tccacaggca gctgccgttc tcacactgct ctacttagac tcaatagcag caccattaag    1020
   tatctagact ttgtctttgc tgtgaaaaat gaaaaccgat tttatctgaa ggaagtgaac    1080
   atcagcatgt atttggttaa tggctccgtt ttcagcattg caaataacaa tctcagctac    1140
   tgggatgccc ccctgggaag ttcttatatg tgcaacaaag agcagactgt ttcagtgtct    1200
   ggagcatttc agataaatac ctttgatcta agggttcagc ctttcaatgt gacacaagga    1260
   aagtattcta cagctgaaga atgttctgct gactctgacc tcaactttct tattcctgtt    1320
   gcagtgggtg tggccttggg cttccttata attgttgtct ttatctctta tatgattgga 1380
   agaaggaaaa gtcgtactgg ttatcagtct gtgtaatcag ttaaatctag tgtttgtttg 1440
   tttttttcaa ttagaagtta cgtttccatt ggctaaaagc caggacatgc tgtgcaatag 1500
   attgtttaag atatgcagac taacttcagt gagttcctag ctaacttggg catgagtaca 1560
   cttatttaag acaaaatata ttaggaccaa tttttttctg ttttttttct tcctttgtta 1620
   aagtataatt aaaagaaaaa ttgtggctta gaatttttta agtaaataat gattttaagc 1680
   ccctggatcc aattatgaaa gcatttttgc tgatgtgtaa ttttatatgt tacagttact 1740
   tatattttac tactttgatg ttatttgcaa aatcaaaggt gttaaagaat ttaacttgct 1800
   tcaggaaata aattcaagaa catagtggat tcattttcat tggtggcaga cacgaaattt 1860
   ggttcatgat aagacttcct ttccccacct cctgatcagc attatttaaa tctgtatttt 1920
   tctgttagtt aagaaagaaa tggcttcatg atattgtatt taatagcaaa agtttggctg 1980
   tcttcttcat tactgttaat agctactata ttttaacaag gagatttctt tttttgttgt 2040
   tgttgttcta gagtttggaa tatactgatt atctcagact tgacatttat actgaaggat 2100
   gaagtaagac ctccagcttt ttttaaaaaa ggtgttgatt tggaacacct gtatgggtta 2160
   tggtttatta aggttatggt ttagaaagtt tttttccctc agagccttaa cttgttaaga 2220
   aggttcattt atcctgcact gaaaacaaaa actctatata ctttgtttgt gtgcctcctg 2280
   cactctccca ttccctatgt gaatatgctc tagttgatat ttttaatata ttgatttctt 2340
   ttttctcaca gcaacaagtg cttactctag aggttagtgg gccctgatat gtcatcagtc 2400
   agatgcctgc ctagccaaag ctggactaag attattctgt acatttgttg atcttgatat 2460
   agacttatat ccctgtaggg actgctaatg gctccggctt ctggagtaag gtactggaga 2520
   ccactcatcc ctgtgtctgc ttgattggtt cagctgttga attgcccttt tatttggaag 2580
   cagtgttgaa gttgtctagg gttcaaatgg ctgctttgta cacctgtcat tagtataagg 2640
   cagatgttta ttttatcaag ctattttatc tctacattta actaaaaaca aaagttccca 2700
   aagatctgcc ttcacttcag aaattttttt tggattaaaa aaattaagcc tgaaccttaa 2760
   ataaagtgag ttggttattc attccaagga ttaagtccca atctacctct cagcacaatg 2820
   cagaagctca ccactgtatt gctgccatta actcatgcca gaaccctttg ccaataactg 2880
   gaattacaaa tttttgttaa agaaaattta tcaagatctt tctttactgc cttctctata 2940
   tgtacatctc aaaaacatgt acatctcaaa aactggagta gaaagttaga ttgctcaact 3000
   acaactcctc tagaactcta tagctctgac atacagattc acactctcct ctatttgcta 3060
   agtatgtaaa gaatgttttc ttttaaaatg ttctcttttg agaacaactg cttatttgtt 3120
   ataaaagcat ttggttaaaa tgatgtcatc ataaaaaaca gtggctttgt ttcaatacat 3180
   atttttgaga tgattatcta gaagccagat taataaaatc agcttgtgac cttgctaagc 3240
   atataaactg gaaattcaga tacattcaaa attatgggtt catttaaaag tgttctacct 3300
   tttgggtatg agactaatat cactaattcc tcaatagtta tcatggctct atcttaatta 3360
   attagaaaat atgtgtgttt aattctttga gaattaaaat agagaatatt aacagagggt 3420
   taaaaactgc ttcaactcca ataagataaa ggaagctcaa aatctatgag ctgagtgttc 3480
   aattagcttt gcctactgag ttcaatttta tgtcaataca acagtggatc agacagtacg 3540
   actttgaact ggtgaatgta aacaattgtt tttcacctaa gctgctttgg aagaactgat 3600
   gcttgctgct aactaaagtt ttggatgtat cgatttagag aaccaattaa tacctgcaaa 3660
   ataaagcata ctgtggtact tctgtttgat ctagtatgtg tgattttaga ttgatggatt 3720
   aaaaattaat aaagatcata cattccatac caaaaaaaaa aaaaaaa 3767
<210> 34
   <211> 4625
   <212> DNA
   <213> Homo sapiens
<400> 34
   tcgggagaca tggcgggcgt taaagagaag aaaccagtgt gtgtgtagta tgtgtttttt     60
   gcatggggca gttggtaaaa acaccgcgtc ccttatctgt atggcttcag agcaatgcga    120
   gacggaaaag gttttttgca aggcttcctg tattttgctc tcgtggcatt atccttcagt    180
   ggggctattg gactgacttt tcttatgctg ggatgtgcct tagaggatta tggcgtttac    240
   tggcccttat tcgtcctgat tttccacgcc atctccccca tcccccattt cattgccaaa    300
   agagtcacct atgactcaga tgcaaccagt agtgcctgtc gggaactggc atatttcttc    360
   actactggaa ttgttgtttc tgcctttgga tttcctgtta ttcttgctcg tgtggctgtg    420
   atcaaatggg gagcctgcgg ccttgtgttg gcaggcaatg cagtcatttt ccttacaatt    480
   caagggtttt tccttatatt tggaagagga gatgatttta gctgggagca gtggtagcac    540
   tttattctga ttacagtgca ttgaatttct tagaactcat actatctgta tacatgtgca    600
   catgcggcat tttactatga aatttaatat gctgggtttt ttaatacctt tatatatcat    660
   gttcacttta agaaagactt cataagtagg agatgagttt tattctcagc aaatagacct    720
   gtcaaattta gattatgtta ctcaaattat gttacttgtt tggctgttca tgtagtcacg    780
   gtgctctcag aaaatatatt aacgcagtct tgtaggcagc tgccacctta tgcagtgcat    840
   cgaaaccttt tgcttgggga tgtgcttgga gaggcagata acgctgaagc aggcctctca    900
   tgacccagga aggccggggt ggatccctct ttgtgttgta gtccatgcta ttaaaagtgt    960
   ggcccacaga ccaagagcct caacatttcc tagagcctta ttagaaatgc agaatctgaa   1020
   gccccactct ggacccagga cattttgatg agatccaaag gagttgtatg cacatgaaag   1080
   tttgagaagc atcatcatag agaagtaaac atcacaccca acttccttat ctttccagtg 1140
   gctaaaccac ttaacctctc tgggtgttac ctgctcattt gtttaaaaaa aaaaaaaaag 1200
   tctcacctgc tttcatgctg aggacaagtt cagatgttca agcctataat atttaggcag 1260
   ttcctcaaat ttatgaaaag tgttctcaga attgggagac agtcaaaggg tacaaagcct 1320
   cagttaggag gaataagtgt gatttttttt taaagatcac ttgcacagca tgctaaatat 1380
   aggaataatt gaatgtatat ttcaatattg ctaagagagt aaatttctaa tgttctcata 1440
   aaaaagttaa atatttgaga tcatatgtta attagtgtaa tcattccacc ttatattcaa 1500
   aaatcataaa accgtattgt accctataaa aatatacaat aatttgtcaa tatataatca 1560
   aaataaaaaa caaaacatac tctctccccc aaaaaaacat ctcagtgggg aacagatgta 1620
   tcttttcatc tgaaagacaa tgctggggga agagctccac tgagatgcgg gcagggaggc 1680
   tgggctcgag ccagcccctg cgttagcagg agggggagaa cagataggta actcttttac 1740
   atttccttta tgatctggca cttctcccca gctccttccc tctgcccccc acccctactc 1800
   ctcaacagtt ctggtttgcc ctgacttctc tacggctctg gcttcttccc gaagagatat 1860
   aggagccatg taagcacgca gtgggtgaac tgcttaattt cactacgtgt tgatgtactt 1920
   gtcttccgtc ctgtaggtct tttctatata actttatgcc acccttaaat gaatcattgg 1980
   gtatacctgt catgttggat cctgtaatca cagttttccc tgctcacctt tttgtctaag 2040
   atctattgag aaagggaaat atgggaagga gaaccatttg atcagaatac aaccaatagt 2100
   ctttaagcat tgttaaagta tgaaactgaa atacattcaa aacacttaat ccttgaggct 2160
   tgtgatctga gtaattagca ggtatgatgc tgggactgga aaatagaaag taataactaa 2220
   agggttaatg tgcaacgtta ttttttggcc ttgttcatga ttttatgttt tcagtgtcct 2280
   gtgtacatat agaattgtta aagttgtcat ttccaatatt tatattagaa aaattattta 2340
   gatactttat aattttaacc ggcattttta ataatgacac ttgcatttat tgtattgtaa 2400
   taaatttcac ttttaacttt aaaagtttaa ctttaaaatt tttttgtgat gttgccttgc 2460
   ctgaaaagat aacaaaaatg agagaatttc ttgatgtttt aaaatgggca gttttgagca 2520
   ataatctgtc ctaacagaac agtagcaata agttttagga taccatcttg aatgtctagt 2580
   tggtgtgcaa tagcttttct ttctaagatg gcaataatga ttcatttcta ctacattttg 2640
   caaaagtgtt tttgttgctt atacacattt tcaataacca aggtagcctt catatgtagc 2700
   cttaaagcat tacctcttga ttgtatcttt agattgatat aaagtacttg catatagagt 2760
   atttgaagtg atagattatt agatttgctc tatgtctgaa aagagagcta ttctgcagtg 2820
   cctaaatatc atttaaacag taaatattaa taggaaatat tgctatatct gaatatataa 2880
   tacaaaagtt tgatcatggt gacacaaatg ttggacattt ttttccttat aaaaggctct 2940
   ttttttatat attgtacaat atatttggag attcagagca tagtgactat agtcgaaaac 3000
   tgagattgca cttccaaaat tggccacaag taaataatct tatgaaggga ttctttatca 3060
   tgtttcaaac aagtgggtta caagcagact ttgagacact tttccacaga aacaatacta 3120
   tgaattggtg attgagttcc caggccaagc ctccctcaac aggttcaact ctaatatacc 3180
   taacctgtga tactgaaggt gcctgcctga gttttgggtc tctgagacag ggtagtgtga 3240
   gtagtttgga ggaaggacag tgcaactttc cacccctttt cctaagaaca aggtctttct 3300
   ccttttaatt tttccactca ttttcacctc ctaatgccct tgagatccag gtacactcct 3360
   gggagttttg ttcacctctc ccaactgaga accttcccac tgggctccat cctccctcct 3420
   gaggttcttc atattccaga gtcacccacc cttctcctcc cattagtcag ttctctaagt 3480
   acagctgatg tcatgtggtg ctgagaagaa agcagatcac acttcatcac agaaagaatg 3540
   ccttgtgatt atcttctcca catctgaaat tccttttgac acctgcattg ggccgactgc 3600
   cattcccatg actgctgcac ctgcgttttt agagaatgcc tcataaccca ctgattctca 3660
   ttcacagaga atgggagaac ggaatgaaga aagattccag cagcttatag aaggatagca 3720
   atattttggg acagggaaaa tcctgtcata cctcatctct tcctcaggag gagttctgag 3780
   ctggtcctgc ttttcatagt tgtttctttt cttccactta agaactcata gatttttctt 3840
   actgtcctaa ggaagtcctt acctctgagg tatctcctca atgaatactg ttttcaaggc 3900
   tgaaatagtt cattatgtta ataaccttct ttatgttctc agggaaatgc ttaggtggtg 3960
   tcacaaaatg tgccttttct tttctttttt tttttttttt tttgaggcag agtctcgctc 4020
   tgttgcccag gctggagtgc agtggtgcga tcttggctca ctgcaagctc cgcctcccgg 4080
   gttcacgcca ttctcctgcc tcagcctccc aaggagctgg gactacaggc tcccgccacc 4140
   acgcctggct aatttttttg tatttttagt aaagacgggt ttcatcgtgt tagccaggat 4200
   ggtctcgatc tcctgacctc atgatccgcc cgcctctgcc tcccaaagtg ctgggattac 4260
   aggcctgagc cactgtgccc agccaaaatg tgcctttgca aagtttgcga aatcagattt 4320
   tgtatcccaa tagaaccaaa atatttatga ggatgctagc attttccaag catagtaatt 4380
   agttcacaac tgagaaatat tatgtctgta gtagataaat attagttgtg cattttaatt 4440
   taattctcct ttttccattt tgtctcatga agtaccttat tgcaaaaatc ccactgagta 4500
   atagctcata aattataatc tttcaaatag ccatgctacc agcgtacaac agtgatacat 4560
   gtaaccccaa atgtgatgtg agaggacgat tactttgtaa ataaaacttg ttattgacat 4620
   tttaa 4625
<210> 35
   <211> 744
   <212> DNA
   <213> Homo sapiens
<400> 35
   gagtatttga ggctcggagc caccgccccg ccggcgcccg cagcacctcc tcgccagcag      60
   ccgtccggag ccagccaacg agcggaaaat ggcagacaat ttttcgctcc atgatgcgtt     120
   atctgggtct ggaaacccaa accctcaagg atggcctggc gcatggggga accagcctgc     180
   tggggcaggg ggctacccag gggcttccta tcctggggcc taccccgggc aggcaccccc     240
   aggggcttat cctggacagg cacctccagg cgcctaccct ggagcacctg gagcttatcc     300
   cggagcacct gcacctggag tctacccagg gccacccagc ggccctgggg cctacccatc     360
   ttctggacag ccaagtgcca ccggagccta ccctgccact ggcccctatg gcgcccctgc     420
   tgggccactg attgtgcctt ataacctgcc tttgcctggg ggagtggtgc ctcgcatgct     480
   gataacaatt ctgggcacgg tgaagcccaa tgcaaacaga attgctttag atttccaaag     540
   agggaatgat gttgccttcc actttaaccc acgcttcaat gagaacaaca ggagagtcat     600
   tgtttgcact tacatgtgta aaggtttcat gttcactgtg agtgaaaatt tttacattca     660
   tcaatatccc tcttgtaagt catctactta ataaatatta cagtgaatta cctgtctcaa     720
   tatgtcaaaa aaaaaaaaaa aaaa                                            744
<210> 36
   <211> 2094
   <212> DNA
   <213> Homo sapiens
<400> 36
   cacttgttca atgatgtacc cccagtgtca ggcgctttgc aaacacacga tacatacggg      60
   ttgatgtttg gtcaagagag gaattaagac caggcagaca gcaggctggg atcagagaga     120
   ccccatttct gtctgaaatg tctgcagaga acctggtgcc tgcctcagcc ctagctctgg     180
   ggaaatgaaa gccaggctgg ggttcaaatg agggcagttt cccttcctgt gggctgctga     240
   tggaacaacc ccatgacgag aaggacccag cctccaagcg gccacaccct gtgtgtctct     300
   ttgtcctgcc ggcactgagg actcatccat ctgcacagct ggggcccctg ggaggagacg     360
   ccatgatccc caccttcacg gctctgctct gcctcgggct gagtctgggc cccaggaccc     420
   acatgcaggc agggcccctc cccaaaccca ccctctgggc tgagccaggc tctgtgatca     480
   gctgggggaa ctctgtgacc atctggtgtc aggggaccct ggaggctcgg gagtaccgtc     540
   tggataaaga ggaaagccca gcaccctggg acagacagaa cccactggag cccaagaaca     600
   aggccagatt ctccatccca tccatgacag aggactatgc agggagatac cgctgttact     660
   atcgcagccc tgtaggctgg tcacagccca gtgaccccct ggagctggtg atgacaggag     720
   cctacagtaa acccaccctt tcagccctgc cgagtcctct tgtgacctca ggaaagagcg     780
   tgaccctgct gtgtcagtca cggagcccaa tggacacttt ccttctgatc aaggagcggg     840
   cagcccatcc cctactgcat ctgagatcag agcacggagc tcagcagcac caggctgaat     900
   tccccatgag tcctgtgacc tcagtgcacg gggggaccta caggtgcttc agctcacacg 960
   gcttctccca ctacctgctg tcacacccca gtgaccccct ggagctcata gtctcaggat 1020
   ccttggagga tcccaggccc tcacccacaa ggtccgtctc aacagctgca ggccctgagg 1080
   accagcccct catgcctaca gggtcagtcc cccacagtgg tctgagaagg cactgggagg 1140
   tactgatcgg ggtcttggtg gtctccatcc tgcttctctc cctcctcctc ttcctcctcc 1200
   tccaacactg gcgtcaggga aaacacagga cattggccca gagacaggct gatttccaac 1260
   gtcctccagg ggctgccgag ccagagccca aggacggggg cctacagagg aggtccagcc 1320
   cagctgctga cgtccaggga gaaaacttct gtgctgccgt gaagaacaca cagcctgagg 1380
   acggggtgga aatggacact cggagcccac acgatgaaga cccccaggca gtgacgtatg 1440
   ccaaggtgaa acactccaga cctaggagag aaatggcctc tcctccctcc ccactgtctg 1500
   gggaattcct ggacacaaag gacagacagg cagaagagga cagacagatg gacactgagg 1560
   ctgctgcatc tgaagccccc caggatgtga cctacgccca gctgcacagc tttaccctca 1620
   gacagaaggc aactgagcct cctccatccc aggaaggggc ctctccagct gagcccagtg 1680
   tctatgccac tctggccatc cactaatcca ggggggaccc agaccccaca agccatggag 1740
   actcaggacc ccagaaggca tggaagctgc ctccagtaga catcactgaa ccccagccag 1800
   cccagacccc tgacacagac cactagaaga ttccgggaac gttgggagtc acctgattct 1860
   gcaaagataa ataatatccc tgcattatca aaataaagta gcagacctct caattcacaa 1920
   tgagttaact gataaaacaa aacagaagtc agacaatgtt ttaaattgaa tgatcatgta 1980
   aatattacac atcaaaccaa tgacatggga aaatgggagc ttctaatgag gacaaacaaa 2040
   aaatagagaa aaattaataa agtcaaaatg tttattcttg aaaaaaaaaa aaaa 2094
<210> 37
   <211> 1562
   <212> DNA
   <213> Homo sapiens
<400> 37
   gcgggcggca ttctggcgcg gagcggagcg gcggcgggcg cagctagcgg gtcggccgcg      60
   gagcggaggt gcagctcggc ttcccccggc acccctcccc ctcgggcgcc agccccaccc     120
   ctccgccggc cgggccgacc ccgccgtact atcccctgcg gcgcgagccc ggggcggctc     180
   caagcgcccc ccagcagacc cccatcatgg gcagccagag ctccaaggct ccccggggcg     240
   acgtgaccgc cgaggaggca gcaggcgctt cccccgcgaa ggccaacggc caggagaatg     300
   gccacgtgaa aagcaatgga gacttatccc ccaagggtga aggggagtcg ccccctgtga     360
   acggaacaga tgaggcagcc ggggccactg gcgatgccat cgagccagca ccccctagcc    420
   agggtgctga ggccaagggg gaggtccccc ccaaggagac ccccaagaag aagaagaaat    480
   tctctttcaa gaagcctttc aaattgagcg gcctgtcctt caagagaaat cggaaggagg     540
   gtgggggtga ttcttctgcc tcctcaccca cagaggaaga gcaggagcag ggggagatcg    600
   gtgcctgcag cgacgagggc actgctcagg aagggaaggc cgcagccacc cctgagagcc     660
   aggaacccca ggccaagggg gcagaggcta gtgcagcctc agaagaagag gcagggcccc     720
   aggctacaga gccatccact ccctcggggc cggagagtgg ccctacacca gccagcgctg     780
   agcagaatga gtagctaggt aggggcaggt gggtgatctc taagctgcaa aaactgtgct     840
   gtccttgtga ggtcactgcc tggacctggt gccctggctg ccttcctgtg cccagaaagg     900
   aaggggctat tgcctcctcc cagccacgtt ccctttcctc ctctccctcc tgtggattct     960
   cccatcagcc atctggttct cctcttaagg ccagttgaag atggtccctt acagcttccc    1020
   aagttaggtt agtgatgtga aatgctcctg tccctggccc tacctccttc cctgtcccca    1080
   cccctgcata aggcagttgt tggttttctt ccccaattct tttccaagta ggttttgttt    1140
   accctactcc ccaaatccct gagccagaag tggggtgctt atactcccaa accttgagtg    1200
   tccagccttc ccctgttgtt tttagtctct tgtgctgtgc ctagtggcac ctgggctggg    1260
   gaggacactg ccccgtctag gtttttataa atgtcttact caagttcaaa cctccagcct    1320
   gtgaatcaac tgtgtctctt ttttgacttg gtaagcaagt attaggcttt ggggtggggg    1380
   gaggtctgta atgtgaaaca acttcttgtc tttttttctc ccactgttgt aaataacttt   1440
   taatggccaa accccagatt tgtacttttt ttttttttct aactgctaaa accattctct   1500
   tccacctggt tttactgtaa catttggaaa aggaataaat gtcgtccctt tagtggtgct    1560
   tt                                                                   1562
<210> 38
   <211> 2118
   <212> DNA
   <213> Homo sapiens
<400> 38
   cacgtgaccg aggcacagat cagctgatgc cggagggttt gaagccgcgc cgcgagggag      60
   cgaggtcgca gtgacagcgg cgggcgatcg gacccaggct gccccgccgt acccgcctgc     120
   gtcccgcgct cccgccccag catgacagcc ccggcgggtc cgcgcggctc agagaccgag     180
   cggcttctga cccccaaccc cgggtatggg acccaggcgg ggccttcacc ggcccctccg     240
   acacccccag aagaggaaga ccttcgccgt cgtctcaaat actttttcat gagtccctgc     300
   gacaagtttc gagccaaggg ccgcaagccc tgcaagctga tgctgcaagt ggtcaagatc     360
   ctggtggtca cggtgcagct catcctgttt gggctcagta atcagctggc tgtgacattc     420
   cgggaagaga acaccatcgc cttccgacac ctcttcctgc tgggctactc ggacggagcg     480
   gatgacacct tcgcagccta cacgcgggag cagctgtacc aggccatctt ccatgctgtg     540
   gaccagtacc tggcgttgcc tgacgtgtca ctgggccggt atgcgtatgt ccgtggtggg 600
   ggtgaccctt ggaccaatgg ctcagggctt gctctctgcc agcggtacta ccaccgaggc 660
   cacgtggacc cggccaacga cacatttgac attgatccga tggtggttac tgactgcatc 720
   caggtggatc cccccgagcg gccccctccg ccccccagcg acgatctcac cctcttggaa 780
   agcagctcca gttacaagaa cctcacgctc aaattccaca agctggtcaa tgtcaccatc 840
   cacttccggc tgaagaccat taacctccag agcctcatca ataatgagat cccggactgc 900
   tataccttca gcgtcctgat cacgtttgac aacaaagcac acagtgggcg gatccccatc 960
   agcctggaga cccaggccca catccaggag tgtaagcacc ccagtgtctt ccagcacgga 1020
   gacaacagct tccggctcct gtttgacgtg gtggtcatcc tcacctgctc cctgtccttc 1080
   ctcctctgcg cccgctcact ccttcgaggc ttcctgctgc agaacgagtt tgtggggttc 1140
   atgtggcggc agcggggacg ggtcatcagc ctgtgggagc ggctggaatt tgtcaatggc 1200
   tggtacatcc tgctcgtcac cagcgatgtg ctcaccatct cgggcaccat catgaagatc 1260
   ggcatcgagg ccaagaactt ggcgagctac gacgtctgca gcatcctcct gggcacctcg 1320
   acgctgctgg tgtgggtggg cgtgatccgc tacctgacct tcttccacaa ctacaatatc 1380
   ctcatcgcca cactgcgggt ggccctgccc agcgtcatgc gcttctgctg ctgcgtggct 1440
   gtcatctacc tgggctactg cttctgtggc tggatcgtgc tggggcccta tcatgtgaag 1500
   ttccgctcac tctccatggt gtctgagtgc ctgttctcgc tcatcaatgg ggacgacatg 1560
   tttgtgacgt tcgccgccat gcaggcgcag cagggccgca gcagcctggt gtggctcttc 1620
   tcccagctct acctttactc cttcatcagc ctcttcatct acatggtgct cagcctcttc 1680
   atcgcgctca tcaccggcgc ctacgacacc atcaagcatc ccggcggcgc aggcgcagag 1740
   gagagcgagc tgcaggccta catcgcacag tgccaggaca gccccacctc cggcaagttc 1800
   cgccgcggga gcggctcggc ctgcagcctt ctctgctgct gcggaaggga cccctcggag 1860
   gagcattcgc tgctggtgaa ttgattcgac ctgactgccg ttggaccgta ggccctggac 1920
   tgcagagacc cccgcccccg accccgctta tttatttgta gggtttgctt ttaaggatcg 1980
   gctccctgtc gcgcccgagg agggcctgga cctttcgtgt cggacccttg ggggcgggga 2040
   gactgggtgg ggagggtgtt gaataaaagg gaaaataaat gtgtcgtttt catttttaaa 2100
   aaaaaaaaaa aaaaaaaa 2118
<210> 39
   <211> 4588
   <212> DNA
   <213> Homo sapiens
<400> 39
   aaacttccga gttaagccgc cgctgaggcc ggaaggagct agacggcggt cgggtggaaa 60
   gtggttggtt tctgataact tcctaaacat caccaatgta gcttttgatg accgtgggct 120
   ctatacctgt ttcgtcacct ctccaattcg tgcctcctac tctgtcaccc tacgtgttat 180
   cttcacctcg ggagacatga gtgtctatta catgattgtt tgcctgattg cctttacaat 240
   cacactcatc ttgaatgtca cacggctgtg catgatgagc agccatcttc gcaagactga 300
   gaaggctatc aatgagttct ttagaactga aggggctgag aaacttcaga aggcctttga 360
   gattgcaaaa cgtatcccca tcattacctc agccaaaact ctggagctcg ccaaagtcac 420
   acaatttaag accatggagt ttgctcgtta tattgaagaa ctggcaagaa gtgtccctct 480
   tccacctctt attctaaact gtcgagcctt tgttgaggag atgtttgagg ctgtgcgagt 540
   ggacgaccct gatgacctgg gtgaaagaat taaagagaga cctgccttga atgctcaagg 600
   tggcatctat gtcattaacc cagagatggg acggagtaat tcaccaggag gagattcaga 660
   tgatggctct ctgaatgaac aaggccagga aatagcagtt caggtttctg tccaccttca 720
   gtcagaaacc aaaagtattg atacagagtc tcaaggcagc agtcatttca gtccacctga 780
   tgatatagga tctgcagaat ctaactgtaa ctacaaagat ggggcatatg aaaactgtca 840
   gctgtaacct acaatgctgt aacccagtac ctacaaaatc agctcgctct cagaaaagga 900
   acctgtttct tagaagaagt aacatttttg ccaaaagatg actggggttt tccgtttgtt 960
   aatattaagc acatcagaac gtgaattgcc aaagtcttca ttagaaggca gcatttttcc 1020
   tctctgatac ttttcagtca ttttccttag agctttatta aattatgcat gctaagattt 1080
   aaaggagccc cagataaaca tgatggggaa aagcactgaa ctaagagtcc catggtttct 1140
   cttctggtca cagttcttcc attggttgga tctgatactt atcttgggac ttcagttttt 1200
   ccgtcaataa gatgagggat taggtgagat ctgaagttgt ttctagctct atagcttctt 1260
   aacccatcac ctttaaatta ccagaatctt cactcatctc taagtaaacc tttcccggga 1320
   cttttactcg cttcttttgg aaaggattaa gctgagatct aaatttccac accaatgtca 1380
   taatgcacag aggtttttga aaaacatctg agtgtttttc agatgttttg ccatgtggag 1440
   catataatga tatgtgcaag attgaatctt ttcaatgtag cacatgtctg tagggttata 1500
   cagatgtcag agagctaact gctctgtaaa ctactttcca tgagtaaatt ggtccttggt 1560
   gggggtgtat catattttta acttactgag atatcatttt agttcattga ggttggcagg 1620
   gattgcctaa ctgatcttcc aaagtgagca gtttatttct aaggtataac gtctttgatg 1680
   cttttagaat aagaacagtg tcaaatcatc tgtcttctgg aaaatcatgg attttcatat 1740
   ttctgttaac agaatttctc aggctttccc tttttaaaag tattggactc tacaaatagg 1800
   ttctatattt gggatctcat cctaggagaa aacccaaaac tggattcctc ttaagacctc 1860
   tgtcaaccct cctgcccttt gtggtcttag gtatggtgct ataggttgca tgcgtcttta 1920
   tcttgtttgt ttggttaata ttttgttgtt gaggttttta tttttgttta cttcagatac 1980
   ataattctga gctatggctg cttttgtagc ctttccaaga agcactaacc tgaaataaga 2040
   ttagataatt gtgagggtgt gttactatat taaaatacac acacaactga tctagacatc 2100
   aagaggaaga aaaatgatgg atgatgccac ctgcttcaac tgtatataat aaacacttat 2160
   gatgacattt cttgcctggc tgagatctga tataatggaa ttgtaaatac tcttcagaac 2220
   aatttcttca gctacaggaa gcgtggtgcc atataatttt aagaacttgg cagtggagct 2280
   ccatttgggg cttcacgttt cttcatatga cttctgatta ttgaagcatt acttcagcta 2340
   gaggctcctg agggaaatgt tcagaggagg ttttatcagg attttaattt aaggtttcaa 2400
   acaggggaag acaggaaatt caaagcgtga cgggataaaa ctcatgcctc cctttgtcca 2460
   ggcttatcag aagtaataca tctgctctga atagcatgaa tgaatcaatg tgcagtttta 2520
   tcagatggca tcatggataa agatgataat gctgcctttt ctgtctctca ggctgtttcc 2580
   atggaaacct ctagagccaa ataaaagcta accaatcatt tagccaaagc ttgccttggc 2640
   tcatagactg gtatttcttt aaggaaaatt gtttttatat atttgactat aagagcaaag 2700
   gctcttgaac atatcctaga ttatggaaca ctttttccct tcccttttct ctggaaaaat 2760
   taaatttttt ttctgtccac tgagactgga gtgcagtggc aagatcatag ctcactgcag 2820
   cctccaactc ctggggtcaa gcgatcgtcc tgcttcagcc tcccaagcag ctaggactac 2880
   aggtgtgcac cacctttcct ggctaatttt tttttatttt ttgtagagat gggggtctca 2940
   ctttgttgcc caggctgacc ttgtacttgt ggcttcaaat gatcctcctg ccttacctcc 3000
   caaagtgttg agattacagg catgagccac tgtgcctggc caaaaaacat tttaaatccc 3060
   ttgtctgggg gtcagtcctc taaacatccc tcagatttca gatagtactt tcaaccctgt 3120
   cctaacatag caggtttgca gtaatctttt agaatatagc ttgtcaaatt agagaatggt 3180
   tttaccccac atgttctcat aggagacatt attatttaga accctaaggt agacatgttt 3240
   aaaatcaaag tcctaagaaa cagaactttg gaaaaatgga ggaaatgttt ttaaagtctg 3300
   taagtttgca cgatactgta tagtaactaa atgcatgcta ctccgttgta tcctagttat 3360
   tttagaaaca gaggtggcct aatttggtgg ccaaagtaac tggtttactt tgagtgtacc 3420
   agcttatggt gccattgatg aggaattaaa gtaggtcaaa atttaattgg agttggtatt 3480
   acttcgtaaa gctagttttc aagaggaaga aaaccacact tactaatgtt ttctgtatct 3540
   aatcaaatac tcttcatata taattaagcc tcatgttatc ttttttttaa atcaaccttt 3600
   tgaacttcaa ctacagtcta aaagtcttga tggtaactat agtgtaatta tctttttgtc 3660
   tcactggatt ttatagttag tggaaaatgc ctttacaaaa tgtatttaaa atagctgtca 3720
   tctcatttgt aaattttgtc gtgtattgtg atatagtgaa ccttattgtc cttatgaaat 3780
   ggtagctttg tgaaatacat tcaccaaaaa tcaaaatttg aacatcttta tgattcctta 3840
   ccagctgaag ccagatagac aggtattaat tgagctgatg ccccacttga gtttatagac 3900
   tgtttgataa ctgcctgtcc tccaaattgt gtatgtatat gttacgatgg tttaattctt 3960
   gagtcagggc cagcacgcat ttatattttc taccaattac cttgatagaa atatcttaga 4020
   aattgctgac ctggaacggt tgtgagaaga ctcctggctt ctttcttgcc tcacttaaca 4080
   aatattttaa ggtcaaagca atatctgtgc acggctttcc ttttgctcct ccagacaagt 4140
   gaggctgttg gtatagtcct cttcaccctc tgcatgtagc ttcaccctag atcagacttt 4200
   tgtctcttgg gtcccagatg gcacaggagc actgcatgct tgttttctag agcccagcca 4260
   gtcatgggtg ctagcctagt ctccacacac cagcaagtag aacccaagtg tattgtataa 4320
   atatttcctg agtaccagta agagaatgca ttcttttctc atctaggcca ggaatgttga 4380
   aaatgctcag ccttacatag aaactcctag attttcacta acgcatttca caaaagtaaa 4440
   taagtatttc atataattca gaggatgttt aaattgtcag cattttaata aatacttgca 4500
   ttataatttt gtctcttttt taaagaaagt catacttgaa tataatttat taaacgttca 4560
   atggagtata tagtctattt gaaatttt 4588
<210> 40
   <211> 2482
   <212> DNA
   <213> Homo sapiens
<400> 40
   cccagatcca ggccgggccg cggctctcgc cgcccagccc agcccagccc ggcccggccc      60
   ggccctgccg cggaggcgag gccgccagtg tcccgcgccc ctgatatctg cagtgagcct     120
   gatacctgcc tctgcccttc tgagcctgtt cctcttccct gagtacaggg cacaaagctt     180
   gcgccctgag gggcggccgg cgcgctccct ggcccggtcc ccgcccggcc ccgggccccc     240
   cgcccctccc cgacccgggg ccggggcccc tgccgccgcc gccgccgcct tccgacccct     300
   gcgccccggc cccggtcccc cgggccatgc agcctcggcc ccgcgggcgc ccgccgcgca     360
   cccgaggaga tgaggctccg caatggcacc ttcctgacgc tgctgctctt ctgcctgtgc     420
   gccttcctct cgctgtcctg gtacgcggca ctcagcggcc agaaaggcga cgttgtggac     480
   gtttaccagc gggagttcct ggcgctgcgc gatcggttgc acgcagctga gcaggagagc     540
   ctcaagcgct ccaaggagct caacctggtg ctggacgaga tcaagagggc cgtgtcagaa     600
   aggcaggcgc tgcgagacgg agacggcaat cgcacctggg gccgcctaac agaggacccc     660
   cgattgaagc cgtggaacgg ctcacaccgg cacgtgctgc acctgcccac cgtcttccat     720
   cacctgccac acctgctggc caaggagagc agtctgcagc ccgcggtgcg cgtgggccag     780
   ggccgcaccg gagtgtcggt ggtgatgggc atcccgagcg tgcggcgcga ggtgcactcg     840
   tacctgactg acactctgca ctcgctcatc tccgagctga gcccgcagga gaaggaggac     900
   tcggtcatcg tggtgctgat cgccgagact gactcacagt acacttcggc agtgacagag 960
   aacatcaagg ccttgttccc cacggagatc cattctgggc tcctggaggt catctcaccc 1020
   tccccccact tctaccctga cttctcccgc ctccgagagt cctttgggga ccccaaggag 1080
   agagtcaggt ggaggaccaa acagaacctc gattactgct tcctcatgat gtacgcgcag 1140
   tccaaaggca tctactacgt gcagctggag gatgacatcg tggccaagcc caactacctg 1200
   agcaccatga agaactttgc actgcagcag ccttcagagg actggatgat cctggagttc 1260
   tcccagctgg gcttcattgg taagatgttc aagtcgctgg acctgagcct gattgtagag 1320
   ttcattctca tgttctaccg ggacaagccc atcgactggc tcctggacca tattctgtgg 1380
   gtgaaagtct gcaaccccga gaaggatgcg aagcactgtg accggcagaa agccaacctg 1440
   cggatccgct tcaaaccgtc cctcttccag cacgtgggca ctcactcctc gctggctggc 1500
   aagatccaga aactgaagga caaagacttt ggaaagcagg cgctgcggaa ggagcatgtg 1560
   aacccgccag cagaggtgag cacgagcctg aagacatacc agcacttcac cctggagaaa 1620
   gcctacctgc gcgaggactt cttctgggcc ttcacccctg ccgcggggga cttcatccgc 1680
   ttccgcttct tccaacctct aagactggag cggttcttct tccgcagtgg gaacatcgag 1740
   cacccggagg acaagctctt caacacgtct gtggaggtgc tgcccttcga caaccctcag 1800
   tcagacaagg aggccctgca ggagggccgc accgccaccc tccggtaccc tcggagcccc 1860
   gacggctacc tccagatcgg ctccttctac aagggagtgg cagagggaga ggtggaccca 1920
   gccttcggcc ctctggaagc actgcgcctc tcgatccaga cggactcccc tgtgtgggtg 1980
   attctgagcg agatcttcct gaaaaaggcc gactaagctg cgggcttctg agggtaccct 2040
   gtggccagcc ctgaagccca catttctggg ggtgtcgtca ctgccgtccc cggagggcca 2100
   gatacggccc cgcccaaagg gttctgcctg gcgtcgggct tgggccggcc tggggtccgc 2160
   cgctggcccg gaggccctag gagctggtgc tgcccccgcc cgccgggccg cggaggaggc 2220
   aggcggcccc cacactgtgc ctgaggcccg gaaccgttcg cacccggcct gccccagtca 2280
   ggccgtttta gaagagcttt tacttgggcg cccgccgtct ctggcgcgaa cactggaatg 2340
   catatactac tttatgtgct gtgtttttta ttcttggata catttgattt tttcacgtaa 2400
   gtccacatat acttctataa gagcgtgact tgtaataaag ggttaatgaa gtgtgtgcct 2460
   caaaaaaaaa aaaaaaaaaa aa 2482
<210> 41
   <211> 7725
   <212> DNA
   <213> Homo sapiens
<400> 41
   agaatcttct gtaggccttt ctcttgcctt cttttattca caactgatga cactgcatat 60
   cttcccctgt tcttattggg agaaggcctt gtgtgtcacc aagaggttct cagaagggac 120
   ctgtcagttt ttggttaaaa gaacccggaa agagaaggac tatgggggaa ctgatggcgt 180
   tcctgttacc tctcatcatt gtgttaatgg tgaagcacag cgattcccgg acgcactctc 240
   tgagatattt tcgcctgggc gtttcggatc ccatccatgg ggtccctgaa tttatttcgg 300
   ttgggtacgt ggactcgcac cctatcacca catatgacag tgtcactcgg cagaaggagc 360
   cacgggcccc atggatggca gagaacctcg cgcctgatca ctgggagagg tacactcagc 420
   tgctgagggg ctggcagcag atgttcaagg tggaactgaa gcgcctacag aggcactaca 480
   atcactcaga taatgtggct cacaccatca agcaggcatg ggaggccaat cagcatgagt 540
   tgctgtatca aaagaattgg ctggaagaag aatgtattgc ctggctaaag agattcctgg 600
   agtatgggaa agacacccta caaagaacag agcccccact ggtcagagta aatcgcaaag 660
   aaacttttcc aggggttaca gctctcttct gcaaagctca tggcttttac cccccagaaa 720
   tttacatgac atggatgaaa aacggggaag aaattgtcca agaaattgat tatggagaca 780
   ttcttcccag tggggatgga acctatcagg cgtgggcatc aattgagctt gatcctcaga 840
   gcagcaacct ttactcctgt catgtggagc actgcggtgt ccacatggtt cttcaggtcc 900
   cccaggaatc agaaactatc cctcttgtga tgaaagctgt ctctgggtcc attgtccttg 960
   tcattgtgct ggctggagtt ggtgttctag tctggagaag aaggccccga gagcaaaatg 1020
   gagccatcta ccttccaaca ccagatcgat gattgcagat ccctcttttc cagttctcct 1080
   tcctctagga gccatgttat cctctgtccc ccatagagtc aagcctagtg cttgaaggtc 1140
   ctgacgacac ccacaacata catgagagta atgggattga gcatttatgg cagcaacaga 1200
   ggagccacaa aatgttcttt gttctttggc tccaaaaaga ctgtcagctt tcagtctctt 1260
   ttgatggact gttttatcag agttgacttt aaatacagct tgtctcatga cacaacgctt 1320
   ccctacattc tatttgtcaa tgatgatttg caactagttg gagattctca gagcaggaag 1380
   gaatcttttc aaccagagca ggaactgtct tctgcaatgc cttggacttg agcctccagc 1440
   ctccacttga acaccatgtg aagggaacct cagtacttca taaaatggcc tttctcattc 1500
   atctttcatg ggaacattta ttgtacaagc gctttgaata tcatgggcac catgactgtg 1560
   accctacagg taggattgga tcactccatg agagtagccg gcaggtttct acaatggcct 1620
   gggaatggac tgattatttt tatacatttt ctggcctgag agaaagccaa agtcccctgc 1680
   tgttcacagc aaccctgcct gggagcttgg aatcttggta atctgcccgg ttggatctat 1740
   ggaggtagtc tcaccctttt tgtcttttgt gggaaattaa gagaaataat tatcagacat 1800
   atcatcacct ccagtggaac tacagagacc tggacccagc tgcactattt taatgtaaaa 1860
   ataacagtat ggccaggtgc agtggctcac gcctgtaatc ccatcacttt gagcagccaa 1920
   ggcgggcgga tcacgaggtc aggagattaa gaccatcctg gccaatatgg tgaaaccctg 1980
   tctctactaa aatacaaaaa attagctggg catggtgttg cgtgcctgta gtcccagcta 2040
   cttgggaggc tgagacaggg gaattgcttg aacccgggag gcagagattg cagtgagccg 2100
   agatcacgcc actgcactcc agcctggcga cagagtgaga ctctatctca aaataataat 2160
   aataataata ataataataa taataataat aacagtatat ttggtgtcag gagagggctc 2220
   aattctcatt tctgcctttc ctgtgctggc tcatggtagc tgggcatgac ttgccttcct 2280
   acataggttg tcttcataca tatgcactgg gaatcaataa aagcccatgg tgagaatgaa 2340
   catcccctta atgttcctta ctatccccaa cccctgaggc ctcacctact gccctgccat 2400
   gtggagctac ttgccctggg gctgccagtc acacattcct cggtcctact tctctgaccc 2460
   cgtttgactc tgcacctgag ccctaatgct tacttcagtg acctgaactt tgacaagtgg 2520
   cttttgtcct gcacctcagg tttgacctct gctctccctt gaccttgact gtgacatttg 2580
   acctttggct ttaatcatta cagcctcaga taaaggtacc ttcagcccgg gcacagtggc 2640
   tcacgcctgt aatcctagca ctttgggagg ctgaggcagg cggattccct gagctcagga 2700
   gttcaagacc agcctgggca acacggtgaa accctgtctc tactaaaata caaataatta 2760
   gctgggcatg gtggcatgtg cctatagtcc cagctacttg ggaggctaag gcaggagaat 2820
   cacttgaatc tataaggcag aggtggcagt gagccgagat cacaccactg cactccagcc 2880
   tgggcaacgg agcaagactc tgtctccaaa aaaaaaaaga aagataccct cagtgtgcca 2940
   ggcctctaag agctcacctg ccaggcttcc tccttgctcc actgtcccat gtaattccat 3000
   atatgaagct accactgtac atctctcttt tccggtgcct gttgagttgc atagaagcac 3060
   agttgtgttt attttgtttt tagggttgcc atgggcaatt tccgtgccac ttttaagcag 3120
   tgttgcactg tgaagagaat gtaggcaagt ttatttctgg aatggtttct tcttacaatc 3180
   agaatagtta ggatgtaata tatttttggg tgggcattta aagtgaaaag gtacatattt 3240
   acatagacac aggtgataat gtatctatgt aaatgccttt tgattctgca actgcaggat 3300
   actctcatca aagacacaga taaaaagcct ctgtgtttcc aaggccttgc cctacaccta 3360
   acacataata tgtccaaatg gatgaagagg aggcaaggac aaggatgtga tgacaaaaca 3420
   ttctgttatg cacttgtagc atttatgttt cttcctgggg gattttataa tactaaaaga 3480
   atcataatat aaagagatga ttaaaaaaaa aatactgccg ggcacggtgg ctcatgcctg 3540
   taatcccagc attttgggag gccgaggtgg gcagatcacc tgaggtcggg agttcgagac 3600
   cagcctgacc aacatggaga aaccctgtct ctaccaaaca tacaaaatta gccggggatg 3660
   gtggcgcatg cctataatcc cagctactcg ggagtctgag gcagaagaac cgcttgaacc 3720
   cgggaggcag aggttgtggt gagccgagat cgcgccatcg cactctagcc tgggcaacaa 3780
   gagtgaaact ccatctcaaa aaaataaaaa taaaataagt aagtaatacc taaaattctg 3840
   caaccttcat tttactatag atggttgaag attatattac ttcttaattg ttttagcctt 3900
   gttattgctt cattacttca tggttgttga gtacagatgc tcagtaatca taacctatga 3960
   aatatttgac accatgatct aaacattaaa aacaaataac tgtgctattg ccacagctat 4020
   gttttggctt tgaattttct ttactgaata ttttggatca agaacactag atgagaaacc 4080
   tgttcaactc tgttcttttt ttttttttta actccactgt atttattacc tgtttttgtt 4140
   ttttttgttt gtttgttttt gatgtgcata tagaccaaga tgtggtaaat ttaaatggca 4200
   gatgtttttg atgtggtaca tttaagggag agaaagcagt ttaaagagca gggtgaaaaa 4260
   tccaacaaga ctccatcgag agtttctgag ctctcccatc aggggccagt cctcctttcc 4320
   tctctcctct tactcccatg atttccaagt tgtgatcctt tccttatttc tgaggaatga 4380
   cttggtttct cctcttcttt ttttggcctg agagaagatg tttttgcact tgtagctatg 4440
   aggaacagat tgtccactag ggaggccagc tgatcatttt ctgccagagt cacacagagc 4500
   agtcacacct tattttgaaa accactgtct ggggtctttg tcctcacata tgcaggtcta 4560
   gtgtccccac aaagtgatca gatggatata taaagtggag tgccaatgta ttaatttact 4620
   gtgagaaaca caattgctaa gtgggtcaga tatctgtctc agctggtcag tacaccttcc 4680
   agcaggaaaa tctacataag aacaactaaa tcacaatctg tagagtgctt gatgacccca 4740
   gaattggtgc aaggggacac attcttgctt gttagcacct gctctctgga gtttgctatt 4800
   ttctcacaca cagtgtatta gttcacagaa tgttctccaa ggaggacagg gggctttgcc 4860
   catagccatg tgctgtgggc agcagagcta ggaagaagca caggcatctc ccagcccagg 4920
   tgtttcccac ttaactgcat tgcccttttc atcttttttt tttccccaat agcttcagga 4980
   cattcagtac attgtgcttt ttagaggttg atgattccac tgcctgagct gccttcacct 5040
   ctctttcttt ggaaattgcc atctttgagc attgtatgtc tctgtaacat ctctgcatct 5100
   ccttttcact ctggcctccc tttctccatt gtccttctag cttctggttg ccccaaaccc 5160
   cacagactgt gtaacaaaac ccaaaaccta ttggtttaaa acaataccca ttttattttc 5220
   tttcatagtt tctatgggtc aggaatttgg atatggcttg ggtaagcagt tctggcttct 5280
   catggagttg gaggcaagtg gtggctggaa cagaaatggg gcagccaggg gtggtggcca 5340
   ggcatcagct acatctcctc catgtagtct caggaactct ccatgtaatc tctctgtgtg 5400
   ggttgggctt cctcacaaca tggtggcctc agggcagtca ctgcttacat ggcagctagc 5460
   ttccctcaga gtgagtatcc caagagatca cagtcaaagt gcatagcatt tatataatct 5520
   aatcttggaa gtcacaaaat gtcattcttg tgatactctc attggtgaag aagtcataaa 5580
   atcctgctca ggttcaaaaa gagggtgtat agaacccaac actcaatagg aagattgtca 5640
   gagttacatt gtaagaagag catgtagttg ggagatatca ttgtggccat tttgggaaag 5700
   atgcaacatg tcacaatgtc atgttctcca tcgtctcccc gttccacctt gtactggttt 5760
   ccaagggtag tcgtttaaaa gtaccacaaa aactgggtgg cttggaacaa cagaaattta 5820
   ttgtttcaca gttctggagg ccagaagttc aaaatgaagt gttggcagaa acatgctgcc 5880
   tctaaaggga ctgggaaagg atctgttcca ggcttccctc ctatcttctg gttgttcctt 5940
   gccttctggc tgcgtaactc tagtcttcat ttggtgttct ccctgcatgc atggctttgt 6000
   gtccaaattt ccttttttca tgaggccatc agtcatactg gattagggac ccactctact 6060
   actgcaggat gacctcacct tcccaaatta catctgcaac aaccctattt ccaaagaagg 6120
   ccacattctg aggtactggg ggttataact tcaacatgaa ttttttggag gaaacaattc 6180
   aactcctaac acatctctac ccaacctctg taggttccct caatccacca aaattttttg 6240
   gctccactgg attatctagc agttaggaaa ccaagatcat taaatgaatt caccgcaact 6300
   acagtgagac tgactacctt agcttacctc tgtgaaagga gttaagccaa aggaatctgt 6360
   ggaattttgg agagtttggg agcatttggg ggcaggaggc aaatgttctt cctttaaatc 6420
   acaacactta gttctcttcc atttataaga ctcacccctc catcccaacc cctgcaccac 6480
   aggacaagga agtgttcttg gtcttcaact ttcatccctg atggtgaaag cagttgctcc 6540
   tgacctattt gcccaccagc ttctcctctg gagcctgagg cttctgatgc ctgcctggct 6600
   ggttctcagt aagaaggtca agttcaacca gaggggagat gctgatgcct ttcagtactt 6660
   aaatatgagt tcagaccctg gggcctggac ataagatttg gggtcccctg gatataagat 6720
   ttctgaaaac actcagactg tggagacccc tgctgaggga gaagccccaa actgtggctt 6780
   caggggaatg caccaaggct ctcattgagg ccaccttctc caacaagctc ccctcctgct 6840
   tccccatggc tggcatggct gaggaaaaag gacactgagc acagcccgtg catgagcggc 6900
   ttgccatgca acaggataaa acccataatg ccactcagca agccttggtt gtaaatctag 6960
   tttgattaca tttgtaatca aatgatggcc atttgttctg tttctggttt gtgaaccaac 7020
   tgaagacata agcagggcct cagctaaccc acaaatagca catgtgtgca aactggaaaa 7080
   atgaaccctt cttctgggag gacgccagcc caggccaggt cacccggctt ggccagcaga 7140
   acacagagta gattttggtc ccgtttgttc cccagtgggg tatctatcct tgtgcagggc 7200
   acaagcctac atggtggctc tggtcatatc attagaaaat agacagaaat gggctgcaca 7260
   ccagaatgaa tgaattgaat tgaaagggag gagtgatggt ggaaaaaaaa acaagtcaat 7320
   tcatttagac tggtagaacc agaaccactg tgtagtacat ccaaacggtt aaaattccct 7380
   ggaagatgtt acataatcct atcatggtgt ttatttatgg aaatctattt taaaaatttt 7440
   atgtaatact gcacagtctg tttgcatgat gccttgtacg tagtagcaac tcagtaaata 7500
   ctttttgaat gaactagtat agtattttaa ttagctagtc ttcatgtact ggtacaaaag 7560
   aacagtgtca tcttacagct gaagtcatag aggggaaata tcccactcaa gatcatataa 7620
   cattccaggt actcaggatg aatggtttga ggactggtct gaattcttca aaggtttcag 7680
   ctgtattaac attctccatc taataaactt tatcttgtca ttgca 7725
<210> 42
   <211> 4033
   <212> DNA
   <213> Homo sapiens
<400> 42
   cggcggccgc ggggcccggc gggcgcgacg ctgcctcctc accggcgcag gctaggaggg 60
   ggcggcctga gtgccgtagc cgagccgggg ctggagcgcg cggtctgacc tacgagaaac 120
   atggcaacca gcgccgtccc cagtgacaac ctccccacat acaagctggt ggtggtgggg 180
   gatgggggtg tgggcaaaag tgccctcacc atccagtttt tccagaagat ctttgtgcct 240
   gactatgacc ccaccattga agactcctac ctgaaacata cggagattga caatcaatgg 300
   gccatcttgg acgttctgga cacagctggg caggaggaat tcagcgccat gcgggagcaa 360
   tacatgcgca cgggggatgg cttcctcatc gtctactccg tcactgacaa ggccagcttt 420
   gagcacgtgg accgcttcca ccagcttatc ctgcgcgtca aagacaggga gtcattcccg 480
   atgatcctcg tggccaacaa ggtcgatttg atgcacttga ggaagatcac cagggagcaa 540
   ggaaaagaaa tggcgaccaa acacaatatt ccgtacatag aaaccagtgc caaggaccca 600
   cctctcaatg tcgacaaagc cttccatgac ctcgttagag taattaggca acagattccg 660
   gaaaaaagcc agaagaagaa gaagaaaacc aaatggcggg gagaccgggc cacaggcacc 720
   cacaaactgc aatgtgtgat cttgtgacag gcctgaggcc ctgggcacag tgacggtggc 780
   ctggccagcc ctcgggaccc ctccccacct aactgcactg aaaccatttc taaccacaac 840
   ccttggccca aggacttggt acaggaaggg agaagggcag gtgggcaggg agcagacagg 900
   gtctggcttt gcccagaggg cacgggcttt cccacctctc aaagagacaa ggaagccacc 960
   tgtaagcaga agcagcatcc aagtgcccct ggccccccca tgtgttgatt caacccggtt 1020
   cctccccctc tctcggtggg tgtgttgttt attgtaacta catagtgttg gtttgatgtg 1080
   gaagtgttta tccacataca aagtacaaaa caagccatga acaagcttct ttcccttacc 1140
   ccccatccac aatgtctgag cttggatgtc ttttatagat ttttaaatta ttttagtgat 1200
   tattatttta ttaaaggggt ctgggctcac tgcctggtga agtttcaagt gttcagcaga 1260
   cctctctggt aacatatctg gaatattgtt gttgtttttt aaccgagttt tcccatcagt 1320
   gccaaaactc aactcaatct gaaagtagag tgtctgagag gacagaaggt aatgggaact 1380
   gtagctggag gcctcaggcc atgggtcaaa cctgggaggg aaagagaccc tacacatggc 1440
   ctagaaatga gagaagagag aggtatttac ccagaggatt ttcctatggt tggggatgca 1500
   aatattagaa aacagattgt attttgctga ggggagtggc tgtcatgagc atgtcagttc 1560
   taaaaggggt tttcattatc ctggaaatgt ataaactaaa gtaagctgat tggctttgca 1620
   aacatgttca tttgtttttc agacagtatg ggttaagttc tctgccctcc ccaggggtct 1680
   gaggaggctc tgggtttctc agatctgtct cttgctgcgt tttcacatca gctgtgctgc 1740
   ttggtgcctc tctgatacga atacactgac acgtcaaagt aacctaatgt ggacaccatc 1800
   cagaaaactc cagttcatgc tggatcttaa ccaaaaatga ttcaatactg ttatcactaa 1860
   aacagcacca agacctgaag ccatcttccc ttggagtcaa ctgactacca cctctataag 1920
   cctagtcaat gagcagaccc cttccagtat ttgtaaaagt agtactaggt tgcctttttg 1980
   gcaattttta ttgacctgtt gaatcttgac tataaaatga tctgagaagt aaggaaggct 2040
   gggctgatgt gtggctctca tataccttct gcaagggggc agtctcccca gctccctgat 2100
   gatgctcacc cccgcccccc cacctcaggt gctgctggtg tgagccaaag actggagttt 2160
   ttccagctgg ggtgggagtg gagagacaac aggaacaacg ctgcaccaaa gaaaaggtca 2220
   gaataaaagg cagcacagct ggtgacctta ttttctagat gttacaaatc aggtcactat 2280
   gcaaactaga atatcctcag caggtggcct ggccactctg gagaaagaaa cccaaggaaa 2340
   gtgagcaccc aactggatgc caagacaccc gggttctgaa aatgtgctgt gttcctacct 2400
   cggcaagatc accagcactg aggggcccag ctggagaatg attctgctac aaaaggagac 2460
   agttgagact tttgcttgtt ggaaatcaaa cttcttattt gtctaaattg cccctttttc 2520
   tgttcctaaa aggaaggata agagagaaca ttccaggtga ggcacttcaa agtttcctta 2580
   gaccctatag tgttaagagg tattttaaac actaaaagga caaagctctt cccaatcctt 2640
   atgcttccct aagtggtatc tgcagcagtt tgttgtgtgc agtttgatgg cagctgcaaa 2700
   ctggaggtga ggcggaggaa aggcaggtag gaaggagtaa ggatggagat gctcagaatc 2760
   aagagcatgg cggagtagga gaagaagccc tgcacacagg gcagtgtcca cagccagaaa 2820
   actcctgctg ggcaccaacc actacgagca taccccatgc ccaccgtgga gctgcaactc 2880
   ctcgacagca ctgagtttga tagtctcact ggaagcagat cagctgatgt agaacagaga 2940
   cctcggccat aaaggtgaga agacataggg atttcaacca cacagttggg acagaaggga 3000
   cagtgcatct gttcatccat cctgcacttg gcccacgttg aactccatgg tgcctgagag 3060
   agactagtta agggttggtc ttctgtatcc tctgctgttg agcctctggt aagctttcat 3120
   ctcccatgaa ctcatttccc cataaatgaa atgggtaaat aatgccccat ttgtagaagt 3180
   gggccctcat gactgaggta gcttccagat aggccagagt agagtgtaga gtgtgccccg 3240
   tgacatccct ccatcttctc ctccattatc atctagcagg gtcagactgg gaaacctggt 3300
   tggccacgcc acaccatgac cgaggagcca actgggactt ctggctgttt gacatcctca 3360
   tgttcccgtt ggtcttccgg agaatagtgc taccctcaca tcccctggag cacagccttc 3420
   ctgaaatgcc ctcaccccat gcctttgcca ttgtgtgctc tcagatttct tccactgttt 3480
   gacaccctcc ttagagggct gctctttttt ttccagagat aatcctagcc atcctctcca 3540
   ctcccacggc tggggacaat ggccacttac tacctgtgca ctttgccact cgggacacct 3600
   ggatggtttc tcttaggact ttgcccacct ccttctcatg gcacttgctg tggaaaatgc 3660
   ctggctggcc tcgtggggcc tgtctcactt ttccaggaga catgacccac taacgtggca 3720
   actttaaccc aaaggcccct cagacatgtt acagcaaatc tggagccaca gacaggttcc 3780
   ctccattggc agcccattgt gtttgaaatt ccatgtcggg tttacttgga atgaaagata 3840
   cttgaattat tgtgcgcctg tgagcgccca gcttctgttt catagtctta acaggtggcc 3900
   attgtcgtga aacgagtgat gcctgaagat ctcagtgatg tttgaacctt ctgtgtaact 3960
   ttttattaag tctttgtatc tctcgactga ttaataaaga agagaaacac gtaaaaaaaa 4020
   aaaaaaaaaa aaa 4033
<210> 43
   <211> 2960
   <212> DNA
   <213> Homo sapiens
<400> 43
   aaatttagat tttgcaaacc tgtgcattga tgagagtgct attgaaacac attaagaaag      60
   attttcaacg caggaatgtg tcatttcctt tcttcatgta ccagatgctg aaatactatg     120
   agataaagat tttaggtttc aattgtaaag agagagaagt ggataaatca gtgctgcttt     180
   ctttaggacg aaagaagtat ggagcagtgg gatcactttc acaatcaaca ggaggacact     240
   gatagctgct ccgaatctgt gaaatttgat gctcgctcaa tgacagcttt gcttcctccg     300
   aatcctaaaa acagcccttc ccttcaagag aaactgaagt ccttcaaagc tgcactgatt     360
   gccctttacc tcctcgtgtt tgcagttctc atccctctca ttggaatagt ggcagctcaa     420
   ctcctgaagt gggaaacgaa gaattgctca gttagttcaa ctaatgcaaa tgatataact     480
   caaagtctca cgggaaaagg aaatgacagc gaagaggaaa tgagatttca agaagtcttt     540
   atggaacaca tgagcaacat ggagaagaga atccagcata ttttagacat ggaagccaac     600
   ctcatggaca cagagcattt ccaaaatttc agcatgacaa ctgatcaaag atttaatgac     660
   attcttctgc agctaagtac cttgttttcc tcagtccagg gacatgggaa tgcaatagat     720
   gaaatctcca agtccttaat aagtttgaat accacattgc ttgatttgca gctcaacata     780
   gaaaatctga atggcaaaat ccaagagaat accttcaaac aacaagagga aatcagtaaa     840
   ttagaggagc gtgtttacaa tgtatcagca gaaattatgg ctatgaaaga agaacaagtg     900
   catttggaac aggaaataaa aggagaagtg aaagtactga ataacatcac taatgatctc     960
   agactgaaag attgggaaca ttctcagacc ttgagaaata tcactttaat tcaaggtcct    1020
   cctggacccc cgggtgaaaa aggagatcga ggtcccactg gagaaagtgg tccacgagga 1080
   tttccaggtc caataggtcc tccgggtctt aaaggtgatc ggggagcaat tggctttcct 1140
   ggaagtcgag gactcccagg atatgccgga aggccaggaa attctggacc aaaaggccag 1200
   aaaggggaaa aggggagtgg aaacacatta agaccagtac aactcactga tcatattagg 1260
   gcagggccct cttaagatca ggtgggttgg gcgggacatc ctctgctacc atctcattaa 1320
   aaggcccttc acctctggac aagtcatctg cacaactgac ttccaagatc cttttgtgac 1380
   tcctccaaat gactttggtt cccgtgttgt acctgacttc cacatggcct tctctcctgg 1440
   tccctggtgc tgtttgggcc tctgctccca tgctcatacc tcttcttact ccaattactc 1500
   caccatcacc tctctcccct atcaccccca gcctggacac ctctcatgca cggactggag 1560
   ggctgctcca accagtcctc agttctctgc cacccattga cctagagtct tgaacccaat 1620
   ttaatttatt gggttctagg agaactgctg tgttctcacc ctaacttgga agagtgatgt 1680
   ttcagtcaag caaagcgatt cctaccatac aatataacac ttgtgtgagg ctctgtccta 1740
   aatatctcaa ttaccaatat gtggtttggt agtatttctc gccatgcttt gctcatgcgc 1800
   aatgagacta caactagggt gtaaatttta agtatcccat ctaaaactca tacaatgata 1860
   ggaaaaatcc atttgttttt catttgattt ttactgagga atcagctcaa tcttcaatga 1920
   atactggtct ctttccaaag catttttgat caaagtaaag actgagtcaa gggctttttt 1980
   tttttctttt tcttgtttta agagacagag ccttgttcta ttgcacaggc tggactacac 2040
   gcattcacct agagtctaga acacaattta atttattggg ttctaggaga actgtcatga 2100
   gtattgataa tatgagagtt ctttatattc aaacattatt ctcaaccaga gatagggatg 2160
   tcatagaaga aaatccattc attcaatcat taattcacat gtccattatg tacctccatg 2220
   agctggacat aacagctaat aagagataat tgtctctggt tttacagagc taattgtccc 2280
   taagagatgt agacaaatga acaagcaatt acaatacatc taagctatac tgggggagga 2340
   acagggctgg ataggtatgc agaggagata aaaaaatttt aattccttag aatatttttt 2400
   aaaaattgat tcttatttta ccttctcatc ttcttatttt ccaaattaca gcatatatat 2460
   atatatatat atatatatat atatatatat atatatatat attttttttt tttttttttt 2520
   ttttttttta agttttgaag tgtagtcgag cttgggcaat ttatccaacc catttaaacc 2580
   aaaaataaaa cttttcatgt attacctggt catttcaaac aaaaatattt tgatcatgaa 2640
   aaagaatacc aatattcttt tgttctaaaa atctcttatg ggattacatg ttatattttt 2700
   ggtttctctc tactgatcaa cagactacat tttcacaact cttctttcct ttacgtttta 2760
   acacacagac ccaagattca tactattaag attctagtag aactctagat ggtatgcctc 2820
   tgtgtatctc agcattttta ttcccactct tgtataatga acatgttaac acctacctca 2880
   cagggttgtt gtgaggatca agtaagatat tgtgtgtgtg aagatgctct gtgaaatcat 2940
   aaagtccttt aaagatgtaa 2960
<210> 44
   <211> 2088
   <212> DNA
   <213> Homo sapiens
<400> 44
   gagctacttg aagaccaatt agagtccggg aagcgcggcg gggcctccag accggggcgg 60
   gcttaagggt gacatctgcg ctttaaaggg tccgggtcag ctgactcccg actctgtgga 120
   gtctagctgc cagggtcgcg gcagctgcgg ggagagatga ctggggagcg acccagcacg 180
   gcgctcccgg acagacgctg ggggccgcgg attctgggct tctggggagg ctgtagggtt 240
   tgggtgtttg ccgcgatctt cctgctgctg tctctggcag cctcctggtc caaggctgag 300
   aacgacttcg gtctggtgca gccgctggtg accatggagc aactgctgtg ggtgagcggg 360
   agacagatcg gctcagtgga caccttccgc atcccgctca tcacagccac tccgcggggc 420
   actcttctcg cctttgctga ggcgaggaaa atgtcctcat ccgatgaggg ggccaagttc 480
   atcgccctgc ggaggtccat ggaccagggc agcacatggt ctcctacagc gttcattgtc 540
   aatgatgggg atgtccccga tgggctgaac cttggggcag tagtgagcga tgttgagaca 600
   ggagtagtat ttcttttcta ctccctttgt gctcacaagg ccggctgcca ggtggcctct 660
   accatgttgg tatggagcaa ggatgatggt gtttcctgga gcacaccccg gaatctctcc 720
   ctggatattg gcactgaagt gtttgcccct ggaccgggct ctggtattca gaaacagcgg 780
   gagccacgga agggccgcct catcgtgtgt ggccatggga cgctggagcg ggacggagtc 840
   ttctgtctcc tcagcgatga tcatggtgcc tcctggcgct acggaagtgg ggtcagcggc 900
   atcccctacg gtcagcccaa gcaggaaaat gatttcaatc ctgatgaatg ccagccctat 960
   gagctcccag atggctcagt cgtcatcaat gcccgaaacc agaacaacta ccactgccac 1020
   tgccgaattg tcctccgcag ctatgatgcc tgtgatacac taaggccccg tgatgtgacc 1080
   ttcgaccctg agctcgtgga ccctgtggta gctgcaggag ctgtagtcac cagctccggc 1140
   attgtcttct tctccaaccc agcacatcca gagttccgag tgaacctgac cctgcgatgg 1200
   agcttcagca atggtacctc atggcggaaa gagacagtcc agctatggcc aggccccagt 1260
   ggctattcat ccctggcaac cctggagggc agcatggatg gagaggagca ggccccccag 1320
   ctctacgtcc tgtatgagaa aggccggaac cactacacag agagcatctc cgtggccaaa 1380
   atcagtgtct atgggacact ctgagctgtg ccactgccac aggggtattc tgccttcagg 1440
   actctgcctt caggaacacg ggtctgtaga gggtctgctg gagacgcctg aaagacagtt 1500
   ccatcttcct ttagactcca gccttggcaa aatcaccttc cctttaccag ggaaatcact 1560
   tcctttagga ctgaaagcta ggcgtcctct cccacaaaaa agtcctgccc tcatctgaga 1620
   atactgtctt tccatatggc taagtgtggc cccaccaccc tctctgccct cccgggacat      1680
   tgattggtcc tgtcttgggc aggtctagtg agctgtagaa ttgaatcaat gtgaactcag      1740
   ggaactgggg aaggctgagc ctcctctttg gtgttgcggt aagataaccg acagggctgg      1800
   tgaaagtccc cagatggcag gatatttggt ttcagagtaa ggactaggtg caccaccatg      1860
   actgactatc aatcaaaatg tttgtaactt aaaattttta atgaaggata atgaatattt      1920
   gtagagtctc tatggttctg tcaatgcaca tcttcgtgtc tgttttcctc atgtatcctt      1980
   gtgagcctgg gtgagttctg gggagagacc tgatgtgcgt actgcctgtg aaaatctgac      2040
   tttggcaaat caaatcctct tttccttttg aaaaaaaaaa aaaaaaaa                   2088
<210> 45
   <211> 4827
   <212> DNA
   <213> Homo sapiens
<400> 45
   gaagggcaac acggggacct tgaagcgggg tcgcggcggc gccccagccc gggccaggga 60
   gtcccggcag cggcacctcc cagaaagggc ggagccgacg acgccttctt ccttcctgac 120
   cggcgcgcgc agcctgctgc cgcggtcagc gcctgctcct gctcctccgc tcctcctgcg 180
   cggggtgctg aaacagcccg gggaagtaga gccgcctccg gggagcccaa ccagccgaac 240
   gccgccggcg tcagcagcct tgcgcggcca cagcatgacc gctcgcggcc tggcccttgg 300
   cctcctcctg ctgctactgt gtccagcgca ggtgttttca cagtcctgtg tttggtatgg 360
   agagtgtgga attgcatatg gggacaagag gtacaattgc gaatattctg gcccaccaaa 420
   accattgcca aaggatggat atgacttagt gcaggaactc tgtccaggat tcttctttgg 480
   caatgtcagt ctctgttgtg atgttcggca gcttcagaca ctaaaagaca acctgcagct 540
   gcctctacag tttctgtcca gatgtccatc ctgtttttat aacctactga acctgttttg 600
   tgagctgaca tgtagccctc gacagagtca gtttttgaat gttacagcta ctgaagatta 660
   tgttgatcct gttacaaacc agacgaaaac aaatgtgaaa gagttacaat actacgtcgg 720
   acagagtttt gccaatgcaa tgtacaatgc ctgccgggat gtggaggccc cctcaagtaa 780
   tgacaaggcc ctgggactcc tgtgtgggaa ggacgctgac gcctgtaatg ccaccaactg 840
   gattgaatac atgttcaata aggacaatgg acaggcacct tttaccatca ctcctgtgtt 900
   ttcagatttt ccagtccatg ggatggagcc catgaacaat gccaccaaag gctgtgacga 960
   gtctgtggat gaggtcacag caccatgtag ctgccaagac tgctctattg tctgtggccc 1020
   caagccccag cccccacctc ctcctgctcc ctggacgatc cttggcttgg acgccatgta 1080
   tgtcatcatg tggatcacct acatggcgtt tttgcttgtg ttttttggag cattttttgc 1140
   agtgtggtgc tacagaaaac ggtattttgt ctccgagtac actcccatcg atagcaatat 1200
   agctttttct gttaatgcaa gtgacaaagg agaggcgtcc tgctgtgacc ctgtcagcgc 1260
   agcatttgag ggctgcttga ggcggctgtt cacacgctgg gggtctttct gcgtccgaaa 1320
   ccctggctgt gtcattttct tctcgctggt cttcattact gcgtgttcgt caggcctggt 1380
   gtttgtccgg gtcacaacca atccagttga cctctggtca gcccccagca gccaggctcg 1440
   cctggaaaaa gagtactttg accagcactt tgggcctttc ttccggacgg agcagctcat 1500
   catccgggcc cctctcactg acaaacacat ttaccagcca tacccttcgg gagctgatgt 1560
   accctttgga cctccgcttg acatacagat actgcaccag gttcttgact tacaaatagc 1620
   catcgaaaac attactgcct cttatgacaa tgagactgtg acacttcaag acatctgctt 1680
   ggcccctctt tcaccgtata acacgaactg caccattttg agtgtgttaa attacttcca 1740
   gaacagccat tccgtgctgg accacaagaa aggggacgac ttctttgtgt atgccgatta 1800
   ccacacgcac tttctgtact gcgtacgggc tcctgcctct ctgaatgata caagtttgct 1860
   ccatgaccct tgtctgggta cgtttggtgg accagtgttc ccgtggcttg tgttgggagg 1920
   ctatgatgat caaaactaca ataacgccac tgcccttgtg attaccttcc ctgtcaataa 1980
   ttactataat gatacagaga agctccagag ggcccaggcc tgggaaaaag agtttattaa 2040
   ttttgtgaaa aactacaaga atcccaatct gaccatttcc ttcactgctg aacgaagtat 2100
   tgaagatgaa ctaaatcgtg aaagtgacag tgatgtcttc accgttgtaa ttagctatgc 2160
   catcatgttt ctatatattt ccctagcctt ggggcacatg aaaagctgtc gcaggcttct 2220
   ggtggattcg aaggtctcac taggcatcgc gggcatcttg atcgtgctga gctcggtggc 2280
   ttgctccttg ggtgtcttca gctacattgg gttgcccttg accctcattg tgattgaagt 2340
   catcccgttc ctggtgctgg ctgttggagt ggacaacatc ttcattctgg tgcaggccta 2400
   ccagagagat gaacgtcttc aaggggaaac cctggatcag cagctgggca gggtcctagg 2460
   agaagtggct cccagtatgt tcctgtcatc cttttctgag actgtagcat ttttcttagg 2520
   agcattgtcc gtgatgccag ccgtgcacac cttctctctc tttgcgggat tggcagtctt 2580
   cattgacttt cttctgcaga ttacctgttt cgtgagtctc ttggggttag acattaaacg 2640
   tcaagagaaa aatcggctag acatcttttg ctgtgtcaga ggtgctgaag atggaacaag 2700
   cgtccaggcc tcagagagct gtttgtttcg cttcttcaaa aactcctatt ctccacttct 2760
   gctaaaggac tggatgagac caattgtgat agcaatattt gtgggtgttc tgtcattcag 2820
   catcgcagtc ctgaacaaag tagatattgg attggatcag tctctttcga tgccagatga 2880
   ctcctacatg gtggattatt tcaaatccat cagtcagtac ctgcatgcgg gtccgcctgt 2940
   gtactttgtc ctggaggaag ggcacgacta cacttcttcc aaggggcaga acatggtgtg 3000
   cggcggcatg ggctgcaaca atgattccct ggtgcagcag atatttaacg cggcgcagct 3060
   ggacaactat acccgaatag gcttcgcccc ctcgtcctgg atcgacgatt atttcgactg 3120
   ggtgaagcca cagtcgtctt gctgtcgagt ggacaatatc actgaccagt tctgcaatgc 3180
   ttcagtggtt gaccctgcct gcgttcgctg caggcctctg actccggaag gcaaacagag 3240
   gcctcagggg ggagacttca tgagattcct gcccatgttc ctttcggata accctaaccc 3300
   caagtgtggc aaagggggac atgctgccta tagttctgca gttaacatcc tccttggcca 3360
   tggcaccagg gtcggagcca cgtacttcat gacctaccac accgtgctgc agacctctgc 3420
   tgactttatt gacgctctga agaaagcccg acttatagcc agtaatgtca ccgaaaccat 3480
   gggcattaac ggcagtgcct accgagtatt tccttacagt gtgttttatg tcttctacga 3540
   acagtacctg accatcattg acgacactat cttcaacctc ggtgtgtccc tgggcgcgat 3600
   atttctggtg accatggtcc tcctgggctg tgagctctgg tctgcagtca tcatgtgtgc 3660
   caccatcgcc atggtcttgg tcaacatgtt tggagttatg tggctctggg gcatcagtct 3720
   gaacgctgta tccttggtca acctggtgat gagctgtggc atctccgtgg agttctgcag 3780
   ccacataacc agagcgttca cggtgagcat gaaaggcagc cgcgtggagc gcgcggaaga 3840
   ggcacttgcc cacatgggca gctccgtgtt cagtggaatc acacttacaa aatttggagg 3900
   gattgtggtg ttggcttttg ccaaatctca aattttccag atattctact tcaggatgta 3960
   tttggccatg gtcttactgg gagccactca cggattaata tttctccctg tcttactcag 4020
   ttacataggg ccatcagtaa ataaagccaa aagttgtgcc actgaagagc gatacaaagg 4080
   aacagagcgc gaacggcttc taaatttcta gccctctcgc agggcatcct gactgaactg 4140
   tgtctaaggg tcggtcggtt taccactgga cgggtgctgc atcggcaagg ccaagttgaa 4200
   caccggatgg tgccaaccat cggttgtttg gcagcagctt tgaacgtagc gcctgtgaac 4260
   tcaggaatgc acagttgact tgggaagcag tattactaga tctggaggca accacaggac 4320
   actaaacttc tcccagcctc ttcaggaaag aaacctcatt ctttggcaag caggaggtga 4380
   cactagatgg ctgtgaatgt gatccgctca ctgacactct gtaaaggcca atcaatgcac 4440
   tgtctgtctc tccttttagg agtaagccat cccacaagtt ctataccata tttttagtga 4500
   cagttgaggt tgtagataca ctttataaca ttttatagtt taaagagctt tattaatgca 4560
   ataaattaac tttgtacaca tttttatata aaaaaacagc aagtgatttc agaatgttgt 4620
   aggcctcatt agagcttggt ctccaaaaat ctgtttgaaa aaagcaacat gttcttcaca 4680
   gtgttcccct agaaaggaag agatttaatt gccagttaga tgtggcatga aatgagggac 4740
   aaagaaagca tctcgtaggt gtgtctactg ggttttaact tatttttctt taataaaata 4800
   cattgttttc ctaaaaaaaa aaaaaaa 4827
<210> 46
   <211> 2393
   <212> DNA
   <213> Homo sapiens
<400> 46
   attcttctat tagataacag tagctattta aatacttctg cagaagctca catattttta 60
   gtttgttgaa gttcgtgact gcttcactct ctcattctta gcttgaattt ggaaatgact 120
   tttgatgacc taaagatcca gactgtgaag gaccagcctg atgagaagtc aaatggaaaa 180
   aaagctaaag gtcttcagtt tctttactct ccatggtggt gcctggctgc tgcgactcta 240
   ggggtccttt gcctgggatt agtagtgacc attatggtgc tgggcatgca attatcccag 300
   gtgtctgacc tcctaacaca agagcaagca aacctaactc accagaaaaa gaaactggag 360
   ggacagatct cagcccggca acaagcagaa gaagcttcac aggagtcaga aaacgaactc 420
   aaggaaatga tagaaaccct tgctcggaag ctgaatgaga aatccaaaga gcaaatggaa 480
   cttcaccacc agaatctgaa tctccaagaa acactgaaga gagtagcaaa ttgttcagga 540
   cttcatccag caagcaattt cctattccag ttttccattc tggatggggc tgtctcggag 600
   gaaccccagc tacccatggc tctgggagga cggttctcct ttgatgcccc acttatttag 660
   agtccgaggc gctgtctccc agacataccc ttcaggtacc tgtgcatata tacaacgagg 720
   agctgtttat gcggaaaact gcattttagc tgccttcagt atatgtcaga agaaggcaaa 780
   cctaagagca cagtgaattt gaaggctctg gaagaaaaga aaaaagtctt tgagttttat 840
   tctggaattt aagctattct ttgtcacttg ggtgccaaac atgagagccc agaaaactgt 900
   catttagctg gctgcagaac tcctttgcag aaactggggt tccaggtgcc tggcaccttt 960
   atgtcaacat ttttgattct agctacctgt attatttcac ctagcttgtc ccaagcttcc 1020
   ctgccagcct gaagtccatt ttcccctttt tattttaaaa tttgactcct cttcaagctt 1080
   gaaaaccctc tgaactcagt cttctttacc tcattatcac cttcccctca cactcctaaa 1140
   attgcatgaa agacagaaca tggagaactt gctcaagtgc aggcagagag caaaaagggg 1200
   aaatatgtct gggaaaaagt gcacgtgaag aaacaaagaa ggacagaggc cattccgaaa 1260
   tcaagaaact catgttctta actttaaaaa aggtatcaat ccttggtttt taaactgtgg 1320
   tccatctcca gactctacca cttacggaca gacagacaga cagacacaca cacacacaca 1380
   cacacacatt ttgggacaag tggggagccc aagaaagtaa ttagtaagtg agtggtcttt 1440
   tctgtaagct aatccacaac ctgttaccac ttcctgaatc agttattatt tcttcatttt 1500
   tttttctacc agaggacaga ttaatagatt taacccttca caacagttct tgttagaatc 1560
   atgggatgtg tggcccagag gtaagaatag aatttctttc cctaaagaac ataccttttg 1620
   tagatgaact cttctcaact ctgttttgct atgctataat tccgaaacat acaagacaaa 1680
   aaaaatgaag acactcaatc tagaacaaac taagccaggt atgcaaatat cgctgaatag 1740
   aaacagatgg aattagaaat atatcttcta tttttaggct tctatttcct ttccacccac 1800
   tcttcacagg ctattctact ttaaaggaag cctttttatt ttgctgcaca caatctagca     1860
   ggaatctttt ttttttttta agagctgtgt catccttatg taggcaagag atgtttgctt     1920
   ttgttaaaag ctttattgag atataattaa cataaaataa actgaacata tttaaagtgt     1980
   actatttgat aagttttcac accttgtgga gaacatgcat actacaatta agagagtgaa     2040
   catatccatc atccctcaaa gtgtcacaat gctcctcctg atgactcctc cccagaaaac     2100
   caccaatcgg ctttcatttt gcattttgta gttttatgtg aatggaatca tatagtatgt     2160
   cttttttttt tgtctggctt ctttcacttt gcataattat tttgagattc atatgtctcc     2220
   atcttgatgc tcgtatgaat tcattctttt aaatgttgaa tattcccttg tatggatata     2280
   ccacaattca tttacccatt tacttgttga tgacatttgg gttgttttag ttttgggata     2340
   ttacaaataa agctgctgtg aacatttgtg tacaagaaaa aaaaaaaaaa aaa            2393
<210> 47
   <211> 1945
   <212> DNA
   <213> Homo sapiens
<400> 47
   ataaatttga gtcagcacca gcgacagctc tgcagtcctc ctatgtggta ctgatcaggt      60
   ggttgcagag cttcagctca cagcaacaca atgcagctga gcaggcaagc acagcccaca     120
   gccagaaaca gttccgactc tacagaacaa gacgaccttt aagtttccca gagaaaatga     180
   gatgctgatg ttgaagacga caccacggct ttgatggaat atcagatatt gaaaatgtct     240
   ctctgcctgt tcatccttct gtttctcaca cctggtattt tatgcatttg tcctctccaa     300
   tgtatatgca cagagaggca caggcatgtg gactgttcag gcagaaactt gtctacatta     360
   ccatctggac tgcaagagaa tattatacat ttaaacctgt cttataacca ctttactgat     420
   ctgcataacc agttaaccca atataccaat ctgaggaccc tggacatttc aaacaacagg     480
   cttgaaagcc tgcctgctca cttacctcgg tctctgtgga acatgtctgc tgctaacaac     540
   aacattaaac ttcttgacaa atctgatact gcttatcagt ggaatcttaa atatctggat     600
   gtttctaaga acatgctgga aaaggttgtc ctcattaaaa atacactaag aagtctcgag     660
   gttctcaacc tcagtagtaa caaactttgg acagttccaa ccaacatgcc ctccaaacta     720
   catatcgtgg acctgtctaa taattctttg acacaaattc ttccaggtac attaataaac     780
   ctgacaaatc tcacacatct ttacctgcac aacaataagt tcacattcat tccagaccaa     840
   tcttttgacc aactctttca gttgcaagag ataacccttt acaataacag gtggtcatgt     900
   gaccacaaac aaaacattac ttacttactg aagtggatga tggaaacaaa agcccatgtg     960
   atagggactc catgttctac ccaaatatca tctttaaagg aacataacat gtatcccaca    1020
   ccttctggat ttacctcaag cttattcact gtaagtggga tgcagacagt ggacaccatt    1080
   aactctctga gtgtggtaac tcaacccaaa gtgaccaaaa tacccaaaca atatcgaaca     1140
   aaggaaacaa cgtttggtgc cactctaagc aaagacacca cctttactag cactgataag     1200
   gcttttgtgc cctatccaga agatacatcc acagagacta tcaattcaca tgaagcagca     1260
   gctgcaactc taactattca tctccaagat ggaatggtca caaacacaag cctcactagc     1320
   tcaacaaaat catccccaac acccatgacc ctaagtatca ctagtggcat gccaaataat     1380
   ttctctgaaa tgcctcaaca aagcacaacc cttaacttat ggagggaaga gacaaccaca     1440
   aatgtaaaga ctccattacc ttctgtggca aatgcttgga aagtaaatgc ttcatttctc     1500
   ttattgctca atgttgtggt catgctggct gtctgagggt ctgcattttc tgaaactaat     1560
   gaaagcactc ctccctgatg tacagttggg aaaatatgtc catatctaac cagtgattcg     1620
   agctatattt aagtattcaa gaaagccagt cttaacattt ctaactctga tgtaaatgaa     1680
   gtaacttgtc ttaaataaaa gaaatgcaca atgtcttggt acttgctgct attttactgt     1740
   cttaattaag taaactaatg agtttctttt ataaaaaaaa tgaaatgttt taaggcttca     1800
   atttattgca caaaatataa agcatctaaa ctttaatatg tattttatgt atgtttacac     1860
   tgtcaaacat ctggaaaata aaaggtctat gctcaaaaaa aaaaaaaaaa aaaaaaaaaa     1920
   aaaaaaaaaa aaaaaaaaaa aaaaa                                           1945
<210> 48
   <211> 2091
   <212> DNA
   <213> Homo sapiens
<400> 48
   aagtgctggg atgacaggtg tgagccaccg cccccggccc ctcgcccgcc ttttgaagga      60
   gcctttcgtc ctcaagggcg aggccactcc ccccccgcga gttccatgcc ccctagaggg     120
   tcatcgttcc cgacggggag gtggcgccct cccccgggcc ccgggccccg accgcccgtg     180
   ctgcctcctt ccgggccctc ctccgcgatg acggcgccgc cagcaggcca ggcggactgg     240
   gcggggctcc gagcggggac tgggacccag accgactagg ggactgggag cgggcggcgc     300
   ggccatggcg ggctgctgcg ccgcgctggc ggccttcctg ttcgagtacg acacgccgcg     360
   catcgtgctc atccgcagcc gcaaagtggg gctcatgaac cgcgccgtgc aactgctcat     420
   cctggcctac gtcatcgggt gctaccatcc ccatttggca gaagtggaaa tggagtcccc     480
   tagaaggtgg gtgtttgtgt gggaaaaggg ctaccaggaa actgactccg tggtcagctc     540
   cgttacgacc aaggtcaagg gcgtggctgt gaccaacact tctaaacttg gattccggat     600
   ctgggatgtg gcggattatg tgataccagc tcaggaggaa aactccctct tcgtcatgac     660
   caacgtgatc ctcaccatga accagacaca gggcctgtgc cccgagattc cagatgcgac     720
   cactgtgtgt aaatcagatg ccagctgtac tgccggctct gccggcaccc acagcaacgg     780
   agtctcaaca ggcaggtgcg tagctttcaa cgggtctgtc aagacgtgtg aggtggcggc 840
   ctggtgcccg gtggaggatg acacacacgt gccacaacct gcttttttaa aggctgcaga 900
   aaacttcact cttttggtta agaacaacat ctggtatccc aaatttaatt tcagcaagag 960
   gaatatcctt cccaacatca ccactactta cctcaagtcg tgcatttatg atgctaaaac 1020
   agatcccttc tgccccatat tccgtcttgg caaaatagtg gagaacgcag gacacagttt 1080
   ccaggacatg gccgtggagg gaggcatcat gggcatccag gtcaactggg actgcaacct 1140
   ggacagagcc gcctccctct gcttgcccag gtactccttc cgccgcctcg atacacggga 1200
   cgttgagcac aacgtatctc ctggctacaa tttcaggttt gccaagtact acagagacct 1260
   ggctggcaac gagcagcgca cgctcatcaa ggcctatggc atccgcttcg acatcattgt 1320
   gtttgggaag gcagggaaat ttgacatcat ccccactatg atcaacatcg gctctggcct 1380
   ggcactgcta ggcatggcga ccgtgctgtg tgacatcata gtcctctact gcatgaagaa 1440
   aagactctac tatcgggaga agaaatataa atatgtggaa gattacgagc agggtcttgc 1500
   tagtgagctg gaccagtgag gcctacccca cacctgggct ctccacagcc ccatcaaaga 1560
   acagagagga ggaggaggga gaaatggcca ccacatcacc ccagagaaat ttctggaatc 1620
   tgattgagtc tccactccac aagcactcag ggttccccag cagctcctgt gtgttgtgtg 1680
   caggatctgt ttgcccactc ggcccaggag gtcagcagtc tgttcttggc tgggtcaact 1740
   ctgcttttcc cgcaacctgg ggttgtcggg ggagcgctgg cccgacgcag tggcactgct 1800
   gtggctttca gggctggagc tggctttgct cagaagcctc ctgtctccag ctctctccag 1860
   gacaggccca gtcctctgag gcacggcggc tctgttcaag cactttatgc ggcaggggag 1920
   gccgcctggc tgcagtcact agacttgtag caggcctggg ctgcaggctt ccccccgacc 1980
   attccctgca gccatgcggc agagctggca tttctcctca gagaagcgct gtgctaaggt 2040
   gatcgaggac cagacattaa agcgtgattt tcttaaaaaa aaaaaaaaaa a 2091
<210> 49
   <211> 4185
   <212> DNA
   <213> Homo sapiens
<400> 49
   cggccgcgag cgcagtggtg tggagcgcgc cgggtcccgg agccggctgt ctgagggatg      60
   gacgagacga gcccactagt gtcccccgag cgggcccaac ccccggacta caccttcccg     120
   tcgggctcgg gcgctcactt tccgcaggtg cccgggggcg cggtccgagt ggcggcggcg     180
   gccggctcgg gcccctctcc gccaggctcg ccgggccacg accgcgagcg gcagccactg     240
   ttggatcggg cccggggcgc ggcggcccag ggccagaccc aaaccgtggc ggcgcaggcc     300
   caggctctgg ccgctcaggc cgcggcggca gcccacgccg ctcaggccca ccgcgagcgg     360
   aacgagttcc cggaggatcc tgagttcgag gcggtggtgc ggcaggccga gctggccatc 420
   gagcgctgca tctttcccga gcgcatctac cagggctcca gcggaagcta cttcgtcaag 480
   gaccctcagg ggaggatcat tgctgtcttc aaacccaaga atgaagagcc ctatgggcat 540
   cttaatccta agtggaccaa gtggctgcag aagctgtgct gtccttgctg ctttggccgt 600
   gactgccttg tccttaacca gggctatctc tcagaagcag gggccagcct ggtggaccaa 660
   aaactggaac tcaacattgt tccccgtaca aaggtagtat acctggccag tgagaccttc 720
   aactatagtg ccattgaccg agtgaagtcc aggggcaagc ggcttgcact agagaaagtg 780
   ccaaaagttg gacagcggtt taaccgcatc gggctaccac caaaggttgg ttcattccag 840
   ctctttgttg aaggctacaa agatgcagac tattggctgc ggcgttttga agcagaacct 900
   cttcctgaga acactaaccg gcaactactg ctccagtttg agcggttggt ggtgctggat 960
   tacatcatcc gcaacactga tcgaggcaat gacaactggc tgattaaata tgactgtcca 1020
   atggatagtt ctagctctcg ggacacagac tgggtggtgg tgaaggagcc tgttatcaag 1080
   gtggctgcca tagacaatgg gctggccttc ccactgaagc atcctgactc ctggagggca 1140
   tatccttttt actgggcctg gttgccccag gcgaaagtcc cattttctca ggagatcaaa 1200
   gatctgatcc ttccaaagat atcggaccct aacttcgtca aggacttgga agaggaccta 1260
   tatgaactct tcaagaaaga tcctggtttc gacaggggcc agttccataa gcagattgct 1320
   gtcatgcggg gccagatctt aaatctgacc caggccttga aagacaacaa gagtcccctg 1380
   cacctcgtcc agatgccacc tgtgattgtc gagacggccc gttcccacca gcggtcttct 1440
   agcgagtcct acacacagag ctttcagagc cggaagccct tcttttcatg gtggtagctc 1500
   cagaggcagg cagaggaaat attgtcagag actggtggga ggaagcctgg ggagtggggt 1560
   gcaggaaaag ccagagaagc cggtggagag cagcaccttt aagagccctc tctctctgct 1620
   tgccaccctg ctcagagctt tccacccaca gggagaagca caatcaggaa cagtgagtgc 1680
   tcctcgccct tctgatgtgg gggaggctgg agctccatgc acgtagtcca gatgcctggg 1740
   aaggaacatc tcccttccag catctgctgg tagcaggctg ggacagtccc ttccttccct 1800
   gaaaccctgc tctattgcaa ttccctatta tattctgcat cagaaaaaca aacaaaacaa 1860
   aaacaacttt aaatgcttgt agcagaaccc cgggtcatct catgtcagaa acctttaatc 1920
   caggcctaaa tttgcataga cctgacattc agctgccttg cagttgcttc ctcccatgag 1980
   ccaaggtggt gtcagagggc aactggatga ctcgcagtac cacagcactg ggacagacag 2040
   aagccacacc tttcttttgg gtttttgcca agcctcctcc atctcccatc agtgctgtgg 2100
   gctggctgca agcctcgaaa cagttctcct ggaagggagg tttttgcttt acccccgcca 2160
   gcacttccgc acacaatcat agagaacctc tctgctctct gctggcctac agcttgtctg 2220
   tttctcaagc agaggcagga agagctagtc ttagcattta tattttaata ggaagttgac 2280
   tcccagcatg taaaagtgat ccacgcagcc ggagtgtatg ccgggagcta agtggtctat 2340
   gggtgaacat atcccacctt gcttcctgag tccttggtcc caatcttctc atttgttcct 2400
   ctcgttttaa attttttccc cccaactctt ttgatgtaag agttcagttt gtcttcggga 2460
   gtgggtctct gcaagggctc tgggatgagt cttggcttcc aagaggacag gctattaggt 2520
   tcttggactt ttttctgtgc taccgctgct gcttggtgga agtaacagga cgtggattct 2580
   gcctcataag tggcagtttc ccttttctct ctgacttgtc ctaggccgat ttctctatgg 2640
   cttccctgag aaaggtgagg cccaaaggag agaggccttc aaactgtccc aggtcctgcg 2700
   cagctcagtg cgtatcttct tgcttccatg tgtcttttcc cctgctgcct cactccccac 2760
   ccccacttgc caggtgtttg agccatttct acaccaaagc aaagtacggc ctcaggaggg 2820
   agtaaaaagg gtgccatctg tgtctggagg ggcagctgtg ttcatgccct gtgctactgg 2880
   acatttcaca attctggcac cttgcgattg gtcagtcaac ctcagaaagt aactatcttg 2940
   aaggtttgaa aaacaaccaa agaaagggag tgaggactat ggctgcatgt cctctgcttg 3000
   cccggctgca gagcagagat gtgcagccct ctggtcagct ggtccaggct ggtccccgcc 3060
   ggtccccttc cagtccagcc accaagagtc cacttgtccc gggcttccac ctggctgaca 3120
   ggaagaattt ctgagagctg gatgtgcatg ccctgtggac gaaggtacag ctcgcctgcc 3180
   tgccccaatc ccagccccga caatcacatg cagctgactc ggacactggc cttgggaaca 3240
   atgttcgaga gaacacttgc cccttgactg taggagccag aaggggaccc aggtgtgcat 3300
   agctctctgt agacattttt acccaaacct gttggtaaag tgcccatctg gtgctcaaga 3360
   gagcctgggg gtctaacagg gagcccggct gcctcacctg gccacagcct ccacaccaga 3420
   tctccacatt gtcttgatcc agaccagctc tgtgatcaga aggaaattgg gtccagtgta 3480
   ggagagagct ggtcctgggc ctggcaggca agagtgtggg catcctttcc tggcctttct 3540
   ccactctccc tcaagcctgt gctcaggttg ccttgaatgt ggactctgga agagccaggg 3600
   gcccagaatg ccgggggagg cttctgagtg gcactcatgg aacaccgtcc ctctgccagc 3660
   cataggccct gcctccagtg tcagggaatg gaggctgggc tgcgagagtg ttgctgcccc 3720
   ctgtgtcatt cttctaatcc aatgtagaaa ttgtacgtaa tgtatttaaa tcaacgcaaa 3780
   tgtatgaata acaaatacag ttctgacctt ttttgtccag tttctttggg ggaaggaaga 3840
   caaagaaggt aggaacggaa ttttgagggc aaagaaacct gtgtttccat ggaattgctg 3900
   agacgtggct cctggggcta tttctcccta ataaaggatg atccaggtcc tcatttccaa 3960
   agtcccaatg ctctgaaaac caaaagtatt ttcataaccc atttgaaacc aaacctgacc 4020
   tgaacttaca ctgataggaa gctatgggta attatgatgt gttcctttta gtgtgattct 4080
   ttgttgcaga aatgtcaata tattttatga catggttccc tactagggat tatacagtat 4140
   ttgctgacta cttcctaaga gccaaaaata aaaaatctga attcc 4185
<210> 50
   <211> 1455
   <212> DNA
   <213> Homo sapiens
<400> 50
   gccgagccag ccccttcacc accagccggc cgcgccccgg gaagggaagt ttgtggcgga 60
   ggaggttcgt acgggaggag ggggaggcgc ccacgcatct ggggctgact cgctctttcg 120
   caaaacgtct gggaggagtc cctggggcca caaaactgcc tccttcctga ggccagaagg 180
   agagaagacg tgcagggacc ccgcgcacag gagctgccct cgcgacatgg gtcacccgcc 240
   gctgctgccg ctgctgctgc tgctccacac ctgcgtccca gcctcttggg gcctgcggtg 300
   catgcagtgt aagaccaacg gggattgccg tgtggaagag tgcgccctgg gacaggacct 360
   ctgcaggacc acgatcgtgc gcttgtggga agaaggagaa gagctggagc tggtggagaa 420
   aagctgtacc cactcagaga agaccaacag gaccctgagc tatcggactg gcttgaagat 480
   caccagcctt accgaggttg tgtgtgggtt agacttgtgc aaccagggca actctggccg 540
   ggctgtcacc tattcccgaa gccgttacct cgaatgcatt tcctgtggct catcagacat 600
   gagctgtgag aggggccggc accagagcct gcagtgccgc agccctgaag aacagtgcct 660
   ggatgtggtg acccactgga tccaggaagg tgaagaaggg cgtccaaagg atgaccgcca 720
   cctccgtggc tgtggctacc ttcccggctg cccgggctcc aatggtttcc acaacaacga 780
   caccttccac ttcctgaaat gctgcaacac caccaaatgc aacgagggcc caatcctgga 840
   gcttgaaaat ctgccgcaga atggccgcca gtgttacagc tgcaagggga acagcaccca 900
   tggatgctcc tctgaagaga ctttcctcat tgactgccga ggccccatga atcaatgtct 960
   ggtagccacc ggcactcacg aacgctcact ctggggaagc tggttgccat gtaaaagtac 1020
   tactgccctg agaccaccat gctgtgagga agcccaagct actcatgtat aaatgccatg 1080
   tggagataga gccccagatg tttcagccat ctcagcccag gcaccagaca agtgggtgaa 1140
   gaagccacct tggacatgta gccccagcag atgtgatata gagaagaaac aggaaacttg 1200
   gctatattag tttcctaggg ctgcctgtga taaattatta caaactttat aaactaacac 1260
   attgtgtgcc tatatcaaaa catcatggaa ggacaggcac agtggctcat gcctgtagtc 1320
   ctagcacttt gggagggtga gaaaggaaga tctcttgagc tcaggagttc aagatcagcc 1380
   tgggcaacac agtgagacct catctccact aaaaataaaa aaaaattggc tggaaaaaaa 1440
   aaaaaaaaaa aaaaa 1455
<210> 51
   <211> 1828
   <212> DNA
   <213> Homo sapiens
<400> 51
   cagttacagg gagcaccacc agggaacatc tcggggagcc tggttggaag ctgcaggctt 60
   agtctgtcgg ctgcgggtct ctgactgccc tgtggggagg gtcttgcctt aacatccctt 120
   gcatttggct gcaaagaaat ctgcttggaa gaaggggtta cgctgtttgg ccgggcagaa 180
   actccgctga gcagaacttg ccgccagaat gctcctcctg ttgctgagta tcatcgtcct 240
   ccacgtcgcg gtgctggtgc tgctgttcgt ctccacgatc gtcagccaat ggatcgtggg 300
   caatggacac gcaactgatc tctggcagaa ctgtagcacc tcttcctcag gaaatgtcca 360
   ccactgtttc tcatcatcac caaacgaatg gctgcagtct gtccaggcca ccatgatcct 420
   gtcgatcatc ttcagcattc tgtctctgtt cctgttcttc tgccaactct tcaccctcac 480
   caaggggggc aggttttaca tcactggaat cttccaaatt cttgctggtc tgtgcgtgat 540
   gagtgctgcg gccatctaca cggtgaggca cccggagtgg catctcaact cggattactc 600
   ctacggtttc gcctacatcc tggcctgggt ggccttcccc ctggcccttc tcagcggtgt 660
   catctatgtg atcttgcgga aacgcgaatg aggcgcccag acggtctgtc tgaggctctg 720
   agcgtacata gggaagggag gaagggaaaa cagaaagcag acaaagaaaa aagagctagc 780
   ccaaaatccc aaactcaaac caaaccaaac agaaagcagt ggaggtgggg gttgctgttg 840
   attgaagatg tatataatat ctccggttta taaaacctat ttataacact ttttacatat 900
   atgtacatag tattgtttgc tttttatgtt gaccatcagc ctcgtgttga gccttaaaga 960
   agtagctaag gaactttaca tcctaacagt ataatccagc tcagtatttt tgttttgttt 1020
   tttgtttgtt tgttttgttt tacccagaaa taagataact ccatctcgcc ccttcccttt 1080
   catctgaaag aagatacctc cctcccagtc cacctcattt agaaaaccaa agtgtgggta 1140
   gaaaccccaa atgtccaaaa gcccttttct ggtgggtgac ccagtgcatc caacagaaac 1200
   agccgctgcc cgaacctctg tgtgaagctt tacgcgcaca cggacaaaat gcccaaactg 1260
   gagcccttgc aaaaacacgg cttgtggcat tggcatactt gcccttacag gtggagtatc 1320
   ttcgtcacac atctaaatga gaaatcagtg acaacaagtc tttgaaatgg tgctatggat 1380
   ttaccattcc ttattatcac taatcatcta aacaactcac tggaaatcca attaacaatt 1440
   ttacaacata agatagaatg gagacctgaa taattctgtg taatataaat ggtttataac 1500
   tgcttttgta cctagctagg ctgctattat tactataatg agtaaatcat aaagccttca 1560
   tcactcccac atttttctta cggtcggagc atcagaacaa gcgtctagac tccttgggac 1620
   cgtgagttcc tagagcttgg ctgggtctag gctgttctgt gcctccaagg actgtctggc 1680
   aatgacttgt attggccacc aactgtagat gtatatatgg tgcccttctg atgctaagac 1740
   tccagacctt ttgtttttgc tttgcatttt ctgattttat accaactgtg tggactaaga 1800
   tgcattaaaa taaacatcag agtaactc 1828
<210> 52
   <211> 3324
   <212> DNA
   <213> Homo sapiens
<400> 52
   gacctcgtga aataaaagtg cagaaaacaa acccaggcga tcacagcagc agccgccgcg 60
   gcagcagcac caacagcagg aggagcagga ggagccggag gaggaggagg aggaggaggc 120
   aaagttagag ttggggctgg cgctccggag ttgctgggct cagcgcagct cccattcatt 180
   aaggaaccag ctgcggagga aggtggccga gcgcccgcgc tgcccactcg ctcgctcgcg 240
   cactcagacg cgcgccacaa cagcgcgccc caagctgcgc agctctgcaa aagtttctgc 300
   tcgggatctg gctctcttcc ccttggactt tagaacgatt tagggttgac agaggaaagc 360
   agaggcgcgc aggaggagca gaaaacacca ccttctgcag ttggaggcag gcagccccgg 420
   ctgcactcta gccgccgcgc ccggagccgg ggccgacccg ccactatccg cagcagcctc 480
   ggccaggagg cgacccgggc gcctgggtgt gtggctgctg ttgcgggacg tcttcgcggg 540
   gcgggaggct cgcgccgcag ccagcgccat gcaaaactac aagtacgaca aagcgatcgt 600
   cccggagagc aagaacggcg gcagcccggc gctcaacaac aacccgagga ggagcggcag 660
   caagcgggtg ctgctcatct gcctcgacct cttctgcctc ttcatggcgg gcctcccctt 720
   cctcatcatc gagacaagca ccatcaagcc ttaccaccga gggttttact gcaatgatga 780
   gagcatcaag tacccactga aaactggtga gacaataaat gacgctgtgc tctgtgccgt 840
   ggggatcgtc attgccatcc tcgcgatcat cacgggggaa ttctaccgga tctattacct 900
   gaagaagtcg cggtcgacga ttcagaaccc ctacgtggca gcactctata agcaagtggg 960
   ctgcttcctc tttggctgtg ccatcagcca gtctttcaca gacattgcca aagtgtccat 1020
   agggcgcctg cgtcctcact tcttgagtgt ctgcaaccct gatttcagcc agatcaactg 1080
   ctctgaaggc tacattcaga actacagatg cagaggtgat gacagcaaag tccaggaagc 1140
   caggaagtcc ttcttctctg gccatgcctc cttctccatg tacactatgc tgtatttggt 1200
   gctatacctg caggcccgct tcacttggcg aggagcccgc ctgctccggc ccctcctgca 1260
   gttcaccttg atcatgatgg ccttctacac gggactgtct cgcgtatcag accacaagca 1320
   ccatcccagt gatgttctgg caggatttgc tcaaggagcc ctggtggcct gctgcatagt 1380
   tttcttcgtg tctgacctct tcaagactaa gacgacgctc tccctgcctg cccctgctat 1440
   ccggaaggaa atcctttcac ctgtggacat tattgacagg aacaatcacc acaacatgat 1500
   gtaggtgcca cccacctcct gagctgtttt tgtaaaatga ctgctgacag caagttcttg 1560
   ctgctctcca atctcatcag acagtagaat gtagggaaaa acttttgccc gactgatttt 1620
   taaaaaggaa aaaaaaaatg ttttactatg tggccttcca aaataggtag tgtttgccta      1680
   tgtggaaaca acagcaaact aacaccaagt gccagagtcc tggatgtgca attggtttaa      1740
   gtgttcatgt tctagtgaac acagcttgtt caggaacaaa cactaaaacg atttgagtag      1800
   aggctgctgg tcaccttttg tgacctgagg aatcccaggc ctgtgagaaa agcaaaaatt      1860
   cacattgcag cacatgatgc cagaaatagc actgaatcaa gaaaatagcc attgcggagc      1920
   tgccctcttg agtctttctg tccatcccat tctattctgt actgtgactt tagttcagga      1980
   agttttgttt tgtgttttaa ataaaaggaa agagcaagtt tgctcagtca agtgatcaga      2040
   tcccgaatct agattcccag ttctaaggcc ttatcacctc ccctgcccat aggccaacaa      2100
   ccatagttcc tcacattagt gattagcaga ctctttgtgg acgagtgaat ttcacaaaca      2160
   gcacaatttc agaagaaatc gagggcacag gccctcactt ttcttctttt gacgcatcat      2220
   cctgtgatgt tgaaatgtca attgcaggat gctgatgttg tgcacgtcaa tcaccgggca      2280
   cttgcatact cttagaaaca gttcgacttc ggttactgcc ttctcccttg aaatccttgc      2340
   tgcgtgccca ccaggatttc ctgtgagggc ccaggaatga gcaaggcatg gtctgccacc      2400
   agctgacgga aagcagcctt ctgtacaaca gatgggaggg tgaagggggc agaatgaaaa      2460
   tcgaaccaac cttttagctg ttgcaaatca gaaggagcca gagaagcagg cagtctcatg     2520
   catgagaggt taccctccag gatgacagag ctgagggtct ttgtaggagt tgctcttgct     2580
   gtgtaaagca ctattgtctt ggggttgagc cctagggcag ttcttggtag gttctgctgg      2640
   gcagaacata tgggttaaat ctcggtagag agtttccctc atcctctatc cgtaagtgtc      2700
   cttccatgca aggtcccact ctaggtgata gacagggacc ccttctactg aacctttgag      2760
   gaaaggagga aggaagaaat gcgtttagat cttggatgca gacctttcaa agggttaaat      2820
   gtaaccatat ggatcaacca catgcacatc cttactacag aatccgtcct ttcatttcaa      2880
   cttatagcaa gctatgattt ttatatataa atattatata aataatgtat aaaacattaa      2940
   aagttaacta tgtaagatat tatttctgaa acaatttagc tatatccact atgattataa      3000
   actgtgtctc gacctgtgtt atttacatta gctgcttaaa aaagcattga gttaattttt      3060
   ttaaatatca actaaaatat catagttctg tggtagacat tgttttataa tgaaataact      3120
   gcaactagag aaaactgtat aaaaacatta aattgtcagt atttttgtaa ggttccattt      3180
   tgtaaagaga ataatattca aagacttttg tagcatacaa agtgaaaact tgtatctgcg      3240
   aaactatact tgtattaaat gtgcttttta aataaaagct cgtaacacaa ctaattaagg      3300
   acttgcaaaa aaaaaaaaaa aaaa                                             3324
<210> 53
   <211> 6107
   <212> DNA
   <213> Homo sapiens
<400> 53
   aaatgacgac aacggtgagg gttctcgggc ggggcctggg acaggcagct ccggggtccg 60
   cggtttcaca tcggaaacaa aacagcggct ggtctggaag gaacctgagc tacgagccgc 120
   ggcggcagcg gggcggcggg gaagcgtata cctaatctgg gagcctgcaa gtgacaacag 180
   cctttgcggt ccttagacag cttggcctgg aggagaacac atgaaagaaa gaacctcaag 240
   aggctttgtt ttctgtgaaa cagtatttct atacagttgc tccaatgaca gagttacctg 300
   caccgttgtc ctacttccag aatgcacaga tgtctgagga caaccacctg agcaatactg 360
   tacgtagcca gaatgacaat agagaacggc aggagcacaa cgacagacgg agccttggcc 420
   accctgagcc attatctaat ggacgacccc agggtaactc ccggcaggtg gtggagcaag 480
   atgaggaaga agatgaggag ctgacattga aatatggcgc caagcatgtg atcatgctct 540
   ttgtccctgt gactctctgc atggtggtgg tcgtggctac cattaagtca gtcagctttt 600
   atacccggaa ggatgggcag ctaatctata ccccattcac agaagatacc gagactgtgg 660
   gccagagagc cctgcactca attctgaatg ctgccatcat gatcagtgtc attgttgtca 720
   tgactatcct cctggtggtt ctgtataaat acaggtgcta taaggtcatc catgcctggc 780
   ttattatatc atctctattg ttgctgttct ttttttcatt catttacttg ggggaagtgt 840
   ttaaaaccta taacgttgct gtggactaca ttactgttgc actcctgatc tggaattttg 900
   gtgtggtggg aatgatttcc attcactgga aaggtccact tcgactccag caggcatatc 960
   tcattatgat tagtgccctc atggccctgg tgtttatcaa gtacctccct gaatggactg 1020
   cgtggctcat cttggctgtg atttcagtat atgatttagt ggctgttttg tgtccgaaag 1080
   gtccacttcg tatgctggtt gaaacagctc aggagagaaa tgaaacgctt tttccagctc 1140
   tcatttactc ctcaacaatg gtgtggttgg tgaatatggc agaaggagac ccggaagctc 1200
   aaaggagagt atccaaaaat tccaagtata atgcagaaag cacagaaagg gagtcacaag 1260
   acactgttgc agagaatgat gatggcgggt tcagtgagga atgggaagcc cagagggaca 1320
   gtcatctagg gcctcatcgc tctacacctg agtcacgagc tgctgtccag gaactttcca 1380
   gcagtatcct cgctggtgaa gacccagagg aaaggggagt aaaacttgga ttgggagatt 1440
   tcattttcta cagtgttctg gttggtaaag cctcagcaac agccagtgga gactggaaca 1500
   caaccatagc ctgtttcgta gccatattaa ttggtttgtg ccttacatta ttactccttg 1560
   ccattttcaa gaaagcattg ccagctcttc caatctccat cacctttggg cttgttttct 1620
   actttgccac agattatctt gtacagcctt ttatggacca attagcattc catcaatttt 1680
   atatctagca tatttgcggt tagaatccca tggatgtttc ttctttgact ataacaaaat 1740
   ctggggagga caaaggtgat tttcctgtgt ccacatctaa caaagtcaag attcccggct 1800
   ggacttttgc agcttccttc caagtcttcc tgaccacctt gcactattgg actttggaag 1860
   gaggtgccta tagaaaacga ttttgaacat acttcatcgc agtggactgt gtccctcggt 1920
   gcagaaacta ccagatttga gggacgaggt caaggagata tgataggccc ggaagttgct 1980
   gtgccccatc agcagcttga cgcgtggtca caggacgatt tcactgacac tgcgaactct 2040
   caggactacc gttaccaaga ggttaggtga agtggtttaa accaaacgga actcttcatc 2100
   ttaaactaca cgttgaaaat caacccaata attctgtatt aactgaattc tgaacttttc 2160
   aggaggtact gtgaggaaga gcaggcacca gcagcagaat ggggaatgga gaggtgggca 2220
   ggggttccag cttccctttg attttttgct gcagactcat cctttttaaa tgagacttgt 2280
   tttcccctct ctttgagtca agtcaaatat gtagattgcc tttggcaatt cttcttctca 2340
   agcactgaca ctcattaccg tctgtgattg ccatttcttc ccaaggccag tctgaacctg 2400
   aggttgcttt atcctaaaag ttttaacctc aggttccaaa ttcagtaaat tttggaaaca 2460
   gtacagctat ttctcatcaa ttctctatca tgttgaagtc aaatttggat tttccaccaa 2520
   attctgaatt tgtagacata cttgtacgct cacttgcccc agatgcctcc tctgtcctca 2580
   ttcttctctc ccacacaagc agtctttttc tacagccagt aaggcagctc tgtcgtggta 2640
   gcagatggtc ccattattct agggtcttac tctttgtatg atgaaaagaa tgtgttatga 2700
   atcggtgctg tcagccctgc tgtcagacct tcttccacag caaatgagat gtatgcccaa 2760
   agacggtaga attaaagaag agtaaaatgg ctgttgaagc actttctgtc ctggtatttt 2820
   gtttttgctt ttgccacaca gtagctcaga atttgaacaa atagccaaaa gctggtggtt 2880
   gatgaattat gaactagttg tatcaacaca aagcaagagt tggggaaagc catatttaac 2940
   ttggtgagct gtgggagaac ctggtggcag aaggagaacc aactgccaag gggaaagaga 3000
   aggggcctcc agcagcgaag gggatacagt gagctaatga tgtcaaggag gagtttcagg 3060
   ttattctcgt cagctccaca aatgggtgct ttgtggtctc tgcccgcgtt acctttcctc 3120
   tcaatgtacc tttgtgtgaa ctgggcagtg gaggtgcctg ctgcagttac catggagttc 3180
   aggctctggg cagctcagtc aggcaaaaca cacaaacagc catcagcctg tgtgggctca 3240
   gggcacctct ggacaaaggc ttgtggggca taaccttctt taccacagag agcccttagc 3300
   tatgctgatc agaccgtaag cgtttatgag aaacttagtt tcctcctgtg gctgaggagg 3360
   ggccagcttt ttcttctttt gcctgctgtt ttctctccca atctatgata tgatatgacc 3420
   tggtttgggg ctgtctttgg tgtttagaat atttgttttc tgtcccagga tatttcttat 3480
   aagaacctaa cttcaagagt agtgtgcgag tactgatctg aatttaaatt aaaattggct 3540
   tatattaggc agtcacagac aggaaaaata agagctatgc aaagaaaggg ggatttaaag 3600
   tagtaggttc tatcatctca attcattttt ttccatgaaa tcccttcttc caagattcat 3660
   tccctctctc agacatgtgc tagcatgggt attatcattg agaaagcaca gctacagcaa 3720
   agccacctga atagcaattt gtgattggaa gcattcttga gggatcccta atctagagta 3780
   atttatttgt gtaaggatcc caaatgtgtt gcacctttca tgatacattt cttctctgaa 3840
   gagggtacgt ggggtgtgtg tatttaaatc catcctatgt attactgatt gtcctgtgta 3900
   gaaagatggc aattattctg tctctttctc caagtttgag ccacatctca gccacattgt 3960
   tagacagtgt acagagaacc tatctttcct tttttttttt ttaaaggaca ggattttgct 4020
   gtgttgccca ggctagactt gaactcctgg gctcaagtaa tccacctcag cctgagtagc 4080
   tgagactaca gcccatctta tttctttaaa tcattcatct caggcagaga acttttccct 4140
   caaacattct ttttagaatt agttcagtca ttcctaaaac atccaaatgc tagtcttcca 4200
   ccatgaaaaa tagattgtca ctggaaagaa cagtagcaat ttccataagg atgtgccttc 4260
   actcacacgg gacaggcggt ggttatagag tcgggcaaaa ccagcagtag agtatgacca 4320
   gccaagccaa tctgcttaat aaaaagatgg aagacagtaa ggaaggaaag tagccactaa 4380
   gagtctgagt ctgactgggc tacagaataa agggtattta tggacagaat gtcattacat 4440
   gcctatggga ataccaatca tatttggaag atttgcagat tttttttcag agaggaaaga 4500
   ctcaccttcc tgtttttggt tctcagtagg ttcgtgtgtg ttcctagaat cacagctctg 4560
   actccaaatg actcaatttc tcaattagaa aaagtagaag ctttctaagc aacttggaag 4620
   aaaacagtca taagtaagca atttgttgat tttactacag aagcaacaac tgaagaggca 4680
   gtgtttttac tttcagactc cgggattccc attctgtagt ctctctgctt ttaaaaaccc 4740
   tccttttgca atagatgccc aaacagatga tgtttattac ttgttattta cgtggcctca 4800
   gacagtgtat gtattctcga tataacttgt agagtgtgaa atataagttt aactaccaaa 4860
   taaggtctcc cagggttaga tgactgcggg aagcctttga tcccaacccc caaggctttg 4920
   tatatttgat catttgtgat ctaaccctgg aagaaaaaga gctcagaaac cactatgaaa 4980
   aaatttgttc agtgttttct gtgttcccgt aggttctgga gtctgaggat gcaaagatga 5040
   ataagataaa ttctcagaat gtagttataa tctcttgttt tctggtatat gccatctttc 5100
   tttaacttct ctaaaatatt gggtatttgt caaataacca cttttaacag ttaccattac 5160
   tgagggctta tacattggtg ttataaaagt gacttgattc agaaatcaat ccattcagta 5220
   aagtactcct tctctaaatt tgctgttatg tctataagga acagtttgac ctgcccttct 5280
   cctcacctcc tcacctgcct tccaacattg aatttggaag gagacgtgaa aattggacat 5340
   ttggttttgc ccttgggctg gaaactatca tataatcata agtttgagcc tagaagtgat 5400
   ccttgtgatc ttctcacctc tttaaattcc cacaacacaa gagattaaaa acagaggttt 5460
   cagctcttca tagtgcgttg tgaaatggct ggccagagtg taccaacaaa gctgtcatcg 5520
   ggctcacagc tcagagacat ctgcatgtga tcatctgcat agtcctctcc tctaacggga 5580
   aacacctcag atttgcatat aaaaaagcac cctggtgctg aaatgaaccc ctttcttgaa 5640
   catcaaagct gtctcccaca gccttgggca gcagggtgcc tcttagtgga tgtgctgggt 5700
   ccaccctgag ccctgacatg tggtggcagc attgccagtt ggtctgtgtg tctgtgtagc 5760
   agggacgatt tcccagaaag caattttcct tttgaaatac gtaattgttg agactaggca 5820
   gtttcaaagt cagctgcata tagtagcaag tacaggactg tcttgttttt ggtgtccttg 5880
   gaggtgctgg ggtgagggtt tcagtgggat catttactct cacatgttgt ctgccttctg 5940
   cttctgtgga cactgctttg tacttaattc agacagactg tgaatacacc ttttttataa 6000
   atacctttca aattcttggt aagatataat tttgatagct gattgcagat tttctgtatt 6060
   tgtcagatta ataaagactg catgaatcca aaaaaaaaaa aaaaaaa 6107
<210> 54
   <211> 2869
   <212> DNA
   <213> Homo sapiens
<400> 54
   agaattcttt ggcaggggcg accttagaat cctggggagg agcgagaatg gaatcccggg      60
   gaggaacagg ggtggaatcc ggggggcggg gtcagaacgc caggaggggg cggggccgga     120
   gccagggtcg gcttgactcg ggggagcagc gggtggatcc tgtgacgtca gcgggttcga     180
   accgccggag ctgagcgaga ggccgggggt gccgagccgg gcggggagag ctgggccggg     240
   agagcagaac agggaggcta gagcgcagcg ggaaccggcc cggagccgga gccggagccc     300
   cacaggcacc tactaaaccg cccagccgat cggcccccac agagtggccc gcgggcctcc     360
   ggccgggccc agtcccctcc cgggccctcc atggcccggg ccgctgccct cctgccgtcg     420
   agatcgccgc cgacgccgct gctgtggccg ctgctgctgc tgctgctcct ggaaaccgga     480
   gcccaggatg tgcgagttca agtgctaccc gaggtgcgag gccagctcgg gggcaccgtg     540
   gagctgccgt gccacctgct gccacctgtt cctggactgt acatctccct ggtgacctgg     600
   cagcgcccag atgcacctgc gaaccaccag aatgtggccg ccttccaccc taagatgggt     660
   cccagcttcc ccagcccgaa gcctggcagc gagcggctgt ccttcgtctc tgccaagcag     720
   agcactgggc aagacacaga ggcagagctc caggacgcca cgctggccct ccacgggctc     780
   acggtggagg acgagggcaa ctacacttgc gagtttgcca ccttccccaa ggggtccgtc     840
   cgagggatga cctggctcag agtcatagcc aagcccaaga accaagctga ggcccagaag     900
   gtcacgttca gccaggaccc tacgacagtg gccctctgca tctccaaaga gggccgccca     960
   cctgcccgga tctcctggct ctcatccctg gactgggaag ccaaagagac tcaggtgtca    1020
   gggaccctgg ccggaactgt cactgtcacc agccgcttca ccttggtgcc ctcgggccga    1080
   gcagatggtg tcacggtcac ctgcaaagtg gagcatgaga gcttcgagga accagccctg    1140
   atacctgtga ccctctctgt acgctaccct cctgaagtgt ccatctccgg ctatgatgac 1200
   aactggtacc tcggccgtac tgatgccacc ctgagctgtg acgtccgcag caacccagag 1260
   cccacgggct atgactggag cacgacctca ggcaccttcc cgacctccgc agtggcccag 1320
   ggctcccagc tggtcatcca cgcagtggac agtctgttca ataccacctt cgtctgcaca 1380
   gtcaccaatg ccgtgggcat gggccgcgct gagcaggtca tctttgtccg agagaccccc 1440
   aacacagcag gcgcaggggc cacaggcggc atcatcgggg gcatcatcgc cgccatcatt 1500
   gctactgctg tggctgccac gggcatcctt atctgccggc agcagcggaa ggagcagacg 1560
   ctgcaggggg cagaggagga cgaagacctg gagggacctc cctcctacaa gccaccgacc 1620
   ccaaaagcga agctggaggc acaggagatg ccctcccagc tcttcactct gggggcctcg 1680
   gagcacagcc cactcaagac cccctacttt gatgctggcg cctcatgcac tgagcaggaa 1740
   atgcctcgat accatgagct gcccaccttg gaagaacggt caggaccctt gcaccctgga 1800
   gccacaagcc tggggtcccc catcccggtg cctccagggc cacctgctgt ggaagacgtt 1860
   tccctggatc tagaggatga ggagggggag gaggaggaag agtatctgga caagatcaac 1920
   cccatctatg atgctctgtc ctatagcagc ccctctgatt cctaccaggg caaaggcttt 1980
   gtcatgtccc gggccatgta tgtgtgagct gccatgcgcc tggcgtctca catctcacct 2040
   gttgatccct tagctttctt gccaaggatc tagtgccccc tgacctctgg ccaggccact 2100
   gtcagttaac acatatgcat tccatttgtg atgtctacct tggtggctcc actatgaccc 2160
   ctaacccatg agcccagaga aattcaccgt gataatggaa tcctggcaac cttatctcat 2220
   gaggcaggag gtggggaagg tgcttctgca caacctctga tcccaaggac tcctctccca 2280
   gactgtgacc ttagaccata cctctcaccc cccaatgcct cgactccccc aaaatcacaa 2340
   agaagaccct agacctataa tttgtcttca ggtagtaaat tcccaatagg tctgctggag 2400
   tgggcgctga gggctccctg ctgctcagac ctgagccctc caggcagcag ggtcccactt 2460
   accccctccc caccctgttc cccaaaggtg ggaaagaggg gattccccag cccaaggcag 2520
   ggttttccca gcaccctcct gtaagcagaa gtctcagggt ccagaccctt ccctgagccc 2580
   ccacccccac cccaattcct gcctaccaag caagcagccc cagcctaggg tcagacaggg 2640
   tgagcctcat acagactgtg ccttgatggc cccagccttg ggagaagaat ttactgttaa 2700
   cctggaagac tactgaatca ttttaccctt gcccagtgga ataggaccta aacatccccc 2760
   ttccggggaa agtgggtcat ctgaattggg ggtagcaatt gatactgttt tgtaaactac 2820
   atttcctaca aaatatgaat ttatactttg accaggaaaa aaaaaaaaa 2869
<210> 55
   <211> 1211
   <212> DNA
   <213> Homo sapiens
<400> 55
   ctgggctccg tgccgctctg tttgccaacc gtccagtccc gcctaccagt gccgggcgct      60
   ccccacccct cccccggctc ccccggtgtc cgccatggcc aaagcctacg accacctctt     120
   caagttgctg ctgatcgggg actcgggggt gggcaagact tgtctgatca ttcgctttgc     180
   agaggacaac ttcaacaaca cttacatctc caccatcgga attgatttca agatccgcac     240
   tgtggatata gaggggaaga agatcaaact acaagtctgg gacacggctg gccaagagcg     300
   gttcaagaca ataactactg cctactaccg tggagccatg ggcattatcc tagtatacga     360
   catcacggat gagaaatctt tcgagaatat tcagaactgg atgaaaagca tcaaggagaa     420
   tgcctcggct ggggtggagc gcctcttgct ggggaacaaa tgtgacatgg aggccaagag     480
   gaaggtgcag aaggagcagg ccgataagtt ggctcgagag catggaatcc gatttttcga     540
   aactagtgct aaatccagta tgaatgtgga tgaggctttt agttccctgg cccgggacat     600
   cttgctcaag tcaggaggcc ggagatcagg aaacggcaac aagcctccca gtactgacct     660
   gaaaacttgt gacaagaaga acaccaacaa gtgctccctg ggctgaggac cctttcttgc     720
   ctccccaccc cggaagctga acctgaggga gacaacggca gagggagtga gcaggggaga     780
   aatagcagag gggcttggag ggtcacatag gtagatggta aagagaatga ggagaaaaag     840
   gagaaaaggg aaaagcagaa aggaaaaaaa ggaagagaga ggaagggaga agggagagga     900
   atgaattgag gaagtgaaag aaggcaagga ggtaggaaga gagggaggag gaaaggaagg     960
   agagatgcct caggcttcag accttacctg ggttttcagg gcaaacataa atgtaaatac    1020
   actgatttat tctgttacta gatcaggttt tagggtcctg caaaaggcta gctcggcact    1080
   acactaggga atttgctcct gttctgtcac ttgtcatggt ctttcttggt attaaaggcc    1140
   accatttgca caaaaaaaaa aaaaaaaaaa aaaaaaaaaa aaaaaaaaaa aaaaaaaaaa    1200
   aaaaaaaaaa a                                                         1211
<210> 56
   <211> 4877
   <212> DNA
   <213> Homo sapiens
<400> 56
   ggcggattgg cgggcacgcc ccctcgcccg cggccccctc cccgcctctc tccaccgcct      60
   cctctggctc cccggtcaga gggccggagc gagaagatgg cgaagacgta cgattatctc     120
   ttcaagctcc tgctgatcgg cgactcgggg gtaggcaaga cctgcctcct gttccgcttc     180
   tcagaggacg ccttcaacac caccttcatc tccaccatcg gaattgattt taaaattaga     240
   acgatagaac tagatggaaa gaaaattaag cttcagatat gggacacagc gggtcaggaa     300
   agattccgaa caatcacgac agcgtactac agaggagcca tgggcattat gctggtctat     360
   gacatcacaa atgaaaaatc ctttgacaat attaaaaatt ggatcagaaa cattgaagag      420
   catgcctctt ccgatgtcga aagaatgatc ctgggtaaca aatgtgatat gaatgacaaa      480
   agacaagtgt caaaagaaag aggggagaag ctagcaattg actatgggat taaattcttg      540
   gagacaagcg caaaatccag tgcaaatgta gaagaggcat tttttacact tgcacgagat      600
   ataatgacaa aactcaacag aaaaatgaat gacagcaatt cagcaggagc aggtggacca      660
   gtgaaaataa cagaaaaccg atcaaagaag accagtttct ttcgttgctc gctactttga      720
   tgaactcttt ctgagagact gcagcacacc tagagggccc tttcctgctt ctctgaaagc      780
   acaggtcacc cagcctcaga atcacacctc ccggctgctg ctgagagcac cactgaactt      840
   agacctctca acacagtatg ccaagtggat tccagcctca tggcctagca aaagaacaga      900
   ctcccttttt caaacatgga agcaatgaag tggagacaca tgcaggacct aactcgtttt      960
   ttccttgttt tattacctgt tgcagaagcg gttatctttc tttttacttt tgcacatcag      1020
   tgttagcctt tccctatttc agcacaatct tagactcata tttgcacact tttgtgtcgt     1080
   gaagttctag acaaatttgt acatgtggca atgttaaaag agcatttaca gcagaggtta     1140
   atatactaaa attaaagggt atttggtctg gttcatatgg tcaaatatta ctgccttggt     1200
   agcatttatt taagggcttt ttcttaaata agaatcatta aagtcattaa aaaaatttac     1260
   tgaaatgccc atcttgtcat caaaggccac aatttcttta tttcttcaga ttaagagctt     1320
   tgcctcatcc ccgacctgtt ttccagagtc tgggtagctg aatgaatcac tttaaaatga     1380
   ttacctctgc ctaatctata gaaactgcat ttggaaatca ccataatctc atttttccct     1440
   ggggtttgta tttgctattc tttcccatgt ttgacttaag tgtaatcact cttaagtaat     1500
   atttgaacat tattatctgt ttctatttgt gaacttcttg agctgaaatt ttacgtgggc     1560
   tgagagatat accatttagg gttttagtgc agcatctaac tgtgattctg tcaataagga     1620
   tatgtaatat atttttcttt aggttcactc cttagctggc tggtttagtt gtaataccaa     1680
   attcctacca taatccctgt ctacaaaagt taggtttaga ttttagtttg cggaaacctt     1740
   ccctatatag agacagatta acttgttgat ataaatttaa tagagctagc tcttggtaat     1800
   ggtgaaaata atgagttttg gttggtttta tttggcagat gtttttagaa ataaaagtac     1860
   ttagacctag tgcagcctct aggaaaagtc ttgccttttc attagagaaa acaggaccaa     1920
   ggtttcagtt ttcaaacagc tgttgttgat tgtgtagaac ccagttccat ctgttttggt     1980
   tcattgttac agaacttagt ccagtcattt gggctaaagc caaccaaaag cttagttgcc     2040
   tttctcaaca aacactggta ctggtatact tttgtagatg aaaccatcac aaggtattta     2100
   gtgttaactt gtgtgccaaa ttcagatcac tatgtcgttg ttgctctagc cttcagtgtc     2160
   ataacacagg ggggataaaa cagaggggat gagggaaatg aattctgtta ataattattc     2220
   ttcctggtat gcctgttttg cttcacaaag gctactatca tgctggatag ataagaacag     2280
   gagatggcag tggaaaggga ttgcttgtta ccacagagaa ttctcttcaa attaagatat 2340
   gtcattagaa tgcttggacc agtcaatctt ttgtacttat ttgaaaatat aggaacaatt 2400
   tagcagctgc aaatatgccc aagctatttt taatagatat actaaactta ttgttgacaa 2460
   atttcagcct ccttaatttt tttttttttg gtaattacct ataggcttaa aagtcattcg 2520
   ttgctgttct agtataatta gtagttccgt gattgaaagt ttattgtaat tctactatct 2580
   tcaaattaga tacattttca aaaggaaaag ataatttttt agaaacatct taatattcac 2640
   tatttcctga aaaaatccaa gcagttaacg ctttctggat ggtaacaaag taccttctaa 2700
   aagataagtg ctatgacacc atgtatgaat gtaattctct tagtaattta cctctgacta 2760
   tttggtgtct taacgctttg gtttatataa tctcaggggt tgtctgcaaa ccaaaactga 2820
   catattctgt ggttagcttt tattactttt ttttttaaca gaatgctttg ctttggttat 2880
   atttcttctt tcttcttttg tcttatatgg attaataact agtctccatg aacttcactt 2940
   gaaagagcct gtaaaagtta gatgagtcta aaagtgctct ttgaagtagc agcaaattgg 3000
   gagtatatgc tctgttatat agaaataaat tgtccttgct attttcttac atttagcttt 3060
   gctagattgt atatacattg agctaagacc ttaggaaatt cactttctgc atgataaaat 3120
   gacccaataa atattccact ttgcttaata atgtacatac agtgcattat tttttctatt 3180
   tgtagatgaa tttaatgaca gataattgtc tgttccccgc tgaaactgaa gaaatctagt 3240
   ttttttgtga acatttttgt ggtcttatgg ataaggtaca tgaagatttt tgcagcagta 3300
   ttagtggttc agtggctgca ctttattaat cagtgtgtta atttttgaca gtgattggac 3360
   tagacctttt caaatagagt ctgagggtat cagacagtga aaatgtgctg taactaagta 3420
   gcatgtaaat cagttgattg taaaacgttt cgctgggaac ttattttagc tatattttac 3480
   ttcagacaga ttatgataca ataatctacc tgtgcatcag ttagtaggtg ctgcagggtt 3540
   tcttactatt tacagaaaca ttttgtgcag tctttgttat aaattttcag aagactatac 3600
   tctttacttt gaaggtctat tttttaatta tacctcattt agctaactag tattctaata 3660
   cctggtagaa aaacagtgag ccagccttta agcatctaac aaaatttaga ctctttgttt 3720
   tgttttgaac tgaagacatc attgggaaag gtaggaaata ttagtttagg atagagcata 3780
   catgtcatat ccagtagcat aaaaaaagta ttatctccct gtctccatta ataaatttag 3840
   ctgtgcaata taggtgcgtt tttgcagaag tttctagtaa gagattataa ctccatttta 3900
   caggttctga atgctcagag tctatattaa ggcttataaa gtttttcctg tgatcagtaa 3960
   gtgacacatt taagcagaca ttcttttcaa gttcatgact tagattcctt tacaaattta 4020
   gttctcaatc tttaaaaacc acatttcatt atgttggtta attattataa attttaagca 4080
   ctcatttctg caatcaggtt tctcagaatt tttttttttt aaacagaaga gacattcctt 4140
   tttccttgtt acatccaagg ggggcacagg ggtgggtgga aaggaatgtc taaaaatgaa     4200
   atcccttgat atagagggac ctagacggac aggatatgct aaaataattt caaattctag     4260
   cacaatttta gagtagaata agtcattttt ttagactaat aaaattaatg gctgtcatgt     4320
   tcactctgaa aaaaatctaa atgactgaaa tgtacagaaa taaaaattag caaacaatta     4380
   ttctagggat attttcagat tttacttcat ttcttgaaat gcgtgtgcca tatgcaattg     4440
   catttcttgt gccaagaaac taatagaact tatttcactt tacctttttt taaaatgtga     4500
   atttagttat tatagttcaa ttttatggcc ttacagatgg cttttatttt gtttgcagct     4560
   gacactgcag ttcctttcat gcaaaatacc ataaactgtt tgatgaaaat catgccccta     4620
   atggaaactc tctagttttt ccatataact atcctactgt acatgtttaa acatatttta     4680
   tttttgctcc aatggcttaa tgtgaaaagc tcctgcagat aagtggacct gtcatgtggt     4740
   taatcttgtt taagccaatt cattaactgt gtactgatac tgatgctatg ttttttttaa      4800
   atggatttta ttccaggtga actttttttt tataataatg ttcgtctaaa ataaaaacta     4860
   cataatgaaa atgaaaa                                                    4877
<210> 57
   <211> 1288
   <212> DNA
   <213> Homo sapiens
<400> 57
   cgtcgccatg ttgttccctc cgcgctggac gggagcagct ggagcgggag cctggctgcg 60
   ctaccgcggc tgcctcctgc tgtgcaggtc cccgaccctc tctctgtcct cattgcgccc 120
   agacgggccg gcccagagct cccgggtcgt ctttcgtgtg gccgcgaggg ttcttgaagc 180
   ttttgagatt aacaatggca ggaaaatcat cactttttaa agtaattctc cttggagatg 240
   gtggagttgg gaagagttca cttatgaaca gatatgtaac taataagttt gatacccagc 300
   tcttccatac aataggtgtg gaatttttaa ataaagattt ggaagtggat ggacattttg 360
   ttaccatgca gatttgggac acggcaggtc aggagcgatt ccgaagcctg aggacaccat 420
   tttacagagg ttctgactgc tgcctgctta cttttagtgt cgatgattca caaagcttcc 480
   agaacttaag taactggaag aaagaattca tatattatgc agatgtgaaa gagcctgaga 540
   gctttccttt tgtgattctg ggtaacaaga ttgacataag cgaacggcag gtgtctacag 600
   aagaagccca agcttggtgc agggacaacg gcgactatcc ttattttgaa acaagtgcaa 660
   aagatgccac aaatgtggca gcagcctttg aggaagcggt tcgaagagtt cttgctaccg 720
   aggataggtc agatcatttg attcagacag acacagtcaa tcttcaccga aagcccaagc 780
   ctagctcatc ttgctgttga ttgttagatt gttgatgcat tctaaccaac tcacacatat 840
   acacaaaatc aacatgggga tggagaagag aattagcgtt tgcagcagtg tatcatctac 900
   taataaaatt aaactaatgt tgctgcttca ttagttggtg ggagaaggga cacatccact 960
   cttggaggaa tatatttact caataatggc accttacatt tataaattgt aacagttgtc 1020
   taataacgtt tctttaattt aaatatgtaa gttgcagagc taataaatga aatgaccaag 1080
   actttaatta taataaaaat aagaaacttg actattctag aagttatact tggatttttt 1140
   cctgggaaaa tggagaacta ctttttatat gtgtatgttt ttatgcaatt agcattgtat 1200
   tcttggttca gggaaatact ttcctaaagc aataatgtta gatattaaag attaaaatct 1260
   aatgtatttg caaaaaaaaa aaaaaaaa 1288
<210> 58
   <211> 2828
   <212> DNA
   <213> Homo sapiens
<400> 58
   agcgccccgg aagtgatctg tggcggctgc tgcagagccg ccaggaggag ggtggatctc      60
   cccagagcaa agcgtcggag tcctcctcct ccttctcctc ctcctcctcc tcctcctcca     120
   gccgcccagg ctcccccgcc acccgtcaga ctcctccttc gaccgctccc ggcgcggggc     180
   cttccaggcg acaaggaccg agtaccctcc ggccggagcc acgcagccgc ggcttccgga     240
   gccctcgggg cggcggactg gctcgcggtg cagattcttc ttaatccttt ggtgaaaact     300
   gagacacaaa atggctgcaa ataagcccaa gggtcagaat tctttggctt tacacaaagt     360
   catcatggtg ggcagtggtg gcgtgggcaa gtcagctctg actctacagt tcatgtacga     420
   tgagtttgtg gaggactatg agcctaccaa agcagacagc tatcggaaga aggtagtgct     480
   agatggggag gaagtccaga tcgatatctt agatacagct gggcaggagg actacgctgc     540
   aattagagac aactacttcc gaagtgggga ggggttcctc tgtgttttct ctattacaga     600
   aatggaatcc tttgcagcta cagctgactt cagggagcag attttaagag taaaagaaga     660
   tgagaatgtt ccatttctac tggttggtaa caaatcagat ttagaagata aaagacaggt     720
   ttctgtagaa gaggcaaaaa acagagctga gcagtggaat gttaactacg tggaaacatc     780
   tgctaaaaca cgagctaatg ttgacaaggt attttttgat ttaatgagag aaattcgagc     840
   gagaaagatg gaagacagca aagaaaagaa tggaaaaaag aagaggaaaa gtttagccaa     900
   gagaatcaga gaaagatgct gcattttata atcaaagccc aaactccttt cttatcttga     960
   ccatactaat aaatataatt tataagcatt gccattgaag gcttaattga ctgaaattac    1020
   tttaacattt tggaaattgt tgtatatcac taaaagcatg aattggaact gcaatgaaag    1080
   tcaaatttac tttaaaaaga aattaatatg gcttcaccaa gaagcaaagt tcaacttatt    1140
   tcataattgc ctacatttat catggtcctg aatgtagcgt gtaagcttgt gtttcttggg    1200
   cagtctttct tgaaattgaa gaggtgaaat gggggtgggg agtgggagga aaggtgactt    1260
   cctctggtgt ttattataaa gcttaaattt tatatcattt taaaatgtct tggtcttcta 1320
   ctgccttgaa aaatgacaat tgtgaacatg atagttaaac taccactttt tttaaccatt 1380
   attatgcaaa atttagaaga aaagttattg gcatggttgt tgcatatagt taaactgaga 1440
   gtaattcatc tgtgaatctg ctttaattac ctggtgagta acttagaaaa gtggtgtaaa 1500
   cttgtacatg gaattttttg aatatgcctt aatttagaaa ctgaaaaata tctggttata 1560
   tcattctggg tgtgttctta ctgacaccag gggtccgctg ccccatgtgt cctggtgaga 1620
   aaatatatgc ctggcacagc ttttgtatag aaaattcttg agaagtaact gtccgctaga 1680
   agtctgtcca aatttaaaat gtgtgccata ttctggttct tgaaaataag attccagagc 1740
   tctttgatcg cttttaataa actgcaagtt cattttaaat gaagggccag catatatact 1800
   tgcaagataa ttttcagctg caaggattca gcaccagtta tgtttgaatg aaccctcctt 1860
   ttctctgaga ttctggtccc tggaaatccc tttctgctag tggtgagcat gtaagtgtta 1920
   agtttttaat ctgggagcag ggcataggaa gaaaatgtca gtagtgctaa tgcattttgc 1980
   actagaacgc ttcgggaaaa tattcatgct tgccatctgt tcatttctaa atttatattc 2040
   ataaagttac agtttgatac aggaattatt aggagtaatt cttttctgtt tctgtttata 2100
   atgaagaaca ctgtagctac attttcagaa gttaacatca agccatcaaa cctgggtata 2160
   gtgcagaaaa cgtggcacac actgaccaca cattaggctg tgtcaccatt gtgtggtgta 2220
   cctgctggaa gaattctagc atgctacttg gggacataat ttcagtggga aatatgccac 2280
   tgaccgattt tttttttttc ctctttgcag tggggctagg acagttgatt caacaaagta 2340
   tttttttctt ttttctcagt cctaatttga acaggtcaaa gatgtgttca ggcattccag 2400
   gtaacaggtg tgtatgtaaa gttaaaaata ggctttttag gaactcactc tttagatatt 2460
   tacatccagc ttctcatgtt aaatatttgt ccttaaaggg tttgagatgt acatctttca 2520
   tttcgtattt ctcataggct atgccatgtg cggaattcaa gttaccaatg taacactggc 2580
   cagcgggccc agcaatctcc atgtgtactt attacagtct tatttaacca ggggtcctaa 2640
   ccactaacat tgtgactttg ctttgagacc tttcctctcc tgggtactga ggtgctatga 2700
   agccaactga caaagatgca tcacgtgtct taggctgatg ccactacccg atttgtttat 2760
   ttgcaatttg agccatttaa agaccaataa acttcctttt ttaaaatgtt aaaaaaaaaa 2820
   aaaaaaaa 2828
<210> 59
   <211> 2092
   <212> DNA
   <213> Homo sapiens
<400> 59
   ggcgtaatta aaaagcggcg gaagaaggtg ggagggtcat gacgcagcga gtttcagtcg 60
   tgacttttct gggggcatcg cggcgtcccc tttttttgcc tttaaagtaa aacgtcgccc 120
   cgacgcaccc cccgcgtatt tcggggggcg gaggcggcgg gccacggcgc gaagaggggc 180
   ggtgctgacg ccggccggtc acgtgggcgt gttgtggggg ggaggggcgc cgccgcgcgg 240
   tcggttccgg gcggttggga gcgcgcgagc tagcgagcga gaggcagccg cgcccgccgc 300
   cgcccctgct ctgtatgccg ctctctcccg gcgcggccgc cgccgatcac agcagcagga 360
   gccaccgccg ccgcggttga tgtggttggg ccggggctga ggaggccgcc aagatgccgc 420
   agtccaagtc ccggaagatc gcgatcctgg gctaccggtc tgtggggaaa tcctcattga 480
   cgattcaatt tgttgaaggc caatttgtgg actcctacga tccaaccata gaaaacactt 540
   ttacaaagtt gatcacagta aatggacaag aatatcatct tcaacttgta gacacagccg 600
   ggcaagatga atattctatc tttcctcaga catactccat agatattaat ggctatattc 660
   ttgtgtattc tgttacatca atcaaaagtt ttgaagtgat taaagttatc catggcaaat 720
   tgttggatat ggtggggaaa gtacaaatac ctattatgtt ggttgggaat aagaaagacc 780
   tgcatatgga aagggtgatc agttatgaag aagggaaagc tttggcagaa tcttggaatg 840
   cagctttttt ggaatcttct gctaaagaaa atcagactgc tgtggatgtt tttcgaagga 900
   taattttgga ggcagaaaaa atggacgggg cagcttcaca aggcaagtct tcatgctcgg 960
   tgatgtgatt ctgctgcaaa gcctgaggac actgggaata tattctacct gaagaagcaa 1020
   actgcccgtt ctccttgaag ataaactatg cttctttttt cttctgttaa cctgaaagat 1080
   atcatttggg tcagagctcc cctcccttca gattatgtta actctgagtc tgtccaaatg 1140
   agttcacttc cattttcaaa ttttaagcaa tcatattttc aatttatata ttgtatttct 1200
   taatattatg accaagaatt ttatcggcat taatttttca gtgtagtttg ttgtttaaaa 1260
   taatgtaatc atcaaaatga tgcatattgt tacactacta ttaactaggc ttcagtatat 1320
   cagtgtttat ttcattgtgt taaatgtata cttgtaaata aaatagctgc aaacctcagt 1380
   cctttgtgct acttgatgtg gctttcaaag aagagaagcc ttgtcctgag tttctcactt 1440
   ggcttcagga aggccccagg ttggattcca gaaaccagtg aagatgtggc cacaggagga 1500
   ggtgtgctga ggtggctgct gaccgtggac tccctgcgca gtggcctgca gatgttgggg 1560
   ctgggttaca gctgattgaa gctgagtggc cctggggggt ctgtgagggg agttcctccc 1620
   cagtgatgaa attctctcct tccaccctca aatccctaga ccttgactga aatgctccgt 1680
   ggtcgggagc ctggtcaagg aggaggagct gctgagaggc attgttcgcc cttgctcata 1740
   gcttagctcg atgtccgtgt cagacaggag atgattgaga acagccttgc ctgtcactgt 1800
   cctagaacac cctggagttt agtgttctgt gtcagagtct tgggagcctc cttcagaccc 1860
   agatgacggg cctccctctg tccaaggagc agctgtaaag gagaagaggg atttcatttg 1920
   tttggtggct gttaccttgt ctgtaagtca aacttggagt tgagcagtgc tttttaaacg 1980
   attccctttt gcagctaaaa tttcacaggg ctatttctaa tacgtaagca aatgttacca 2040
   ttgactttat taataaaata tagttttgct ttgcaaaaaa aaaaaaaaaa aa 2092
<210> 60
   <211> 820
   <212> DNA
   <213> Homo sapiens
<400> 60
   gtataaggtc cacaccccgg gagctgagtg attgcagaaa ctggccttcc atctctctca 60
   gacaccaagc tgcagatcca ggcttttctg ggaaagtgag gccaccatgg ctctggagaa 120
   gtctcttgtc cggctccttc tgcttgtcct gatactgctg gtgctgggct gggtccagcc 180
   ttccctgggc aaggaatccc gggccaagaa attccagcgg cagcatatgg actcagacag 240
   ttcccccagc agcagctcca cctactgtaa ccaaatgatg aggcgccgga atatgacaca 300
   ggggcggtgc aaaccagtga acacctttgt gcacgagccc ctggtagatg tccagaatgt 360
   ctgtttccag gaaaaggtca cctgcaagaa cgggcagggc aactgctaca agagcaactc 420
   cagcatgcac atcacagact gccgcctgac aaacggctcc aggtacccca actgtgcata 480
   ccggaccagc ccgaaggaga gacacatcat tgtggcctgt gaagggagcc catatgtgcc 540
   agtccacttt gatgcttctg tggaggactc tacctaaggt cagagcagcg agatacccca 600
   cctccctcaa cctcatcctc tccacagctg cctcttccct cttccttccc tgctgtgaaa 660
   gaagtaacta cagttagggc tcctattcaa cacacacatg cttccctttc ctgagtccca 720
   tccctgcgtg attttggggg tgaagagtgg gttgtgaggt gggccccatg ttaacccctc 780
   cactctttct ttcaataaaa cgcagttgca aacacctgaa 820
<210> 61
   <211> 7019
   <212> DNA
   <213> Homo sapiens
<400> 61
   gggccggggg gcgatggaat aaaagaagat ggagagactt cagcgcctgg gactcgggtg 60
   ggcgaggcgg aaggtgtcct cgcagcacgg cttttctccg cgccgcggtt ggttagcgag 120
   tgccctctgg gtgctaggcg ttgggcggat ggtaggatcg cggtagcata cggatccgag 180
   tcctgcgccg agtgagagga gaggctggca ggggctaagt gatggatctt gtactccgtg 240
   ttgcagatta ctattttttt acaccatacg tgtatccagc cacatggcca gaagatgaca 300
   tcttccgaca agctattagt cttctgattg taacaaatgt tggtgcttac atcctttatt 360
   tcttctgtgc aacactgagc tattattttg tcttcgatca tgcattaatg aaacatccac 420
   aatttttaaa gaatcaagtc cgtcgagaga ttaagtttac tgtccaggca ttgccatgga 480
   taagtattct tactgttgca ctgttcttgc tggagataag aggttacagc aaattacatg 540
   atgacctagg agagtttcca tatggattgt ttgaacttgt cgttagtata atatctttcc 600
   tctttttcac tgacatgttc atctactgga ttcacagagg ccttcatcat agactggtat 660
   ataagcgcct acataaacct caccatattt ggaagattcc tactccattt gcaagtcatg 720
   cttttcaccc tattgatggc tttcttcaga gtctacctta ccatatatac ccttttatct 780
   ttccattaca caaggtggtt tatttaagtc tgtacatctt ggttaatatc tggacaattt 840
   ccattcatga cggtgatttt cgtgtccccc aaatcttaca gccatttatt aatggctcag 900
   ctcatcatac agaccaccat atgttctttg actataatta tggacaatat ttcactttgt 960
   gggataggat tggcggctca ttcaaaaatc cttcatcctt tgaggggaag ggaccgctca 1020
   gttatgtgaa ggagatgaca gagggaaagc gcagcagcca ttcaggaaat ggctgtaaga 1080
   atgaaaaatt attcaatgga gagtttacaa agactgaata gattattgcc cagttattct 1140
   taagtaagga caaagaagga aatatcatcg tatttctttt ttttaataag gaaaaaataa 1200
   tatccataca gtcaagatac atagtaaatg gtatcatttg gaaatcagca tcgtgggcac 1260
   tgctgaggaa tgatcctagt ggtaggtcag aagaagatgc tgtgaacacc aggactttaa 1320
   tcttatgctt aaaatgccag atgttgttcg gggacaactt tgtatctttc tagcagcaga 1380
   tctgtagttt gtatagcctc aacaacaatt ttaaataaga tggagaataa attattgagg 1440
   ggactaggct atatgcattt gccttcatcc acccatgttt attaagaatc attgtgctta 1500
   ataataccaa gactaagcac cataaccaag aaatactaat gtaaagattg tttcttgttt 1560
   caggaatggt taattcttca acgttggtat gataatgata acttgttttg acttgaataa 1620
   agtactacat cagtgtggaa aaaaattctg atacattagc agctatgtaa atgacctaat 1680
   tgatagcagg tgtaataaga ctatcgtctt cctacacata ggaggctcat tctctggaca 1740
   cactatcacc tattacattt tactgattaa caaataaatt ggaatttaaa aatatcgata 1800
   tcaccatgat ttaatccaga tctgggatta tgtagctaaa cattgtgatg attattattt 1860
   aaaaccatta tttaataaga gtaaaaatat gtgaatctgg atatatttaa aaaaagaaat 1920
   ttgatgccca gataatatat taggcactac tgatttttta gttaaattga tgcactacac 1980
   ttttgatgtt tgaagttaca aacctgtaat ttttttgtaa aggaaataat tgccaaatac 2040
   ctaggcccat tgctgacgat tagttctaaa atcttattcc tcctcttctc ccctcacttt 2100
   tccctacttc ctctgcaaaa agatttaaca aatacattca taaggaaatg tgtgttgtaa 2160
   caaatatatt gcaaaaacat agtttgtaaa ggcattctat aagctattta tgtaaaatca 2220
   ataaaagttg atcataatta aactgtatca gttgagtatt atagcagcac aaagtattct 2280
   ttgtacagat tttgtgccaa tttgaagcca cagaaatgat gtggattgtt aattgtgttt 2340
   tagaacatcc ccggacactc agtgtcacag ggggaaagaa gtgggtacca cattctgttt 2400
   atatttcaca ttttaactag atttgagtgt ttttagcaag aaatcagtct taaaatctaa 2460
   tgtctgggat ccagaagaaa atgtctttaa tctgtgagtt attgtcacaa tgtcatctta 2520
   tttaaatgta ccaattagca ttttgtaata ggcaaatgtc atttagtgct tttcaccaat 2580
   cccactcacc cccggtgctc cgccttgcct aagaaaaaga aattaaggag aagtaaactt 2640
   tatttcctaa tataatgtca gctgatattt attgagcttt tcctctttgc ccagagacta 2700
   ggacccaaag aagttaagta actattccca ggtttatttc tctctcatat gatgtcccat 2760
   gtggatgttt gtggtcagtg gacagctttc cacctagtct ttctgcgacc caggctcctt 2820
   cctcttgggg ctctgccttt ctctcagtcc atagagccct ctttgttgaa agagcacata 2880
   ggaaaagaag gaaaagtctg tgtggaaaat gtttctgggt caggcctgga agtggtgcat 2940
   atctcttccg cccatgttcc tttggacaga actccgtcac atggcccacc tagagagatt 3000
   ttgggaaatg tgtccagctg tgtgcctggg aggaaggggg caccattttc ttgagcagct 3060
   agacagtttg ccgtatttgt ggtgttctcc tcttgttgat gttgaaatgg tgaatgagcc 3120
   ataaagtatt tcaggttatc cacacactaa tcatctcagt gtctttaatt cttaactcca 3180
   atatgaatgt ttaaagcttc ctctagattc ttattcctat ataactaata gagaagaaag 3240
   gacagcttcc tatggggaag acagaggctt cctcatagat gttaggaata atcaaacttg 3300
   cccctgccct ttcacccgtc tcaaattctg gtcttttaaa gcagcgttat gttaagtagt 3360
   cctaacattg taatatacag tactgccaca ttctcctact ttctattaga ggaagtcaga 3420
   gaatatttat ggaagtgagg acccaaatta ccttctacag atgactttta tagttacagg 3480
   acagaaagtg aaaatcaagg ttacgttttc tacttttgtg gtagaaattg agaagtgggt 3540
   ggatatggtt cgagaagacc tttcagaaac acagagactg agtctttgtc ttccatgctg 3600
   tctctgcagt actgagtgaa tttccttata cccttgtatc atgttttcct cccatcttct 3660
   agaagctggg gacagatttg gaagagaatt acacaagttc agttttttga tacatggatt 3720
   ttacagtgca tgcaggttat ttatgtagag aggaggtctg ggagaaagat ggaaactagg 3780
   gagatgactg agaacaaaga tatttgggat taacacagat agaagaaaag tttgaaacca 3840
   tgagattgtc acagcatgaa aaaagatatt caaagacact aaccaacttg aggggtgcag 3900
   tggtctgaat gtgtcctcca aaattcaaat gtggaaatgt atttgccagt gagatagtat 3960
   gtagaggtgg ggccttgagg aagtgattaa gtcatgaggg ctctgggatt aatgacttaa 4020
   aaagaggtgt gaggcagctg ttcagccctt ctgtctcctg tccctttttt tttttttttt 4080
   tttgagatgg agtcttgctc tgttgcccca ggctggagtg cagtggcgtg atcttggttc 4140
   actgcaagct ccacctcccg ggttcacgcc attctcctgc ctcagcctcc cgagtagctg 4200
   ggactacagg tgcccgccac caccacgccc ggctaatttt ttatattttt agtagagatg 4260
   gggtttcacc gtgttagcca ggatggtctt gatctcctga cctcatgatc tgcctgcctc 4320
   ggcctcccaa agtgctggga ttataggtgt gagccaccat gcccagcccc ttctgtccct 4380
   tctaacgtga ggacccagca acaaggtacc atcttgaaag cagcaactgg ggactcagca 4440
   gacaccaaac caactggtgc ttaaccgtga cttcccagcc tccagagctg taagaaaata 4500
   aatttctatt atttatagat tacccagttg aagatacttt gttaaggcag tacaaatgga 4560
   ctaagacaag ggggaagaaa tttgcattcc tgatcttccc aacttcttca aattcacaac 4620
   acttgaatga cagtcttatt tcagcactta ctgctatcac ctattacttt tatgtgtgtc 4680
   ttacctattt gagatgccag attccttgga agtagagacc gtgtctgaat catcattgta 4740
   ttaaaccact catccttaac aaatgcccaa gccatggtta aagttcaata aatactttgt 4800
   tgaatttatt aataaaatgg cagaaatgtc attctcttcc atatatgttt aataaatccc 4860
   tgataggtgc taagcactgc actaggtaaa aattctcttc tgatgctgtc tttttggcca 4920
   accatttttt tatcatttat tcattagctg acatttgcta agtgctttgg aggggtcaaa 4980
   aggggaagta atgagaaatc aaagatggtc cctacatcaa ggataaacta tcttttttta 5040
   gtcactcaaa gtcataaccc tttggaaaca aaacccacca gtaccccaga ttttgaccac 5100
   agatgaatca gtactacaag gactggttag agggttgaat gaatctgtat actcagcact 5160
   taacacagca ctctgggtaa aagaaaaaag atcctcaaag atattagttg gttacatcaa 5220
   gaaaggacaa acttaggtta atctataact tcatctcaga ggaacaggaa ctttggagat 5280
   aaacagggct ctgccacttg caagttgcac catccctggt ttctccatct gtaaattgat 5340
   taaaacactg cctatctaat aagattaaat aagttagaag cattcagtta aatgtcaact 5400
   gaaactattg ttcatgtaaa ttgtgcttga tgctttttct ttctagattc aatgattatt 5460
   gtcattttac ctccataggc cctcaataga aatcagttgc agagggcaga agcctagata 5520
   ttttcacctt aaaattggag ggtgaaagac attgaggtga agtagagata gagggtacac 5580
   agaaaaatcg tataagtaaa actaacatcg ttaacattat ttactgtaag ttatctttgt 5640
   aagagtggta aaatacattg tgttgttaaa taatttcatt taaaaaatgc atcactttgt 5700
   gtgtttttat attgctaaaa ccataaggcc agtctacaag gtttgtagat aaaatagaaa 5760
   cataccttcc ttgaaaagca gaataaattt tttaaaggca ggaaggaagt gtttgaacca 5820
   tgtgtcaaca agctttactg tcaaagcagg cttttggtat gggaagaaaa atacttataa 5880
   atacttgttt taatatttgc tttattaaaa tacatttaaa atacagcatt tttaaatctc 5940
   taagctcaac ttgaagatat aagaacagta aatttgataa aaatgagaaa ttacattccc 6000
   atttctttaa caatttgtaa attccaatta tcctgaacat ttaataccat ttacatattt 6060
   tattaatcac attttcttaa acatttgata agagatttaa tattttgatc caactaccaa 6120
   aaaagcagac ttgtgtactt gacagatttt tctaaacact tcacaactca cgattcaaac 6180
   aaagacaaaa tagcatatca aaagttaatc actcagttgg aaagcactca taccataggc 6240
   ttttattcat ttcttgaata attttgttat atcttcctct tttaggctgc aatgagctat 6300
   aattgcacta ctgcactcca cgctgggtga cagagcaaga ccctatctct aaaaataaaa 6360
   aagtatatat atataaaaat atcttcctct attataattt aactcattaa gccatttatt 6420
   tagatgtaaa cttgcccccc tgacatgtgg tatgaaacaa atagaaacct agaaatttag 6480
   tgcatattca aatattaaga cagacactgg tgtggtgact tttgtctgtc gcttcattgg 6540
   gacgtttttt ctttctgatc aacttaatga aattataatt tactataatt aagtgtagcc 6600
   atttttactg tagagttcaa tgatctttga tgaacgtgta cacccatgta accaccaccc 6660
   ccaatcaaag taaagaacat tttcttacca gaataaattt cctctccgtt tgcagtcatt 6720
   ctccccagcc ctaggtcacc actgatccac cttctgttac tggaaggtta gttttcttcc 6780
   ctgatttaga atttcatata aattaaatca gatagtatat actcttgtgt ttagtttctt 6840
   tagcttaaca tgtttagaga tatttgctgt tgcctgtgtc tgtagctttt tgttttcatt 6900
   gctgaatagt atttcattgt aatataccac agttggttta tgtatttgct gatgaatatt 6960
   tgtgttattt ccagcgtggg attattatga ataaagttgc tacaaacatt tgtatacaa 7019
<210> 62
   <211> 3485
   <212> DNA
   <213> Homo sapiens
<400> 62
   agtcgcccag gggagcccgg agaagcaggc tcaggaggga gggagccaga ggaaaagaag      60
   aggaggagaa ggaggaggac ccggggaggg aggcgcggcg cgggaggagg aggggcgcag     120
   ccgcggagcc agtggccccg cttggacgcg ctgctctcca gatacccccg gagctccagc      180
   cgcgcggatc gcgcgctccc gccgctctgc ccctaaactt ctgccgtagc tccctttcaa     240
   gccagcgaat ttattcctta aaaccagaaa ctgaacctcg gcacgggaaa ggagtccgcg     300
   gaggagcaaa accacagcag agcaagaaga gcttcagaga gcagccttcc cggagcacca     360
   actccgtgtc gggagtgcag aaaccaacaa gtgagagggc gccgcgttcc cggggcgcag     420
   ctgcgggcgg cgggagcagg cgcaggagga ggaagcgagc gcccccgagc cccgagcccg      480
   agtccccgag cctgagccgc aatcgctgcg gtactctgct ccggattcgt gtgcgcgggc     540
   tgcgccgagc gctgggcagg aggcttcgtt ttgccctggt tgcaagcagc ggctgggagc     600
   agccggtccc tggggaatat gcggcgcgcg tggatcctgc tcaccttggg cttggtggcc     660
   tgcgtgtcgg cggagtcgag agcagagctg acatctgata aagacatgta ccttgacaac     720
   agctccattg aagaagcttc aggagtgtat cctattgatg acgatgacta cgcttctgcg     780
   tctggctcgg gagctgatga ggatgtagag agtccagagc tgacaacatc tcgaccactt     840
   ccaaagatac tgttgactag tgctgctcca aaagtggaaa ccacgacgct gaatatacag 900
   aacaagatac ctgctcagac aaagtcacct gaagaaactg ataaagagaa agttcacctc 960
   tctgactcag aaaggaaaat ggacccagcc gaagaggata caaatgtgta tactgagaaa 1020
   cactcagaca gtctgtttaa acggacagaa gtcctagcag ctgtcattgc tggtggagtt 1080
   attggctttc tctttgcaat ttttcttatc ctgctgttgg tgtatcgcat gagaaagaag 1140
   gatgaaggaa gctatgacct tggagaacgc aaaccatcca gtgctgctta tcagaaggca 1200
   cctactaagg agttttatgc gtaaaactcc aacttagtgt ctctatttat gagatcactg 1260
   aacttttcaa aataaagctt ttgcatagaa taatgaagat ctttgttttt tgttttcatt 1320
   aaagagccat tctggcactt taatgataaa atcccattgt atttaaaaca tttcatgtat 1380
   ttctttagaa caacataaaa ttaaaattta acatctgcag tgttctgtga atagcagtgg 1440
   caaaatatta tgttatgaaa accctcgatg ttcatggaat tggtttaaac ttttatgcgc 1500
   aaatacaaaa tgattgtctt tttcctatga ctcaaagatg aaagctgttt catttgtgtc 1560
   agcatgtctc agattgacct taccaagttg gtcttacttt gttaatttat ctgttgtccc 1620
   cttcctctcc tctgccctcc cttcttgtgc ccttaaaacc aaaccctatg ccttttgtag 1680
   ctgtcatggt gcaatttgtc tttggaaaat tcagataatg gtaatttagt gtatatgtga 1740
   ttttcaaata tgtaaacttt aacttccact ttgtataaat ttttaagtgt cagactatcc 1800
   attttacact tgctttattt ttcattacct gtagctttgg gcagatttgc aacagcaaat 1860
   taatgtgtaa aattggatta ttactacaaa accgtttagt catatctatc taatcagatc 1920
   ttcttttggg aggatttgat gtaagttact gacaagcctc agcaaaccca aagatgttaa 1980
   cagtatttta agaagttgct gcagattcct ttggccactg tatttgttaa tttcttgcaa 2040
   tttgaaggta cgagtagagg tttaaagaaa aatcagtttt tgttcttaaa aatgcattta 2100
   agttgtaaac gtctttttaa gcctttgaag tgcctctgat tctatgtaac ttgttgcaga 2160
   ctggtgttaa tgagtatatg taacagttta aaaaaaaagt tggtatttta taagcacaga 2220
   caattctaat ggtaactttt gtagtcttat gaatagacat aaattgtaat ttgggaacat 2280
   aaaaactact gaataaatca tgtggcctaa tattgaaaat gtcactgtta taaattttgt 2340
   acatttttga tcaaatgtac atctcccctt tgctaacggc cgtctgctct caaggatgac 2400
   gtgggtttga tttctaagtg tttcacagtg tctgtaaatc aagaccaaag agcctgtcga 2460
   tgagactgtt tattaccaga ttcacttctg aattggccag aggaaatctg aatgtattat 2520
   cctgtgtgtg tctaggtaga gatattggaa ggctgccagg ggatttcgaa gtttgcaacc 2580
   tttataggat aactgatggc aatattaaga cagacgcctg cttttgcaaa taacttacaa 2640
   gactgtaaat tccaaagatc tgaatggggc tttcctgatg ttggtatcta aggcttaggc 2700
   ctatagattg atttaccttt ggaattgtgc tccaaatgtc tactgaagct taaccgaaga 2760
   actaataaat ggactacagt agctcacgtt acagggaagg agggtaggca gggaggctct 2820
   gtgtgttaaa atgagggtct cactgcttta ggattgaagt ggctggaaag agtgatgcct 2880
   ggggaaggag atggagttat gagggtactg tggctggtac tttctgtact aaacatttcc 2940
   tttttctatt ttaccactaa ttttgtttta aactgtgagc cgtccaagtc agaagaagac 3000
   agcaaaaaaa gcaacttttc caacatacaa tttactttta ataaagtatg aatatttcat 3060
   tttgagaaca ttccctggaa ttgccacata attcattaaa aacatttttt taagcaacac 3120
   ttggaacagt gtttacttta aatccttaat ggccttaatt aattctcaga ttcctgcccc 3180
   atcacttaca gaaccaattc actttagagt gactaaaagg aaacgatagc ctagctttct 3240
   aaagccacgc tgtgtccctc aattacagag ggtaggaatg ggtatacctc taactgtgca 3300
   aagcagagtg aaattcaatt catagaataa caactgctgg gaatatccgt gccaggaaaa 3360
   gaaaaatttc tggcaaatat tttgtcactg ctgtaaagca aaatatttgt gaaagtgcca 3420
   aaataaagtc tgtcatgcca aaagtaaatc attgtataga ctgacatcca gttttcttca 3480
   actgt 3485
<210> 63
   <211> 3961
   <212> DNA
   <213> Homo sapiens
<400> 63
   gaggtcttcc tgtttttgga gggggcaggc accccagggc cgattgtcgc gaatcacctt 60
   cacctctggg ggctcagaat ccctgagcca gaaggccagg gcagggctgg gggatacccc 120
   tgctgctcag acaggaaaat gggctcggag cttgcccaat acttcccaga aggacgaggc 180
   tgctgaactt ctcattcggg gctccgggac ctggactgta cccctttctg gcgtcacctc 240
   ctcctgtcgc ctggccctcg ccatgcagac cccgcgagcg tcccctcccc gcccggccct 300
   gctgcttctg ctgctgctac tggggggcgc ccacggcctc tttcctgagg agccgccgcc 360
   gcttagcgtg gcccccaggg actacctgaa ccactatccc gtgtttgtgg gcagcgggcc 420
   cggacgcctg acccccgcag aaggtgctga cgacctcaac atccagcgag tcctgcgggt 480
   caacaggacg ctgttcattg ggacagggga caacctctac cgcgtagagc tggagccccc 540
   cacgtccacg gagctgcggt accagaggaa gctgacctgg agatctaacc ccagcgacat 600
   aaacgtgtgt cggatgaagg gcaaacagga gggcgagtgt cgaaacttcg taaaggtgct 660
   gctccttcgg gacgagtcca cgctctttgt gtgcggttcc aacgccttca acccggtgtg 720
   cgccaactac agcatagaca ccctgcagcc cgtcggagac aacatcagcg gtatggcccg 780
   ctgcccgtac gaccccaagc acgccaatgt tgccctcttc tctgacggga tgctcttcac 840
   agctactgtt accgacttcc tagccattga tgctgtcatc taccgcagcc tcggggacag 900
   gcccaccctg cgcaccgtga aacatgactc caagtggttc aaagagcctt actttgtcca 960
   tgcggtggag tggggcagcc atgtctactt cttcttccgg gagattgcga tggagtttaa 1020
   ctacctggag aaggtggtgg tgtcccgcgt ggcccgagtg tgcaagaacg acgtgggagg 1080
   ctccccccgc gtgctggaga agcagtggac gtccttcctg aaggcgcggc tcaactgctc 1140
   tgtacccgga gactcccatt tctacttcaa cgtgctgcag gctgtcacgg gcgtggtcag 1200
   cctcgggggc cggcccgtgg tcctggccgt tttttccacg cccagcaaca gcatccctgg 1260
   ctcggctgtc tgcgcctttg acctgacaca ggtggcagct gtgtttgaag gccgcttccg 1320
   agagcagaag tcccccgagt ccatctggac gccggtgccg gaggatcagg tgcctcgacc 1380
   ccggcccggg tgctgcgcag cccccgggat gcagtacaat gcctccagcg ccttgccgga 1440
   tgacatcctc aactttgtca agacccaccc tctgatggac gaggcggtgc cctcgctggg 1500
   ccatgcgccc tggatcctgc ggaccctgat gaggcaccag ctgactcgag tggctgtgga 1560
   cgtgggagcc ggcccctggg gcaaccagac cgttgtcttc ctgggttctg aggcggggac 1620
   ggtcctcaag ttcctcgtcc ggcccaatgc cagcacctca gggacgtctg ggctcagtgt 1680
   cttcctggag gagtttgaga cctaccggcc ggacaggtgt ggacggcccg gcggtggcga 1740
   gacagggcag cggctgctga gcttggagct ggacgcagct tcggggggcc tgctggctgc 1800
   cttcccccgc tgcgtggtcc gagtgcctgt ggctcgctgc cagcagtact cggggtgtat 1860
   gaagaactgt atcggcagtc aggaccccta ctgcgggtgg gcccccgacg gctcctgcat 1920
   cttcctcagc ccgggcacca gagccgcctt tgagcaggac gtgtccgggg ccagcacctc 1980
   aggcttaggg gactgcacag gactcctgcg ggccagcctc tccgaggacc gcgcggggct 2040
   ggtgtcggtg aacctgctgg taacgtcgtc ggtggcggcc ttcgtggtgg gagccgtggt 2100
   gtccggcttc agcgtgggct ggttcgtggg cctccgtgag cggcgggagc tggcccggcg 2160
   caaggacaag gaggccatcc tggcgcacgg ggcgggcgag gcggtgctga gcgtcagccg 2220
   cctgggcgag cgcagggcgc agggtcccgg gggccggggc ggaggcggtg gcggtggcgc 2280
   cggggttccc ccggaggccc tgctggcgcc cctgatgcag aacggctggg ccaaggccac 2340
   gctgctgcag ggcgggcccc acgacctgga ctcggggctg ctgcccacgc ccgagcagac 2400
   gccgctgccg cagaagcgcc tgcccactcc gcacccgcac ccccacgccc tgggcccccg 2460
   cgcctgggac cacggccacc ccctgctccc ggcctccgct tcatcctccc tcctgctgct 2520
   ggcgcccgcc cgggcccccg agcagccccc cgcgcctggg gagccgaccc ccgacggccg 2580
   cctctatgct gcccggcccg gccgcgcctc ccacggcgac ttcccgctca ccccccacgc 2640
   cagcccggac cgccggcggg tggtgtccgc gcccacgggc cccttggacc cagcctcagc 2700
   cgccgatggc ctcccgcggc cctggagccc gcccccgacg ggcagcctga ggaggccact 2760
   gggcccccac gcccctccgg ccgccaccct gcgccgcacc cacacgttca acagcggcga    2820
   ggcccggcct ggggaccgcc accgcggctg ccacgcccgg ccgggcacag acttggccca    2880
   cctcctcccc tatggggggg cggacaggac tgcgcccccc gtgccctagg ccgggggccc    2940
   cccgatgcct tggcagtgcc agccacggga accaggagcg agagacggtg ccagaacgcc    3000
   ggggcccggg gcaactccga gtgggtgctc aagtcccccc cgcgacccac ccgcggagtg    3060
   gggggccccc tccgccacaa ggaagcacaa ccagctcgcc ctccccctac ccggggccgc    3120
   aggacgctga gacggtttgg gggtgggtgg gcgggaggac tttgctatgg atttgaggtt    3180
   gaccttatgc gcgtaggttt tggttttttt tgcagttttg gtttcttttg cggttttcta    3240
   accaattgca caactccgtt ctcggggtgg cggcaggcag gggaggcttg gacgccggtg    3300
   gggaatgggg ggccacagct gcagacctaa gccctccccc acccctggaa aggtccctcc    3360
   ccaacccagg cccctggcgt gtgtgggtgt gcgtgcgtgt gcgtgccgtg ttcgtgtgca    3420
   aggggccggg gaggtgggcg tgtgtgtgcg tgccagcgaa ggctgctgtg ggcgtgtgtg    3480
   tcaagtgggc cacgcgtgca gggtgtgtgt ccacgagcga cgatcgtggt ggccccagcg    3540
   gcctgggcgt tggctgagcc gacgctgggg cttccagaag gcccgggggt ctccgaggtg    3600
   ccggttagga gtttgaaccc cccccactct gcagagggaa gcggggacaa tgccggggtt    3660
   tcaggcagga gacacgagga gggcctgccc ggaagtcaca tcggcagcag ctgtctaaag    3720
   ggcttggggg cctgggggcc ggcgaaggtg ggtggggccc ctctgtaaat acggccccag    3780
   ggtggtgaga gagtcccatg ccacccgtcc ccttgtgacc tcccccctct gacctccagc    3840
   tgaccatgca tgccacgtgg ctggctgggt cctctgccct ctctggagtt tgcctccccc    3900
   agccccctcc ccatcaataa aactctgttt acaaccaccg gcaaaaaaaa aaaaaaaaaa    3960
   a                                                                    3961
<210> 64
   <211> 4201
   <212> DNA
   <213> Homo sapiens
<400> 64
   tccggcccgc acccaccccc aagaggggcc ttcagctttg gggctcagag gcacgacctc      60
   ctggggaggg ttaaaaggca gacgcccccc cgccccccgc gcccccgcgc cccgactcct      120
   tcgccgcctc cagcctctcg ccagtgggaa gcggggagca gccgcgcggc cggagtccgg     180
   aggcgagggg aggtcggccg caacttcccc ggtccacctt aagaggacga tgtagccagc     240
   tcgcagcgct gaccttagaa aaacaagttt gcgcaaagtg gagcggggac ccggcctctg     300
   ggcagccccg gcggcgcttc cagtgccttc cagccctcgc gggcggcgca gccgcggccc     360
   atggagcccg ccggcccggc ccccggccgc ctcgggccgc tgctctgcct gctgctcgcc     420
   gcgtcctgcg cctggtcagg agtggcgggt gaggaggagc tgcaggtgat tcagcctgac 480
   aagtccgtgt tggttgcagc tggagagaca gccactctgc gctgcactgc gacctctctg 540
   atccctgtgg ggcccatcca gtggttcaga ggagctggac caggccggga attaatctac 600
   aatcaaaaag aaggccactt cccccgggta acaactgttt cagacctcac aaagagaaac 660
   aacatggact tttccatccg catcggtaac atcaccccag cagatgccgg cacctactac 720
   tgtgtgaagt tccggaaagg gagccccgat gacgtggagt ttaagtctgg agcaggcact 780
   gagctgtctg tgcgcgccaa accctctgcc cccgtggtat cgggccctgc ggcgagggcc 840
   acacctcagc acacagtgag cttcacctgc gagtcccacg gcttctcacc cagagacatc 900
   accctgaaat ggttcaaaaa tgggaatgag ctctcagact tccagaccaa cgtggacccc 960
   gtaggagaga gcgtgtccta cagcatccac agcacagcca aggtggtgct gacccgcgag 1020
   gacgttcact ctcaagtcat ctgcgaggtg gcccacgtca ccttgcaggg ggaccctctt 1080
   cgtgggactg ccaacttgtc tgagaccatc cgagttccac ccaccttgga ggttactcaa 1140
   cagcccgtga gggcagagaa ccaggtgaat gtcacctgcc aggtgaggaa gttctacccc 1200
   cagagactac agctgacctg gttggagaat ggaaacgtgt cccggacaga aacggcctca 1260
   accgttacag agaacaagga tggtacctac aactggatga gctggctcct ggtgaatgta 1320
   tctgcccaca gggatgatgt gaagctcacc tgccaggtgg agcatgacgg gcagccagcg 1380
   gtcagcaaaa gccatgacct gaaggtctca gcccacccga aggagcaggg ctcaaatacc 1440
   gccgctgaga acactggatc taatgaacgg aacatctata ttgtggtggg tgtggtgtgc 1500
   accttgctgg tggccctact gatggcggcc ctctacctcg tccgaatcag acagaagaaa 1560
   gcccagggct ccacttcttc tacaaggttg catgagcccg agaagaatgc cagagaaata 1620
   acacaggaca caaatgatat cacatatgca gacctgaacc tgcccaaggg gaagaagcct 1680
   gctccccagg ctgcggagcc caacaaccac acggagtatg ccagcattca gaccagcccg 1740
   cagcccgcgt cggaggacac cctcacctat gctgacctgg acatggtcca cctcaaccgg 1800
   acccccaagc agccggcccc caagcctgag ccgtccttct cagagtacgc cagcgtccag 1860
   gtcccgagga agtgaatggg accgtggttt gctctagcac ccatctctac gcgctttctt 1920
   gtcccacagg gagccgccgt gatgagcaca gccaacccag ttcccggagg gctggggcgg 1980
   tgcaggctct gggacccagg ggccagggtg gctcttctct ccccacccct ccttggctct 2040
   ccagcacttc ctgggcagcc acggccccct ccccccacat tgccacatac ctggaggctg 2100
   acgttgccaa accagccagg gaaccaacct gggaagtggc cagaactgcc tggggtccaa 2160
   gaactcttgt gcctccgtcc atcaccatgt gggttttgaa gaccctcgac tgcctccccg 2220
   atgctccgaa gcctgatctt ccagggtggg gaggagaaaa tcccacctcc cctgacctcc 2280
   accacctcca ccaccaccac caccaccacc accaccacta ccaccaccac ccaactgggg 2340
   ctagagtggg gaagatttcc cctttagatc aaactgcccc ttccatggaa aagctggaaa 2400
   aaaactctgg aacccatatc caggcttggt gaggttgctg ccaacagtcc tggcctcccc 2460
   catccctagg ctaaagagcc atgagtcctg gaggaggaga ggacccctcc caaaggactg 2520
   gagacaaaac cctctgcttc cttgggtccc tccaagactc cctggggccc aactgtgttg 2580
   ctccacccgg acccatctct cccttctaga cctgagcttg cccctccagc tagcactaag 2640
   caacatctcg ctgtggacgc ctgtaaatta ctgagaaatg tgaaacgtgc aatcttgaaa 2700
   ctgaggtgtt agaaaacttg atctgtggtg ttttgttttg ttttttttct taaaacaaca 2760
   gcaacgtgat cttggctgtc tgtcatgtgt tgaagtccat ggttgggtct tgtgaagtct 2820
   gaggtttaac agtttgttgt cctggaggga ttttcttaca gcgaagactt gagttcctcc 2880
   aagtcccaga accccaagaa tgggcaagaa ggatcaggtc agccactccc tggagacaca 2940
   gccttctggc tgggactgac ttggccatgt tctcagctga gccacgcggc tggtagtgca 3000
   gccttctgtg accccgctgt ggtaagtcca gcctgcccag ggctgctgag ggctgcctct 3060
   tgacagtgca gtcttatcga gacccaatgc ctcagtctgc tcatccgtaa agtggggata 3120
   gtgaagatga cacccctccc caccacctct cataagcact ttaggaacac acagagggta 3180
   gggatagtgg ccctggccgt ctatcctacc cctttagtga ccgcccccat cccggctttc 3240
   tgagctgatc cttgaagaag aaatcttcca tttctgctct caaaccctac tgggatcaaa 3300
   ctggaataaa ttgaagacag ccagggggat ggtgcagctg tgaagctcgg gctgattccc 3360
   cctctgtccc agaaggttgg ccagagggtg tgacccagtt accctttaac ccccaccctt 3420
   ccagtcgggt gtgagggcct gaccgggccc agggcaagca gatgtcgcaa gccctattta 3480
   ttcagtcttc actataactc ttagagttga gacgctaatg ttcatgactc ctggccttgg 3540
   gatgcccaag ggatttctgg ctcaggctgt aaaagtagct gagccatcct gcccattcct 3600
   ggaggtccta caggtgaaac tgcaggagct cagcatagac ccagctctct gggggatggt 3660
   cacctggtga tttcaatgat ggcatccagg aattagctga gccaacagac catgtggaca 3720
   gctttggcca gagctcccgt gtggcatctg ggagccacag tgacccagcc acctggctca 3780
   ggctagttcc aaattccaaa agattggctt gtaaaccttc gtctccctct cttttaccca 3840
   gagacagcac atacgtgtgc acacgcatgc acacacacat tcagtatttt aaaagaatgt 3900
   tttcttggtg ccattttcat tttattttat tttttaattc ttggaggggg aaataaggga 3960
   ataaggccaa ggaagatgta tagctttagc tttagcctgg caacctggag aatccacata 4020
   ccttgtgtat tgaaccccag gaaaaggaag aggtcgaacc aaccctgcgg aaggagcatg 4080
   gtttcaggag tttattttaa gactgctggg aaggaaacag gccccatttt gtatatagtt 4140
   gcaacttaaa ctttttggct tgcaaaatat ttttgtaata aagatttctg ggtaataatg 4200
   a 4201
<210> 65
   <211> 3333
   <212> DNA
   <213> Homo sapiens
<400> 65
   gtggccgcgg ggcggtgtca tcgcccccgc cccgcccggt ccagccagct cggcccgggg 60
   gcttcgggct gtcgggccgg cgctcccttc tctgccaggt ggcgagtaca cctgctcacg 120
   taggcgtcat gaggtctccg gttcgagacc tggcccggaa cgatggcgag gagagcacgg 180
   accgcacgcc tcttctaccg ggcgccccac gggccgaagc cgctccagtg tgctgctctg 240
   ctcgttacaa cttagcaatt ttggcctttt ttggtttctt cattgtgtat gcattacgtg 300
   tgaatctgag tgttgcgtta gtggatatgg tagattcaaa tacaacttta gaagataata 360
   gaacttccaa ggcgtgtcca gagcattctg ctcccataaa agttcatcat aatcaaacgg 420
   gtaagaagta ccaatgggat gcagaaactc aaggatggat tctcggttcc tttttttatg 480
   gctacatcat cacacagatt cctggaggat atgttgccag caaaataggg gggaaaatgc 540
   tgctaggatt tgggatcctt ggcactgctg tcctcaccct gttcactccc attgctgcag 600
   atttaggagt tggaccactc attgtactca gagcactaga aggactagga gagggtgtta 660
   catttccagc catgcatgcc atgtggtctt cttgggctcc ccctcttgaa agaagcaaac 720
   ttcttagcat ttcatatgca ggagcacagc ttgggacagt aatttctctt cctctttctg 780
   gaataatttg ctactatatg aattggactt atgtcttcta cttttttggt actattggaa 840
   tattttggtt tcttttgtgg atctggttag ttagtgacac accacaaaaa cacaagagaa 900
   tttcccatta tgaaaaggaa tacattcttt catcattaag aaatcagctt tcttcacaga 960
   agtcagtgcc gtgggtaccc attttaaaat ccctgccact ttgggctatc gtagttgcac 1020
   acttttctta caactggact ttttatactt tattgacatt attgcctact tatatgaagg 1080
   agatcctaag gttcaatgtt caagagaatg ggtttttatc ttcattgcct tatttaggct 1140
   cttggttatg tatgatcctg tctggtcaag ctgctgacaa tttaagggca aaatggaatt 1200
   tttcaacttt atgtgttcgc agaattttta gccttatagg aatgattgga cctgcagtat 1260
   tcctggtagc tgctggcttc attggctgtg attattcttt ggccgttgct ttcctaacta 1320
   tatcaacaac actgggaggc ttttgctctt ctggatttag catcaaccat ctggatattg 1380
   ctccttcgta tgctggtatc ctcctgggca tcacaaatac atttgccact attccaggaa 1440
   tggttgggcc cgtcattgct aaaagtctga cccctgataa cactgttgga gaatggcaaa 1500
   ccgtgttcta tattgctgct gctattaatg tttttggtgc cattttcttt acactattcg 1560
   ccaaaggtga agtacaaaac tgggctctca atgatcacca tggacacaga cactgaagga 1620
   accaataaat aatcctgcct ctattaatgt atttttattt atcatgtaac ctcaaagtgc      1680
   cttctgtatt gtgtaagcat tctatgtctt tttttaattg tacttgtatt agatttttaa      1740
   ggcctataat catgaaatat cactagttgc cagaataata aaatgaactg tgtttaatta      1800
   tgaataatat gtaagctagg acttctactt taggttcaca tacctgcctg ctagtcgggc      1860
   aacatgaagt aggacagttc tgttgatttt ttagggccat actaaaggga atgagctgaa      1920
   acagacctcc tgataccttt gcttaattaa actagatgat aattctcagg tactgataaa      1980
   cacctgttgt tgttcacttt cctcataaaa attgtcagct ctctctgaca cttagacctc      2040
   aaactttagc atctctgtgg agctgccatc cactgtataa tttcgcctgg caactggact      2100
   gaggggagtg tgcccaggca gctgccaagc actccctccc tggcttcagg gtcagagtgc      2160
   ccagcgttta tcagaggcag catccaagcc cagagccagt gtcgactctt cggctggtgc      2220
   ctttcctctg aggggctatc aatgtgtaga taaagccctg agtaggcaag agcagtgaga      2280
   tccactgcta tggtcttgat acatcctcaa actttccctt cccagcacag aggaatattg      2340
   gctggcatgc aacctgcaaa agaaaaatgc gaagcggccg ggcacggtgg ctcatgcctg      2400
   taatcccagc actttggggg gctgaggtgg gcgaatcatg agatcaggag ttcgagacca      2460
   gcctggccag catggtgaaa ccccatctct actaaaaata caaaaaatta gctgggcgtg      2520
   gtgacgggcg cctgtaatcc cagatactca ggaggctgag gtaggagaat cacttgaacc      2580
   tgggaggtgg aagttgcagt gaaccaagat cacgccactg cactccagcc tgggcgatgg      2640
   agcgagactc caactcaaaa aaaaaaaaag aataaagaaa gaaaagtgcg atgcccagtc      2700
   aatcacaaat aagatcatcc tggtttaaat ctactctcac atggatcaca gtataaattt      2760
   ctatgtgctg tgttttgttc gtttgtattt tgtagagatg gggtctcgtt ttgtcgccca      2820
   ggctggtttt gaactcctgg cttcaagcga tcctcctgtc tcggcctcac aaagtgttga      2880
   gactacaggc atgagccact gtgcccagcc tgttctatgt ttttaagcta cacgagaatt      2940
   ttttttttaa ttaattctca ctgtttgttc agtctgtctt catctaagtt tgtgttgcag      3000
   tttaaagtta aagtgacttt taaaggccac atcacctgag actagggtaa tcatctttac      3060
   ttctggttcc tgaaatcata tttttccagt ggaccatcct ccagtggctg tggttgttga      3120
   gcatgctttc agaacaccta tgtggcttaa aacttagttt atgttttgtg ttcaacacta      3180
   cgtgtaatat tttaaaactg tttaatgtga tgtgaataca tttatgtaca tttattttta      3240
   aatttgtaaa tagctttaaa ttgctatggc aatgtttctt ttataaatca tcaaaataaa      3300
   cctttgtgaa ttgaaaaaaa aaaaaaaaaa aaa                                   3333
<210> 66
   <211> 3743
   <212> DNA
   <213> Homo sapiens
<400> 66
   tttcttctag aagagctttg cgtcttaaac gcccataccg cagacagctg cctggcgggc 60
   gacccagccc catcctgatg gccccaggac acaagcccag gagcgccccg gtgcgcgcgg 120
   ttccgcggcc caggggcgcc gggtttggtg gcacagcgag ccccctttct ctagcgaaac 180
   ctgtttccct ccaatcttgt ggttgcagct ttccgtacgt atgcaaccga ttataaagtc 240
   gtgacccaga acagcagctc tggaaatgta acccatgaaa agatccccat aggcactgag 300
   atagaaggga tgaacatttt aggattggtc ctgtttgctc tggtgttagg agtggcctta 360
   aagaaactag gctccgaagg agaagacctc atccgtttct tcaattccct caacgaggcg 420
   acgatggtgc tggtgtcctg gattatgtgc tcagcgaccc ttccctctat gatgaagtgc 480
   attgaagaga acaatggtgt ggacaagagg atcagcaggt ttattctccc catcggggcc 540
   accgtgaaca tggacggagc agccatcttc cagtgtgtgg ccgcggtgtt cattgcgcaa 600
   ctcaacaacg tagagctcaa cgcaggacag attttcacca ttctagtgac tgccacagcg 660
   tccagtgttg gagcagcagg cgtgccagct ggaggggtcc tcaccattgc cattatcctg 720
   gaggccattg ggctgcctac tcatgacctg cctctgatcc tggctgtgga ctggattgtg 780
   gaccggacca ccacggtggt gaatgtggaa ggggatgccc tgggtgcagg cattctccac 840
   cacctgaatc agaaggcaac aaagaaaggc gagcaggaac ttgctgaggt gaaagtggaa 900
   gccatcccca actgcaagtc tgaggaggag acatcgcccc tggtgacaca ccagaacccc 960
   gctggccccg tggccagtgc cccagaactg gaatccaagg agtcggttct gtgatggggc 1020
   tgggctttgg gcttgcctgc cagcagtgat gtcccaccct gttcacccag ccgccagtca 1080
   tggacacagg gcactgccct tgccaacttt taccctccca agcaatgctt tggcccagtc 1140
   gctggcctga ggcttacctc tcggcactgg cattgggctc cccagccgga actggttacc 1200
   aaggacaagg acactctgac attcggcttg atccatgtcc aggtgcaact gtgtgtacac 1260
   cagggatctg tttggaaaca accccttgag ctgccaggct caagaaatca tggactcaca 1320
   gggtcctgtg tggttacatc ttggaaaaaa tgcagatgta tttcactctc cccggtcagc 1380
   tctgcatcag gtgttttctg agcaaaccaa gggggtttat agtcatctgt cgcattgcct 1440
   cgagttgcag taattgaaaa aatgctcaaa ttcttagcca tggctggcct ttgctgagct 1500
   gggactcagg tgtttaaaga gtttgtgcta tagctaggtg tggatagctt ctgatccctg 1560
   ggttctggga gactgcaggt gccgcacatt gtcaagttag aaatactcca ggtgggtgtt 1620
   agcactgtgg tggtctctgg tccacagcct taggtaaaca acttagattc tgaggtcaaa 1680
   gaaaaaagga gagggaatgc agccttgtgg gggagaagcg gggcagaggg ttctctaatc 1740
   taatcaggac aggacaggtt tcacatacaa ttgtcccagt tcgcatccca gccctggggc 1800
   acttttctgc ttccttccag aggcctgggc ctctgataac actttggctt tttctccatt 1860
   cacgctgatt tggcaaaagg ccagagatgg gcctccttcc ctggggaggt gtgatgtagt 1920
   tatcacattc aggacccttg ttgatttatc atctattatt tgaattcaac tggacactct 1980
   gtaaaatgct gcactgcagc aaaaacaaaa ccaccaccac cccagagaaa accatgtact 2040
   aattggagtg gggtaccccc attcacaggt tcccaggtcc cctggctttg gctgatttca 2100
   aaatatagag ccctttcttg ccagtacatc caagtttaaa attatcagcg aaatggtcca 2160
   tgtttttcca attacctgct gacacggttc taagctaagt gaaggggaag atctgagagc 2220
   gtgctgtttg tggctgttga tgcatattcg tgatgtaaca ggtcctgggg cctcacttta 2280
   ccccatttgt aaaatggggc taatgtcacc tgcctcttac ctacctcaga gggatttggt 2340
   gaagcaaact gttaatcttc gaaaacgacc atttcacttc ttggatatca agtgctaacc 2400
   cagtatgttc ttctttttta tgtaagggac agctttctcc acagagtcct ttctgctggt 2460
   gaggacagca tttctgagca gggctttgtt ctctatgtgc attaggactt ttatcatgcc 2520
   cttgttctgt gtgtagttac ttgacagcat caaatgccgc ctcttcctaa tgtccttcaa 2580
   gttttcatga actagcaacc ccaccttcca ccatggttct gggcgcctga ttttgctgtg 2640
   actcccagac ccagccactg tttctgccac cctgtaacag gccattaaag ctccccagtg 2700
   ttcagcctcc ttcactccct tgttttccct gttgctatgt gtcacctggg ccctacagac 2760
   aggggcacac gcttatggat gtgtgtacca ttgagatgag aatgggtaga tggaacggag 2820
   accatcaagc cacaccccct tcttaaaact ggggacatga gcctgagcag aaagggtgaa 2880
   gaagagccat gggacacaga gttgacccag ccagggggaa agcccagctc tctttaaacc 2940
   agctaagcca ttccagtctc ctgtgaagcc aaaagggacc aggaaccgtg caaaggaaac 3000
   tggaaacttt tccccgctgg gtagagcatg ttgctgatac tcttctgttt tcaagggaaa 3060
   caatcacatt gtttgattcc aaatggtaaa tgaacactca ctattcttca ggcttcagta 3120
   aatctttttt tcttccttca tatatatata cacaacacac acacacatat gtatatctat 3180
   acacacatgt gtgttgtgta tatgcatgtg tgtgtgtgcg tgtgtgtata gttttagctc 3240
   caagccaagc aagtttgtgt ttggatagag gggaacttaa ctattaacta caagttgtat 3300
   gtctgtggta tcttgatttt cccatttcta aagatgaatt tcacaaagcc ataaagcgtg 3360
   aaattagagc tggacttaag actcattggc cgaccatcct gtgtcctggc ctggccctgc 3420
   agtaagaagc gtgtctgggt ctggagaagg gtgcttccga gagtgtgcag gtggcccttc 3480
   cccttggagg cgagaagaga gaatgtgctg tctatcttcc tggttttcag tccacagagt 3540
   cggtagacca ggggttacgt gactggggaa aatctcacat ctccttgtct gaaaacattt 3600
   cccctgctgt tctctttcta acatgttgtg gtaaatctgt tcagatactg ctcatctgac 3660
   tgttttgtac atgtgacaat tgccttaaaa cctagcacag tcctcagaaa tgaataccgt 3720
   gtttccactg gaaaaaaaaa aaa 3743
<210> 67
   <211> 2214
   <212> DNA
   <213> Homo sapiens
<400> 67
   cctgtttccc aggaacggtc cccggcttcg cgccccaatt tctaacagcc tgcctgtccc      60
   ccgggaacgt tctaacatcc ttggggagcg ccccagctac aagacactgt cctgagaacg     120
   ctgtcatcac ccgtagttgc aagtttcgga gcggcagtgg gaagcatgcg ggactacgac     180
   gaggtgatcg ccttcctggg cgagtggggg cccttccagc gcctcatctt cttcctgctc     240
   agcgccagca tcatccccaa tggcttcaat ggtatgtcag tcgtgttcct ggcggggacc     300
   ccggagcacc gctgtcgagt gccggacgcc gcgaacctga gcagcgcctg gcgcaacaac     360
   agtgtcccgc tgcggctgcg ggacggccgc gaggtgcccc acagctgcag ccgctaccgg     420
   ctcgccacca tcgccaactt ctcggcgctc gggctggagc cggggcgcga cgtggacctg     480
   gggcagctgg agcaggagag ctgcctggat ggctgggagt tcagccagga cgtctacctg     540
   tccaccgtcg tgaccgagtg gaatctggtg tgtgaggaca actggaaggt gcccctcacc     600
   acctccctgt tcttcgtagg cgtgctcctc ggctccttcg tgtccgggca gctgtcagac     660
   aggtttggca ggaagaacgt tctcttcgca accatggctg tacagactgg cttcagcttc     720
   ctgcagattt tctccatcag ctgggagatg ttcactgtgt tatttgtcat cgtgggcatg     780
   ggccagatct ccaactatgt ggtagccttc atactaggaa cagaaattct tggcaagtca     840
   gttcgtatta tattctctac attaggagtg tgcacatttt ttgcagttgg ctatatgctg     900
   ctgccactgt ttgcttactt catcagagac tggcggatgc tgctgctggc gctgacggtg     960
   ccgggagtgc tgtgtgtccc gctgtggtgg ttcattcctg aatctccccg atggctgata    1020
   tcccagagaa gatttagaga ggctgaagat atcatccaaa aagctgcaaa aatgaacaac    1080
   atagctgtac cagcagtgat atttgattct gtggaggagc taaatcccct gaagcagcag    1140
   aaagctttca ttctggacct gttcaggact cggaatattg ccataatgac cattatgtct    1200
   ttgctgctat ggatgctgac ctcagtgggt tactttgctc tgtctctgga tgctcctaat    1260
   ttacatggag atgcctacct gaactgtttc ctctctgcct tgattgaaat tccagcttac    1320
   attacagcct ggctgctatt gcgaaccctg cccaggcgtt atatcatagc tgcagtactg    1380
   ttctggggag gaggtgtgct tctcttcatt caactggtac ctgtggatta ttacttctta    1440
   tccattggtc tggtcatgct gggaaaattt gggatcacct ctgctttctc catgctgtat    1500
   gtcttcactg ctgagctcta cccaaccctg gtcaggaaca tggcggtggg ggtcacatcc    1560
   acggcctcca gagtgggcag catcattgcc ccctactttg tttacctcgg tgcttacaac    1620
   agaatgctgc cctacatcgt catgggtagt ctgactgtcc tgattggaat cctcaccctt     1680
   tttttccctg aaagtttggg aatgactctt ccagaaacct tagagcagat gcagaaagtg     1740
   aaatggttca gatctgggaa aaaaacaaga gactcaatgg agacagaaga aaatcccaag     1800
   gttctaataa ctgcattctg aaaaaatatc taccccattt ggtgaagtga aaaacagaaa     1860
   aataagaccc tgtggagaaa ttcgttgttc ccactgaaat ggactgactg taacgattga     1920
   caccaaaatg aaccttgcta tcaagaaatg ctcgtcatac agtaaactct ggatgattct     1980
   tccagataat gtccttgctt tacaaaccaa ccatttctag agagtctcct tactcattaa     2040
   ttcaatgaaa tggattggta agatgtcttg aaaacatgtt agtcaaggac tggtaaaata     2100
   catataaaga ttaacactca tttccaatca tacaaatact atccaaataa aaataacatc     2160
   attgtattaa cgcaaatatt aggtgacaac aaaaaaaaaa aaaaaaaaaa aaaa           2214
<210> 68
   <211> 4797
   <212> DNA
   <213> Homo sapiens
<400> 68
   ggcgggaggg ggcgggaaat cctcggcctc ggtggcggtg gtggacacgt cgagccgggt 60
   agaagtggag gggccgttcg aagagtcgtg agggggtgac gggttaagat tcggagagag 120
   aggtgctagt ggctggactt gacctggaaa gaatcttctg ctgactctca acttttcctg 180
   gaaaaaatgg atcattccca ccatatgggg atgagctata tggactccaa cagtaccatg 240
   caaccttctc accatcaccc aaccacttca gcctcacact cccatggtgg aggagacagc 300
   agcatgatga tgatgcctat gaccttctac tttggcttta agaatgtgga actactgttt 360
   tccggtttgg tgatcaatac agctggagaa atggctggag cttttgtggc agtgttttta 420
   ctagcaatgt tctatgaagg actcaagata gcccgagaga gcctgctgcg taagtcacaa 480
   gtcagcattc gctacaattc catgcctgtc ccaggaccaa atggaaccat ccttatggag 540
   acacacaaaa ctgttgggca acagatgctg agctttcctc acctcctgca aacagtgctg 600
   cacatcatcc aggtggtcat aagctacttc ctcatgctca tcttcatgac ctacaacggg 660
   tacctctgca ttgcagtagc agcaggggcc ggtacaggat acttcctctt cagctggaag 720
   aaggcagtgg tagtggatat cacagagcat tgccattgac atcaaactct atggcgtggc 780
   cttatcgatt gcagtgggaa gttgttgaag acttgaagac gtgattcctg ctccaatcat 840
   cccttcttgc tcctctttgt gcacgtacac acacacacac acacacacac acacacaccc 900
   ctgctcaaca gaggtttagt ttacagtctc tgaactaaag tagtaacctc ccaaattgtt 960
   ttttctaata agctgagatt cccatttctc ttaaggagaa gccacccatg agatgtcttt 1020
   tccttctcca tcatcttaga gccaagttat atgttcttgt ctaatccatg tagctttttg 1080
   ttcaatgact tgatcatctg cttccttttt gaatttttaa cagatagtaa gtaaatttgg 1140
   tggttttttc ccctgggtca gtgatggaaa ggggttaact tcagccagga ttgatggcag 1200
   ctgagggaaa ttcttgccca actaaaccca gaactcaaac ttaacattag aaaataaggt 1260
   ccagggccgg acacagtggc ccatgcctgt aatcccagca ctttgggggg ccaaggcagg 1320
   ctggatcacc tgaggacagg agttcgagac cagtctggcc aacatgggga aaccccgtct 1380
   ctactaaaaa tacaaaaatt agccgggcat ggtggtgggc gcctgtaatc ccagctactc 1440
   agaaggctga ggcaggagaa tcacttgaac ctaggaggcg gaggttgcag tgagccaaga 1500
   tggcgccatt gcactccagc ctgggtgaca agagtgaaac tccatctcaa aaaaaaagaa 1560
   aagaaggtcc agcttttgga ttcaatgagt gggaaataca ttgtgccttt ctctagatgt 1620
   gatacgttat accaaaatct ttgtagtgtg cagagcggtg gtttgagact aaatacaggc 1680
   ttagaacttg cagagtgtgt attcttggat ggctgatgca tcgacttgca ttcccactta 1740
   acactttgat tagcatgaac ttgccaatca aaaaatgaca atcaatttga gaaaatagaa 1800
   atagatattt ttaaataaaa ccattcacag tttactttgt cttgatacct tggtttgtcc 1860
   cagctgaagt gaagcaagag agtttgaatt aatttttcca ttataatgtt ttcgcatgtc 1920
   tgcctctaaa actgtgattt tcaagcttta gcgtgcatca gaatcacctg tagggcttgt 1980
   taaaacacta attgctgggc ctcaacccaa aagcccccaa agtggcactt ctgagttcct 2040
   gctgatgtta caagggacca cacttttgag aatctgtgct ttaagctaag gaaatattgc 2100
   ctggtgggtt ggctgcctgg tattgggcat ggaaatttga attgctgatt ggtagatggt 2160
   gtgtctggac ttaactcacg tagtaaatac tgctgatcaa tacctaatca ttccacattt 2220
   attgagctcc acctgtgtgt atgtgtaccc aagcacacat gtgtgaaggg ctatagccaa 2280
   agtattttta ctagcctgta tgaaatcact agtccttatt tttaaaggtc tatggtttct 2340
   tggaagtagt ttgattgttg agagagacct ttgatctgca gtgtaaatct accagtcatg 2400
   ggccagaagg gcaaaagccc agctttctct ttggaaagac tcaggctgtg gtttgttgat 2460
   ggccaggttt tcctcaggct ccaacaactg tgcttatacc aagcagatcc tcatcctcca 2520
   tataatcatc tttgttattc gtgggggttt aattacatta caagtggcca aaacccctgt 2580
   tctcagtgaa gaaccacatt ggatttgtat tctgttcagt tgtagtctac actgcagtct 2640
   tattcctggt tcaaactacc tctttaaatt gatttgtctt gtgctggtct gttaaatcct 2700
   gccctccttg gtgctagatc cagttgtgcc tcagggcaga ggaaacaaaa cacagctatc 2760
   ctgttggcct gtgttgtggt tttgaagttt gtactttctc tgtgggtgcc agttaaatat 2820
   tggagagcaa ggaatgtgga cttgtatggc tttgaaccaa gagagggtta tgagcctact 2880
   ggattgaggt taaaatccaa gaaccaacat ttagagcttt gtgcttttct ctcattccat 2940
   cactttgtaa tgatgatact taacatgagc agggtgaatg acaggtactg acgaagtcca 3000
   acacaaaggt ataatacagc ctgttgtcta aagccaagga gtcataaaac catgagaaat 3060
   aaataggaat caatagttag tagtgacatt ggtgctctct agaaatctca gcatgagctg 3120
   ctatagaata ccctcccagc aacaaaacct aatcagtaag gccagctaga cccaatggct 3180
   catgcctgta atcccaacac tttgggaggc caaggtggga ggatggcttg tgtccagaag 3240
   ttcgagacca gcctggacaa catagtgaga tcctatctct ataaaaaatc aaaaattagc 3300
   caggcatggt ggtgcatacc tatagtcctg gctatttggg aggctgaggc aggaggattg 3360
   ctttagccct ggaggtcgag gctgcagtaa gccatgattg cgccactgca ctcagcccgg 3420
   gtgacaaagc aagaccctgt ctcagaaaaa aagaaaattc aaggccagtt aagacaaaat 3480
   gctatgactt tgaaattcac agaaagaaat aacagtttag attaggtctt caggtattca 3540
   ggatagagat aatctcctga aaaacctgaa tttcagagat tcttagactg gctgccaaag 3600
   gatgaagcta gtgaaggaga aaaagcttaa attccatctt gagctcttgg attgtgataa 3660
   tacaatgatt tcattaactt ttcatttctg tatacctgtt catttggaat ttaatgcttg 3720
   acttctttgt tcattttgga tctaaacttc tcttttcttc cttccccatt cacatctatt 3780
   agaagactgc atcaccattt ctttggcccc cttactctgt tgtcctttcc cttttctttc 3840
   agttttttta atcgcatgtc tagtatatta agtctccata gccctcctga tgcagtagac 3900
   agtgctatgc tgtggatata ataccaacca gaaattggca tttataaacc tgttaagaga 3960
   ctttaagcat gcttcaagag gcagttgacc cactggaatt tctataaggc tggtaccctt 4020
   cccagagtta cagaatctta ggtgccgtct ctagtctgtg agggaggaac tcccagcatc 4080
   cccattgccc acaaatggaa tcctcactgt atccactagg agattagaaa ttaaggtttc 4140
   ttcactactt ctatggtagg gttgtctgaa attccctttc aggctgtggg tactggtctt 4200
   gggttctagt cataaggggt tccttataag gagcaggcgg aggggagtac actttcatgt 4260
   gatttaattt tgatcctgcc ctctccagct gctccttcaa aagatacatc aaaagataga 4320
   aactctgggc tgggcacagt ggctcacaca ctttgggagg ccaagcgggg gtgcagatca 4380
   cctgaggtca ggagtttgag accagcctgg ccaatgtggt gaaaccccat ctctactaaa 4440
   aatacaaaaa ttagctgggc gtggtggtgc atgcctgtaa tcccagctac tcgggaggct 4500
   gaggcaggag aatcgcttga acccaggagg cggaggttgc agtgagccaa gatggcgcca 4560
   ttgcactcca gcctgggcga caagagcaaa actccgtctc aaaaaaaaaa aaaagataca 4620
   aagtctgcat ttgatataat gccttaatta ctgggtctac aattaatgtt gactgtttta 4680
   gattgtaagc tcctggagag cagtattgct gtagtaggaa tgttttaaca gtgtcatatg 4740
   aaaaagaaca aaataaatat tttgattttg tgattctaaa aaaaaaaaaa aaaaaaa 4797
<210> 69
   <211> 2347
   <212> DNA
   <213> Homo sapiens
<400> 69
   ggcgtgggac gtgctgcggc gtcctagctg gcttacaggg cggcggcggg gtgtgtgtcc 60
   tctgttaaga gtgctactcg cccggggttg atctgtgcat gccactcctg ggtcagacgg 120
   tgaggtcggc gtctgcgagg acgcggcggt ggagtagaag ggcagccgga gacaggcccg 180
   gcgccccttc cgaggctaga cggccccagc ttcgcgggga tcatggcatt gctggtggac 240
   cgagtgcggg gccactggcg aatcgccgcc gggctcctgt tcaacctgct ggtgtccatc 300
   tgcattgtgt tcctcaacaa atggatttat gtgtaccacg gcttccccaa catgagcctg 360
   accctggtgc acttcgtggt cacctggctg ggcttgtata tctgccagaa gctggacatc 420
   tttgccccca aaagtctgcc gccctccagg ctcctcctcc tggccctcag cttctgtggc 480
   tttgtggtct tcactaacct ttctctgcag aacaacacca taggcaccta tcagctggcc 540
   aaggccatga ccacgccggt gatcatagcc atccagacct tctgctacca gaaaaccttc 600
   tccaccagaa tccagctcac gctgattcct ataactttag gtgtaatcct aaattcttat 660
   tacgatgtga agtttaattt ccttggaatg gtgtttgctg ctcttggtgt tttagttaca 720
   tccctttatc aagtgtgggt aggagccaaa cagcatgaat tacaagtgaa ctcaatgcag 780
   ctgctgtact accaggctcc gatgtcatct gccatgttgc tggttgctgt gcccttcttt 840
   gagccagtgt ttggagaagg aggaatattt ggtccctggt cagtttctgc tttgcttatg 900
   gtgctgctat ctggagtaat agctttcatg gtgaacttat caatttattg gatcattggg 960
   aacacttcac ctgtcaccta taacatgttc ggacacttca agttctgcat tactttattc 1020
   ggaggatatg ttttatttaa ggatccactg tccattaatc aggcccttgg cattttatgt 1080
   acattatttg gcattctcgc ctatacccac tttaagctca gtgaacagga aggaagtagg 1140
   agtaaactgg cacaacgtcc ttaattgggt ttttgtggag aaaagaatgt tgtcccaaga 1200
   agataaaaaa tattgttaag tgtgcaagtt attaaaaaaa aaaaattggg ccaggcacgg 1260
   tggctcacgc ctgtaatccc agcactttgg gaggccaagg ccagcggatc acttgaggtc 1320
   aggagttcga gaccagcctg accaacatgg agaaaccctg tctcaactaa taatacaaaa 1380
   ttagccaggc gtggtggcgc atgcctgtaa tcccagctac tcgggaggct gaggcaggag 1440
   aatcacttga acccgggagg cggcggttgc agtgagccga gatcgtacca ttgcactcca 1500
   gcctgggcaa caagagcgaa actccatttc aaaaaaaaaa aattggtgac agactcaatg 1560
   atggaatgat ttgtcggaat taacacaaag cagattttat tcatataatg actttttttt 1620
   aagagtctct tttttaaaaa aacttaattc tctaaaaccg aaatggttca tgcttctttt 1680
   taaaaatgat tgtataaaat gtatggaatg gttagcctgg tgtggtggtg cacacctgta 1740
   atcccagcta cttgggagac tgagacatga gaatcgcttg agcctgggag gcggaggttg 1800
   caatgagcca agatcgtacc actgcactcc agcctgggcg acagagcaag acactgtctc 1860
   tctctctctc tccatatata tatgtgtgtg tgtgtatata tatatatatg tgtgtgtgtg 1920
   tgtgtgtata catatataca catatataca cacacacata catatacatg tgtatatata 1980
   taccatccca tatatatgtg ggatatatat atatatatat atggatatgg ttatatatat 2040
   gggatggttt ggttggtccc agcaaagtat atgaaaatta aagttctgtg ataatgacaa 2100
   aggaattgct gttactgtac tgcaaatatg ctgtgggttc tcggtgttca aactcttcta 2160
   aggaaggaca cacagtagct ctctgcttgc tgatagatgg tttcccagtg tgagatttgt 2220
   tattttgatc agagtattca aatcagaatt taaatctagt gtttctattt tagtttagct 2280
   tcctgattta tataaatgaa atctcattta taaagtataa taaagatgac tgtaagacaa 2340
   aatccaa 2347
<210> 70
   <211> 1681
   <212> DNA
   <213> Homo sapiens
<400> 70
   agtcctgggc gaagggggcg gtggttcccc gcggcgctgc gcgcggcggt aattagtgat 60
   tgtcttccag cttcgcgaag gctaggggcg cggctgccgg gtggctgcgc ggcgctgccc 120
   ccggaccgag gggcagccaa cccaatgaaa ccaccgcgtg ttcgcgcctg gtagagattt 180
   ctcgaagaca ccagtgggcc cgttccgagc cctctggacc gcccgtgtgg aaccaaacct 240
   gcgcgcgtgg ccgggccgtg ggacaacgag gccgcggaga ctgtttcaat gcatcaaagc 300
   tactgacatc tcatggcatg ggcatccagg ttccgctgaa tgcaacagag ttcaactatc 360
   tctgtccagc catcatcaac caaattgatg ctagatcttg tctgattcat acaagtgaaa 420
   agaaggctga aatccctcca aagacctatt cattacaaat agcctgggtt ggtggtttta 480
   tagccatttc catcatcagt ttcctgtctc tgctgggggt tatcttagtg cctctcatga 540
   atcgggtgtt tttcaaattt ctcctgagtt tccttgtggc actggccgtt gggactttga 600
   gtggtgatgc ttttttacac cttcttccac attctcatgc aagtcaccac catagtcata 660
   gccatgaaga accagcaatg gaaatgaaaa gaggaccact tttcagtcat ctgtcttctc 720
   aaaacataga agaaagtgcc tattttgatt ccacgtggaa gggtctaaca gctctaggag 780
   gcctgtattt catgtttctt gttgaacatg tcctcacatt gatcaaacaa tttaaagata 840
   agaagaaaaa gaatcagaag aaacctgaaa atgatgatga tgtggagatt aagaagcagt 900
   tgtccaagta tgaatctcaa ctttcaacaa atgaggagaa agtagataca gatgatcgaa 960
   ctgaaggcta tttacgagca gactcacaag agccctccca ctttgattct cagcagcctg 1020
   cagtcttgga agaagaagag gtcatgatag ctcatgctca tccacaggaa gtctacaatg 1080
   aatatgtacc cagagggtgc aagaataaat gccattcaca tttccacgat acactcggcc 1140
   agtcagacga tctcattcac caccatcatg actaccatca tattctccat catcaccacc 1200
   accaaaacca ccatcctcac agtcacagcc agcgctactc tcgggaggag ctgaaagatg 1260
   ccggcgtcgc cactctggcc tggatggtga taatgggtga tggcctgcac aatttcagcg 1320
   atggcctagc aattggtgct gcttttactg aaggcttatc aagtggttta agtacttctg 1380
   ttgctgtgtt ctgtcatgag ttgcctcatg aattaggtga ctttgctgtt ctactaaagg 1440
   ctggcatgac cgttaagcag gctgtccttt ataatgcatt gtcagccatg ctggcgtatc 1500
   ttggaatggc aacaggaatt ttcattggtc attatgctga aaatgtttct atgtggatat 1560
   ttgcacttac tgctggctta ttcatgtatg ttgctctggt tgatatggta agtttttaag 1620
   aagtcttatt acattattga ccaacaataa aatagagaaa atattcaaaa aaaaaaaaaa 1680
   a 1681
<210> 71
   <211> 6512
   <212> DNA
   <213> Homo sapiens
<400> 71
   gagagctgcc tgctcagaca acagacacgc gaggtcagga agaagccgct tataaattac     60
   cgcttccttc gcgccgccgc caacgccgag ccccgaggac cgcaagccca gaggacaagc    120
   tgcgccaaga gggagtgcgg agcgttcacc cagcgggtca gagagcgagc gggcaggcag    180
   cccccggccg gcggaacccg gcacagccga gcagagcgcg ggcggcgccg cagccacccc    240
   agatccagaa ccagaaccac agcccttctg aggagctccc aaaccaagga gatggccacc    300
   aaggagaagc tgcagtgtct gaaagatttc cacaaggaca tcctgaagcc ctcaccaggg    360
   aagagcccag gcacgcggcc tgaggacgag gctgagggaa aacctccgca gagggagaag    420
   tggtctagca agatcgactt tgtgctctct gtggctggcg gcttcgtggg cttgggcaac    480
   gtctggcgct tcccgtacct ctgctacaag aatggtggag gtgcgtttct cataccgtat    540
   tttattttcc tgtttgggag cggcctgcct gtgtttttct tggagatcat cataggccag    600
   tacacctctg aagggggcat cacctgctgg gaaaagatct gccccttgtt ctctggtatc    660
   ggctatgcct ccgttgtaat tgtgtccctc ctgaatgtct actacatcgt catcctggcc    720
   tgggccacat actacctgtt ccagtccttc cagaaggagc tgccctgggc acactgcaac    780
   cacagctgga acacacctca ctgcatggag gacaccatgc gcaagaacaa gagtgtctgg    840
   atcaccatca gctccaccaa cttcacctcc cctgtcatcg agttctggga gcgcaacgtg    900
   ctgagcttgt cccctggaat cgaccaccca ggctctctga aatgggacct cgctctctgc    960
   cttcttttag tctggctagt gtgtttcttc tgcatctgga agggcgtcag gtccactggg 1020
   aaggtcgtct acttcacagc cacttttcca ttcgccatgc tcctggtgct gctggtccga 1080
   gggctgacgc tgccgggcgc gggcgcaggc atcaagttct atctgtatcc tgacatcacc 1140
   cgccttgagg acccacaggt gtggattgac gctgggactc agatattctt ctcttatgcc 1200
   atctgcctgg gggctatgac ctcgctgggg agctacaaca agtacaagta taactcgtac 1260
   agggactgta tgctgctggg atgcctgaac agtggtacca gttttgtgtc tggcttcgca 1320
   attttttcca tcctgggctt catggcacaa gagcaagggg tggacattgc tgatgtggct 1380
   gagtcaggtc ctggcctggc cttcattgcc tacccaaaag ctgtgacaat gatgccgctg 1440
   cccacatttt ggtccattct tttttttatt atgcttctct tgcttggact ggatagccag 1500
   tttgttgaag ttgaaggaca gatcacatcc ttggttgatc tttacccatc cttcctaagg 1560
   aagggttatc gtcgggaaat cttcatcgcc ttcgtgtgta gcatcagcta cctgctgggg 1620
   ctgacgatgg tgacggaggg tggcatgtat gtgtttcagc tctttgacta ctatgcagct 1680
   agcggtgtat gccttttgtg ggttgcattc tttgaatgtt ttgttattgc ctggatatat 1740
   ggaggtgata acctttatga tggtattgag gacatgattg gctatcggcc cgggccctgg 1800
   atgaagtaca gctgggctgt gatcactcca gttctctgtg ttggatgttt catcttctcg 1860
   ctcgtcaagt acgtacccct gacctacaac aaaacatacg tgtaccccaa ctgggccatt 1920
   gggctgggct ggagcctggc cctttcctcc atgctctgcg ttcccttggt catcgtcatc 1980
   cgcctctgcc agactgaggg gccgttcctt gtgagagtca agtacctgct gaccccaagg 2040
   gaacccaacc gctgggctgt ggagcgcgag ggagccacac cttacaactc tcgcaccgtc 2100
   atgaacggcg ctctcgtgaa accgacccac atcattgtgg agaccatgat gtgagctctc 2160
   tcgggtcgac ggggccggcg gctttcctgc tgtttactaa cattagattc tcataggacc 2220
   aggtttacag agctttatat ttgcactagg attttttttt ttttgtaatt gtcacagaaa 2280
   atgtaattgt gggtatgtgt gcgtgcgtgt gtgtgtgtgt gtgtatcgtg tgtgtgtgtt 2340
   ttgttttgat ttgggggata ttttgtacaa aaagaaaacc cacgggaaga tgtccgtgga 2400
   gaggcagagc tttcatactg aattagatgt attttatggg aatttggtaa atttttcttt 2460
   gtattttttt ttttacatat aagtatatat acacttagag attgtcatat acttttacca 2520
   cttgaattga tcttcttgcc agcaatagat ctcattttca aaagcaattc ttcggtgctg 2580
   tgtagctggc agaaagttct gtccagtaaa cgcaggatgg aattttcctg ggactctaca 2640
   cccatcttaa ggtggtatac cttccaaatc ctggttcaga tggaagaaat agcaggagag 2700
   aggacccatt agctggcaga cccaggggga agaaaggagg gctgtgagga gatacctcat 2760
   taaacttggc ttagtgaaga agagagatgc caaaggaatg aaccaaccct tcacataaag 2820
   gagactggct gaagctgaat gaggaggccc tatagcagaa gtctgattct aagagcagta 2880
   gaaacttgta ccagaagcaa aatcccactt ttaattttga gatggtgagt ggatagtcag 2940
   tagaccgtca gaaccactgg ccagagaggg agctgctaga gatccaagaa ggctggcagg 3000
   agtgaggctc acaactcagc ctcgcaagag gtggcagagg cacaggaggc cacagtcctt 3060
   cctggggcat tccaggcaga gaaggagcag aggctctccc ggcaggagct ggggtctcag 3120
   ggctcagatg agtctgttgc atttgaatgg ggtcatagca ggttctggtc attccccaag 3180
   caacatctca gcatctctta aagttgcctg caggaatgaa gcatgacata cctgttgagg 3240
   gactagggga gtggtgggga ggtgagtgga ccaaaggata taggccccag gcatgcagat 3300
   gggcccggtg tcggggaggg gtgctttctt tcctcatctc cccactcccc actctcagcc 3360
   tgggagactc ctgccaagcc ctcattaaag atgccaccct gggctgccct ggcacctagc 3420
   aaggcacacc aagaacagct tttgagtctg tatcctccac tgggggaagt gctcccagtt 3480
   cagaacaagg gcagcccgtg gtgctgacct aggatataac aaagctcttc acttcaaaac 3540
   ccctgcaata gctgggttta cagacattta ccacctgcgg acccaaaaga gaaggcctag 3600
   gagagttttc tagaaggttg ggattgtcag ggtcctggcc cctcagaact ggcttgatca 3660
   agggccttat gtggagcaga ggttgtctct gaaccaggag agaaggtact atacctttca 3720
   aatccccagg gcagacacac ccccacccag cccctatttg gacctaaact gtgccatttg 3780
   aacagtcact tccaagctca gtctaaatga aaccgaaacg tgaccacgca caaaggcagt 3840
   cactgcctcg aggggtgcag accgcagaat tttcacagca ggggctcttg gaaccctgga     3900
   aacccccttc ttaaatttgg gaggaggagt atgcctttgg tgtccccctc ccaaggggca     3960
   attctgaacc ccatctttgg caggcataca tatttcactg tttccaaagc tatctactct 4020
   gccaaacaac acccagtcct attccaaact ctcaacgatt ctatcttgtt cctgtttttc 4080
   tatgtattta tggttgccgt ttgtgtctga tttgatttta ctgttttttc cctgatttta 4140
   tggagtagca ttgtgacctg ttttcctttg tcttatataa ctttagtaaa ctaaccactg 4200
   tcaatgattg agggcaggtg gcacgtgggg aagaggggac ttggcacgca gtggctacct 4260
   gggcatttgt ggtcatttca gtttccatct ccccagcggg ggctccctgg gtgaaaggcc 4320
   acagtatttt gggttggtag gcaaattgca acattctgga catggcctga ggaaggcctc 4380
   ttcttataag attctcagac caaattctag accaaagaca caggcagacc aagtccccag 4440
   gccccgcctg gaaggaagtc gttcctcaac tctccccaag gcacctgtct ccaatcagag 4500
   ccctctcgcc cagccagccc tggctctgtg tgcagagcat agctctgcga gtacctgtgt 4560
   aataatgctc aaccttcatg tctccgtata aacgaaactt tccatgagag ctcatgactc 4620
   tggtccacct gtctatagag aatgggcaaa gtccttcacc tgctttctgc ttgggatggg 4680
   tcagaaatgc tgatgcccgc acatagccca gccagccaga tctggaaagg aagcgagggg 4740
   gttgtttaaa tcaatttttt aagatgaaga agtgggagac actgcgttga gatgggccat 4800
   gctagggcca cagagatttc ctgacggtca gggagagaag ggcctccagg gtcccctaac 4860
   ccaacgccct tgttgtaaat gaggtaactg aggctcaggg aggcactgtg agccaggaat 4920
   ggattttctt gaaacagctc tagctgcagg ttctccgagg taggtgcagg gaatggtgag 4980
   tgtctaacca gggctacatc cagcaacatc ctcaaggtct tcctgacaac caaagacaag 5040
   cctttatgga aaaggaaatg cgctcccctc catgttcagg gatgagggga gcagcagcag 5100
   ccacactccc accatcctca cagaattcct ggacccatgc ggtggctccg tgagctgggt 5160
   gactccagcc tcacctgcac accccagccc tgcacggggc cctccttcct cccagcagcc 5220
   cttggtgagc taggaattga gatccctgtt tgtgaaagag ggaactgagg tgcagagaag 5280
   ccagaggtgt gccagatcct taggcaggat ttagatgaag tcgccctggc tccagactga 5340
   ccccgaggct ctgcggggag tttccaggca gcaggaagtg gccttggatg ctctccttcc 5400
   aggacagcat aacccctggg ccatgtgcag ctccttcact gccccctgga tccccagcat 5460
   acccccaaag acagtgggga aacacaaggg gagagcacag catggcccct ccagcccact 5520
   tcagggcact cttgtatcac ccgggtaccg ccacactggt cccccaccca gccagcatct 5580
   cccagcacag cccctctccc tggggaaatg ctctgggtag ccagtctaaa ggcagaggca 5640
   cctaactgct ccccgcagcc caccccaccc aagattcaga cacaagccag gaaaggaccc 5700
   aagagaaaat ccttcaaggt ggcctgaggt cccatccctc cctcagaccc atgtggtccc 5760
   aggccaggct gcctgggaca cggtaaatac cactgtgtgc aaaaatcgaa gtacaaaacc 5820
   acaagactaa acaaaacaaa cccagagagc caaacttgta gaggtgggca gtccagaaag 5880
   cagggggcag ccctccccct ttccttctct ccctgatcct cagaatatat attgttgtaa 5940
   taggaagcat ttttgcattg ttctcttgtg ggtgtcacta cagacatgtt ctggcgtgtt 6000
   ctccgaggga tggagcatcc tgttatatat ttgacttcaa attgagatgt tggcttcatt 6060
   tttttttttt acccaattaa tctcccaatc cctagcaact gtgactctgt atttagcaca 6120
   agagaaagct gagaatgtgg gtcttgcctc cttccagaaa tatgtctggc tcatcaggac 6180
   atttttttaa aacttcaaaa tatttttaag atattttaaa cttttataaa aaaaaaatca 6240
   accaacaaga gacttttctg aggaggaaca tttgtatttg aacaagatcc ttggtgtgta 6300
   gttcagtctt gcagtataca agcttttgtg tataaatgtt ttatgatatg attccctgta 6360
   ttttgcaggg gtttttttct cttttgcttt ttagataaat atgtatatca atattttaaa 6420
   ttcatctttg ctttttttag aggagtttgt aatcacctta taacatgaaa ataaacattt 6480
   cctttttaac atccaaaaaa aaaaaaaaaa aa 6512
<210> 72
   <211> 3550
   <212> DNA
   <213> Homo sapiens
<400> 72
   gccgggcccc gccgccgccc gcgcgccccc gggcccccga cacacatgag attcttcagg 60
   ctcactttca agtgcttcgt ggactgcttc tgactgcgcc gcccgcgccc cgcaccccgc 120
   cgcccgcccg ccgccccgtc ccccggcccg gccgcccccc ggcccccggc cggcccgcgc 180
   cctcggggcc ctccccggtg ccgccggtgc cccccgcctg accgccgccc cccgtgaggc 240
   gccgcgaccc cggcccggcc gtgcggcccg ccgaggccat ggcgaagaag agcgccgaga 300
   acggcatcta tagcgtgtcc ggcgacgaga agaagggccc cctcatcgcg cccgggcccg 360
   acggggcccc ggccaagggc gacggccccg tgggcctggg gacacccggc ggccgcctgg 420
   ccgtgccgcc gcgcgagacc tggacgcgcc agatggactt catcatgtcg tgcgtgggct 480
   tcgccgtggg cttgggcaac gtgtggcgct tcccctacct gtgctacaag aacggcggag 540
   gtgtgttcct tattccctac gtcctgatcg ccctggttgg aggaatcccc attttcttct 600
   tagagatctc gctgggccag ttcatgaagg ccggcagcat caatgtctgg aacatctgtc 660
   ccctgttcaa aggcctgggc tacgcctcca tggtgatcgt cttctactgc aacacctact 720
   acatcatggt gctggcctgg ggcttctatt acctggtcaa gtcctttacc accacgctgc 780
   cctgggccac atgtggccac acctggaaca ctcccgactg cgtggagatc ttccgccatg 840
   aagactgtgc caatgccagc ctggccaacc tcacctgtga ccagcttgct gaccgccggt 900
   cccctgtcat cgagttctgg gagaacaaag tcttgaggct gtctggggga ctggaggtgc 960
   caggggccct caactgggag gtgacccttt gtctgctggc ctgctgggtg ctggtctact 1020
   tctgtgtctg gaagggggtc aaatccacgg gaaagatcgt gtacttcact gctacattcc 1080
   cctacgtggt cctggtcgtg ctgctggtgc gtggagtgct gctgcctggc gccctggatg 1140
   gcatcattta ctatctcaag cctgactggt caaagctggg gtcccctcag gtgtggatag 1200
   atgcggggac ccagattttc ttttcttacg ccattggcct gggggccctc acagccctgg 1260
   gcagctacaa ccgcttcaac aacaactgct acaatgggac cagcttcttt gctggcttcg 1320
   tggtcttctc catcctgggc ttcatggctg cagagcaggg cgtgcacatc tccaaggtgg 1380
   cagagtcagg gccgggcctg gccttcatcg cctacccgcg ggctgtcacg ctgatgccag 1440
   tggccccact ctgggctgcc ctgttcttct tcatgctgtt gctgcttggt ctcgacagcc 1500
   agtttgtagg tgtggagggc ttcatcaccg gcctcctcga cctcctcccg gcctcctact 1560
   acttccgttt ccaaagggag atctctgtgg ccctctgttg tgccctctgc tttgtcatcg 1620
   atctctccat ggtgactgat ggcgggatgt acgtcttcca gctgtttgac tactactcgg 1680
   ccagcggcac caccctgctc tggcaggcct tttgggagtg cgtggtggtg gcctgggtgt 1740
   acggagctga ccgcttcatg gacgacattg cctgtatgat cgggtaccga ccttgcccct 1800
   ggatgaaatg gtgctggtcc ttcttcaccc cgctggtctg catgggcatc ttcatcttca 1860
   acgttgtgta ctacgagccg ctggtctaca acaacaccta cgtgtacccg tggtggggtg 1920
   aggccatggg ctgggccttc gccctgtcct ccatgctgtg cgtgccgctg cacctcctgg 1980
   gctgcctcct cagggccaag ggcaccatgg ctgagcgctg gcagcacctg acccagccca 2040
   tctggggcct ccaccacttg gagtaccgag ctcaggacgc agatgtcagg ggcctgacca 2100
   ccctgacccc agtgtccgag agcagcaagg tcgtcgtggt ggagagtgtc atgtgacaac 2160
   tcagctcaca tcaccagctc acctctggta gccatagcag cccctgcttc agccccaccg 2220
   cacccctcca gggggcctgc ctttccctga cacttttggg gtctgcctgg gggaggaggg 2280
   gagaaagcac catgagtgct cactaaaaca actttttcca tttttaataa aacgccaaaa 2340
   atatcacaac ccaccaaaaa tagatgcctc tccccctcca gccctagccg agctggtcct 2400
   aggccccgcc tagtgcccca cccccaccca cagtgctgca ctcctcctgc ccctgccacg 2460
   cccaccccct gcccacctct ccaggctctg ctctgcagca cacccgtggg tgacccctca 2520
   ccccagaagc agcagtggca gcttgggaaa tgtgaggaag ggaaggaggg agagacggga 2580
   gggaggagag agaggagaag ggaggcaggg gaggggcagc agaaccaagg caaatatttc 2640
   agctgggcta tacccctctc cccatccctg ttatagaagc ttagagagcc agccagcaat 2700
   ggaaccttct ggttcctgcg ccaatcgcca ccagtatcaa ttgtgtgagc ttgggtgcga 2760
   gtgcacgcgt gcgtgagtac ggagagtata tatagatctc tatctcttag caaaggtgaa 2820
   tgccagatgt aaatggcgcc tctgggcaaa ggaggcttgt attttgcaca ttttataaaa 2880
   acttgagaga atgagatttc tgcttgtata tttctaaaaa gaggaaggag cccaaaccat 2940
   cctctcctta ccactcccat ccctgtgagc cctaccttac ccctctgccc ctagccaagg 3000
   agtgtgaatt tatagatcta actttcatag gcaaaacaaa agcttcgagc tgttgcgtgt 3060
   gtgagtctgt tgtgtggatg tgcgtgtgtg gtccccagcc ccagactgga ttggaaaagt 3120
   gcatggtggg ggcctcgggg ctgtccccac gctgtccctt tgccacaagt ctgtggggca 3180
   agaggctgca atattccgtc ctgggtgtct gggctgctaa cctggcctgc tcaggcttcc 3240
   caccctgtgc ggggcacacc cccaggaagg gaccctggac acggctccca cgtccaggct 3300
   taaggtggat gcacttcccg cacctccagt cttctgtgta gcagctttaa cccacgtttg 3360
   tctgtcacgt ccagtcccga gacggctgag tgaccccaag aaaggcttcc ccgacaccca 3420
   gacagaggct gcagggctgg ggctgggtga gggtggcggg cctgcgggga cattctactg 3480
   tgctaaaaag ccactgcaga catagcaata aaaacatgtc attttccaaa gcaggaaaaa 3540
   aaaaaaaaaa 3550
<210> 73
   <211> 9648
   <212> DNA
   <213> Homo sapiens
<400> 73
   ggtttgtaat gatagggcgg cagcagcagc agcagcagca gtggtggaac gaggaggtgg 60
   agaattgaga gcacgatgca tacacaggtg tttctgagta gtaattagat cgctgtgaag 120
   gaaaaagcac acctttgagt tttcacctgt gaacactata gcgctgagag agacagtctg 180
   aaagcagagg aagacatcga tcagtaacac caagagacac caaagttgaa agttttgttt 240
   tctttccctc tgttttattt ttcccccgtg tgtccctact atggtcagaa agcctgttgt 300
   gtccaccatc tccaaaggag gttacctgca gggaaatgtt aacgggaggc tgccttccct 360
   gggcaacaag gagccacctg ggcaggagaa agtgcagctg aagaggaaag tcactttact 420
   gaggggagtc tccattatca ttggcaccat cattggagca ggaatcttca tctctcctaa 480
   gggcgtgctc cagaacacgg gcagcgtggg catgtctctg accatctgga cggtgtgtgg 540
   ggtcctgtca ctatttggag ctttgtctta tgctgaattg ggaacaacta taaagaaatc 600
   tggaggtcat tacacatata ttttggaagt ctttggtcca ttaccagctt ttgtacgagt 660
   ctgggtggaa ctcctcataa tacgccctgc agctactgct gtgatatccc tggcatttgg 720
   acgctacatt ctggaaccat tttttattca atgtgaaatc cctgaacttg cgatcaagct 780
   cattacagct gtgggcataa ctgtagtgat ggtcctaaat agcatgagtg tcagctggag 840
   cgcccggatc cagattttct taaccttttg caagctcaca gcaattctga taattatagt 900
   ccctggagtt atgcagctaa ttaaaggtca aacgcagaac tttaaagacg ccttttcagg 960
   aagagattca agtattacgc ggttgccact ggctttttat tatggaatgt atgcatatgc 1020
   tggctggttt tacctcaact ttgttactga agaagtagaa aaccctgaaa aaaccattcc 1080
   ccttgcaata tgtatatcca tggccattgt caccattggc tatgtgctga caaatgtggc 1140
   ctactttacg accattaatg ctgaggagct gctgctttca aatgcagtgg cagtgacctt 1200
   ttctgagcgg ctactgggaa atttctcatt agcagttccg atctttgttg ccctctcctg 1260
   ctttggctcc atgaacggtg gtgtgtttgc tgtctccagg ttattctatg ttgcgtctcg 1320
   agagggtcac cttccagaaa tcctctccat gattcatgtc cgcaagcaca ctcctctacc 1380
   agctgttatt gttttgcacc ctttgacaat gataatgctc ttctctggag acctcgacag 1440
   tcttttgaat ttcctcagtt ttgccaggtg gctttttatt gggctggcag ttgctgggct 1500
   gatttatctt cgatacaaat gcccagatat gcatcgtcct ttcaaggtgc cactgttcat 1560
   cccagctttg ttttccttca catgcctctt catggttgcc ctttccctct attcggaccc 1620
   atttagtaca gggattggct tcgtcatcac tctgactgga gtccctgcgt attatctctt 1680
   tattatatgg gacaagaaac ccaggtggtt tagaataatg tcagagaaaa taaccagaac 1740
   attacaaata atactggaag ttgtaccaga agaagataag ttatgaacta atggacttga 1800
   gatcttggca atctgcccaa ggggagacac aaaataggga tttttacttc attttctgaa 1860
   agtctagaga attacaactt tggtgataaa caaaaggagt cagttatttt tattcatata 1920
   ttttagcata ttcgaactaa tttctaagaa atttagttat aactctatgt agttatagaa 1980
   agtgaatatg cagttattct atgagtcgca caattcttga gtctctgata cctacctatt 2040
   ggggttagga gaaaagacta gacaattact atgtggtcat tctctacaac atatgttagc 2100
   acggcaaaga accttcaaat tgaagactga gatttttctg tatatatggg ttttgtaaag 2160
   atggttttac acactataga tgtctatact gtgaaaagtg ttttcaattc tgaaaaaaag 2220
   catacatcat gattatggca aagaggagag aaagaaattt attttacatt gacattgcat 2280
   tgcttcccct tagataccaa tttagataac aaacactcat gctttaatgg attataccca 2340
   gagcactttg aacaaaggtc agtggggatt gttgaataca ttaaagaaga gtttctaggg 2400
   gctactgttt atgagacaca tccaggagtt atgtttaagt aaaaatcctt gagaatttat 2460
   tatgtcagat gttttttcat tcattatcag gaagttttag ttatctgtca tttttttttt 2520
   tcacatcagt ttgatcagga aagtgtataa cacatcttag agcaagagtt agtttggtat 2580
   taaatcctca ttagaacaac cacctgtttc actaataact tacccctgat gagtctatct 2640
   aaacatatgc attttaagcc ttcaaattac attatcaaca tgagagaaat caccaacaaa 2700
   gaagatgttc aaaataatag tcccatatct gtaatcatat ctacatgcaa tgttagtaat 2760
   tctgaagttt tttaaattta tggctatttt tacacgatga tgaattttga cagtttgtgc 2820
   attttcttta tacattttat attcttctgt taaaatatct cttcagatga aactgtccag 2880
   attaattagg aaaaggcata tattaacata aaaattgcaa aagaaatgtc gctgtaaata 2940
   agatttacaa ctgatgtttc tagaaaattt ccacttctat atctaggctt tgtcagtaat 3000
   ttccacacct taattatcat tcaacttgca aaagagacaa ctgataagaa gaaaattgaa 3060
   atgagaatct gtggataagt gtttgtgttc agaagatgtt gttttgccag tattagaaaa 3120
   tactgtgagc cgggcatggt ggcttacatc tgtaatccca gcactttggg aggctgaggg 3180
   ggtggatcac ctgaggtcgg gagttctaga ccagcctgac caacatggag aaaccccatc 3240
   tctactaaaa atacaaaatt agctgggcat ggtggcacat gctggtaatc tcagctattg 3300
   aggaggctga ggcaggagaa ttgcttgaac ccgggaggcg gaggttgcag tgagccaaga 3360
   ttgcaccact gtactccagc ctgggtgaca aagtcagact ccatctccaa aaaaaaaaga 3420
   ttatatatat atatatatgt gtgtgtatgt gtgtgtgtgt gtgtgtgtgt atatatatat 3480
   atatatatat acacacacac acacacactt tttatatata tatatatata tatatagtgg 3540
   aacttacaaa tgagagtaat ataatgatga aattttgaac tgttatttat aaacatctaa 3600
   ggtaaaatgg ttagtcatgg ccagagtatg tttcatcctt taatttttgt ccatttgaaa 3660
   ataaggattt ttgaaagaat tataccaatt aaaattatta aaggcaaaca tagaattcat 3720
   aaaaaattgt ccaaagtaga aatgatgacc tataatttgg agcatttcca attcagtaat 3780
   ttcaattttg ctcttgaaaa catttaatat atatccaaga ctgacatttc tttagctgaa 3840
   cctaacgttt gggtctctga gtgaatttat aataactcct tccttcctta gcatagggtt 3900
   ttcaaaattt gatttataat tcctatttcc agtaaatatt gttcatttgt ccacatctct 3960
   ccctatgata tgttgctgga ggtaagaatt tctttcatat tcctattttt tttttcccca 4020
   tagactaggc tcatagaatt taaacaagca aattttcctg agctttttct tgccaaatga 4080
   aagaagactg gtaaattctc atagagaggt ttgtgtagtt cttggctctt cctggggtta 4140
   atgtgcttat attcacagtg gcaaattggt ctcagacttt aatttattta tttttgattt 4200
   gaatttctct ttaaaagtat caatttaaaa ggtaactaga attattcttt ctcattttca 4260
   aaagtgattt ttgcattatt aaatttccct gccattgtaa tgccatttca cgcagaaaaa 4320
   aagtcagcca gtaattaaga aaaaaagtga tggagattaa gtagtatttt ggcttatttt 4380
   taggactcat catgagaaga cacagttcct ttaatcagga aattaatatc cataattttc 4440
   actcaaaatt gcagtatgta aagcagattc tcaaaaactc tcctgaacac ttatttatat 4500
   atatgttttt atataagtaa aatttttctc atatttttat acgatatgca cacacacaca 4560
   tacatgcaca tactacttac tacatgttct gtacttgtac tttgtaccat gcatattcaa 4620
   atgtttatat acataagttt attataacat aaacagtaaa agtaatgaat actgtttaaa 4680
   ataactaata tagtattttt taatttttgt ggggatggat tctcaaatac ttgtgatttt 4740
   aaaagattct aaagctaaaa cacaacttga ttttaaaaag aatgattctc cttacacaat 4800
   tataaatatt tgcagtaaat attttcctta taatactgtt ttgaccccat ttaaaaagta 4860
   ttagattata ttcctttgat ccaatgaaaa ctgaacctta taaatggtta gctgaaagta 4920
   gaccttattc ttgtccttct ttagaagagt aaagatttgt cctagggaag atggctgact 4980
   tcggttccca acatgcgtat gcatttagac tgtagctcct cagccctgtg gacacaaaat 5040
   ttggacagct tattaggtta cgttagcaat gcatgacggt ttctccaaca ctaagatatt 5100
   cacgttgaaa cagatttcct gttcgtctta tgtgtctggt aaaattgttt ccccaattac 5160
   aatttgacat atcaatagag ggttaacaag agtataatta cataacagaa ttcctcatga 5220
   actgtaatca gtctacagga aaatcattat tttatcttga tttgcagatg aatatactgc 5280
   taagaaaggg agcaactctg acctttgtta aagttgatct tttgtaattg aggtataagg 5340
   tatgaaaaga taaaaaaccg aaggccagag aatcaggaaa tgaaagatag tatggactga 5400
   aggtaacaat attttaatgt tatgcaatat agtcagagaa atattaaaaa ttagttgttt 5460
   gctgtgcata ggtggatctc gcaggaagct aatgaaacct aagcttcagt gcctctcact 5520
   tagacatgtt ccattcgagg tcctgaacct aactttgtat taggaattct gtactaattt     5580
   tgttgaagaa gaccagcaaa gttgtgtaca cttctacccc cacaaaatct gcattgtcca     5640
   tgtgagtaaa gtaaaataat tcctgttatt tttttctgtt agaaataagt atggaggata     5700
   tgtttttaaa aatttatgag ttaattgaaa tatccatata taacaagtga ctttctcaca     5760
   atatatatga tgtgatatat agggagatag tttcactttc atcatatttt atacgttgat     5820
   tctgaactat agaaaaataa taaatgggat tttaattata gctcttagtt gggaaagaaa     5880
   tatagagaga tgtgggattt gaatgcccat gaaagacatt ttattttact tgaatatatt     5940
   cttgcttcac tttaccctcc ataatatgtt gtacattagt gctgatcaag tttacagagt     6000
   tacattttgc tttcctaacc attcagtcag gaattaaaat atggcattgt ataacaactg     6060
   ggaagaagct catagtggat ataaattaga gtagataatg ggtcaccttg atagcctctg     6120
   tttacattac ttgtatatgg gcaaaataat tattacctat acgtgtattt aagcttaatt     6180
   ttcatataaa cagtattttt aatctatgtt aaaatagata atatctaaaa gtgtgatctc     6240
   taggtagtcc ttagtttatt agtactgtac ttcaaaaaga tttttaaata ggtccggcac     6300
   ggtggctcat gcctgtaatc ccagcacttt gggaggctga ggcgggcgaa tcacctgagg     6360
   tcaggagttc gagatcagcc tggccaacat ggtgaaaccc tgtctcaact aaaaatataa     6420
   aaattagccg ggcgtggtgg caggcgcctg taatcccagc tactcgggag gctgaggcag     6480
   gagaatcact tgaacccaag gggcagaagc tgcagttagc caagatcgca tcattgcact     6540
   ccagcctagg ggacaagagc gcgagacttc atctcaaaaa aaaaaaaaaa aaaaaaaaaa     6600
   gatttttaaa taatagctaa aggtatgctc tctaggtcat ccttagttta ttagtactgt     6660
   acttaaaaat tattttttaa tagtcaattt tgggagataa ttatttcttt ccttatattt     6720
   tccaattagt tggtgtctaa aaataaatgt tttgtctaat tttagatcag gtatacattc     6780
   acaaaagcat aaatcatagt ctcacaggaa attcaccaat tttccatatg tcgtgagata     6840
   actgtccttt ctacaacctc ataacaatga atttatataa ttacctagat tttcttagtg     6900
   tgaatctacc cattagtttt attttcttgg tagttatttt tttccctcct ctctgttact     6960
   attggcctta aaatacacag aggacggtta cagtgtccta atagctgtta catgtgtgtg     7020
   tttcagcgta cttgaatcaa gtgtacattt atagtaccaa taaccgcctt tacagcttta     7080
   cagttaacaa ttctctcaca aaactgtaga gcattaggca tctgagagcc atagagggcc     7140
   aactttgttc cagagtgaac atgctttttt tcctcaacat atacactact gatttttttt     7200
   aaaagtatga ctttcaagtg aattaatgta ttggttagga gaactgcttg ctaagtcctt     7260
   attacctctt gttaaagcct cagaaggccg tgctgaaagc cagaggggaa aaaaagagta     7320
   atgcacaggt atctcttttg cagtggtgac tgtattttga gtaccttgtg tgacagggta     7380
   ttattacagc atcttgtggg aaaacctatt aggcctttgc atgttaaagc tgtataattt 7440
   gttgggttgt gagtggtctg acttaaatgt gtattataaa atttagacat caaattttcc 7500
   tactaactaa ctttattaga tgcatacttg gaagcacagt catatcacac tgggaggcaa 7560
   tgcaatgtgg ttacctggtc ctaggtttga actgtcttat ttcaaaagat ttctgaatta 7620
   atttttccct agaatttctc cttcattcca aagtacaaac atactttgaa gaatgaaaca 7680
   gattgttccc atgaatgtat gctcatactc gactagaaac gatctatgtt aaatgactgt 7740
   gtatatgaat tatttcaagt actaccccaa ataactttct tattgctctg aaagaagaaa 7800
   agcaatgtaa atcactatga ttattgcaca aacaaccaga attctccaac aattttaagt 7860
   aatctgatcc tcttcttgga gaaaattgtt acctaatagt ttttccttat gaatgttatt 7920
   actactggta taaatcaaat ttctataaat ttcctactta agtcttaaga actgggttct 7980
   tcctttgatg ttattcatgt tcagaaagga aacaacactt tactctttta ggacaattcc 8040
   tagaatctat agtagtatca ggatatattt tgctttaaaa tatattttgg ttattttgaa 8100
   tacagacatt ggctccaaat tttcatcttt gcacaatagt atgacttttc actagaactt 8160
   ctcaacattt gggaactttg caaatatgag catcatatgt gttaaggctg tatcatttaa 8220
   tgctatgaga tacattgttt tctccctatg ccaaacaggt gaacaaacgt agttgttttt 8280
   tactgatact aaatgttggc tacctgtgat tttatagtat gcacatgtca gaaaaaggca 8340
   agacaaatgg cctcttgtac tgaatacttc ggcaaactta ttgggtcttc attttctgac 8400
   agacaggatt tgactcaata tttgtagagc ttgcgtagaa tggattacat ggtagtgatg 8460
   cactggtaga aatggttttt agttattgac tcagaattca tctcaggatg aatcttttat 8520
   gtctttttat tgtaagcata tctgaattta ctttataaag atggttttag aaagctttgt 8580
   ctaaaaattt ggcctaggaa tggtaacttc attttcagtt gccaaggggt agaaaaataa 8640
   tatgtgtgtt gttatgttta tgttaacata ttattaggta ctatctatga atgtatttaa 8700
   atatttttca tattctgtga caagcattta taatttgcaa caagtggagt ccatttagcc 8760
   cagtgggaaa gtcttggaac tcaggttacc cttgaaggat atgctggcag ccatctcttt 8820
   gatctgtgct taaactgtaa tttatagacc agctaaatcc ctaacttgga tctggaatgc 8880
   attagttatg accttgtacc attcccagaa tttcaggggc atcgtgggtt tggtctagtg 8940
   attgaaaaca caagaacaga gagatccagc tgaaaaagag tgatcctcaa tatcctaact 9000
   aactggtcct caactcaagc agagtttctt cactctggca ctgtgatcat gaaacttagt 9060
   agaggggatt gtgtgtattt tatacaaatt taatacaatg tcttacattg ataaaattct 9120
   taaagagcaa aactgcattt tatttctgca tccacattcc aatcatatta gaactaagat 9180
   atttatctat gaagatataa atggtgcaga gagactttca tctgtggatt gcgttgtttc 9240
   ttagggttcc tagcactgat gcctgcacaa gcatgtgata tgtgaaataa aatggattct 9300
   tctatagcta aatgagttcc ctctggggag agttctggta ctgcaatcac aatgccagat 9360
   ggtgtttatg ggctatttgt gtaagtaagt ggtaagatgc tatgaagtaa gtgtgtttgt 9420
   tttcatctta tggaaactct tgatgcatgt gcttttgtat ggaataaatt ttggtgcaat 9480
   atgatgtcat tcaactttgc attgaattga attttggttg tatttatatg tattatacct 9540
   gtcacgcttc tagttgcttc aaccatttta taaccatttt tgtacatatt ttacttgaaa 9600
   atattttaaa tggaaattta aataaacatt tgatagttta cataataa 9648
<210> 74
   <211> 6384
   <212> DNA
   <213> Homo sapiens
<400> 74
   agcttgtccc cgcctagcaa ggagtcggct aagaactgga tcctagcgag gagcccggca 60
   cagacagcga atgaccgcag ccagacagtc gctcttgctc ttcctcggcc ctgcggcagg 120
   atccgccggt gcaggggcct ctccccggac tccacgcgtg tctggagggc tctcgggtta 180
   gggaaggggg ctttggagac gccccgggcg gccgggcggt ggcgggacgc gggcccttta 240
   agaaggagcg aggggcgcgg ccaggtaggg gcgggtccag ggcggatcag cgctgcgccg 300
   gcgccggccc gggagccgga tttggagcgc gaggcgccgg tgggggcgga gggggctgcg 360
   cggcggaggc tcccgtggcc tcggacgctc ctcctagcta gcggccgccg cccgccgccg 420
   cctgcgcctc cagctccttc gccccggcgg gcccggccgc cgcttccggc agctcacctg 480
   ggaagcgctc acctgggacg cgctcacctg ggacgcgcta cctgcctccg ggcgcctggg 540
   cttcaggatg aaggaccgtc tggagcagct gaaggccaag cagctgacac aggatgatga 600
   tactgatgcg gttgagattg ctatcgacaa cacggctttt atggacgagt tcttttctga 660
   gattgaggaa actcggctta acattgacaa gatctcagaa catgtagagg aggctaagaa 720
   actctacagt atcattctct ctgcaccgat tccagagcca aaaaccaagg atgacctaga 780
   gcagctcacg actgagatta agaaaagggc caacaacgtc cggaacaaac tgaagagcat 840
   ggagaagcat attgaagaag atgaggtcag gtcatcggca gaccttcgga ttcggaaatc 900
   ccagcactct gtcctttctc ggaagtttgt ggaggtgatg accaaataca atgaagctca 960
   agtggacttc cgagaacgca gcaaagggcg aatccagcgg cagctcgaaa ttactggcaa 1020
   aaagacaacc gatgaggagc tggaggagat gttggagagt ggcaacccgg ccatcttcac 1080
   ttctgggatc attgactcac agatttccaa gcaagccctc agtgagattg agggacgaca 1140
   caaggacatt gtgaggctgg agagcagcat caaggagctt cacgacatgt ttatggacat 1200
   cgccatgctg gtggagaatc agggtgagat gttagataac atagagttga atgtcatgca 1260
   cacagtggac cacgtggaga aggcacgaga tgaaacgaaa aaagctgtga aataccagag 1320
   tcaggcccgg aagaaactga tttcactcca gactggtgtg gccacccttg tcttcagatg 1380
   agaatggagt ctgaatggcc ttcctgagag cgagtgcgac ccgttccttt gtttccttgc 1440
   aaccaccctt ggacctgact cagctaacaa tctagccctg ggggaatgtg atctacctga 1500
   tgcgaccctg agttctcccc agagcctcct cctgccccac cagctctcaa gtaccttttc 1560
   tcctggactg tgtggaccca cccagctttc ttcctccctg ttgtgtgtca gattatgcct 1620
   tgcacttggg aaagctcttg tgagactctc ccaaggtgct gtatttttct acctcatgga 1680
   gtattctccc agaaactgca atgtattttt ttaggggagt atctttaaca aagcagaatg 1740
   attcttctaa gtttggcaac aagaaggctt ggatctgagt cttctacctg gcaggatgcc 1800
   aatcctgttt gttgtccgta tgtcctgaaa acatgaggga ctggcagatg tcattttggt 1860
   ctaaagagct gacttgtttg aaattcagcc ttaaattaag ctcttagttg ttcagcttgg 1920
   ggggcaactt tgatttttct ctgtgttgta gtctctcata tttactcaag gagggaccag 1980
   gatgatacag tcatctgagg ttatgctttg caaaaggctg acggtatgga atatgtttcc 2040
   atgtctgagt cttagaaact ggctgctcat tgttagaaag tgatgctttg tgagactatt 2100
   gtcttggggc caaaaataat cagggatttt aaattgggca agggacaagg tgctagaatc 2160
   ctaagctctg gaaatatttc atgacactgg tgtattcact catgtgttcc agatgtattc 2220
   taattgtgta tgaaatgtat gtacacataa gtgtgtgtgt ctcaggaagt aggaaataaa 2280
   aatggaagct attatgacct caaaaaaaaa aagccaactt tgagctagga taaaaattgg 2340
   gtaaaggaca tttgcttacc tgcaaatgaa tcactgtgga aatgtgatct tcccatatca 2400
   tcaagaaact tgttttctgg atgaatactg ggagaataaa atgagaactc tggagtgagc 2460
   taaattgatc ccaattaagt ttttctgctt agcagacaga aggtataatt ttttgacacc 2520
   ctttcccacc tggtgcctat gctaggcttg tcctgagaac atccctcagt aacttgatat 2580
   tcacatgacc tacaggatgt cccatctgca gggctgagtc agttggggaa caccagaggc 2640
   tacacagtag ctcttcctgc tactcggtta atgagcttgg caggttcttt gtctcactga 2700
   attcttatca tggaaacagc agcagcagcc gctaggaaat cttcaagtgt agtgtctgtg 2760
   ctaacccagt ggtaaatccc ttagatcccc tgctggtctc tggcagtctc cttgattttg 2820
   ggtaccatgt atattttccg ctttgacttt aacgctttct aggatagggt aagcaccctt 2880
   aattcaggca ctgtccatta gcttcctttg caaaggctac ttatggccgg tcacaatcca 2940
   gcactcagac agagccaagg caatatcctc ttgcccatgg ctatgatgtc agacagtgga 3000
   tgggctccag caacaagaga caaaataact aaaggccttt gctctcctct gacattgagg 3060
   cctggggctt acagtttgga atacaacatg tgaaggtttt tgttgttgtt tgtatttttt 3120
   agatgtaaac ttgattattt tattgctaat ttaaaaataa aaatgacttt gtattgattg 3180
   tgaaacggtt ctggctctgt ctcgatgcag aaacacaatg atctggtgcc accatgtggt 3240
   gatttttatt caggttttag aatgcagttc acaccttttt aagccatgtg ctggatcaga 3300
   tggttcaaaa gtgcaatttt tgaacatggt ttaactccca cagaatgcag tgtaactatg 3360
   tttgtgtttc agatttgagg tgttcccccc aaaagaattt ggttcagtcc ttgggagtat 3420
   ctggctttag gaggaaatgg gggagatctg tcacgatgtt atctagaagg tggaatgacc 3480
   ataccaaaca tccttttaat ctaacttgaa tgtctcacca aaaataacat ttctgttggc 3540
   attctgggtc ctagaagcca gatccatctc ctttttcctt ctgttgctct cttccttcac 3600
   accctcttcc atgtccacat gcacttatct ccctgcagaa tactttttgc gatgatgttt 3660
   ctcatgtatt ctttctttcc ttgtctggat gagcagaaga agatcatgat catgatctgc 3720
   tgtattatcc ttgcgatcat cttagcttcc accattggga gcatatttgc ctgaaaaagg 3780
   tgagccatct gtggggaggg tcagaccttc tttcactgac ttgaaacctt tgtgtcttgg 3840
   gggcactcta ggtgccttaa tctgggtggg attaggtgct aataatggtt agagaaaact 3900
   aaagaaaggg atgtttcaga gacagaaaag tgagtgaaga atgaactgtt agtaggtagt 3960
   ctgtgggagg aggggggaga cagaaggtgg caatctgtcc tataacctgg tgtggcagaa 4020
   tgctttgtac aggtgaagga tagtgattcc tgctaaacag tttgagcctt ggtatctgga 4080
   agtgacaaaa agaacaagaa ttagttcttg cattaggtgc atcttgaact ttttggaaga 4140
   gggccggcca cacagtaaat tcaaattaaa tttcttccct ttcaaggtta atgaaagtta 4200
   acacagcctt gtatgtagtc ctttaccccg ggtaagaggg atttggtgat cccagccacg 4260
   aacaccatgc tatataatct atgatttttt tcctccattt ttctgttatc tcccacagcc 4320
   ctcacagatt gatcgactgg catttctaat cctccttcca cttctgtggt accatcactt 4380
   ctccacgcag actcctcatc agcttctcct ctttccatta tgaaacttct taagaaacag 4440
   ggcaccaatc aactacttat taagaattat gcaaagaata aacgtataca gaattgggcg 4500
   gaggacaggg acagggagta cagatacata gctgattagg cagatggttt aaaggaggac 4560
   tgcagggtag agaagcaagc agagtgggcg cctctttagg aagtgacaca gcctgcatgt 4620
   gcagtatggc tgtgaagggg cagatatcat agcacaccta actcaacagg atcttacttg 4680
   aactgctgtg agttggtcaa gtcagggcac ttctgctctt caggctcctc taggtcacac 4740
   ctttgaccac cctacatctg tttcctcttt caggctccag tagtagtctt aaaagtgaag 4800
   tttatctaag gataagcaca tgcctatctt gctcactgct gtgtccccac tttttagcac 4860
   agcgcctgct gcttacaggt actcaaatat ctgctgactt aattacttat aattaagctc 4920
   ttatttcagt catggacaaa tccttgggtt tgactcctaa actctttaag gtaccaatga 4980
   gaacgtggtt tgttactgtc agaggctgtg taaagccgct tgggaatggg ctgatctgct 5040
   tatgcaaaaa tgctaccaac ctttcaaacc ccattaccac aacatgaaaa tttaaagtgc 5100
   tttttctatg ggtggtgatg gtggagttct agcaacagcc tcttaatctg tggagaagat 5160
   ggctggctcc aggactgtgg ttcaataacg aaatacaggc ccacaaaata aaataggttt 5220
   atagcatgac tgaactcaca catcagtaga acactctgtg aaccataaca aagaacagat 5280
   aaaggtgtca agtgagaaag gtgaaatgag gttatcactc acttcacctc tcacctgatt 5340
   tgtgttgcta gctgaaactg ctggccagta acgtatgtat aagaaactgt tataggccgg 5400
   gcacggtggc tcatgcctgt aatcacagca ctttgggagg ccaaggcagg tggatcacca 5460
   gactgaccaa catagtgaaa ccccctctct acaaaaaata caaaaattag ccaggcatgg 5520
   tggcttgcac ctgtattccc agctacttgg gaggctgagg caggagaatt gcttgaatgc 5580
   aagaggcgga agttgcagtg agccgagatc gcgccactgc actccagcct gggcaacaag 5640
   agctaaattc catctaaaaa aaaaaaaaaa acaaaaaaaa aaaactgttc ttaatactta 5700
   atactgtccc caattccatt caaggttcag ttgtgttcag ctttaaaacc agctatgtga 5760
   atgtgagttc tagtgcagat tatttagtga ttatgtaact aaaattgatg aaaaaatcac 5820
   actatacaac tgtgaggagc acacaactgc taagtttgta cttttgaaag taaattattc 5880
   tttgtttgat accttatttt ttaagaaagt gggattaaaa atattttggt cagtgctgtt 5940
   ttctacccac cttcaaaagc caatggtttg atatttctat taatttgtgc ttcctttagt 6000
   tttaataggg gatagaagac tgcagctggt tgggtctgga aaacattaat ctgggcaaac 6060
   ggagctggag ctatgaggat ctgagtccta gggggccctc attcactagc agtaagaatt 6120
   taagtggtgc atggcacata gcactgtact agattctgca ggggcacaaa catagagcca 6180
   aacactctcc ctcttggaga atttcagacc cagaaaaggc cacgcagttc taacttttgt 6240
   catcggttcc ttttgctaaa aggcaaaggg tatgttcctt gcctattgtc caacataccc 6300
   cttccaagat tgtgagaaga tgggtagctg ggcatcaata aatattgaat caattgacct 6360
   aaaaaaaaaa aaaaaaaaaa aaaa 6384
<210> 75
   <211> 4548
   <212> DNA
   <213> Homo sapiens
<400> 75
   gggactggag gctgccgagg gggccggcgc ccgagtccgg gattcggcca gtggtgctga 60
   gcgagtgctg gaccagcggc cgtcctgtgc acctggcctg tgcgcgtgcc cgctgctcgg 120
   cttcacccag actaaggcgc gggcagctgc gggaacaggc ggggtgggcg gagggagacc 180
   gggaggcacg ggcgccctgt gcgcggagga ggtgaaggcg gccggggccg ggacgccatg 240
   tccatggagg accccttctt tgtggtgaaa ggagaggtac agaaagcagt caacactgcc 300
   cagggattgt ttcagagatg gacagagctc ctccaggacc cctccacagc aacaagggaa 360
   gaaatcgact ggaccaccaa cgagctgaga aataacctcc ggagcataga gtgggatcta 420
   gaggaccttg atgaaaccat cagcatagtt gaagcaaatc ctagaaaatt taaccttgat 480
   gcaactgaat tgagtataag aaaagccttc attacaagta ctcggcaagt tgtcagggac 540
   atgaaagatc agatgtcaac ttcatctgtg caggcattag ctgaaagaaa aaatagacag 600
   gcactgctgg gagacagtgg cagccagaac tggagcactg gaacaacaga taaatatggg 660
   cgtctggacc gagagctcca gagagccaat tctcatttca ttgaggagca gcaggcacag 720
   cagcagttga tcgtggaaca gcaggatgag cagttggagc tggtctctgg cagcatcggg 780
   gtgctgaaga acatgtccca gcgcatcgga ggggagctgg aggaacaggc agttatgttg 840
   gaagatttct ctcacgaatt ggagagcact cagtcccggc tggacaatgt gatgaagaaa 900
   cttgcaaaag tatctcatat gaccagtgat cggcgccaat ggtgtgccat agccatcctc 960
   tttgcagtcc tgttggttgt gctcatcctc ttcttagtgc tgtgacggcg gggcctctgg 1020
   gtgcgagttc ctcctgcata tgaaccgagg ggaggaggag aagctgagca cgtgtgacat 1080
   tgccgtctac tcacattcct atcctggaaa catactgctg cactgacttt tctccgtgtg 1140
   accccacaat tgacatggct cctccatccc agcgctggaa gggccagtgg gaagaggaaa 1200
   tagatgtctg cactcctggc tgcagctgga caacagaagc cccatgccgc ctgtccagtt 1260
   cggaggagaa ctagctgctg ccttgccttc cgggacctcg tttgctgagg agggacttac 1320
   agactccact ggtgttttgc tgttgctcat tccatgcatc tttggcagct cttttcttct 1380
   gctcagaccc ttccccgtgc tcagacagtg caccgctgtc ccatctaaag aaacctgtca 1440
   ggaatacgag cttctgggta tgtttcgttt cccattgctg tagcatttct tatcccctga 1500
   gagctgatga ttattgagga cagaaggctc agaaacagtt tgtgacagaa aatgcagtgt 1560
   ttcatttttc agggataaat gctaagataa aattgctttt ccaggtcatt tttttttgtg 1620
   gtaagaataa ctaatggaaa ataatgaaac accctggggt ttgggggtgc taacaacttg 1680
   tggctttaac tgacaggagc aattaaaaag agcaagaggg ttctgcattg gcatagctta 1740
   gggaagggtt aatgatgtcg ccacaggtca gctcctgatc cttgccgact tgatgttgct 1800
   gtaccagggc ttcctcccca gaggtgcagc ttgcgttttg agggtgattg ctacatatgt 1860
   tgttgctaaa cagctcagta acacacttga atgaatttgg ataccagatt gtcctcatta 1920
   cagttctttt actcttaggg cactctacac tgggggttgg ggttgggagt ggttagtaca 1980
   tttattacat ttattaagaa acgtaatgac ataaaaggtt agctctgggc cagacttctc 2040
   ttactctgtg ggtaatggca aggatgtgta ggtaaacttg gttctttttt ttccctaaga 2100
   tgacagcttg attttatcat ctgcagtcaa ataactgagc caatccaaat ttaaatgata 2160
   gatgctttaa ttgagtttaa gtagctgaaa ctgctgagac actaaacttt aaccttctga 2220
   tgacttttta aaatgcctca aatgtgcaca tgtatatagg atatttttat aacttccctg 2280
   atgaataatc tgatattaaa gtagtatttg gacccagagc cagaactcgg tggtggaggc 2340
   tgctggtctc tcctcaccac cttcttttgc acttggaaag aacagcaaca tctggataga 2400
   gttctggctt tgacttctca tttccttgtc tttttgggtg cattcctcag cacacttttt 2460
   ttttaaacct ttttgttttg ttttgtttgt atttcatgtg gttttatttg ggggttttgg 2520
   ttttttcacc cttttttgtg atttgcaatg atgtgcttgc ccagctaact tttgaattgc 2580
   actttttaat aaatattctt aacaattttt gaagaaggat attttatctc atttgagatc 2640
   atggtaggtt aagaaaatat gcctgttgat gaaagctaaa agcaaattta tgaaactaaa 2700
   agggtgattg acatccatgt ttacactccg ctctaatgtt tgatatataa ataagttatt 2760
   ttcaaattag gaaaaaacag tgagtattac aaagggcttc agatgtttag agtactaggt 2820
   tatttatgtt ttacaaagtt tgaatcttct ataaactaag aaaggggatg atccttagat 2880
   ttgcattaaa atatagaagt cttttaaagt aaatgtgaac cttgtctaag tactgtaatc 2940
   cacacaacac attataagaa gcaaaccagc atcttaagga attataaaat taccctattt 3000
   aaaagccatg ctattgttct gctattacca gatttattgt gccacacaaa aggatcatgt 3060
   gtgtcagcag gggccgtttg gaacaaacct agtcattaat gagtaagata ctcctgttag 3120
   ttcagggacc aagttttatg acccagaggc ttaatgatgt ttggatatat ttcaaatcgg 3180
   cgtgcttacc tcactgattt aaattatttt ctaaatagtg gccattgtag acctgactca 3240
   ggctgaagct aaatagagaa caatttagaa agttaactaa caatacagtg cattctaccc 3300
   gtagggccac catgcccttc tgcccctggc tgatttgatc ctgtgtctga tcccattgca 3360
   ccctgactgg gcagtcccta cagaaccagt gttaatttga agggcctcca ctcaggctcc 3420
   aaatgtggca gccaaagaga acaatccagg gaacctacat ttatttttaa ggacaaatat 3480
   ttcctcctca gtggtcctaa tgttcagggc tttagaggga acccaggtgg tctcttcacc 3540
   ctgtgtccta gaatgggaga gtaagtagac agtggtgata accccacact gcttataagt 3600
   gcatctttat agtatttggg gctttcctac ccctttagcc ttctgtacct agtaccatat 3660
   tccagtttta aagaactggc agaatgtgat ggataacaga ggaagagctc aatttatgtt 3720
   tattggaaga acattttact taaatgattt gaggggtggg agggagtgaa ctactgagtt 3780
   tgccagagtg aaaatccatc tgaaaaactc agctaccttt agtttttagt cctcattttt 3840
   ggtcttgtct ctgcggactg tgaagaatca caatgctcta tatgccctgg actgtgtggc 3900
   aaatgcaggt tgcagcgtgt gtgttacatg aggatcttcc acaatttcag aatgcacgcc 3960
   agagctgaag ggggaaactt ggtaacttgc ccattattct ctgcttttag ccagagttaa 4020
   acagactgat gggtctggta gccaacaact tggcaacttc cactccttct cacctcgtga 4080
   gattaagggc tgtgaaaaga aatctagtct aactccaaca gaaatctgtc tctgttaagt 4140
   gttttacctt ctgtaagtag agatggtaga gccaagattt ttcttttggt aatttccctg      4200
   tctataaagt gagaccaaag ggatatctgt tccctgttac ctttttggag aattcataac      4260
   atttgaagat caaaaaattg aatgataaat atgaatggct tttcaattct gtggactttg      4320
   taccatttgg cttcaccttg tactgcaaga tgaatttgta aacaaaacaa aattggactg      4380
   tctggaaagc taaagttctg aaatatggaa tgtactgcct ctaatttttc tttgtcttcc      4440
   tctcactggc atttttttct ctcccaggtt tcttaagaat aatgtttttt aaaggaggct      4500
   ttttgcccat caagaataaa aagaaataaa accaaaaaaa aaaaaaaa                   4548
<210> 76
   <211> 2124
   <212> DNA
   <213> Homo sapiens
<400> 76
   ggccgcgctg ccgatcgccg ggaggacccc cgcctcgccg aagacgggcg gggcaagccg 60
   agcctcacgg ggtccccgga gctgggccgg gcctccagat ggagaaggcg caacggggag 120
   ttcttgagta agccagagcg gtgtccagcg cggtgtagcc gcagccgccg ctgtcaggcg 180
   cagcaacggg caaccccgta gaagtcggtc ggcaggtcct ctccaacccg ccgctaccgc 240
   gccgctgtgg gagagacccc agcaggagcc caaaggcagc tacgggggcg cgaaggccgc 300
   tggcgccgcc tcggccagcc cttcccgcgc ggttccactg ccttaaggat gacagtcgta 360
   gggaaccctc gaagttggag ctgccagtgg ttgccaatcc tgatactgtt gctgggcaca 420
   ggccatgggc caggggtgga aggcgtgaca cactacaagg ccggcgaccc tgttattctg 480
   tatgtcaaca aagtgggacc ctaccataac cctcaggaaa cttaccacta ctatcagctt 540
   ccagtctgct gccctgagaa gatacgtcac aaaagcctta gcctgggtga agtgctggat 600
   ggggaccgaa tggctgagtc tttgtatgag atccgctttc gggaaaacgt ggagaagaga 660
   attctgtgcc acatgcagct cagttctgca caggtggagc agctgcgcca ggccattgaa 720
   gaactgtact actttgaatt tgtggtagat gacttgccaa tccggggctt tgtgggctac 780
   atggaggaga gtggtttcct gccacacagc cacaagatag gactctggac ccatttggac 840
   ttccacctag aattccatgg agaccgaatt atatttgcca atgtttcagt gcgggacgtc 900
   aagccccaca gcttggatgg gttacgacct gacgagttcc taggccttac ccacacttat 960
   agcgtgcgct ggtctgagac ttcagtggag cgtcggagtg acaggcgccg tggtgacgat 1020
   ggtggtttct ttcctcgaac actggaaatc cattggttgt ccatcatcaa ctccatggtg 1080
   cttgtgtttt tactggtggg ttttgtggct gtcattctaa tgcgtgtgct tcggaatgac 1140
   ctggctcggt acaacttaga tgaggagacc acctctgcag gttctggtga tgactttgac 1200
   cagggtgaca atggctggaa aattatccat acagatgtct tccgcttccc cccataccgt 1260
   ggtctgctct gtgctgtgct tggcgtgggt gcccagttcc tggcccttgg cactggcatt 1320
   attgtcatgg cactgctggg catgttcaat gtgcaccgtc atggggccat taactcagca 1380
   gccatcttgt tgtatgccct gacctgctgc atctctggct acgtgtccag ccacttctac 1440
   cggcagattg gaggcgagcg ttgggtgtgg aacatcattc tcaccaccag tctcttctct 1500
   gtgcctttct tcctgacgtg gagtgtggtg aactcagtgc attgggccaa tggttcgaca 1560
   caggctctgc cagccacaac catcctgctg cttctgacgg tttggctgct ggtgggcttt 1620
   cccctcactg tcattggagg catctttggg aagaacaacg ccagcccctt tgatgcaccc 1680
   tgtcgcacca agaacatcgc ccgggagatt ccaccccagc cctggtacaa gtctactgtc 1740
   atccacatga ctgttggagg cttcctgcct ttcaggtatc ctccctttat tccatggcta 1800
   ttactgtcag gttcctgacc tcaatttttc ctgtccctac tcatccagta ccctaaccca 1860
   acccgttgat ccctggttca gtggtaccat tcagagatca ttaaatggtt cctcctatcc 1920
   ccaagcagga ctgagcttga atgatatgag agtgtctcac ttataaagct ctccggagac 1980
   atttccccct tcaccttcct ggtttctgac tttaatgcct atggacatca tgtggggttt 2040
   aaagcccatt tgatgaccca tttactttgt tgaatacctc tttgtgccag gcaaagaata 2100
   aagtggaata aaatggaaaa aaaa 2124
<210> 77
   <211> 2375
   <212> DNA
   <213> Homo sapiens
<400> 77
   gggcgcggag ccccagccga gcctagccct gcccggcccc ggaggacttg caacactccg      60
   aggccaggaa cgctccgtct ggaacggcgc aggtcccagc agctggggtt ccccctcagc     120
   ccgtgagcag ccatgtccaa ccccagcgcc ccaccaccat atgaagaccg caaccccctg     180
   tacccaggcc ctccgccccc tgggggctat gggcagccat ctgtcctgcc aggagggtat     240
   cctgcctacc ctggctaccc gcagcctggc tacggtcacc ctgctggcta cccacagccc     300
   atgcccccca cccacccgat gcccatgaac tacggcccag gccatggcta tgatggggag     360
   gagagagcgg tgagtgatag cttcgggcct ggagagtggg atgaccggaa agtgcgacac     420
   acttttatcc gaaaggttta ctccatcatc tccgtgcagc tgctcatcac tgtggccatc     480
   attgctatct tcacctttgt ggaacctgtc agcgcctttg tgaggagaaa tgtggctgtc     540
   tactacgtgt cctatgctgt cttcgttgtc acctacctga tccttgcctg ctgccaggga     600
   cccagacgcc gtttcccatg gaacatcatt ctgctgaccc tttttacttt tgccatgggc     660
   ttcatgacgg gcaccatttc cagtatgtac caaaccaaag ccgtcatcat tgcaatgatc     720
   atcactgcgg tggtatccat ttcagtcacc atcttctgct ttcagaccaa ggtggacttc     780
   acctcgtgca caggcctctt ctgtgtcctg ggaattgtgc tcctggtgac tgggattgtc 840
   actagcattg tgctctactt ccaatacgtt tactggctcc acatgctcta tgctgctctg 900
   ggggccattt gtttcaccct gttcctggct tacgacacac agctggtcct ggggaaccgg 960
   aagcacacca tcagccccga ggactacatc actggcgccc tgcagattta cacagacatc 1020
   atctacatct tcacctttgt gctgcagctg atgggggatc gcaattaagg agcaagcccc 1080
   cattttcacc cgatcctggg ctctcccttc caagctagag ggctgggccc tatgactgtg 1140
   gtctgggctt taggcccctt tccttcccct tgagtaacat gcccagtttc ctttctgtcc 1200
   tggagacagg tggcctctct ggctatggat gtgtgggtac ttggtgggga cggaggagct 1260
   agggactaac tgttgctctt ggtgggcttg gcagggacta ggctgaagat gtgtcttctc 1320
   cccgccacct actgtatgac accacattct tcctaacagc tggggttgtg aggaatatga 1380
   aaagagccta ttcgatagct agaagggaat atgaaaggta gaagtgactt caaggtcacg 1440
   aggttcccct cccacctctg tcacaggctt cttgactacg tagttggagc tatttcttcc 1500
   cccagcaaag ccagagagct ttgtccccgg cctcctggac acataggcca ttatcctgta 1560
   ttcctttggc ttggcatctt ttagctcagg aaggtagaag agatctgtgc ccatgggtct 1620
   ccttgcttca atcccttctt gtttcagtga catatgtatt gtttatctgg gttagggatg 1680
   ggggacagat aatagaacga gcaaagtaac ctatacaggc cagcatggaa cagcatctcc 1740
   cctgggcttg ctcctggctt gtgacgctat aagacagagc aggccacatg tggccatctg 1800
   ctccccattc ttgaaagctg ctggggcctc cttgcaggct tctggatctc tggtcagagt 1860
   gaactcttgc ttcctgtatt caggcagctc agagcagaaa gtaaggggca gagtcatacg 1920
   tgtggccagg aagtagccag ggtgaagaga gactcggtgc gggcagggag aatgcctggg 1980
   ggtccctcac ctggctaggg agataccgaa gcctactgtg gtactgaaga cttctgggtt 2040
   ctttccttct gctaacccag ggagggtcct aagaggaagg tgacttctct ctgtttgtct 2100
   taagttgcac tgggggattt ctgacttgag gcccatctct ccagccagcc actgccttct 2160
   ttgtaatatt aagtgccttg agctggaatg gggaaggggg acaagggtca gtctgtcggg 2220
   tgggggcaga aatcaaatca gcccaaggat atagttagga ttaattactt aatagagaaa 2280
   tcctaactat atcacacaaa gggatacaac tataaatgta ataaagttta tgtctagaag 2340
   ttaaaaccca aaaaaaaaaa aaaaaaaaaa aaaaa 2375
<210> 78
   <211> 7717
   <212> DNA
   <213> Homo sapiens
<400> 78
   acggaaatga aaggagcact tccgggttcg gcaataacct ggagccggcg gcgtaggttg 60
   gctctttagg gcttcacccc gaagctccac cttcgctccc gtctttctgg aaacaccgct 120
   ttgatctcgg cggtgcggga caggtacctc ccggctgctg cgggtgccct ggatccagtc 180
   ggctgcacca ggcgagcgag acccttccct ggtggaggct cagagttccg gcagggtgca 240
   tccggcctgt gtgtggcgcg aggcagggaa gccggtaccc gggtcctggc cccagcgctg 300
   acgttttctc tcccctttct tctctcttcg cggttgcggc gtcgcagacg ctagtgtgag 360
   cccccatggc agatacgacc ccgaacggcc cccaaggggc gggcgctgtg caattcatga 420
   tgaccaataa actggacacg gcaatgtggc tttctcgctt gttcacagtt tactgctctg 480
   ctctgtttgt tctgcctctt cttgggttgc atgaagcagc aagcttttac caacgtgctt 540
   tgctggcaaa tgctcttacc agtgctctga ggctgcatca aagattacca cacttccagt 600
   taagcagagc attcctggcc caggctttgt tagaggacag ctgccactac ctgttgtatt 660
   cactcatctt tgtaaattcc tatccagtta caatgagtat cttcccagtc ttgttattct 720
   ctttgcttca tgctgccaca tatacgaaaa aggtccttga cgcaaggggc tcaaatagtt 780
   tacctctgct gagatctgtc ttggacaaat taagtgctaa tcaacaaaat attctgaaat 840
   tcattgcttg caatgaaata ttcctgatgc ctgcgacagt ttttatgctt tttagtggtc 900
   aaggaagttt gctccaacct tttatatact atagatttct tacccttcga tattcgtctc 960
   gaagaaaccc atattgtcgg accttattta atgaactgag gattgttgtt gaacacataa 1020
   taatgaaacc tgcttgccca ctgtttgtga gaagactttg tctccagagc attgccttta 1080
   taagcagatt ggcaccaaca gttccatagt ttaacatcta gttaagctac aaatatagta 1140
   taagcattat tagcagctgg tacttctgct aggggttgta aattccaggt gttacactga 1200
   cctcaatcca atttacataa tttacataaa tgcatctcgg tggaaaaata atcattttct 1260
   tggcatgtta aatcaagctt aaaaagtttt gagaaaattt tactgcgctg tgttgctaat 1320
   ggttaaagaa gtctgtatct agtgataaat ataccagttt ttttaaaaag atgctgttgt 1380
   gcctatatca tgaagtacat taatttctca tgtaaaaaaa atagctctaa aatttgtttc 1440
   aacctaattg gtaacctgag tttatatctg gcatgaattc attatggtga tacacatatg 1500
   tgaattcagt acattttgag acagtattct accattcagt aattttggtt aatgatttta 1560
   acacttctca gtgtatttaa tttcaaattg tttttttaat tggttttatg ctgctttgtt 1620
   aggacagatg tgttttgaat gtaccattat aagaagaatt ctatgtatct taaactatga 1680
   tcttctaaaa ttttatttcc gtaagtactt ctgtggcctt gagtattttt taaaaggctc 1740
   aactgtaagc ctcttagcca gttggataaa tatttggggt cacctagcca ttgaaagcag 1800
   aaagcagtag tgacacagct ttcccttcaa agagccattg agaaacattt ctcaaacagg 1860
   aaatccttct tttactaatg tggacatata gattattcgt attatagttt gtagaactac 1920
   ctagttcaga atcttgactg ccagttttct tggtttctta ggcttgaatt ttcatagaca 1980
   attgcaacag tttagatgcc ttttgaaagg aatgtaatga agattcagca tctgactata 2040
   tgtgtgtcta tcctgaaata ataatggaga gtatactgta gattacatgt ttacccatca 2100
   aatctgactt aaaaggttaa atggaaggtt ttataggtaa ggtaattgat tgggaatggg 2160
   gtagggggag gagttgtggg ggaataatgt gcatttcagt ctcaacgcat agataaattt 2220
   aggggaattg gatgtattat tcaactttga tttgggttgt aaaatgtgtt aaatcctgtt 2280
   cattgaactc ccatcaactc ttataaaatt catgctgatc ttcattaccg ttgcatgatt 2340
   ggaaatgttt aaaacattgt acagttttag tatagagaaa tgtaatggtt tttgtgacca 2400
   gtttctgtct gcatgtaatt tggatttctc aaatacattc attagtaatt tatcagtaac 2460
   attagtttta tttttgttca tctccttatc tataaaaagg ggatattctt aggataaata 2520
   catgaaaaat tatacttgat agcttaacta taatcagcta tttttgtatt tttgtaatat 2580
   ttgtccacta agctggagaa gcagcctcat acagttgatt ttgtgtatgt ggctagtctt 2640
   attgtcacta tgtaagtaat ccaatggttt tagaaactaa actttctaga gcaataaaat 2700
   gactataatg ttaagtaaac ataatgttga tttctaatta tgttttaaaa aatgaagtct 2760
   tgaattatat caagaaattt tggcagctga agtcatgttt attttgaagc tgttagtttt 2820
   ttcctataat ttaaaaagat cttttagatt tatagaagag tcagaaatgt acaagagagt 2880
   ttttttgttg ttgtttttgt tttttgagac agagtctgtc tctgtcgcca aggctggagt 2940
   gcagtggcgc aatcctggct cactgtagcc tctgcctcct gggttcaagt gcttctcctg 3000
   cctcagcctc ccgagtagct gggactacag gtgcacgcca ccacgcctgt agtcccagct 3060
   gtattgtaaa aatacaaaat tttagtattt ttagtagaga cagggtttca ccatgttggc 3120
   caggatggtc tcgatctcct gacctcgtga tctgcctgcc tcggcctccc aaagtgccgg 3180
   gattacaggt gtgagccacc gcgccctgcc aagaagagtt cttttgcata ccctttactc 3240
   aggtcctctc atgttaacgt tttacataac tgtagaacat ttatctaaag taagatatta 3300
   gcccagaaca atactactaa ctgaagtata aaacttattt gaatttcaac agtttttttt 3360
   tcatttctta ttttcctttt gtgtgctctg tttataccat gatccatgat ttttttaaaa 3420
   tcatgattgt cttttaaaga tctgtgtgtc tctgttttga gtttttcctg tttattttga 3480
   aaagtactgt tggtcaagat aattggtcaa taatccatgt tggttttaac aaaaagcatt 3540
   ttaacattaa aaatattaca gtataaaata acactctgtg ctttaaattg aggttttatg 3600
   tcattttagc agaattataa tatttctgat atactcatgt ttgacaagtt gaaacagatt 3660
   tgtttcttaa aggaaggttt aatatacaaa aaaaggtaat cttaaactta cgaaaaagta 3720
   aattttacaa tttgagcatt actagatgtt tagtttgcat gaactcatag ttagaaattc 3780
   tgcaatagga atatctacaa ccggctgatt tggaatttga aattatagtg ttacatgtat 3840
   acctatcaaa ttaaaattaa ggaaatacaa tagcaatata tagaatgaat gtagtaacag 3900
   aaattaactc tttactgcat cattgaactt attgttagtt acaggtttaa aagaagttca 3960
   tttaacatcc agtgtgtcta attcttctgg aagtggtgta gtaccattgt tcttctggca 4020
   tttttaaata ttaaaccttt ttggatagat ggaagcctta tacaaaatct actttatttt 4080
   agcaaggatt ctctgtcctt ttgtatagtt ggtaccttac taatttaaac tctaatatca 4140
   atctaaagag aaatttatta tgcaatttgt atttaggttt tttttttttt ttttggaatg 4200
   aagttcagag gtagatcctc ctggaagaaa gaaagcaagc gaacttttta aagaaaatta 4260
   gacttgaata tttaagaatg tcccttacag agaaaaggcc aactataata ctaagctaaa 4320
   agttatgaaa aattaatagg ttcttttata gagctaagaa tgatgaaacc atcaatactt 4380
   ccttcttcct aaaaatccag atcaaaactt caggttaggt ttctaagttt aggacatgaa 4440
   tattattttt ttctggaaaa gaagatgagt atatgtgtaa taagacaagt agaactgaga 4500
   gatttagttt tttttttttt taagttttag ttcagaataa cattaatttt gagagattga 4560
   ggtaaagaac cttaactaat gctaaggagt ttattttgat taacataggt tattctgacc 4620
   accacctctt ccttccttaa tctccttaga atctgacagt ctcaaagctg tcacacaaat 4680
   tagactaatt ttgacacttt gaaatgaaaa cttcaaggaa gaagtagcca cggacagtta 4740
   tgtttataat cagtaggtgg cactctttcc tcaggtagcc ccccattttc acatgatgtg 4800
   tttgaaggtt aaatgccacc aaaagtgctg agtcagctat aaaactaagt ccctgaattc 4860
   catggccctt ttaaatatgt aatcattcaa gattgaaaaa aaaaattaag cattttttgt 4920
   ttgtttgctt gtttgttttt gagacggagt ttcactcttg ttggccaggc tggagtgcaa 4980
   tggcgccatc tcagctcact gcaacctctg cctcccggat tcaagcaatt ctccttcagc 5040
   cctccaagta gctggggtta caggtgcccg ccaccatgcc cagctagttt ttgtattttt 5100
   agtagagatg aggtttcacc atgttggcca ggctggtctt gaactcctga cctcgtgatc 5160
   cccccacctc ggccttccaa agtgctggat tacaggcgtg agccactgtg cctggcttgc 5220
   atttttaaaa tactgaatta ttcaaaagaa gtaccctgtc aatatgtgct ttctaggaaa 5280
   acagtaaaat aggccacaat ttggagtgac accattcaga tcaaggtcta tccagttttt 5340
   tcttttcatg ctaagtgcct acatcaccga aacacactaa tataaaatta tcctttctcc 5400
   ttcattttca gatgtgtaaa aaatggtact taaagtgttt tcatgatcat tttgtaggta 5460
   gactagatat agcccgttga acctctttta aaatttagac ttttgatagt aatataaaag 5520
   catattgaaa tttgtagata ttatatgagg aatggcacct agatttgaaa attatgcttg 5580
   gcttgtagag acaactagtt tctctcgctc tttttttttt tttttttttt ttttttgaga 5640
   cagattctca ctcagttgcc caggctggag tgcagtggtg cagtcttggc tcactgcaac 5700
   ctctgcctcc tgggttaaag cgattctcat gcctcagcct ccctagtagc tgagactaca 5760
   ggcgtgcacc accacgccca gctaattttt gtatttttag tagagacagg atttcaccat 5820
   gttcaccatg ttggtcaggc tggtcttgaa ctcctggcct caagtgatct gcccgcctcg 5880
   acctcccaga gtgctgggat tataggtgtg agccactaag cctggctgag acaactagtt 5940
   tcccttaact cattggaatt ctctaggatt aggagaattc cacagagcct atatgatatt 6000
   atagctcaac atttagtata ccaaaggcat acccgtgtaa atctaggagt tatttccaga 6060
   gattgtttta aggagcagtc ttatattcag ggtagaaagt tatgattgga tctgctgtta 6120
   aggagaacaa aggagcttct aaaggtttgg gaggtttact ggtagtaact attctaggaa 6180
   atatttatgt tttaaggtga tgttcacatg ggttctttag aaggaacata gtcaagtgtg 6240
   atggattaac tctatatagt ctttctcctc ttgtgcgtgt aggaaatctg acctgcagtg 6300
   tcagttgatg tgacaagaga taaagaaagc acagtatttt aaaatctaaa gcagattcct 6360
   ttcttagaaa acaataggaa aaaattatag atggatgtct ttgctgaaat ctaacaatta 6420
   gctcatattc catgagaaag agtggcctaa gaattatttc atgttaccta gccttctgaa 6480
   gctactcact tgatgtgcct agcactttga aactaacctt ttctttcttt gttcatgaca 6540
   gtttaattcc aaatatttac tattttctct tgtaactgtt agaacagttc cttttgacat 6600
   taatttttgc ctacatatat atttttaagt tgagaccaaa tcggtgaagt gttgagcaag 6660
   taacatttat gatgtgtgta tattggaaca aatgtaaaag ggttacaaag attagaaaca 6720
   gagtcataaa aaatggcttg atttataaag gcattacttt tggtgcttta tataatggca 6780
   tatattgaac taaaaatttg tatatacagt atgtcagcat ttcttagtaa cttctcttga 6840
   atccattttt aatatctaat attgtacagg ttggggagtt acattcttca ggccaatact 6900
   atccagacta tataaattta taaaataaat tgaaaaattc attcccctgt attcaagacc 6960
   aaagcacata aatgctaatg tagggctcag aggggaaata cagttctcct gcatatttga 7020
   gaaaatgtga agtcctttca agaaaatcta ataaacataa taatcatagc ctgctgacac 7080
   taaggaaaaa ggacctcatt cactctttct tttatgcagt gatttactgg tccctactga 7140
   tttccaaatt ggatcacgat agtaaattat ccatgctggt acctgtgaaa gtaagccctg 7200
   ggatccatat ttgttttgtg ttctgcttaa atcagcaaga atgataaatt tgatggtgtg 7260
   aaattggaag tatcaagggc tttctttggt gattgaggga aataatgtct ctacttgtaa 7320
   tttattgtga ccctttttca ctgtatatgc tttgtatgtc taatatttat ttcaatgcaa 7380
   attcaattgt tccttcatct gtattgttat atctaagatt ttattgatgt taaaatctaa 7440
   ttgtggaata aaaatctctc tggaatttag cagatacaaa aatgttatct tgcaaaagaa 7500
   ctaagaacat ttgtagttag aaatcagctt tcctttgagc ttaattgcct ttttgttaga 7560
   ataaggtgaa tttgaacaca ctcctcttat cctcagccca tcacaaataa tagagatgcc 7620
   atgattttga ggtctgatgt gaaactggta aaaatgtgat ctaaggtgta actggaaaaa 7680
   aaaaggaaag aaaaattaca ttgatgcctc agctgtt 7717
<210> 79
   <211> 4154
   <212> DNA
   <213> Homo sapiens
<400> 79
   acttggacgc gcttgcggag gattgcgttg acgagactct tatttattgt caccaacctg      60
   tggtggaatt tgcagttgca cattggatct gattcgcccc gccccgaatg acgcctgccc     120
   ggaggcagtg aaagtacagc cgcgccgccc caagtcagcc tggacacata aatcagcacg     180
   cggccggaga accccgcaat ctctgcgccc acaaaataca ccgacgatgc ccgatctact     240
   ttaagggctg aaacccacgg gcctgagaga ctataagagc gttccctacc gccatggaac     300
   aacggggaca gaacgccccg gccgcttcgg gggcccggaa aaggcacggc ccaggaccca     360
   gggaggcgcg gggagccagg cctgggcccc gggtccccaa gacccttgtg ctcgttgtcg     420
   ccgcggtcct gctgttggtc tcagctgagt ctgctctgat cacccaacaa gacctagctc     480
   cccagcagag agcggcccca caacaaaaga ggtccagccc ctcagaggga ttgtgtccac     540
   ctggacacca tatctcagaa gacggtagag attgcatctc ctgcaaatat ggacaggact     600
   atagcactca ctggaatgac ctccttttct gcttgcgctg caccaggtgt gattcaggtg     660
   aagtggagct aagtccctgc accacgacca gaaacacagt gtgtcagtgc gaagaaggca     720
   ccttccggga agaagattct cctgagatgt gccggaagtg ccgcacaggg tgtcccagag     780
   ggatggtcaa ggtcggtgat tgtacaccct ggagtgacat cgaatgtgtc cacaaagaat     840
   caggtacaaa gcacagtggg gaagtcccag ctgtggagga gacggtgacc tccagcccag     900
   ggactcctgc ctctccctgt tctctctcag gcatcatcat aggagtcaca gttgcagccg     960
   tagtcttgat tgtggctgtg tttgtttgca agtctttact gtggaagaaa gtccttcctt    1020
   acctgaaagg catctgctca ggtggtggtg gggaccctga gcgtgtggac agaagctcac    1080
   aacgacctgg ggctgaggac aatgtcctca atgagatcgt gagtatcttg cagcccaccc    1140
   aggtccctga gcaggaaatg gaagtccagg agccagcaga gccaacaggt gtcaacatgt    1200
   tgtcccccgg ggagtcagag catctgctgg aaccggcaga agctgaaagg tctcagagga    1260
   ggaggctgct ggttccagca aatgaaggtg atcccactga gactctgaga cagtgcttcg    1320
   atgactttgc agacttggtg ccctttgact cctgggagcc gctcatgagg aagttgggcc    1380
   tcatggacaa tgagataaag gtggctaaag ctgaggcagc gggccacagg gacaccttgt    1440
   acacgatgct gataaagtgg gtcaacaaaa ccgggcgaga tgcctctgtc cacaccctgc    1500
   tggatgcctt ggagacgctg ggagagagac ttgccaagca gaagattgag gaccacttgt    1560
   tgagctctgg aaagttcatg tatctagaag gtaatgcaga ctctgccatg tcctaagtgt 1620
   gattctcttc aggaagtcag accttccctg gtttaccttt tttctggaaa aagcccaact 1680
   ggactccagt cagtaggaaa gtgccacaat tgtcacatga ccggtactgg aagaaactct 1740
   cccatccaac atcacccagt ggatggaaca tcctgtaact tttcactgca cttggcatta 1800
   tttttataag ctgaatgtga taataaggac actatggaaa tgtctggatc attccgtttg 1860
   tgcgtacttt gagatttggt ttgggatgtc attgttttca cagcactttt ttatcctaat 1920
   gtaaatgctt tatttattta tttgggctac attgtaagat ccatctacac agtcgttgtc 1980
   cgacttcact tgatactata tgatatgaac cttttttggg tggggggtgc ggggcagttc 2040
   actctgtctc ccaggctgga gtgcaatggt gcaatcttgg ctcactatag ccttgacctc 2100
   tcaggctcaa gcgattctcc cacctcagcc atccaaatag ctgggaccac aggtgtgcac 2160
   caccacgccc ggctaatttt ttgtattttg tctagatata ggggctctct atgttgctca 2220
   gggtggtctc gaattcctgg actcaagcag tctgcccacc tcagactccc aaagcggtgg 2280
   aattagaggc gtgagccccc atgcttggcc ttacctttct acttttataa ttctgtatgt 2340
   tattatttta tgaacatgaa gaaactttag taaatgtact tgtttacata gttatgtgaa 2400
   tagattagat aaacataaaa ggaggagaca tacaatgggg gaagaagaag aagtcccctg 2460
   taagatgtca ctgtctgggt tccagccctc cctcagatgt actttggctt caatgattgg 2520
   caacttctac aggggccagt cttttgaact ggacaacctt acaagtatat gagtattatt 2580
   tataggtagt tgtttacata tgagtcggga ccaaagagaa ctggatccac gtgaagtcct 2640
   gtgtgtggct ggtccctacc tgggcagtct catttgcacc catagccccc atctatggac 2700
   aggctgggac agaggcagat gggttagatc acacataaca atagggtcta tgtcatatcc 2760
   caagtgaact tgagccctgt ttgggctcag gagatagaag acaaaatctg tctcccacgt 2820
   ctgccatggc atcaaggggg aagagtagat ggtgcttgag aatggtgtga aatggttgcc 2880
   atctcaggag tagatggccc ggctcacttc tggttatctg tcaccctgag cccatgagct 2940
   gccttttagg gtacagattg cctacttgag gaccttggcc gctctgtaag catctgactc 3000
   atctcagaaa tgtcaattct taaacactgt ggcaacagga cctagaatgg ctgacgcatt 3060
   aaggttttct tcttgtgtcc tgttctatta ttgttttaag acctcagtaa ccatttcagc 3120
   ctctttccag caaacccttc tccatagtat ttcagtcatg gaaggatcat ttatgcaggt 3180
   agtcattcca ggagtttttg gtcttttctg tctcaaggca ttgtgtgttt tgttccggga 3240
   ctggtttggg tgggacaaag ttagaattgc ctgaagatca cacattcaga ctgttgtgtc 3300
   tgtggagttt taggagtggg gggtgacctt tctggtcttt gcacttccat cctctcccac 3360
   ttccatctgg catcccacgc gttgtcccct gcacttctgg aaggcacagg gtgctgctgc 3420
   ctcctggtct ttgcctttgc tgggccttct gtgcaggacg ctcagcctca gggctcagaa 3480
   ggtgccagtc cggtcccagg tcccttgtcc cttccacaga ggccttccta gaagatgcat 3540
   ctagagtgtc agccttatca gtgtttaaga tttttctttt atttttaatt tttttgagac 3600
   agaatctcac tctctcgccc aggctggagt gcaacggtac gatcttggct cagtgcaacc 3660
   tccgcctcct gggttcaagc gattctcgtg cctcagcctc cggagtagct gggattgcag 3720
   gcacccgcca ccacgcctgg ctaatttttg tatttttagt agagacgggg tttcaccatg 3780
   ttggtcaggc tggtctcgaa ctcctgacct caggtgatcc accttggcct ccgaaagtgc 3840
   tgggattaca ggcgtgagcc accagccagg ccaagctatt cttttaaagt aagcttcctg 3900
   acgacatgaa ataattgggg gttttgttgt ttagttacat taggctttgc tatatcccca 3960
   ggccaaatag catgtgacac aggacagcca tagtatagtg tgtcactcgt ggttggtgtc 4020
   ctttcatgct tctgccctgt caaaggtccc tatttgaaat gtgttataat acaaacaagg 4080
   aagcacattg tgtacaaaat acttatgtat ttatgaatcc atgaccaaat taaatatgaa 4140
   accttatata aaaa 4154
<210> 80
   <211> 3809
   <212> DNA
   <213> Homo sapiens
<400> 80
   ggcagccgcg cccgctgggc cacagaggcc gctgaggccg cggcgcccgc cagcctgtcc 60
   cgcgccatgg ccccgcgcgc ccggcggcgc cgcccgctgt tcgcgctgct gctgctctgc 120
   gcgctgctcg cccggctgca ggtggctttg cagatcgctc ctccatgtac cagtgagaag 180
   cattatgagc atctgggacg gtgctgtaac aaatgtgaac caggaaagta catgtcttct 240
   aaatgcacta ctacctctga cagtgtatgt ctgccctgtg gcccggatga atacttggat 300
   agctggaatg aagaagataa atgcttgctg cataaagttt gtgatacagg caaggccctg 360
   gtggccgtgg tcgccggcaa cagcacgacc ccccggcgct gcgcgtgcac ggctgggtac 420
   cactggagcc aggactgcga gtgctgccgc cgcaacaccg agtgcgcgcc gggcctgggc 480
   gcccagcacc cgttgcagct caacaaggac acagtgtgca aaccttgcct tgcaggctac 540
   ttctctgatg ccttttcctc cacggacaaa tgcagaccct ggaccaactg taccttcctt 600
   ggaaagagag tagaacatca tgggacagag aaatccgatg cggtttgcag ttcttctctg 660
   ccagctagaa aaccaccaaa tgaaccccat gtttacttgc ccggtttaat aattctgctt 720
   ctcttcgcgt ctgtggccct ggtggctgcc atcatctttg gcgtttgcta taggaaaaaa 780
   gggaaagcac tcacagctaa tttgtggcac tggatcaatg aggcttgtgg ccgcctaagt 840
   ggagataagg aaatgtgact ggaaacagta actccacgtt catctccagc gggcaggtga 900
   tgaacttcaa gggcgacatc atcgtggtct acgtcagcca gacctcgcag gagggcgcgg 960
   cggcggctgc ggagcccatg ggccgcccgg tgcaggagga gaccctggcg cgccgagact 1020
   ccttcgcggg gaacggcccg cgcttcccgg acccgtgcgg cggccccgag gggctgcggg 1080
   agccggagaa ggcctcgagg ccggtgcagg agcaaggcgg ggccaaggct tgagcgcccc 1140
   ccatggctgg gagcccgaag ctcggagcca gggctcgcga gggcagcacc gcagcctctg 1200
   ccccagcccc ggccacccag ggatcgatcg gtacagtcga ggaagaccac ccggcattct 1260
   ctgcccactt tgccttccag gaaatgggct tttcaggaag tgaattgatg aggactgtcc 1320
   ccatgcccac ggatgctcag cagcccgccg cactggggca gatgtctccc ctgccactcc 1380
   tcaaactcgc agcagtaatt tgtggcacta tgacagctat ttttatgact atcctgttct 1440
   gtgggggggg gggtctgttt tccccccata tttgtattcc ttttcataac ttttcttgat 1500
   atctttcctc cctctttttt aatgtaaagg ttttctcaaa aattctccta aaggtgaggg 1560
   tctctttctt ttctcttttc cttttttttt tctttttttg gcaacctggc tctggcccag 1620
   gctagagtgc agtggtgcga ttatagcccg gtgcagcctc taactcctgg gctcaagcaa 1680
   tccaagtgat cctcccacct caaccttcgg agtagctggg atcacagctg caggccacgc 1740
   ccagcttcct ccccccgact ccccccccag agacacggtc ccaccatgtt acccagcctg 1800
   gtctcaaact ccccagctaa agcagtcctc cagcctcggc ctcccaaagt actgggatta 1860
   caggcgtgag cccccacgct ggcctgcttt acgtattttc ttttgtgccc ctgctcacag 1920
   tgttttagag atggctttcc cagtgtgtgt tcattgtaaa cacttttggg aaagggctaa 1980
   acatgtgagg cctggagata gttgctaagt tgctaggaac atgtggtggg actttcatat 2040
   tctgaaaaat gttctatatt ctcatttttc taaaagaaag aaaaaaggaa acccgattta 2100
   tttctcctga atctttttaa gtttgtgtcg ttccttaagc agaactaagc tcagtatgtg 2160
   accttacccg ctaggtggtt aatttatcca tgctggcaga ggcactcagg tacttggtaa 2220
   gcaaatttct aaaactccaa gttgctgcag cttggcattc ttcttattct agaggtctct 2280
   ctggaaaaga tggagaaaat gaacaggaca tggggctcct ggaaagaaag ggcccgggaa 2340
   gttcaaggaa gaataaagtt gaaattttaa tttgcatttt ttttgtctag ataagaatag 2400
   cgtgaataga tcctctttta ttcgtaaata atcgtgcatc tgtgggttag ccttgtagaa 2460
   gtggaaaaca ttccattttc caatgcattt aaatgtaaag ccaaatctgc atgttgtgaa 2520
   tttaagaaaa cttattatcc taaaggtgcc tttctcttgg catcatcccg cttgtgagaa 2580
   gcctagagga cgctccaggt ggaaggaaat cccctgggtg gttttatctt ttgttaccca 2640
   gtgagcactg gttccccgca aatactgggg aaaagcaaaa atacacaagc aagttaaaat 2700
   taattttgca catctgggag gttataaaag aaagcactaa tagtagtcac tgcccagact 2760
   ttactggcca caaatgccca gctgaagagc atgactgtgg atcactggtt tttccctcct 2820
   gctggaaatg ctggggtggt agcggtcgat taggattttc agtggagaag cacaggacag 2880
   ttctgtaatt tatgggactc cttagccaac ataaagaact gcaggaaata actgcacagc 2940
   caggaggatc cgttggtggg aatttaccgt cattcctgcc cttttattta acatcatcca 3000
   cagagagatg ttatacaaat ggaggaaacc attatacctt ttgatatgga atatattaca 3060
   gagttacagt tcacaaagta gaatgctgag ctgaaaaccc gagtttctgc tgtgactgtc 3120
   atctcactga gcacttcgct tttctttgct tttatttttt ccttctataa aaaggcaata 3180
   atgataattt ataatagttt ccctacccag agatattaga agtatgctac agtgaatgtt 3240
   aaagtacctt gagatcctta aatcaaaggt gctatataca taagtaagac tctactttca 3300
   gaaaaaggta atattatttc ctgcactgat ccctactaat tctatattga tccaaaggca 3360
   actcaatgct aaaaaatgta tagaaaatat aagtctgtgt ctgtgtactg tagagatgta 3420
   tgtgacaagt gtaaacaaaa tgaactgaag cagtaatgaa cagttattag ggggaacatg 3480
   ataaagagat tatattaagc ttatgtttca ccataaaatc ctttttatgg cttactaaaa 3540
   ccgagctcac tgtaaaatca tgatccaact tattgctaat ctttatgata tgcttattcc 3600
   taatctttat ggtatggtgt caaccgttca tttgtatctt attgctcatt ccctggacca 3660
   cagactaggg acagaaaata cttgctttaa taatatatat gctgttgatt tcacaaaaat 3720
   ttattaaaat acagcctggg tacccagtga aagagctgaa atggaaatgg agtatcatgt 3780
   ttcctactca cattttactc agctgtcgg 3809
<210> 81
   <211> 2258
   <212> DNA
   <213> Homo sapiens
<400> 81
   ctcctccagc tcttcctgtc ccgctgttgc aacactgcct cactcttccc ctcccacctt      60
   ctctcccctc ctctctgctt taattttctc agaattctct ggactgaggc tccagttctg     120
   gcctttgggg ttcaagatca ctgggaccag gccgtgatct ctatgcccga gtctcaaccc     180
   tcaactgtca ccccaaggca cttgggacgt cctggacaga ccgagtcccg ggaagcccca     240
   gcactgccgc tgccacactg ccctgagccc aaatggggga gtgagaggcc atagctgtct      300
   ggcatgggcc tctccaccgt gcctgacctg ctgctgccac tggtgctcct ggagctgttg     360
   gtgggaatat acccctcagg ggttattgga ctggtccctc acctagggga cagggagaag    420
   agagatagtg tgtgtcccca aggaaaatat atccaccctc aaaataattc gatttgctgt     480
   accaagtgcc acaaaggaac ctacttgtac aatgactgtc caggcccggg gcaggatacg     540
   gactgcaggg agtgtgagag cggctccttc accgcttcag aaaaccacct cagacactgc     600
   ctcagctgct ccaaatgccg aaaggaaatg ggtcaggtgg agatctcttc ttgcacagtg     660
   gaccgggaca ccgtgtgtgg ctgcaggaag aaccagtacc ggcattattg gagtgaaaac 720
   cttttccagt gcttcaattg cagcctctgc ctcaatggga ccgtgcacct ctcctgccag 780
   gagaaacaga acaccgtgtg cacctgccat gcaggtttct ttctaagaga aaacgagtgt 840
   gtctcctgta gtaactgtaa gaaaagcctg gagtgcacga agttgtgcct accccagatt 900
   gagaatgtta agggcactga ggactcaggc accacagtgc tgttgcccct ggtcattttc 960
   tttggtcttt gccttttatc cctcctcttc attggtttaa tgtatcgcta ccaacggtgg 1020
   aagtccaagc tctactccat tgtttgtggg aaatcgacac ctgaaaaaga gggggagctt 1080
   gaaggaacta ctactaagcc cctggcccca aacccaagct tcagtcccac tccaggcttc 1140
   acccccaccc tgggcttcag tcccgtgccc agttccacct tcacctccag ctccacctat 1200
   acccccggtg actgtcccaa ctttgcggct ccccgcagag aggtggcacc accctatcag 1260
   ggggctgacc ccatccttgc gacagccctc gcctccgacc ccatccccaa cccccttcag 1320
   aagtgggagg acagcgccca caagccacag agcctagaca ctgatgaccc cgcgacgctg 1380
   tacgccgtgg tggagaacgt gcccccgttg cgctggaagg aattcgtgcg gcgcctaggg 1440
   ctgagcgacc acgagatcga tcggctggag ctgcagaacg ggcgctgcct gcgcgaggcg 1500
   caatacagca tgctggcgac ctggaggcgg cgcacgccgc ggcgcgaggc cacgctggag 1560
   ctgctgggac gcgtgctccg cgacatggac ctgctgggct gcctggagga catcgaggag 1620
   gcgctttgcg gccccgccgc cctcccgccc gcgcccagtc ttctcagatg aggctgcgcc 1680
   cctgcgggca gctctaagga ccgtcctgcg agatcgcctt ccaaccccac ttttttctgg 1740
   aaaggagggg tcctgcaggg gcaagcagga gctagcagcc gcctacttgg tgctaacccc 1800
   tcgatgtaca tagcttttct cagctgcctg cgcgccgccg acagtcagcg ctgtgcgcgc 1860
   ggagagaggt gcgccgtggg ctcaagagcc tgagtgggtg gtttgcgagg atgagggacg 1920
   ctatgcctca tgcccgtttt gggtgtcctc accagcaagg ctgctcgggg gcccctggtt 1980
   cgtccctgag cctttttcac agtgcataag cagttttttt tgtttttgtt ttgttttgtt 2040
   ttgtttttaa atcaatcatg ttacactaat agaaacttgg cactcctgtg ccctctgcct 2100
   ggacaagcac atagcaagct gaactgtcct aaggcagggg cgagcacgga acaatggggc 2160
   cttcagctgg agctgtggac ttttgtacat acactaaaat tctgaagtta aagctctgct 2220
   cttggaaaaa aaaaaaaaaa aaaaaaaaaa aaaaaaaa 2258
<210> 82
   <211> 3682
   <212> DNA
   <213> Homo sapiens
<400> 82
   gcgagcgcag cggagcctgg agagaaggcg ctgggctgcg agggcgcgag ggcgcgaggg 60
   cagggggcaa ccggaccccg cccgcaccca tggcgcccgt cgccgtctgg gccgcgctgg 120
   ccgtcggact ggagctctgg gctgcggcgc acgccttgcc cgcccaggtg gcatttacac 180
   cctacgcccc ggagcccggg agcacatgcc ggctcagaga atactatgac cagacagctc 240
   agatgtgctg cagcaaatgc tcgccgggcc aacatgcaaa agtcttctgt accaagacct 300
   cggacaccgt gtgtgactcc tgtgaggaca gcacatacac ccagctctgg aactgggttc 360
   ccgagtgctt gagctgtggc tcccgctgta gctctgacca ggtggaaact caagcctgca 420
   ctcgggaaca gaaccgcatc tgcacctgca ggcccggctg gtactgcgcg ctgagcaagc 480
   aggaggggtg ccggctgtgc gcgccgctgc gcaagtgccg cccgggcttc ggcgtggcca 540
   gaccaggaac tgaaacatca gacgtggtgt gcaagccctg tgccccgggg acgttctcca 600
   acacgacttc atccacggat atttgcaggc cccaccagat ctgtaacgtg gtggccatcc 660
   ctgggaatgc aagcatggat gcagtctgca cgtccacgtc ccccacccgg agtatggccc 720
   caggggcagt acacttaccc cagccagtgt ccacacgatc ccaacacacg cagccaactc 780
   cagaacccag cactgctcca agcacctcct tcctgctccc aatgggcccc agccccccag 840
   ctgaagggag cactggcgac ttcgctcttc cagttggact gattgtgggt gtgacagcct 900
   tgggtctact aataatagga gtggtgaact gtgtcatcat gacccaggtg aaaaagaagc 960
   ccttgtgcct gcagagagaa gccaaggtgc ctcacttgcc tgccgataag gcccggggta 1020
   cacagggccc cgagcagcag cacctgctga tcacagcgcc gagctccagc agcagctccc 1080
   tggagagctc ggccagtgcg ttggacagaa gggcgcccac tcggaaccag ccacaggcac 1140
   caggcgtgga ggccagtggg gccggggagg cccgggccag caccgggagc tcagattctt 1200
   cccctggtgg ccatgggacc caggtcaatg tcacctgcat cgtgaacgtc tgtagcagct 1260
   ctgaccacag ctcacagtgc tcctcccaag ccagctccac aatgggagac acagattcca 1320
   gcccctcgga gtccccgaag gacgagcagg tccccttctc caaggaggaa tgtgcctttc 1380
   ggtcacagct ggagacgcca gagaccctgc tggggagcac cgaagagaag cccctgcccc 1440
   ttggagtgcc tgatgctggg atgaagccca gttaaccagg ccggtgtggg ctgtgtcgta 1500
   gccaaggtgg gctgagccct ggcaggatga ccctgcgaag gggccctggt ccttccaggc 1560
   ccccaccact aggactctga ggctctttct gggccaagtt cctctagtgc cctccacagc 1620
   cgcagcctcc ctctgacctg caggccaaga gcagaggcag cgagttgtgg aaagcctctg 1680
   ctgccatggc gtgtccctct cggaaggctg gctgggcatg gacgttcggg gcatgctggg 1740
   gcaagtccct gactctctgt gacctgcccc gcccagctgc acctgccagc ctggcttctg 1800
   gagcccttgg gttttttgtt tgtttgtttg tttgtttgtt tgtttctccc cctgggctct 1860
   gccccagctc tggcttccag aaaaccccag catccttttc tgcagagggg ctttctggag 1920
   aggagggatg ctgcctgagt cacccatgaa gacaggacag tgcttcagcc tgaggctgag 1980
   actgcgggat ggtcctgggg ctctgtgcag ggaggaggtg gcagccctgt agggaacggg 2040
   gtccttcaag ttagctcagg aggcttggaa agcatcacct caggccaggt gcagtggctc 2100
   acgcctatga tcccagcact ttgggaggct gaggcgggtg gatcacctga ggttaggagt 2160
   tcgagaccag cctggccaac atggtaaaac cccatctcta ctaaaaatac agaaattagc 2220
   cgggcgtggt ggcgggcacc tatagtccca gctactcaga agcctgaggc tgggaaatcg 2280
   tttgaacccg ggaagcggag gttgcaggga gccgagatca cgccactgca ctccagcctg 2340
   ggcgacagag cgagagtctg tctcaaaaga aaaaaaaaag caccgcctcc aaatgccaac 2400
   ttgtcctttt gtaccatggt gtgaaagtca gatgcccaga gggcccaggc aggccaccat 2460
   attcagtgct gtggcctggg caagataacg cacttctaac tagaaatctg ccaatttttt 2520
   aaaaaagtaa gtaccactca ggccaacaag ccaacgacaa agccaaactc tgccagccac 2580
   atccaacccc ccacctgcca tttgcaccct ccgccttcac tccggtgtgc ctgcagcccc 2640
   gcgcctcctt ccttgctgtc ctaggccaca ccatctcctt tcagggaatt tcaggaacta 2700
   gagatgactg agtcctcgta gccatctctc tactcctacc tcagcctaga ccctcctcct 2760
   cccccagagg ggtgggttcc tcttccccac tccccacctt caattcctgg gccccaaacg 2820
   ggctgccctg ccactttggt acatggccag tgtgatccca agtgccagtc ttgtgtctgc 2880
   gtctgtgttg cgtgtcgtgg gtgtgtgtag ccaaggtcgg taagttgaat ggcctgcctt 2940
   gaagccactg aagctgggat tcctccccat tagagtcagc cttccccctc ccagggccag 3000
   ggccctgcag aggggaaacc agtgtagcct tgcccggatt ctgggaggaa gcaggttgag 3060
   gggctcctgg aaaggctcag tctcaggagc atggggataa aggagaaggc atgaaattgt 3120
   ctagcagagc aggggcaggg tgataaattg ttgataaatt ccactggact tgagcttggc 3180
   agctgaacta ttggagggtg ggagagccca gccattacca tggagacaag aagggttttc 3240
   caccctggaa tcaagatgtc agactggctg gctgcagtga cgtgcacctg tactcaggag 3300
   gctgagggga ggatcactgg agcccaggag tttgaggctg cagcgagcta tgatcgcgcc 3360
   actacactcc agcctgagca acagagtgag accctgtctc ttaaagaaaa aaaaagtcag 3420
   actgctggga ctggccaggt ttctgcccac attggaccca catgaggaca tgatggagcg 3480
   cacctgcccc ctggtggaca gtcctgggag aacctcaggc ttccttggca tcacagggca 3540
   gagccgggaa gcgatgaatt tggagactct gtggggcctt ggttcccttg tgtgtgtgtg 3600
   ttgatcccaa gacaatgaaa gtttgcactg tatgctggac ggcattcctg cttatcaata 3660
   aacctgtttg ttttaaaaaa aa 3682
<210> 83
   <211> 2894
   <212> DNA
   <213> Homo sapiens
<400> 83
   cgagactcca tctcaaaaac aaaacaaata aacgaacaaa aaaacccaca acgtattatt 60
   ttcttgttta cgaggtttct tgtctctctg gctccaccag aagaggagca gggacccttc 120
   ttgctgttgt tcattgctgc atcccccaca ccgagagcag agcctggcat gggcagaaag 180
   tcctcagtcg atatttggtg gccccaagcg aatgaagcat ccaagaaggg aaagctgggg 240
   gctccccact gcacttgcca cctgagtcac attttcagaa gcctctggaa agtcgtgcac 300
   agcccaggag tgttgagcaa tttcggtttc ctctgaggtt gaaggaccca ggcgtgtcag 360
   ccctgctcca gacaccttgg gcatggagga gagtgtcgta cggccctcag tgtttgtggt 420
   ggatggacag accgacatcc cattcacgag gctgggacga agccaccgga gacagtcgtg 480
   cagtgtggcc cgggtgggtc tgggtctctt gctgttgctg atgggggccg ggctggccgt 540
   ccaaggctgg ttcctcctgc agctgcactg gcgtctagga gagatggtca cccgcctgcc 600
   tgacggacct gcaggctcct gggagcagct gatacaagag cgaaggtctc acgaggtcaa 660
   cccagcagcg catctcacag gggccaactc cagcttgacc ggcagcgggg ggccgctgtt 720
   atgggagact cagctgggcc tggccttcct gaggggcctc agctaccacg atggggccct 780
   tgtggtcacc aaagctggct actactacat ctactccaag gtgcagctgg gcggtgtggg 840
   ctgcccgctg ggcctggcca gcaccatcac ccacggcctc tacaagcgca caccccgcta 900
   ccccgaggag ctggagctgt tggtcagcca gcagtcaccc tgcggacggg ccaccagcag 960
   ctcccgggtc tggtgggaca gcagcttcct gggtggtgtg gtacacctgg aggctgggga 1020
   gaaggtggtc gtccgtgtgc tggatgaacg cctggttcga ctgcgtgatg gtacccggtc 1080
   ttacttcggg gctttcatgg tgtgaaggaa ggagcgtggt gcattggaca tgggtctgac 1140
   acgtggagaa ctcagagggt gcctcagggg aaagaaaact cacgaagcag aggctgggcg 1200
   tggtggctct cgcctgtaat cccagcactt tgggaggcca aggcaggcgg atcacctgag 1260
   gtcaggagtt cgagaccagc ctggctaaca tggcaaaacc ccatctctac taaaaataca 1320
   aaaattagcc ggacgtggtg gtgcctgcct gtaatccagc tactcaggag gctgaggcag 1380
   gataattttg cttaaacccg ggaggcggag gttgcagtga gccgagatca caccactgca 1440
   ctccaacctg ggaaacgcag tgagactgtg cctcaaaaaa aagaaaggaa gaaaaaagaa 1500
   aactcaggaa acagatcttg ggggacactc cagggaaccc aaaactcaaa ggcggagagc 1560
   tcagtgggca ccaccaaggc gagatgaagc cccagcaggc accttcagaa gacccacgta 1620
   gactgcagac cctgccacgg acaatactaa ggacaaaaac ccagagactt ggggtctgtg 1680
   ggcccccaaa catggggtaa agttgatttg cctgatattc aggaagaagg ggtgaggggt 1740
   gggtatttat gcttttgatt cagaagaaag tggggcttgg gattccaggg acttggctgg 1800
   gggtgggaaa cttcatccac ttccctactc tcatcatgag tacggacagg gtgggcggga 1860
   gactgatcat cgggactcat catgaagagc ccagccccac cccacatact cagatcccac 1920
   ccacagactg gtggccacac ctcagcctgg tcacaaagag ttacactcag atacatgagc 1980
   acggcagcgt gctcataact gtttaacaac cagctgtcct gggaggggga cagctttgta 2040
   atgtttgcca atttccatgg tgtaaatgct accaccatgg ctgatttcat cactgccaag 2100
   catagacatc cctaatagga caccacggat ctgtccccgg catccggccc agggcctggc 2160
   acaaagcatg ctctagggaa atgcttgctg attgaaagga aggaagaatg actctacagt 2220
   cacacctatg gcatcccaca aaatctgtca catggctgca taatctcagc cactctttca 2280
   caactataga ctcatacacg cgaagtgcca gattcatgca caaccacaca atcacatgga 2340
   agtcacagac ggcatcacag acagtcacag cactgtgtgt atgttataac acaagcacac 2400
   aaaactcaga cagcatccca gctacacagc cactcccaga ggtgtcaccg tcacacttgg 2460
   taattaatac tcattacatt agacacagac agaccaagtt atagtcagac ctggttacac 2520
   acatacacac acacaatatc accatgacaa atacacatta cacacacaca acatcacaat 2580
   gacaaacaca cattacacac acaacatcac gatgacaaac acacattaca cacacaacat 2640
   cacgatgaca aacacacatt acacacacat cacaatgaca aacacaacat tacacacaca 2700
   caacatcaca atgacacaca catcacacac acatcacaat gacaaacaca caacattaca 2760
   cacatataca cacagcctga gggccctccc cagcccagac taacacatct cggggtgagg 2820
   accagacctt gttcataacc ctgggcctct taaccactga tctttgaaat aaatggcaaa 2880
   tagttgtacc tgga 2894
<210> 84
   <211> 1680
   <212> DNA
   <213> Homo sapiens
<400> 84
   aaaaagcggc gcgctgtgtc ttcccgcagt ctctcgtcat ggaatacgcc tctgacgctt 60
   cactggaccc cgaagccccg tggcctcccg cgccccgcgc tcgcgcctgc cgcgtactgc 120
   cttgggccct ggtcgcgggg ctgctgctgc tgctgctgct cgctgccgcc tgcgccgtct 180
   tcctcgcctg cccctgggcc gtgtccgggg ctcgcgcctc gcccggctcc gcggccagcc 240
   cgagactccg cgagggtccc gagctttcgc ccgacgatcc cgccggcctc ttggacctgc 300
   ggcagggcat gtttgcgcag ctggtggccc aaaatgttct gctgatcgat gggcccctga 360
   gctggtacag tgacccaggc ctggcaggcg tgtccctgac ggggggcctg agctacaaag 420
   aggacacgaa ggagctggtg gtggccaagg ctggagtcta ctatgtcttc tttcaactag 480
   agctgcggcg cgtggtggcc ggcgagggct caggctccgt ttcacttgcg ctgcacctgc 540
   agccactgcg ctctgctgct ggggccgccg ccctggcttt gaccgtggac ctgccacccg 600
   cctcctccga ggctcggaac tcggccttcg gtttccaggg ccgcttgctg cacctgagtg 660
   ccggccagcg cctgggcgtc catcttcaca ctgaggccag ggcacgccat gcctggcagc 720
   ttacccaggg cgccacagtc ttgggactct tccgggtgac ccccgaaatc ccagccggac 780
   tcccttcacc gaggtcggaa taacgtccag cctgggtgca gcccacctgg acagagtccg 840
   aatcctactc catccttcat ggagacccct ggtgctgggt ccctgctgct ttctctacct 900
   caaggggctt ggcaggggtc cctgctgctg acctcccctt gaggaccctc ctcacccact 960
   ccttccccaa gttggacctt gatatttatt ctgagcctga gctcagataa tatattatat 1020
   atattatata tatatatata tttctattta aagaggatcc tgagtttgtg aatggacttt 1080
   tttagaggag ttgttttggg gggggggggg tcttcgacat tgccgaggct ggtcttgaac 1140
   tcctggactt agacgatcct cctgcctcag cctcccaagc aactgggatt catcctttct 1200
   attaattcat tgtacttatt tgcttatttg tgtgtattga gcatctgtaa tgtgccagca 1260
   ttgtgcccag gctagggggc tatagaaaca tctagaaata gactgaaaga aaatctgagt 1320
   tatggtaata cgtgaggaat ttaaagactc atccccagcc tccacctcct gtgtgatact 1380
   tgggggctag cttttttctt tctttctttt ttttgagatg gtcttgttct gtcaaccagg 1440
   ctagaatgca gcggtgcaat catgagtcaa tgcagcctcc agcctcgacc tcccgaggct 1500
   caggtgatcc tcccatctca gcctctcgag tagctgggac cacagttgtg tgccaccaca 1560
   cttggctaac tttttaattt ttttgcggag acggtattgc tatgttgcca aggttgttta 1620
   catgccagta caatttataa taaacactca tttttcctcc ctctgaaaaa aaaaaaaaaa 1680
<210> 85
   <211> 2029
   <212> DNA
   <213> Homo sapiens
<400> 85
   ggcggcgggc ggcgggcggc ggggaccggg tgcggtggtg gctgcggcgg cggcggcggg      60
   agcagcatgg attggggcac tgagctgtgg gatcagttcg aggtgctcga gcgccacacg     120
   cagtgggggc tggacctgtt ggacagatat gtaaagttcg tgaaagaacg caccgaagtg     180
   gaacaggctt acgccaaaca actgcggagc ctggtgaaaa aatatctgcc caagagacct     240
   gccaaggatg atcctgagtc caaattcagc cagcaacagt ccttcgtaca gattctccag     300
   gaggtgaatg actttgcagg ccagcgggag ctggtggctg agaacctcag tgtccgtgta     360
   tgtcttgagc tgaccaagta ctcacaagag atgaaacagg agaggaagat gcacttccaa     420
   gaagggcggc gggcccagca gcagctggaa aatggcttta aacagctgga gaatagtaag     480
   cgtaaatttg agcgggactg ccgggaggca gagaaggcag cccagactgc tgaacggcta     540
   gaccaggata tcaacgccac caaggctgat gtggagaagg ccaagcagca agcccacctt      600
   cggagtcaca tggccgaaga aagcaaaaac gaatatgcgg ctcaactgca gcgcttcaac      660
   cgagaccaag cccacttcta tttttcacag atgccccaga tattcgataa gctccaagac      720
   atggatgaac gcagggccac ccgcctgggt gccgggtatg ggctcctgtc ggaggccgag      780
   ctggaggtgg tgcccataat agccaagtgc ttggagggca tgaaggtggc tgcaaatgct      840
   gtggatccca agaacgactc ccacgtcctt atagagctgc acaagtcagg ttttgcccgc      900
   ccgggcgacg tggaattcga ggacttcagc cagcccatga accgtgcacc ctccgacagc      960
   agtctgggca ccccctcgga tggacggcct gaactccgag gcccgggtcg cagccgcacc     1020
   aagcgctggc cttttggcaa gaagaacaag acagtggtga ccgaggattt tagccacttg     1080
   cccccagagc agcagcgaaa acggcttcaa cagcagttgg aagaacgcag tcgtgaactt     1140
   cagaaggagg ttgaccagag ggaagcccta aagaaaatga aggatgtcta tgagaagaca     1200
   cctcagatgg gggaccccgc cagcttggag ccccagatcg ctgaaaccct gagcaacatt     1260
   gaacggctga aattggaagt gcagaagtat gaggcgtggc tggcagaagc tgaaagtcga     1320
   gtccttagca accggggaga cagcctgagc cggcacgccc ggcctcccga cccccccgct     1380
   agcgccccgc cagacagcag cagcaacagc gcatcacagg acaccaagga gagctctgaa     1440
   gagcctccct cagaagagag ccaggacacc cccatttaca cggagtttga tgaggatttc     1500
   gaggaggaac ccacatcccc cataggtcac tgtgtggcca tctaccactt tgaagggtcc     1560
   agcgagggca ctatctctat ggccgagggt gaagacctca gtcttatgga agaagacaaa     1620
   ggggacggct ggacccgggt caggcggaaa gagggaggcg agggctacgt gcccacctcc     1680
   tacctccgag tcacgctcaa ttgaaccctg ccagagacgg gaagaggggg gctgtcggct     1740
   gctgcttctg ggccacgggg agccccagga cctatgcact ttatttctga ccccgtggct     1800
   tcggctgaga cctgtgtaac ctgctgcccc ctccaccccc aacccagtcc tacctgtcac     1860
   accggacgga cccgctgtgc cttctaccat cgttccacca ttgatgtaca tactcatgtt     1920
   ttacatcttt tctttctgcc gctcggctcc ggccattttg ttttatacaa aaatgggaaa     1980
   aaaaaaaaag aaattatata aagttcctag aaaaaaaaaa aaaaaaaaa                 2029
<210> 86
   <211> 1762
   <212> DNA
   <213> Homo sapiens
<400> 86
   gacgtcatcg gaggcgtggt cgtccccaaa attagggagg aagaggaaaa aaaaaagcca      60
   gaaaaagttt tcttttctgg agtcccaaac gaggtgcggg acggaagagg gggtgaaggc     120
   cagaggctcg gggcttcaag accgctgtct ggagtccccc tttccaggcc atgtcggggc     180
   ccacctggct gcccccgaag cagccggagc ccgccagagc ccctcagggg agggcgatcc     240
   cccgcggcac cccggggcca ccaccggccc acggagcagc actccagccc caccccaggg     300
   tcaatttttg cccccttcca tctgagcagt gttaccaggc cccaggggga ccggaggatc     360
   gggggccggc gtgggtgggg tcccatggag tactccagca cacgcagggg ctccctgcag     420
   acaggggggg ccttcgccct ggaagcctgg acgccgagat agacttgctg agcagcacgc     480
   tggccgagct gaatgggggt cggggtcatg cgtcacggcg accagaccga caggcatatg     540
   agcccccgcc acctcctgcc taccgcacgg gctccctgaa gccaaatcca gcctcgccgc     600
   tcccagcgtc tccctatggg ggccccactc cagcctctta cactaccgcc agcaccccgg     660
   ctggcccagc cttccccgtg caagtgaagg tggcacagcc agtgaggggc tgcggcccac     720
   ccaggcgggg agcctctcag gcctctgggc ccctcccggg cccccacttt cctctcccag     780
   gccgaggtga agtctggggg cctggctata ggagccagag agagccaggg ccaggggcca     840
   aagaggaagc tgctggggtc tctggccctg caggaagagg aagaggaggc gagcacgggc     900
   cccaggtgcc cctgagccag cctccagagg atgagctgga taggctgacg aagaagctgg     960
   ttcacgacat gaaccacccg cccagcgggg agtactttgg ccagtgtggt ggctgcggag    1020
   aagatgtggt tggggatggg gctggggttg tggcccttga tcgcgtcttt cacgtgggct    1080
   gctttgtatg ttctacatgc cgggcccagc ttcgcggcca gcatttctac gccgtggaga    1140
   ggagggcata ttgcgagggc tgctacgtgg ccaccctgga gaaatgtgcc acgtgctccc    1200
   agcccatcct ggaccggatc ctgcgggcta tggggaaggc ctaccaccct ggctgcttca    1260
   cctgcgtggt gtgtcaccgc ggcctcgacg gcatcccctt cacagtggat gctacgagcc    1320
   agatccactg cattgaggac tttcacagga agtttgcccc aagatgctca gtgtgcggtg    1380
   gggccataat gcctgagcca ggtcaggagg agactgtgag aattgttgct ctggatcgaa    1440
   gttttcacat tggctgttac aagtgcgagg agtgtgggct gctgctctcc tctgagggcg    1500
   agtgtcaggg ctgctacccg ctggatgggc acatcttgtg caaggcctgc agcgcctggc    1560
   gcatccagga gctctcagcc accgtcacca ctgactgctg agtcttccta gaagtacctg    1620
   ctgggttctc agttccagtt cccatccttt gattgatcac tctccctgac atccacctgt    1680
   atgactttgt caccaaatgc tgtcttctct ttctccaatc aagaaataat aatccctcga    1740
   gtttacaaaa caaaaaaaaa aa                                             1762
<210> 87
   <211> 2783
   <212> DNA
   <213> Homo sapiens
<400> 87
   gattgcgagc caggaggagg aagccggcgg tggccccgtc agcagccggc tgctgagagg 60
   ccggtaggcg gcggcggtcc cgaggggcgg cggccgcgct gctccctgag aacgggtccc      120
   gcagctgggc aggcgggcgg cctgagggcg cggagccatg aagctgtaca gcctcagcgt       180
   cctctacaaa ggcgaggcca aggtggtgct gctcaaagcc gcatacgatg tgtcttcctt      240
   cagctttttc cagagatcca gcgttcagga attcatgacc ttcacgagtc aactgattgt      300
   ggagcgctca tcgaaaggca ctagagcttc tgtcaaagaa caagactatc tgtgccacgt      360
   ctacgtccgg aatgatagtc ttgcaggtgt ggtcattgct gacaatgaat acccatcccg      420
   ggtggccttt accttgctgg agaaggtact agatgaattc tccaagcaag tcgacaggat      480
   agactggcca gtaggatccc ctgctacaat ccattaccca gccctggatg gtcacctcag      540
   tagataccag aacccacgag aagctgatcc catgactaaa gtgcaggccg aactagatga      600
   gaccaaaatc attctgcaca acaccatgga gtctctgtta gagcgaggtg agaagctaga      660
   tgacttggtg tccaaatccg aggtgctggg aacacagtct aaagccttct ataaaactgc      720
   ccggaaacaa aactcatgct gtgccatcat gtgatgcagc ctgccagagg cccaatgctg      780
   gaatggcacc atcattcaca tcagaactgc agcccctgga aaagaagaga cagccataga      840
   cgaggagcca gagtgggggc agactggcca tttttatttt gaagttcctg cgagaaatgg      900
   atggtggaag ggtggcgaat gttcaaattc atatgtgtgg tagtgattct tggaaagaat      960
   ttgaggtccc caaaggtgta tttttgggca aatgaaacca taaactccga ctggcttctg     1020
   tagatgccaa agggctcttt ttcagctaac cctgggaagg ctctgtggga gggaggtcgg     1080
   agccagctgt ttctcgatct ttggtatatc tttggatctt atttgtacat taatgatatt     1140
   aacactccag tggggggtgg ggagtccctg atgctagggc tggggtgggt ggagtttgaa     1200
   gactcttggg aaagcctctc ctggggccac tgttgggggt gggagtgagc ccaccacaga     1260
   ggccacaggc aggcccccac ttcaggccca aggcctgggg cggggggaac agtcactggg     1320
   tctcagattc tgagactgtt gtttagctta cctttctgct aggattggct tcccgcagag     1380
   ggcagggccc atcctaagca gcttccaagt cccacaaagg tggcttgtgg gaggatttgg     1440
   aaggagctgc attgtgggcg gggagtgtgt gggttgggtt cgtaccagca agtagactag     1500
   gaactgagcc caggaaaggg ggatgttttc ctggtgtttg gatggtcagc tgggagtgtc     1560
   catcatcagg ggaagatcaa acacaggtgc actcagctgc ccagggcctc tgggacactt     1620
   gccttgactt gcaacttgcc ttgaacatca cgatcaaagc agcaggtgct gtggtctctc     1680
   aaaattgatt tttatttgac tctgtggctc taagactgcc ttgaaccgcc tgaggcctat     1740
   gcatctgaac aagtgggtct ctcccttgag caccaggagt gggtgccagc cggccccgag     1800
   gattcccagc accccaccta tggtcttgcc agcataggct tgctagttcc ttcttggtca     1860
   gaggtagctg cagagggggg aggccaaggg tttggtctaa gctgtgccct gccacctggc     1920
   aggaggccca ctcactgccc aagtcatggc aacaggctgg agcagcccag gagatgggcc      1980
   taaaatgttc tggatccctt gggtcctagt gttatgttcc agtctgccca cctgtgctca      2040
   ggatgcagcc ctgggatcca gcacccatgg aagcttctgc tgggatggtg tcacctatgg      2100
   gttttgaacc agtgtggtat ggtccttggg agctctgctc tgagcttgcc acactgctga      2160
   gagcacccac tgtcctgacc agagtctcag tggtcctgac ccccaatgtg ggcaggggct      2220
   gggcaggagg gtggggtctg ctgtgggttc agaggactcc acctcctggc tggtttacct      2280
   gctgctgccc attttctctg ggtactgctg gccagaggac tttagcctac ccctgaagag      2340
   cctgtccatg tcattttcct actgccatag ataccctaag cccagggccc cttgaggccc      2400
   agactcagcc tgcccactgg tgccggagac ggagtggagt gggcctggat ccgagggatg      2460
   ctacctctcc ctttcccact tgaggaccct ggggagagat gggggcgggg aaaatggagg      2520
   tatgaatttg ggtaagaggg aagtgagatc tccgcttgca ggtcagcccc tgccttgcag      2580
   ggcgggctgg cttgactcag gccctgtgag atagagggcc cagcccagcc ccacccacag      2640
   atcccctgct cctgttgtgt tctgttgtaa atcatttggc gagactgtat tttagtaact      2700
   gctgcctaac ttccctgtgt tctatttgag aggcgcctgt ctggataaag ttgtcttgaa      2760
   atttcaaaaa aaaaaaaaaa aaa                                              2783
<210> 88
   <211> 3341
   <212> DNA
   <213> Homo sapiens
<400> 88
   ccgggccccg gcggctgcgc cgagtccccg cccctccctg ctccgtaggg gtaggagggg      60
   gccggcggag tttccctccc cgcccagcgg ccctgggcgg gcttttcggc tgcttctcat     120
   aagcaggtgg tttcgtttct ccggcacagg taggtttctc tggcaccgat tcggggcctg     180
   cccggacttc gccgcacgct gcagaacctc gcccagcgcc caccatgccc cggcagctca     240
   gcgcggcggc cgcgctcttc gcgtccctgg ccgtaatttt gcacgatggc agtcaaatga     300
   gagcaaaagc atttccagaa accagagatt attctcaacc tactgcagca gcaacagtac     360
   aggacataaa aaaacctgtc cagcaaccag ctaagcaagc acctcaccaa actttagcag     420
   caagattcat ggatggtcat atcacctttc aaacagcggc cacagtaaaa attccaacaa     480
   ctaccccagc gactacaaaa aacactgcaa ccaccagccc aattacctac accctggtca     540
   caacccaggc cacacccaac aactcacaca cagctcctcc agttactgaa gttacagtcg     600
   gccctagctt agccccttat tcactgccac ccaccatcac cccaccagct catacaactg     660
   gaaccagttc atcaaccgtc agccacacaa ctgggaacac cactcaaccc agtaaccaga     720
   ccacccttcc agcaacttta tcgatagcac tgcacaaaag cacaaccggt cagaagcctg     780
   ttcaacccac ccatgcccca ggaacaacgg cagctgccca caataccacc cgcacagctg 840
   cacctgcctc cacggttcct gggcccaccc ttgcacctca gccatcgtca gtcaagactg 900
   gaatttatca ggttctaaac ggaagcagac tctgtataaa agcagagatg gggatacagc 960
   tgattgttca agacaaggag tcggtttttt cacctcggag atacttcaac atcgacccca 1020
   acgcaacgca agcctctggg aactgtggca cccgaaaatc caaccttctg ttgaattttc 1080
   agggcggatt tgtgaatctc acatttacca aggatgaaga atcatattat atcagtgaag 1140
   tgggagccta tttgaccgtc tcagatccag agacaattta ccaaggaatc aaacatgcgg 1200
   tggtgatgtt ccagacagca gtcgggcatt ccttcaagtg cgtgagtgaa cagagcctcc 1260
   agttgtcagc ccacctgcag gtgaaaacaa ccgatgtcca acttcaagcc tttgattttg 1320
   aagatgacca ctttggaaat gtggatgagt gctcgtctga ctacacaatt gtgcttcctg 1380
   tgattggggc catcgtggtt ggtctctgcc ttatgggtat gggtgtctat aaaatccgcc 1440
   taaggtgtca atcatctgga taccagagaa tctaattgtt gcccgggggg aatgaaaata 1500
   atggaattta gagaactctt tcatcccttc caggatggat gttgggaaat tccctcagag 1560
   tgtgggtcct tcaaacaatg taaaccacca tcttctattc aaatgaagtg agtcatgtgt 1620
   gatttaagtt caggcagcac atcaatttct aaatactttt tgtttatttt atgaaagata 1680
   tagtgagctg tttattttct agtttccttt agaatatttt agccactcaa agtcaacatt 1740
   tgagatatgt tgaattaaca taatatatgt aaagtagaat aagccttcaa attataaacc     1800
   aagggtcaat tgtaactaat actactgtgt gtgcattgaa gattttattt tacccttgat     1860
   cttaacaaag cctttgcttt gttatcaaat ggactttcag tgcttttact atctgtgttt 1920
   tatggtttca tgtaacatac atattcctgg tgtagcactt aactcctttt ccactttaaa 1980
   tttgtttttg ttttttgaga cggagtttca ctcttgtcac ccaggctgga gtacagtggc 2040
   acgatctcgg cttatggcaa cctccgcctc ccgggttcaa gtgattctcc tgcttcagct 2100
   tcccgagtag ctgggattac aggcacacac taccacgcct ggctaatttt tgtattttta 2160
   ttatagacgg ggtttcacca tgttggccag actggtcttg aactcttgac ctcaggtgat 2220
   ccacccacct cagcctccca aagtgctggg attacaggca tgagccattg cgcccggcct 2280
   taaatgtttt ttttaatcat caaaaagaac aacatatctc aggttgtcta agtgttttta 2340
   tgtaaaacca acaaaaagaa caaatcagct tatatttttt atcttgatga ctcctgctcc 2400
   agaatcgcta gactaagaat taggtggcta cagatggtag aactaaacaa taagcaagag 2460
   acaataataa tggcccttaa ttattaacaa agtgccagag tctaggctaa gcactttatc 2520
   tatatctcat ttcattctca caacttatag gtgaatgagt aaactgagac ttaagggaac 2580
   tgaatcactt aaatgtcacc tggctaactg atggcagagc cagagcttga attcatgttg 2640
   gtctgacatc aaggtctttg gtcttctccc tacaccaagt tacctacaag aacaatgaca 2700
   ccacactctg cctgaaggct cacacctcat accagcatac gctcacctta cagggaaatg      2760
   ggtttatcca ggatcatgag acattagggt agatgaaagg agagctttgc agataacaaa      2820
   atagcctatc cttaataaat cctccactct ctggaaggag actgaggggc tttgtaaaac      2880
   attagtcagt tgctcatttt tatgggattg cttagctggg ctgtaaagat gaaggcatca      2940
   aataaactca aagtattttt aaattttttt gataatagag aaacttcgct aaccaactgt      3000
   tctttcttga gtgtatagcc ccatcttgtg gtaacttgct gcttctgcac ttcatatcca      3060
   tatttcctat tgttcacttt attctgtaga gcagcctgcc aagaatttta tttctgctgt      3120
   tttttttgct gctaaagaaa ggaactaagt caggatgtta acagaaaagt ccacataacc      3180
   ctagaattct tagtcaagga ataattcaag tcagcctaga gaccatgttg actttcctca      3240
   tgtgtttcct tatgactcag taagttggca aggtcctgac tttagtctta ataaaacatt      3300
   gaattgtagt aaaggttttt gtaataaaaa cttactttgg a                          3341
<210> 89
   <211> 3524
   <212> DNA
   <213> Homo sapiens
<400> 89
   caggaagagg tatttcttgg ggatgctacc aaggcagaga ctgtgaagaa ggaagaacgt      60
   tgcttgggca aaaggagcat attctcagga gacggggccc ctgcctgcca caccaagcat     120
   taggccacca ggaagacccc catctgcaag caagcctagc cttccaggga gaaagaggcc     180
   cctgcagctc cttcatcatg aactggcaca tgatcatctc tgggcttatt gtggtagtgc     240
   ttaaagttgt tggaatgacc ttatttctac tttatttccc acagattttt aacaaaagta     300
   acgatggttt caccaccacc aggagctatg gaacagtctc acagattttt gggagcagtt     360
   ccccaagtcc caacggcttc attaccacaa ggagctatgg aacagtctgc cccaaagact     420
   gggaatttta tcaagcaaga tgtttttctt tatccacttc tgaatcatct tggaatgaaa     480
   gcagggactt ttgcaaagga aaaggatcca cattggcaat tgtcaacacg ccagagaaac     540
   tgaagtttct tcaggacata actgatgctg agaagtattt tattggctta atttaccatc     600
   gtgaagagaa aaggtggcgt tggatcaaca actctgtgtt caatggcaat gttaccaatc     660
   agaatcagaa tttcaactgt gcgaccattg gcctaacaaa gacatttgat gctgcatcat     720
   gtgacatcag ctaccgcagg atctgtgaga agaatgccaa atgatcacag ttccctgtga     780
   caagaactat acttgcaact ctttttgaat ccatacaggt cgtctggcca atgattcttt     840
   tacttaccta tctgtctacc agtagcggtc cttgcccatt tgggaaactg agcttctttc     900
   ttctgcactg ggggactgga tgctagccat ctccaggaga caggatcagt tttacggaaa     960
   caactcagtt agtatagaga tgaggtccgc ttctgtagta ctgagcattt ctgactgatc    1020
   aaaaaggcct agtctgttga cagggtttgt tttattttag cctcagagta taccatacta 1080
   ctagggagta actgtagagt gagaaattat aaacattatt tagggattac catggtggaa 1140
   gagggataaa cataggtcct gtgacttcgt ctctgttctc aagggaaccc cattcacatg 1200
   cccctcctaa ctccacaagc gagggtagca gaggctctcc tcagtctgaa ctaaggcttg 1260
   gccttgggga gggctcctag tgctgagctt ggagcagcac ggacagcagc attgtttatg 1320
   ggaatggaga gaggtctggg caggatagga accttcttgg agaccccttt gaagaaaacc 1380
   aggcagccaa gggagccaaa cacactagat ttctgttctt cagcaaagcc ctgaagagac 1440
   acttaagcta aaaattccct tgtcatattt ctgaaactcc attataacat atgtaactcc 1500
   tttgtaacca aaatttaggt aagcaggctt cctttgctct gaaggttttg agtacctggc 1560
   tgtatttgtt gagtattttt aaaattttgg atagtctctt aggcaacaat aatcacaata 1620
   tattcatccc ttcagttctg gagaaagcct gataccaggc acagcctact gaccccaagg 1680
   agcctggcac tgattggcat cacattgatc tagaactggt ccagccgacg aagagtagga 1740
   aaagagaagg gctgctcagg gaaacattgg ctgggggcac ggaataagca catagtaaaa 1800
   agggaacatc agggtcaaat ggaaatcacc tgagacagga aacagggagt tcatttggcc 1860
   acactggaag aaaggcaaga aagaggaaga caagtcttgg agtaccctgg ctgttctcca 1920
   cactcacaag acatcagcta tatactctgc ttggtgcata agaaagagaa aagagatgcc 1980
   ttttgtgttt tgagtaagaa taattaaacc ataaggaaga ccatgtataa aactgatgga 2040
   aataatagtc accaaagtac agcacatacc attttgtgtc taataacaat gtagcacagt 2100
   aatgactgta catgtcattg tatgtatacc aaacaagatt gttgtaaatc atatttttta 2160
   ttacaacact aagttctgct tctgcattcc taggtttcat catttttggc tccttagcat 2220
   ggccacttac aattttttaa catgagataa cacatcaggt gtcagaactt gcttgaaggg 2280
   aattaccaga agtaatttgt gtttgagatg gggtggaaat tggaattata ttagtagccg 2340
   gtggagatac aagttctctg actgtgttgg gaaaggataa gtgctaccgt tgagaaggga 2400
   agaaaggctg agtctaggtg gagaaaaata tcaacagaac tctagccaaa ggcaagcccc 2460
   agaactcaga caacagaaag gaaatcctaa tccttctgtt ttgagaagag agaactgtag 2520
   ttgcttcact tcctatttca tgacagaata actgcaaact tttaagatca ggaaatgtag 2580
   acatctagtg atttctttag tagacagttt aatttccccc aagattagga gacacttctg 2640
   tgcaggttct aaaaggagcc caatggcctg gggtgggagt ggggagtaga tagggaatat 2700
   gtgggatttg gtttaagttc atcattggaa gagttcctgg atccttgcaa gcttagataa 2760
   atgtgatctt tattagatag cagtggcatg ctttaaaaaa aaaaaaggca atgaaaattt 2820
   agcaagccac tgaatttgag ttttcacttt gtttctaata tgctgtgtga atcagtacag 2880
   ttttcttacc ctttcttggt cttaatttcc ttactgataa aatggggtag taatacctat     2940
   ctcaaaaaat tattgcacat attaaataac attcctctat gtatctcaat ggcattagac     3000
   attaggagaa gcattttgtg gaggatttga agttgagatc ttcatccaag aagtagcttt     3060
   tcaatttgct agaagcttaa tgtaggcaag ccacttcatt tttcagaact tgtttactca     3120
   tttataatat gggaataaaa atttgtgcaa gtcagagaag ggtgccttaa aaatgttgtg     3180
   gccaagccac atgagatcaa agacacactt ttcatgacct caaatgtggg cccagcctag     3240
   gtcagccaac ccccatccaa cccttagact cacgaacaaa tccacctgag atcagcagag     3300
   ccaccctaga tcagctgaaa ctctaagcac aaaaataaaa acttatcact gtataccact     3360
   ggagttttct ggttatctct cgtatagcaa aatctaactg atgcaatctc catctggcct     3420
   tcatccttct ccctttattg tcctttcgtg tattgttcat ccagcaacca ggatgatctt     3480
   gttaaaacat taaacagatt ctgtcactct taaaaaaaaa aaaa                      3524
<210> 90
   <211> 4787
   <212> DNA
   <213> Homo sapiens
<400> 90
   gagctggtgc tctcccccag cccctaggga attggagctg aggaggagct gaaaatgcag      60
   atttagcatc aagcacagac ctacactcgc tctttctctc cggtacacac agctccccac     120
   attcgcaccc ctgcccgcgc gccgggccgc ctgactgcac ggcttcccct ccagccagat     180
   gctggagaac acacactgat tcgctgcttt ccaagaccct gttcagtctc tttctctata     240
   caaagatttt tttaaaaact atatataaga attctttatt tgcaccctcc ctccgagtcc     300
   cctgctccgc cagcctgcgc gcctcctagc accacttttc actcccaaag aaggatgaag     360
   ggtggttgtg tctcccagtg gaaggcggcc gccgggttcc tcttctgtgt catggttttt     420
   gcatctgctg agcgaccggt cttcacgaat cattttcttg tggagttgca taaaggggga     480
   gaggacaaag ctcgccaagt tgcagcagaa cacggctttg gagtccgaaa gcttcccttt     540
   gctgaaggtc tgtaccactt ttatcacaat ggccttgcaa aggccaagag aagacgcagc     600
   ctacaccaca agcagcagct ggagagagac cccagggtaa agatggcttt gcagcaggaa     660
   ggatttgacc gaaaaaagcg aggttacaga gacatcaatg agatcgacat caacatgaac     720
   gatcctcttt ttacaaagca gtggtatctg atcaatactg ggcaagctga tggcactcct     780
   ggccttgatt tgaatgtggc tgaagcctgg gagctgggat acacagggaa aggtgttacc     840
   attggaatta tggatgatgg gattgactat ctccacccgg acctggcctc caactataat     900
   gccgaagcaa gttacgactt cagcagcaac gacccctatc cttaccctcg gtacacagat     960
   gactggttta acagccacgg gacccgatgt gcaggagaag tttctgctgc cgccaacaac    1020
   aatatctgtg gagttggagt agcatacaac tccaaggttg caggcatccg gatgctggac 1080
   cagccattca tgacagacat catcgaggcc tcctccatca gtcatatgcc acagctgatt 1140
   gacatctaca gcgccagctg gggccccaca gacaacggca agacagtgga tgggccccgg 1200
   gagctcacgc tgcaggccat ggccgatggc gtgaacaagg gccgcggcgg caaaggcagc 1260
   atctacgtgt gggcctccgg ggacggcggc agctatgacg actgcaactg cgacggctac 1320
   gcctccagca tgtggaccat ctccatcaac tcagccatca acgacggcag gactgccctg 1380
   tacgacgaga gctgctcttc caccttggct tccaccttca gcaacgggag gaaaaggaac 1440
   cccgaggccg gtgtggcaac cacagatttg tacggcaact gcactctgag gcattctggg 1500
   acatctgcag ctgcccccga ggcagctggt gtgtttgcac tggctctgga ggctaacctg 1560
   ggtctgacct ggcgggacat gcagcatctg actgtgctca cctccaaacg gaaccagctt 1620
   cacgacgagg tccatcagtg gcggcgcaat ggggtcggcc tggaatttaa tcacctcttt 1680
   ggctacgggg tccttgatgc aggtgccatg gtgaaaatgg ctaaagactg gaaaaccgtg 1740
   cctgagagat tccactgtgt gggaggctcc gtgcaggacc ctgagaaaat accatccact 1800
   ggcaagttgg tgctgacact cacaaccgac gcctgtgagg ggaaggaaaa ttttgtccgc 1860
   tacctggagc atgtccaggc tgtcatcacg gtcaacgcaa ccagaagagg agacctgaac 1920
   atcaacatga cttcccctat gggcaccaag tccattttgc tgagccggcg tccaagggat 1980
   gacgactcca aggtgggctt tgacaagtgg cctttcatga ccactcacac gtggggggaa 2040
   gacgcccgag gcacctggac cctggagctg ggatttgtcg gcagcgcccc gcagaagggg 2100
   gtgctgaagg agtggaccct gatgctgcat ggcactcaga gtgccccgta catcgaccag 2160
   gtggtgcggg attaccagtc caagttggcc atgtccaaga aagaggagct ggaggaagag 2220
   ctggacgaag ccgtggagag aagcctgaaa agcatcctta acaagaacta gcgctgcaca 2280
   tccgcctttc ccaccgccct ccctccccag ctccgcctct gtcctcgctc cacgtttcag 2340
   gcaggcacct agcaattcca tcacccgtac aggcaattcc gtcttcttaa tctgaagctt 2400
   cactcactgt caatgattat tttcattaca atggaaacaa tcttttttac tctatgcccc 2460
   aaatatagcg ttcccaacaa catccatgtc ctatgtgtga ctctaaattc tttatttctg 2520
   tcattcaaat gggtgatatc ctgaaaaaaa aaaaaaaaaa aaaactggga cagctttccc 2580
   ctcatttttt tttttgtttc tgagaaaaga acgtatttta aaagccacat agagtgactc 2640
   caagaacaat tgtccatggt ctcaaacaag gggctgttac ataacaagaa aatcaaagct 2700
   gaggacaggg tgtgagcgcc acatctctga aagcacagga gacactgtgc tataaatcct 2760
   ttggggagcg atgttttgaa tttagtgaga tttaccaggg atgtagatta aggtgatgtg 2820
   attcaaaaga tgccattcat agagagccct agttactgca tggggaaaga gatccaggaa 2880
   gcatgagtgc tggatatttt actaccaatg ccaagataat tcactctact cagccggcgt 2940
   ggcaaatata aaacttacag agcgtggctg tgctctcacc agctgctgct ctgagttatg 3000
   ttaaaatccg ctagagcagc ccaaattttt ctcagtttgt atagagttca tcccagcccc 3060
   aattttctgg ggctcctcac atagctaccc aaaagagaaa aaaaattaag acaagcctgg 3120
   caacacacct ggtgaagagt agtttactag cttttcaaac aagaatgtcc cttttcctaa 3180
   gtcactttga ggtgtctcaa tctgatctga gtgagaggcg acaggagtat tttttttttt 3240
   ttacagcttt acacacacag atgtgggctt tgatttccaa gtaatataat ggaagagaaa 3300
   tctcatactc ccccacagtt tgatgtcatt aatgtgttgg gaaaaaggcc tctgtcccgg 3360
   aagagtcatg ggaggtgaaa ggggcacgtt tgaagatgcg agcgctatct tcacatagtt 3420
   ctccagttgt atggagcctc ttctgccaag agagggccat gcaattcatc ccagaggaac 3480
   ctgaggcctg aaggaggtga gagaagacct ctgtgaggaa agcacacagt caccttctcg 3540
   gcaactaagc agtccctgag accatttaac atgcaacccg aaggttatgg tcaatcccaa 3600
   aagtcaccac tccattccca actagacatt accaaagtga cctacccaga gattgcttct 3660
   catccccagt cccaatgcac atccattccc aagaaatgct ttgtcttcag cctctccagg 3720
   caccatctcc cttcctgtgg gagcagagag cttagcctgg agcacctttc cttcaagcca 3780
   gcaacacaga gcactaggtt caattccctg aaggtggcca ctttaagaga gaaatctgaa 3840
   aaccccattt gctttctttt ctcccatatt ggcatggatt tctgtcttct ctaacacctt 3900
   gtgaccttct ctatatcatg ctttaaagtg taataatatg attttttaaa agaaatttat 3960
   tacttgttgc aaaggtcttt ttaaaccagt ttagatttca agaaaaaata aatggaaatc 4020
   atcgaaaatt catttcacat taatggtcta aaaataaacc aaaggacatt atgtgtgcat 4080
   gtgtgtataa gtgcacacag aaatatatat acatatgtag actatataca tgtgtgtata 4140
   tatgtgtata tatacataca cttgtataaa tgtatataca catataccta taatgtgtgt 4200
   atgtgtattt attgaagaaa cagataccat actcatttct aaaagaatat tcagagaata 4260
   tcaagatgat tctggctgaa aaaggccagt ggaaattcag gtgaaaatgt tcatcaattc 4320
   ccattgcatc acctctgtaa tttttcagct ctctgtataa acattaaatg tcttatatag 4380
   cagcaaaaat ataaaatagt tgtccatatt ttcacaggtg tggtgtaatt tataaaatta 4440
   gaaagcaact tatcagctac ttaagagaaa tggcaagttt tgatatgagt atacaatata 4500
   taaaaatata tatagtgcta tatatataaa tatttggtct ctatttcatt ttttgcatca 4560
   gtattaatac taaaatatgt ctcgctagtg atgtttttat gatatccctg atcctaactg 4620
   aagagacagt tatttatagt catttatttt aaaaaatgaa aataagtgaa taataattag 4680
   gttaacattg ttgctccctg tgacaaaatt ttataagcaa atttcaaaag acatgttgta 4740
   aattaggagg ctcaacaata aaacattatg ctccagaaaa aaaaaaa 4787
<210> 91
   <211> 5820
   <212> DNA
   <213> Homo sapiens
<400> 91
   agccgctgcg cccgagctgg cctgcgagtt cagggctcct gtcgctctcc aggagcaacc 60
   tctactccgg acgcacaggc attccccgcg cccctccagc cctcgccgcc ctcgccaccg 120
   ctcccggccg ccgcgctccg gtacacacag gatccctgct gggcaccaac agctccacca 180
   tggggctggc ctggggacta ggcgtcctgt tcctgatgca tgtgtgtggc accaaccgca 240
   ttccagagtc tggcggagac aacagcgtgt ttgacatctt tgaactcacc ggggccgccc 300
   gcaaggggtc tgggcgccga ctggtgaagg gccccgaccc ttccagccca gctttccgca 360
   tcgaggatgc caacctgatc ccccctgtgc ctgatgacaa gttccaagac ctggtggatg 420
   ctgtgcgggc agaaaagggt ttcctccttc tggcatccct gaggcagatg aagaagaccc 480
   ggggcacgct gctggccctg gagcggaaag accactctgg ccaggtcttc agcgtggtgt 540
   ccaatggcaa ggcgggcacc ctggacctca gcctgaccgt ccaaggaaag cagcacgtgg 600
   tgtctgtgga agaagctctc ctggcaaccg gccagtggaa gagcatcacc ctgtttgtgc 660
   aggaagacag ggcccagctg tacatcgact gtgaaaagat ggagaatgct gagttggacg 720
   tccccatcca aagcgtcttc accagagacc tggccagcat cgccagactc cgcatcgcaa 780
   aggggggcgt caatgacaat ttccaggggg tgctgcagaa tgtgaggttt gtctttggaa 840
   ccacaccaga agacatcctc aggaacaaag gctgctccag ctctaccagt gtcctcctca 900
   cccttgacaa caacgtggtg aatggttcca gccctgccat ccgcactaac tacattggcc 960
   acaagacaaa ggacttgcaa gccatctgcg gcatctcctg tgatgagctg tccagcatgg 1020
   tcctggaact caggggcctg cgcaccattg tgaccacgct gcaggacagc atccgcaaag 1080
   tgactgaaga gaacaaagag ttggccaatg agctgaggcg gcctccccta tgctatcaca 1140
   acggagttca gtacagaaat aacgaggaat ggactgttga tagctgcact gagtgtcact 1200
   gtcagaactc agttaccatc tgcaaaaagg tgtcctgccc catcatgccc tgctccaatg 1260
   ccacagttcc tgatggagaa tgctgtcctc gctgttggcc cagcgactct gcggacgatg 1320
   gctggtctcc atggtccgag tggacctcct gttctacgag ctgtggcaat ggaattcagc 1380
   agcgcggccg ctcctgcgat agcctcaaca accgatgtga gggctcctcg gtccagacac 1440
   ggacctgcca cattcaggag tgtgacaaga gatttaaaca ggatggtggc tggagccact 1500
   ggtccccgtg gtcatcttgt tctgtgacat gtggtgatgg tgtgatcaca aggatccggc 1560
   tctgcaactc tcccagcccc cagatgaacg ggaaaccctg tgaaggcgaa gcgcgggaga 1620
   ccaaagcctg caagaaagac gcctgcccca tcaatggagg ctggggtcct tggtcaccat 1680
   gggacatctg ttctgtcacc tgtggaggag gggtacagaa acgtagtcgt ctctgcaaca 1740
   accccacacc ccagtttgga ggcaaggact gcgttggtga tgtaacagaa aaccagatct 1800
   gcaacaagca ggactgtcca attgatggat gcctgtccaa tccctgcttt gccggcgtga 1860
   agtgtactag ctaccctgat ggcagctgga aatgtggtgc ttgtccccct ggttacagtg 1920
   gaaatggcat ccagtgcaca gatgttgatg agtgcaaaga agtgcctgat gcctgcttca 1980
   accacaatgg agagcaccgg tgtgagaaca cggaccccgg ctacaactgc ctgccctgcc 2040
   ccccacgctt caccggctca cagcccttcg gccagggtgt cgaacatgcc acggccaaca 2100
   aacaggtgtg caagccccgt aacccctgca cggatgggac ccacgactgc aacaagaacg 2160
   ccaagtgcaa ctacctgggc cactatagcg accccatgta ccgctgcgag tgcaagcctg 2220
   gctacgctgg caatggcatc atctgcgggg aggacacaga cctggatggc tggcccaatg 2280
   agaacctggt gtgcgtggcc aatgcgactt accactgcaa aaaggataat tgccccaacc 2340
   ttcccaactc agggcaggaa gactatgaca aggatggaat tggtgatgcc tgtgatgatg 2400
   acgatgacaa tgataaaatt ccagatgaca gggacaactg tccattccat tacaacccag 2460
   ctcagtatga ctatgacaga gatgatgtgg gagaccgctg tgacaactgt ccctacaacc 2520
   acaacccaga tcaggcagac acagacaaca atggggaagg agacgcctgt gctgcagaca 2580
   ttgatggaga cggtatcctc aatgaacggg acaactgcca gtacgtctac aatgtggacc 2640
   agagagacac tgatatggat ggggttggag atcagtgtga caattgcccc ttggaacaca 2700
   atccggatca gctggactct gactcagacc gcattggaga tacctgtgac aacaatcagg 2760
   atattgatga agatggccac cagaacaatc tggacaactg tccctatgtg cccaatgcca 2820
   accaggctga ccatgacaaa gatggcaagg gagatgcctg tgaccacgat gatgacaacg 2880
   atggcattcc tgatgacaag gacaactgca gactcgtgcc caatcccgac cagaaggact 2940
   ctgacggcga tggtcgaggt gatgcctgca aagatgattt tgaccatgac agtgtgccag 3000
   acatcgatga catctgtcct gagaatgttg acatcagtga gaccgatttc cgccgattcc 3060
   agatgattcc tctggacccc aaagggacat cccaaaatga ccctaactgg gttgtacgcc 3120
   atcagggtaa agaactcgtc cagactgtca actgtgatcc tggactcgct gtaggttatg 3180
   atgagtttaa tgctgtggac ttcagtggca ccttcttcat caacaccgaa agggacgatg 3240
   actatgctgg atttgtcttt ggctaccagt ccagcagccg cttttatgtt gtgatgtgga 3300
   agcaagtcac ccagtcctac tgggacacca accccacgag ggctcaggga tactcgggcc 3360
   tttctgtgaa agttgtaaac tccaccacag ggcctggcga gcacctgcgg aacgccctgt 3420
   ggcacacagg aaacacccct ggccaggtgc gcaccctgtg gcatgaccct cgtcacatag 3480
   gctggaaaga tttcaccgcc tacagatggc gtctcagcca caggccaaag acgggtttca 3540
   ttagagtggt gatgtatgaa gggaagaaaa tcatggctga ctcaggaccc atctatgata 3600
   aaacctatgc tggtggtaga ctagggttgt ttgtcttctc tcaagaaatg gtgttcttct     3660
   ctgacctgaa atacgaatgt agagatccct aatcatcaaa ttgttgattg aaagactgat     3720
   cataaaccaa tgctggtatt gcaccttctg gaactatggg cttgagaaaa cccccaggat     3780
   cacttctcct tggcttcctt cttttctgtg cttgcatcag tgtggactcc tagaacgtgc     3840
   gacctgcctc aagaaaatgc agttttcaaa aacagactca gcattcagcc tccaatgaat     3900
   aagacatctt ccaagcatat aaacaattgc tttggtttcc ttttgaaaaa gcatctactt     3960
   gcttcagttg ggaaggtgcc cattccactc tgcctttgtc acagagcagg gtgctattgt     4020
   gaggccatct ctgagcagtg gactcaaaag cattttcagg catgtcagag aagggaggac     4080
   tcactagaat tagcaaacaa aaccaccctg acatcctcct tcaggaacac ggggagcaga     4140
   ggccaaagca ctaaggggag ggcgcatacc cgagacgatt gtatgaagaa aatatggagg     4200
   aactgttaca tgttcggtac taagtcattt tcaggggatt gaaagactat tgctggattt     4260
   catgatgctg actggcgtta gctgattaac ccatgtaaat aggcacttaa atagaagcag     4320
   gaaagggaga caaagactgg cttctggact tcctccctga tccccaccct tactcatcac     4380
   ctgcagtggc cagaattagg gaatcagaat caaaccagtg taaggcagtg ctggctgcca     4440
   ttgcctggtc acattgaaat tggtggcttc attctagatg tagcttgtgc agatgtagca     4500
   ggaaaatagg aaaacctacc atctcagtga gcaccagctg cctcccaaag gaggggcagc     4560
   cgtgcttata tttttatggt tacaatggca caaaattatt atcaacctaa ctaaaacatt     4620
   ccttttctct tttttcctga attatcatgg agttttctaa ttctctcttt tggaatgtag     4680
   atttttttta aatgctttac gatgtaaaat atttattttt tacttattct ggaagatctg     4740
   gctgaaggat tattcatgga acaggaagaa gcgtaaagac tatccatgtc atctttgttg     4800
   agagtcttcg tgactgtaag attgtaaata cagattattt attaactctg ttctgcctgg     4860
   aaatttaggc ttcatacgga aagtgtttga gagcaagtag ttgacattta tcagcaaatc     4920
   tcttgcaaga acagcacaag gaaaatcagt ctaataagct gctctgcccc ttgtgctcag     4980
   agtggatgtt atgggattct ttttttctct gttttatctt ttcaagtgga attagttggt     5040
   tatccatttg caaatgtttt aaattgcaaa gaaagccatg aggtcttcaa tactgtttta     5100
   ccccatccct tgtgcatatt tccagggaga aggaaagcat atacactttt ttctttcatt     5160
   tttccaaaag agaaaaaaat gacaaaaggt gaaacttaca tacaaatatt acctcatttg     5220
   ttgtgtgact gagtaaagaa tttttggatc aagcggaaag agtttaagtg tctaacaaac     5280
   ttaaagctac tgtagtacct aaaaagtcag tgttgtacat agcataaaaa ctctgcagag     5340
   aagtattccc aataaggaaa tagcattgaa atgttaaata caatttctga aagttatgtt     5400
   ttttttctat catctggtat accattgctt tatttttata aattattttc tcattgccat      5460
   tggaatagat atctcagatt gtgtagatat gctatttaaa taatttatca ggaaatactg 5520
   cctgtagagt tagtatttct atttttatat aatgtttgca cactgaattg aagaattgtt 5580
   ggttttttct tttttttgtt ttgttttttt tttttttttt ttttgctttt gacctcccat 5640
   ttttactatt tgccaatacc tttttctagg aatgtgcttt tttttgtaca catttttatc 5700
   cattttacat tctaaagcag tgtaagttgt atattactgt ttcttatgta caaggaacaa 5760
   caataaatca tatggaaatt tatatttata aaaaaaaaaa aaaaaaaaaa aaaaaaaaaa 5820
<210> 92
   <211> 1648
   <212> DNA
   <213> Homo sapiens
<400> 92
   cagagaaggc ttaggctccc gagtcaacag ggcattcacc gcctggggcg cctgagtcat      60
   caggacactg ccaggagaca cagaacccta gatgccctgc agaatccttc ctgttacggt     120
   ccccctccct gaaacatcct tcattgcaat atttccagga aaggaagggg gctggctcgg     180
   aggaagagag gtggggaggt gatcagggtt cacagaggag ggaactgaat gacatcccag     240
   gattacataa actgtcagag gcagccgaag agttcacaag tgtgaagcct ggaagccggc     300
   gggtgccgct gtgtaggaaa gaagctaaag cacttccaga gcctgtccgg agctcagagg     360
   ttcggaagac ttatcgacca tggagcgcgc gtcctgcttg ttgctgctgc tgctgccgct     420
   ggtgcacgtc tctgcgacca cgccagaacc ttgtgagctg gacgatgaag atttccgctg     480
   cgtctgcaac ttctccgaac ctcagcccga ctggtccgaa gccttccagt gtgtgtctgc     540
   agtagaggtg gagatccatg ccggcggtct caacctagag ccgtttctaa agcgcgtcga     600
   tgcggacgcc gacccgcggc agtatgctga cacggtcaag gctctccgcg tgcggcggct     660
   cacagtggga gccgcacagg ttcctgctca gctactggta ggcgccctgc gtgtgctagc     720
   gtactcccgc ctcaaggaac tgacgctcga ggacctaaag ataaccggca ccatgcctcc     780
   gctgcctctg gaagccacag gacttgcact ttccagcttg cgcctacgca acgtgtcgtg     840
   ggcgacaggg cgttcttggc tcgccgagct gcagcagtgg ctcaagccag gcctcaaggt     900
   actgagcatt gcccaagcac actcgcctgc cttttcctgc gaacaggttc gcgccttccc     960
   ggcccttacc agcctagacc tgtctgacaa tcctggactg ggcgaacgcg gactgatggc    1020
   ggctctctgt ccccacaagt tcccggccat ccagaatcta gcgctgcgca acacaggaat    1080
   ggagacgccc acaggcgtgt gcgccgcact ggcggcggca ggtgtgcagc cccacagcct    1140
   agacctcagc cacaactcgc tgcgcgccac cgtaaaccct agcgctccga gatgcatgtg    1200
   gtccagcgcc ctgaactccc tcaatctgtc gttcgctggg ctggaacagg tgcctaaagg    1260
   actgccagcc aagctcagag tgctcgatct cagctgcaac agactgaaca gggcgccgca    1320
   gcctgacgag ctgcccgagg tggataacct gacactggac gggaatccct tcctggtccc    1380
   tggaactgcc ctcccccacg agggctcaat gaactccggc gtggtcccag cctgtgcacg    1440
   ttcgaccctg tcggtggggg tgtcgggaac cctggtgctg ctccaagggg cccggggctt    1500
   tgcctaagat ccaagacaga ataatgaatg gactcaaact gccttggctt caggggagtc    1560
   ccgtcaggac gttgaggact tttcgaccaa ttcaaccctt tgccccacct ttattaaaat    1620
   cttaaacaac gggtcaaaaa aaaaaaaa                                       1648
<210> 93
   <211> 1616
   <212> DNA
   <213> Homo sapiens
<400> 93
   gccaaggctg gggcagggga gtcagcagag gcctcgctcg ggcgcccagt ggtcctgccg      60
   cctggtctca cctcgctatg gttcgtctgc ctctgcagtg cgtcctctgg ggctgcttgc     120
   tgaccgctgt ccatccagaa ccacccactg catgcagaga aaaacagtac ctaataaaca     180
   gtcagtgctg ttctttgtgc cagccaggac agaaactggt gagtgactgc acagagttca     240
   ctgaaacgga atgccttcct tgcggtgaaa gcgaattcct agacacctgg aacagagaga     300
   cacactgcca ccagcacaaa tactgcgacc ccaacctagg gcttcgggtc cagcagaagg     360
   gcacctcaga aacagacacc atctgcacct gtgaagaagg ctggcactgt acgagtgagg     420
   cctgtgagag ctgtgtcctg caccgctcat gctcgcccgg ctttggggtc aagcagattg     480
   ctacaggggt ttctgatacc atctgcgagc cctgcccagt cggcttcttc tccaatgtgt     540
   catctgcttt cgaaaaatgt cacccttgga caagctgtga gaccaaagac ctggttgtgc     600
   aacaggcagg cacaaacaag actgatgttg tctgtggtcc ccaggatcgg ctgagagccc     660
   tggtggtgat ccccatcatc ttcgggatcc tgtttgccat cctcttggtg ctggtcttta     720
   tcaaaaaggt ggccaagaag ccaaccaata aggcccccca ccccaagcag gaaccccagg     780
   agatcaattt tcccgacgat cttcctggct ccaacactgc tgctccagtg caggagactt     840
   tacatggatg ccaaccggtc acccaggagg atggcaaaga gagtcgcatc tcagtgcagg     900
   agagacagtg aggctgcacc cacccaggag tgtggccacg tgggcaaaca ggcagttggc     960
   cagagagcct ggtgctgctg ctgctgtggc gtgagggtga ggggctggca ctgactgggc    1020
   atagctcccc gcttctgcct gcacccctgc agtttgagac aggagacctg gcactggatg    1080
   cagaaacagt tcaccttgaa gaacctctca cttcaccctg gagcccatcc agtctcccaa    1140
   cttgtattaa agacagaggc agaagtttgg tggtggtggt gttggggtat ggtttagtaa    1200
   tatccaccag accttccgat ccagcagttt ggtgcccaga gaggcatcat ggtggcttcc    1260
   ctgcgcccag gaagccatat acacagatgc ccattgcagc attgtttgtg atagtgaaca    1320
   actggaagct gcttaactgt ccatcagcag gagactggct aaataaaatt agaatatatt    1380
   tatacaacag aatctcaaaa acactgttga gtaaggaaaa aaaggcatgc tgctgaatga    1440
   tgggtatgga actttttaaa aaagtacatg cttttatgta tgtatattgc ctatggatat    1500
   atgtataaat acaatatgca tcatatattg atataacaag ggttctggaa gggtacacag    1560
   aaaacccaca gctcgaagag tggtgacgtc tggggtgggg aagaagggtc tggggg        1616
<210> 94
   <211> 2757
   <212> DNA
   <213> Homo sapiens
<400> 94
   gacaagtact gagtgaactc aaaccctctg taaagtaaca gaagttagaa ggggaaatgt      60
   cgcctctctg aagattaccc aaagaaaaag tgatttgtca ttgctttata gactgtaaga     120
   agagaacatc tcagaagtgg agtcttaccc tgaaatcaaa ggatttaaag aaaaagtgga     180
   atttttcttc agcaagctgt gaaactaaat ccacaacctt tggagaccca ggaacaccct     240
   ccaatctctg tgtgttttgt aaacatcact ggagggtctt ctacgtgagc aattggattg     300
   tcatcagccc tgcctgtttt gcacctggga agtgccctgg tcttacttgg gtccaaattg     360
   ttggctttca cttttgaccc taagcatctg aagccatggg ccacacacgg aggcagggaa     420
   catcaccatc caagtgtcca tacctcaatt tctttcagct cttggtgctg gctggtcttt     480
   ctcacttctg ttcaggtgtt atccacgtga ccaaggaagt gaaagaagtg gcaacgctgt     540
   cctgtggtca caatgtttct gttgaagagc tggcacaaac tcgcatctac tggcaaaagg     600
   agaagaaaat ggtgctgact atgatgtctg gggacatgaa tatatggccc gagtacaaga     660
   accggaccat ctttgatatc actaataacc tctccattgt gatcctggct ctgcgcccat     720
   ctgacgaggg cacatacgag tgtgttgttc tgaagtatga aaaagacgct ttcaagcggg     780
   aacacctggc tgaagtgacg ttatcagtca aagctgactt ccctacacct agtatatctg     840
   actttgaaat tccaacttct aatattagaa ggataatttg ctcaacctct ggaggttttc     900
   cagagcctca cctctcctgg ttggaaaatg gagaagaatt aaatgccatc aacacaacag     960
   tttcccaaga tcctgaaact gagctctatg ctgttagcag caaactggat ttcaatatga    1020
   caaccaacca cagcttcatg tgtctcatca agtatggaca tttaagagtg aatcagacct    1080
   tcaactggaa tacaaccaag caagagcatt ttcctgataa cctgctccca tcctgggcca    1140
   ttaccttaat ctcagtaaat ggaatttttg tgatatgctg cctgacctac tgctttgccc    1200
   caagatgcag agagagaagg aggaatgaga gattgagaag ggaaagtgta cgccctgtat    1260
   aacagtgtcc gcagaagcaa ggggctgaaa agatctgaag gtcccacctc catttgcaat    1320
   tgacctcttc tgggaacttc ctcagatgga caagattacc ccaccttgcc ctttacgtat    1380
   ctgctcttag gtgcttcttc acttcagttg ctttgcagga agtgtctaga ggaatatggt 1440
   gggcacagaa gtagctctgg tgaccttgat caaggtgttt tgaaatgcag aattcttgag 1500
   ttctggaagg gactttagag aataccagtg ttattaatga caaaggcact gaggcccagg 1560
   gaggtgaccc gaattataaa ggccagcgcc agaacccaga tttcctaact ctggtgctct 1620
   ttccctttat cagtttgact gtggcctgtt aactggtata tacatatata tgtcaggcaa 1680
   agtgctgctg gaagtagaat ttgtccaata acaggtcaac ttcagagact atctgatttc 1740
   ctaatgtcag agtagaagat tttatgctgc tgtttacaaa agcccaatgt aatgcatagg 1800
   aagtatggca tgaacatctt taggagacta atggaaatat tattggtgtt tacccagtat 1860
   tccatttttt tcattgtgtt ctctattgct gctctctcac tcccccatga ggtacagcag 1920
   aaaggagaac tatccaaaac taatttcctc tgacatgtaa gacgaatgat ttaggtacgt 1980
   caaagcagta gtcaaggagg aaagggatag tccaaagact taactggttc atattggact 2040
   gataatctct ttaaatggct ttatgctagt ttgacctcat ttgtaaaata tttatgagaa 2100
   agttctcatt taaaatgaga tcgttgttta cagtgtatgt actaagcagt aagctatctt 2160
   caaatgtcta aggtagtaac tttccatagg gcctccttag atccctaaga tggctttttc 2220
   tccttggtat ttctgggtct ttctgacatc agcagagaac tggaaagaca tagccaactg 2280
   ctgttcatgt tactcatgac tcctttctct aaaactgcct tccacaattc actagaccag 2340
   aagtggacgc aacttaagct gggataatca cattatcatc tgaaaatctg gagttgaaca 2400
   gcaaaagaag acaacatttc tcaaatgcac atctcatggc agctaagcca catggctggg 2460
   atttaaagcc tttagagcca gcccatggct ttagctacct cactatgctg cttcacaaac 2520
   cttgctcctg tgtaaaacta tattctcagt gtagggcaga gaggtctaac accaacataa 2580
   ggtactagca gtgtttcccg tattgacagg aatacttaac tcaataattc ttttcttttc 2640
   catttagtaa cagttgtgat gactatgttt ctattctaag taattcctgt attctacagc 2700
   agatactttg tcagcaatac taagggaaga aacaaagttg aaccgtttct ttaataa 2757
<210> 95
   <211> 2207
   <212> DNA
   <213> Homo sapiens
<400> 95
   cacttcctcc ccagacaggg gtagtgcgag gccgggcaca gccttcctgt gtggttttac      60
   cgcccagaga gcgtcatgga cctggggaaa ccaatgaaaa gcgtgctggt ggtggctctc     120
   cttgtcattt tccaggtatg cctgtgtcaa gatgaggtca cggacgatta catcggagac     180
   aacaccacag tggactacac tttgttcgag tctttgtgct ccaagaagga cgtgcggaac     240
   tttaaagcct ggttcctccc tatcatgtac tccatcattt gtttcgtggg cctactgggc     300
   aatgggctgg tcgtgttgac ctatatctat ttcaagaggc tcaagaccat gaccgatacc      360
   tacctgctca acctggcggt ggcagacatc ctcttcctcc tgacccttcc cttctgggcc      420
   tacagcgcgg ccaagtcctg ggtcttcggt gtccactttt gcaagctcat ctttgccatc      480
   tacaagatga gcttcttcag tggcatgctc ctacttcttt gcatcagcat tgaccgctac      540
   gtggccatcg tccaggctgt ctcagctcac cgccaccgtg cccgcgtcct tctcatcagc      600
   aagctgtcct gtgtgggcat ctggatacta gccacagtgc tctccatccc agagctcctg      660
   tacagtgacc tccagaggag cagcagtgag caagcgatgc gatgctctct catcacagag      720
   catgtggagg cctttatcac catccaggtg gcccagatgg tgatcggctt tctggtcccc      780
   ctgctggcca tgagcttctg ttaccttgtc atcatccgca ccctgctcca ggcacgcaac      840
   tttgagcgca acaaggccat caaggtgatc atcgctgtgg tcgtggtctt catagtcttc      900
   cagctgccct acaatggggt ggtcctggcc cagacggtgg ccaacttcaa catcaccagt      960
   agcacctgtg agctcagtaa gcaactcaac atcgcctacg acgtcaccta cagcctggcc     1020
   tgcgtccgct gctgcgtcaa cccctttctg tacgccttca tcggcgtcaa gttccgcaac     1080
   gatctcttca agctcttcaa ggacctgggc tgcctcagcc aggagcagct ccggcagtgg     1140
   tcttcctgtc ggcacatccg gcgctcctcc atgagtgtgg aggccgagac caccaccacc     1200
   ttctccccat aggcgactct tctgcctgga ctagagggac ctctcccagg gtccctgggg     1260
   tggggatagg gagcagatgc aatgactcag gacatccccc cgccaaaagc tgctcaggga     1320
   aaagcagctc tcccctcaga gtgcaagccc ctgctccaga agatagcttc accccaatcc     1380
   cagctacctc aaccaatgcc aaaaaaagac agggctgata agctaacacc agacagacaa     1440
   cactgggaaa cagaggctat tgtcccctaa accaaaaact gaaagtgaaa gtccagaaac     1500
   tgttcccacc tgctggagtg aaggggccaa ggagggtgag tgcaaggggc gtgggagtgg     1560
   cctgaagagt cctctgaatg aaccttctgg cctcccacag actcaaatgc tcagaccagc     1620
   tcttccgaaa accaggcctt atctccaaga ccagagatag tggggagact tcttggcttg     1680
   gtgaggaaaa gcggacatca gctggtcaaa caaactctct gaacccctcc ctccatcgtt     1740
   ttcttcactg tcctccaagc cagcgggaat ggcagctgcc acgccgccct aaaagcacac     1800
   tcatcccctc acttgccgcg tcgccctccc aggctctcaa caggggagag tgtggtgttt     1860
   cctgcaggcc aggccagctg cctccgcgtg atcaaagcca cactctgggc tccagagtgg     1920
   ggatgacatg cactcagctc ttggctccac tgggatggga ggagaggaca agggaaatgt     1980
   caggggcggg gagggtgaca gtggccgccc aaggcccacg agcttgttct ttgttctttg     2040
   tcacagggac tgaaaacctc tcctcatgtt ctgctttcga ttcgttaaga gagcaacatt     2100
   ttacccacac acagataaag ttttcccttg aggaaacaac agctttaaaa gaaaaagaaa     2160
   aaaaaagtct ttggtaaatg gcaaaaaaaa aaaaaaaaaa aaaaaaa                   2207
<210> 96
   <211> 2307
   <212> DNA
   <213> Homo sapiens
<400> 96
   gattatcaca gattctggag aagagtgagg acttgggttc accacctaca gcctggctcc 60
   cgcgacgccg gaggtgaagg tggcttgctc cgaagatgtg gacttgccct gcaccgcccc 120
   ctgggatccg caggttccct acacggtctc ctgggtcaag ttattggagg gtggtgaaga 180
   gaggatggag acaccccagg aagaccacct caggggacag cactatcatc agaaggggca 240
   aaatggttct ttcgacgccc ccaatgaaag gccctattcc ctgaagatcc gaaacactac 300
   cagctgcaac tcggggacat acaggtgcac tctgcaggac ccggatgggc agagaaacct 360
   aagtggcaag gtgatcttga gagtgacagg atgccctgca cagcgtaaag aagagacttt 420
   taagaaatac agagcggaga ttgtcctgct gctggctctg gttattttct acttaacact 480
   catcattttc acttgtaagt ttgcacggct acagagtatc ttcccagatt tttctaaagc 540
   tggcatggaa cgagcttttc tcccagttac ctccccaaat aagcatttag ggctagtgac 600
   tcctcacaag acagaactgg tatgagcagg atttctgcag gttcttcttc ctgaagctga 660
   ggctcagggg tgtgcctgtc tgttacactg gaggagagaa gaatgagcct acgctgaaga 720
   tggcatcctg tgaagtcctt cacctcactg aaaacatctg gaaggggatc ccaccccatt 780
   ttctgtgggc aggcctcgaa aaccatcaca tgaccacata gcatgaggcc actgctgctt 840
   ctccatggcc accttttcag cgatgtatgc agctatctgg tcaacctcct ggacattttt 900
   tcagtcatat aaaagctatg gtgagatgca gctggaaaag ggtcttggga aatatgaatg 960
   cccccagctg gcccgtgaca gactcctgag gacagctgtc ctcttctgca tcttggggac 1020
   atctctttga attttctgtg ttttgctgta ccagcccaga tgttttacgt ctgggagaaa 1080
   ttgacagatc aagctgtgag acagtgggaa atatttagca aataatttcc tggtgtgaag 1140
   gtcctgctat tactaaggag taatctgtgt acaaagaaat aacaagtcga tgaactattc 1200
   cccagcaggg tcttttcatc tgggaaagac atccataaag aagcaataaa gaagagtgcc 1260
   acatttattt ttatatctat atgtacttgt caaagaaggt ttgtgttttt ctgcttttga 1320
   aatctgtatc tgtagtgaga tagcattgtg aactgacagg cagcctggac atagagaggg 1380
   agaagaagtc agagagggtg acaagataga gagctattta atggccggct ggaaatgctg 1440
   ggctgacggt gcagtctggg tgctcgccca cttgtcccac tatctgggtg catgatcttg 1500
   agcaagttcc ttctggtgtc tgctttctcc attgtaaacc acaaggctgt tgcatgggct 1560
   aatgaagatc atatacgtga aaattatttg aaaacatata aagcactata cagattcgaa 1620
   actccattga gtcattatcc ttgctatgat gatggtgttt tggggatgag agggtgctat 1680
   ccatttctca tgttttccat tgtttgaaac aaagaaggtt accaagaagc ctttcctgta     1740
   gccttctgta ggaattcttt tggggaagtg aggaagccag gtccacggtc tgttcttgaa     1800
   gcagtagcct aacacactcc aagatatgga cacacgggag ccgctggcag aagggacttc     1860
   acgaagtgtt gcatggatgt tttagccatt gttggctttc ccttatcaaa cttgggccct     1920
   tcccttcttg gtttccaaag gcattttatt gcttgagtta tatgttcact gtccccctaa     1980
   tattagggag taaaacggat accaagttga tttagtgttt ttacctctgt cttggctttc     2040
   atgttattaa acgtatgcat gtgaagaaag ggtgtttttc tgttttatat tcaactcata     2100
   agactttggg ataggaaaaa tgagtaatgg ttactaggct taatacctgg gtgattacat     2160
   aatctgtaca atgaaccccc atgatgtaag tttacctatg taacaaacct gcacttatac     2220
   ccatgaactt aaaatgaaag ttaaaaataa aaaacatata caaataaaaa aatcccgact     2280
   ttgggatgag tgctaggatg ttgtaaa                                         2307
<210> 97
   <211> 2348
   <212> DNA
   <213> Homo sapiens
<400> 97
   agatgtgagt cctcaatgag ctataaccac agccataaat atctctcaaa gatgaggaac        60
   attctcatga tgttgacact gcaatttttt gacaatttcc caacactctt aagaaacatt       120
   ccccaatctc acacgaaaag tgggggtttt aattttcttg ttcaacttct aaagagaaat       180
   tggagaagat aaaactggac actggggaga ccacaacttc atgctgcgtg ggatctccca       240
   gctacctgca gtggccacca tgtcttgggt cctgctgcct gtactttggc tcattgttca       300
   aactcaagca atagccataa agcaaacacc tgaattaacg ctccatgaaa tagtttgtcc       360
   taaaaaactt cacattttac acaaaagaga gatcaagaac aaccagacag aaaagcatgg       420
   caaagaggaa aggtatgaac ctgaagttca atatcagatg atcttaaatg gagaagaaat       480
   cattctctcc ctacaaaaaa ccaagcacct cctggggcca gactacactg aaacattgta       540
   ctcacccaga ggagaggaaa ttaccacgaa acctgagaac atggaacact gttactataa       600
   aggaaacatc ctaaatgaaa agaattctgt tgccagcatc agtacttgtg acgggttgag       660
   aggatacttc acacatcatc accaaagata ccagataaaa cctctgaaaa gcacagacga       720
   gaaagaacat gccgtcttta catctaacca ggaggaacaa gacccagcta accacacatg       780
   tggtgtgaag agcactgacg ggaaacaagg cccaattcga atctctagat cactcaaaag       840
   cccagagaaa gaagactttc ttcgggcaca gaaatacatt gatctctatt tggtgctgga       900
   taatgccttt tataagaact ataatgagaa tctaactctg ataagaagct ttgtgtttga       960
   tgtgatgaac ctactcaatg tgatatataa caccatagat gttcaagtgg ccttggtagg      1020
   tatggaaatc tggtctgatg gggataagat aaaggtggtg cccagcgcaa gcaccacgtt 1080
   tgacaacttc ctgagatggc acagttctaa cctggggaaa aagatccacg accatgctca 1140
   gcttctcagc gggattagct tcaacaatcg acgtgtggga ctggcagctt caaattcctt 1200
   gtgttcccca tcttcggttg ctgttattga ggctaaaaaa aagaataatg tggctcttgt 1260
   aggagtgatg tcacatgagc tgggccatgt ccttggtatg cctgatgttc cattcaacac 1320
   caagtgtccc tctggcagtt gtgtgatgaa tcagtatctg agttcaaaat tcccaaagga 1380
   tttcagtaca tcttgccgtg cacattttga aagatacctt ttatctcaga aaccaaagtg 1440
   cctgctgcaa gcacctattc ctacaaatat aatgacaaca ccagtgtgtg ggaaccacct 1500
   tctagaagtg ggagaagact gtgattgtgg ctctcctaag gagtgtacca atctctgctg 1560
   tgaagcccta acgtgtaaac tgaagcctgg aactgattgc ggaggagatg ctccaaacca 1620
   taccacagag tgaatccaaa agtctgcttc actgagatgc taccttgcca ggacaagaac 1680
   caagaactct aactgtccca ggaatcttgt gaattttcac ccataatggt ctttcacttg 1740
   tcattctact ttctatattg ttatcagtcc aggaaacagg taaacagatg taattagaga 1800
   cattggctct ttgtttaggc ctaatctttc tttttacttt tttttttctt ttttcttttt 1860
   ttttaaagat catgaatttg tgacttagtt ctgccctttg gagaacaaaa gaaagcagtc 1920
   ttccatcaaa tcaccttaaa atgcacggct aaactattca gagttaacac tccagaattg 1980
   ttaaattaca agtactatgc tttaatgctt ctttcatctt actagtatgg cctataaaaa 2040
   aaataatacc acttgatggg tgaaggcttt ggcaatagaa agaagaatag aattcaggtt 2100
   ttatgttatt cctctgtgtt cacttcgcct tgctcttgaa agtgcagtat ttttctacat 2160
   catgtcaaga atgattcaat gtaaatattt ttcattttat catgtatatc ctatacacac 2220
   atctccttca tcatcatata tgaagtttat tttgagaagt ctacattgct tacattttaa 2280
   ttgagccagc aaagaaggct taatgattta ttgaaccata atgtcaataa aaacacaact 2340
   tttgaggc 2348
<210> 98
   <211> 2517
   <212> DNA
   <213> Homo sapiens
<400> 98
   aaaacactca ttttgtttta tagcatgaca ggctgtctga ttccatcttt ataaccaaag      60
   ccaattaaga tcttaaaacc aaacatataa cttcatcttt ttacaagtac ttagagcctg     120
   agttgctcca caggaatcca ggaactgggc acaggaaaag gatctaagct ggtggtgtgg     180
   gaagatggaa accaacttct ccattcctct gaatgaaact gaggaggtgc tccctgagcc     240
   tgctggccac accgttctgt ggatcttctc attgctagtc cacggagtca cctttgtctt     300
   cggggtcctg ggcaatgggc ttgtgatctg ggtggctgga ttccggatga cacgcacagt 360
   caacaccatc tgttacctga acctggccct agctgacttc tctttcagtg ccatcctacc 420
   attccgaatg gtctcagtcg ccatgagaga aaaatggcct tttggctcat tcctatgtaa 480
   gttagttcat gttatgatag acatcaacct gtttgtcagt gtctacctga tcaccatcat 540
   tgctctggac cgctgtattt gtgtcctgca tccagcctgg gcccagaacc atcgcaccat 600
   gagtctggcc aagagggtga tgacgggact ctggattttc accatagtcc ttaccttacc 660
   aaatttcatc ttctggacta caataagtac tacgaatggg gacacatact gtattttcaa 720
   ctttgcattc tggggtgaca ctgctgtaga gaggttgaac gtgttcatta ccatggccaa 780
   ggtctttctg atcctccact tcattattgg cttcagcgtg cctatgtcca tcatcacagt 840
   ctgctatggg atcatcgctg ccaaaattca cagaaaccac atgattaaat ccagccgtcc 900
   cttacgtgtc ttcgctgctg tggtggcttc tttcttcatc tgttggttcc cttatgaact 960
   aattggcatt ctaatggcag tctggctcaa agagatgttg ttaaatggca aatacaaaat 1020
   cattcttgtc ctgattaacc caacaagctc cttggccttt tttaacagct gcctcaaccc 1080
   aattctctac gtctttatgg gtcgtaactt ccaagaaaga ctgattcgct ctttgcccac 1140
   tagtttggag agggccctga ctgaggtccc tgactcagcc cagaccagca acacagacac 1200
   cacttctgct tcacctcctg aggagacgga gttacaagca atgtgaggtc ggggatattt 1260
   ttgggctctg tctctttcta ccctgcgtta agcggaaaaa aaaaattctg acagtgtttt 1320
   tcttcctctt tcataccacc accaccacaa tcatcaacat aaaggaagtc tgtaccaaat 1380
   ctgtaggggg tttttcccac aaccaagcaa tagacaccag ctgggtgtcc tacaattaaa 1440
   ttccaacact atctacctgg agctactgtc agatcccaca ggtttaaggg ctcattcccc 1500
   aagtctgctc ctccagttga gacacaagtc acaaatccag gcttctgaga cttcggacca 1560
   accagcttca atcagggttc ccactacccc ctctttgggg gtagagtggc tcatggaact 1620
   cagagaaaca tttatttcgg cttgctggtt tattataaaa gcaaggttta ttataaaaga 1680
   tactacaaag gatacagatg aagaggcaca tagggcaagg tacggggttc cacgccctcc 1740
   ctgagtgcat caccctctgg gaacctccgt gtgttcacgt ctcatgaagc tctccaaatc 1800
   cagtcctctt gggtttttat ggaagcttca tgatgtcagc attctttcct ccagtgtata 1860
   ggatgggatc ctctctgggg agggtcttaa gacccacaat tagaaaggca agggaagatt 1920
   agagtcctgc tttggggtag atgaaaggaa aggagagaga ttctgtttcc tgaggcttaa 1980
   tacacccaac attataacaa aggactgtag caagggctat gggagttctg aagcagaaac 2040
   catgggctaa aaccaacata catcttaata ccagataccc taatcccagt cctaacttca 2100
   tttaaccttg gtcacattga gtcattccag gatgagtggc tcaagtattt cctcagggaa 2160
   aatacttctg tgccccctga tttgagggta agaagtagat aatgaggcca ctgtgggtgt 2220
   tattttttca tgtctggacc tcagcctata tcctgagact aagtggaagt gggaaaagag 2280
   tacaagagaa gagacaaagt ggggatattt gtaaggctta gatgagatag tgttttttta 2340
   gaaaaaaact ttatcttacc attaagtaaa atgtttgcca taggctttct ggggctttct 2400
   ctttttaaag tcagactgtt gaaggtttct tctattctta tttgttaaga gttttctttt 2460
   attgtttaaa tcatgaatga atgttgaatt ttattaaatg cagtttctgt aaatatt 2517
<210> 99
   <211> 2790
   <212> DNA
   <213> Homo sapiens
<400> 99
   ccctttctgt atttgagttc taccgtcagt cctggcatta tttctctctc tacaaggagc      60
   cttaggaggt acggggagct cgcaaatact ccttttggtt tattcttacc accttgcttc     120
   tgtgttcctt gggaatgctg ctgtgcttat gcatctggtc tctttttgga gctacagtgg     180
   acaggcattt gtgacagcac tatgggactg agtaacattc tctttgtgat ggccttcctg     240
   ctctctggtg ctgctcctct gaagattcaa gcttatttca atgagactgc agacctgcca     300
   tgccaatttg caaactctca aaaccaaagc ctgagtgagc tagtagtatt ttggcaggac     360
   caggaaaact tggttctgaa tgaggtatac ttaggcaaag agaaatttga cagtgttcat     420
   tccaagtata tgggccgcac aagttttgat tcggacagtt ggaccctgag acttcacaat     480
   cttcagatca aggacaaggg cttgtatcaa tgtatcatcc atcacaaaaa gcccacagga     540
   atgattcgca tccaccagat gaattctgaa ctgtcagtgc ttgctaactt cagtcaacct     600
   gaaatagtac caatttctaa tataacagaa aatgtgtaca taaatttgac ctgctcatct     660
   atacacggtt acccagaacc taagaagatg agtgttttgc taagaaccaa gaattcaact     720
   atcgagtatg atggtattat gcagaaatct caagataatg tcacagaact gtacgacgtt     780
   tccatcagct tgtctgtttc attccctgat gttacgagca atatgaccat cttctgtatt     840
   ctggaaactg acaagacgcg gcttttatct tcacctttct ctatagagct tgaggaccct     900
   cagcctcccc cagaccacat tccttggatt acagctgtac ttccaacagt tattatatgt     960
   gtgatggttt tctgtctaat tctatggaaa tggaagaaga agaagcggcc tcgcaactct    1020
   tataaatgtg gaaccaacac aatggagagg gaagagagtg aacagaccaa gaaaagagaa    1080
   aaaatccata tacctgaaag atctgatgaa gcccagcgtg tttttaaaag ttcgaagaca    1140
   tcttcatgcg acaaaagtga tacatgtttt taattaaaga gtaaagccca tacaagtatt    1200
   catttttctt accctttcct ttgtaagttc ctgggcaacc tttttgattt cttccagaag    1260
   gcaaaaagac attaccatga gtaataaggg ggctccagga ctccctctaa gtggaatagc    1320
   ctccctgtaa ctccagctct gctccgtatg ccaagaggag actttaattc tcttactgct 1380
   tcttttcact tcagagcaca cttatgggcc aagcccagct taatggctca tgacctggaa 1440
   ataaaattta ggaccaatac ctcctccaga tcagattctt ctcttaattt catagattgt 1500
   gttttttttt taaatagacc tctcaatttc tggaaaactg ccttttatct gcccagaatt 1560
   ctaagctggt gccccactga attttgtgta cctgtgacta aacaactacc tcctcagtct 1620
   gggtgggact tatgtattta tgaccttata gtgttaatat cttgaaacat agagatctat 1680
   gtactgtaat agtgtgatta ctatgctcta gagaaaagtc tacccctgct aaggagttct 1740
   catccctctg tcagggtcag taaggaaaac ggtggcctag ggtacaggca acaatgagca 1800
   gaccaaccta aatttgggga aattaggaga ggcagagata gaacctggag ccacttctat 1860
   ctgggctgtt gctaatattg aggaggcttg ccccacccaa caagccatag tggagagaac 1920
   tgaataaaca ggaaaatgcc agagcttgtg aaccctgttt ctcttgaaga actgactagt 1980
   gagatggcct ggggaagctg tgaaagaacc aaaagagatc acaatactca aaagagagag 2040
   agagagaaaa aagagagatc ttgatccaca gaaatacatg aaatgtctgg tctgtccacc 2100
   ccatcaacaa gtcttgaaac aagcaacaga tggatagtct gtccaaatgg acataagaca 2160
   gacagcagtt tccctggtgg tcagggaggg gttttggtga tacccaagtt attgggatgt 2220
   catcttcctg gaagcagagc tggggaggga gagccatcac cttgataatg ggatgaatgg 2280
   aaggaggctt aggactttcc actcctggct gagagaggaa gagctgcaac ggaattagga 2340
   agaccaagac acagatcacc cggggcttac ttagcctaca gatgtcctac gggaacgtgg 2400
   gctggcccag catagggcta gcaaatttga gttggatgat tgtttttgct caaggcaacc 2460
   agaggaaact tgcatacaga gacagatata ctgggagaaa tgactttgaa aacctggctc 2520
   taaggtggga tcactaaggg atggggcagt ctctgcccaa acataaagag aactctgggg 2580
   agcctgagcc acaaaaatgt tcctttattt tatgtaaacc ctcaagggtt atagactgcc 2640
   atgctagaca agcttgtcca tgtaatattc ccatgttttt accctgcccc tgccttgatt 2700
   agactcctag cacctggcta gtttctaaca tgttttgtgc agcacagttt ttaataaatg 2760
   cttgttacat tcatttaaaa aaaaaaaaaa 2790
<210> 100
   <211> 2312
   <212> DNA
   <213> Homo sapiens
<400> 100
   ctccacaggt ccgccccaat ccccgctcac acttgggaaa cttgggactg cgctggggcc 60
   gcgtgtggca cctcaggggg gcggcccccg gcctcaagag gagggggagg agaaggagga 120
   agaggaggaa gtgagcccga aggatccgct cggagctgtt tgtccagctg tttctattcg 180
   cacccggagc agtacagcca gaagggggcc gagccgaagg tggctggctt taggcgctaa 240
   tttccaactc ttttcctcac agcttgtctt ttccaggcac cctggagtcc cctcaggcca 300
   gctcggtggg cgcgcacctg ccagccgccc ctgacctcgc aggccaggcg acctccgagc 360
   ctgagaagat ggcccagtcc aagctcgatt gccgctcacc tgtcggcctc gactgctgca 420
   actgctgcct ggacctggcc catcggagtg ggctccagcg aggcagcagc ggggagaaca 480
   acaacccggg cagccctaca gtgagcaact ttcggcagct gcaggaaaag ctggtctttg 540
   agaacctcaa taccgacaag ctcaacagca taatgcggca ggattcgcta gagccggtgc 600
   tgcgggaccc ctgctacctg atcaacgagg gcatctgcaa ccgcaacatc gaccagacca 660
   tgctctccat cctgctcttc ttccacagtg cctccggagc cagcgtggtg gccatagaca 720
   acaagatcga acaggccatg gatctggtga agaatcatct gatgtatgct gtgagagagg 780
   aggtggagat cctgaaggag cagatccgag agctggtgga gaagaactcc cagctagagc 840
   gtgagaacac cctgttgaag accctggcaa gcccagagca gctggagaag ttccagtcct 900
   gtctgagccc tgaagagcca gctcccgaat ccccacaagt gcccgaggcc cctggtggtt 960
   ctgcggtgta agtggctctg tcctcagggt gggcagagcc actaaacttg ttttacctag 1020
   ttctttccag tttgtttttg gctccccaag catcatctca cgaggagaac tttacaccta 1080
   gcacagctgg tgccaagaga tgtcctaagg acatggccac ctgggtccac tccagcgaca 1140
   gacccctgac aagagcaggt ctctggaggc tgagttgcat ggggcctagt aacaccaagc 1200
   cagtgagcct ctaatgctac tgcgccctgg gggctcccag ggcctgggca acttagctgc 1260
   aactggcaaa ggagaagggt agtttgaggt gtgacaccag tttgctccag aaagtttaag 1320
   gggtctgttt ctcatctcca tggacatctt caacagcttc acctgacaac gactgttcct 1380
   atgaagaagc cacttgtgtt ttaagcagag gcaacctctc tcttctcctc tgtttcgtga 1440
   aggcagggga cacagatggg agagattgag ccaagtcagc cttctgttgg ttaatatggt 1500
   ataatgcatg gctttgtgca cagcccagtg tgggattaca gctttgggat gaccgcttac 1560
   aaagttctgt ttggttagta ttggcatagt ttttctatat agccataaat gcgtatatat 1620
   acccataggg ctagatctgt atcttagtgt agcgatgtat acatatacac atccacctac 1680
   atgttgaagg gcctaaccag ccttgggagt attgactggt cccttacctc ttatggctaa 1740
   gtctttgact gtgttcattt accaagttga cccagtttgt cttttaggtt aagtaagact 1800
   cgagagtaaa ggcaaggagg ggggccagcc tctgaatgcg gccacggatg ccttgctgct 1860
   gcaacccttt ccccagctgt ccactgaaac gtgaagtcct gttttgaatg ccaaacccac 1920
   cattcactgg tgctgactac atagaatggg gttgagagaa gatcagtttg ggcttcacag 1980
   tgtcatttga aaacgttttt tgttttgttt tgtaattatt gtggaaaact ttcaagtgaa 2040
   cagaaggatg gtgtcctact gtggatgagg gatgaacaag ggatggcttt tgatccaatg 2100
   gagcctggga ggtgtgccca gaaagcttgt ctgtagcggg ttttgtgaga gtgaacactt 2160
   tccacttttt gacaccttat cctgatgtat ggttccagga tttggatttt gattttccaa 2220
   atgtagcttg aaatttcaat aaactttgct ctgtttttct aaaaataaaa aaaaaaaaaa 2280
   aaaaaaaaaa aaaaaaaaaa aaaaaaaaaa aa 2312
<210> 101
   <211> 1944
   <212> DNA
   <213> Homo sapiens
<400> 101
<210> 102
   <211> 5266
   <212> DNA
   <213> Homo sapiens
<400> 102
   gcttaaaaat ttctgtgtct tacacagaag atagaaaaaa tagagtgtct ccaattggat      60
   ggatttttta aaaaatttgg ttattgtaat ggatttattt tttcttagag ctgagctgat     120
   tgtactttgg ccaactaatg ggttaatact gtcaagggaa attagccctg actaaacatt     180
   gccgctggct catgaatgca ctaggcttgg ggcagtataa aaactcagag aaatcagtgt     240
   gtaggagaca cagaaatcag tgtcactcag tgacagaagc aacaataatt gtgaaaaata     300
   cttcagcagt tatggactca tctgtcattc aaaggaaaaa agtagctgtc attggtggtg     360
   gcttggttgg ctcattacaa gcatgctttc ttgcaaagag gaatttccag attgatgtat     420
   atgaagctag ggaagatact cgagtggcta ccttcacacg tggaagaagc attaacttag     480
   ccctttctca tagaggacga caagccttga aagctgttgg cctggaagat cagattgtat     540
   cccaaggtat tcccatgaga gcaagaatga tccactctct ttcaggaaaa aagtctgcaa     600
   ttccctatgg gacaaagtct cagtatattc tttctgtaag cagagaaaat ctaaacaagg     660
   atctattgac tgctgctgag aaatacccca atgtgaaaat gcactttaac cacaggctgt     720
   tgaaatgtaa tccagaggaa ggaatgatca cagtgcttgg atctgacaaa gttcccaaag     780
   atgtcacttg tgacctcatt gtaggatgtg atggagccta ttcaactgtc agatctcacc     840
   tgatgaagaa acctcgcttt gattacagtc agcagtacat tcctcatggg tacatggagt     900
   tgactattcc acctaagaac ggagattatg ccatggaacc taattatctg catatttggc     960
   ctagaaatac ctttatgatg attgcacttc ctaacatgaa caaatcattc acatgtactt    1020
   tgttcatgcc ctttgaagag tttgaaaaac ttctaaccag taatgatgtg gtagatttct    1080
   tccagaaata ctttccggat gccatccctc taattggaga gaaactccta gtgcaagatt    1140
   tcttcctgtt gcctgcccag cccatgatat ctgtaaagtg ctcttcattt cactttaaat 1200
   ctcactgtgt actgctggga gatgcagctc atgctatagt gccgtttttt gggcaaggaa 1260
   tgaatgcggg ctttgaagac tgcttggtat ttgatgagtt aatggataaa ttcagtaacg 1320
   accttagttt gtgtcttcct gtgttctcaa gattgagaat cccagatgat cacgcgattt 1380
   cagacctatc catgtacaat tacatagaga tgcgagcaca tgtcaactca agctggttca 1440
   tttttcagaa gaacatggag agatttcttc atgcgattat gccatcgacc tttatccctc 1500
   tctatacaat ggtcactttt tccagaataa gataccatga ggctgtgcag cgttggcatt 1560
   ggcaaaaaaa ggtgataaac aaaggactct tttcttgggg atcactgata gccatcagca 1620
   gtacctacct acttatacac tacatgtcac cacgatcttt cctccgcttg agaagaccat 1680
   ggaactggat agctcacttc cggaatacaa catgtttccc cgcaaaggcc gtggactccc 1740
   tagaacaaat ttccaatctc attagcaggt gatagaaagg ttttgtggta gcaaatgcat 1800
   gatttctctg tgaccaaaat taagcatgaa aaaaatgttt ccattgccat atttgattca 1860
   ctagtggaag atagtgttct gcttataatt aaactgaatg tagagtatct ctgtatgtta 1920
   attgcaatta ctggttgggg ggtgcatttt aaaagatgaa acatgcagct tccctacatt 1980
   acacacactc aggttgagtc attctaacta taaaagtgca atgactaaga tccttcactt 2040
   ctctgaaagt aaggccctag atgcctcagg gaagacagta atcatgcctt ttctttaaaa 2100
   gacacaatag gactcgcaac agcattgact caacacctag gactaaaaat cacaacttaa 2160
   ctagcatgtt aactgcactt ttcattacgt gaatggaact tacctaacca cagggctcag 2220
   acttactaga taaaaccaga aatggaaata aggaattcag gggagttcca gagacttaca 2280
   aaatgaactc attttatttt cccaccttca aatataagta ttatcatcta tctgtttatc 2340
   gtctatctat ctatcatcta tctatctatc tatcatctat ctatctatct atctatctat 2400
   ctatctatct atctatctct atttatttat gtatttagag atcaggtctc actctgttga 2460
   ccaggctgga gtgcagtggt gagatctggg ttcactgcaa cctctgcctc ctgggctcaa 2520
   gcaatcctcc cacttcagcc tcccaaatag ctggggctac catggtattt ttcagtagag 2580
   accgggtctt gccatgctgc ccaggccagt ctcaaactcc tggcctcatg tgatctgccc 2640
   acctcagcct cccaaagtac agggattaga gttgtgagcc accgctgcca gcccagagtt 2700
   accctctaaa gataagaaaa aggctattaa tatcatacta agtgaaggac aggaaagggt 2760
   tttattcata aattaaatgt ctacatgtgc cagaatggaa aggaaacaag gggagacaac 2820
   ttttatagaa atacaaagcc attactttat tcaatttcag accctcagaa gcaatttact 2880
   aatttattct tcgactacat actgcagcag aaccagcaat acacttgatt tttaaaagca 2940
   catttagtga aatgttttct ttggttcatc cttctttaac aggctgctga gtcactcaga 3000
   aatccttcaa acatgattaa ttatgaagat gaaacactag agtcatataa gaaataaaaa 3060
   ttgggcaata aaataaaatg attcagtgtt tcttttctat attgtcaatg aaaaccttga 3120
   gttctaataa tccatgttca gtttgtaggg aaagaaaaaa taatttttcc ttctacccac 3180
   tttaggttcc ttggctgggg cccctataac aaaagacaga ttgacaagag aaaaacaaac 3240
   ataaatttat tagcgggtat atgtaatata tatgtgggaa atacagggga atgagcaaat 3300
   ctcaaagagc tggcgtctta gaactccctg gcttatatag catcgacaaa gaacagtaaa 3360
   tttttagaga aacaacaaaa caaagaaaaa gagctttgag tctgtagggg cagcaatttg 3420
   ggggaagcaa atatatggga gtttgccttg tagattcctc tggtgctggt ctccaggctg 3480
   acaaggattc aaagttgtct ctgaaactcc tctttgtcat actgcacata taaaacgtct 3540
   tttgtttcca acaagaggat tttctttttc attctagaat tatctccttg ataacttgat 3600
   cagatatagg acatgacact gaatagagtc caacagtaca aaaaaaattc agtatgttct 3660
   agctacttca cacatgtgta cgcgacagtt atttttacag taaggtattt tcgagaaaaa 3720
   tgcattacgt gttttggaaa atagagtaat ttaaaaaata tatttgaaat gaaaatctcc 3780
   aacacattag aagatgatga tgttagatgc ccatcgtgtg ccacaagtgg ttttttcatt 3840
   atgtaaagca cccgttgaat taaaagaatt tgtttttgtt caacctcttc ctgaggccca 3900
   agagcatatg ggcaattcgg atttcctgct ggaccacaag gttctgttga tattacatag 3960
   aacgggtatt ccagacactt cttatgatga aagtccaaaa gtggcatcca atttaaggcc 4020
   ccatctttcg ttgccattct tcattcctac aaaggacgaa cttggattac atcaactttg 4080
   gacccattgg ttttgtcgct gtcgtcaact gacagtgatt catcactggt gatgataaaa 4140
   atgatggaag aagagttgaa agtcactttt ttctttggcc tgtccccatc tttctgtgac 4200
   atcacaatgg gtctgatctg catttcactt ccagctgctg gtaggtcttt agcaggcctc 4260
   tggcacctca gcagtcggag gcacagaagc tgcaaaaggg atcttcgaaa ctgggcagag 4320
   aaaaaataaa gtggaatatt aagtaaaagt tgggcactaa tctggattaa cattcgagga 4380
   aatcagttga gctgaattta agttgttttt tgtttgttag caggtgtgga tgtggggtta 4440
   tgtggtcatg ctcagatcta cctaaatcac cccagagctt tatgtctttt attcattcta 4500
   attcttatta accggaatat gtaggaccat ttcaatacct tgtaatcctc caagcttcaa 4560
   tctgcacaca ctttctatga gggcaggtac aactattaag agattttgaa cattaagtta 4620
   gtccacaaat attcagtggg catctactag gtgacagcca ctgtgctata attagagact 4680
   ttttactata agcatcaaaa acagataagg ctcttcctgg cagagtttac agcctggtgt 4740
   acttgctaat gtctctttaa ttaggtgaag aatttttttt ttctatcgaa attactaatc 4800
   agttggggaa aaaaatacta tagcagacag cactaatgtc atcaacaaac attgttcttc 4860
   tccgtgtcct gggtacaaca tcgaataata tttcttggcc tcctttccgc ttctcctctc 4920
   tgctgttcct ctctacaaga acctgggagg ccaacgccta aagatcataa tatcacacaa 4980
   tggaaggaac ctagattcct aaatgactgc ataggacaga tcccatctcc tccacccaat 5040
   acattattag actgaactgt gacctgaaat gagcaataaa ctctgtatta attcactgaa 5100
   atgttggggt tgcttgttat agtagtcggt ccatcatgac cagtaaaaca taaatcaaaa 5160
   gttaatgtaa ttgttatccc attatttaga gcgaaataaa tgttgaatat atggactttc 5220
   tcagattagg aaataccaat taaaaatata ataaatagct acattg 5266
<210> 103
   <211> 2217
   <212> DNA
   <213> Homo sapiens
<400> 103
   ccccgccgcc gccgcccttc gcgccctggg ccatctccct cccacctccc tccgcggagc 60
   agccagacag cgagggcccc ggccgggggc aggggggacg ccccgtccgg ggcacccccc 120
   cggctctgag ccgcccgcgg ggccggcctc ggcccggagc ggaggaagga gtcgccgagg 180
   agcagcctga ggccccagag tctgagacga gccgccgccg cccccgccac tgcggggagg 240
   agggggagga ggagcgggag gagggacgag ctggtcggga gaagaggaaa aaaacttttg 300
   agacttttcc gttgccgctg ggagccggag gcgcggggac ctcttggcgc gacgctgccc 360
   cgcgaggagg caggacttgg ggaccccaga ccgcctccct ttgccgccgg ggacgcttgc 420
   tccctccctg ccccctacac ggcgtccctc aggcgccccc attccggacc agccctcggg 480
   agtcgccgac ccggcctccc gcaaagactt ttccccagac ctcgggcgca ccccctgcac 540
   gccgccttca tccccggcct gtctcctgag cccccgcgca tcctagaccc tttctcctcc 600
   aggagacgga tctctctccg acctgccaca gatcccctat tcaagaccac ccaccttctg 660
   gtaccagatc gcgcccatct aggttatttc cgtgggatac tgagacaccc ccggtccaag 720
   cctcccctcc accactgcgc ccttctccct gaggacctca gctttccctc gaggccctcc 780
   taccttttgc cgggagaccc ccagcccctg caggggcggg gcctccccac cacaccagcc 840
   ctgttcgcgc tctcggcagt gccggggggc gccgcctccc ccatgccgcc ctccgggctg 900
   cggctgctgc cgctgctgct accgctgctg tggctactgg tgctgacgcc tggccggccg 960
   gccgcgggac tatccacctg caagactatc gacatggagc tggtgaagcg gaagcgcatc 1020
   gaggccatcc gcggccagat cctgtccaag ctgcggctcg ccagcccccc gagccagggg 1080
   gaggtgccgc ccggcccgct gcccgaggcc gtgctcgccc tgtacaacag cacccgcgac 1140
   cgggtggccg gggagagtgc agaaccggag cccgagcctg aggccgacta ctacgccaag 1200
   gaggtcaccc gcgtgctaat ggtggaaacc cacaacgaaa tctatgacaa gttcaagcag 1260
   agtacacaca gcatatatat gttcttcaac acatcagagc tccgagaagc ggtacctgaa 1320
   cccgtgttgc tctcccgggc agagctgcgt ctgctgaggc tcaagttaaa agtggagcag 1380
   cacgtggagc tgtaccagaa atacagcaac aattcctggc gatacctcag caaccggctg 1440
   ctggcaccca gcgactcgcc agagtggtta tcttttgatg tcaccggagt tgtgcggcag 1500
   tggttgagcc gtggagggga aattgagggc tttcgcctta gcgcccactg ctcctgtgac 1560
   agcagggata acacactgca agtggacatc aacgggttca ctaccggccg ccgaggtgac 1620
   ctggccacca ttcatggcat gaaccggcct ttcctgcttc tcatggccac cccgctggag 1680
   agggcccagc atctgcaaag ctcccggcac cgccgagccc tggacaccaa ctattgcttc 1740
   agctccacgg agaagaactg ctgcgtgcgg cagctgtaca ttgacttccg caaggacctc 1800
   ggctggaagt ggatccacga gcccaagggc taccatgcca acttctgcct cgggccctgc 1860
   ccctacattt ggagcctgga cacgcagtac agcaaggtcc tggccctgta caaccagcat 1920
   aacccgggcg cctcggcggc gccgtgctgc gtgccgcagg cgctggagcc gctgcccatc 1980
   gtgtactacg tgggccgcaa gcccaaggtg gagcagctgt ccaacatgat cgtgcgctcc 2040
   tgcaagtgca gctgaggtcc cgccccgccc cgccccgccc cggcaggccc ggccccaccc 2100
   cgccccgccc ccgctgcctt gcccatgggg gctgtattta aggacacccg tgccccaagc 2160
   ccacctgggg ccccattaaa gatggagaga ggactgcgga aaaaaaaaaa aaaaaaa 2217
<210> 104
   <211> 1629
   <212> DNA
   <213> Homo sapiens
<400> 104
   acacatcagg ggcttgctct tgcaaaacca aaccacaaga cagacttgca aaagaaggca      60
   tgcacagctc agcactgctc tgttgcctgg tcctcctgac tggggtgagg gccagcccag     120
   gccagggcac ccagtctgag aacagctgca cccacttccc aggcaacctg cctaacatgc     180
   ttcgagatct ccgagatgcc ttcagcagag tgaagacttt ctttcaaatg aaggatcagc     240
   tggacaactt gttgttaaag gagtccttgc tggaggactt taagggttac ctgggttgcc     300
   aagccttgtc tgagatgatc cagttttacc tggaggaggt gatgccccaa gctgagaacc     360
   aagacccaga catcaaggcg catgtgaact ccctggggga gaacctgaag accctcaggc     420
   tgaggctacg gcgctgtcat cgatttcttc cctgtgaaaa caagagcaag gccgtggagc     480
   aggtgaagaa tgcctttaat aagctccaag agaaaggcat ctacaaagcc atgagtgagt     540
   ttgacatctt catcaactac atagaagcct acatgacaat gaagatacga aactgagaca     600
   tcagggtggc gactctatag actctaggac ataaattaga ggtctccaaa atcggatctg     660
   gggctctggg atagctgacc cagccccttg agaaacctta ttgtacctct cttatagaat     720
   atttattacc tctgatacct caacccccat ttctatttat ttactgagct tctctgtgaa     780
   cgatttagaa agaagcccaa tattataatt tttttcaata tttattattt tcacctgttt     840
   ttaagctgtt tccatagggt gacacactat ggtatttgag tgttttaaga taaattataa     900
   gttacataag ggaggaaaaa aaatgttctt tggggagcca acagaagctt ccattccaag     960
   cctgaccacg ctttctagct gttgagctgt tttccctgac ctccctctaa tttatcttgt    1020
   ctctgggctt ggggcttcct aactgctaca aatactctta ggaagagaaa ccagggagcc    1080
   cctttgatga ttaattcacc ttccagtgtc tcggagggat tcccctaacc tcattcccca    1140
   accacttcat tcttgaaagc tgtggccagc ttgttattta taacaaccta aatttggttc    1200
   taggccgggc gcggtggctc acgcctgtaa tcccagcact ttgggaggct gaggcgggtg    1260
   gatcacttga ggtcaggagt tcctaaccag cctggtcaac atggtgaaac cccgtctcta    1320
   ctaaaaatac aaaaattagc cgggcatggt ggcgcgcacc tgtaatccca gctacttggg    1380
   aggctgaggc aagagaattg cttgaaccca ggagatggaa gttgcagtga gctgatatca    1440
   tgcccctgta ctccagcctg ggtgacagag caagactctg tctcaaaaaa taaaaataaa    1500
   aataaatttg gttctaatag aactcagttt taactagaat ttattcaatt cctctgggaa    1560
   tgttacattg tttgtctgtc ttcatagcag attttaattt tgaataaata aatgtatctt    1620
   attcacatc                                                            1629
<210> 105
   <211> 12
   <212> PRT
   <213> Artificial Sequence
<220>
   <223> peptide
<400> 105
   His His Leu Gly Gly Ala Lys Gln Ala Gly Asp Val
   1               5                   10

## Description

### FIELD OF THE INVENTION

The present invention relates to methods for producing immuno-suppressive dendritic cells. The present invention in particular relates to a method of preferentially producing immuno-suppressive dendritic cells relative to immuno-stimulatory dendritic cells

### BACKGROUND OF THE INVENTION

Dendritic cells (DC) are recognized to be potent antigen presenting cells for the initiation and control of cellular immunologic responses in humans. Since DC can either be immuno-stimulatory or immuno-suppressive, depending on which set of their potential properties they express at the moment of interaction with responsive specific clones of T cells, they are considered profoundly important pivotal players in T cell-mediated immune reactions. As a broad, but widely held generalization, immature DC are more "tolerogenic" than their more mature counterparts, while mature DC are thought to be more "immunogenic" than their immature precursors.

The capacity of DC, generated ex vivo from monocytes and armed with specific antigen, to function effectively in either immunologic direction, is dependent on their viability and vigor after being returned to the patient. It is logically concluded that the balance between counteractive immuno-stimulatory and immune-suppressive DC will be a major determinant of both the direction and potency of DC-dependent therapeutic immune responses.

The purpose of this invention is to facilitate production of DC populations particularly conducive to the generation of powerful and clinically relevant immune responses. Despite the tremendous promise of DC-based therapy, such as efforts to enhance anti-cancer immunity, clinical results have generally been disappointing. For example, Provenge, the recently first FDA-approved immunotherapy for a solid tumor, adapting the conventional method of ex vivo production of DC from blood monocytes, has yielded merely a four-month improvement in survival of patients with advanced prostate cancer. Further, Provenge has so far not been shown to diminish the size of the treated prostate cancer. That conventional method of inducing DC, as well as Extracorporeal Photopheresis (ECP), an FDA-approved therapy for the "liquid" malignancy Cutaneous T Cell Lymphoma (CTCL), is encumbered by production of relatively heterogeneous DC populations under conditions which handicap the in vivo vigor, and viability, of the resulting DC. By employing more physiologic conditions to the production of therapeutic DC, the present invention enables production of more maturationally synchronized DC, whose survival and vigor are not inhibited by factors inherent to the method by which they are produced. Moreover, this method is applicable to both human and animal leukocytes.

DC prime both CD8⁺ cytotoxic T-cell (CTL) and CD4⁺ T helper (Th1) responses. DC are capable of capturing and processing antigens and migrating to the regional lymph nodes to present the captured antigens and induce T-cell responses. In humans, DC are a relatively rare component of peripheral blood (<1 % of leukocytes). However, large quantities of DC can be differentiated by laboratory procedures from CD34⁺ precursors or blood monocytes.

For the afore-described properties, DC have been identified as an important cellular agent for eliciting effective anti-tumor immune responses. The idea is to generate dendritic cells, which present tumor-specific antigens on their MHC Class I and MHC Class II complex and can be (re)introduced into a patient to thereby launch an immune-attack against the tumor. However, generation of such immuno-stimulatory dendritic cells usually requires differentiation of CD34⁺ precursors or blood monocytes using complex and rather expensive cytokine cocktails. In those standard methods, the cytokines are employed at concentrations very much higher (often by orders of magnitude) than those encountered in vivo under physiological conditions. Therefore, one proffered reason for the overall disappointing clinical results from DC-based immuno-modulation is that DC produced by the common cytokine method may not function effectively at the far lower cytokine concentrations actually in patients. DC produced ex vivo at markedly supra-physiologic concentrations of growth factors, such as cytokines, are selected to be dependent on conditions which are reproduced in the in vivo environment in patients.

In addition to the afore-mentioned immuno-stimulatory dendritic cells, other types of dendritic cells, namely tolerogenic dendritic cells, which may also be designated as immuno-suppressive or immuno-inhibiting dendritic cells have been described. These types of dendritic cells play an important role in maintaining immune tolerance and they have been discussed for therapeutic settings such as solid-organ transplantation, Graft-versus-Host-disease, autoimmune diseases, and hypersensitivity diseases (see e.g. Hu et al. Immunology, (2010), 132, 307-314).

Classical Extracorporeal Photopheresis (ECP) has been used successfully to treat cutaneous T-cell lymphoma (CTCL) in subsets of patients. In ECP, patients suffering from CTCL receive the photoactivatable compound 8-methoxypsoralen (8-MOP).

Patients are then leukapheresed to obtain buffy coats and these buffy coats are passed through a continuous closed circuit ultraviolet exposure device to irradiate the leukapheresed buffy coats and thereby lethally damage exposed lymphocytes. In this manner, 8-MOP is induced to covalently crosslink base pairs of DNA. The concept of ECP is to destroy proliferating metastatic T-cells of CTCL and to then to intravenously re-introduce the dying cells to the patient. It has been learned that this process additionally leads to conversion of passaged blood monocytes to DC without the need for stimulation by addition of exogenous cytokines. These ECP-induced DC are furthermore assumed to internalize, process and display antigens from the tumor cells, which were destroyed by the combination of 8-MOP and UV irradiation. It has been hypothesized that reintroduction of these loaded dendritic cells to the patient account for at least part of the success of ECP when treating CTLC. In fact ECP-like processes, in which neither 8-MOP nor UV light irradiation are used, but in which extracorporeal blood sample comprising monocytes are passed under shear stress through an ECP device have also been assumed to initiate monocyte differentiation into DC.

However, it has also been found that the ECP or ECP-like process leads to truncated, i.e. immuno-suppressive or tolerogenic DC, likely contributing heavily to ECP's clinical efficacy in the treatment of Graft versus Host Disease which commonly follows post-bone marrow stem allotransplants. The precise mechanistic aspects of ECP on differentiation of monocytes into either immuno-stimulatory or immuno-suppressive DC have remained elusive (for review of the ECP process see Girardi et al. (2002), Transfusion and Apheresis Science, 26, 181-190).

WO 2011/137365 describes methods for treating blood monocytes to produce functional antigen presenting dendritic cells. An extracorporeal quantity of a subject's blood is treated with a photoactivatable agent and passed through a plastic treatment device which allows for transmittance of light to the interior of the plastic device. A light source then produces light of a wave length selected to activate the photoactivatable agent.

Spisek et al. (2006, Transfusion, 46, 55-65) report on the dynamics of dendritic cell activation during the process of photophoreses in patients treated for refractory Graft versus Host Disease.

Baird et al. (2011, Journal of Invenstigative Dermatology, 130, S136) describes the treatment of normal donor peripheral blood mononuclear cells (PBMC) in an in vitro model of the ECP apparatus. Phenotype and functionality of the generated cells was investigated using two-color flow cytometry and functional assays.

Hu et al. (2011, Immunology, 132, 307-314) describes tolerogenic dendritic cells and their potential applications.

The present ECP and ECP-like processes are thus conceived to lead to complex mixtures of immuno-stimulatory and immuno-suppressive DC. Of course, from *inter alia* a clinical perspective, it would be important to understand how the ECP and ECP-like processes can be modified to to overcome these limitations and how one can obtain purposively and selectively preferentially immuno-suppressive relative to immuno-stimulatory DC and vice versa. Further, the classical ECP process is, in principle an in vivo method as the obtained dendritic cell mixtures are reinfused into the patient. It would, however, be desirable to have methods available that allow preferential production of immuno-suppressive over immuno- stimulatory DC and vice versa outside the human or animal body.

Thus, there is a continuing need for methods that allow predictable and reproducible production of individual-specific, i.e. autologous immuno-suppressive dendritic cells, which upon re-introduction into a patient allow for treatment of e.g. autoimmune disease such as multiple sclerosis or Graft-versus-Host disease.

### OBJECTIVES AND SUMMARY OF THE INVENTION

One objective of the present invention is to provide methods for producing immuno-suppressive dendritic cells.

Another objective of the present invention is to provide methods for producing immuno-suppressive dendritic cells in an extracorporeal amount of blood obtained from a patient.

These and other objectives as they will become apparent from the ensuing description hereinafter are solved by the subject matter of the independent claims. Some of the preferred embodiments of the present invention form the subject matter of the dependent claims. Yet other embodiments of the present invention may be taken from the ensuing description.

The present invention is based to some extent on data presented hereinafter, which for a miniaturized and scalable device allowed (i) to mimic some aspects of the classical ECP procedure and (ii) to elucidate the cellular and molecular mechanism and biophysical conditions of induction of differentiation of monocytes into immuno-stimulatory autologous dendritic cells in an extracorporeal amount of blood. This data shows that the activation of platelets and binding of monocytes to such activated platelets under conditions of shear force is essential for obtaining immuno-stimulatory autologous dendritic cells. As is shown by the experiments described hereinafter, these immuno-stimulatory autologous dendritic cells can be characterized by expression of molecular markers indicative of immuno-stimulatory autologous dendritic cells. The data also shows that conditions that lead to an increased expression of Glucocorticoid-induced Leucine Zipper (GILZ) will favorably allow monocytes to differentiate into immuno-suppressive dendritic cells. These immuno-suppressive dendritic cells, which show an increased expression of GILZ as consequence of treating monocyte-derived immature dendritic cells with 8-MOP and exposing to UVA, will show an even stronger expression of GILZ upon contact with apoptotic leukocytes. Further, such dendritic cells show increased expression of IL-10 and decrease production of various pro-inflammatory cytokines and chemokines as can be taken form a reduced IL-10 to IL-12p70 ratio. These findings thus allow for a rationalized approach to obtain immuno-suppressive dendritic cells by thus carefully selecting the properties of the devices to be used and the parameters of the process. The findings of the present invention allows preferential production of immuno-suppressive dendritic cells relative to immuno-stimulatory dendritic cells and thus overcome the limitations of the classical ECP procedure because, in the classical ECP, procedure the lack of understanding what type of dendritic cells are produced and how their production can be manipulated to some extent prevents the extension of using this method for other than the authorized applications (see Girardi et al. (2002), Transfusion and Apheresis Science, 26, 181-190). Moreover, other than for the classical ECP procedure as used in the device obtainable from Therakos, the present invention allows to obtain such immuno-stimulatory dendritic cells in an experimental setting, where the extracorporeal amount of blood is not in a continous connection with the body. The data *inter alia* suggests that the process of obtaining immuno-stimulatory dendritic cells includes a global monocyte activation step and a subsequent monocyte to immuno-stimulatory antigen-presenting cell (e.g. dendritic cell) differentiation step. These steps seem to be initially dependent on physical activation of monocytes with the physical forces occurring during e.g. initial purification or enrichment of monocytes being sufficient for activation even though passage of e.g. initially activated monocytes through devices as described herein allow improvement of activation and differentiation. Further, the data suggest that once monocyte activation has occurred, differentiation is channeled into the direction of immuno-suppressive antigen-presenting dendritic cells by applying photoactivatable agents such as 8-MOP and UV-A thereby increasing expression of GILZ. The present data suggest that these processes take place in the absence of e.g. apoptosis of cytotoxic T-cells or other foreign cells such that the immuno-suppressive antigen-presenting dendritic cells can be obtained before loading them with e.g. a desired antigen.

Some of the embodiments, which are based on this data, are described in more detail hereinafter.
The present invention pertains to a method for inducing differentiation of monocytes contained in an extracorporeal quantity of a mammalian subject's blood sample into immuno-suppressive dendritic cells, said method comprising at least the steps of:
a) subjecting said extracorporeal quantity of said mammalian subject's blood sample to a physical force such that said monocytes are activated and induced to differentiate into immuno-suppressive dendritic cells, which are identified by at least one molecular marker, wherein said at least one molecular marker is indicative of immuno-suppressive dendritic cells;
b) determining expression of GILZ to identify said immuno-suppressive dendritic cells;
and wherein said monocytes are exposed to UV light in the presence of a DNA-cross linking agent, preferably 8-MOP, to effect increased expression of GILZ, wherein said extracorporeal quantity of said mammalian subject's blood sample is subjected to a physical force by passing said extracorporeal quantity of said mammalian subject's blood sample through a flow chamber of a device, which allows for fixed or tunable adjustment of the flow rate of said extracorporeal quantity of said mammalian subject's blood sample through said flow chamber of said device such that a shear force is applied to said monocytes contained within said mammalian subject's blood sample and wherein said immuno-suppressive dendritic cells are resistant to full maturation by LPS stimulation.

Additional molecular markers indicative of immuno-suppressive dendritic cells include IL-10. Thus, dendritic cells as they are obtainable by the methods described herein, which show an increased expression of GILZ or GILZ and IL-10 are considered to be immuno-suppressive dendritic cells. GILZ is a molecular marker being indicative for immuno-suppressive dendritic cells. It is to be understood that an increased expression is to be determined in comparison to the monocytes before they are subjected to the methods described herein. Such immuno-suppressive dendritic cells may also show an GILZ-induced reduced IL-10 to IL-12p70 ratio.

In addition, immuno-suppressive dendritic cells can be distinguished from immuno-stimulatory autologous dendritic cells in that they do not show an increased expression of molecular markers, which are indicative of immuno-stimulatory autologous dendritic cells. It is to be understood that an expression is to be determined in comparison to the monocytes before they are subjected to the methods described herein

Molecular markers indicative of immuno-stimulatory autologous dendritic cells are described hereinafter and may be taken from e.g. Table 1. These molecular markers may be grouped according to their known function as e.g. molecular markers of antigen-presenting cells, molecular markers of cellulara adhesion etc. Preferred molecular markers the expression of which is considered indicative of immuno-stimulatory dendritic cells include PLAUR, NEU1, CD80, CCR7, LOX1, CD83, ADAM Decysin, FPRL2, GPNMB, ICAM-1, HLA-DR, and/or CD86. Markers like HLA-DR, PLAUR and ICAM-1 may be considered to be indicative of global monocyte activation while increased expression of e.g. CD83 and ADAM-Decysin seems indicative of monocyte to dendritic cell differentiation. Thus, if these molecular markers show an increased expression and particularly if GILZ expression is not increased, dendritic cells as they are obtainable by the methods described herein will be considered to be immuno-stimulatory autologous dendritic cells. In turn, dendritic cells as they are obtainable by the methods described herein will be considered immuno-suppressive dendritic cells if they show an increased expression of GILZ and/or IL-10 and particularly if molecular markers taken from Table 1 do not show an increased expression.

Activation of monocytes is *inter alia* achieved in that said extracorporeal quantity of said mammalian subject's blood sample is subjected to a physical force by passing or cycling said extracorporeal quantity of said mammalian subject's blood sample through a flow chamber of a device, which allows adjustment of the flow rate of said extracorporeal quantity of said mammalian subject's blood sample through said flow chamber of said device such that a shear force is applied to said monocytes contained within said mammalian subject's blood sample.

Thus, activation of monocytes and induction of differentiation into immuno-suppressive dendritic cells can be achieved and influenced by varying the flow forces of the extracorporeal quantity of the mammalian subject's blood sample through the flow chamber of such a device, by varying the geometry of the flow path of the flow chamber, by varying the dimensions of the flow chamber, by varying the temperature of the flow chamber and thus of the extracorporeal quantity of the mammalian subject's blood sample, by changing the biophysical and geometric surface properties of the flow path, by allowing the exposure of the extracorporeal quantity of the mammalian subject's blood sample in the flow chamber to UV light, by adding DNA-cross linking agents such as 8-MOP, etc..

As is shown hereinafter, activation of monocytes and induction of differentiation into immuno-suppressive dendritic cells is mediated e.g. by interaction of monocytes with activated platelets and/or specific plasma components in a situation where the monocytes experience physical force which may be provided by a device as described hereinafter.

The present disclosure thus describes activation of monocytes, which experience a physical force and which interact with activated platelets and/or plasma components such as fibrinogen or fibronectin. Activation may be a process of subsequent steps including the steps of (i) immobilizing plasma components such as fibrinogen or fibronectin either as isolated components or as part of the extracorporeal quantity of the mammalian subject's blood sample in the flow chamber of said device (ii) passing platelets, which may be obtained as a purified fraction from the extracorporeal quantity of the mammalian subject's blood sample or as part of the extracorporeal quantity of the mammalian subject's blood sample, through the flow chamber such that the platelets can interact with and become activated by the plasma components and (iii) passing monocytes, which may be obtained as a purified fraction from the extracorporeal quantity of the mammalian subject's blood sample or as part of the extracorporeal quantity of the mammalian subject's blood sample, through the flow chamber such that the monocytes can interact with and become activated by the activated platelets and/or the plasma components.

Thus, in addition and/or alternatively to the above described parameters and variable touching on the architecture of and the conditions under which the device is operated, activation of monocytes and induction of differentiation into immuno-suppressive dendritic cells can be achieved and influenced by varying the nature, purity and concentrations of the plasma components, the nature, purity and concentration of the platelets, the order of steps by which the plasma components and/or the platelets are passed through and/or disposed on the flow chamber, the density by which the flow chamber is coated with the plasma components and/or the platelets, the flow forces of the extracorporeal quantity of the mammalian subject's blood sample and in particular the platelets and/or the monocytes are passed through the flow chamber of such a device, the temperature and/or time at which the extracorporeal quantity of the mammalian subject's blood sample and in particular the platelets and/or the monocytes are passed through the flow chamber of such a device, etc., the nature, purity and concentrations of additional factors such as 8-MOP and/or cytokines are added to the extracorporeal quantity of the mammalian subject's blood sample and in particular to the monocytes, etc.

It needs, however, to be understood that while such devices may be particularly effective in inducing monocyte activation and differentiation into immuno-suppressive dendritic cells in the presence of a photo-activatable agent such as 8-MOP and UVA, shear forces which monocytes experience during initial purification or enrichment such as during Ficoll-Hypaque enrichment as described hereinafter may already be sufficient to activate monocytes and to induce their differentiation. Similarly even though activated platelets and/or specific plasma components may be helpful in increasing monocyte activation and differentiation into antigen-presenting cells such as dendritic cells they may not be absolutely necessary. In order to effect monocyte activation and differentiation into antigen-presenting cells such as dendritic cells the invention thus contemplates as a mimimal requirement the application of shear forces. Once the activation and differentiation process has started application of photoactivatable agents such as 8-MOP and UVA or other means that increase GILZ expression (such as dexamethasone) can be used to selectively channel the differentiation process towards immuno-suppressive dendritic cells.

In the above and ensuing aspects and embdodiment, the extracorporeal quantity of the mammalian subject's blood sample and in particular the monocytes thus may or may not have been obtained by leukapheresis.

Additionally or alternatively to these embodiments, the invention in particular also relates to such methods which are conducted under conditions which favor an increased expression of GILZ and an increased number of CD4⁺CD25⁺Foxp3⁺ cells and/or a down-regulations of CD80, CD86 and CD83. The invention thus relates to e.g. methods, which are conducted in the presence of a photoactivatable agent such as 8-MOP and with exposure to light such as UV-A. In one embodiment, the invention correspondingly relates to methods which are conducted under conditions which avoid activation of monocytes and induction of immuno-stimulatory autologous dendritic cells as is identified by determining expression of GILZ and IL-10 and/or of the molecular markers of Table 1. In one embodiment, the invention thus does not relate to e.g. methods, which are conducted in the absence of a photoactivatable agent such as 8-MOP and without exposure to light such as UV-A

The present disclosure relates to methods as described hereinafter for obtaining , autologous and/or allogenic immuno-suppressive antigen-presenting cells, which can be used e.g. in treating autoimmune diseases, hypersensitivity diseases, rejection of solid organ transplant, and Graft-versus-Host Disease.

Another embodiment relates to methods as described hereinafter for producing immuno-suppressive dendritic cells which may be mammalian cells. Even though some of the preferred embodiments of the invention relate to producing human immuno-suppressive allogeneic dendritic cells, the present invention considers also to use the methods for producing animal immuno-suppressive dendritic cells such as for mice, rats, etc. These embodiments of the invention provide useful animal models and thus scalability of the methods and results described herein from e.g. mice to man. Moreover, as there are genetically identical lines of animals such as mice available, animal immuno-suppressive dendritic cells such as mice immuno-suppressive dendritic cells may be introduced either in the individual from which the extracorporeal amount of blood sample was taken and thus be autologous in the strict sense or be introduced in a genetically identical individual. This will allow e.g. testing for any unexpected effects of these cells.

It is to be understood that the methods described hereinafter have been shown to produce immune-suppressive cells, which due to their molecular markers seem to be related to if not correspond to cells that are commonly named dendritic cells. Thus the immune-suppressive cells according to the invention have been named immune-suppressive dendritic cells. However, dendritic cells are representatives of a broader class of cells, which may be designated as antigen-presenting cells.
The present disclosure also describes to autologous and/or allogenic immuno-suppressive dendritic cells or autologous and/or allogenic immuno-suppressive antigen-presenting cells obtainable by a method as described hereinafter for use in treating autoimmune diseases, hypersensitivity diseases, rejection of solid organ transplant, and Graft-versus-Host Disease.

Further embodiments will be described hereinafter.

### FIGURE LEGENDS

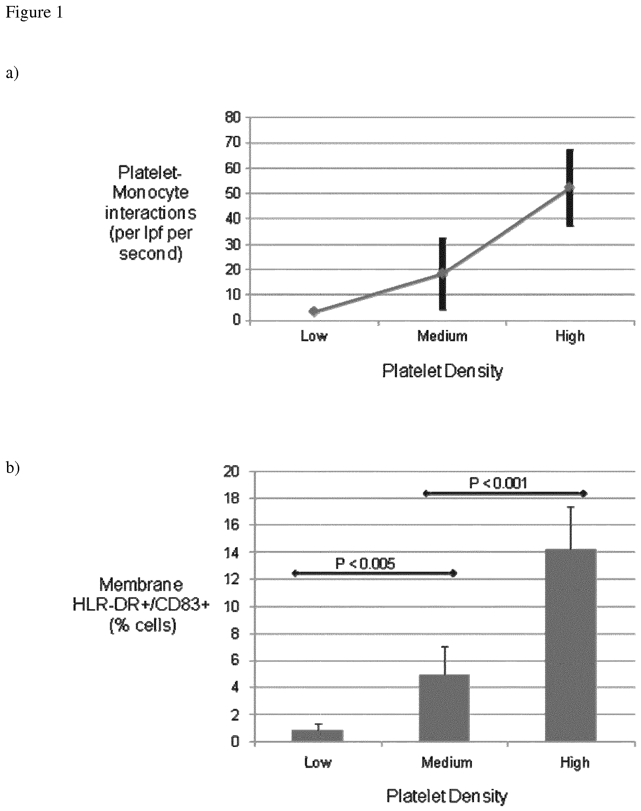

- Figure 1: Effect of platelet density on number of monocyte-platelet interactions and subsequent monocyte phenotype. Monocytes were passed through parallel plates coated with platelets at low, medium, or high density. (A) The number of monocyte-platelet interactions increased substantially for plates coated with higher densities of platelets. (B) After overnight incubation, monocytes which were exposed to high levels of platelets were significantly more likely to develop a phenotype consistent with DC differentiation, as assessed by expression of membrane CD83 and HLA-DR (high versus medium or low density: p <0.0001; medium versus low density: p < 0.005). Data shown are the means (+/- SD) of at least 6 independent experiments. lpf, low power field.

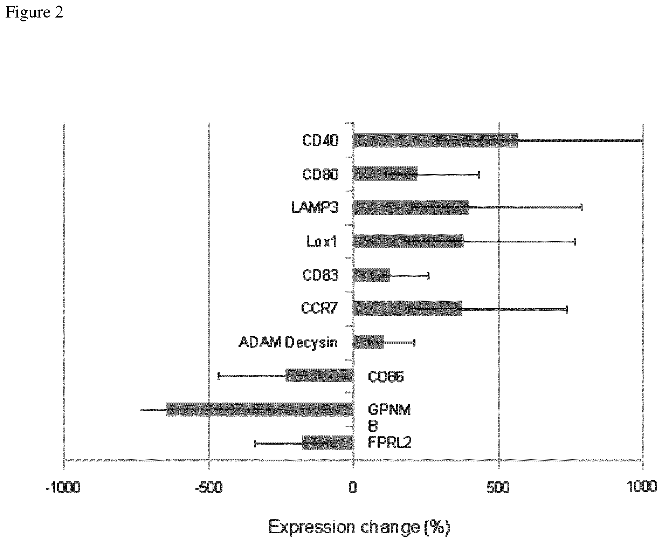

- Figure 2: Gene expression following exposure to platelets. Monocytes were exposed to high or low levels of platelets in flow. Following overnight incubation, cells were assessed for differences in gene expression using RT-PCR. Figure 2 shows gene expression changes in monocytes exposed to high levels of platelets relative to those exposed to low levels. Seven genes associated with DC-differentiation and/or function were found to be upregulated, while three were downregulated. Of the genes downregulated, GPNMB and FPRL2 have known functions in decreasing cytokine production and inhibiting DC maturation, respectively. Of the genes upregulated, all have either pro-immune functions or miscellaneous roles in DC biology. See text for specific description of genes. Data shown are the means (+/- SD) of 2 independent experiments.

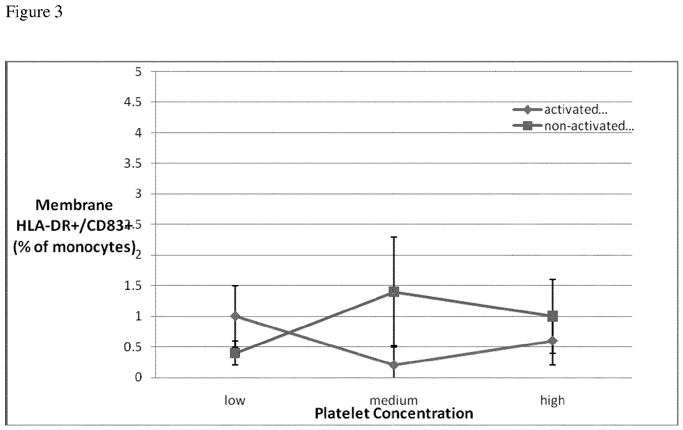

- Figure 3: Platelet influence on monocyte differentiation in static conditions. Monocytes were co-cultured for 18 hours with low, medium, or high concentrations of platelets in static conditions lacking flow. Under these conditions, there was no observable platelet influence on DC differentiation; all conditions resulted in low, baseline levels of cells expressing DC markers. Furthermore, activating platelets with thrombin in culture (blue line) did not cause a discernible difference in monocyte differentiation relative to those cultures containing platelets not activated by thrombin (red line).

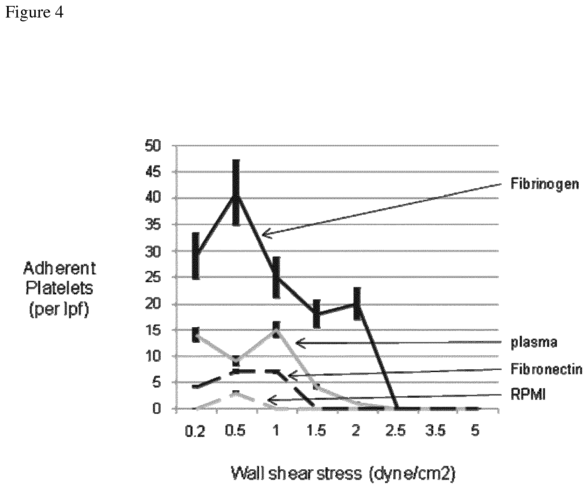

- Figure 4: Plasma protein influence on platelet adhesion to plates. Platelets were passed through plates coated with fibrinogen, plasma, fibronectin, or RMPI at the shear stress level indicated by the x-axis. Platelets in flow adhered optimally to fibronectin. For all proteins, platelet adhesion occurred maximally between 0.5 and 1.0 dyne/cm² lpf, low power field. Data shown are the means (+/- SD) of at least 2 independent experiments.

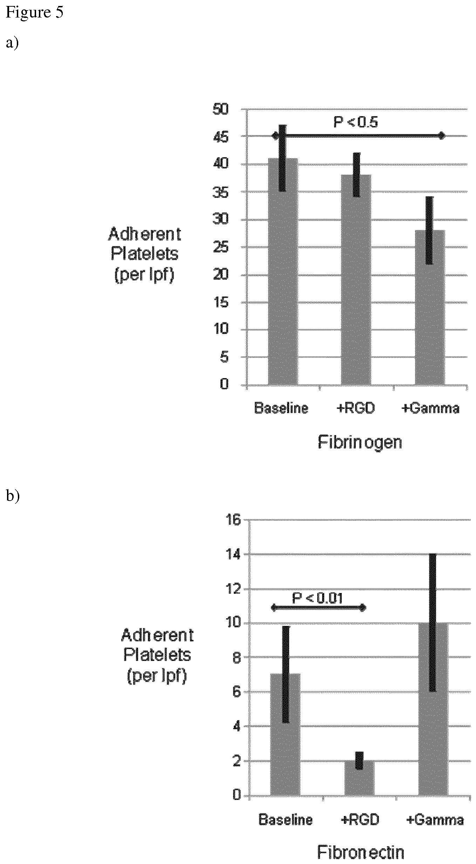

- Figure 5: Plasma protein influence on platelet adhesion to plates coated with Fibrinogen (A) or Fibronectin (B). Platelets were either untreated (baseline), or pretreated with either RGD fragments (+RGD) or gamma fragments (+Gamma) and their subsequent adhesion to fibrinogen (left panel) and fibronectin (right panel) was assessed. Platelet binding to fibrinogen was decreased by gamma fragments (p < 0.05), while binding to fibronectin was decreased by RGD peptides (p < 0.001). lpf, low power field. Data shown are the means (+/- SD) of at least 2 independent experiments.

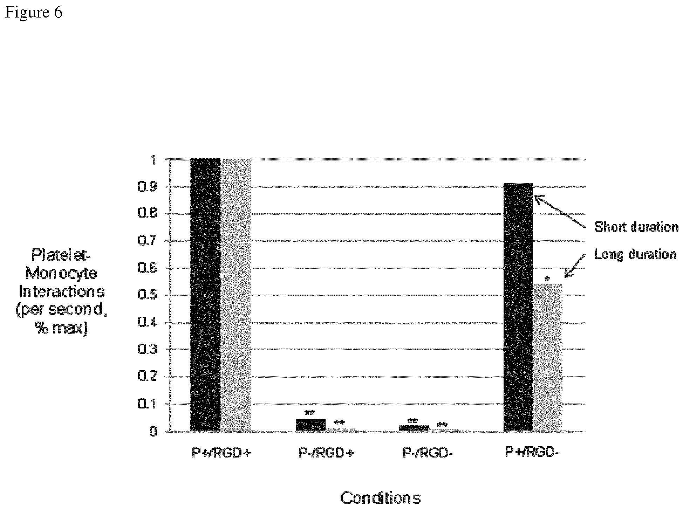

- Figure 6: Proteins involved in monocyte-platelet interactions. Monocytes were passed between platelet-coated plates at a wall shear stress of 0.5 dyne/cm2 under the conditions indicated by the x-axis: platelets were either pretreated with anti-P-selectin (P-) or an isotype control (P+); monocytes were either pretreated with RGD peptides (RGD-) or a control fragment (RGD+). Monocyte-platelet interactions were quantified under each set of conditions using digital microscopy, and are expressed in the figure as a fraction of the maximum seen under conditions of P+/RGD+. Interactions were divided into those lasting less than 3 second (short duration, black bars) and those lasting greater than 3 seconds, including stable binding (long-duration, gray bars). All conditions which involved blocking with anti-P-selectin (P-) resulted in a significant decrease in both short and long duration interactions (**, p < 0.01); Blocking only RGD (RGD-) resulted in a significant decrease in long-duration interactions (*, p < 0.05) but no change in short-duration interactions. Data shown are the means (+/- SD) of 3 independent experiments.

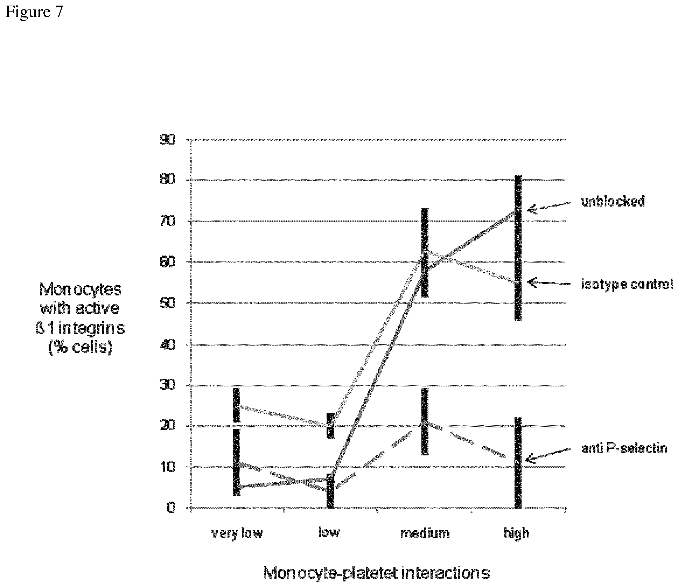

- Figure 7: Effect of p-Selectin exposure on monocyte integrins. Plastic plates were coated with platelets at the relative density indicated by the x-axis. Platelets were then pretreated with anti p-selectin (dashed line) or an isotype control (gray line), or received no pretreatment (black line). Monocytes were passed through the plates at 0.5 dyne/cm2 and then immediately assessed by flow cytometry for expression of active β1 integrins. The y-axis indicates the percent of monocyte which bound an antibody directed at an epitope only exposed when the integrin is in the open confirmation. Data shown are the means (+/- SD) of 3 independent experiments.

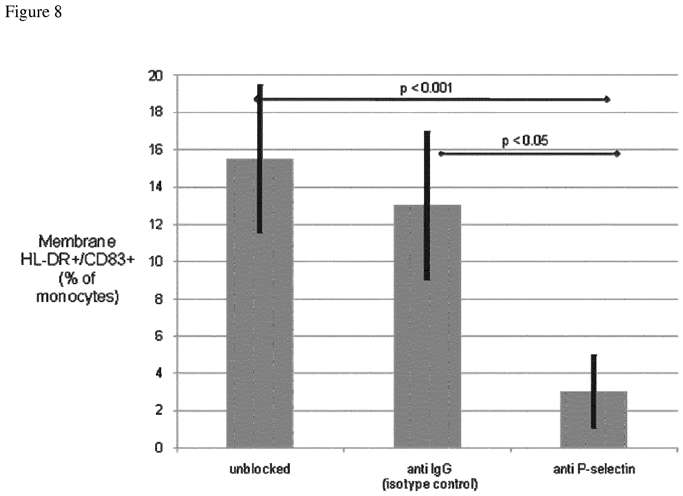

- Figure 8: Effect of P-selectin exposure on monocyte phenotype after overnight incubation. Platelet-coated plates were either untreated (first column), or pretreated with an isotype control (second column) or anti-P-selectin (third column). Monocytes were passed through the plates at 0.5 dyne/cm2 then incubated overnight. The y-axis indicates the percent of monocytes which developed a phenotype consistent with DC differentiation, i.e., membrane HLA-DR+/CD83+. Data shown are the means (+/- SD) of 3 independent experiments.

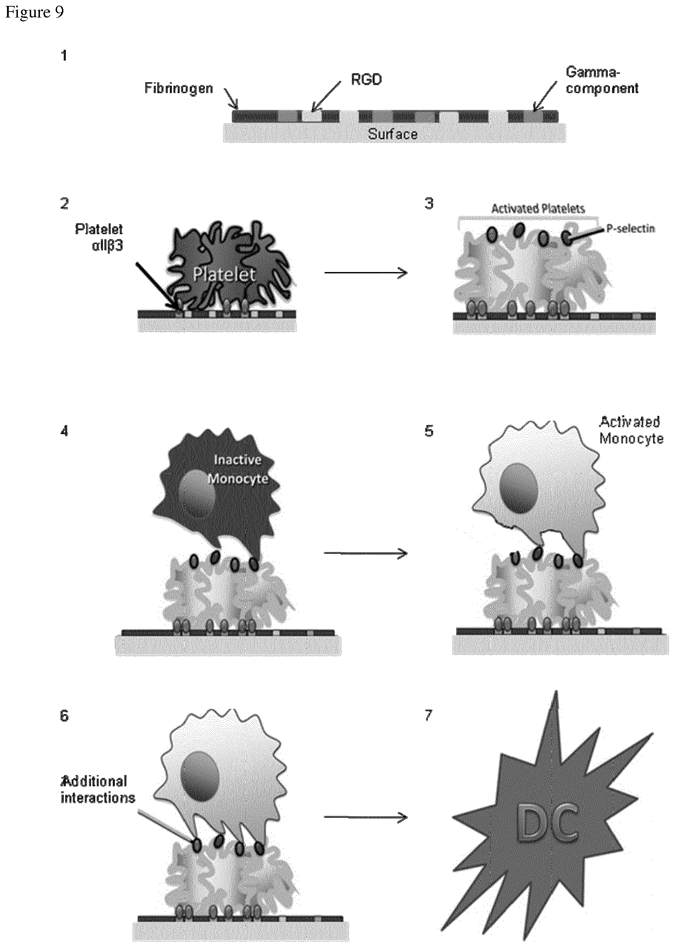

- Figure 9: Proposed mechanism for induction of monocyte-to-DC differentiation. Based on data presented in this manuscript, the following sequence of events is postulated: (1) plasma fibrinogen coats the plastic surface of the flow chamber; (2) through their αIIbβ3 receptor, unactivated platelets bind to the gamma-component of immobilized fibrinogen; (3) platelets become activated and instantaneously express preformed P-selectin and other surface proteins; (4) passaged monocytes transiently bind P-selectin via PSGL-1, causing partial monocyte activation and integrin receptor conformational changes; (5) partially-activated monocytes, now capable of further interactions, bind additional platelet-expressed ligands, including those containing RGD domains; (6) finally, so influenced, monocytes efficiently enter the DC maturational pathway within 18 hours. Note that, in-vivo, step (1) above may be replaced physiologically by inflammatory signals from tissue acting on local endothelium, causing it to recruit and activate platelets in a similar manner.

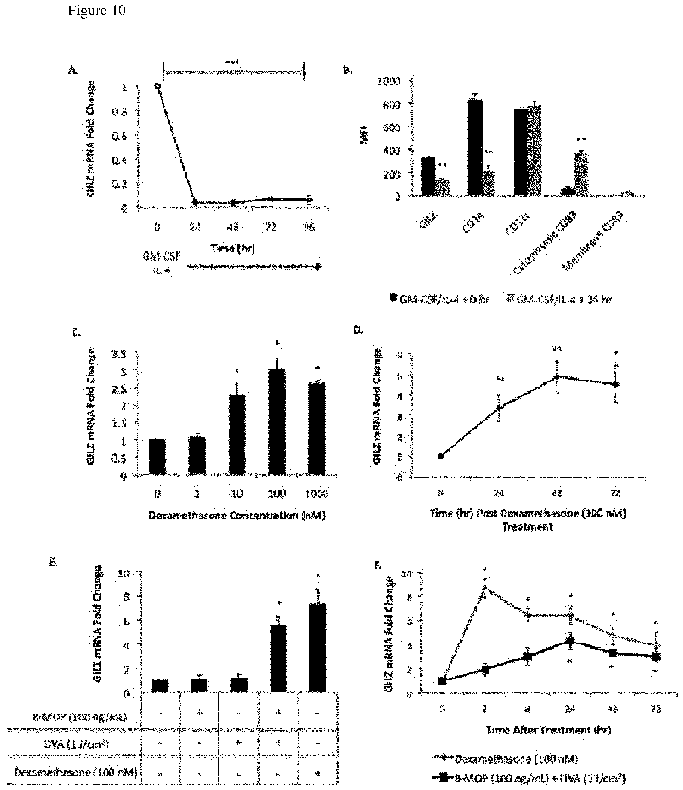

- Figure 10:: Expression of GILZ is rapidly down-regulated as monocytes differentiate into immature MoDC, and up-regulated after exposure to dexamethasone. A.) GILZ mRNA expression in CD11c+ MoDC is presented as a fold change relative to freshly isolated monocytes. B.) Median fluorescence intensities for intracellular and cell surface markers after 0 and 36 hr. C.) GILZ mRNA expression in CD11c+ MoDC after 24 hr is presented as a fold change relative to MoDC receiving no dexamethasone. D.) GILZ mRNA expression in CD11c+ MoDC is presented as a fold change relative to MoDC at time 0 hr. E.) GILZ mRNA expression in CD11c+ MoDC after 24 hr is presented as a fold change relative to untreated MoDC. F.) GILZ mRNA expression in CD11c+ MoDC is presented as a fold change relative to untreated MoDC. All data are expressed as mean ± standard deviation for a minimum of 3 independent experiments. For differential gene expression: * ≥ 2.5-fold change and p < 0.05, ** ≥ 2.5-fold change and p < 0.01, *** ≥ 2.5-fold change and p < 0.001

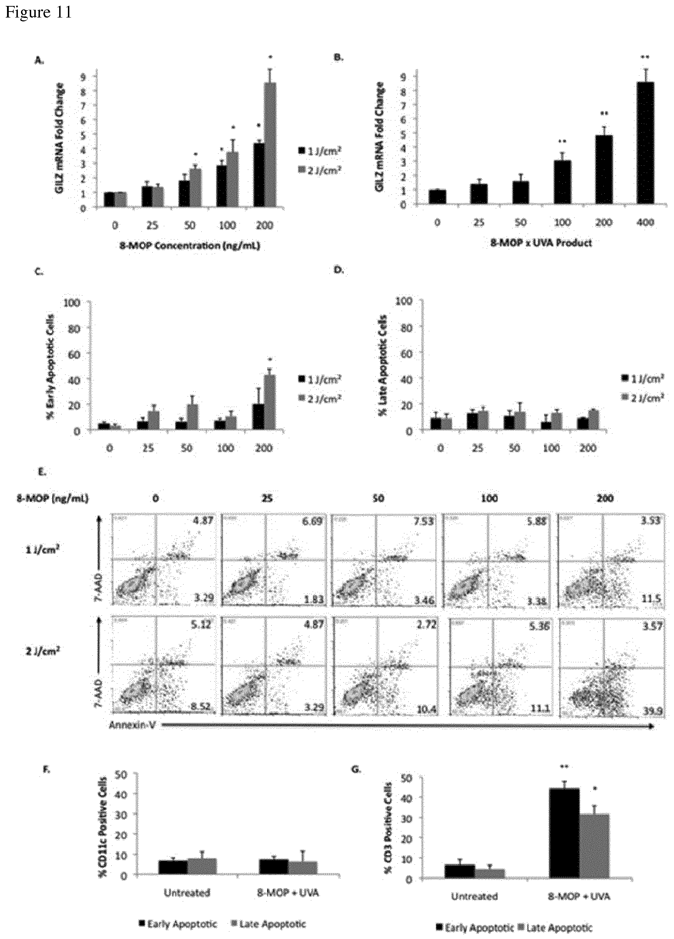

- Figure 11:: 8-MOP plus UVA light up-regulates GILZ in immature MoDC in a dose-dependent fashion. A.) GILZ expression is presented as a function of the 8-MOP concentration at 1 J/cm2 and 2 J/cm2 of UVA light. GILZ mRNA expression in CD11c+ MoDC 24 hr after PUVA treatment is presented as a fold change relative to MoDC receiving no 8-MOP. B.) GILZ expression is presented as a function of the 8-MOP concentration multiplied by the UVA dose. C.) The percentage of early apoptotic CD11c+ cells after 24 hr. D.) The percentage of late apoptotic CD11c+ cells after 24 hr E.) Dot plots of CD11c+-gated cells for UVA doses of 1 J/cm2 and 2 J/cm2 are shown for 1 representative experiment of 4. The percentage of CD11c+ cells displaying Annexin-V+/7-AAD- or Annexin-V+/7-AAD+ phenotypes are indicated. The percentage of F.) CD11c+ cells and G.) CD3+ cells expressing early and late apoptotic markers were quantified 24 hr after treatment with 8-MOP (100 ng/mL) and UVA light (1 J/cm2). All data represent mean ± standard deviation of at least 4 independent experiments. For differential gene expression: * ≥ 2.5-fold change and p < 0.05, ** ≥ 2.5-fold change and p < 0.01

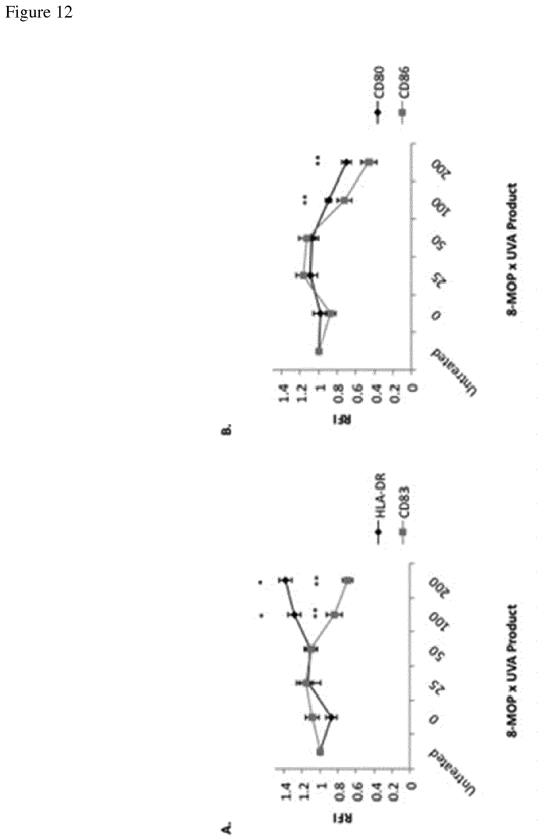

- Figure 12:: 8-MOP plus UVA light down-regulates CD83, CD80 and CD86 and up-regulates HLA-DR in immature MoDC in a dose-dependent manner. Relative fluorescence intensities for membrane expression of A.) HLA-DR and CD83, and B.) CD80 and CD86 are presented as a function of the 8-MOP concentration (0 to 200 ng/mL) multiplied by the UVA dose (1 or 2 J/cm2) 24 hr after PUVA treatment. Untreated MoDC served as controls and were assigned an RFI value of 1. Data represent mean ± standard deviation of 4 independent experiments. *p < 0.05, **p < 0.01

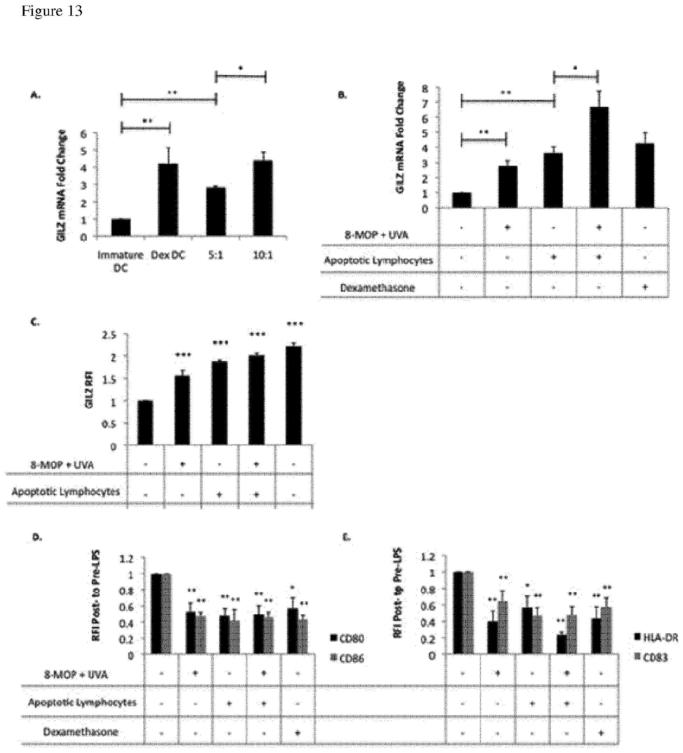

- Figure 13:: Immature MoDC exposed to apoptotic lymphocytes up-regulate GILZ. A.) GILZ mRNA expression in CD11c+ MoDC 24 hr after co-culture is presented as a fold change relative to untreated MoDC that were cultured alone. B.) GILZ mRNA expression in CD11c+ MoDC 24 hr after co-culture is presented as a fold change relative to untreated MoDC that were cultured alone. C.) Relative fluorescence intensity for intracellular GILZ 24 hr after co-culture. Relative fluorescence intensities post- to pre-LPS stimulation for D.) CD80 and CD86 and E.) HLA-DR and CD83 were calculated as follows: (MFItreated after LPS - MFItreated before LPS) / (MFIuntreated after LPS - MFIuntreated before LPS). Data represent mean ± standard deviation for at least 4 independent experiments. For differential gene expression: * ≥ 2.5-fold change and p < 0.05

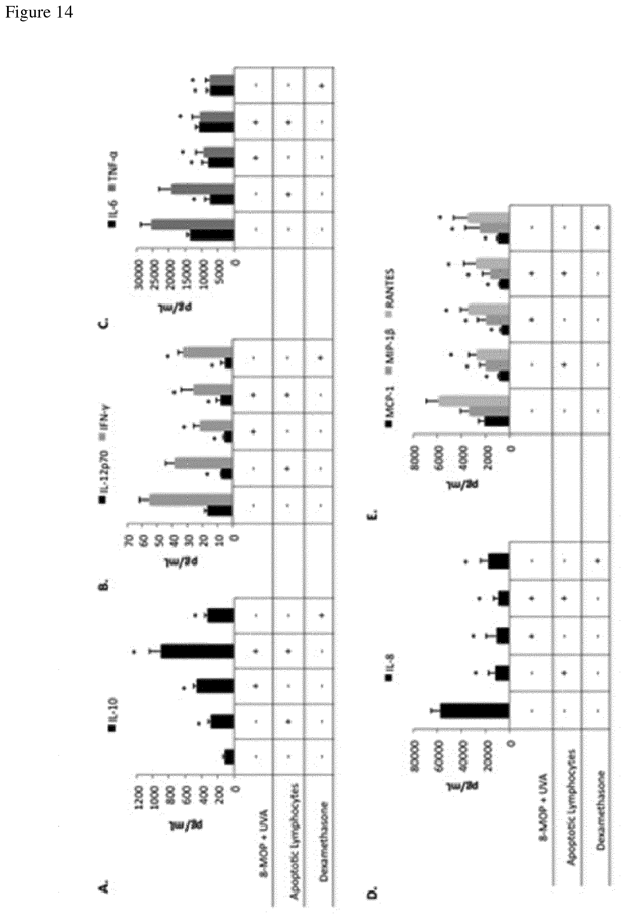

- Figure 14:: MoDC expressing GILZ increase production of IL-10, and decrease production of various pro-inflammatory cytokines and chemokines. 24 hr after LPS stimulation, culture supernatants were harvested for cytokine quantification by magnetic bead multiplex immunoassays for A.) IL-10, and the pro-inflammatory cytokines B.) IL-12p70 and IFN-γ, C.) IL-6 and TNF-α. The same analysis was performed for the pro-inflammatory chemokines D.) IL-8, and E.) MCP-1, MIP-1β and RANTES. Data are presented as mean ± standard deviation of 3 independent experiments. * p < 0.05 compared to the untreated MoDC group.

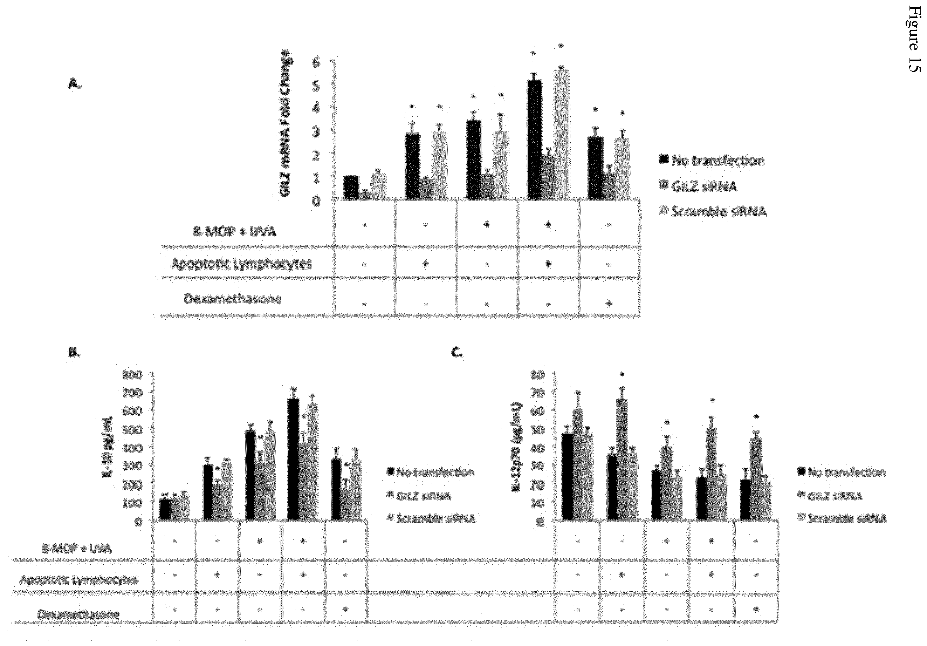

- Figure 15:: siRNA-mediated knockdown of GILZ abolishes the increased IL-10 to IL-12p70 ratio characteristic of tolerogenic dendritic cells. A.) GILZ mRNA expression is presented as fold change compared to untreated MoDC that were cultured alone. * ≥ 2.5-fold change and p < 0.05. B.) Quantification of IL-10 and IL-12p70 protein levels in culture supernatants after LPS stimulation. Data represent mean ± standard deviation of 3 independent experiments. *p < 0.05, compared to identically treated MoDC not transfected with siRNA.

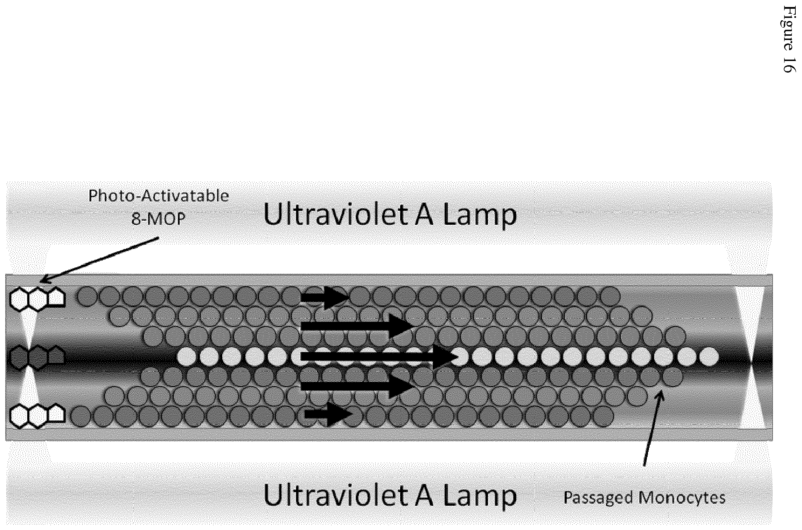

- Figure 16:: depicts the flow of monocytes in a classical ECP process in the presence of UVA and 8-MOP. The monocytes in the middle experience lower UVA exposure than the monocytes towards the surfaces of the channels.

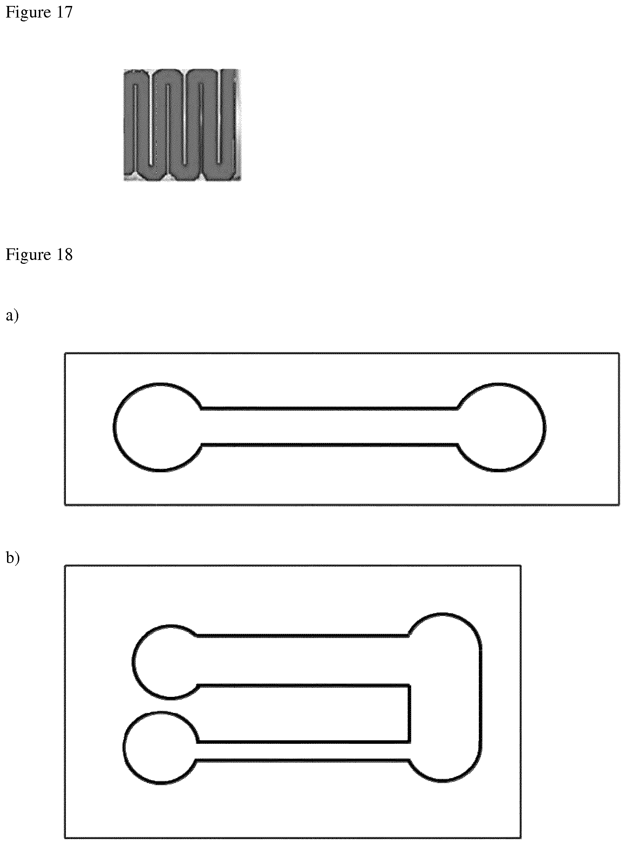

- Figure 17:: depicts the design of the channels of the device used in a classical ECP process.

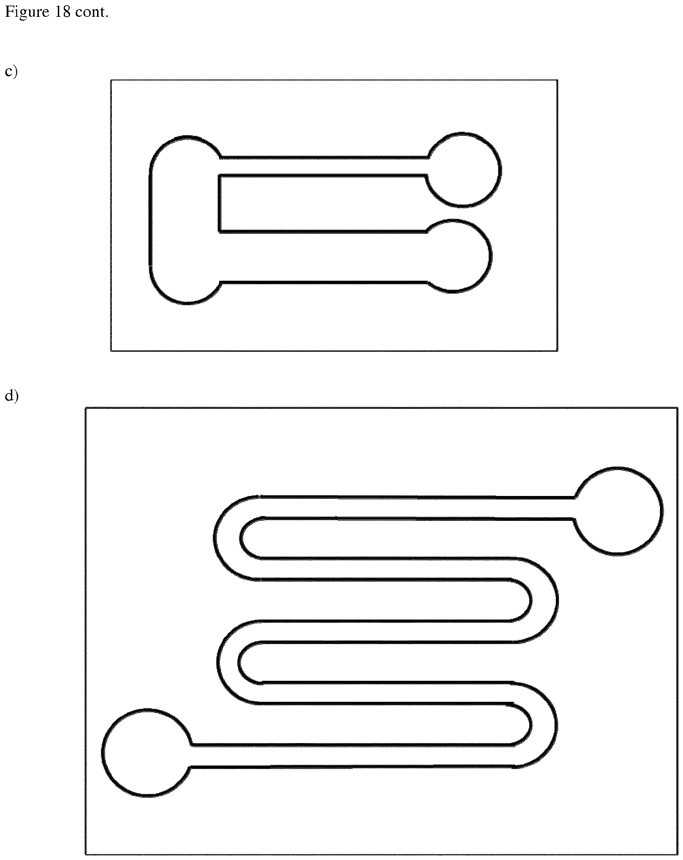

- Figure 18:: a) to d) depict different geometries of the flow chamber of a device that may be used for the methods of the invention.

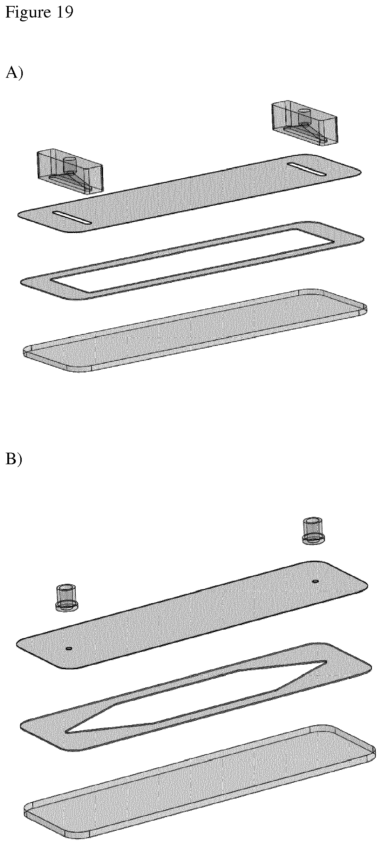

- Figure 19:: A) depicts the geometry of a device used in some of the examples. B) depicts the geometry of an alternative device.

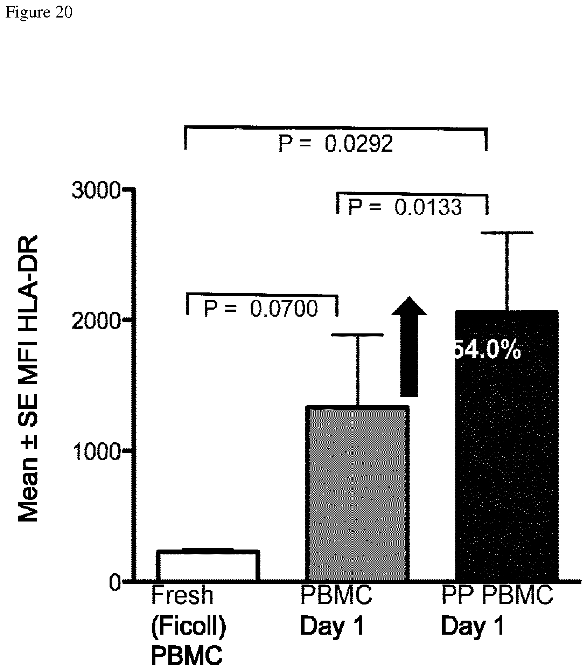

- Figure 20:: depicts increase of expression of HLA-DR upon physical activation of monocytes through a device of Figure 19

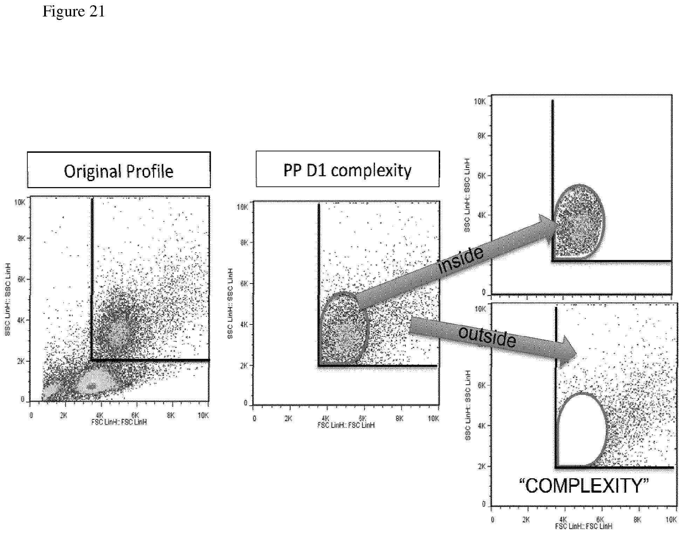

- Figure 21:: depicts increase of FSC/SSC complexity upon physical activation of monocytes through a device of Figure 19

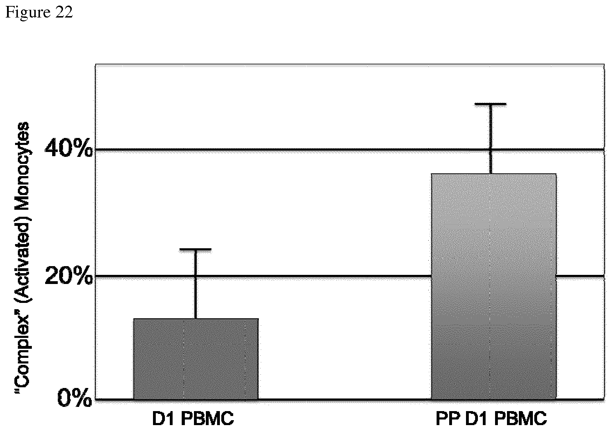

- Figures 22:: depicts increase of FSC/SSC complexity upon physical activation of monocytes by passing through a device of Figure 19

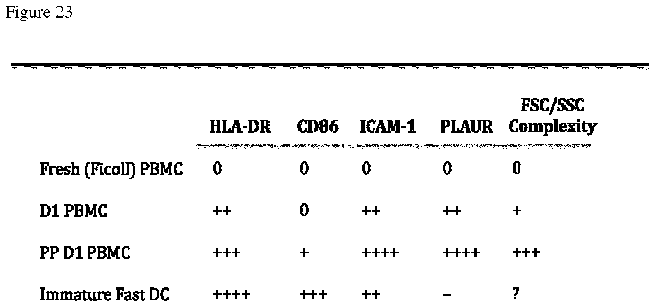

- Figure 23:: depicts increase of expression of HLA-DR, CD86, ICAM-1, PLAUR and or FSC/SSC complexity upon physical activation of monocytes through a device of Figure 19
- Figure 24:: depicts increase of expression of GILZ upon physical activation of monocytes through a device of Figure 19 and application of 8-MOP and UVA

### DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of' is considered to be a preferred embodiment of the term "comprising of'. If hereinafter a group is defined to comprise at least a certain number of embodiments, this is also to be understood to disclose a group, which preferably consists only of these embodiments.

For the purposes of the present invention, the term "obtained" is considered to be a preferred embodiment of the term "obtainable". If hereinafter e.g. an antibody is defined to be obtainable from a specific source, this is also to be understood to disclose an antibody, which is obtained from this source.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of ±20 %, preferably ±15 %, more preferably ±10 %, and even more preferably ±5 %.

Furthermore, the terms "first", "second", "third" or "(a)", "(b)", "(c)", "(d)" or "(i)", "(ii)", "(iii)", "(iv)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" or "(i)", "(ii)", "(iii)", "(iv)" etc. relate to steps of a method or use or assay there is no time or time interval coherence between the steps unless indicated otherwise, i.e. the steps may be carried out simultaneously or there may be time intervals of seconds, minutes, hours, days, weeks, months or even years between such steps, unless otherwise indicated in the application as set forth herein above or below.

Technical terms are used by their common sense. If a specific meaning is conveyed to certain terms, definitions of terms will be given in the following in the context of which the terms are used.

As already mentioned, the present invention is based to some extent on data presented hereinafter, which for a miniaturized device allowed (i) to mimic some aspects of the classical ECP procedure and (ii) to elucidate the cellular and molecular mechanism of induction of differentiation of monocytes into immuno-stimulatory dendritic cells in an extracorporeal amount of blood. As is shown by the experiments described hereinafter, these immuno-stimulatory autologous dendritic cells can be characterized by expression of molecular markers indicative of immuno-stimulatory autologous dendritic cells. The data also shows that conditions that lead to an increased expression of Glucocorticoid-induced Leucine Zipper (GILZ) will favorably allow monocytes to differentiate into immuno-suppressive dendritic cells. For the purposes of the present invention such immuno-suppressive dendritic cells are also designated as immuno-inhibiting autologous dendritic cells, tolerogenic autologous dendritic cells or truncated autologous dendritic cells. This data shows that e.g. the sequential activation of platelets and binding of monocytes to such activated platelets under conditions of shear force is essential for differentiation of monocytes into dendritic cells and, depending on the specific conditions chosen, in particular into immuno-stimulatory or immuno-suppressive dendritic cells. Further, these findings immediately allow for a rationalized approach to obtain immuno-stimulatory and immuno-suppressive dendritic cells. Given that one can mimic and dissect the series of molecular events leading to the formation of immuno-stimulatory autologous dendritic cells and immuno-suppressive dendritic cells obtained in the classical ECP process, one can now design devices and more particularly flow chambers, which allow to further dissect the molecular events leading to differentiation of monocytes into immuno-stimulatory autologous dendritic cells and immuno-suppressive dendritic cells on a scale suitable for research purposes, but also which allow to obtain immuno-stimulatory autologous dendritic cells and immuno-suppressive dendritic cells for therapeutic purposes. This will be explained in further detail.

In the classical ECP procedure, 2.5 L to 6 L blood is typically obtained from patients suffering from CTLC by apheresis such as leukaphereses. This extracorporeal amount of blood, which typically is processed by apheresis such as leukaphereses to give a final volume of about 200 ml to 500 ml comprising leukocytes including monocytes as well as plasma components, platelets and cancerous T-cells, is then passed under shear stress through a photopheresis device having transparent plastic channels together with the photoactivatable drug 8-MOP. This extracorporeal amount of blood comprising 8-MOP is then irradiated by exposing the transparent channels to UV-A having a wavelength of 315 to 380 nm. The irradiated extracorporeal amount of blood is then re-introduced into the patient. The beneficial effects of this procedure on the course of CTLC in some of the treated patients was originally hypothesized to result from the destruction of cancerous T-cells. Based on this hypothesis, it was assumed that patients would have to undergo repeated cycles of ECP. However, for some of the patients beneficial long-term effects were observed making repeated treatment superfluous and, in the following, interesting and partially non-reconcilable effects were found, which could explain some of the positive outcomes of ECP for CTLC treatment.

For example, as is described in US 6,524,855 induction of DC was observed in the extracorporeal amount of blood and it was hypothesized that some of the beneficial effects of ECP on CTLC resulted from cancer-specific antigens that were shed by cancerous T-cells as a consequence of the 8-MOP induced apoptosis of these cells and loading of DC, which had started to differentiate, with these antigens. The re-introduction of the extracorporeal amount of blood comprising such cancer-antigen loaded autologous DC was assumed to provide a vaccination-like long-term lasting therapeutic effect. However, at the same time it was observed that so-called "truncated" DC were formed during the ECP procedure, which did not provide an immuno-stimulatory effect, but rather the opposite, namely an immuno-suppressant effect. The induction of such different types of DC with opposing effects by the same process was puzzling and, from a practical perspective, posed hurdles as to a rationalized use of ECP for obtaining immuno-stimulatory or immuno-suppressant DC. Further, the need for apheresis such as leukaphereses to obtain a sufficient amount of extracorporeal blood is another factor negatively affecting treatment quality for patients.

The data presented hereinafter suggest that shear stress is in principle responsible for the induction of DC. By using the miniaturized model device as described hereinafter, it was shown that induction of immuno-stimulatory DC occurs even if substantially lower amounts of extracorporeal blood, which has not been obtained by apheresis such as leukaphereses, are used, and if 8-MOP is not added to the extracorporeal amount of blood and even if no irradiation with UV-A takes place. Thus, induction of DC occurred despite omission of central steps of the classical ECP procedure. However, shear stress seems to be one factor that is crucial for obtaining DC as such. Other crucial steps for the induction of dendritic cell formation seem to be the activation of platelets by plasma components and the activation of monocytes by such activated platelets. The data further suggests that, if shear-stress induced induction of dendritic cell formation takes place in the presence of 8-MOP and irradiation with UVA, expression of the Glucocorticoid-induced Leucine Zipper (GILZ) is increased, which in turn activates a pathway leading to formation of truncated, i.e. immuno-suppressive, tolerogenic DC (see Example 2).

The fact that shear-stress induced induction of immuno-stimulatory DC could be achieved by applying shear stress without the addition of 8-MOP and without irradiation with UV-A further suggests that in the classical ECP procedure due to the dimensions of the plastic channels some of the initially shear-stress induced DC were not effectively irradiated (maybe because they were moving in the middle of the channels of the ECP device) with the consequence that these DC could further develop into immuno-stimulatory DC (see Figure 16). This previous data was obtained using a device having the general architecture of Figure 17. However, in the classical ECP and ECP-like procedures, mixtures of immuno-stimulatory autologous and immuno-suppressive autologous DC were obtained. Based on the data presented hereinafter, it is now possible to e.g. dispense with some of the requirements of the ECP and ECP-like processes of the prior art, e.g. to use large amounts of blood which needs to be processed by apheresis such as leukaphereses. Further, one can now deliberately adapt the process parameters and the design of the device, which is used to exert a physical force on monocytes, to deliberately obtain either immuno-stimulatory autologous or immuno-suppressive autologous DC. For example, by selecting the design and dimensions of the flow chamber, one can make sure that basically all DC are contacted with 8-MOP and irradiated with UVA such that immuno-suppressive DC will form. Moreover, as the molecular markers being indicative of immuno-suppressive DC and of immuno-stimulatory autologous DC as they are obtainable by the methods described herein have been deciphered, one can further purify dendritic cell populations, which depending on the chosen method parameters will be substantially already only immuno-suppressive DC or immuno-stimulatory autologous DC, by e.f. FACS analysis to homogeneity.

The methods as described hereinafter may be performed without the need of molecular cocktails to achieve maturation and differentiation of monocytes into immuno-suppressive dendritic cells. Further, as the invention is based on inducing differentiation of monocytes contained in an extracorporeal quantity of mammalian subject's blood sample, the differentiation process is not limited to the molecular events which can be triggered by typical cytokine cocktails. Rather, dendritic cells as obtainable with the methods described hereinafter seem to have more complex molecular patterns, which seem representative of a broader functionality of these dendritic cells.

The present invention relates to a method for inducing differentiation of monocytes contained in an extracorporeal quantity of a mammalian subject's blood sample into immuno-suppressive dendritic cells, said method comprising at least the steps of:
a) subjecting said extracorporeal quantity of said mammalian subject's blood sample to a physical force such that said monocytes are activated and induced to differentiate into immuno-suppressive dendritic cells, which are identified by at least one molecular marker, wherein said at least one molecular marker is indicative of immuno-suppressive dendritic cells;
b) determining expression of GILZ to identify said immuno-suppressive dendritic cells;
and wherein said monocytes are exposed to UV light in the presence of a DNA-cross linking agent, preferably 8-MOP, to effect increased expression of GILZ, wherein said extracorporeal quantity of said mammalian subject's blood sample is subjected to a physical force by passing said extracorporeal quantity of said mammalian subject's blood sample through a flow chamber of a device, which allows for fixed or tunable adjustment of the flow rate of said extracorporeal quantity of said mammalian subject's blood sample through said flow chamber of said device such that a shear force is applied to said monocytes contained within said mammalian subject's blood sample and wherein said immuno-suppressive dendritic cells are resistant to full maturation by LPS stimulation.

As has already been mentioned, the methods described hereinafter have been shown to produce immune-suppressive cells, which due to their molecular markers seem to be related to if not correspond to cells that are commonly named immune-suppressive or tolerogenic dendritic cells. Thus the immune-suppressive cells according to the invention have been named immune-suppressive dendritic cells. However, dendritic cells are representatives of a broader class of cells, which may be designated as antigen-presenting cells.

The term "immuno-suppressive dendritic cells", which is used synonymous to tolerogenic, immuno-inhibiting, or truncated dendritic cells, thus refers to cells derivable from monocytes by treating the monocytes contained in an extracorporeal quantity of said mammalian subject's blood sample as it is described herein and identified by as the molecular marker GILZ as described in the following. Additional molecular markers include IDO (Indoleamine), KMO (kynurenine 3-hydroxylase), transforming growth factor beta (TGFβ), and/or IL-10 (Interleukin 10).. Thus, dendritic cells as they are obtainable by the methods described herein, which show an increased expression of GILZ are considered to be immuno-suppressive dendritic cells. It is to be understood that an increased expression is to be determined in comparison to the monocytes before they are subjected to the methods described herein. Such immuno-suppressive dendritic cells may also show an GILZ-induced increased IL-10 to IL-12p70 ratio or a decreased production of various pro-inflammatory cytokines and chemokines such as IL-12, TNF-α, and IL-6. The molecular marker, which is considered indicative for immune-suppressive dendritic cells, is currently GILZ. As will be explained further below in more detail the immuno-suppressive dendritic cells may be autologous and/or allogenic immuno-suppressive dendritic cells. Autologous immuno-suppressive dendritic cells may be produced by the methods described herein for treatment of e.g. autoimmune diseases. Allogenic immuno-suppressive dendritic cells may be produced by the methods described herein for treatment of e.g. Graft-versus-Host disease. It needs to be understood that where immuno-suppressive antigen-presenting cells such as dendritic cells are mentioned herein, this refers to immuno-suppressive antigen-presenting cells such as dendritic cells which have the capacity of displaying e.g. disease-specific antigens in their surfaces after these cells have been contacted with such antigens. It is to be further understood that immuno-suppressive dendritic cells as obtainable by the methods described herein and identified by the molecular markers described herein in one embodiment may be considered as dendritic cells which have already differentiated enough and internalized and even display e.g. antigens from e.g. cells being involved in an autoimmune disease or antigens of cells from a recipient in e.g. a Graft-versus-Host disease application, such that they can be considered more generally to be immuno-suppressive autologous and/or allogenic antigen-presenting cells. These immuno-suppressive autologous and/or allogenic antigen-presenting cells will nevertheless be of a tolerogenic type and thus suppress an immune answer against the displayed antigens.. However, the process can also be conducted in a way such that the dendritic cells express molecular markers indicative of immuno-suppressive autologous and/or allogenic dendritic cells, which have not yet internalized and displayed antigens, because e.g. the cells were obtained from a sample of a donor that does not suffer from an auto-immune disease or Graft-versus-host disease. However, the insight provided by the data and conclusions of the present invention allows to make immuno-suppressive dendritic cells, which then can be loaded with antigens characteristic an auto-immune disease or with antigens from a donor in a transplantation setting, which have been obtained separately and not concurrently with the immuno-suppressive dendritic cells. Such antigens may then be co-incubated with immuno-suppressive dendritic cells to effect their presentation in the subsequent therapeutic setting.

That dendritic cells, which overexpress GILZ and optionally also, IDO, KMO, PDL1, PDL2, TGFβ and/or IL-10and which have been obtained by activating monocytes using processes described herein, show properties reminiscent of tolerogenic dendritic cells can be taken *inter alia* from the observation that such dendritic cells expressing GILZ are resistant to full maturation as it can be induced by e.g. LPS and be determined by increased expression of HLA-DR, CD83, CD80 and CD86. Further, the upregulation of GILZ and the decreased production of various pro-inflammatory cytokines and chemokines such as IL-12, TNF-α, and IL-6 are reminiscent of the known effects of dexamethasone, which can be used to obtain tolerogenic dendritic cells (Cohen et al., Blood (2006), 107(5), 2037-2044).

Thus, immuno-suppressive dendritic cells can additionally be distinguished from immuno-stimulatory autologous dendritic cells in that they do not show an increased expression of molecular markers, which are indicative of immuno-stimulatory autologous dendritic cells. It is to be understood that an expression is to be determined in comparison to the monocytes before they are subjected to the methods described herein.

The term "immuno-stimulatory autologous dendritic cells" refers to dendritic cells, which can be obtained by activating monocyte using the methods described herein and which show an increased expression of molecular markers, which are indicative of immuno-stimulatory autologous dendritic cells. These molecular markers, which are indicative of immuno-stimulatory autologous dendritic cells, have been discussed in the literature for dendritic cells, which can present antigens by way of MHC I and MHC II. It is to be understood that immuno-stimulatory autologous dendritic cells as obtainable by the methods described herein and identified by the molecular markers described herein may be considered as dendritic cells which have already differentiated enough and internalized and even display e.g. tumor-specific antigens from apoptotic cells such as cytotoxic T-cells, which are contained in the extracorporeal quantity of a respective mammalian subject's blood sample, or e.g. viral or bacterial antigens, which are contained in the extracorporeal quantity of a respective mammalian subject's blood sample, such that they can be considered to be immuno-stimulatory autologous antigen-presenting dendritic cells. However, the process can also be conducted in a way such that the dendritic cells express molecular markers indicative of immuno-stimulatory dendritic cells, which have not yet internalized and display antigens. The term "immuno-stimulatory autologous dendritic cells" in one embodiment thus encompasses immuno-stimulatory autologous antigen-presenting dendritic cells. It is to be understood that the designation of immuno-stimulatory dendritic cells as immuno-stimulatory autologous dendritic cells depends on whether the obtained immuno-stimulatory dendritic cells are later re-introduced into the same donor. This can be done in a continous or dis-continous fashion where the dendritic cells are cultivated for extended periods of time before they are re-introduced into the donor. All of the issues discussed hereinafter for immuno-stimulatory dendritic cells refer to immuno-stimulatory autologous dendritic cells. It is, however, to be understood that the discussion of such embodiments always includes the scenario where the processes are used to make immuno-stimulatory dendritic cells as such and where only the later administration of these cells will make them potentially immuno-stimulatory autologous dendritic cells.

The present invention allows preferential production of immuno-suppressive DC relative to immuno-stimulatory DC. The preferential production of immuno-suppressive dendritic cells relative to immuno-stimulatory dendritic cells means that starting from an extracorporeal amount of blood sample, more immuno-suppressive dendritic cells than immuno-stimulatory DC may be selectively obtained compared to a situation where e.g. the same extracorporeal amount of blood sample was subjected to a classical ECP procedure. Even though production of immuno-suppressive DC will be produced preferentially relative to immuno-stimulatory DC, immuno-stimulatory DC may be still present after the methods in accordance with the invention have been performed. Nevertheless, the present invention provides the parameters and variables that can be manipulated to skew production of one dendritic cell population over the other. Further, the eventually remaining immuno-stimulatory dendritic cells may be removed by e.g. affinity purification against molecular markers that are indicative of immuno-stimulatory dendritic cells.

As is described in the examples, molecular marker which are indicative of immuno-stimulatory autologous dendritic cells obtainable by the methods described herein were identified by subjecting monocytes contained in the extracorporeal quantity of mammalian subjects' blood samples derived either from healthy volunteers to the process using a miniaturized device (see markers 88 to 99 of Table 1). Further, as is also described in the example, molecular markers, which are indicative of immuno-stimulatory autologous dendritic cells, were identified by subjecting monocytes contained in the extracorporeal quantity of mammalian subjects' blood samples derived either from healthy volunteers or from patients suffering from CTCL to an ECP process. It is also described in the example, how molecular markers, which are indicative of immuno-suppressive dendritic cells, were identified by subjecting monocytes contained in the extracorporeal quantity of mammalian subjects' blood samples derived either from healthy volunteers or from patients suffering from GvH disease (GvHD)to an ECP process (see markers 1 to 87 of Table 1). The dendritic cells were then isolated and up-regulated expression of molecular markers, which are known or suspected to play a role in immuno-stimulatory dendritic cells, was analyzed.

Some of the markers identified for the ECP process, which is assumed to lead to a complex mixture of immune-stimulatory and immune-suppressive dendritic cells, are the same as they were observed for the dendritic cells obtained by the process with the miniaturized device, which should lead to immune-stimulatory dendritic cells only. Thus to the extent that the ECP process leads to up-regulation of molecular markers, which can be associated with dendritic cell function, it seems justified to assume that these markers will also be suitable to identify immune-stimulatory dendritic cells as they are obtainable by the processes described herein such as with the miniaturized device. A set of overall 99 molecular markers was identified as being upregulated for immuno-stimulatory autologous dendritic cells obtainable by methods described herein. This set may be extended by further molecular markers in the future through comparable analysis

Thus, the data of examples 1 and 3 lead to a set of 99 markers, which are considered indicative of immuno-stimulatory autologous dendritic cells. These markers are summarized in Table 1.

**Table 1**

| No. | Marker | NCBI Gene ID No. | mRNA REF | SEQ ID No. |
|---|---|---|---|---|
| 1 | ABCA1 | 19 | NM_005502.3 | 1 |
| 2 | ACVR1B | 91 | NM_004302.4 | 2 |
| 3 | ANPEP | 290 | NM_001150.2 | 3 |
| 4 | AQP9 | 366 | NM_020980.3 | 4 |
| 5 | ATP6V0B | 533 | NM_001039457.1 | 5 |
| 6 | BASP1 | 10409 | NM_001271606.1 | 6 |
| 7 | BEST1 | 7439 | NM_001139443.1 | 7 |
| 8 | CD63 | 967 | NM_001257389.1 | 8 |
| 9 | CD68 | 968 | NM_001040059.1 | 9 |
| 10 | CDCP1 | 64866 | NM_022842.3 | 10 |
| 11 | CPM | 1368 | NM_001005502.2 | 11 |
| 12 | CRK | 1398 | NM_005206.4 | 12 |
| 13 | CSF2RA | 1438 | NM_001161529.1 | 13 |
| 14 | CTNND1 | 1500 | NM_001085458.1 | 14 |
| 15 | CTSB | 1508 | NM_001908.3 | 15 |
| 16 | CXCL16 | 58191 | NM_001100812.1 | 16 |
| 17 | EMP1 | 2012 | NM_001423.2 | 17 |
| 18 | ENG | 2022 | NM_000118.2 | 18 |
| 19 | EPB41L3 | 23136 | NM_012307.2 | 19 |
| 20 | FLOT1 | 10211 | NM_005803.2 | 20 |
| 21 | GNA15 | 2769 | NM_002068.2 | 21 |
| 22 | GPNMB | 93695 | NM_053110.4 | 22 |
| 23 | GPR137B | 83924 | NM_031999.2 | 23 |
| 24 | GPR157 | 269604 | NM_177366.3 | 24 |
| 25 | HEXB | 3074 | NM_000521.3 | 25 |
| 26 | HOMER3 | 9454 | NM_001145721.1 | 26 |
| 27 | ICAM1 | 3383 | NM_000201.2 | 27 |
| 28 | IL1R1 | 3554 | NM_000877.2 | 28 |
| 29 | IRAK1 | 3654 | NM_001025242.1 | 29 |
| 30 | ITGA5 | 3678 | NM_002205.2 | 30 |
| 31 | ITGB8 | 3696 | NM_002214.2 | 31 |
| 32 | KCTD11 | 147040 | NM_001002914.2 | 32 |
| 33 | LAMP2 | 3920 | NM_001122606.1 | 33 |
| 34 | LEPROT | 54741 | NM_001198681.1 | 34 |
| 35 | LGALS3 | 3958 | NM_001177388.1 | 35 |
| 36 | LILRB4 | 11006 | NM_001081438.1 | 36 |
| 37 | MARCKSL1 | 65108 | NM_023009.6 | 37 |
| 38 | MCOLN1 | 57192 | NM_020533.2 | 38 |
| 39 | MFAP3 | 4238 | NM_001135037.1 | 39 |
| 40 | MGAT4B | 11282 | NM_014275.4 | 40 |
| 41 | MR1 | 3140 | NM_001194999.1 | 41 |
| 42 | MRAS | 22808 | NM_001085049.2 | 42 |
| 43 | MSR1 | 4481 | NM_002445.3 | 43 |
| 44 | NEU1 | 4758 | NM_000434.3 | 44 |
| 45 | NPC1 | 4864 | NM_000271.4 | 45 |
| 46 | OLR1 (LOX1) | 4973 | NM_001172632.1 | 46 |
| 47 | OMG | 4974 | NM_002544.4 | 47 |
| 48 | P2RX4 | 5025 | NM_001256796.1 | 48 |
| 49 | PI4K2A | 55361 | NM_018425.2 | 49 |
| 50 | PLAUR | 5329 | NM_001005376.2 | 50 |
| 51 | PMP22 | 5376 | NM_000304.2 | 51 |
| 52 | PPAP2B | 8613 | NM_003713.4 | 52 |
| 53 | PSEN1 | 5663 | NM_000021.3 | 53 |
| 54 | PVRL2 | 5819 | NM_001042724.1 | 54 |
| 55 | RAB13 | 5872 | NM_002870.2 | 55 |
| 56 | RAB8B | 51762 | NM_016530.2 | 56 |
| 57 | RAB9A | 9367 | NM_001195328.1 | 57 |
| 58 | RALA | 5898 | NM_005402.3 | 58 |
| 59 | RHEB | 6009 | NM_005614.3 | 59 |
| 60 | RNASE 1 | 6035 | NM_002933.4 | 60 |
| 61 | SC5DL | 6309 | NM_001024956.2 | 61 |
| 62 | SDC2 | 6383 | NM_002998.3 | 62 |
| 63 | SEMA6B | 10501 | NM_032108.3 | 63 |
| 64 | SIRPA | 140885 | NM_001040022.1 | 64 |
| 65 | SLC17A5 | 26503 | NM_012434.4 | 65 |
| 66 | SLC1A4 | 6509 | NM_001193493.1 | 66 |
| 67 | SLC22A4 | 6583 | NM_003059.2 | 67 |
| 68 | SLC31A1 | 1317 | NM_001859.3 | 68 |
| 69 | SLC35E3 | 55508 | NM_018656.2 | 69 |
| 70 | SLC39A6 | 25800 | NM_001099406.1 | 70 |
| 71 | SLC6A6 | 6533 | NM_001134367.1 | 71 |
| 72 | SLC6A8 | 6535 | NM_001142805.1 | 72 |
| 73 | SLC7A11 | 23657 | NM_014331.3 | 73 |
| 74 | STX3 | 6809 | NM_001178040.1 | 74 |
| 75 | STX6 | 10228 | NM_005819.4 | 75 |
| 76 | TM9SF1 | 10548 | NM_001014842.1 | 76 |
| 77 | TMBIM1 | 64114 | NM_022152.4 | 77 |
| 78 | TMEM33 | 55161 | NM_018126.2 | 78 |
| 79 | TNFRSF10B | 8795 | NM_003842.4 | 79 |
| 80 | TNFRSF11A | 8792 | NM_001270949.1 | 80 |
| 81 | TNFRSF1A | 7132 | NM_001065.3 | 81 |
| 82 | TNFRSF1B | 7133 | NM_001066.2 | 82 |
| 83 | TNFSF14 | 8740 | NM_003807.3 | 83 |
| 84 | TNFSF9 | 8744 | NM_003811.3 | 84 |
| 85 | TRIP10 | 9322 | NM_004240.2 | 85 |
| 86 | TRIP6 | 7205 | NM_003302.2 | 86 |
| 87 | YKT6 | 10652 | NM_006555.3 | 87 |
| 88 | DC-LAMP (LAMP3) | 27074 | NM_014398.3 | 88 |
| 89 | CLEC5A | 23601 | NM_013252.2 | 89 |
| 90 | SPC2 (PCSK2) | 5126 | NM_002594.3 | 90 |
| 91 | THBS1 | 7057 | NM_003246.2 | 91 |
| 92 | CD14 | 929 | NM_000591.3 | 92 |
| 93 | CD40 | 958 | NM_001250.4 | 93 |
| 94 | CD80 | 941 | NM_005191.3 | 94 |
| 95 | CCR7 | 1236 | NM_001838.3 | 95 |
| 96 | CD83 | 9308 | NM_001251901.1 | 96 |
| 97 | ADAM Decysin | 27299 | NM_014479.3 | 97 |
| 98 | FPRL2 (FPR3) | 2359 | NM_002030.3 | 98 |
| 99 | CD86 | 942 | NM_006889.4 | 99 |

Of the 87 genes (markers 1 to 87) that represent surface markers /functional mediators of immunostimulatory DC function, 66 were found to be uniquely identified in the ECP-induced process (plate passaged, overnight cultured, see example) dendritic cells, after comparison to expression databases for "classical" dendritic cells. These are: ABCA1 , ACVR1B, ATP6V0B, BASP1, BEST1, CPM, CRK, CSF2RA, CTNND1, CTSB, CXCL16, ENG, FLOT1, GNA15, GPR137B, GPR157, HEXB, HOMER3, ICAM1, IRAK1, ITGA5, ITGB8, KCTD11, LAMP2, LEPROT, MARCKSL1, MCOLN1, MFAP3, MGAT4B, MR1, MRAS, MSR1, NEU1, OLR1, OMG, PI4K2A, PLAUR, PMP22, PVRL2, RAB13, RAB8B, RAB9A, RALA, RNASE1, SC5DL, SEMA6B, SIRPA, SLC1A4, SLC22A4, SLC31A1, SLC35E3, SLC39A6, SLC6A6, SLC6A8, STX3, STX6, TM9SF1, TMBIM1, TMEM33, TNFRSF10B, TNFRSF11A, TNFRSF1A, TNFRSF1B, TNFSF14, TNFSF9, YKT6.

Immuno-suppressive dendritic cells can thus be distinguished from immune-stimulatory dendritic cells by determining expression of at least one molecular marker for the immuno-stimulatory autologous dendritic cells obtainable by the methods described herein and by comparing its expression for monocytes contained within the extracorporeal quantity of a mammalian subject's blood sample. If no increased expression of these markers is observed for presumed immuno-suppressive dendritic cells vs. monocytes, this is indicative of the differentiation of monocytes to immuno-suppressive dendritic cells.

Immuno-suppressive dendritic cells, in addition to increased expression of GILZ (SEQ ID No.: 100), are thus identifiable by determining expression of at least one molecular marker for the immuno-stimulatory autologous dendritic cells obtainable by the methods described herein and by comparing its expression for monocytes contained within the extracorporeal quantity of a mammalian subject's blood sample. If no increased expression of such molecular markers for dendritic cells vs. monocytes is observed, this is indicative of the differentiation of monocytes to immuno-suppressive autologous and/or allogenic dendritic cells. Additional markers which are indicative of immuno-suppressive dendritic cells when increasingly expressed include IDO (SEQ ID No.: 101), KMO (SEQ ID No.: 102), TGFβ (SEQ ID No.: 103) and/or IL-10 (SEQ ID No.: 104).

Immuno-stimulatory autologous dendritic cells are identifiable by determining expression for at least 2, 3, 4, 5, 6, 7, 8, 9, 10, 15, 20, 25, 30, 35, 40, 45, 50 or more molecular markers selectable from Table 1. For example, one may identify immuno-stimulatory autologous dendritic cells by determining expression for at least 1, 2, 3, 4, 5, 6, 7, 8, 9, 10, 11, 12, 13, 14, 15, 16, 17, 18, 19, 20, 21 or 22 molecular markers selectable from the group comprising PLAUR, NEU1, CTSB, CXCL16, ICAM1, MSR1, OLR1, SIRPA, TNFRSF1A, TNFSF14, TNFSF9, PMB22, CD40, LAMP3, CD80, CCR7, LOX1, CD83, ADAM Decysin, FPRL2, GPNMB and/or CD86. More preferably, one may identify immuno-stimulatory autologous dendritic cells by determining expression for at least 1, 2, 3, 4, 5, 6, 7, 8, 9, or 10 molecular markers selectable from the group comprising PLAUR, NEU1, CD80, CCR7, LOX1, CD83, ADAM Decysin, FPRL2, GPNMB and/or CD86. The most preferred markers which are considered indicative of immuno-stimulatory autologous dendritic cells are PLAUR, NEU1, CD80, CD83, and/or CD86. The data and conclusions presented herein suggest that the process of obtaining immuno-stimulatory dendritic cells seems to include a global monocyte activation step and a monocyte to immuno-stimulatory antigen-presenting cell (e.g. dendritic cell) differentiation step. These different steps seem to be traceable by molecular markers as described above and by Forward Scattering/Side Scattering Complexity (FSC/SSC Complexity) which is determinable by FACS analysis. The molecular markers may moreover be be grouped according to their know function as e.g. molecular markers of antigen-presentation, molecular markers of cellular adhesion etc.. HLA-DR, CD86, and CD 80 may be considered to representative of antigen-presentation. PLAUR, and ICAM-1 may be considered to representative of cell adhesion. Markers like HLA-DR, PLAUR and ICAM-1 as well as FSC/SSC complexity may be moreover considered to be indicative of global monocyte activation while increased expression of e.g. CD83, ADAM-Decysin, CD40, CD80, LAMP-3, and CCR7 seems indicative of monocyte to dendritic cell differentiation.

The immuno-stimulatory suppressive dendritic cells as they are obtainable by the methods described herein can thus not only be positively identified by the molecular marker, GILZ which is indicative of immune-suppressive dendritic cells and also optionally IDO, KMO, TGFβ, and/or IL-10, but also by the absence of up-regulation of molecular markers, which are indicative of immune-stimulatory dendritic cells such as PLAUR, CD80 and CD83. Further both of these cell types, i.e. immune-suppressive and immune-stimulatory dendritic cells can be distinguished from the monocytes, which are subjected to a physical force to induce the differentiation process thereof, by determining the expression of molecular markers which are considered indicative of monocytes such as CD33 , CD36, and/or FCGR1a (Receptor for IgGFc fragment 1A). If it is found that the expression of these factors is down-regulated compared to expression of the monocytes, before they have been subjected to a physical force and process as described herein, then this is considered indicative that the monocytes have entered the maturation and differentiation pathway towards immune-stimulatory and/or immuno-suppressive dendritic cells. The distinction between these later two dendritic cell population can then be made by determining expression of molecular markers such as PLAUR, CD80, CD83 and GILZ.

As mentioned above, the method as described hereinafter may be performed without the need of molecular cocktails to achieve maturation and differentiation of monocytes into immuno-suppressive autologous and/or allogenic dendritic cells. Such cocktails may comprise factors such as e.g. dexamethasone IL-10, IL-4 or TGF-β. However, in one embodiment it is considered to add such maturation cocktails to the immuno-suppressive autologous and/or allogenic dendritic cells as they are obtainable by methods in accordance with the invention, e.g. to push the differentiation towards a certain dendritic cell profile that can be achieved with such cocktails.

Given that one now has the understanding and correspondingly the tools, e.g. the molecular markers at hand to distinguish between immuno-suppressive autologous and/or allogenic dendritic cells and immuno-stimulatory autologous dendritic cells, one can now deliberately vary both the design of the device and the flow chamber through which the extracorporeal quantity of a mammalian subject's blood sample and thus the monocytes are passed to experience a physical force, and the parameters at which the process of inducing differentiation of monocytes into immuno-suppressive autologous and/or allogenic dendritic cells is performed, to purposively enable differentiation of monocytes into immuno-suppressive autologous and/or allogenic dendritic cells.

As mentioned above, an extracorporeal quantity of a mammalian subject's blood sample is passed through a flow chamber of a device, such that a shear force is applied to said monocytes contained within said mammalian subject's blood sample. Alterations of the design of the device and the flow chamber which have an influence on the differentiation of monocytes into immuno-suppressive autologous and/or allogenic dendritic cells include variation of flow forces, variation of the geometry of the flow path of the flow chamber, variation of the dimensions of the flow chamber, the possibility to adjust temperature, the possibility of exposure of the extracorporeal quantity of the mammalian subject's blood sample in the flow chamber to visible or UV light, etc.. Application of a physical force may not only be achieved by e.g. passing an extracorporeal amount of blood sample through a flow chamber, but also by placing such an extracorporeal amount of blood sample in e.g. an EVA plastic bag as obtainable from Macopharma and gently moving or shaking this blood sample-filled bag (see e.g. Andreu et al., (1994), Trans. Sci., 15(4), 443-454)

As also mentioned above and shown hereinafter, activation of monocytes and induction of differentiation into immuno-suppressive autologous and/or allogenic dendritic cells is dependent on interaction of monocytes with activated platelets and/or specific plasma components in a situation where the monocytes experience physical force which may be provided by a device as described hereinafter. Variation of process parameters thus include varying the nature, purity and concentrations of plasma components; the nature, purity and concentration of platelets; the order of steps by which plasma components and/or platelets are passed through and/or disposed on the flow chamber; the density by which the flow chamber is coated with plasma components and/or platelets, the flow forces of the extracorporeal quantity of the mammalian subject's blood sample and in particular the platelets and/or the monocytes are passed through the flow chamber of such a flow chamber, the temperature and/or time at which the extracorporeal quantity of the mammalian subject's blood sample and in particular the platelets and/or the monocytes are passed through the flow chamber of such a device, etc., the nature, purity and concentrations of additional factors such as 8-MOP and/or cytokines are added to the extracorporeal quantity of the mammalian subject's blood sample and in particular to the monocytes, etc.

Factors relating to the design of the device and the flow chamber as well as to process parameter will now be discussed in more detail as regards their relevance for the differentiation of monocytes into immuno-suppressive autologous and/or allogenic dendritic cells. It is to be understood that for any of the embodiments discussed in the following differentiation of monocytes into immuno-suppressive autologous and/or allogenic dendritic cells is achieved wherein immuno-suppressive autologous and/or allogenic dendritic cells are identified by determining expression of at least GILZ and/or optionally by determining expression of molecular markers of Table 1. If e.g. expression of GILZ is increased and if expression of molecular markers of Table 1 is not increased, this is considered indicative that monocytes have differentiated into immuno-suppressive autologous and/or allogenic dendritic cells. Further, for all embodiments discussed in the following it is to be understood that monocytes that are contained in an extracorporeal quantity of a mammalian subject's blood sample are subjected to shear stress in order to allow them to differentiate into dendritic cells, e.g. upon interaction with activated platelets and/or plasma components.

In an embodiment, the invention of claim 1 relates to a method of inducing differentiation of monocytes contained in an extracorporeal quantity of a mammalian subject's blood sample into immuno-suppressive autologous and/or allogenic dendritic cells, wherein said extracorporeal quantity of said mammalian subject's blood sample is subjected to a physical force by passing said extracorporeal quantity of said mammalian subject's blood sample through a flow chamber of a device, which allows adjustment of the flow rate of said extracorporeal quantity of said mammalian subject's blood sample through said flow chamber of said device such that a shear force is applied to said monocytes contained within said mammalian subject's blood sample.

In another embodiment, the invention of claim 1 relates to a method of inducing differentiation of monocytes contained in an extracorporeal quantity of a mammalian subject's blood sample into immuno-suppressive autologous and/or allogenic dendritic cells, wherein said extracorporeal quantity of said mammalian subject's blood sample is subjected to a physical force by passing said extracorporeal quantity of said mammalian subject's blood sample through a flow chamber of a device, which allows adjustment of the flow rate of said extracorporeal quantity of said mammalian subject's blood sample through said flow chamber of said device such that a shear force is applied to said monocytes contained within said mammalian subject's blood sample, and wherein said flow chamber of said device has a design allowing to apply a shear force to said monocytes contained within said mammalian subject's blood sample.

In another embodiment, the invention of claim 1 relates to a method of inducing differentiation of monocytes contained in an extracorporeal quantity of a mammalian subject's blood sample into immuno-suppressive autologous and/or allogenic dendritic cells, wherein said extracorporeal quantity of said mammalian subject's blood sample is subjected to a physical force by passing said extracorporeal quantity of said mammalian subject's blood sample through a flow chamber of a device, which allows adjustment of the flow rate of said extracorporeal quantity of said mammalian subject's blood sample through said flow chamber of said device such that a shear force is applied to said monocytes contained within said mammalian subject's blood sample, and wherein said device additionally allows for adjustment of at least one parameter selected from the group comprising temperature, and light exposure.

In another embodiment, the invention of claim 1 relates to a method of inducing differentiation of monocytes contained in an extracorporeal quantity of a mammalian subject's blood sample into immuno-suppressive autologous and/or allogenic dendritic cells, wherein said extracorporeal quantity of said mammalian subject's blood sample is subjected to a physical force by passing said extracorporeal quantity of said mammalian subject's blood sample through a flow chamber of a device as mentioned before and wherein said monocytes are activated and induced to differentiate into immuno-suppressive autologous and/or allogenic dendritic cells through interaction with activated platelets and/or plasma components.

For all of these embodiments and for all of the embodiment discussed in the following, the monocyte contained within the extracorporeal amount of a mammalian's blood sample are subjected to shear stress in the presence of DNA-cross linking agent such as 8-MOP and exposing the monocytes to UV light such that an increased expression of GILZ is effected. The design and dimensions of the flow chamber for all of these embodiments should be preferably selected such that substantially all of the monocytes are in contact with a DNA-cross linking agents such as 8-MOP and that substantially all of the monocytes are exposed to UV.

For example, in one embodiment, the invention of claim 1 relates to a method of inducing differentiation of monocytes contained in an extracorporeal quantity of a mammalian subject's blood sample into immuno-suppressive autologous and/or allogenic dendritic cells, wherein said method comprises at least the steps of:
a) applying said extracorporeal quantity of said mammalian subject's blood sample comprising at least monocytes to a device, which is configured to provide for a flow chamber through which said extracorporeal quantity of said mammalian subject's blood sample can be passed,
b) activating platelets, which may be comprised within said extracorporeal quantity of said mammalian subject's blood or which may be provided separate from said mammalian subject's blood sample comprising at least monocytes,
c) treating said extracorporeal quantity of said mammalian subject's blood sample comprising at least monocytes in said device by applying a physical force to the monocytes contained within said extracorporeal quantity of said mammalian subject's blood sample such that said monocytes are activated and induced to differentiate into immuno-suppressive autologous and/or allogenic dendritic cells by binding to said activated platelets obtained in step b).

In another embodiment, the invention of claim 1 relates to a method of inducing differentiation of monocytes contained in an extracorporeal quantity of a mammalian subject's blood sample into immuno-suppressive autologous and/or allogenic dendritic cells, wherein said method comprises at least the steps of:
a) applying said extracorporeal quantity of said mammalian subject's blood sample comprising at least monocytes to a device, which is configured to provide for a flow chamber through which said extracorporeal quantity of said mammalian subject's blood sample can be passed,
b) passing plasma components, which may be comprised within said extracorporeal quantity of said mammalian subject's blood sample or which may be provided separate from said mammalian subject's blood sample,
c) treating said extracorporeal quantity of said mammalian subject's blood sample comprising at least monocytes in said device by applying a physical force to the monocytes contained within said extracorporeal quantity of said mammalian subject's blood sample such that said monocytes are activated and induced to differentiate into immuno-suppressive autologous and/or allogenic dendritic cells by binding to said plasma components obtained in step b).

In yet another embodiment, the invention of claim 1 relates to a method of inducing differentiation of monocytes contained in an extracorporeal quantity of a mammalian subject's blood sample into immuno-suppressive autologous and/or allogenic dendritic cells, wherein said method comprises at least the steps of:
a) applying said extracorporeal quantity of said mammalian subject's blood sample comprising at least monocytes to a device, which is configured to provide for a flow chamber through which said extracorporeal quantity of said mammalian subject's blood sample can be passed,
b) passing plasma components, which may be comprised within said extracorporeal quantity of said mammalian subject's blood or which may be provided separate from said mammalian subject's blood sample,
c) activating platelets, which may be comprised within said extracorporeal quantity of said mammalian subject's blood sample or which may be provided separate from said mammalian subject's blood sample comprising at least monocytes,
d) treating said extracorporeal quantity of said mammalian subject's blood comprising at least monocytes in said device by applying a physical force to the monocytes contained within said extracorporeal quantity of said mammalian subject's blood sample such that said monocytes are activated and induced to differentiate into immuno-suppressive autologous and/or allogenic dendritic cells by binding to said activated platelets and/or plasma components obtained in steps b) and c).

In yet another embodiment, the invention of claim 1 relates to a method of inducing differentiation of monocytes contained in an extracorporeal quantity of a mammalian subject's blood sample into immuno- suppressive autologous and/or allogenic dendritic cells, wherein said method comprises at least the steps of:
a) optionally passing platelets-rich plasma through a a device, which is configured to provide for a flow chamber through which said extracorporeal quantity of said mammalian subject's blood sample can be passed,
b) applying said extracorporeal quantity of said mammalian subject's blood sample comprising at least monocytes to a device, which is configured to provide for a flow chamber through which said extracorporeal quantity of said mammalian subject's blood sample can be passed,
c) treating said extracorporeal quantity of said mammalian subject's blood comprising at least monocytes in said device by applying a physical force to the monocytes contained within said extracorporeal quantity of said mammalian subject's blood sample such that said monocytes are activated and induced to differentiate into immuno-suppressive autologous and/or allogenic dendritic cells optionally by binding to said platelets-rich plasma obtained of step a).

As can be taken from the experiment described herein, the method for inducing differentiation of monocytes contained in an extracorporeal quantity of a mammalian subject's blood into immuno-suppressive autologous and/or allogenic dendritic cells works optimal, if platelets which are comprised within said extracorporeal quantity of said mammalian subject's blood are activated and if the extracorporeal quantity of said mammalian subject's blood comprising at least monocytes in said device is treated by applying a physical force to the monocytes contained within said extracorporeal quantity of said mammalian subject's blood such that said monocytes are activated and induced to differentiate into dendritic cells by binding to said activated platelets. However, activation of monocytes may also be achieved by direct interaction with plasma components, i.e. without interaction with activated platelets. These dendritic cells can then be made embarking on differentiation into immuno-suppressive autologous and/or allogenic dendritic cells by exposing the monocytes additionally to DNA-cross linking agents such as 8-MOP and light such as UVA light.

The steps of activating platelets and the subsequent activation and differentiation of monocytes into dendritic cells will be discussed in the following for the embodiment that (i) plasma components such as plasma proteins are passed through the flow chamber of the device so that these components adhere to the walls of the flow chamber, that (ii) platelets are passed through the flow chamber and are activated by binding to the plasma components and that (iii) monocytes-containing fractions such as an extracorporeal quantity of said mammalian subject's blood comprising at least monocytes are passed through the flow chamber and are activated for differentiation into dendritic cells by binding to the activated platelets. It is, however, to be understood that these activities also occur if the plasma fraction or plasma proteins or fragments thereof, the platelet fraction and the monocytes-containing fraction are passed simultaneously through the channels or channel-like structures as is the case for a whole blood fraction if obtained from the extracorporeal amount of blood as described below. It is further to be understood that the process may be performed even though not with same effectiveness by adhering only plasma components to the walls of the flow chamber and letting monocytes interact with the plasma components. Nevertheless, in the following these aspect will be discussed for a preferred embodiment, i.e. where steps (i), (ii), and (iii) are realized.

As regards the first step, plasma components including proteins like fibrinogen or fibronectin, or fragments thereof like the gamma component of fibrinogen may be provided either as fractions obtained from the extracorporeal amount of blood sample or in purified form from other resources e.g. in the form of recombinantly expressed proteins. Even though it seems that activation of platelets by plasma proteins such as fibrinogen and fibronectin is sufficient so that recombinantly expressed forms of these proteins are sufficient, it can be preferred to use plasma fractions which are obtained from the extracorporeal amount of blood sample and comprise these proteins as these plasma fractions have a more complex composition and may comprise all plasma components, which provide for an optimal activation of platelets.

Plasma protein fractions, plasma proteins or fragments thereof may be passed through the flow chamber, which may be made of plastic or non-plastic materials such as glass in order to adhere to the walls of the channels or channel-like structures. There is no requirement that the plasma fractions or plasma proteins are passed through the flow chamber at a specific physical force such as e.g. a specific pressure. However, in order to streamline the process, it is envisaged to pass the plasma fractions or plasma proteins through the flow chamber at a shear stress which is comparable if not identical to the shear stress required for monocyte activation being described in more detail below. In general, the plasma fractions or plasma proteins are first pumped through the flow chamber to coat the surfaces thereof with plasma proteins, including fibronectin and fibrinogen. The flow rate of the plasma protein fractions, plasma proteins or fragments thereof through the flow chamber is controlled to obtain a desired level of protein adherence to the plastic surfaces. If desired, the flow can be stopped for a period of time and the plasma component can "soak" the surfaces of the flow chamber. By controlling the speed and timing of the pump that propels the plasma components through the flow chamber, the degree of coating of can be controlled. In one approach, the plasma fractions or plasma proteins are exposed to the surfaces of the flow chamber structures for a period between about 1 to 60 min, between about 1 to about 30 min, between about 1 to about 20 min, or between about 1 to about 10 min. To enhance plasma protein adherence to the surfaces of the flow chamber, the flow may be temporarily discontinued (for up to about 60 min), before resumption, or the flow rate may be slowed from the filling rate (up to 100 ml/minute) to as low as 5 ml/minute, during this phase of the procedure.

One can also envisage a scenario, where a device with a flow chamber is used for which the surfaces of the flow chamber have been pre-coated with e.g. purified plasma proteins or fragments thereof such as the gamma component of fibrinogen. Such pre-coated devices may be used if the whole process s conducted in a handheld device comprising a cartridge providing the flow chamber which is configured for e.g. one time use. One can also envisage a scenario, where a device with a flow chamber is used for which the surfaces of the flow chamber have been pre-coated with e.g. platelets-rich plasma.
After the plasma fractions or plasma proteins or fragments thereof have been passed through the channels or channel-like structures and the surfaces thereof have been coated with plasma proteins, the platelet fraction is passed by e.g. pumping into and through the flow chamber. The flow rate and residence time of the platelets within the flow chamber is selected to allow the platelets to bind to the plasma components or proteins or fragments thereof which have adhered before to the surfaces of the flow chamber and to thereby become activated.

The data presented herein suggest that activation of platelets by plasma components is a sequential process in which inactivated platelets first bind to the gamma component of fibronectin, get activated thereby and can then bind to the RGD motif (Arginine, Glycine, Aspartic Acid) which is found in many plasma proteins such as fibronectin or fibrinogen. If purified and/or recombinantly expressed plasma proteins or fragments thereof are used for activation of platelets, it can therefore be envisaged to pre-coat channels or channel-like structures with at least the gamma-component of fibrinogen and optionally additionally with RGD peptides. These plasma protein fragments and peptides may allow for efficient activation of platelets and at the same time for an optimal control of the coating process of the surfaces of the channels or channel-like structures. Of course, all of these components are present if a plasma fraction obtained from the extracorporeal amount of blood is used for coating and activation.

For efficient binding of the platelets to the plasma components and activation thereby, the flow rate may be adjusted upward or downward compared to the coating step of the plasma components, or flow may be stopped for a period of time, to obtain the desired level of platelets bound to the plasma components. The flow rates for plasma activation will typically be in the range of about 5 ml/min to about 200 ml/min, of about 10 ml/min to about 150 ml/min, of about 10 ml/min to about 100 ml/min, or of about 5 ml/min to about 50 ml/min. Typically, it will be desirable to allow between about 1 to 60 min, between about 1 to about 30 min, between about 1 to about 20 min, or between about 1 to about 10 min for the platelets to bind to the plasma components.

Even though shear stress does not seem to of the same importance for activation of platelets as for activation of monocytes, it can be preferred to pass the platelets fraction through the flow chamber under a shear force of about 0.1 to about 20.0 dynes/cm², of about 0.2 to about 15.0 dynes/cm², of about 0.3 to about 10.0 dynes/cm² such as from about 0.2 to about 0.4, to about 0.5, to about 0.6, to about 0.7, to about 0.8, to about 0.9, to about 1, to about 2, to about 3, to about 4, to about 5, or to about 6 dynes/cm². Typical flow rates of the platelets-containing fraction may be in the range of about 5 ml/min to about 200 ml/min, of about 10 ml/min to about 150 ml/min, of about 10 ml/min to about 100 ml/min, or of about 5 ml/min to about 50 ml/min. The flow rates will depend to some extent on the size and geometry of the flow chamber and can particularly be used if flow chamber of the below-mentioned dimensions are used. In general, one will select flow rates to achieve the afore-mentioned shear stress values.

Thus, it is contemplated to pass the platelets-containing fraction through the channels or channel-like structures with a flow rate of about 10 ml/minute to about 200 ml/minute to produce a shear force of about 0.1 to about 10.0 dynes/cm².

After the platelets have been passed through the channels or channel-like structures and have been activated by the plasma proteins or fragments thereof, which have been disposed on the surfaces of the channels or channel-like structures thereof, the monocytes-containing fraction, e.g. the extracorporeal quantity of said mammalian subject's blood sample or the below-mentioned leukocyte or buffy coat fraction, which have been obtained from the extracorporeal amount of blood sample, is passed by e.g. pumping into and through the channels or channel-like structures, by applying a physical force. It is to be understood that activation of platelets through interaction with plasma components will lead to adherence of platelets to plasma components.

It is also to be understood that the same events as described above will happen if an extracorporeal quantity of a mammalian subject's blood sample comprising platelets and plasma components is passed through the flow chamber. In this case, plasma components will adhere to the walls to the flow chamber and then activate platelets. However, in this scenario the process may be less controllable and account may be taken of this by increasing the residence time of the extracorporeal quantity of a mammalian subject's blood sample comprising platelets and plasma components in the flow chamber.

It is further to be noticed that instead of activated platelets, factors derived from platelets may be used, which are sufficient to monocytes. These factors include e.g. fibronectin and may also include factors such as P-selectin, Integrin α5β1the C-type lectin receptor, CD61, CD36, CD47 and complement inhibitors such as CD55 and CD59, or TREM-like transcipt-1. Such platelet-derived factors may also be disposed directly on the surfaces of the flow chamber either as e.g. mixtures of purified components or mixtures of components obtained by e.g. lysis of platelets contained within the extracorporeal quantity of a mammalian subject's blood sample. In this case, the need for e.g. coating the surfaces of the flow chamber with plasma components may be bypassed.

The data presented herein suggest that once platelets have been activated, proteins such as P-selectin and RGD-containing ligands are expressed by the activated platelets, which can then interact with monocytes and activate their differentiation into dendritic cells. Moreover, it was found monocyte activation and dendritic cell induction by activated platelets do not occur under static conditions. Rather monocytes need to be passed through the channels or channel-like structures under application of a physical force. Given that platelets upon activation need about 60 to about 120 min to express factors such as P-selectin, which then activates monocytes, passing of monocytes may be delayed until platelets have started to express these factors, e.g. for about 60 to about 120 min. If an extracorporeal quantity of a mammalian subject's blood sample comprising monocytes, platelets and plasma components is passed through the flow chamber, this time period may have to be adjusted to longer times.

It is to be understood that interaction of monocytes with activated platelets, platelet-derived factors or plasma components is not sufficient for activation and differentiation of monocytes without the application of a physical force at the same time.

Application of a physical force for moving the monocytes-containing fraction through the flow chamber means that a monocytes-containing fraction such as the extracorporeal quantity of a mammalian subject's blood sample is moved through the flow chamber under shear stress. Typically, monocytes-containing fraction may be passed through the flow chamber under a shear force of about 0.1 to about 20.0 dynes/cm², of about 0.2 to about 10.0 dynes/cm², such as from about 0.2 to about 0.3, to about 0.4, to about 0.5, to about 0.6, to about 0.7, to about 0.8, to about 0.9, to about 1, to about 1.5, or to about 2 dynes/cm². Typical flow rates of the monocytes-containing fraction may be in the range of about 5 ml/min to about 200 ml/min, of about 10 ml/min to about 150 ml/min, of about 10 ml/min to about 100 ml/min, or of about 5 ml/min to about 50 ml/min. The flow rates will depend to some extent on the size and geometry of the flow chamber and can particularly be used if channels or channel-like structures of the below-mentioned dimensions are used. In general, one will select flow rates to achieve the afore-mentioned shear stress values.

Thus, it is contemplated to pass the monocytes-containing fraction through the channels or channel-like structures with a flow rate of about 10 ml/minute to about 200 ml/minute to produce a shear force of about 0.1 to about 0.5 dynes/cm². In any case it must be made sure that a shear force is generated that allows binding of monocytes to activated platelets and differentiation of such activated monocytes into dendritic cells.

The data presented herein suggests that monocyte-platelet interaction can be divided into short-acting interactions which are arbitrarily defined as contact occurring for less than 3 seconds by detection with a light microscope and long-acting interactions, defined as contact longer than 3 seconds by detection with a light microscope. It seems that the initial short-acting interactions are mediated by P-selectin which is expressed on activated platelets. These initial contacts can then subsequently trigger long-acting interactions mediated by RGD-containing proteins expressed by the activated platelets.

The activation of monocytes and differentiation into dendritic cells may be positively influenced by allowing the monocytes to establish long-acting contacts with platelets, e.g. by giving the monocytes and platelets enough time to interact. As the monocytes flow through the channels or channel-like structures, they alternately bind to and disadhere from the platelets by the shearing force induced by the flow through the flow chamber. The residence time of the monocyte/platelet interaction may be controlled by varying the flow rate, e.g. by controlling the speed of the pump. For example, the pump may initially be operated at a slow speed/low flow rate to enhance monocyte/platelet interaction, and the speed/flow rate may then be increased to facilitate disadherence and collection of the treated monocytes from the treatment device. It seems that adherence of the monocytes to the platelets may be best accomplished at about 0.1 to about 2 dynes/cm², at about 0.1 to about 1 dynes/cm², and preferably at about 0.1 to about 0.5 dynes/cm², while disadherence and collection of the monocytes may be best accomplished at increased shear levels.

It is to be understood that activation of monocytes leads to immobilization, e.g. by interacting with activated platelets, platelets-derived factors or plasma components. In order to harvest the induced immuno-suppressive autologous and/or allogenic dendritic cells, one may increase the shear stress to e.g. 20 Dynes/cm² and/or may treat the immuno-suppressive autologous and/or allogenic dendritic cells with factors allowing disadherence from activated platelets, platelets-derived factors or plasma components by adding factors such as Plavic, Aspirin or other blood thinners.

Temperature is another factor to influence activation of monocytes and their differentiation into dendritic cells. The methods in accordance with the invention may be performed in a range of about 18°C to about 42°C, preferably in a range of about 22°C to about 41°C and more preferably in a range of about 37°C to about 41°C.

One parameter that can also be varied to tune activation of monocytes is the density by which the flow chamber is coated with plasma components and thus with platelets that bind to the plasma components. In general, the denser the surfaces of the flow chamber are coated with plasma components and platelets, the more efficient will be the monocyte activation.

It has been mentioned above, that platelets are activated by binding to plasma components. The term "activated platelets" in accordance with the invention is used to refer to platelets which show an increased expression of P-selectin, αIIb-β3 integrin and/or RGD-containing proteins such as fibronectin, fibrinogen or vitronectin as a consequence of binding of platelets to plasma components such as fibronectin and/or fibrinogen. Expression may be determined by conventional methods such as RT-PCR, Western-Blotting or FACS analysis. The term "unactivated platelets" in accordance with the invention is used to refer to platelets for which binding to plasma proteins such as fibronectin or fibrinogen cannot be reduced by pre-incubating platelets with the gamma component of fibrinogen.

It has been mentioned above, that monocytes are activated and start to differentiate into dendritic cells by binding to activated platelets under shear stress conditions. The term "activated monocytes" in accordance with the invention is used to refer to monocytes which upon binding to activated platelets under shear stress conditions express increased levels of the open confirmation of β1 -integrin and start expressing markers of maturing dendritic cells such as HLA-DR⁺/CD83⁺. As a control to determine whether interaction of monocytes with activated platelets leads to activation and differentiation of dendritic cells one can compare expression of HLA-DR⁺/CD83⁺ after binding of monocytes to activated platelets under shear stress condition either in the absence of anti-P-selectin antibodies (activation) or presence of anti-P-selectin antibodies (control). Expression may be determined by conventional methods such as RT-PCR, Western-Blotting or FACS analysis. If one the additionally applies DNA-cross linking agents such as 8-MOP and exposes the activated monocytes to light such as UVA, one can direct the differentiation process towards immuno-suppressive autologous and/or allogenic dendritic cells and thus to increase expression of GILZ. As one can take from the data of example 2, such cells even if treated with LPS will be resistant to differentiation into immuno-stimulatory autologous dendritic cells and will thus not continue to express HLA-DR, CD83, CD80 and CD86 at high levels, but rather will maintain the tolerogenic state.

After monocytes have been activated by a method in accordance with the invention, they start differentiating into immuno-suppressive autologous and/or allogenic dendritic cells. The term "immuno-suppressive autologous and/or allogenic dendritic cells s" in accordance with the invention is used as mentioned above. These immuno-suppressive autologous and/or allogenic dendritic cells are identified by increased expression of e.g. GILZ and preferably additionally no increased expression of molecular markers of Table 1.

The experimental findings described herein further immediately suggest various embodiments of this first aspect that can provide for different advantages.

The finding, that activation of monocytes and subsequent induction of differentiation of these monocytes into immuno-suppressive autologous and/or allogenic dendritic cells can be achieved in a miniaturized device, allows to conduct the process with smaller amounts of an extracorporeal blood sample. As mentioned above, the classical ECP procedure requires processing of 2.5 L to 6 L blood, which is typically obtained from patients by apheresis such as leukaphereses, to obtain a final volume of about 200 ml to 500 ml comprising leukocytes including monocytes as well as plasma components and platelets.

However, the methods in accordance with the invention may require substantial lower amount of blood samples thus bypassing the need of apheresis such as leukaphereses or other processes, which are a considerable burden to patients.

Thus, the present invention can be performed without the need for apheresis such as leukaphereses and the whole process of obtaining such immuno-suppressive autologous dendritic cells may be performed in a handheld device.

Thus, in one embodiment of the first aspect of the invention, which may be combined with the above described embodiments, it is contemplated to perform the method in accordance with the first aspect, wherein said extracorporeal quantity of said mammalian subject's blood has not been obtained by apheresis such as leukaphereses.

Said extracorporeal quantity of said mammalian subject's blood may be between about 5 ml to about 500 ml, between about 10 ml to about 450 ml, between about 20 ml to about 400 ml, between about 30 ml to about 350 ml, between about 40 ml to about 300 ml, or between about 50 ml to about 200 ml or between about 50 ml to about 100 ml of extracorporeal blood of said mammalian subject to give a final volume between about 1 ml to about 100 ml, between about 1 ml to about 50 ml, between about 1 ml to about 40 ml, or between about 1 ml to about 30 ml an extracorporeal amount of a mammalian's blood sample.

The quantity of extracorporeal blood withdrawn and applied to the device may be whole blood. Alternatively, said extracorporeal quantity of said mammalian subject's blood may be obtained by isolating leukocytes from between about 5 ml to about 500 ml, between about 10 ml to about 450 ml, between about 20 ml to about 400 ml, between about 30 ml to about 350 ml, between about 40 ml to about 300 ml, or between about 50 ml to about 200 ml or between about 50 ml to about 100 ml of extracorporeal whole blood of said mammalian subject.

Said extracorporeal quantity of said mammalian subject's blood may also be obtained by isolating buffy coats from between about 5 ml to about 500 ml, between about 10 ml to about 450 ml, between about 20 ml to about 400 ml, between about 30 ml to about 350 ml, between about 40 ml to about 300 ml, or between about 50 ml to about 200 ml or between about 50 ml to about 100 ml of extracorporeal whole blood of said mammalian subject.

In all of the afore-mentioned cases (whole blood, leukocyte fraction, buffy coats), said extracorporeal amount of blood will typically comprise between about 1x10⁴ to about 1x10⁸ such as about 5x10⁶ mononuclear cells/ml.

The person skilled in the art is familiar how to obtain whole blood, a leukocyte fraction thereof or a buffy coat fraction thereof (see e.g. Bruil et al., Transfusion Medicine Reviews (1995), IX (2), 145-166) an include filtration, differential centrifugation. A preferred method relies on filters, as they are available from e.g. Pall. Such filters may be incorporated into the device such that processing of the extracorporeal sample can be done in the handheld device. As a source one can also use e.g. blood of the umbilical cord.

If one uses centrifugation, one may obtain whole blood through a syringe with e.g. a 17 or 18 gauge-gauge needle. Such a whole blood sample may be centrifuged to remove debris and other components. The whole blood sample may then be filtered through common filters, as they are available from Pall.

For obtaining a mononuclear leukocyte fraction, one may obtain a whole blood sample as described and then layer such a sample on e.g. Ficoll-Hypaque. Subsequently a centrifugation step is performed at e.g. about 100g to about 200 g such as 180 g and the mononuclear leukocyte fraction can then be collected from the interface and washed with common buffers such as HBSS. The washed mononuclear leukocyte fraction can then be resuspended in serum-free cell culture medium such as RPMI-1640 medium (GIBCO). Other methods for obtaining mononuclear leukocyte fractions include elutriation, filtration, density centrifugation, etc..

As pointed out above, crucial steps for the induction of dendritic cells formation seem to involve the activation of platelets by plasma components and the activation of monocytes by such activated platelets. In principle, one could pass a whole blood sample through the device under shear stress. The plasma components of such a sample will then bind to the surfaces of the flow chamber and allow for adherence and activation of platelets within such a sample by plasma-components. The monocytes of such a sample will then bind to the activated platelets and be activated themselves.

Similarly one may obtain combinations of the various components such as a platelet-rich plasma containing fraction which may be obtained by centrifuging a whole blood sample which has been obtained as described above at about 100 g to about 180 g such as about 150 g for about 10 min to about 20 min such as about 15 min to separate the debris of the whole blood sample. The platelet-rich plasma layer is then collected and recentrifuged at about 700 g to about 1000 g such as about 900 g for about 3 min to about 10 min such as about 5 min. The resultant pellet is then resuspended in serum-free cell culture medium.

However, in order to have the best control over the process, it may be desirable to first pass plasma components through the flow chamber and let them adhere, then platelets and then the monocytes-containing fraction. For this approach, it may be desirable to obtain a leukocyte fraction comprising a monocytes- or buffy-coat fraction comprising monocytes, which does not comprise plasma components and which does not comprise platelets. Such plasma- and platelet-free monocytes-containing fractions may be obtained as is described in the art. If leukocyte or buffy-coat fractions are obtained as described above, they will be sufficiently free of plasma or platelets for the purposes of the invention. For this approach, it may also be desirable to have platelet- and/or plasma-fractions.

Thus, the invention contemplates to use platelets which have been separated from the extracorporeal quantity of said mammalian subject's blood before said extracorporeal quantity of said mammalian subject's blood is applied to said device. These platelets may then be passed through the flow chamber, which has been coated with plasma components such as fibronectin.

In another embodiment, the invention considers to use plasma components, which have been separated from the extracorporeal quantity of said mammalian subject's blood before said extracorporeal quantity of said mammalian subject's blood is applied to said device. These plasma components may then be passed through flow chamber so that they can adhere.

Instead of using plasma components which have been obtained from the extracorporeal amount of blood, one may also use plasma components, which have been isolated from other sources such as e.g. by recombinant protein expression. Such plasma components include fibrinogen, fibronectin, P-selectin, and fragments thereof such as the gamma component of fibrinogen.

Even though it may be preferred to use an extracorporeal amount of blood, which has not been obtained by apheresis such as leukaphereses, using an extracorporeal amount of blood, which has been obtained by apheresis such as leukaphereses is not excluded by the invention.

Thus, in another embodiment of the first aspect of the invention it is contemplated to perform the method as described above, wherein said extracorporeal quantity of said mammalian subject's blood has been obtained by apheresis such as leukaphereses.

Apheresis such as leukaphereses may be performed as is known in the art. Thus, an extracorporeal quantity of blood such as 2.5 L to 6 L may be obtained from a subject and treated by conventional leukaphereses to obtain three fractions, namely the plasma, the platelets and the buffy coats. The plasma, which contains proteins such as fibronectin and fibrinogen, is the lightest blood fraction, and therefore is the first portion of the blood selectively removed from the centrifuge and passaged through channels or channel-like structures. After the plasma has been pumped through the channels or channel-like structures and the surfaces thereof have been coated with plasma proteins, the second lightest component in the leukaphereses centrifuge, the platelet fraction, is pumped into and through the channels or channel-like structures. The third lightest fraction to be eluted from the leukaphereses centrifuge is the buffy coat, which contains the white blood cells, including the blood monocytes. The buffy coat including the monocytes is then pumped through the channels or channel-like structures. Blood sample may be obtained using the Therakos device, the Spectra cell separator (see Andreu et al., (1994), Transf. Sci., 15(4), 443-454), or the Theraflex device from Macopharma.

Thus, the invention in one embodiment the invention considers to use platelets which have been separated from the extracorporeal quantity of said mammalian subject's blood obtained by apheresis such as leukaphereses before said extracorporeal quantity of said mammalian subject's blood comprising monocytes is applied to said device.

In another embodiment the invention considers to use plasma components, which have been separated from the extracorporeal quantity of said mammalian subject's blood obtained by apheresis such as leukaphereses before said extracorporeal quantity of said mammalian subject's blood comprising monocytes and/or platelets is applied to said device.

Instead of using plasma components which have been obtained from the extracorporeal amount of blood, one may use also either plasma components which have been isolated from other sources such as e.g. by recombinant protein expression. Such plasma components include fibrinogen, fibronectin, or P-selectin. One can also use fragments of plasma proteins such as the gamma component of fibrinogen which corresponds to amino acids 400-411 (SEQ ID NO.: 105, His-His-Leu-Gly-Gly-Ala-Lys-Gln-Ala-Gly-Asp-Val). This gamma component is shown by the data presented herein to be able to activate platelets. It can therefore be preferred to use plasma fractions which at least, if not predominantly comprise fibronectin. Similarly, it can be preferred to use e.g. recombinantly expressed and/or purified fibronectin or the gamma component thereof to activate platelets.

For both embodiments of the first aspect of the invention where the extracorporeal amount of blood has been obtained or has not been obtained by apheresis such as leukaphereses, it may be considered to pass all three fractions, namely plasma components, platelets and the monocytes-containing fraction at once, e.g. even in the form of a whole blood sample or by using only pre-purified fractions of whole blood, through the flow chamber even though the afore-described sequential passing of these fractions through the flow chamber may provide for better control over the process. Pre-purified fractions of whole blood may be obtained by e.g. centrifuging a blood bag and squeezing out the supernatant, which would be enriched in white blood cells and platelets.

As mentioned the flow rate through flow chamber and thus the resulting shear stress will effect the differentiation of the monocytes into dendritic cells. Further, application of photo-activatable DNA-cross linking agents such as 8-MOP and exposure to light such as UVA light is used to induce increased expression of GILZ. Aside from the flow rate, the design and the dimensions of the flow chamber may be varied to manipulate and even improve the application of a physical force to the monocytes as well as to make sure that substantially all monocytes can be contacted with DNA-cross linking agents such as 8-MOP and exposed to light such as UVA..

A device having a flow chamber with channels or channel-like structures may be suitable. Such a flow chamber having the general architecture, albeit at smaller dimensions, of a device, which is used for the classical ECP procedure is depicted in Fig. 17.

However, other geometries such as those depicted in Fig. 18 a) to d) may also be used. Thus, the findings described herein allow to consider flow chambers of significantly simplified geometry, which also allows having better control over the process in terms of turbulences and shear stress occurring during the process.

A device having a multiplicity of flow chambers may be suitable. Such a flow chamber having the general architecture, albeit at smaller dimensions, of a device, which is used for the classical ECP procedure is depicted in Fig. 17.

Typically, a flow gradient will be created in the flow chamber such as channels as the monocytes-containing fraction is passed through. The monocytes will alternately bind to and disengage from the platelets and/or plasma components. Maturation of monocytes into dendritic cells is greatly enhanced by this interaction, with increased exposure to the platelets and/or plasma components thereby providing increased signaling of this maturational process.

In order to obtain a homogenous population of immuno-suppressive autologous and/or allogenic dendritic cells as possible, it is therefore desirable that the design and the dimensions of the flow chamber, such as channels is selected avoid different flow zones in the flow chamber.

The flow chamber such as channels may in principle have any cross-sectional shape suitable for the above described purposes. They thus may have a rectangular, round, elliptical, or other cross-sectional form. Even though the dimensions of such flow chamber will be discussed in the following mainly with respect to a rectangular cross-section, it can be preferred that flow chamber such as channels with an elliptical or round cross-section are used as such cross-sections should allow for e.g. more homogenous coating with plasma components and/or more continuous flow properties with less turbulences.

If having a rectangular cross-sections, flow chamber such as channels may have dimensions of about 5 µm to up to about 500 µm of height and of about 5 µm to up to about 500 µm of width. The channels or channel-like structures may also have dimensions of about 10 µm to up to and including about 400 µm of height and of about 10 µm to up to and including about 400 µm of width, of about 10 µm to up to and including about 300 µm of height and of about 10 µm to up to and including about 300 µm of width, of about 10 µm to up to and including about 250 µm of height and of about 10 µm to up to and including about 250 µm of width, of about 10 µm to up to and including about 100 µm of height and of about 10 µm to up to and including about 100 µm of width, or of about 10 µm to up to and including about 50 µm of height and of about 10 µm to up to and including about 50 µm of width.

If flow chambers such as channels of elliptical cross-section are used, the afore-mentioned dimensions of height and width would have to be adapted correspondingly to allow for a comparable volume.

If flow chambers such as channels of round cross-sections are used, the diameter may typically be in the range of about 5 µm to up to and including about 500 µm, of about 10 µm to up to and including about 400 µm, of about 10 µm to up to and including about 300 µm, of about 10 µm to up to and including about 250 µm, of about 10 µm to up to and including about 100 µm, or of about 10 µm to up to and including about 50 µm.

Smaller dimensions are generally preferred for the flow chambers with a particular preference for height, widths or diameters of below 100 µm such as 50 µm the reason being that it is assumed that for such smaller dimensions interaction of monocytes with platelets is more efficient and uniform and flow properties at the surfaces and in the center of the flow chamber are more comparable.

The length of the flow chamber such as channels channel-like structures is usually selected such that the flow chamber allows for passage of the volume of extracorporeal blood. For example the flow chamber and the device may be configured to allow for passing of an overall volume of between about 1 ml to about 50 ml, between about 1 ml to about 40 ml, or between about 1 ml to about 30 ml.

The flow chamber may have internal sub structures to increase the surface area or to make the flow conditions less heterogeneous.

The flow chamber may be filled with particles to increase the surface area or to make the flow conditions less heterogeneous.

The material of the flow chamber may be plastic or non-plastic.

If non-plastic materials are considered, one may use glass.

The surface of the chamber may be coated covalently or via adsorption.

Materials for auxiliary tubing, chambers, valves etc. may be selected to for having reduced interactions with blood components.

Surfaces of auxiliary tubing, chambers, valves etc. may be treated/coated for having reduced interactions with blood components.

If plastic materials are considered, one may use acrylics, polycarbonate, polyetherimide, polysulfone, polyphenylsulfone, styrenes, polyurethane, polyethylene, teflon or any other appropriate medical grade plastic. In a preferred embodiment of the present invention, the flow chamber is made from an acrylic plastic.

The flow chamber may be made of a material that provides a degree of transparency such that the sample within the flow chamber such as the monocytes-containing fractions can be irradiated with UV light, preferably with UV-A. As is shown by the experiments, exposure to UV-A and 8-MOP leads to increased expression of GILZ and thus to activation and differentiation of monocytes into immuno-suppressive autologous and/or allogenic dendritic cells. Thus exposure to UV light and DNA-cross linking agents such as 8-MOP should be optimized when producing immuno-suppressive autologous and/or allogenic dendritic cells.

However, once monocytes have embarked on the maturation pathway long enough such that immuno-suppressive autologous and/or allogenic dendritic cells have formed as can be determined by the molecular markers mentioned above, one can envisage to stop adding DNA-cross linking agents such as 8-MOP and to stop exposing the immuno-suppressive autologous and/or allogenic dendritic cells to e.g. UV-A. As shown in Example 2, once the immuno-suppressive autologous and/or allogenic dendritic cells have formed as is determinable by increased expression of GILZ, they are resistant to e.g. LPS-induced maturation. However, by contacting autologous and/or allogenic leukocytes, which are contained within the extracorporeal amount of a mammalian's blood sample and which have become apoptotic by e.g. the UVA administration, GILZ expression of the immuno-suppressive autologous and/or allogenic dendritic cells can be further increased.

Thus, one can e.g. incubate immuno-suppressive autologous and/or allogenic dendritic cells with such apoptotic leukocytes such that that the immuno-suppressive autologous and/or allogenic dendritic cells will take up and display antigens from the apoptotic leukocytes to obtain immuno-suppressive autologous and/or allogenic antigen-presenting cells. Given that the immuno-suppressive autologous and/or allogenic dendritic cells have already embarked on a tolerogenic state, such immuno-suppressive autologous and/or allogenic antigen-presenting cells can then be used for treatment of autoimmune diseases, hypersensitivity diseases, rejection in solid-organ transplantation or treatment of Graft-versus-Host disease.

A typical flow chamber may have the geometry depicted in Fig. 19A). The flow path has dimensions of 20 mm by 80 mm. The chamber is made of polystyrene, PET (polyethylenteherephtalate), PMMA (poly (methyl mathacrylate)) and silicon. A blood sample may be spun at low speed through a Ficoll gradient to obtain e.g. 8 ml of sample with a concentration of white blood cells of e.g. 10¹⁰ cells/ml. The chamber may be pre-coated with platelets-rich plasma. The sample may be passed through the chamber at about 0.028 Pa for about ... min. The chamber may then be washed with about 3 ml RPMI at 0.028 Pa. A second wash with 30-55 ml RPMI may be performed at about 1.2 Pa. The collected activated monocytes will then be combined and used for further analysis.

In this way immuno-suppressive autologous and/or allogenic dendritic cells and immuno-suppressive autologous and/or allogenic antigen-presenting cells can be obtained without the need for rather expensive cocktails of cytokines, which moreover use unphysiologically high concentrations and require cell incubation for a couple of days, for induction of monocyte to dendritic cell differentiation. Even though not necessary, it can be considered to cultivate such immuno-suppressive autologous and/or allogenic dendritic cells and immuno-suppressive autologous and/or allogenic antigen-presenting cells in a buffered culture medium with one or more cytokines and/or chemokines, such as IL-10, IL-4 or TGF-β during the incubation period, to further enhance differentiation into the tolerogenic state. Cultivation, if considered, may be performed under standard conidtions, e.g. at 37° C and 5% CO₂ in standard mediums for culturing of human cells such as in RPMI-1640 medium (obtainable e.g. from GIBCO), supplemented with 15% AB serum (obtainable from e.g. Gemini Bio-Products).

Further, immuno-suppressive autologous and/or allogenic dendritic cells can then be manipulated ex vivo, prior to re-administration to the subject, in order to tailor them for the desired therapeutic purpose.

Thus, prior to re-administration to the subject, such immuno-suppressive autologous and/or allogenic dendritic cells can e.g. be processed ex vivo, such as by loading them with antigens, being derived from cells involved in autoimmune diseases, hypersensitivity diseases, solid-organ transplantation rejection and Graft-versus-Host disease. Such antigens may e.g. provided in the form of apoptotic cells comprising these antigens. This will lead to immuno-suppressive autologous and/or allogenic antigen-presenting cells. One of the advantages of the invention provided by the insight presented herein is that obtaining immuno-suppressive autologous and/or allogenic dendritic cells can be separated from loading with antigens, being derived from cells involved in autoimmune diseases, hypersensitivity diseases, solid-organ transplantation rejection and Graft-versus-Host disease. In this way, immuno-suppressive autologous and/or allogenic dendritic cells can be isolated and tailored for specific purposes.

The immuno-suppressive autologous and/or allogenic dendritic cells may in particular be loaded with disease antigens to produce antigen presenting dendritic cells which upon re-introduction into the subject will suppress an immune response against the antigens.

For treatment of autoimmune diseases the antigens may be selected for diseases such as wherein the autoimmune disease is selected from the group comprising rheumatoid arthritis, psoriasis, multiple sclerosis, type 1 diabetes and systemic lupus erythematosus.

The immuno-suppressive autologous and/or allogenic dendritic cells obtained in accordance with the invention and disease effector agents are incubated for a period of time sufficient to maximize the number of functional antigen presenting dendritic cells in the incubated cell population. Typically, the treated blood cell concentrate and possibly antigens are incubated for a period of from about 1 to about 24 hours, with the preferred incubation time extending over a period of from about 12 to about 24 hours. Additional incubation time may be necessary to fully mature the loaded immuno-suppressive antigen-presenting cells prior to reintroduction to the subject. Preferably, the blood cell concentrate and disease effector agents are incubated at a temperature of between 35° C and 40° C. In a particularly preferred embodiment, the incubation is performed at about 37°C.

As mentioned above, by loading immune-suppressive dendritic cells obtainable by the methods described herein with antigens allows producing immuno-suppressive antigen-presenting cells.

In order to avoid e.g. protein degradation of delivered antigen and inefficient processing of soluble antigens by dendritic cells, it is contemplated to enhance formation of immuno-suppressive antigen-presenting cells by encapsulation of antigens in polymeric nanoparticles (NPs), which may be made from biodegradable polymers such as polylactic acid (Waeckerle-Men et al., Adv Drug Deliv Rev (2005), 57:475-82). Such NPs may be further modified with targeting moieties for DEC-205 such as an DEC-205 antibody to improve receptor-mediated endocytosis and antigen presentation.

Thus the invention contemplates to use encapsulation of antigens in polymeric nanoparticles to optionally further enhance formation of immuno-suppressive antigen-presenting cells.

Inducing monocyte differentiation according to the method described above provides immuno-suppressive autologous and/or allogenic dendritic cells which equal or exceed the numbers of dendritic cells that are obtained by expensive and laborious culture of leukocytes in the presence of cytokines such as IL-10, IL4 or TGF-β for a couple of days. The large numbers of functional dendritic cells generated by the method described above provide a ready means of presenting selected material, such as, for example, apoptotic cells, disease antigens, antigens, plasmids, DNA or a combination thereof, and are thereby conducive to efficient immunotherapy.

As mentioned above, immuno-suppressive autologous and/or allogenic dendritic cells can be preferably obtained by a method in accordance with the invention in the presence of a photoactivatable DNA-cross linking agent such as 8-MOP and by exposure to light such as UV-A.

The present invention thus aims at obtaining individual-specific functionally and maturationally synchronized immuno-suppressive autologous and/or allogenic dendritic cells.

The present disclosure also relates to immuno-suppressive autologous and/or allogenic dendritic cells obtainable by a method described herein, preferably for use in treating autoimmune diseases, hypersensitivity diseases, solid-organ transplantation rejection and Graft-versus-Host disease. The autoimmune disease may be selected from the group comprising rheumatoid arthritis, psoriasis, multiple sclerosis, type 1 diabetes and systemic lupus erythematosus.

The present disclosure further relates to a method of treating autoimmune diseases, hypersensitivity diseases, solid-organ transplantation rejection or Graft-versus-Host disease by administering to a patient in need thereof immuno-suppressive autologous and/or allogenic dendritic cells obtainable by a method described herein.

The invention is now described with respect to some specific examples which, however, are for illustrative purposes and not to be construed in a limiting manner.

### Experiments

### Experiment 1 - Shear stress and platelet activation for inducing monocyte activation

### Materials and Methods

### Procurement of leukocytes and platelets

All samples were acquired from young, healthy subjects not taking medications, including aspirin, known to influence platelet function. Samples were obtained under the guidelines of the Yale Human Investigational Review Board, and informed consent was provided according to the Declaration of Helsinki. Peripheral blood specimens were collected through a 19-gauge needle from the antecubital vein into syringes containing heparin, then layered on Ficoll-Hypaque (Gallard-Schlessinger, Carle Place, N.Y.). Following centrifugation at 180g, the interface containing the mononuclear leukocyte fraction was collected and washed twice in HBSS, then resuspended in RPMI-1640 medium (GIBCO) to a final concentration of 5 x 10⁶ mononuclear cells/ml. Cells were utilized within one hour of being acquired.

### Preparation of Platelet-rich-Plasma

Whole blood was centrifuged at 150 g for 15 min at room temperature. The platelet-rich-plasma (PRP) layer was collected and centrifuged at 900 g for 5 min, and the platelet pellet resuspended in RPMI 1640 to the desired concentration.

### Preparation of Parallel-Plates

Two likes of parallel-plate flow chambers were used to model the flow dynamics of ECP. Experiments involving the assessment of cell phenotype post-flow were conducted using the larger Glycotech system (Glycotech, Rockville, MD). This system consisted of a volumetric flow path measuring 20000 x 10000 x 254 microns (length x width x height). The bottom plate in this system was composed of a 15mm petri dish (BD Biosciences, Durham, NC) separated by a gasket and vacuum-connected to an acrylic flow deck, which formed the upper plate. For experiments requiring the plates to be pre-coated with platelets, prior to assembling the flow chamber, 20 drops of the desired concentration of PRP was placed in the center of the petri dish and platelets allowed to settle for 20 minutes at room temperature. The petri dish was washed twice with 2ml of RPMI, and the flow chamber then assembled.

For experiments not involving the collection and phenotyping of cells post-flow, Vena8 biochips (Cellix Ltd, Dublin, Ireland) were used to generate laminar flow. The volumetric flow path for a channel of the Vena8 biochips measured 20000 x 400 x 100 microns (length x width x height). Protein coating of these chips is described in the appropriate section below.

### Experiments using Parallel-Plates

The parallel-plate flow chamber was mounted on the stage of a phase contrast optical microscope (CK40, Olympus, Japan) with a 10x objective. All runs were performed at room temperature. A uniform laminar flow field was simulated by use of a syringe pump (KD Scientific, New Hope, PA) capable of generating near-constant volumetric flow rates. The components of the configuration were devised to minimize tubing. Prior to infusing cell suspensions through the plates, the system was washed with 5 ml of RPMI at a flow rate producing a wall shear stress of approximately 1 dyne/cm². Cell suspensions of interest were then passed through the chamber at a fixed flow rate and wall shear stress.

All experiments were viewed in real time, recorded at 15.2 frames per second using a DP 200 digital camera and software (DeltaPix, Maalov, Denmark), and analyzed using Image J software (NIH).

### Overnight culture

When overnight culture was required, cells were centrifuged and resuspended in RPMI-1640 medium (GIBCO), supplemented with 15% AB serum (Gemini Bio-Products) to a final concentration of 5 x 106 cells/ml. Cells were cultured overnight for 18 hours in 12-well polystyrene tissue culture plates (2 ml per well) at 37° C in 5% CO2.

### Immunophenotyping

Monoclonal antibodies for immunophenotyping included CD14 (LPS receptor; monocytes), CD11c (integrin subunit; monocytes and DC), HLA- DR (class II MHC molecule), CD83 (DC marker), CD62p (P-selectin; activated platelets), and CD61 (integrin subunit; platelets). Antibodies were obtained from Beckman Coulter (CD14, CD11c, HLADR, CD83) or Sigma (CD62p, CD61) and used at their pre-determined optimal dilutions. Background staining was established with appropriate isotype controls, and immunofluorescence was analyzed using a FC500 flow cytometer (Beckman Coulter). Two-color membrane staining was performed by adding the pre-determined optimal concentrations of both antibodies directly conjugated to FITC or PE and incubating for 20 min at 4°C, followed by washing to remove unbound antibodies. Combined membrane and cytoplasmic staining was performed following manufacturer's instructions for cell fixation and permeabilization (Intraprep kit, Beckman Coulter).

### Quantitative real-time PCR

Gene expression was compared between cells exposed during flow through the parallel plates to low (10 ± 5/low power field [lpf]) versus high (102 ± 32/lpf) levels of platelets, followed by overnight culture. Cell RNA was isolated using RNeasy Mini Kit columns with on-column DNase I treatment (QIAGEN). RNA yield and purity were measured using a NanoDrop ND-1000 Spectrophotometer and an Agilent 2100 Bioanalyzer. RNA was reverse transcribed to cDNA using the High Capacity cDNA Reverse Transcription Kit (Applied Biosystems). Reverse transcription was carried out in a 96-well thermocycler (MJ Research PTC-200) in the following conditions: 25°C, 10 minutes, 37°C, 120 minutes, 85°C, 5 seconds. TaqMan real-time PCR was used to detect transcripts of DC-LAMP, CD40, ADAM Decysin, Lox1, CCR7, CD80, CD83, CD86, FPRL2, and GPNMB. Primers and probes for each sequence were obtained as inventoried Taqman Gene Expression Assays (Applied Biosystems). HPRT1 was used as a reference gene.

### Co-cultures of Platelets with Monocytes

Experiments involving co-cultures of monocytes with additional platelets were performed as described in the Overnight Culture section, with a few necessary modifications. Following Ficoll-Hypaque separation, mononuclear cells were resuspended in 30% AB serum/RMPI to a final concentration of 10 x 106 cells/ml, of which 1 ml was allocated to each well of a 16- well plate. An additional 1ml of platelets (suspended in RPMI, at 2x the desired final concentration) or RPMI without platelets was then added to each well. To activate platelets, 500 µl containing 2 units of thrombin was added to half the wells, and 500 µl of RPMI was added to the others to balance the volume. Cells were then incubated as described previously.

### Platelet Adhesion Studies

Platelet adhesion experiments were performed using the Vena8 flow chamber described above. Fibrinogen and fibronectin (Sigma) were dissolved in PBS to a final concentration of 200 mcg/ml. Channels of the Vena8 chips were incubated at room temperature in a humidified chamber for 2 hours with the protein solution, autologous plasma, or PBS alone. The channels were washed with 5x the volume RPMI. Platelet-rich-plasma was then perfused through the protein-coated channel at the indicated shear-stress, held constant. For each channel, still images were acquired exactly 90 seconds into the experiment at 4 pre-defined low power fields located along the flow path (fields were centered at 2500, 7500, 12500, and 17500 microns from the start point of infusion).

Some experiments involved pre-treating platelet-rich-plasma with protein fragments prior to infusion through the channels. Small RGD peptides, containing the amino-acid sequence Arg-Gly-Asp-Ser; DRG peptides, contain the amino-acid sequence Ser-Asp- Gly-Arg; or fragment 400-411 of fibrinogen, containing the amino-acid sequence His- His-Leu-Gly-Gly-Ala-Lys-Gln-Ala-Gly-Asp-Val, were incubated at a concentration of 2mM with PRP for 20 minutes at room temperature. The PRP was then perfused through the channels as previously described.

### Receptor-Ligand Studies

Platelet-coated Vena8 channels were pre-treated with either 40 µg/ml anti-P-selectin (R&D Systems) or 40 µg/ml of an isotype control for 30 minutes at room temperature, then washed with 5x the volume RPMI. Mononuclear cell suspensions were pre-treated with either RGD or DGR peptides at a concentration of 2.5 mM. Video samples lasting 400 frames (26.3 seconds) were recorded 60 seconds after commencement of flow using a lower power field of view spanning 400 microns and centered at 7500 microns from the flow start point.

β-1 integrin conformation was assessed using the Glycotech flow chamber. 15mm platelet-coated petri dishes (described above) were pre-treated with 40 µg/ml anti-P-selectin or an isotype control for 20 minutes at room temperature, then washed with 5x the volume RPMI. Immediately following perfusion through the platelets, cells were immunophenotyped with anti-CD29 HUTS-21 (BD Biosciences), an antibody that specifically binds to the active (open) conformation of β1 integrins.

### Results

### Monocytes in flow transiently interact with immobilized platelets

ECP was initially developed as a means to enable extracorporeal chemotherapeutic exposure of pathogenic leukocytes to ultraviolet A (UVA)-activated 8-methoxypsoralen (8-MOP), a DNA-cross-linking drug. Therefore, ECP involves the flow of leukapheresed blood between large transparent plastic parallel-plates separated by 1 mm. To permit detailed analysis of the flow dynamics involved during ECP, independent of UVA/8-MOP exposure, the flow conditions of ECP were reproduced using miniature parallel plates with surface area of only 0.8 mm², separated by 100 microns. This model permitted visualization using digital microscopy. Studies using the model revealed the following sequence (determined by video analysis): initial adherence of platelets from the flow stream to the plate, followed by transient binding of passaged monocytes to the immobilized platelets.

### DC induction correlates with the number of monocyte-platelet interactions

Based on the initial qualitative observations described above, platelets were hypothesized to induce monocyte-to-DC differentiation under conditions of flow. To test the influence of platelets on monocyte-to-DC differentiation, monocytes were passed between parallel plates pre-coated with autologous platelets at low (10 ± 5/low power field [lpf]), medium (44 ± 20/lpf), and high (102 ± 32/lpf) densities. Cells were passed through the plates at a flow rate producing a wall shear stress of 0.5 dyne/cm², analogous to the wall shear stress in post-capillary venules. The number of monocyte-platelet interactions per unit time increased in proportion to augmented density of platelets (determined by video analysis). An average of 52.3 + 15 monocyte-platelet interactions per lpf per second were observed with the high-density plate, dropping to 18.3 ± 14 and 3.4 ± 1 interactions per second with the medium and low-density plates, respectively (Figure 1a).

Following overnight incubation, a correlation was found between the percentage of cells which developed a DC phenotype and the frequency of monocyte-platelet physical interactions observed the previous day (Figure 1b). An increasing number of monocyte-platelet interactions correlated with increasing proportion of cells expressing markers consistent with DC differentiation, membrane HLA-DR and CD83. An average of 14.2% of monocytes exposed to the high-density platelet-coated plate were HLA-DR+/ CD83+ after overnight incubation, compared to 4.9% and 0.8% of monocytes exposed to plates coated with medium and low levels of platelets, respectively.

### Monocyte exposure to platelets results in changes in gene expression

To supplement the described changes in monocyte phenotype observed following platelet exposure, RT-PCR was performed to assess for changes in gene expression. Monocytes were passed through parallel plates coated with high or low densities of platelets as described in the previous section. Following overnight incubation, RNA was extracted and RT-PCR performed to determine level of expression for 10 genes associated with DC (Figure 2). CD40, a costimulatory molecule with known expression on mature dendritic cells (Cella et al., 1996, see reference list), was found to be upregulated by over 567% in monocytes exposed to high densities of platelets relative to monocytes exposed to low levels. LAMP3, a marker specific to DC differentiation (de Saint-Vis at al., 1998, see reference list), was upregulated by 398%. CD80 is a costimulatory molecule known to be upregulated upon APC activation (Slavik et al., 1999, see reference list) upregulated by 220% in monocytes exposed to high levels of platelets. CCR7, a chemokine receptor known to play a role in DC migration to lymphoid organs, was upregulated by 376%. LOX1, CD83, CCR7, and ADAM Decysin, all genes associated with DC (Berger et al., 2010, see reference list), were also upregulated in the monocytes exposed to high levels of platelets. FPRL2, GPNMB, and CD86 were all downregulated in monocytes exposed to high levels of platelets. FPRL2 is a receptor that when activated is known to inhibit DC maturation (Kang et al., 2005, see reference list) GPNMB is a protein involved in decreasing cytokine production (Ripoll et al., 2007, see reference list); CD86 is a costimulatory molecule expressed by APCs.

### DC induction in the presence of platelets does not occur under static conditions

Platelets could potentially influence monocytes through direct receptor-ligand interaction, or via cytokines and other secreted mediators. To determine whether the platelet induction of monocyte-to-DC differentiation requires flow dynamics, we tested the role of platelets under static conditions. Monocytes were co-cultured with low (<50,000/mm³), medium (100-200,000/mm³) and high (>400,000/mm³) concentrations of platelets, with platelets in either an inactive or active state (induced by the addition of thrombin). After overnight incubation in static conditions (shear stress = 0), we found that neither activated nor non-activated platelets were capable of inducting DC differentiation of monocytes in the absence of flow (see Figure 3).

### Platelets suspended in flow bind to serum proteins adsorbed onto the plate

Several proteins abundantly present in plasma, including fibronectin and fibrinogen, are well known adsorb onto glass and plastic surfaces; the contribution of adherent plasma proteins on platelet adhesion and activation was therefore assessed. Parallel plates were pre-coated with either fibrinogen, fibronectin, plasma, or saline. Unactivated platelets were then passed through at shear rates producing wall shear stresses ranging from 0.2 to 6.0 dyne/cm². The highest concentrations of platelets adhered to plates coated with fibrinogen (Figure 4). Adhesion to fibronectin-coated, plasma-coated, and uncoated plates was observed as well, but to a significantly lower extent (p < 0.05). In the absence of flow, platelet adherence was equivalent on all protein substrates.

Both fibrinogen and fibronectin contain segments with the amino acid sequence arginine(R)-gylcine(G)-aspartate(D), RGD. RGD segments are well-known to interact with many integrin receptors, particularly the I/A domain of beta subunits, which are exposed when the integrins are in the active conformation (Xiong et al., 2002, see references). In experiments using fibrinogen-coated plates, platelet adhesion was not significantly altered by pre- incubation of platelets with RGD peptides; however, adhesion was significantly decreased (p < 0.05) by pre-incubation of platelets with peptide fragments corresponding to amino acids 400-411 of fibrinogen, the gamma component of the protein (Figure 5 a). In experiments using fibronectin-coated plates, pre-incubating platelets with RGD peptides decreased adhesion significantly, while pre-incubating platelets with peptide fragments corresponding to amino acids 400-411 of fibrinogen had no effect, (Figure 5b). Interestingly, it should be noted that unlike the I/A domain of integrins, which is known to interact with RGD domains of proteins, the region of the integrin found to interact with the gamma component of fibrinogen is exposed in the integrin's inactive state (Weisel et al., 1992, see references). Therefore, this data suggests that unactivated platelets in flow bind to the gamma-component of fibrinogen-coated plates. The potential for platelets in the unactivated state to bind fibrinogen may explain the greater level of platelet adhesion seen on fibrinogen-coated plates explained in the previous paragraph.

### Platelets are activated by adhesion to the plate

Platelets physiologically circulate in an inactive state, with an array of proteins stored in intracellular granules. Upon encountering stimuli such as damaged endothelium or thrombin, platelets become activated and almost instantaneously translocate these intracellular proteins to the plasma membrane (Kaplan et al., 1979, see references). It was postulated that platelet adhesion to the plastic plate/absorbed proteins caused platelet activation similar to that caused by well-known stimuli. To test this hypothesis, surface expression of P-selectin, a well-known marker of platelet activation, was assessed before and after adhesion. Prior to adhesion, 6 ± 3% of platelets were found to express P-selectin, with a mean fluorescence intensity (MFI) of 12.4 ± 6.9; following adhesion, P-selectin positivity increased to 64 ± 13% (MFI 98.2 ± 14). The positive control, platelets activated with thrombin, was 71 ± 18 % P-selectin positive (MFI 108.3 ± 23). Expression of P-selectin was further assessed at 30, 60, and 90 minutes following platelet adhesion; P-selectin expression remained stable at all time points, with 72 ± 11% of platelets P-selectin positive 90 minutes after adhesion, indicating that platelets remain in an active state for the duration of the procedure. Similar trends were found in assessment of αIIb-β3, a fibrinogen-binding integrin, with surface expression of this protein increasing from 4 ± 3% prior to adhesion, to 49 ± 18% post-adhesion.

### Monocytes interact with P-selectin and RGD-containing ligands expressed on activated platelets

The monocyte-platelet interactions observed on video were divided into two categories: (1) short-acting, arbitrarily defined as contact occurring for less than 3 seconds (46 frames), and (2) long-acting, defined as contact longer than 3 seconds, including stable binding. Since it had been previously determined that the platelets in the ECP system were in an activated state, and that activated platelets express an array of proteins including P- selectin and RGD containing proteins (e.g. fibronectin, fibrinogen, and vitronectin), it was sought to determine the involvement, if any, of these proteins in either short or long- duration interactions. Plates were pre-coated with platelets, and four conditions tested: (1) platelets pre-treated with an irrelevant isotype control, and monocytes untreated (P+RGD+); (2) platelets pre-treated with an irrelevant isotype control, and monocytes pre-incubated with RGD peptides (P+RGD-); (3) platelets pre-treated with anti-P-selectin, and monocytes untreated (P-RGD+); (4) platelets pre-treated with anti- P-selectin, and monocytes pre-treated with RGD peptides (P-RGD-). It was assumed that pre-treating monocytes with RGD peptides should result in a decreased in the number of free RGD- recognizing receptors available to interact with RGD-containing proteins expressed by the platelets. Thus, the four conditions tested represent every permutation of potential interaction with two platelet ligands, P-selectin and RGD-containing-proteins. As shown by Figure 6, both short-acting and long-acting interactions were maximal when neither RGD nor P-selectin were blocked (P+RGD+); the level of interaction in all other conditions was expressed as a percentage of this maximum. Blocking with anti-P-selectin alone (P-RGD+) resulted in a decrease of both short and long monocyte- platelet interactions to almost zero (p < 0.01; Figure 6, also confirmed by video analysis). In contrast, blocking RGD alone (P+RGD-) did not significantly alter the number of short-duration interactions, but decreased the long-duration monocyte-platelet interactions by 44% (p < 0.05; Figure 6). Blocking both P-selectin and RGD simultaneously (P-RGD-) resulted in a pattern similar to that seen when only P-selectin was blocked, with both long and short duration interactions reduced to near zero. The most appropriate conclusions, based on the pattern of interactions observed in each of the four conditions, are as follows: (1) P-selectin is predominantly responsible for the short-duration interactions; (2) RGD-containing proteins expressed by the platelet are involved in long-duration interactions, but not short-duration interactions; (3) monocyte interaction with P-selectin must occur upstream of monocyte interaction with RGD-containing proteins expressed by platelets. This last conclusion is based on the observation that conditions of P-RGD+ decreased both short and long duration interactions to near zero, while P+RGD-conditions only decreased long-duration interactions. If the interactions were not sequential, conditions of P- RGD+ should have produced similar results to P+RGD+ in terms of long-duration interactions. Furthermore, the ordering of the interactions, i.e. that P-selectin acts upstream of RGD-interactions, is apparent by the finding that conditions of P+RGD- only influenced long duration interactions, while conditions of P-RGD+ produced similar results to those of P-RGD-.

### Monocyte exposure to P-selectin results in downstream monocyte integrin-activation

Integrin receptors, in their open conformation, are known to interact with RGD-containing ligands (Ruoslathi et al., 1996, see references). Using an antibody that recognizes an epitope exposed only when the β1 integrin is in its open conformation, we assessed the conformation of monocyte integrins before and after flow through the model. Figure 7 shows that as the number of short-acting monocyte-platelet interactions increased, there was corresponding increase in the percentage of monocytes expressing integrins in their open conformation immediately post-flow. The black line shows that an average of 71% of monocytes which had received a high number of platelet-interactions (> 61± 19/lpf x sec) expressed β1 in the active form, compared to 9% of monocytes which had received a low number of platelet interactions (< 5.1 + 2 /lpf x sec). These results were not significantly affected by pre-treating the adherent platelets with an irrelevant isotype control (gray line). In contrast, pre-treating platelets with anti-P-selectin reduced the monocyte-platelet interactions to near zero, and monocytes emerging from flow in these conditions (dashed line) displayed low levels of active β1 integrins, irrespective of the density of platelets to which they were exposed. It is noteworthy that all cell populations prior to passage through the plates demonstrated similar low levels of baseline integrin activation (<10%); therefore, differences seen in short-duration monocyte-platelet interactions were not the result of differences in integrin conformation preflow.

### Monocyte exposure to P-selectin is required for DC differentiation

Given the dependence of monocyte-platelet interactions on platelet P-selectin, we set out to determine if there was a relationship between monocyte exposure to P-selectin at time 0, and the phenotype later developed by the monocyte after overnight incubation, time 18-hours (Figure 8). Monocytes were passed though parallel plates coated with high densities (108 + 36/lpf) of platelets that were either untreated (unblocked), or pretreated with either anti-P-selectin or an isotype control. 15.5 ± 4 % of monocytes exposed to unblocked platelets became membrane HLA-DR+/CD83+ (markers of maturing DC) after overnight incubation, and 13 + 4 % of the those exposed to platelets blocked with the irrelevant isotype control. In contrast, only 3 ± 2% of the monocytes exposed to platelets blocked with anti-P-selectin became HLA-DR+/CD83+ after overnight incubation.

### Experiment 2 - Identification of further molecular markers for immuno-suppressive dendritic cells

### Materials and Methods

### Sample Collection and Monocyte Enrichment

Peripheral blood specimens were acquired from healthy subjects under the guidelines of the Yale Human Investigational Review Board, and informed consent was provided according to the Declaration of Helsinki. PBMC were isolated by centrifugation over a Ficoll-Hypaque gradient (Isolymph, CTL Scientific). Monocytes were enriched from freshly isolated PBMC by: 1) plastic adherence for dexamethasone dose-titration experiments (purity: 71.6 ± 5.6% CD14⁺); 2) CD14 magnetic bead positive selection (Miltenyi Biotec) for PUVA dose-titration experiments (purity: 88.1 ± 3.5% CD14⁺), and; 3) Monocyte Isolation Kit II (Miltenyi Biotec) for LPS stimulation experiments (purity: 83.8 ± 3.8% CD14⁺).

### Generation of Monocyte-Derived Dendritic cells (MoDC)

Monocytes were cultured at a density of 5 x 10⁶ cells/mL in 6- and 12-well polystyrene tissue culture plates at 37°C and 5% CO₂ in RPMI-1640 (Gibco) supplemented with heat-inactivated 15% AB serum (Gemini) and 1% penicillin/streptomycin (now referred to as complete media). 800 IU/mL recombinant human GM-CSF (R&D Systems) and 1000 IU/mL recombinant human IL-4 (R&D Systems) were added to cultures for 36 hr to induce monocyte to DC differentiation as described.

### 8-MOP and UVA Light Treatment

Cultures were incubated with 8-MOP (Uvadex, 20 µg/mL) for 30 min in the dark, and then irradiated with a desktop UVA light box containing a series of 12 linear fluorescent tubes. The tubes emitted UVA light ranging from 320 to 400 nm. The UVA irradiance (power, W/m²) was measured using a photodiode. Given a measured irradiance and the absorption properties of the various components of the system, it was possible to determine the time (sec) needed to expose the cells to deliver a given dose of UVA radiation (J/cm²).

### MoDC/Lymphocyte Co-Cultures

Non-adherent cells (purity: 66.0 ± 4.5% CD3⁺) removed during plastic adherence will now be generally referred to as lymphocytes. Lymphocytes were treated with 8-MOP (100 ng/mL) and UVA (1 J/cm²), washed with PBS, and co-cultured in complete media at 37°C and 5% CO₂ with either PUVA-treated or untreated-MoDC in a ratio of 5 or 10 lymphocytes to 1 MoDC. MoDC treated for 24 hr with 100 nM dexamethasone (Sigma) served as the positive control group. After 24 hr, cells were harvested and MoDC were re-purified. To ensure that RNA was not isolated in significant amounts from lymphocytes, it was critical to re-purify MoDC from all cultures using CD11c magnetic bead (Miltenyi Biotec) positive selection (purity: 96.4 ± 1.0% CD11c⁺). CD11c⁺ MoDC were re-plated at 0.5-1.0 x 10⁶ cells/mL in complete media and stimulated with 100 ng/mL LPS (Sigma). 24 hr after LPS stimulation, cells were harvested for RNA isolation and immunophenotyping, and supernatants were collected for cytokine quantification. As negative controls, parallel groups did not receive LPS.

### siRNA Experiments

Silencer select pre-designed and validated GILZ siRNA (Invitrogen), with off-target prediction algorithms, was used to knockdown GILZ expression. Mo-dendritic cells were transfected using Lipofectamine RNAiMAX Reagent (Invitrogen). RNAᵢ duplex and lipofectamine reagent were incubated together for 20 min, then added to MoDC cultures and incubated for 2 hr at 37°C and 5% CO₂. Transfected MoDC were treated in an identical fashion as described for the MoDC/lymphocyte co-cultures. MoDC were also transfected with scramble siRNA.

### Immunophenotyping

Monoclonal antibodies included HLA-DR, CD80, CD83, CD3, CD86, CD14, CD11c and GILZ. Antibodies were obtained from Beckman-Coulter and eBioscience and were used at their pre-determined optimal dilutions. Apoptosis was assessed using the Annexin-V Apoptosis Detection Kit (eBioscience), with Annexin-V recognizing phosphatidylserine (PS) on the surface of apoptotic cells. 7-AAD substituted for PI as the cell viability dye. Cells displaying an Annexin-V⁺/7-AAD⁻ phenotype were classified as early apoptotic cells, and cells displaying an Annexin-V⁺/7-AAD⁺ phenotype were classified as late apoptotic cells. Dual membrane and intracytoplasmic staining was performed using the IntraPrep fix and permeabilization kit (Beckman-Coulter). Background staining was established with appropriate isotype and fluorescence minus one controls. Immunofluorescence was analyzed using a FACSCalibur L (BD Biosciences) within 2 hr of fixation with 2% paraformaldehyde. A minimum of 10,000 events were collected for each group.

### Quantitative Real-Time PCR

RNA was isolated from CD11c⁺ MoDC using QIAShredder columns (QIAGEN) and the RNeasy Mini Kit (QIAGEN) with on-column DNase I treatment (QIAGEN). RNA yield and purity were assessed using a NanoDrop ND-1000 spectrophotometer. cDNA was obtained using the High Capacity cDNA Reverse Transcription Kit (Applied Biosystems) in a 96-well thermocycler (MJ Research PTC-200). TaqMan real-time PCR was used to detect transcripts of GILZ, CD80, and CD86. Primers and probes were obtained as pre-designed and validated Taqman Gene Expression Assays (Applied Biosystems). SYBR green real-time PCR (Applied Biosystems) was used to detect transcripts of IL-12, IL-10, IL-6, TNF-alpha, and TGF-β. Primers were designed to span intron junctions using Primer3Plus. Primer melting curves were obtained to confirm a single product. HPRT-1 and GAPDH were used as reference genes. Samples were run in triplicate on a 7500 Real Time PCR System (Applied Biosystems). The delta-delta C(t) method was used to calculate the fold change.

### Cytokine Quantification

Culture supernatants were analyzed in a multiplex format utilizing magnetic beads to IL-6, IL-8, IL-10, IL-12p70, IFN-γ, TNF-α, RANTES, MCP-1, and MIP-1β (BioRad Laboratories). For siRNA experiments, supernatants were analyzed with enzyme-linked immunosorbent assay (ELISA) kits for IL-10 (R&D Systems) and IL-12p70 (Enzo Life Science). All samples and standards were run in duplicate and analyzed using the LUMINEX 200 (LUMINEX), or the BioTek EL800 (BioTek).

### Statistical Analysis

Student's *t*-tests were used for statistical comparisons between groups, with *p*-values < 0.05 considered statistically significant. Differential gene expression was considered statistically significant with a ≥ 2.5-fold change and a *p*-value < 0.05.

### Results

### Expression of GILZ is rapidly down-regulated as monocytes differentiate into immature MoDC

Freshly isolated CD14⁺ monocytes express GILZ, but rapidly down-regulate GILZ by more than 99% as they differentiate into immature MoDC (Figure 10A). A reduction in GILZ mRNA was confirmed by a 61% decrease in GILZ protein levels (Figure 10B). GILZ down-regulation correlated with reduced expression of CD14 (monocyte-specific marker, see Zhou et al., references), and increased expression of cytoplasmic CD83, (immature MoDC marker, see Klein et al., references). Importantly, MoDC remained immature, expressing low membrane CD83 (mature DC marker, see Renzo et al., references, *p* = 0.16). MoDC up-regulate GILZ after treatment with dexamethasone (dex) in a dose-dependent manner (Figure 10C). Treatment with 100 nM dex for 24 hr was selected as the positive control for inducing GILZ expression in MoDC (Dex-dendritic cells) (Figure 10D).

8-MOP or UVA treatment alone did not effect GILZ expression (Figure 10E). However, when MoDC were treated with the combination of 8-MOP and UVA light (PUVA-dendritic cells), GILZ expression increased 5.5-fold. The induction of GILZ exhibited a slow time course, peaking 24 hr after treatment, and remaining significantly elevated for 72 hr (Figure 10F). In comparison, Dex-dendritic cells up-regulated GILZ as little as 2 hr after treatment.

### Immature MoDC treated with the combination of 8-MOP and UVA light up-regulate GILZ and assume a tolerogenic, immuno-suppressive phenotype

It was next examined if there was a PUVA dose-dependent effect on GILZ expression. MoDC treated with 1 J/cm² UVA and 100 or 200 ng/mL 8-MOP up-regulated GILZ 2.9- and 4.4-fold respectively (Figure 11A). A similar dose-dependent phenomenon was observed with 2 J/cm², starting at an 8-MOP concentration of 50 ng/mL. Treatment with 0.5 J/cm² had no effect on GILZ expression until the 8-MOP concentration reached 200 ng/mL, and treatment with 4 J/cm² resulted in high levels of non-specific cell death (data not shown). The number of photo-adducts formed per 10⁶ base pairs is directly related to the product of the 8-MOP concentration and UVA dose, see Gasparro et al., references. As the product of 8-MOP and UVA reached 100, GILZ was up-regulated 3-fold, and as the product increased to 200 and 400, GILZ was up-regulated 4.8- and 8.6-fold respectively (Figure 11B).

The percentage of early apoptotic CD11c⁺ cells was minimally (p > 0.05) higher at 2 J/cm² as compared to 1 J/cm² for all doses of 8-MOP tested (Figure 11C). At 2 J/cm² and 200 ng/mL, there was an increase in the percentage of early apoptotic CD11c⁺ cells as compared to untreated MoDC (Figure 11C). The percentage of late apoptotic CD11c⁺ cells remained less than 13% at 1 J/cm², and less than 16% at 2 J/cm² for all doses of 8-MOP tested (Figure 11D). Moreover, dot plots highlight the relative resistance of MoDC to the pro-apoptotic effect of escalating doses of PUVA (Figure 11E). The number of cells recovered from cultures did not statistically differ in any group treated with 1 or 2 J/cm² (data not shown), and greater than 90% CD11c⁺ cells (range 91.0-97.5%) were harvested after treatment.

In contrast, lymphocytes display Annexin-V as early as 2 hr after treatment with 1 J/cm² and 100 ng/mL (data not shown). In contrast to MoDC treated with 100 ng/mL and 1 J/cm² (Figure 11F), 24 hr after treatment with the same dose of PUVA, the percentage of early apoptotic lymphocytes increased from 6.6% in untreated MoDC to 44.3% in PUVA-dendritic cells, and the percentage of late apoptotic lymphocytes increased from 4.5% to 33.7% (Figure 11G). Given that 64.3 ± 3.2% of lymphocytes were Annexin-V⁺ 24 hr after treatment, PUVA-treated lymphocytes are subsequently referred to as apoptotic lymphocytes (ApoL).

The PUVA dose-dependent induction of GILZ correlated with a decrease in cell surface expression of CD80, CD86, and CD83 (Figure 12A, 3B). Down-regulation of these markers paralleled the induction of GILZ (see Figure 11B), beginning at 8-MOP concentrations of 100 ng/mL for both 1 and 2 J/cm². As the product of 8-MOP and UVA exceeded 100, CD83, CD80 and CD86 expression were reduced by 31%, 30% and 54% respectively, and HLA-DR expression increased by 38%.

### MoDC exposed to apoptotic lymphocytes up-regulate GILZ and are resistant to LPS-induced full maturation

To dissect the individual contributions of PUVA and exposure to apoptotic cells, MoDC were first co-cultured with varying ratios of ApoL. GILZ was up-regulated in an ApoL dose-dependent fashion (Figure 13A). When PUVA-dendritic cells were exposed to ApoL, GILZ was expressed at higher levels than in PUVA-dendritic cells cultured alone (Figure 13B). PUVA-dendritic cells exposed to ApoL also expressed GILZ at higher levels than in untreated MoDC exposed to ApoL (6.7-fold and 3.6-fold higher, respectively). There was a corresponding 1.5-fold increase in the GILZ protein level in all groups in which GILZ mRNA was up-regulated (Figure 13C). Induction of GILZ was not related to an increase in the number of early or late apoptotic CD11c⁺ cells, as there were < 12% early apoptotic (range 3.8-11.4%) and late apoptotic (range 6.3-11.5%) CD11c⁺ cells in all groups demonstrating up-regulation of GILZ.

MoDC expressing GILZ greater than 2.5-fold above untreated MoDC were resistant to full maturation by LPS and exhibited a semi-mature, tolerogenic phenotype. LPS stimulation increased CD80 expression in MoDC up-regulating GILZ to only 50% of the levels seen after LPS stimulation in untreated MoDC (Figure 13D, range 0.48-0.57%), and increased CD86 expression to only 45% of untreated MoDC (Figure 13D, range 0.42-0.47%). Similar results were obtained for HLA-DR and CD83 (Figure 14E, range 47-65% and 23-57% of untreated MoDC after LPS respectively). In addition, MoDC up-regulating GILZ expressed 6% of the CD80 mRNA of untreated MoDC (range 4.5-7.5%), and expressed 50% of the CD86 mRNA of untreated MoDC (range 12.4-85.1%), as assessed by qRT-PCR.

### MoDC expressing GILZ display a tolerogenic cytokine profile, and knockdown of GILZ reduces the IL-10 to IL-12p70 ratio

Supernatants were harvested from co-cultures as described in Figure 13B. Dex-dendritic cells up-regulated GILZ 4.29-fold (see Figure 13B), increased production of IL-10 (Figure 14A), and decreased production of all pro-inflammatory cytokines (Figure 14B, 14C) and chemokines (Figure 14D, 14E) tested. In comparison, PUVA-dendritic cells up-regulated GILZ 2.78-fold (see Figure 13B), increased production of IL-10, and decreased production of all pro-inflammatory cytokines and chemokines tested, except TNF-α and IFN-γ. PUVA-dendritic cells or untreated MoDC, exposed to ApoL expressed GILZ at higher levels that PUVA-dendritic cells cultured alone (3.6- and 6.7-fold higher, respectively; see Figure 13B). These two groups increased production of IL-10, and decreased production of all pro-inflammatory cytokines and chemokines tested. Cytokine levels were confirmed at the RNA level, with MoDC that up-regulated GILZ also demonstrating up-regulation of IL-10 mRNA 8-fold above untreated MoDC (range 5.5-11.8, *p* < 0.01). Reductions in IL-12, TNF-α, and IL-6 were also confirmed at the RNA level (data not shown). TGF-β was up-regulated 2.5-fold in MoDC up-regulating GILZ (data not shown). TGF-β was not included in the multiplex analysis and therefore was only analyzed at the mRNA level.

The IL-10 to IL-12p70 ratio is a useful indicator of tolerogenicity, since tolerogenic dendritic cells are characterized by an increased IL-10 to IL-12p70 ratio, see Steinman et al., references). The ratio of IL-10 to IL-12p70 increased from 6.7 in untreated MoDC to 67.7 in Dex-dendritic cells. Similarly, the IL-10 to IL-12p70 ratio increased to 38.7 in PUVA-dendritic cells, and to 89.4 and 114.9 in untreated MoDC and PUVA-dendritic cells exposed to ApoL, respectively (p < 0.05).

To assess whether induction of GILZ was mediating the tolerogenic cytokine profile, MoDC were transfected with siRNA to knockdown GILZ expression. Transfection with GILZ siRNA reduced GILZ expression in Mo-dendritic cells by 68% (Figure 15A, range 59-79%). Transfection with scramble siRNA did not significantly change GILZ expression. There was also no significant difference in the number of cells recovered from any groups transfected with siRNA as compared to non-transfected groups (data not shown).

Treated MoDC up-regulating GILZ 2.5-fold higher than untreated MoDC produced higher levels of IL-10 (Figure 15B), and knockdown of GILZ reduced IL-10 production by 39% (range 34-48%, *p* < 0.05). Treated MoDC up-regulating GILZ 2.5-fold higher than untreated MoDC also produced lower amounts of IL-12p70 (Figure 15C), and knockdown of GILZ increased IL-12p70 production by 188% (range 149-214%, *p* < 0.05). Treatment with scramble siRNA had no appreciable effect on the production of IL-10 or IL-12p70. Knockdown of GILZ reduced the IL-10 to IL-12p70 ratio that had been elevated after GILZ induction. Dex-dendritic cells treated with GILZ siRNA demonstrated a reduction in the IL-10 to IL-12p70 ratio from 15.3 in non-transfected MoDC to 3.9 in transfected Dex-dendritic cells. In PUVA-dendritic cells the ratio decreased from 8.4 in non-transfected MoDC to 2.9 in PUVA-dendritic cells, and in untreated MoDC and PUVA-dendritic cells exposed to ApoL, reductions in the ratio from 18.1 to 7.8 and 28.4 to 8.3, respectively, were observed.

These results demonstrates that like other immunosuppressive mediators, PUVA induces the expression of GILZ and generates tolerogenic immuno-suppressive dendritic cells, characterized by low expression of the co-stimulatory molecules CD80 and CD86, and the maturation marker CD83. GILZ induction is necessary for the polarization towards a tolerogenic cytokine profile, characterized by increased IL-10 production, and decreased pro-inflammatory cytokine and chemokine production, including IL-12p70. These results further implicate GILZ as the molecular switch mediating the immunosuppressive effects of apoptotic cells.

### Experiment 3 - Identification of further molecular markers for immuno-stimulatory dendritic cells

### Patient samples

Leukocytes from patients undergoing ECP using the UVAR XTS Photopheresis System (Therakos) were obtained under the guidelines of the Yale Human Investigational Review Board. Informed consent was provided according to the Declaration of Helsinki. Aliquots were procured at 3 time points: before treatment (Pre ECP), immediately after 8-MOP/ultraviolet A (UVA) exposure (ECP Day 0) or after 18-hour incubation of treated blood mononuclear leukocytes (ECP Day 1) in a 1-L platelet storage bag (PL-2410; Baxter).

### Normal subjects

To determine whether ECP induces monocytes from healthy subjects to convert to DC, mononuclear leukocytes from normal subjects were examined in 2 ways. Leukapheresed leukocytes from normal subjects (N = 3) were studied pretreatment (pre-ECP), immediately after ECP (ECP Day 0), and 18 hours after ECP (ECP Day 1). A desktop apparatus, incorporating a UVA light source and a plastic exposure plate, enabled laboratory reproduction of the clinical ECP system and sample access for parallel RNA isolation, immunophenotyping, and functional studies. Alternatively, a unit of blood from normal subjects was drawn into a transfer bag and passed through the ECP treatment apparatus in an identical fashion to that of treated patients (N = 3). The cells obtained from the unit of normal blood were used for microarrays and antigen presentation assays.

### Psoralen addition

As is routinely done during ECP, the standard 8-MOP concentrated solution (Therakos) was added directly to the clinical ECP apparatus and to the laboratory model system. That mode of introduction enabled precise 100-200 ng/mL concentrations throughout the clinical procedures and experimentation.

### Overnight culture

In ECP, it is not possible to examine phenotypic and functional changes in treated monocytes, because those cells are immediately reinfused into patients. Therefore, after ECP, cells were cultured for 18 hours (RPMI 1640/15% autologous serum) to study induced monocyte gene activation, maturation and function. Prior to (pre-ECP) and immediately after ECP (ECP Day 0), patient and normal subject samples were isolated by centrifugation over a Ficoll-Hypaque gradient. The cells were resuspended in RPMI-1640 medium (Gibco), supplemented with 7.5% AB serum, 7.5% autologous serum (Gemini Bio-Products) and cultured (for patients) in 6-well polystyrene tissue-culture plates at a density of 5^{∗}10⁶ cells/mL and in Baxter platelet storage bags (for normal subjects 37°C, 5% CO2). After overnight culture (ECP Day 1), cells were harvested before undergoing monocyte enrichment. To generate DC for comparative phenotypic analysis, cells were cultured in RPMI 1640 15% serum in the presence of 1 mL of GMCSF and IL4 (25 ng/mL; R&D Systems) for 6 days.

### Magnetic bead enrichment of the monocyte population

To enable determination of whether ECP activates genes directing monocytes into the dendritic cell maturational pathway, it was necessary to develop a gentle negative monocyte enrichment method that eliminates contribution of lymphocytes to the transcriptome analysis while minimizing monocyte physical or cell membrane perturbation. Monocytes were enriched from the mononuclear cell pool by single passage through affinity columns. This negative selection method limited physical perturbation, whereas lymphocytes adherent to magnetic microbeads (Miltenyi Biotec), conjugated to relevant monoclonal antibodies (anti-CD4, CD8, CD19), were depleted. However, enrichment of ECP Day 1 monocytes beyond 60%-80% proved challenging, because diminished surface display of lymphocyte markers by ECP-damaged lymphocytes permitted a fraction of T and B cells to escape retention in the columns. Repetitive passes through the affinity column, to further enhance monocyte purity, was not an option because that approach compounds the physical perturbation of passively filtered monocytes. Fortuitously, a series of analyses revealed that ECP's preferential damage of lymphocytes precluded the necessity of full purification of monocytes for accurate assessment of level of DC gene activation. Due to their extreme sensitivity to UVA-activated 8-MOP, 99% of ECP-processed lymphocytes were apoptotic after overnight incubation (as determined by staining with APO2-PE, Trypan blue, and/or annexin-fluorescein isothiocynate FITC/ propidium idodide). Because ECP causes global lymphocyte apoptosis, 90%-95% of viable mononuclear leukocytes in the ECP day 1 fraction were monocytes. This phenomenon accounts for the observation that multiple step magnetic bead removal of apoptotic lymphocytes, performed as follows and yielding monocyte purity of greater than 95%, does not alter levels of observed gene expression in the studied cell populations. To accomplish that comparison we modified the monocyte purification procedure by adapting a negative selection protocol using magnetic beads and the EasySep magnet. Peripheral blood mononuclear cells were centrifuged at low speed (120g for 10 minutes) to remove platelets. Cells were then labeled using the Monocyte Isolation Kit II (Miltenyi Biotec) following the manufacturers procedure with the follow- ing modifications: (1) buffer consisted of ice-cold phosphate-buffered saline containing 2% autologous serum and ImM EDTA (ethylenediaminetetraacetic acid); (2) blocking time was increased to 10 minutes; (3) labeling with the Biotin-Antibody Cocktail was increased to 20 minutes; and (4) cells were washed once between labeling with the Biotin-Antibody Cocktail and the Anti-Biotin Microbeads. To avoid stimulating the monocytes by passing them over a column, the magnetically labeled cells were instead separated from the unlabeled monocytes using the EasySep magnet (StemCell Technologies). Cells, in 2 mL of buffer in a 5-mL polystyrene tube, were placed in the magnet for 10 minutes, and then the unlabeled cells were carefully poured off into a new tube. This procedure was repeated 2x, to maximally enhance monocyte purity. At this point, because the purity was still insufficient, cells were relabeled with the Monocyte Isolation Kit II reagents and placed in the EasySep magnet for an additional 10 minutes, and the unlabeled monocytes were eluted. Final purity (X=96%+4.5) was assessed by flow cytometric analysis of CD14 staining.

### Immunophenotyping

Monoclonal antibodies specific for monocytes and dendritic cells, included: CD14 (lipopolysaccharide [LPS] receptor, monocytes); CD36 (receptor for apoptotic cells, monocytes); human leukocyte antigen DR-1 (HLA-DR; class II major histocompatibility complex [MHC] molecule); CD83 (dendritic cell marker); cytoplasmic dendritic cell-lysosome-associated membrane protein (DC-LAMP; dendritic cell marker); and CD80 and CD86 (B7.1 and B7.2 costimulatory molecules). Antibodies were obtained from Beckman Coulter and used at their predetermined optimal dilutions. Background staining was established with appropriate isotype controls, and immunofluorescence was analyzed using a FC500 flow cytometer (Beckman Coulter). Combined membrane and cytoplasmic staining was performed following manufacturer's instruc- tions for cell fixation and permeabilization (Intraprep kit; Beckman Coulter).

### Antigen presentation assay

Volunteer freshly isolated, magnetic bead-enriched, antigen-experienced CD4⁺ populations (2^{∗}10⁶/mL, 50 µL/well) were added to monocytes (2^{∗}10⁶/mL, 50 µL/well) in the presence of tetanus toxoid (10 µg/mL, 100 µL/well) and RPMI medium 1640/15% autologous serum. After 5 days of culture, the cells received 1 µCi of [3H]-thymidine and were incubated overnight, harvested, and counted in a Beta liquid scintillation counter (PerkinElmer). Results are presented as the mean and standard deviation of 5 replicate cultures.

### MLR/CML assay

To assess whether ECP-processed monocytes are functionally capable of stimulating MHC class I-restricted cytotoxicity by CD8 T cells, mononuclear leukocytes from 3 normal subjects were studied. One unit of anti-coagulated blood, freshly procured from each of 3 HLA-A2-positive volunteers, served as sources of stimulator monocyte/dendritic cells, before and after being processed through the clinical ECP apparatus in a manner identical to the actual ECP procedure. Mononuclear fractions were isolated from the blood immediately prior to ECP processing (pre-ECP) and immediately after ECP (ECP D0). After gamma irradiation (3000 rad, Cesium source) to ensure unidirectional T-cell stimulation, the Pre ECP fraction was serially diluted in RPMI 1640/15% autologous serum, and 100 µL containing from 25 000 to 250 cells was plated in round-bottom microtiter plate wells, in 5 replicates. The ECP D0 fraction was incubated for 18 hours in large well plates and harvested by scraping the wells to free adherent cells. The re-suspended cells were then serially diluted and plated as above. An A-2-negative normal donor served as the source of responder CD4 and CD8 T cells, purified by positive selection on Miltenyi magnetic bead columns (average purity 98%). Responder T cells (50 000/well in 100 µL) were then added to the wells containing either Pre-ECP or ECP-D0 stimulators, and the plates were cultured for 7 days at 37°C in a CO2 incubator. For target cells, the A-2-positive T-B hybridoma lymphoblast line, 174 x CWM.T1, was labeled with ⁵¹Cr and added to the MLR cultures at 10⁴ cells/well. After 4-hour incubation, plates were centrifuged, and 100 µL of supernatant was removed from each well for counting in a gamma counter. "Percent-specific lysis" was defined as 100 times the following fraction:
Mean cpm (sample) - Mean cpm (T cell only)
Mean cpm (detergent maximum release) - mean cpm (T cells only)

### RNA isolation and microarray hybridization

Total RNA was isolated using RNeasy Mini Kit columns with on-column DNase I treatment (QIAGEN). RNA yield and purity were measured using the NanoDrop ND-1000 Spectrophotometer and the Agilent 2100 Bioana- lyzer. Fragmented cRNAs were hybridized on Affymetrix HG U133 Plus 2.0 human chips, and screening for approximately 47 400 human genes and ESTs was performed by the Yale University W. M. Keck Resource Laboratory. The microarray results are available on Gene Expression Omnibus under accession number GSE23604.

### Data analysis

Raw data without normalization generated from Affymetrix GeneChip Operating Software Version 1.2 (GCOS 1.2; Affymetrix) were analyzed using GeneSpring software 7.2 (Agilent Technologies-Silicon Genetics). Data were normalized using Robust Multi-Array. Only probe sets with a minimal fold change of >2.0 combined with an average signal intensity of 500 or higher in either apheresis such as leukaphereses or treated samples were included in the analysis. Differential gene expression was considered as a ≥2-fold change and P ≤ .05. Principal component analysis (PCA) of the induced transcriptomes was performed by standard methodology. Signal transduction pathway involvement was identified with MetaCore Software Version 1.0 (GeneGo).

### Quantitative real-time PCR

Microarray expression of selected genes was confirmed in aliquots of the same RNA samples, using quantitative real-time polymerase chain reaction (PCR). RNA was reverse transcribed to cDNA using the High Capacity cDNA Reverse Transcription Kit (Applied Biosystems). Reverse transcription was carried out in a 96-well thermocycler (MJ Research PTC-200) in the following conditions: 25°C, 10 minutes, 37°C, 120 minutes, 85°C, 5 seconds. TaqMan real-time PCR was used to detect transcripts of DC-LAMP, CCR7, CD80, CD86, and CD14. Primers and probes for each sequence were obtained as inventoried Taqman Gene Expression Assays (Applied Biosystems). HPRT1 was used as a reference gene.

### Results

### Large ECP-induced changes in individual gene expressions

The stimulation by ECP of individual gene activation in monocytes was expressed as the ratio of ECP Day 1 to pre-ECP expression for the relevant gene. To preclude inadvertent gene induction during monocyte enrichment, a negative column purification method was used, whereby lymphocytes were retained, and monocytes were passively filtered. The results revealed that the ECP-processed monocytes from both patients and normal subjects remain sufficiently viable to reproducibly express a shared transcriptome signature.

Genes were considered significantly up- or down-regulated by ECP if fold change was ≥2 and significance was P ≤ .05 compared with pre ECP. Levels of RNA transcripts from approximately 3000 genes were significantly changed in each patient group and in normal subjects (Table 2). Overall, 1129 genes were up- or down-regulated in common by ECP-processed monocytes from both CTCL and GVHD patients and from normal subjects, indicating commonality in ECP-induced gene activation.

**Table 2: Number of Monocyte Genes with Altered Expression after ECP.**

| **Monocyte Source** | **Total** | **Up-requlated** | **Down-requlated** |
|---|---|---|---|
| Normal Subjects (alone): N=6 | 3,666 | 1494 (41%) | 2172 (59%) |
| CTCL (alone): N=3 | 4,315 | 2613 (61%) | 1702 (38%) |
| GVHD (alone): N=3 | 4,350 | 2658 (61%) | 1692 (39%) |

| | | | |
|---|---|---|---|
| Number of genes significantly induced or suppressed by ECP. | | | |

Increased expression of numerous genes associated with dendritic cell differentiation, adhesion, and function (Table 3) further support ECP stimulation of entry of monocytes into that pathway.

**Table 3: : ECP-Enhanced Expression of DC Marker Genes, Ratio* of Post-ECP/Pre-ECP Levels**

| Gene | Attributes | CTCL and GVHD (N-6) Induced Expression Ratio | Normal Subjects (N-6) Induced Expression Ratio |
|---|---|---|---|
| DC-LAMP | DC Lysomal Protein | 27.6 p=1.2x10⁻⁰⁹ | 17.2 p=1.4 x10⁻⁰⁷ |
| GPNMB | Transmembrane glycoprotein | 205.7 p=9.6x10⁻¹⁵ | 123.3 p=2.8x10⁻¹⁴ |
| CD80 | Co-stimulatory molecule, B7.1 | 13.4 p=2.3x10⁻¹³ | NC |
| CD86 | Co-stimulatory molecule, B7.2⁸ | NC | 5.0 p=1.4x10⁻⁰⁵ |
| CD40 | Involved in DC survival | 2.3 p=5.7⁻⁰⁴ | NC |
| Decysin | ADAM-like, Expressed in LPS matured DC | 26.5 p=1.0x10⁻⁰⁹ | 7.1 p=5.6x10⁻⁰⁴ |
| CCR7 | Lymph node homing molecule | 2.6 p=7.0x10⁻⁰³ | NC |
| CD83 | DC maturation molecule | NC | 2.3 p=0.03 |
| OLR1 | Lox1, lectin-like receptor | 13.6 p=3.3x10⁻⁰⁵ | 100.1 p=8.3x100⁻⁰⁸ |
| CLEC5A | MDL-1 | 10.9 p=9.5x10⁻⁰⁷ | 45.5 p=1.6x10⁻⁰⁸ |
| FPRL2 | Formyl peptide receptor-like-2 | 33.9 p=2.1x10⁻⁰⁸ | 43.2 p=1.9x10⁻⁰⁸ |
| SDC2 | Syndecan, cell surface proteoglycan | 21.7 p=9.3x10⁻⁰⁸ | 98.9 p=3.3x10⁻⁰⁹ |
| THBS1 | Thrombospondin 1 | 6.2 p=7.8x10⁻⁰⁸ | 10.4 p=4.7x10⁻⁰⁹ |

| | | | |
|---|---|---|---|
| *Ratio = (Pre-ECP Gene Expression) to (Post-ECP Gene Expression), Fold increase in expression of multiple genes involved in DC maturation and function induced by ECP. Impact of treatment on gene expression is displayed as an *Induced Expression Ratio* (ratio of post-ECP to pre-ECP expression for the relevant gene). RNA was isolated from 3 CTCL patients and 3 GVHD patients and 6 normal subjects at the relevant time points. | | | |

Further genes, the expression of which was found to be increased and which can be considered to be molecular markers of immune-stimulatory dendritic cells are depicted in Table 1.

As would be expected during monocyte-to-dendritic cell maturation, CD14 (monocyte marker) expression was diminished, as assessed by measuring the mean fluorescence intensity on the monocyte populations of all patients and normal subjects, after overnight culture of ECP-processed monocytes. This result was confirmed in RT-PCR studies of the patients' post-ECP cells (results not shown). Further factors, the expression of which was reduced indicating monocyte-to-dendritic cell maturation are shown in Table 4.

**Table 4: ECP-Reduced Expression of Monocyte Marker Genes , Ratio* of Post-ECP/Pre-ECP Levels**

| Gene | Attributes | CTCL and GVHD (N=6) Induced Expression Ratio | Normal Subjects (N=6) Induced Expression Ratio |
|---|---|---|---|
| CD33 | Cell surface protein expressed on monocytes | -2.2 p=4.5x10⁻⁰⁴ | NC |
| CD36 | Receptor for apoptotic cells | -7.4 p=7.9x10⁻⁰⁵ | NC |
| FCGR1A | Receptor for IgGFc fragment 1A | -6.9 p=6.6x10⁻⁰⁵ | -4.4 p=2.1x10⁻⁰³ |

| | | | |
|---|---|---|---|
| *Ratio = (Pre-ECP Gene Expression) to (Post-ECP Gene Expression), Fold decrease in expression of genes distinctive of monocytes induced by ECP, as the monocytes differentiate into DC. Impact of treatment on gene expression is displayed as an *Induced Expression Ratio* (ratio of post-ECP to pre-ECP expression for the relevant gene). RNA was isolated from 3 CTCL patients and 3 GVHD patients and 6 normal subjects at the relevant time points. | | | |

Further factors, the expression of which was reduced and thus indicating monocyte-to-immuno suppressive dendritic cell maturation are shown in Table 5.

**Table 5: ECP-Enhanced Expression of Immunosuppression-Associated Genes, Ratio* of Post-ECP/Pre-ECP Levels.**

| Gene | Attributes Normal | CTCL and GVHD (N-6) Induced Expression Ratio | Normal Subjects (N-6) Induced Expression Ratio |
|---|---|---|---|
| IDO | Indoleamine | 27.8 p=4.0x10⁻¹⁰ | 9.4 p=1.1x10⁻⁰⁶ |
| KMO | kynurenine 3-hydroxylase | 6.0 p=2.5x10⁻⁰⁶ | NC |
| IL10 | Interleukin 10 | 6.3 p=9.2x10⁻⁰⁶ | 8.6 p=5.7x10⁻⁰⁶ |

| | | | |
|---|---|---|---|
| *Ratio = (Pre-ECP Gene Expression) to (Post-ECP Gene Expression), ECP-induced fold increase in expression of genes which contribute to DC capacity suppress T cell-mediated immunologic reactions. Impact of treatment on gene expression is displayed as an *Induced Expression Ratio* (ratio of post-ECP to pre-ECP expression for the relevant gene). RNA was isolated from 3 CTCL patients and 3 GVHD patients and 6 normal subjects at the relevant time points. | | | |

### Experiment 4 - Surface molecule markers and functional mediators of immuno-stimulatory DC.

Further analysis of the ECP-induced dendritic cells transcriptome was performed to identify a subset of surface molecule gene products as markers and functional mediators of immuno-stimulatory dendritic cells. Of 466 genes upregulated in ECP-induced dendritic cells were cross referenced to approximately 2000 known or presumed full-length human transmembrane genes to identify 87 shared surface proteins.

### Materials and Methods

### Procurement of leukocytes and platelets

All samples were acquired from young, healthy subjects not taking medications, including aspirin, known to influence platelet function. Samples were obtained under the guidelines of the Yale Human Investigational Review Board, and informed consent was provided according to the Declaration of Helsinki. Peripheral blood specimens were collected through a 19-gauge needle from the antecubital vein into syringes containing heparin, then layered on Ficoll-Hypaque (Gallard-Schlessinger, Carle Place, N.Y.). Following centrifugation at 180g, the interface containing the mononuclear leukocyte fraction was collected and washed twice in HBSS, then resuspended in RPMI-1640 medium (GIBCO) to a final concentration of 5 x 10⁶ mononuclear cells/ml. Cells were utilized within one hour of being acquired.

### Preparation of Platelet-rich-Plasma

Whole blood was centrifuged at 150 g for 15 min at room temperature. The platelet-rich-plasma (PRP) layer was collected and centrifuged at 900 g for 5 min, and the platelet pellet resuspended in RPMI 1640 to the desired concentration.

### Preparation of Plates

Plate passage was conducted using a Glycotech system (Glycotech, Rockville, MD). This system consisted of a volumetric flow path measuring 20000 x 10000 x 254 microns (length x width x height). The bottom plate in this system was composed of a 15mm petri dish (BD Biosciences, Durham, NC) separated by a gasket and vacuum-connected to an acrylic flow deck, which formed the upper plate. For precoating with platelets, prior to assembling the flow chamber, 20 drops of the desired concentration of PRP was placed in the center of the petri dish and platelets allowed to settle for 20 minutes at room temperature. The petri dish was washed twice with 2ml of RPMI, and the flow chamber then assembled.

### Overnight culture

When overnight culture was required, cells were centrifuged and resuspended in RPMI-1640 medium (GIBCO), supplemented with 15% AB serum (Gemini Bio-Products) to a final concentration of 5 x 106 cells/ml. Cells were cultured overnight for 18 hours in 12-well polystyrene tissue culture plates (2 ml per well) at 37° C in 5% CO2.

### Immunophenotyping

Monoclonal antibodies for immunophenotyping included CD14 (LPS receptor; monocytes), CD11c (integrin subunit; monocytes and DC), HLA-DR (class II MHC molecule), CD83 (DC marker), CD62p (P-selectin; activated platelets), and CD61 (integrin subunit; platelets). Antibodies were obtained from Beckman Coulter (CD14, CD11c, HLADR, CD83) or Sigma (CD62p, CD61) and used at their pre-determined optimal dilutions. Background staining was established with appropriate isotype controls, and immunofluorescence was analyzed using a FC500 flow cytometer (Beckman Coulter). Two-color membrane staining was performed by adding the pre-determined optimal concentrations of both antibodies directly conjugated to FITC or PE and incubating for 20 min at 4°C, followed by washing to remove unbound antibodies. Combined membrane and cytoplasmic staining was performed following manufacturer's instructions for cell fixation and permeabilization (Intraprep kit, Beckman Coulter).

### Results

Plate-passed and/or PBMC D1 populations showed significant upregulation of analyzed surface expression of SIRPa, ICAM1, CXCL16, LIGHT, PLAUR (CD87, plasminogen activator, urokinase receptor), MSR1, Neu1 (sialidase), CD137L, and CATB (CTSB, cathepsin B).

### Experiment 5 - Determining expression of molecular markers and FSC/SSC complexity after passing monocytes through flow chamber

### Materials and Methods

Monocytes were passed through a device depicted in Fig. 19. In brief, a blood sample was spun at low speed through a Ficoll gradient to obtain e.g. 8 ml of sample with a concentration of peripheral blood mononuclear cells (PBMC) of e.g. 10¹⁰ cells/ml. The chamber was pre-coated with platelets. The sample was passed through the chamber at about 0.028 Pa. The chamber and then washed with about 3 ml RPMI at 0.028 Pa. A second wash with 30-55 ml RPMI was performed at about 1.2 Pa. The collected activated monocytes were combined, incubated for a day and used for further analysis (PP D1 PBMC). As a control PBMCs were not passed through the device and incubated for a day (D1 PBMC). As another control immature fast DC were obtained by directly cultivating PBMC in the presence of GM-CSF and IL-4 (immature Fast DC). Further, PBMC were analyzed directly after harvest through a Ficoll gradient (Fresh (Ficoll) PBMC).

The cells and controls were then analyzed for expression of HLA-DR, CD86, ICAM-1, and PLAUR. They were further analyzed for FSC/SSC complexity. The results are depicted for HLA-DR in Figure 20 and for FSC/SSC complexity in Figure 21 and 22. A summary is shown in Figure 23.

### Results

The results show that cells subjected to centrifugation through a Ficoll gradient alreads seem to experience enough physical forces to start differentiating as becomes apparent from incubating these cells for one day (D1 PBMC). However, activation and differentiation is more pronounced upon plate passage through the device (PP D1 PDMC). The dendritic cells obtained by methods in accordance with the invention in the absence of e.g. 8-MOP and UV-A moreover have a more complex and distinct pattern than immature Fast DC obtained with cytokine cocktails.

### Experiment 6 - Determining inlfuence of 8-MOP and UVA on activated monocytes

### Materials and Methods

Monocytes were passed through a device derived from the Therakos system. The dimensions of the flow chamber were 30 mm width, 1600 mm length, and 1 mm height. In brief, a blood sample was spun at low speed through a Ficoll gradient to obtain e.g. 80 ml of sample with a concentration of peripheral blood mononuclear cells (PBMC) of e.g. 1.5^{∗}10⁶ cells/ml. The chamber was pre-coated with platelets. The sample was passed through the chamber for 60 min at a flow rate of about 24 ml/min corresponding to a shear stress of about 0.13 Pa. The collected activated monocytes were spun at low speed and resuspended in a small volume of medium comprising 8-MOP and irradiated with combined, incubated for a day and used for further analysis (Day 1PBMC + PP). As a control PBMCs were not passed through the device and incubated for a day (Day 1 PBMC). Further, PBMC were analyzed directly after harvest through a Ficoll gradient (Day0 PBMC). Day 1 PBMC + PP were then further treated with 8-MOP and UVA (PUVA).

The cells and controls were then analyzed for expression of GILZ. The results are depicted for GILZ in Figure 24.

### Results

The results show that cells that activated monocytes that would differentiate into immuno-stimulatory dendritic cells can be channeled into immuno-suppressive dendritic cells by application of 8-MOP and UVA.

### References:

1. Berger C, Hoffmann K, Vasquez JG, Mane S, Lewis J, Filler R et al. Rapid generation of maturationally synchronized human dendritic cells: contribution to the clinical efficacy of extracorporeal photochemotherapy. Blood 2010; 116(23): 4838-4847.
2. Cella M, Scheidegger D, PalmerLehmann K, Lane P, Lanzavecchia A, Alber G. Ligation of CD40 on dendritic cells triggers production of high levels of interleukin-12 and enhances T cell stimulatory capacity: T-T help via APC activation. Journal of Experimental Medicine 1996; 184(2): 747-752.
3. de Saint-Vis B, Vincent J, Vandenabeele S, Vanbervliet B, Pin JJ, Ait-Yahia S et al. A novel lysosome-associated membrane glycoprotein, DC-LAMP, induced upon DC maturation, is transiently expressed in MHC class II compartment. Immunity 1998; 9(3): 325-336.
4. Slavik JM, Hutchcroft JE, Bierer BE. CD80 and CD86 are not equivalent in their ability to induce the tyrosine phosphorylation of CD28. Journal of Biological Chemistry 1999; 274(5): 3116-3124.
5. Kang HK, Lee HY, Kim MK, Park KS, Park YM, Kwak JY et al. The synthetic peptide Trp- Lys-Tyr-Met-Val-D-Met inhibits human monocyte-derived dendritic cell maturation via formyl peptide receptor and formyl peptide receptor-like 2. Journal of Immunology 2005; 175(2): 685-692.
6. Ripoll VM, Irvine KM, Ravasi T, Sweet MJ, Hume DA. Gpnmb is induced in macrophages by IFN-gamma and lipopolysaccharide and acts as a feedback regulator of proinflammatory responses. Journal of Immunology 2007; 178(10): 6557-6566.
7. Chen SQ, Springer TA. Selectin receptor-ligand bonds: Formation limited by shear rate and dissociation governed by the Bell model. Proceedings of the National Academy of Sciences of the United States of America 2001; 98(3): 950-955.
8. Thomas WE. Understanding the counterintuitive phenomenon of catch bonds. Current Nanoscience 2007; 3: 63-83.
9. Xiong JP, Stehle T, Zhang RG, Joachimiak A, Frech M, Goodman SL et al. Crystal structure of the extracellular segment of integrin alpha V beta 3 in complex with an Arg-Gly-Asp ligand. Science 2002; 296(5565): 151-155.
10. Weisel JW, Nagaswami C, Vilaire G, Bennett JS. Examination of the Platelet Membrane Glycoprotein-IIB-IIIA Complex and its Interaction with Fibrinogen and Other Ligands by Electron-Microscopy. Journal of Biological Chemistry 1992; 267(23): 16637-16643.
11. Kaplan KL, Broekman MJ, Chernoff A, Lesznik GR, Drillings M. Platelet alpha-granule proteins - studies on release and subcellular-localization. Blood 1979; 53(4): 604-618.
12. Ruoslahti E. RGD and other recognition sequences for integrins. Annual Review of Cell and Developmental Biology 1996; 12: 697-715.
13. Zhou LJ, Tedder TF. CD14+ blood monocytes can differentiate into functionally mature CD83+ dendritic cells. Proc. Natl. Acad. Sci. U.S.A. 1996;93(6):2588-2592.
14. Klein E. CD83 localization in a recycling compartment of immature human monocyte-derived dendritic cells. International Immunology. 2005;17(4):477-487.
15. Renzo MD, Rubegni P, Pasqui AL, et al. Extracorporeal photopheresis affects interleukin (IL)-10 and IL-12 production by monocytes in patients with chronic graft-versus-host disease. Br J Dermatol. 2005;153(1):59-65.
16. Gasparro FP, Bevilacqua PM, Goldminz D, et al. Repair of 8-MOP photoadducts in human lymphocyte. In DNA Damage and Repair in Human Tissues (edited by B. M. Sutherland and A.D. Woodhead). Plenum Press, New York.
17. Steinman RM, Hawiger D, Nussenzweig MC. Tolerogenic dendritic cells. Annu. Rev. Immunol. 2003;21:685-711.

## Claims

1. A method for inducing differentiation of monocytes contained in an extracorporeal quantity of a mammalian subject's blood sample into immuno-suppressive dendritic cells, said method comprising at least the steps of:
a) subjecting said extracorporeal quantity of said mammalian subject's blood sample to a physical force such that said monocytes are activated and induced to differentiate into immuno-suppressive dendritic cells, which are identified by at least one molecular marker, wherein said at least one molecular marker is indicative of immuno-suppressive dendritic cells;
b) determining expression of GILZ to identify said immuno-suppressive dendritic cells;
; and wherein said monocytes are exposed to UV light in the presence of a DNA-cross linking agent, preferably 8-MOP, to effect increased expression of GILZ, wherein said extracorporeal quantity of said mammalian subject's blood sample is subjected to a physical force by passing said extracorporeal quantity of said mammalian subject's blood sample through a flow chamber of a device, which allows for fixed or tunable adjustment of the flow rate of said extracorporeal quantity of said mammalian subject's blood sample through said flow chamber of said device such that a shear force is applied to said monocytes contained within said mammalian subject's blood sample and wherein said immuno-suppressive dendritic cells are resistant to full maturation by LPS stimulation.

2. Method according to claim 1,
wherein said immuno-suppressive dendritic cells are identifiable
i) by a GILZ induced increased IL-10 to IL-12p70 ratio; and/or
ii) by determining expression of at least 1, 2, 3, 4, 5, 6, 7, 8, 9, 10, 15, 20 , 25, 30, 35, 40, 45, 50, 55, or 60 molecular markers, which are indicative of immuno-stimulatory dendritic cells, wherein preferably said at least 1, 2, 3, 4, 5, 6, 7, 8, 9, 10, 15, 20, 25, 30, 35, 40, 45, 50, 55, or 60 molecular markers are selectable from table 1 and do not show an increased expression; wherein more preferably said at least 1, 2, 3, 4, 5, 6, 7, 8, 9, 10, 15, 20 , 25, 30, 35, 40, 45, 50, 55, or 60 molecular markers include PLAUR, NEU1, CTSB, CXCL16, ICAM1, MSR1, OLR1, SIRPA, TNFRSF1A, TNFSF14, TNFSF9, PMB22, CD40, LAMP3, CD80, CCR7, LOX1, CD83, ADAM Decysin, FPRL2, GPNMB and/or CD86.

3. Method according to any of claims 1 or 2,
wherein
i) said extracorporeal quantity of said mammalian subject's blood sample is subjected to a physical force by passing said extracorporeal quantity of said mammalian subject's blood sample through a flow chamber of a device, which allows adjustment of the flow rate of said extracorporeal quantity of said mammalian subject's blood sample through said flow chamber of said device such that a shear force is applied to said monocytes contained within said mammalian subject's blood sample, and wherein said device additionally allows for adjustment of at least one parameter selected from the group comprising temperature, and light exposure;
ii) wherein said monocytes are activated and induced to differentiate into immuno-suppressive dendritic cells through interaction with activated platelets and/or plasma components; and/or
iii) wherein activation of said monocytes and differentiation into immuno-suppressive dendritic cells can be influenced by the design and dimensions of the flow chamber, the flow rate at which the monocytes are passed through the flow chamber, the light to which the monocytes are exposed to in the presence or absence of DNA cross-linking agents such as 8-MOP, the temperature at which the monocytes, platelets, platelets-derived factors and/or plasma components are passed through the flow chamber, the order by which the monocytes, platelets, platelets-derived factors and/or plasma components are passed through the flow chamber, the density by which plasma components are coated to the surfaces of the flow chamber, the density by which platelets and/or platelets derived factors adhere to the surfaces and or to the plasma components of the flow chamber, and/or the density by which monocytes adhere to the platelets and/or platelets derived factors and or plasma components adhered to the surfaces of the flow chamber.

4. Method according to any of claims 1 to 3,
i) wherein said method comprises at least the steps of:
a) applying said extracorporeal quantity of said mammalian subject's blood sample comprising at least monocytes to a device, which is configured to provide for a flow chamber through which said extracorporeal quantity of said mammalian subject's blood sample can be passed,
b) activating platelets, which may be comprised within said extracorporeal quantity of said mammalian subject's blood or which may be provided separate from said mammalian subject's blood sample comprising at least monocytes,
c) treating said extracorporeal quantity of said mammalian subject's blood sample comprising at least monocytes in said device by applying a physical force to the monocytes contained within said extracorporeal quantity of said mammalian subject's blood sample such that said monocytes are activated and induced to differentiate into immuno-suppressive dendritic cells by binding to said activated platelets obtained in step b); or
ii) wherein said method comprises at least the steps of:
a) applying said extracorporeal quantity of said mammalian subject's blood sample comprising at least monocytes to a device, which is configured to provide for a flow chamber through which said extracorporeal quantity of said mammalian subject's blood sample can be passed,
b) passing plasma components, which may be comprised within said extracorporeal quantity of said mammalian subject's blood sample or which may be provided separate from said mammalian subject's blood sample,
c) treating said extracorporeal quantity of said mammalian subject's blood sample comprising at least monocytes in said device by applying a physical force to the monocytes contained within said extracorporeal quantity of said mammalian subject's blood sample such that said monocytes are activated and induced to differentiate into immuno-suppressive dendritic cells by binding to said plasma components obtained in step b); or
iii) wherein said method comprises at least the steps of:
a) applying said extracorporeal quantity of said mammalian subject's blood sample comprising at least monocytes to a device, which is configured to provide for a flow chamber through which said extracorporeal quantity of said mammalian subject's blood sample can be passed,
b) passing plasma components, which may be comprised within said extracorporeal quantity of said mammalian subject's blood or which may be provided separate from said mammalian subject's blood sample,
c) activating platelets, which may be comprised within said extracorporeal quantity of said mammalian subject's blood sample or which may be provided separate from said mammalian subject's blood sample comprising at least monocytes,
d) treating said extracorporeal quantity of said mammalian subject's blood comprising at least monocytes in said device by applying a physical force to the monocytes contained within said extracorporeal quantity of said mammalian subject's blood sample such that said monocytes are activated and induced to differentiate into immuno-suppressive dendritic cells by binding to said activated platelets and/or plasma components obtained in steps b) and c).

5. Method of claim 4, wherein said extracorporeal quantity of said mammalian subject's blood sample has not been obtained by apheresis; wherein
i) said extracorporeal quantity of said mammalian subject's blood sample is between about 10 ml to about 500 ml of extracorporeal whole blood of said mammalian subject;
ii) wherein said extracorporeal quantity of said mammalian subject's blood sample is obtained by isolating leukocytes from about 10 ml to about 500 ml of extracorporeal whole blood of said mammalian subject; and/or
iii) wherein said extracorporeal quantity of said mammalian subject's blood sample is obtained by isolating buffy coats from about 10 ml to about 500 ml of extracorporeal whole blood of said mammalian subject.

6. Method of claim 5, wherein
i) said extracorporeal quantity of said mammalian subject's blood sample does not comprise plasma components; and/or
ii) wherein said extracorporeal quantity of said mammalian subject's blood sample does not comprise platelets, wherein preferably said platelets have been separated from said extracorporeal quantity of said mammalian subject's blood before said extracorporeal quantity of said mammalian subject's blood said is applied to said device.

7. Method of claim 4, wherein said extracorporeal quantity of said mammalian subject's blood has been obtained by apheresis; and
i) wherein preferably i) said extracorporeal quantity of said mammalian subject's blood is obtained by isolating leukocytes by leukapheresis; wherein preferably said extracorporeal quantity of said mammalian subject's blood is obtained by isolating buffy coats by leukapheresis; and/or
ii) said extracorporeal quantity of said mammalian subject's blood does not comprise plasma components; and/or
iii) said extracorporeal quantity of said mammalian subject's blood does not comprise platelets, wherein preferably said platelets have been separated from said extracorporeal quantity of said mammalian subject's blood before said extracorporeal quantity of said mammalian subject's blood said is applied to said device.

8. Method of any of claims 4 to 7, wherein
i) said flow chamber has dimensions of about 1 µm to up to about 400 µm of height and of about 1 µm to up to about 400 µm of width, wherein preferably said flow chamber has dimensions of about 5 µm to up to and including about 300 µm of height and of about 5 µm to up to and including about 300 µm of width, wherein more preferably said flow chamber has dimensions of about 10 µm to up to and including about 250 µm of height and of about 10 µm to up to and including about 250 µm of width, and wherein even more preferably said flow chamber has dimensions of about 50 µm to up to and including about 200 µm of height and of about 50 µm to up to and including about 200 µm of width; and wherein even more preferably said flow chamber has dimensions of about 50 µm to up to and including about 100 µm of height and of about 50 µm to up to and including about 100 µm of width; and/or
ii) said flow chamber is configured to take up a volume of between about 1 ml to about 50 ml of said extracorporeal amount of said mammalian subject's blood sample.

9. Method of any of claims 3 to 8, wherein
i) the material of said flow chamber is not plastic, and wherein preferably said non-plastic material is selected from the group consisting of glass; or
ii) the material of said flow chamber is plastic,wherein preferably said plastic material is selected from the group consisting of acrylics, polycarbonate, polyetherimide, polysulfone, polyphenylsulfone, styrenes, polyurethane, polyethylene, teflon or any other appropriate medical grade plastic; and/or
iii) said flow chamber is configured to allow for transmittance of light.

10. Method of any of claims 3 to 9, wherein
i) activation of said platelets is achieved by disposing plasma components, which are comprised within said extracorporeal quantity of said mammalian subject's blood sample, on the surface of said flow chamber such that at least some of said platelets can interact with said plasma components and are immobilized on the surface of said flow chamber;
ii) activation of said platelets is achieved by disposing proteins selected from the group comprising fibrinogen, fibronectin, and the gamma component of fibrinogen on the surface of said flow chamber such that at least some of said platelets can interact with said proteins and are immobilized on the surface of said flow chamber, wherein preferably activation of said platelets is achieved by disposing fibronectin on the surface of said flow chamber such that at least some of said platelets can interact with said fibronectin and are immobilized on the surface of said flow chamber; and/or
iii) activation of platelets can be monitored by expression of P-selectin and/or αII-β3 integrin.

11. Method of any of claims 3 to 10, wherein
i) said platelets are passed through said flow chamber under a shear force of about 0.1 to about 10.0 dynes/cm², preferably in a range of about 0.1 to about 2.0 dynes/cm², and/or
ii) said monocytes are passed through said flow chamber with a flow rate of about 10 ml/minute to about 200 ml/minute to produce a shear force of about 0.1 to about 20.0 dynes/cm².

12. Method of any of claims 3 to 11,
i) wherein said monocytes are activated and induced to differentiate into immuno-suppressive dendritic cells by passing said monocytes through said flow chamber under a shear force of about 0.1 to about 10.0 dynes/cm², preferably force of about 0.1 to about 1.0 dynes/cm² such that said monocytes can bind to said activated platelets; or
ii) the method further comprising the step of incubating the activated monocytes to allow the formation of immuno-suppressive dendritic cells.

13. Method of any of claims 1 to 12 for obtaining
i) individual-specific functionally and maturationally synchronized immuno-suppressive dendritic cells; or
ii) immuno-suppressive autologous dendritic cells; or
iii) immuno-suppressive allogenic dendritic cells.

## Patentansprüche

1. Verfahren zum Induzieren der Differenzierung von Monozyten, die in einer extrakorporalen Menge der Blutprobe eines Säugers enthalten sind, in immunsuppressive dendritische Zellen, wobei das Verfahren mindestens die folgenden Schritte umfasst:
a) Unterwerfen der extrakorporalen Menge der Blutprobe des Säugers einer physikalischen Kraft, so dass die Monozyten aktiviert und zur Differenzierung in immunsuppressive dendritische Zellen induziert werden, die durch mindestens einen molekularen Marker identifiziert werden, wobei der mindestens eine molekulare Marker für immunsuppressive dendritische Zellen indikativ ist;
b) Bestimmen der Expression von GILZ, um die immunsuppressiven dendritischen Zellen zu identifizieren;
und wobei die Monozyten in Gegenwart eines DNA*-cross-linking*-Mittels, vorzugsweise 8-MOP, UV-Licht ausgesetzt werden, um eine erhöhte Expression von GILZ zu bewirken, wobei die extrakorporale Menge der Blutprobe des Säugers einer physikalischen Kraft ausgesetzt wird, indem die extrakorporale Menge der Blutprobe des Säugers durch eine Flusskammer einer Vorrichtung geleitet wird, die eine feste oder einstellbare Einstellung der Flussrate der extrakorporalen Menge der Blutprobe des Säugers durch die Flusskammer der Vorrichtung ermöglicht, so dass eine Scherkraft auf die Monozyten ausgeübt wird, die in der Blutprobe des Säugers enthalten sind, und wobei die immunsuppressiven dendritischen Zellen resistent gegen vollständige Reifung durch LPS-Stimulation sind.

2. Verfahren nach Anspruch 1,
wobei die immunsuppressiven dendritischen Zellen identifizierbar sind.
i) durch ein GILZ-induziertes erhöhtes IL-10 zu IL-12p70-Verhältnis; und/oder
ii) durch Bestimmen der Expression von mindestens 1, 2, 3, 4, 5, 6, 7, 8, 9, 10, 15, 20, 25, 30, 35, 40, 45, 50, 55 oder 60 molekularen Markern, die indikativ für immunstimulierende dendritische Zellen sind, wobei vorzugsweise die mindestens 1, 2, 3, 4, 5, 6, 7, 8, 9, 10, 15, 20, 25, 30, 35, 40, 45, 50, 55 oder 60 molekularen Marker aus Tabelle 1 wählbar sind und keine erhöhte Expression aufweisen; wobei die mindestens 1, 2, 3, 4, 5, 6, 7, 8, 9, 10, 15, 20, 25, 30, 35, 40, 45, 50, 55 oder 60 molekularen Marker PLAUR, NEU1, CTSB, CXCL16, ICAM1, MSR1, OLR1, SIRPA, TNFRSF1A, TNFSF14, TNFSF9, PMB22, CD40, LAMP3, CD80, CCR7, LOX1, CD83, ADAM Decysin, FPRL2, GPNMB und/oder CD86 beinhalten.

3. Verfahren nach einem der Ansprüche 1 oder 2,
wobei
i) die extrakorporale Menge der Blutprobe des Säugers einer physikalischen Kraft ausgesetzt wird, indem die extrakorporale Menge der Blutprobe Säugers durch eine Flusskammer einer Vorrichtung geleitet wird, die es ermöglicht, die Flussrate der extrakorporalen Menge der Blutprobe des Säugers durch die Flusskammer der Vorrichtung so einzustellen, dass eine Scherkraft auf die Monozyten ausgeübt wird, die in der Blutprobe des Säugers enthalten sind, und wobei die Vorrichtung zusätzlich die Einstellung mindestens eines Parameters ermöglicht, der aus der Gruppe ausgewählt ist, die Temperatur und Lichteinwirkung umfasst;
ii) wobei die Monozyten aktiviert und induziert werden, um sich durch Interaktion mit aktivierten Blutplättchen und/oder Plasmakomponenten zu immunsuppressiven dendritischen Zellen zu differenzieren; und/oder
iii) wobei die Aktivierung der Monozyten und die Differenzierung zu immunsuppressiven dendritischen Zellen durch das Design und die Abmessungen der Flusskammer, die Strömungsgeschwindigkeit, mit der die Monozyten durch die Flusskammer geleitet werden, das Licht, dem die Monozyten in Gegenwart oder Abwesenheit von DNA-*cross-linking-*Mitteln wie 8-MOP ausgesetzt sind, die Temperatur, bei der die Monozyten, Blutplättchen, von Blutplättchen abgeleitete Faktoren und/oder Plasmakomponenten durch die Flusskammer geleitet werden, beeinflusst werden kann, die Reihenfolge, in der die Monozyten, Blutplättchen, von Blutplättchen abgeleitete Faktoren und/oder Plasmakomponenten durch die Flusskammer geleitet werden, die Dichte, mit der Plasmakomponenten auf die Oberflächen der Flusskammer aufgebracht werden, die Dichte, mit der Blutplättchen und/oder von Blutplättchen abgeleitete Faktoren auf den Oberflächen und/oder auf den Plasmakomponenten der Flusskammer haften, und/oder die Dichte, mit der Monozyten auf die von Blutplättchen und/oder Blutplättchen abgeleiteten Faktoren und/oder oder Plasmakomponenten auf die Oberflächen der Flusskammer aufgebracht werden.

4. Verfahren nach einem der Ansprüche 1 bis 3,
i) wobei das Verfahren mindestens die Schritte umfasst von:
a) Aufbringen der extrakorporalen Menge der Blutprobe des Säugers, die mindestens Monozyten umfasst, auf eine Vorrichtung, die konfiguriert ist, um eine Flusskammer bereitzustellen, durch die die extrakorporale Menge der Blutprobe des Säugers geleitet werden kann,
b) Aktivieren von Blutplättchen, die innerhalb der extrakorporalen Menge des Blutes des Säugers enthalten sein können oder die getrennt von der Blutprobe des Säugers, die mindestens Monozyten umfasst, bereitgestellt werden können,
c) Behandeln der extrakorporalen Menge der Blutprobe des Säugers, die mindestens Monozyten umfasst, in der Vorrichtung durch Aufbringen einer physikalischen Kraft auf die Monozyten, die in der extrakorporalen Menge der Blutprobe des Säugers enthalten sind, so dass die Monozyten aktiviert und induziert werden, sich zu immunsuppressiven dendritischen Zellen zu differenzieren, indem sie an die in Schritt b) erhaltenen aktivierten Blutplättchen gebunden werden; oder
ii) wobei das Verfahren mindestens die Schritte umfasst von:
a) Aufbringen der extrakorporalen Menge der Blutprobe des Säugers, die mindestens Monozyten umfasst, auf eine Vorrichtung, die konfiguriert ist, um eine Flusskammer bereitzustellen, durch die die extrakorporale Menge der Blutprobe des Säugers geleitet werden kann,
b) Durchlaufen von Plasmakomponenten, die innerhalb der extrakorporalen Menge der Blutprobe des Säugers enthalten sein können oder die getrennt von der Blutprobe des Säugers bereitgestellt werden können,
c) Behandeln der extrakorporalen Menge der Blutprobe des Säugers, die mindestens Monozyten umfasst, in der Vorrichtung durch Aufbringen einer physikalischen Kraft auf die Monozyten, die in der extrakorporalen Menge der Blutprobe des Säugers enthalten sind, so dass die Monozyten aktiviert und induziert werden, sich zu immunsuppressiven dendritischen Zellen zu differenzieren, indem sie an die in Schritt b) erhaltenen Plasmakomponenten gebunden werden; oder
iii) wobei das Verfahren mindestens die Schritte umfasst von:
a) Aufbringen der extrakorporalen Menge der Blutprobe des Säugers, die mindestens Monozyten umfasst, auf eine Vorrichtung, die konfiguriert ist, um eine Flusskammer bereitzustellen, durch die die extrakorporale Menge der Blutprobe des Säugers geleitet werden kann,
b) Durchlaufen von Plasmakomponenten, die innerhalb der extrakorporalen Menge des Blutes des Säugers enthalten sein können oder die getrennt von der Blutprobe des Säugers bereitgestellt werden können,
c) Aktivieren von Blutplättchen, die innerhalb der extrakorporalen Menge der Blutprobe des Säugers enthalten sein können oder die getrennt von der Blutprobe des Säugers, die mindestens Monozyten umfasst, bereitgestellt werden können,
d) Behandeln der extrakorporalen Menge des Blutes des Säugers, das mindestens Monozyten umfasst, in der Vorrichtung durch Aufbringen einer physikalischen Kraft auf die Monozyten, die in der extrakorporalen Menge der Blutprobe des Säugers enthalten sind, so dass die Monozyten aktiviert und induziert werden, sich zu immunsuppressiven dendritischen Zellen zu differenzieren, indem sie an die aktivierten Blutplättchen und/oder Plasmakomponenten gebunden werden, die in den Schritten b) und c) erhalten wurden.

5. Verfahren nach Anspruch 4, wobei die extrakorporale Menge der Blutprobe des Säugers nicht durch Apherese gewonnen wurde; wobei
i) die extrakorporale Menge der Blutprobe des Säugers zwischen etwa 10 ml und etwa 500 ml extrakorporales Vollblut des Säugers liegt;
ii) wobei die extrakorporale Menge der Blutprobe des Säugers durch Isolieren von Leukozyten von etwa 10 ml bis etwa 500 ml extrakorporalem Vollblut des Säugers erhalten wird; und/oder
iii) wobei die extrakorporale Menge der Blutprobe des Säugers durch Isolieren von *Buffy Coats* von etwa 10 ml bis etwa 500 ml extrakorporalem Vollblut des Säugers erhalten wird.

6. Verfahren nach Anspruch 5, wobei
i) die extrakorporale Menge der Blutprobe des Säugers keine Plasmakomponenten umfasst; und/oder
ii) wobei die extrakorporale Menge der Blutprobe des Säugers keine Blutplättchen umfasst, wobei vorzugsweise die Blutplättchen von der extrakorporalen Menge des Blutes des Säugers getrennt wurden, bevor die extrakorporale Menge des Blutes des Säugers auf die Vorrichtung aufgebracht wird.

7. Verfahren nach Anspruch 4, wobei die extrakorporale Menge des Blutes des Säugers durch Apherese gewonnen wurde; und
i) wobei vorzugsweise i) die extrakorporale Menge des Blutes des Säugers durch Isolieren von Leukozyten durch Leukapherese erhalten wird; wobei vorzugsweise die extrakorporale Menge des Blutes des Säugers durch Isolieren von *Buffy Coats* durch Leukapherese erhalten wird; und/oder
ii) die extrakorporale Menge des Blutes des Säugers keine Plasmakomponenten umfasst; und/oder
iii) die extrakorporale Menge des Blutes des Säugers keine Blutplättchen umfasst, wobei vorzugsweise die Blutplättchen von der extrakorporalen Menge des Blutes des Säugers getrennt wurden, bevor die extrakorporale Menge des Blutes des Säugers auf die Vorrichtung aufgebracht wird.

8. Verfahren nach einem der Ansprüche 4 bis 7, wobei
i) die Flusskammer Abmessungen von etwa 1 µm bis zu etwa 400 µm Höhe und von etwa 1 µm bis zu etwa 400 µm Breite aufweist, wobei vorzugsweise die Flusskammer Abmessungen von etwa 5 µm bis zu und einschließlich etwa 300 µm Höhe und von etwa 5 µm bis zu und einschließlich etwa 300 µm Breite aufweist, wobei die Flusskammer Abmessungen von etwa 10 µm bis zu und einschließlich etwa 250 µm Höhe und von etwa 10 µm bis zu und einschließlich etwa 250 µm Breite aufweist, und wobei die Flusskammer noch bevorzugter Abmessungen von etwa 50 µm bis zu und einschließlich etwa 200 µm Höhe und von etwa 50 µm bis zu und einschließlich etwa 200 µm Breite aufweist; und wobei die Flusskammer noch bevorzugter Abmessungen von etwa 50 µm bis zu und einschließlich etwa 100 µm Höhe und von etwa 50 µm bis zu und einschließlich etwa 100 µm Breite aufweist; und/oder
ii) die Flusskammer konfiguriert ist, um ein Volumen von zwischen etwa 1 ml und etwa 50 ml der extrakorporalen Menge der Blutprobe des Säugers aufzunehmen.

9. Verfahren nach einem der Ansprüche 3 bis 8, wobei
i) das Material der Flusskammer nicht Plastik ist, und wobei vorzugsweise das nicht-Plastik Material ausgewählt ist aus der Gruppe bestehend aus Glas; oder
ii) das Material der Flusskammer Kunststoff ist,wobei vorzugsweise das Kunststoffmaterial ausgewählt ist aus der Gruppe bestehend aus Acrylaten, Polycarbonat, Polyetherimid, Polysulfon, Polyphenylsulfon, Styrol, Polyurethan, Polyethylen, Teflon oder einem anderen geeigneten medizinischen Kunststoff; und/oder
iii) die Flusskammer konfiguriert ist, um Lichtdurchlässigkeit zu ermöglichen.

10. Verfahren nach einem der Ansprüche 3 bis 9, wobei
i) Aktivierung der Blutplättchen erreicht wird, indem Plasmakomponenten, die innerhalb der extrakorporalen Menge der Blutprobe des Säugers enthalten sind, auf der Oberfläche der Flusskammer so angeordnet werden, dass mindestens einige der Blutplättchen mit den Plasmakomponenten interagieren können und auf der Oberfläche der Flusskammer immobilisiert werden;
ii) die Aktivierung der Blutplättchen durch Disponieren von Proteinen, ausgewählt aus der Gruppe umfassend Fibrinogen, Fibronektin und der Gamma-Komponente von Fibrinogen, auf der Oberfläche der Flusskammer erreicht wird, so dass mindestens einige der Blutplättchen mit den Proteinen interagieren können und auf der Oberfläche der Flusskammer immobilisiert werden, wobei vorzugsweise die Aktivierung der Blutplättchen durch Disponieren von Fibronektin auf der Oberfläche der Flusskammer erreicht wird, so dass mindestens einige der Blutplättchen mit dem Fibronektin interagieren können und auf der Oberfläche der Flusskammer immobilisiert werden; und/oder
iii) Aktivierung von Blutplättchen kann durch Expression von P-Selektin und/oder αIIb-β3 Integrin überwacht werden.

11. Verfahren nach einem der Ansprüche 3 bis 10, wobei
i) die Blutplättchen unter einer Scherkraft von etwa 0,1 bis etwa 10,0 dyn/cm2, vorzugsweise in einem Bereich von etwa 0,1 bis etwa 2,0 dyn/cm2, durch die Flusskammer geleitet werden, und/oder
ii) die Monozyten durch die Flusskammer mit einer Strömungsrate von etwa 10 ml/Minute bis etwa 200 ml/Minute geleitet werden, um eine Scherkraft von etwa 0,1 bis etwa 20,0 Dyn/cm2 zu erzeugen.

12. Verfahren nach einem der Ansprüche 3 bis 11,
i) wobei die Monozyten aktiviert und induziert werden, um sich zu immunsuppressiven dendritischen Zellen zu differenzieren, indem sie die Monozyten durch die Flusskammer unter einer Scherkraft von etwa 0,1 bis etwa 10,0 dyn/cm2, vorzugsweise einer Kraft von etwa 0,1 bis etwa 1,0 dyn/cm2, passieren, so dass die Monozyten an die aktivierten Blutplättchen binden können; oder
ii) wobei das Verfahren ferner den Schritt des Inkubierens der aktivierten Monozyten umfasst, um die Bildung von immunsuppressiven dendritischen Zellen zu ermöglichen.

13. Verfahren nach einem der Ansprüche 1 bis 12 zum Erhalten von
i) Individuum-pezifischen funktionell und reiflich synchronisierten immunsuppressiven dendritischen Zellen; oder
ii) immunsuppressiven autologen dendritischen Zellen; oder
iii) immunsuppressiven allogenen dendritischen Zellen.

## Revendications

1. Procédé d'induction de la différenciation de monocytes contenus dans une quantité extracorporelle d'un échantillon de sang d'un sujet mammifère en des cellules dendritiques immuno-suppressives, ledit procédé comprenant au moins les étapes consistant à :
a) soumettre ladite quantité extracorporelle dudit échantillon de sang de sujet mammifère à une force physique de telle manière que lesdits monocytes sont activés et induits pour se différencier en cellules dendritiques immuno-suppressives, qui sont identifiées par au moins un marqueur moléculaire, dans lequel ledit au moins un marqueur moléculaire est indicateur de cellules dendritiques immuno-suppressives ;
b) déterminer l'expression de GILZ pour identifier lesdites cellules dendritiques immuno-suppressives ;
et dans lequel lesdits monocytes sont exposés à de la lumière UV en présence d'un agent de réticulation de l'ADN, préférentiellement le 8-MOP, pour effectuer une expression accrue de GILZ, dans lequel ladite quantité extracorporelle dudit échantillon sanguin de sujet mammifère est soumise à une force physique en passant ladite quantité extracorporelle dudit échantillon de sang de sujet mammifère à travers une chambre d'écoulement d'un dispositif, qui permet un ajustement fixe ou accordable du débit de ladite quantité extracorporelle dudit échantillon de sang de sujet mammifère à travers ladite chambre d'écoulement dudit dispositif de telle manière qu'une force de cisaillement est appliquée auxdits monocytes contenus dans ledit échantillon de sang de sujet mammifère et dans lequel lesdites cellules dendritiques immuno-suppressives sont résistantes à une maturation complète par stimulation de LPS.

2. Procédé selon la revendication 1,
dans lequel lesdites cellules dendritiques immuno-suppressives sont identifiables
i) par un rapport IL-10 à IL-12p70 accru induit de GILZ ; et/ou
ii) en déterminant l'expression d'au moins 1, 2, 3, 4, 5, 6, 7, 8, 9, 10, 15, 20, 25, 30, 35, 40, 45, 50, 55 ou 60 marqueurs moléculaires, qui sont indicateurs des cellules dendritiques immuno-stimulatrices, dans lequel préférentiellement au moins 1, 2, 3, 4, 5, 6, 7, 8, 9, 10, 15, 20, 25, 30, 35, 40, 45, 50, 55 ou 60 marqueurs moléculaires sont sélectionnables à partir du tableau 1 et ne montrent pas d'expression accrue ; dans lequel plus préférentiellement lesdits au moins 1, 2, 3, 4, 5, 6, 7, 8, 9, 10, 15, 20, 25, 30, 35, 40, 45, 50, 55 ou 60 marqueurs moléculaires comprennent PLAUR, NEU1, CTSB, CXCL16, ICAM1, MSR1, OLR1, SIRPA, TNFRSF1A, TNFSF14, TNFSF9, PMB22, CD40, LAMP3, CD80, CCR7, LOX1, CD83, ADAM Décysine, FPRL2, GPNMB et/ou CD86.

3. Procédé selon l'une quelconque de revendications 1 ou 2, dans lequel
i) ladite quantité extracorporelle dudit échantillon de sang de sujet mammifère est soumise à une force physique en passant ladite quantité extracorporelle dudit échantillon de sang de sujet mammifère à travers une chambre d'écoulement d'un dispositif, qui permet l'ajustement du débit de ladite quantité extracorporelle dudit échantillon de sang de sujet mammifère à travers ladite chambre d'écoulement dudit dispositif de telle manière qu'une force de cisaillement est appliquée auxdits monocytes contenus dans ledit échantillon de sang de sujet mammifère, et dans lequel ledit dispositif permet de plus l'ajustement d'au moins un paramètre choisi dans le groupe comprenant la température et l'exposition à la lumière ;
ii) dans lequel lesdits monocytes sont activés et induits pour se différencier en cellules dendritiques immuno-suppressives par l'interaction avec des plaquettes activées et/ou de composants du plasma ; et/ou
iii) dans lequel l'activation desdits monocytes et la différentiation en cellules dendritiques immuno-suppressives peuvent être influencées par la conception et les dimensions de la chambre d'écoulement, le débit auquel les monocytes sont passés à travers la chambre d'écoulement, la lumière à laquelle les monocytes sont exposés en présence ou en absence d'agents de réticulation de l'ADN tels que le 8-MOP, la température à laquelle les monocytes, les plaquettes, les facteurs dérivés de plaquettes et/ou des composants de plasma sont passés à travers la chambre d'écoulement, l'ordre par lequel les monocytes, les plaquettes, les facteurs dérivés de plaquettes et/ou des composants de plasma sont passés à travers la chambre d'écoulement, la densité par laquelle les composants de plasma sont revêtus sur les surfaces de la chambre d'écoulement, la densité par laquelle les plaquettes ou les facteurs dérivés de plaquettes adhèrent aux surfaces et ou aux composants de plasma de la chambre d'écoulement, et/ou la densité par laquelle les monocytes adhèrent aux plaquettes et/ou aux facteurs dérivés de plaquettes et ou aux composants de plasma collés aux surfaces de la chambre d'écoulement.

4. Procédé selon l'une quelconque des revendications 1 à 3,
i) ledit procédé comprenant au moins les étapes consistant à :
a) appliquer ladite quantité extracorporelle dudit échantillon de sang de sujet mammifère comprenant au moins des monocytes à un dispositif, qui est configuré pour fournir une chambre d'écoulement à travers laquelle ladite quantité extracorporelle dudit échantillon de sang de sujet mammifère peut être passée,
b) activer les plaquettes, qui peuvent être comprises dans ladite quantité extracorporelle dudit échantillon de sang de sujet mammifère ou qui peuvent être fournies séparées dudit échantillon de sang de sujet mammifère comprenant au moins des monocytes,
c) traiter ladite quantité extracorporelle dudit échantillon de sang de sujet mammifère comprenant au moins des monocytes dans ledit dispositif en appliquant une force physique aux monocytes contenus dans ladite quantité extracorporelle dudit échantillon de sang de sujet mammifère de telle manière que lesdits monocytes sont activés et induits pour se différencier en cellules dendritiques immuno-suppressives par la liaison auxdites plaquettes activées obtenues dans l'étape b) ; ou
ii) ledit procédé comprenant les étapes consistant à :
a) appliquer ladite quantité extracorporelle dudit échantillon de sang de sujet mammifère comprenant au moins des monocytes à un dispositif, qui est configuré pour fournir une chambre d'écoulement à travers laquelle ladite quantité extracorporelle dudit échantillon de sang de sujet mammifère peut être passée,
b) passer les composants du plasma, qui peuvent être compris dans ladite quantité extracorporelle dudit échantillon de sang de sujet mammifère ou qui peuvent être fournis séparés dudit échantillon de sang de sujet mammifère,
c) traiter ladite quantité extracorporelle dudit échantillon de sang de sujet mammifère comprenant au moins des monocytes dans ledit dispositif en appliquant une force physique aux monocytes contenus dans ladite quantité extracorporelle dudit échantillon de sang de sujet mammifère de telle manière que lesdits monocytes sont activés et induits pour se différencier en cellules dendritiques immuno-suppressives par la liaison auxdits composants de plasma obtenus dans l'étape b) ; ou
iii) ledit procédé comprenant au moins les étapes consistant à :
a) appliquer ladite quantité extracorporelle dudit échantillon de sang de sujet mammifère comprenant au moins des monocytes à un dispositif, qui est configuré pour fournir une chambre d'écoulement à travers laquelle ladite quantité extracorporelle dudit échantillon de sang de sujet mammifère peut être passée,
b) passer les composants du plasma, qui peuvent être compris dans ladite quantité extracorporelle dudit échantillon de sang de sujet mammifère ou qui peuvent être fournis séparés dudit échantillon de sang de sujet mammifère,
c) activer les plaquettes, qui peuvent être comprises dans ladite quantité extracorporelle dudit échantillon de sang de sujet mammifère ou qui peuvent être fournies séparées dudit échantillon de sang de sujet mammifère comprenant au moins des monocytes,
d) traiter ladite quantité extracorporelle dudit échantillon de sang de sujet mammifère comprenant au moins des monocytes dans ledit dispositif en appliquant une force physique aux monocytes contenus dans ladite quantité extracorporelle dudit échantillon de sang de sujet mammifère de telle manière que lesdits monocytes sont activés et induits pour se différencier en cellules dendritiques immuno-suppressives par la liaison auxdites plaquettes activées et/ou composants de plasma obtenus dans les étapes b) et c).

5. Procédé selon la revendication 4, dans lequel ladite quantité extracorporelle dudit échantillon de sang de sujet mammifère n'a pas été obtenue par aphérèse ; dans lequel
i) ladite quantité extracorporelle dudit échantillon de sang de sujet de mammifère est comprise entre environ 10 ml à environ 500 ml de sang entier extracorporel dudit sujet mammifère ;
ii) dans lequel ladite quantité extracorporelle dudit échantillon de sang mammifère est obtenue en isolant les leucocytes d'environ 10 ml à environ 500 ml de sang entier extracorporel dudit sujet mammifère ; et/ou
iii) dans lequel ladite quantité extracorporelle dudit échantillon de sang de sujet mammifère est obtenue en isolant les couches leuco-plaquettaires d'environ 10 ml à environ 500 ml de sang entier extracorporel dudit sujet mammifère.

6. Procédé selon la revendication 5, dans lequel
i) ladite quantité extracorporelle dudit échantillon de sang de sujet mammifère ne comprend pas de composants du plasma ; et/ou
ii) dans lequel ladite quantité extracorporelle dudit échantillon de sang de sujet mammifère ne comprend pas de plaquettes, dans lequel préférentiellement lesdites plaquettes ont été séparées de ladite quantité extracorporelle dudit échantillon de sang de sujet mammifère avant que ladite quantité extracorporelle dudit échantillon de sang de sujet mammifère ne soit appliquée audit dispositif.

7. Procédé selon la revendication 4, dans lequel ladite quantité extracorporelle dudit échantillon de sang de sujet mammifère a été obtenue par aphérèse ; et
i) dans lequel préférentiellement i) ladite quantité extracorporelle dudit échantillon de sang de sujet mammifère est obtenue en isolant les leucocytes par leucaphérèse ; dans lequel préférentiellement ladite quantité extracorporelle dudit échantillon de sang de sujet mammifère est obtenue en isolant les couches leuco-plaquettaires par leucaphérèse ;
et/ou
ii) ladite quantité extracorporelle dudit échantillon de sang de sujet mammifère ne comprend pas de composants de plasma ; et/ou
iii) ladite quantité extracorporelle dudit échantillon de sang de sujet mammifère ne comprend pas de plaquettes, dans lequel préférentiellement lesdites plaquettes ont été séparées de ladite quantité extracorporelle dudit échantillon de sang de sujet mammifère avant que ladite quantité extracorporelle dudit échantillon de sang de sujet mammifère ne soit appliquée audit dispositif.

8. Procédé selon l'une quelconque des revendications 4 à 7, dans lequel
i) ladite chambre d'écoulement a des dimensions d'environ 1 µm jusqu'à environ 400 µm de hauteur et d'environ 1 µm jusqu'à environ 400 µm de largeur, dans lequel préférentiellement ladite chambre d'écoulement a des dimensions d'environ 5 µm à jusqu'à et comprenant environ 300 µm de hauteur et d'environ 5 µm à jusqu'à et comprenant environ 300 µm de largeur, dans lequel plus préférentiellement ladite chambre d'écoulement a des dimensions d'environ 10 µm jusqu'à et comprenant environ 250 µm de hauteur et d'environ 10 µm jusqu'à et comprenant environ 250 µm de largeur, et dans lequel encore plus préférentiellement ladite chambre d'écoulement a des dimensions d'environ 50 µm jusqu'à et comprenant environ 200 µm de hauteur et d'environ 50 µm jusqu'à et comprenant environ 200 µm de largeur ; et dans lequel encore plus préférentiellement ladite chambre d'écoulement a des dimensions d'environ 50 µm jusqu'à et comprenant environ 100 µm de hauteur et d'environ 50 µm jusqu'à et comprenant environ 100 µm de largeur ; et/ou
ii) ladite chambre d'écoulement est configurée pour prélever un volume compris entre environ 1 ml à environ 50 ml de ladite quantité extracorporelle dudit échantillon de sang de mammifère.

9. Procédé selon l'une quelconque des revendications 3 à 8, dans lequel
i) le matériau de ladite chambre d'écoulement n'est pas en plastique, et dans lequel préférentiellement ledit matériau non en plastique est choisi dans le groupe constitué par le verre ; ou
ii) le matériau de ladite chambre d'écoulement est en plastique, dans lequel préférentiellement ledit matériau en plastique est choisi dans le groupe constitué par les acryliques, le polycarbonate, le polyétherimide, la polysulfone, la polyphénylsulfone, les styrènes, le polyuréthanne, le polyéthylène, le téflon ou n'importe quel autre plastique de grade médical approprié ; et/ou
iii) ladite chambre d'écoulement est configurée pour permettre la transmission de la lumière.

10. Procédé selon l'une quelconque des revendications 3 à 9, dans lequel
i) l'activation desdites plaquettes est obtenue en disposant les composants du plasma, qui sont compris dans ladite quantité extracorporelle dudit échantillon de sang de sujet mammifère, sur la surface de ladite chambre d'écoulement de telle manière qu'au moins certaines desdites plaquettes peuvent interagir avec lesdits composants du plasma et sont immobilisées sur la surface de ladite chambre d'écoulement ;
ii) l'activation desdites plaquettes est obtenue en disposant les protéine choisies dans le groupe comprenant le fibrinogène, la fibronectine et le composant gamma de fibrinogène sur la surface de ladite chambre d'écoulement de telle manière qu'au moins certaines desdites plaquettes peuvent interagir avec lesdites protéines et sont immobilisées sur la surface de la chambre d'écoulement, dans lequel préférentiellement l'activation desdites plaquettes est obtenue en disposant la fibronectine sur la surface de ladite chambre d'écoulement de telle manière qu'au moins certaines desdites plaquettes peuvent interagir avec ladite fibronectine et sont immobilisées sur la surface de ladite chambre d'écoulement ; et/ou
iii) l'activation des plaquettes peut être suivie par l'expression de la P-sélectine et/ou de l'intégrine αIIb-β3.

11. Procédé selon l'une quelconque des revendications 3 à 10, dans lequel
i) lesdites plaquettes sont passées à travers ladite chambre d'écoulement sous une force de cisaillement d'environ 0,1 à environ 10 dynes/cm², préférentiellement dans une plage d'environ 0,1 à environ 2,0 dynes/cm², et/ou
ii) lesdits monocytes sont passés à travers ladite chambre d'écoulement avec un débit d'environ 10 ml/minute à environ 200 ml/minute pour produire une force de cisaillement d'environ 0,1 à environ 20 dynes/cm².

12. Procédé selon l'une quelconque des revendications 3 à 11,
i) dans lequel lesdits monocytes sont activés et induits pour se différencier en cellules dendritiques immuno-suppressives en passant lesdits monocytes à travers ladite chambre d'écoulement sous une forme de cisaillement d'environ 0,1 à environ 10,0 dynes/cm², préférentiellement une force d'environ 0,1 à environ 1,0 dyne/cm² de telle manière que lesdits monocytes peuvent se lier auxdites plaquettes activées ; ou
ii) le procédé comprenant en outre l'étape d'incubation des monocytes activés pour permettre la formation de cellules dendritiques immuno-suppressives.

13. Procédé selon l'une quelconque des revendications 1 à 12 pour obtenir
i) des cellules dendritiques immuno-suppressives fonctionnellement et maturationnellement synchronisées spécifiques d'un individu ; ou
ii) des cellules dendritiques autologues immuno-suppressives ; ou
iii) des cellules dendritiques allogènes immunosuppressives.
